(19) Europäisches Patentamt
European Patent Office
Office européen des brevets

(11) EP 4 582 415 A1

(12) **EUROPEAN PATENT APPLICATION**
published in accordance with Art. 153(4) EPC

(43) Date of publication:
**09.07.2025 Bulletin 2025/28**

(21) Application number: **23860698.2**

(22) Date of filing: **27.07.2023**

(51) International Patent Classification (IPC):
***C07D 333/28*** *(2006.01)* ***C07D 277/32*** *(2006.01)*
***C07D 307/56*** *(2006.01)* ***C07D 345/00*** *(2006.01)*
***H10K 85/60*** *(2023.01)* ***H10K 30/20*** *(2023.01)*

(52) Cooperative Patent Classification (CPC):
**C07D 277/32; C07D 307/56; C07D 333/28; C07D 345/00; H10K 30/00; H10K 30/20; H10K 85/60; H10K 99/00**

(86) International application number:
**PCT/KR2023/010924**

(87) International publication number:
**WO 2024/049017 (07.03.2024 Gazette 2024/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.08.2022 KR 20220109613**

(71) Applicant: **Konkuk University Industrial Cooperation Corp.**
**Gwangjin-gu**
**Seoul 05029 (KR)**

(72) Inventors:
- **MOON, Doo Kyung**
  **Seoul 06670 (KR)**
- **JEON, Sung Jae**
  **Seoul 05029 (KR)**

(74) Representative: **Isarpatent**
**Patent- und Rechtsanwälte**
**Barth Hassa Peckmann & Partner mbB**
**Friedrichstraße 31**
**80801 München (DE)**

(54) **ORGANIC SEMICONDUCTOR COMPOUND WITH HIGH TRANSMITTANCE IN VISIBLE LIGHT SPECTRUM**

(57) The present disclosure relates to an organic semiconductor compound with high transmittance in the visible light spectrum and a use thereof.



Processed by Luminess, 75001 PARIS (FR)

(Cont. next page)

【Fig. 1】

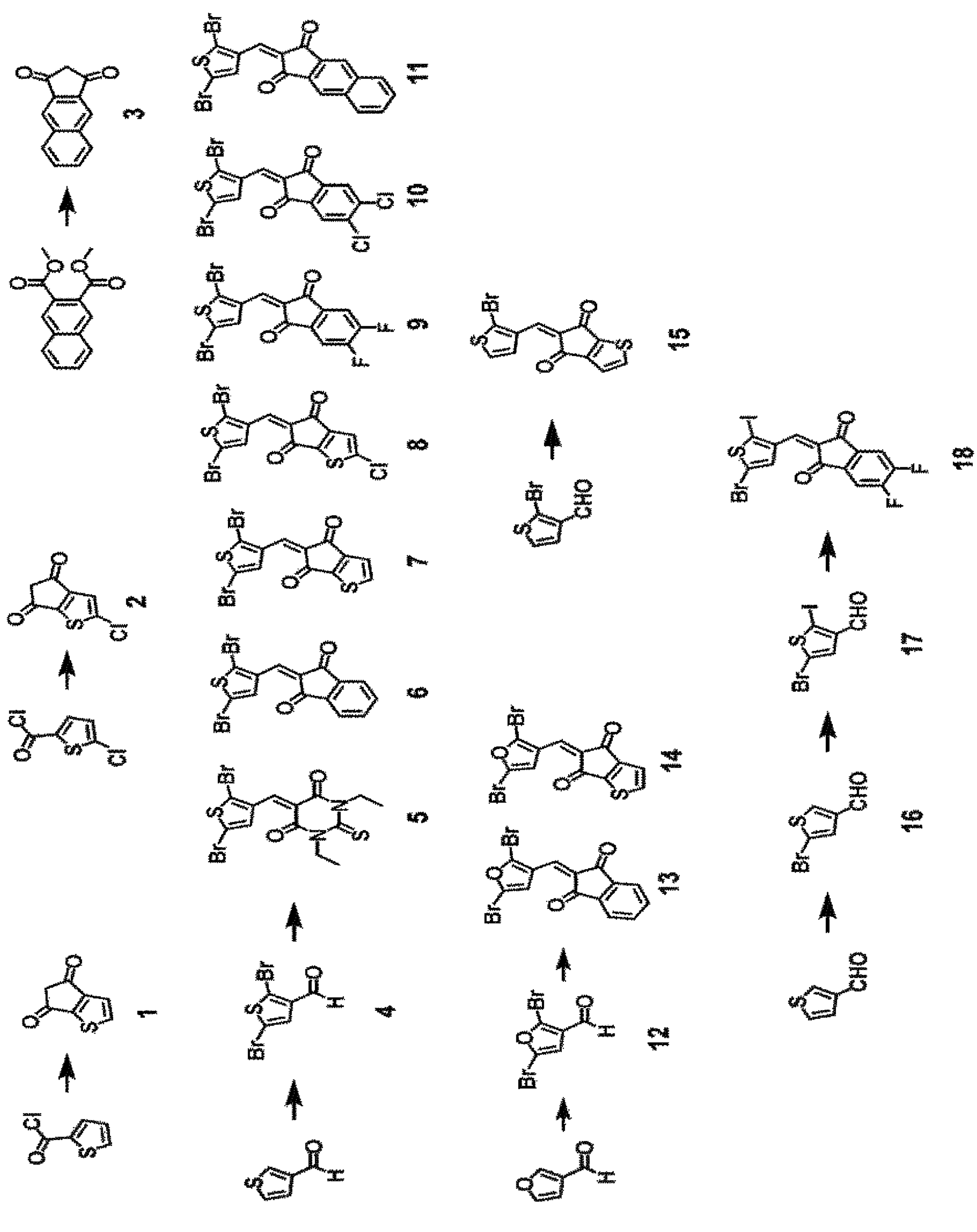

1
2
3
4
5
6
7
8
9
10
11
12
13
14
15
16
17
18

## Description

### [Technical Field]

The present disclosure relates to an organic semiconductor compound with high transmittance in the visible light spectrum and a use thereof.

### [Background Art]

As high oil prices and environmental pollution caused by the use of fossil fuels become a global issue, the demand for sustainable, eco-friendly energy sources is rapidly increasing. Representative eco-friendly energy sources include solar power, wind power, water power, wave power, geothermal heat, and the like, and among them, solar cells, which may produce electricity using solar power, are attracting attention as the most location-independent and infinite source of electrical energy.

It is estimated that the amount of solar energy that may be practically recovered from 1.7 X $10^5$ TW of solar energy reaching the Earth's surface is 600 TW. At this time, if a solar power plant with 10% efficiency is available, about 60 TW of electricity may be supplied. The amount is a huge amount, more than enough to meet future demands for sustainable energy sources, compared to the Earth's estimated energy demand of 28 TW by 2050.

Currently, as solar cells, first-generation crystalline silicon solar cells using inorganic materials account for 90% of the solar power generation market. However, since the generation cost of the silicon solar cells is 5 to 20 times higher than that of fossil fuels, the silicon solar cells are mainly used for long-term, medium- to large-scale power generation, and its utility is limited due to the problem of material finiteness.

To this end, second-generation thin-film solar cell technologies (CdTe, CIGS, etc.) replaced with silicon are rapidly emerging, and account for more than half of the remaining 10% market. However, the second-generation solar cell technologies also require expensive equipment because some materials are classified as precious metals and semiconductor thin films are formed through vacuum and high-temperature processes when manufacturing devices.

There are organic solar cells as the solar cell technology to solve these problems. Since organic materials are used, the organic solar cells may be massproduced through a solution process to lower the cost of solar cells. In addition, due to its mechanical flexibility, ease of design, and diversity, the organic solar cells have endless potential applications in clothing, portable electric and electronic products, and the like, and thus have attracted attention as a next-generation solar cell.

In order to commercialize the organic solar cells, the development of printable photoactive layer materials capable of implementing high efficiency, high stability, large area, and modularization of solar cells is a very important prerequisite. Among these, high efficiency of organic solar cells needs to be achieved.

The production cost may be drastically reduced only by developing a high-performance (high-efficiency and high-stability) material with high solubility that may be processed in a low-temperature solution, and technical problems may be sequentially solved.

Recently, organic solar cells are divided into fullerene-based and non-fullerene-based organic solar cells depending on a type of electron acceptor material in a photoactive layer. As of December 2019, the world's highest efficiency for organic solar cells is 11.5%, certified by HKUST based on the National Renewable Energy Laboratory (NREL) certification standard for fullerene-based organic solar cells, and 18.2%, certified by SJTU/BUAA for non-fullerene-based organic solar cells, which is higher than the world's highest efficiency for the fullerene-based organic solar cells.

While it took about 15 years or more to develop the fullerene-based organic solar cells to a current level, it took less than 5 years for the non-fullerene-based organic solar cells. In addition, it has been reported that non-fullerene-based organic solar cells are superior to fullerene-based organic solar cells in terms of the stability of organic solar cells, and thus, the development of high-performance non-fullerene-based organic solar cells is progressing rapidly (Nature Communication, 2016, 7, 11585; Nature Materials, 2017, 16, 363-369).

Representatively, in the case of PCE11, an analogous derivative that has shown the world's highest efficiency in fullerene-based organic solar cells, a burn-in phenomenon occurs after 5 days of aging in the air, and thus it may be seen that the efficiency rapidly decreases by about 39%. On the other hand, in the case of non-fullerene-based organic solar cells, the efficiency was not reduced by 15% even after 5 days of aging.

Most recently, the efficiency of 20.2% was reported for a tandem-structured organic solar cell device (https://doi.org/10.1016/j.joule.2021.12.017), and the efficiency of almost 19% was reported for a single-structured organic solar cell device (Advanced Materials 2021, 2102420). Despite surpassing the efficiency of currently commercialized silicon solar cells, the organic solar cell field has not yet been commercialized.

This suggests that the commercialization of organic solar cells may not be based solely on pursuing higher efficiency. Therefore, it is now important to develop target-purpose materials that may maximize the advantages of organic

solar cells and in terms of end products/industries that may be achieved by only organic solar cells (preferably, silicon solar cells and perovskite solar cells are applied to indoor power generation or transparent solar cells rather than outdoor power generation strong in many aspects).

[Prior Arts]

[Non-Patent Documents]

(Non-Patent Document 1) Nature Communication, 2016, 7, 11585,
(Non-Patent Document 2) Nature Materials, 2017, 16, 363-369

**[Disclosure]**

**[Technical Problem]**

Accordingly, the present inventors had synthesized a novel unit having both stable high efficiency of 14 to 16.5% or more based on the cost-effective aspect to be required by organic solar cell materials, and high transmittance of 40 to 30% in the entire visible light spectrum of 380 to 780 nm so as to be applied to Internet of Things (IoT) sensors, Building Integrated Photovoltaics (BIPVs), Automobile Integrated Photovoltaics (AIPVs), etc., and then completed the present disclosure.

Therefore, an object of the present disclosure is to provide an organic semiconductor compound with high transmittance in the visible light spectrum and an organic semiconductor material introduced with the organic semiconductor compound.

**[Technical Solution]**

In order to achieve the object, the present disclosure provides a compound represented by the following Chemical Formula 1:

[Chemical Formula 1]

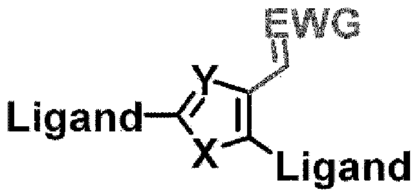

In Chemical Formula 1,

Ligands are reactive ligands which are identical to or different from each other, and each independently H, halogen or pseudohalogen, wherein the halogen is selected from the group consisting of Cl, Br and I, and the pseudohalogen is selected from the group consisting of OTf, $OPO(OR)_2$ and an acetoxy group,
X and Y are identical to or different from each other, and each independently chalcogens, wherein the chalcogen is selected from the group consisting of N, N-R, C, O, S, and Se,
EWG is selected from the group consisting of the following structures,

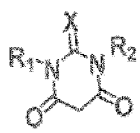 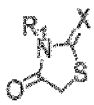 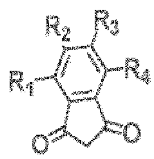 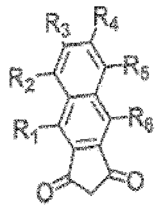 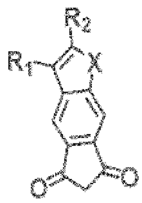

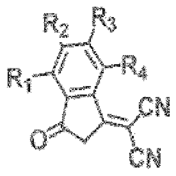 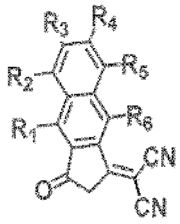 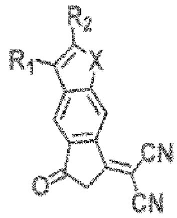 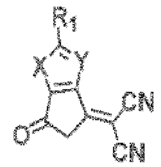 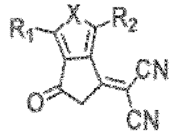

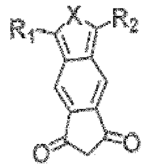 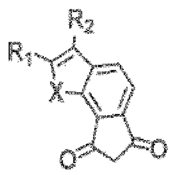 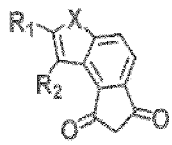 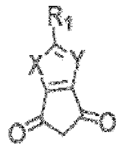 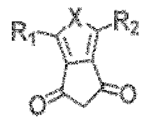

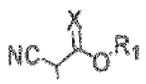 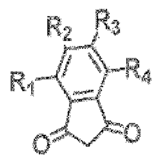 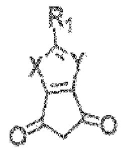 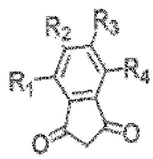 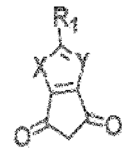

in Chemical Formula above, R and $R_1$ to $R_4$ are identical to or different from each other, and each independently selected from the group consisting of hydrogen; a cyano group; a halogen group; a substituted or unsubstituted alkyl group; a substituted or unsubstituted cycloalkyl group; a substituted or unsubstituted aryloxy group; a substituted or unsubstituted arylsulfoxy group; a substituted or unsubstituted boron group; a substituted or unsubstituted alkylamine group; a substituted or unsubstituted aralkylamine group; a substituted or unsubstituted arylamine group; a substituted or unsubstituted heteroarylamine group; a substituted or unsubstituted aryl group; a substituted or unsubstituted fluorenyl group; a substituted or unsubstituted carbazole group; a substituted or unsubstituted alkoxy group; a substituted or unsubstituted thionyl group; a substituted or unsubstituted silyl group; a substituted or unsubstituted alkenyl group; a substituted or unsubstituted ester group; a substituted or unsubstituted aromatic group; and substituted or unsubstituted heterocyclic groups containing one or more elements selected from the group consisting of N, O, S and Se, or two adjacent substituents may form a condensed ring.

The present disclosure also provides an organic semiconductor compound represented by the following Chemical Formula 2-1:

[Chemical Formula 2-1]

$$\left[ D - A \right]_n$$

In Chemical Formula 2-1 above, A is an electron acceptor unit which is a compound represented by Chemical Formula 1 according to the present disclosure, n is an integer of 1 to 10,000, and D is an electron donor selected from compounds represented by the following structures,

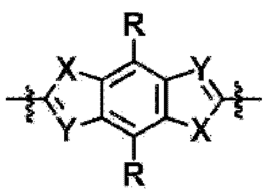 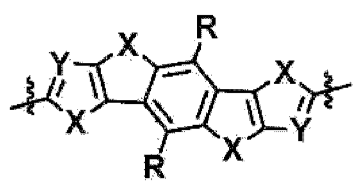

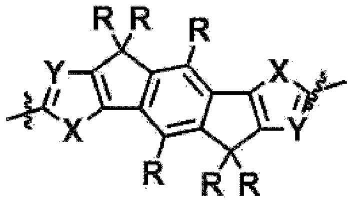

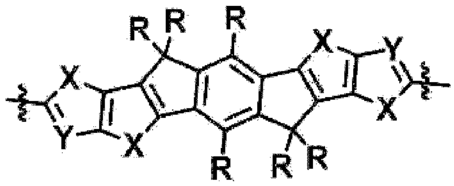

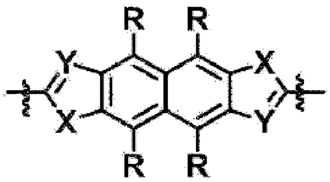

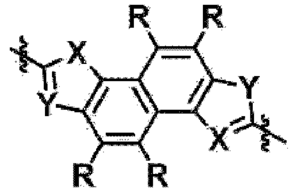

in the structures, X and Y are identical to or different from each other, and are each independently chalcogens, wherein the chalcogen is selected from the group consisting of N, N-R, C, O, S, and Se, and R is identical to or different from each other, and each independently selected from the group consisting of hydrogen; a cyano group; a halogen group; a substituted or unsubstituted alkyl group; a substituted or unsubstituted cycloalkyl group; a substituted or unsubstituted aryloxy group; a substituted or unsubstituted arylsulfoxy group; a substituted or unsubstituted boron group; a substituted or unsubstituted alkylamine group; a substituted or unsubstituted aralkylamine group; a substituted or unsubstituted arylamine group; a substituted or unsubstituted heteroarylamine group; a substituted or unsubstituted aryl group; a substituted or unsubstituted fluorenyl group; a substituted or unsubstituted carbazole group; a substituted or unsubstituted alkoxy group; a substituted or unsubstituted thionyl group; a substituted or unsubstituted silyl group; a substituted or unsubstituted alkenyl group; a substituted or unsubstituted ester group; a substituted or unsubstituted aromatic group; and substituted or unsubstituted heterocyclic groups containing one or more elements selected from the group consisting of N, O, S and Se, or two adjacent substituents may form a condensed ring.

The present disclosure also provides an organic semiconductor compound represented by the following Chemical Formula 4-1:

[Chemical Formula 4-1]

$$\left[ A' - A \right]_n$$

In Chemical Formula 4-1 above, A is an electron acceptor unit which is a compound represented by Chemical Formula 1 according to the present disclosure, n is an integer of 1 to 10,000, and A' is an electron acceptor selected from compounds represented by the following structures, and

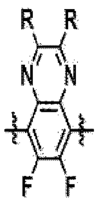

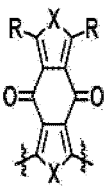

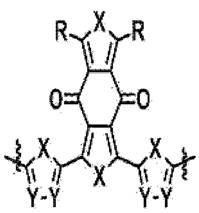

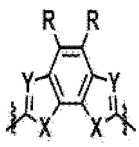

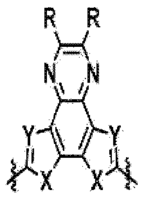

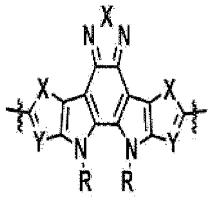 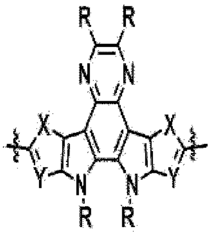 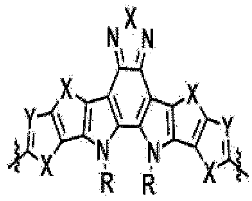 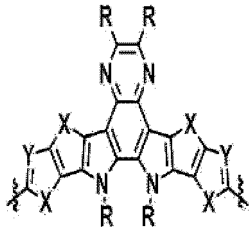

In the structures, X and Y are identical to or different from each other, and each independently chalcogens, wherein the chalcogen is selected from the group consisting of N, N-R, C, O, S, and Se, and R is identical to or different from each other, and each independently selected from the group consisting of hydrogen; a cyano group; a halogen group; a substituted or unsubstituted alkyl group; a substituted or unsubstituted cycloalkyl group; a substituted or unsubstituted aryloxy group; a substituted or unsubstituted arylsulfoxy group; a substituted or unsubstituted boron group; a substituted or unsubstituted alkylamine group; a substituted or unsubstituted aralkylamine group; a substituted or unsubstituted arylamine group; a substituted or unsubstituted heteroarylamine group; a substituted or unsubstituted aryl group; a substituted or unsubstituted fluorenyl group; a substituted or unsubstituted carbazole group; a substituted or unsubstituted alkoxy group; a substituted or unsubstituted thionyl group; a substituted or unsubstituted silyl group; a substituted or unsubstituted alkenyl group; a substituted or unsubstituted ester group; a substituted or unsubstituted aromatic group; and substituted or unsubstituted heterocyclic groups containing one or more elements selected from the group consisting of N, O, S and Se, or two adjacent substituents may form a condensed ring.

The present disclosure also provides an organic semiconductor compound selected from compounds represented by the following Chemical Formulas 6-1 to 6-5:

[Chemical Formula 6-1]

$$\left[\left[D_1\text{-}A_1\right]_l\left[D_1\text{-}A_2\right]_m\right]_n$$

[Chemical Formula 6-2]

$$\left[\left[D_1\text{-}A_1\right]_l\left[D_2\text{-}A_1\right]_m\right]_n$$

[Chemical Formula 6-3]

$$\left[\left[D_1\text{-}A_1\right]_k\left[D_1\text{-}A_2\right]_l\left[D_1\text{-}A_3\right]_m\right]_n$$

[Chemical Formula 6-4]

$$\left[\left[D_1\text{-}A_1\right]_k\left[D_2\text{-}A_1\right]_l\left[D_1\text{-}A_2\right]_m\right]_n$$

[Chemical Formula 6-5]

$$\left[\left[D_1\text{-}A_1\right]_k\left[D_2\text{-}A_1\right]_l\left[D_3\text{-}A_1\right]_m\right]_n$$

In Chemical Formulas 6-1 to 6-5 above, $D_1$, $D_2$ and $D_3$ are identical to or different from each other and are electron donors selected from compounds represented by the following structures,

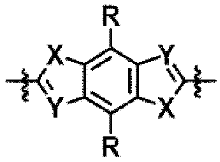
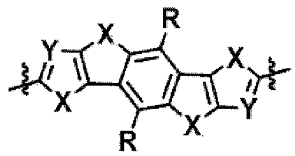

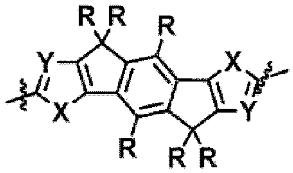
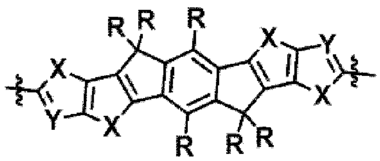

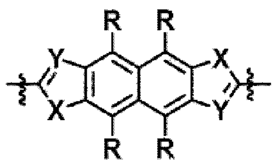
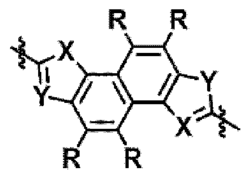

$A_1$, $A_2$, and $A_3$ are identical to or different from each other, and at least one thereof is an electron acceptor unit which is a compound represented by Chemical Formula 1 according to the present disclosure, and the rest are relative electron acceptors selected from compounds represented by the following structures, and

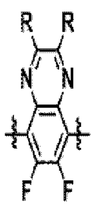
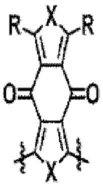
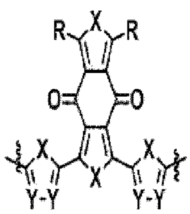
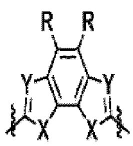
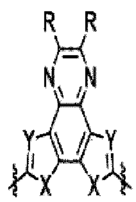

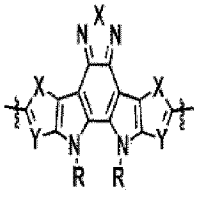
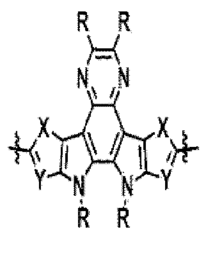
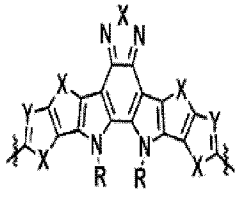
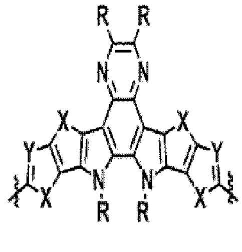

k represents a mole fraction, which is a real number with 0 ≤ k < 1, 1 represents a mole fraction, which is a real number with 0 ≤ 1 < 1, m represents a mole fraction, which is a real number with 0 < m ≤ 1, k + 1 + m = 1, and n is an integer of 1 to 10,000.

The present disclosure also provides an organic semiconductor compound selected from compounds represented by the following Chemical Formulas 8-1 to 8-4:

[Chemical Formula 8-1]

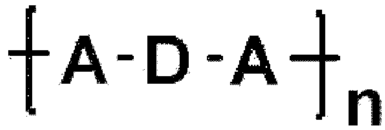

[Chemical Formula 8-2]

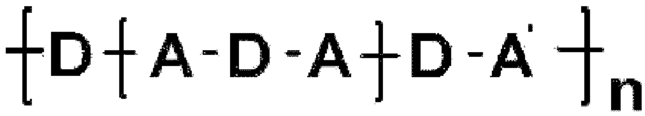

[Chemical Formula 8-3]

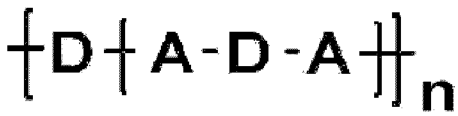

[Chemical Formula 8-4]

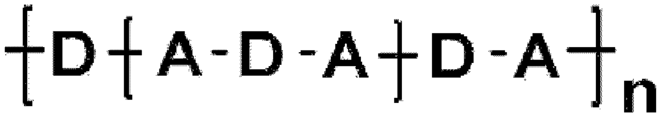

In Chemical Formulas 8-1 to 8-4 above, A is an electron acceptor unit which is a compound represented by Chemical Formula 1 according to the present disclosure, n is an integer of 1 to 10,000, and A' is a relative electron acceptor selected from compounds represented by the following structures,

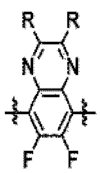
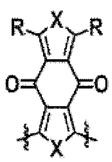
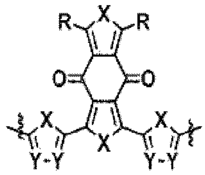
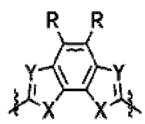
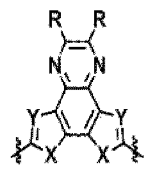

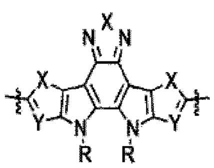
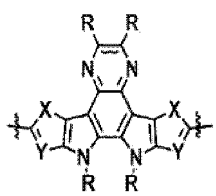
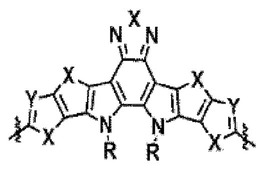
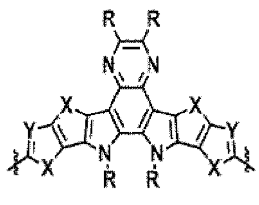

D is an electron donor selected from compounds represented by the following structures, and

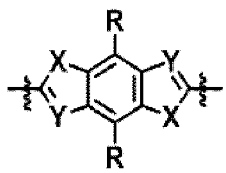
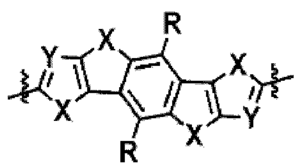

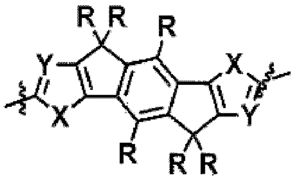
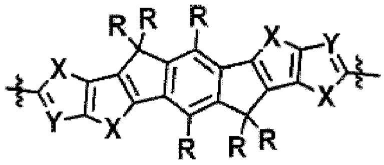

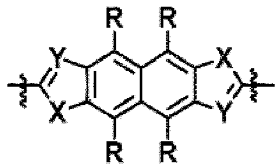
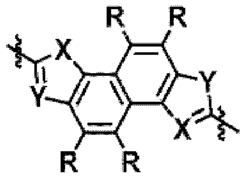

in the structures, X and Y are identical to or different from each other, and each independently chalcogens, wherein the chalcogen is selected from the group consisting of N, N-R, C, O, S, and Se, and R is identical to or different from each other, and each independently selected from the group consisting of hydrogen; a cyano group; a halogen group; a substituted or unsubstituted alkyl group; a substituted or unsubstituted cycloalkyl group; a substituted or unsubstituted aryloxy group; a substituted or unsubstituted arylsulfoxy group; a substituted or unsubstituted boron group; a substituted or unsubstituted alkylamine group; a substituted or unsubstituted aralkylamine group; a substituted or unsubstituted arylamine group; a substituted or unsubstituted heteroarylamine group; a substituted or unsubstituted aryl group; a substituted or unsubstituted fluorenyl group; a substituted or unsubstituted carbazole group; a substituted or unsubstituted alkoxy group; a substituted or unsubstituted thionyl group; a substituted or unsubstituted silyl group; a substituted or unsubstituted alkenyl group; a substituted or unsubstituted ester group; a substituted or unsubstituted aromatic group; and substituted or unsubstituted heterocyclic groups containing one or more elements selected from the group consisting of N, O, S and Se, or two adjacent substituents may form a condensed ring.

In addition, the present disclosure provides an organic solar cell including an organic semiconductor compound according to the present disclosure.

In addition, the present disclosure provides an organic electronic device including an organic semiconductor compound according to the present disclosure.

**[Advantageous Effects]**

According to the present disclosure, the organic semiconductor compound can have both stable high efficiency of 14 to 16.5% or more based on the cost-effective aspect and a high transmittance of 40 to 30% in the entire visible light spectrum of 380 to 780 nm so as to be applied to Internet of Things (IoT) sensors, Building Integrated Photovoltaics (BIPVs), Automobile Integrated Photovoltaics (AIPVs), etc., and thus can be provided as an effective organic solar cell material.

**[Description of Drawings]**

FIG. 1 shows a synthesis process of monomers 1 to 18.
FIG. 2 shows $^1$H NMR of monomer 1.
FIG. 3 shows GC-MS of monomer 1.
FIG. 4 shows $^1$H NMR of monomer 2.
FIG. 5 shows GC-MS of monomer 2.
FIG. 6 shows $^1$H NMR of monomer 3.
FIG. 7 shows GC-MS of monomer 3.
FIG. 8 shows $^1$H NMR of monomer 4.
FIG. 9 shows GC-MS of monomer 4.
FIG. 10 shows $^1$H NMR of monomer 5.
FIG. 11 shows GC-MS of monomer 5.
FIG. 12 shows $^1$H NMR of monomer 6.
FIG. 13 shows $^{13}$C NMR of monomer 6.
FIG. 14 shows GC-MS of monomer 6.
FIG. 15 shows MALDI-TOF of monomer 6.

FIG. 16 shows $^1$H NMR of monomer 7.
FIG. 17 shows $^{13}$C NMR of monomer 7.
FIG. 18 shows GC-MS of monomer 7.
FIG. 19 shows MALDI-TOF of monomer 7.
FIG. 20 shows $^1$H NMR of monomer 8.
FIG. 21 shows $^{13}$C NMR of monomer 8.
FIG. 22 shows GC-MS of monomer 8.
FIG. 23 shows MALDI-TOF of monomer 8.
FIG. 24 shows $^1$H NMR of monomer 9.
FIG. 25 shows $^{13}$C NMR of monomer 9.
FIG. 26 shows GC-MS of monomer 9.
FIG. 27 shows MALDI-TOF of monomer 9.
FIG. 28 shows $^1$H NMR of monomer 10.
FIG. 29 shows $^1$H NMR of monomer 11.
FIG. 30 shows $^1$H NMR of monomer 12.
FIG. 31 shows GC-MS of monomer 12.
FIG. 32 shows $^1$H NMR of monomer 13.
FIG. 33 shows $^1$H NMR of monomer 14.
FIG. 34 shows $^1$H NMR of monomer 15.
FIG. 35 shows $^1$H NMR of monomer 16.
FIG. 36 shows $^1$H NMR of monomer 17.
FIG. 37 shows $^1$H NMR of monomer 18.
FIG. 38 shows a synthesis process of $D_1$-$A_1$ structural polymers 19 to 28.
FIG. 39 shows $^1$H NMR of a $D_1$-$A_1$ structural polymer 19. P(3CHO).
FIG. 40 shows $^1$H NMR of a $D_1$-$A_1$ structural polymer 20. P(3BA).
FIG. 41 shows $^1$H NMR of a $D_1$-$A_1$ structural polymer 21. P(3IN).
FIG. 42 shows $^1$H NMR of a $D_1$-$A_1$ structural polymer 22. P(3TC).
FIG. 43 shows $^1$H NMR of a $D_1$-$A_1$ structural polymer 23. P(3TClCl).
FIG. 44 shows $^1$H NMR of a $D_1$-$A_1$ structural polymer 24. P(3IN2Fl).
FIG. 45 shows $^1$H NMR of a $D_1$-$A_1$ structural polymer 27. P(Fu3IN).
FIG. 46 shows $^1$H NMR of a $D_1$-$A_1$ structural polymer 28. P(Fu3TC).
FIG. 47 shows a synthesis process of $D_1$-$A_1$ structural polymers 29 to 36.
FIG. 48 shows $^1$H NMR of a $D_1$-$A_1$ structural polymer 29. P(F-3BA).
FIG. 49 shows $^1$H NMR of a $D_1$-$A_1$ structural polymer 30. P(F-3IN).
FIG. 50 shows $^1$H NMR of a $D_1$-$A_1$ structural polymer 31. P(Cl-3IN).
FIG. 51 shows $^1$H NMR of a $D_1$-$A_1$ structural polymer 32. P(C6C6-3IN).
FIG. 52 shows $^1$H NMR of a $D_1$-$A_1$ structural polymer 33. P(EDOT-3IN).
FIG. 53 shows $^1$H NMR of a $D_1$-$A_1$ structural polymer 36. P(3IN)-1D.
FIG. 54 shows a synthesis process of $D_1$-$A_1$ structural polymers 37 to 39.
FIG. 55 shows $^1$H NMR of a $D_1$-$A_1$ structural polymer 37. P(IFL-3IN).
FIG. 56 shows $^1$H NMR of a $D_1$-$A_1$ structural polymer 39. P(IDT-3IN).
FIG. 57 shows a synthesis process of $D_1$-$A_1$-$D_1$-$A_2$ structural ternary polymers 40 to 45.
FIG. 58 shows $^1$H NMR of a $D_1$-$A_1$-$D_1$-$A_2$ structural ternary polymer 40. P(3IN)(3IN2F=0.5).
FIG. 59 shows $^1$H NMR of a $D_1$-$A_1$-$D_1$-$A_2$ structural ternary polymer 42. P(3TC)(3IN2F=0.5).
FIG. 60 shows $^1$H NMR of a $D_1$-$A_1$-$D_1$-$A_2$ structural ternary polymer 43. P(3TC)(3TC1Cl=0.5).
FIG. 61 shows $^1$H NMR of a $D_1$-$A_1$-$D_1$-$A_2$ structural ternary polymer 44. P(3TC)(3TC1Cl=0.4).
FIG. 62 shows a synthesis process of $D_1$-$A_1$-$D_2$-$A_1$ structural ternary polymers 46 and 47.
FIG. 63 shows $^1$H NMR of a $D_1$-$A_1$-$D_2$-$A_1$ structural ternary polymer 47. P(3IN)(F-2DBDT=0.5).
FIG. 64 shows a synthesis process of $D_1$-$A_1$-$D_1$-$A_2$ structural ternary polymers 48 and 49.
FIG. 65 shows $^1$H NMR of a $D_1$-$A_1$-$D_1$-$A_2$ structural ternary polymer 48. P(3IN2F)(BDD=0.5).
FIG. 66 shows $^1$H NMR of a $D_1$-$A_1$-$D_1$-$A_2$ structural ternary polymer 49. P(3IN2F)(FTT=0.2).
FIG. 67 shows a synthesis process of $D_1$-$A_1$-$D_1$-$A_2$-$D_1$-$A_3$ structural quaternary polymers 50 to 53.
FIG. 68 shows $^1$H NMR of a $D_1$-$A_1$-$D_1$-$A_2$-$D_1$-$A_3$ structural quaternary polymer 50. P(3IN=0.3)(3IN2F=0.5)(BDD=0.2).
FIG. 69 shows $^1$H NMR of a $D_1$-$A_1$-$D_1$-$A_2$-$D_1$-$A_3$ structural quaternary polymer 51. P(3TC=0.3)(3IN2F=0.5)(BDD=0.2).
FIG. 70 shows $^1$H NMR of a $D_1$-$A_1$-$D_1$-$A_2$-$D_1$-$A_3$ structural quaternary polymer 52. P(3TC=0.3)(3IN2F=0.5)(D18=0.2).
FIG. 71 shows $^1$H NMR of a $D_1$-$A_1$-$D_1$-$A_2$-$D_1$-$A_3$ structural quaternary polymer 53. P(3TC=0.3)(3IN2F=0.5)(3ClTh=0.2).

FIG. 72 shows a synthesis process of $A_1$-$D_1$-$A_1$ structural monomers 54 to 56.
FIG. 73 shows $^1$H NMR of an $A_1$-$D_1$-$A_1$ structural monomer 54. RR-2DBDT-3TC.
FIG. 74 shows $^1$H NMR of an $A_1$-$D_1$-$A_1$ structural monomer 55. RR-2DBDT-3IN2F.
FIG. 75 shows $^1$H NMR of an $A_1$-$D_1$-$A_1$ structural monomer 56. RR-2DBDT-3IN2F-2Br.
FIG. 76 shows a synthesis process of $D_1$-($A_1$-$D_1$-$A_1$)-$D_1$-$A_2$ structural regioregular ternary polymers 57 and 58.
FIG. 77 shows $^1$H NMR of a $D_1$-($A_1$-$D_1$-$A_1$)-$D_1$-$A_2$ structural regioregular ternary polymer 57. RR-P(3IN2F)(BDD=0.5).
FIG. 78 shows $^1$H NMR of a $D_1$-($A_1$-$D_1$-$A_1$)-$D_1$-$A_2$ structural regioregular ternary polymer 58. RR-P(3IN2F)(D18=0.5).
FIG. 79 shows calculation results of the number of synthetic steps and reciprocal yield of acceptor units BDD and D18(=E19) of high-efficiency commercial polymers PM6/PM7 and D18.
FIG. 80 shows calculation results of the number of synthetic steps and reciprocal yield of monomers (acceptor units) of the present disclosure.
FIG. 81 shows results of computing simulations (structural optimization and DFT calculation) of model compounds in repeating unit 1 of polymers P(F-3IN), P(3IN)-in/out, P(3IN2F), and P(3IN2Cl) of the present disclosure through Gaussian16.
FIG. 82 shows results of computing simulations (structural optimization and DFT calculation) of model compounds in repeating unit 1 of polymers P(3TC)-in/out and P(3TC1C1) of the present disclosure through Gaussian16.
FIG. 83 shows results of computing simulations (structural optimization and DFT calculation) of model compounds in repeating unit 2 of polymers P(3IN)-in,in, P(3IN)-in,out, and P(3IN)-out,out of the present disclosure through Gaussian16.
FIG. 84 shows TGA curves for analyzing thermal stability characteristics of polymers (binary polymers, ternary polymers, and quaternary polymers) of the present disclosure.
FIG. 85 shows UV-Vis curves for analyzing optical characteristics of binary polymers of the present disclosure.
FIG. 86 shows UV-Vis curves for analyzing optical characteristics of ternary polymers of the present disclosure.
FIG. 87 shows UV-Vis curves for analyzing optical characteristics of quaternary polymers of the present disclosure.
FIG. 88 shows UV-Vis curves for analyzing optical characteristics of regioregular ternary polymers of the present disclosure.
FIG. 89 shows UV-Vis curves for analyzing optical characteristics according to a temperature in a CB solution of a P(3CHO) polymer of the present disclosure.
FIG. 90 shows UV-Vis curves for analyzing optical characteristics according to a temperature in a CB solution of a P(3BA) polymer of the present disclosure.
FIG. 91 shows UV-Vis curves for analyzing optical characteristics according to a temperature in a CB solution of a P(3IN) polymer of the present disclosure.
FIG. 92 shows UV-Vis curves for analyzing optical characteristics according to a temperature in a CB solution of a P(3TC) polymer of the present disclosure.
FIG. 93 shows UV-Vis curves for analyzing optical characteristics according to a temperature in a CB solution of a P(3IN)-1D polymer of the present disclosure.
FIG. 94 shows UV-Vis curves for analyzing optical characteristics according to a temperature in a CB solution of a P(3IN)(3IN2F=0.5) polymer of the present disclosure.
FIG. 95 shows UV-Vis curves for analyzing optical characteristics according to a temperature in a CB solution of a P(3IN)(3TC1C1=0.4) polymer of the present disclosure.
FIG. 96 shows UV-Vis curves for analyzing optical characteristics according to a temperature in a CB solution of a PM6 polymer of the present disclosure.
FIG. 97 shows UV-Vis curves for analyzing optical characteristics according to a temperature in a CB solution of a PTB7-Th polymer of the present disclosure.
FIG. 98 shows UV-Vis curves for analyzing optical characteristics according to a temperature in a CB solution of a P(3IN=0.3)(3IN2F=0.5)(BDD=0.2) polymer of the present disclosure.
FIG. 99 shows UV-Vis curves for analyzing optical characteristics according to a temperature in an XY solution of a P(3IN=0.3)(3IN2F=0.5)(BDD=0.2) polymer of the present disclosure.
FIG. 100 shows CV curves for analyzing electrochemical characteristics of binary polymers of the present disclosure.
FIG. 101 shows CV curves for analyzing electrochemical characteristics of ternary polymers of the present disclosure.
FIG. 102 shows CV curves for analyzing electrochemical characteristics of quaternary polymers of the present disclosure.
FIG. 103 shows CV curves for analyzing electrochemical characteristics of regioregular ternary polymers of the present disclosure.
FIG. 104 shows optimized J-V curves for analyzing photovoltaic characteristics of organic solar cell devices based on binary polymers of the present disclosure.
FIG. 105 shows optimized EQE curves for analyzing photovoltaic characteristics of organic solar cell devices based on binary polymers of the present disclosure.

FIG. 106 shows optimized J-V curves for analyzing photovoltaic characteristics of organic solar cell devices based on ternary polymers of the present disclosure.

FIG. 107 shows optimized EQE curves for analyzing photovoltaic characteristics of organic solar cell devices based on ternary polymers of the present disclosure.

FIG. 108 shows optimized J-V curves for analyzing photovoltaic characteristics of organic solar cell devices based on quaternary polymers of the present disclosure.

FIG. 109 shows optimized EQE curves for analyzing photovoltaic characteristics of organic solar cell devices based on quaternary polymers of the present disclosure.

FIG. 110 shows optimized J-V curves for analyzing photovoltaic characteristics of organic solar cell devices based on regioregular ternary polymers of the present disclosure.

FIG. 111 shows optimized EQE curves for analyzing photovoltaic characteristics of organic solar cell devices based on regioregular ternary polymers of the present disclosure.

FIG. 112 shows GIWAXS measurement results of binary polymers of the present disclosure. In a clockwise direction from the left, P(3CHO), P(3BA), P(3IN), and P(3TC) are shown.

FIG. 113 shows GIWAXS measurement results for mixed films of binary polymers of the present disclosure and BTP-eC9, respectively. From the left, P(3CHO):BTP-eC9, P(3IN):BTP-eC9, and P(3TC):BTP-eC9 are shown.

FIG. 114 shows line-cutted plots in an out-of-plane direction of the GIWAXS measurement results for the mixed films of the binary polymers of the present disclosure and BTP-eC9, respectively.

FIG. 115 shows GIWAXS measurement results of binary polymers and ternary polymers of the present disclosure. In a clockwise direction from the left, P(3IN2F), P(3IN)(3IN2F=0.5), P(3TC1C1), P(3IN)(3TC1Cl=0.4), and P(3IN2F)(BDD=0.5) are shown.

FIG. 116 shows line-cutted plots in an out-of-plane direction of the GIWAXS measurement results for binary polymers and ternary polymers of the present disclosure.

FIG. 117 shows GIWAXS measurement results for mixed films of ternary polymers of the present disclosure and BTP-eC9, respectively. (a) P(3IN)(3IN2F=0.5):BTP-eC9, P(3IN2F)(BDD=0.5):BTP-eC9)P(3IN):BTP-eC9.

FIG. 118 shows line-cutted plots in an out-of-plane direction of the GIWAXS measurement results for ternary polymers of the present disclosure.

FIG. 119 shows GIWAXS measurement results of a quaternary polymer of the present disclosure and a commercial polymer PM6. From the left, P(3IN=0.3)(3IN2F=0.5)(BDD=0.2) and PM6 are shown.

FIG. 120 shows line-cutted plots in an out-of-plane direction of the GIWAXS measurement results for a quaternary polymer of the present disclosure and a commercial polymer PM6.

FIG. 121 shows AFM results (2D-topography; 3D-topography; 2D phase) measured on a 10 x 10 $\mu m^2$ scale for mixed films of binary polymers of the present disclosure and BTP-eC9, respectively. (a) P(3CHO):BTP-eC9, (b) P(3BA):BTP-eC9, (c) P(3IN):BTP-eC9, and (d) P(3TC):BTP-eC9.

FIG. 122 shows AFM results (2D-topography; 3D-topography; 2D phase) measured on a 10 x 10 $\mu m^2$ scale for mixed films of binary polymers and ternary polymers of the present disclosure and BTP-eC9, respectively. (a) P(3IN2F):BTP-eC9, (b) P(3IN)(3IN2F=0.5):BTP-eC9, (c) P(3TC1C1):BTP-eC9, and (d) P(3IN)(3TC1C1=0.4):BTP-eC9.

FIG. 123 shows AFM results (2D-topography; 3D-topography; 2D phase) measured on a 10 x 10 $\mu m^2$ scale for mixed films of ternary polymers of the present disclosure and BTP-eC9, respectively. (a) P(3IN2F)(BDD=0.5):BTP-eC9.

FIG. 124 shows AFM results (2D-topography; 3D-topography; 2D phase) measured on a 10 x 10 $\mu m^2$ scale for mixed films of quaternary polymers of the present disclosure and BTP-eC9, respectively. (a) P(3IN=0.3)(3IN2F=0.5)(BDD=0.2):BTP-eC9.

FIG. 125 shows contact angle measurement results in DIM and Water for films of binary polymers of the present disclosure, respectively. (a) P(3CHO), (b) P(3BA), (c) P(3IN), and (d) P(3TC).

FIG. 126 shows contact angle measurement results in DIM and Water for films of binary polymers and ternary polymers of the present disclosure, respectively. (a) P(3IN2F), (b) P(3IN)(3IN2F=0.5), (c) P(3TC1C1), and (d) P(3IN)(3TC1C1=0.4).

FIG. 127 shows contact angle measurement results in DIM and Water for a ternary polymer film of the present disclosure. (a) P(3IN2F)(BDD=0.5).

FIG. 128 shows contact angle measurement results in DIM and Water for a quaternary polymer film of the present disclosure. (a) P(3IN=0.3)(3IN2F=0.5)(BDD=0.2).

FIG. 129 shows contact angle measurement results in DIM and Water for a commercial polymer and an acceptor monomer used in the present disclosure. (a) PM6, (b) BTP-eC9.

FIG. 130 shows optimized J-V curves for analyzing photovoltaic characteristics of ternary organic solar cell devices based on a quaternary polymer, P(3IN=0.3)(3IN2F=0.5)(BDD=0.2):PM6:BTP-eC9 of the present disclosure.

FIG. 131 shows optimized EQE curves for analyzing photovoltaic characteristics of ternary organic solar cell devices based on a quaternary polymer, P(3IN=0.3)(3IN2F=0.5)(BDD=0.2):PM6:BTP-eC9 of the present disclosure.

FIGS. 132A to 132F show Nano Convergence Practical Application Center (NCPAC) certificates of the optimization

results of organic solar cell devices based on a quaternary polymer, P(3IN=0.3)(3IN2F=0.5)(BDD=0.2):PM6:BTP-eC9=0.4:0.6:1.2 of the present disclosure.

FIG. 133 shows GIWAXS measurement results of a representative quaternary polymer of the present disclosure and a commercial polymer. (a) P(3IN=0.3)(3IN2F=0.5)(BDD=0.2), (b) PM6.

FIG. 134 shows GIWAXS measurement results according to a ratio combination of ternary organic solar cell devices based on a quaternary polymer, P(3IN=0.3)(3IN2F=0.5)(BDD=0.2):PM6:BTP-eC9 of the present disclosure. (a) 1.0:0:1.2, (b) 0.8:0.2:1.2, (c) 0.6:0.4:1.2, (d) 0.4:0.6:1.2, (e) 0.2:0.8:1.2, and (f) 0:1.0:1.2.

FIG. 135 shows results of measuring TEM (scale: 100 & 10 nm) for a P(3IN=0.3)(3IN2F=0.5)(BDD=0.2):PM6:BTP-eC9-based ternary mixed film and a PM6:BTP-eC9 mixed film under best conditions of the present disclosure. (a) P(3IN=0.3)(3IN2F=0.5)(BDD=0.2):PM6:BTP-eC9=0.4:0.6:1.2, (b) PM6:BTP-eC9=1.0:1.2 (CF).

FIG. 136 shows results of element mapping through TEM (scale: 500 nm) for a P(3IN=0.3)(3IN2F=0.5)(BDD=0.2):PM6:BTP-eC9-based ternary mixed film and a PM6:BTP-eC9 mixed film under best conditions of the present disclosure. (a) P(3IN=0.3)(3IN2F=0.5)(BDD=0.2):PM6:BTP-eC9=0.4:0.6:1.2, (b) PM6:BTP-eC9=1.0:1.2 (CF).

FIG. 137 shows UV-Vis results together with photopic responses in $10^{-5}$ M solutions having different concentrations of a representative quaternary polymer, P(3IN=0.3)(3IN2F=0.5)(BDD=0.2) of the present disclosure and PM6.

FIG. 138 shows UV-Vis results for films of a representative quaternary polymer, P(3IN=0.3)(3IN2F=0.5)(BDD=0.2) of the present disclosure and PM6, together with photopic responses by calculating the absorption coefficient using the Beer-Lambert Equation, respectively.

FIG. 139 shows real photographs for words and color identification ability according to various films having the same thickness of a representative quaternary polymer, P(3IN=0.3)(3IN2F=0.5)(BDD=0.2) of the present disclosure and PM6.

FIG. 140 shows results of performing optical simulation of representative quaternary polymers of the present disclosure and commercial polymers. (a) P(3IN=0.3)(3IN2F=0.5)(BDD=0.2), (b) P(3IN=0.3)(3IN2F=0.5)(BDD=0.2):BTP-eC9=1.0:1.2, (c) ITO/PEDOT:PSS/P(3IN=0.3)(3IN2F=0.5)(BDD=0.2):BTP-eC9=1.0:1.2/P(NDIT-F3N)/Ag, (d) PM6, (e) PM6:BTP-eC9=1.0:1.2, and (f) ITO/PEDOT:PSS/PM6:BTP-eC9=1.0: 1.2/P(NDIT-F3N)/ Ag.

FIG. 141 shows UV-Vis results (absorbance) for optimized ternary mixed films based on P(3IN=0.3)(3IN2F=0.5)(BDD=0.2):PM6:BTP-eC9 of the present disclosure.

FIG. 142 shows UV-Vis results (transmittance) for optimized ternary mixed films based on P(3IN=0.3)(3IN2F=0.5)(BDD=0.2):PM6:BTP-eC9 of the present disclosure.

FIG. 143 shows optimized J-V curves for analyzing photovoltaic characteristics of ternary semitransparent organic solar cell devices based on a quaternary polymer, P(3IN=0.3)(3IN2F=0.5)(BDD=0.2):PM6:BTP-eC9 of the present disclosure.

FIG. 144 shows optimized EQE curves for analyzing photovoltaic characteristics of ternary semitransparent organic solar cell devices based on a quaternary polymer, P(3IN=0.3)(3IN2F=0.5)(BDD=0.2):PM6:BTP-eC9 of the present disclosure.

FIG. 145 shows UV-Vis measurement results (transmittance) of ternary semitransparent organic solar cell devices based on a quaternary polymer, P(3IN=0.3)(3IN2F=0.5)(BDD=0.2):PM6:BTP-eC9 of the present disclosure.

FIG. 146 shows real photographs of ternary semitransparent organic solar cell devices based on a quaternary polymer, P(3IN=0.3)(3IN2F=0.5)(BDD=0.2):PM6:BTP-eC9 of the present disclosure. (a) 1.0:0:1.2, Ag(13 nm)/$WO_3$ (25 nm), (b) 0.4:0.6:1.2, Ag(13 nm)/$WO_3$(25 nm), (c) 1.0:0:1.2, Ag(10 nm)/$WO_3$(25 nm), and (d) 0.4:0.6:1.2, Ag(10 nm)/$WO_3$(25 nm).

FIG. 147 shows real photographs of ternary opaque/semitransparent inversestructural organic solar cell modules based on a quaternary polymer, P(3IN=0.3)(3IN2F=0.5)(BDD=0.2):PM6:BTP-eC9 of the present disclosure. (a) 1:0:1.2, Ag(100 nm), (b) 0.4:0.6:1.2, Ag(15 nm)/$MoO_3$(30 nm).

FIG. 148 shows a synthesis process of a $D_1$-($A_1$-$D_1$-$A_1$) or $D_1$-($A_1$-$D_1$-$A_1$)-$D_1$-$A_1$ structural P(RR-3IN2F)(3TC=0.5).

FIG. 149 shows $^1$H NMR of P(RR-3IN2F)(3TC=0.5).

FIG. 150 shows a synthesis process of an $A_1$-$A_2$ structure P(DPP-3TC).

FIG. 151 shows $^1$H NMR of P(DPP-3TC).

## [Best Mode]

Throughout the present specification, the term "alkyl group" may include linear or branched $C_{1-20}$, $C_{1-10}$ or $C_{1-8}$ alkyl groups, respectively, and may include, for example, methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, heneicosyl, docosyl, tricosyl, tetracosyl, pentacosyl, hexacosyl, heptacosyl, octacosyl, nonacosyl, triacontyl or all possible isomers thereof, but is not limited thereto.

Throughout the present specification, the term "aromatic ring" means including at least one aromatic ring, and

including a $C_{6-30}$ aromatic hydrocarbon ring group, for example, an aromatic ring such as phenyl, naphthyl, biphenyl, terphenyl, fluorene, phenanthrenyl, triphenylenyl, perylenyl, chrysenyl, fluoranthenyl, benzofluorenyl, benzotriphenylenyl, benzocrysenyl, anthracenyl, stilbenyl, pyrenyl, and the like.

Throughout the present specification, the term "halogen" means an element of Group 17 of the periodic table, and means including fluorine (F), chlorine (Cl), bromine (Br), iodine (I), and the like.

Throughout the present specification, the term "alkoxy" means an alkyloxygen radical having an alkyl group, including, for example, methoxy, ethoxy, propoxy, butoxy, pentoxy, and the like.

Throughout the present specification, the term "fusion" means that at least one pair of adjacent atoms is included in two rings, with respect to two or more rings.

Throughout the present specification, the term "fused ring" means one or more fused aromatic rings or unsaturated hydrocarbon rings having 6 to 20 carbon atoms.

The present disclosure relates to a compound represented by the following Chemical Formula 1:

[Chemical Formula 1]

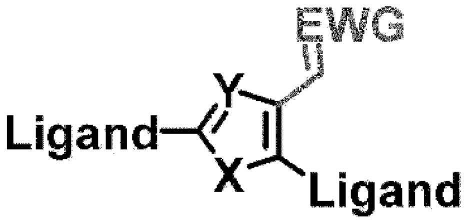

In Chemical Formula 1,

Ligands are reactive ligands which are identical to or different from each other, and each independently H, halogen or pseudohalogen, wherein the halogen is selected from the group consisting of Cl, Br and I, and the pseudohalogen is selected from the group consisting of OTf, $OPO(OR)_2$ and an acetoxy group,

X and Y are identical to or different from each other, and each independently chalcogens, wherein the chalcogen is selected from the group consisting of N, N-R, C, O, S, and Se,

EWG is a monomer structure containing a ketone group, a diketone group, a cyano group, or a monoketone group and a dicyano group, and for example, selected from the group consisting of the following structures, and

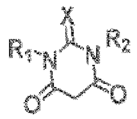
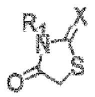
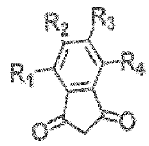
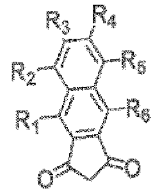
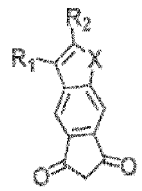
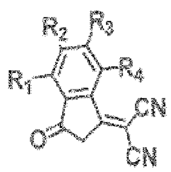
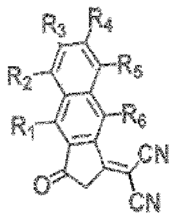
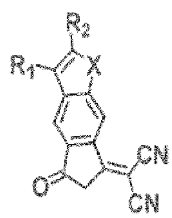
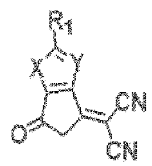
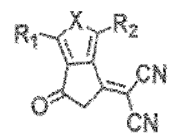
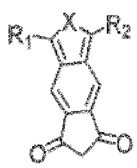
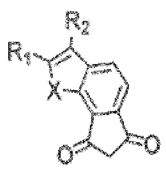
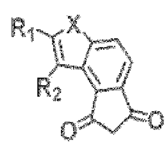
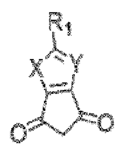
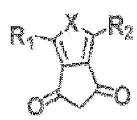

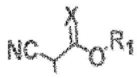 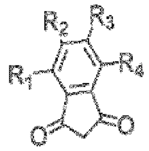 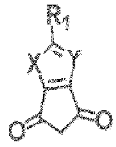 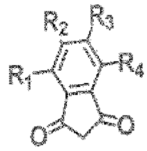 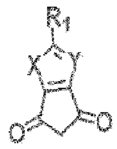

in Chemical Formula above, R and $R_1$ to $R_4$ are identical to or different from each other, and each independently selected from the group consisting of hydrogen; a cyano group; a halogen group; a substituted or unsubstituted alkyl group; a substituted or unsubstituted cycloalkyl group; a substituted or unsubstituted aryloxy group; a substituted or unsubstituted arylsulfoxy group; a substituted or unsubstituted boron group; a substituted or unsubstituted alkylamine group; a substituted or unsubstituted aralkylamine group; a substituted or unsubstituted arylamine group; a substituted or unsubstituted heteroarylamine group; a substituted or unsubstituted aryl group; a substituted or unsubstituted fluorenyl group; a substituted or unsubstituted carbazole group; a substituted or unsubstituted alkoxy group; a substituted or unsubstituted thionyl group; a substituted or unsubstituted silyl group; a substituted or unsubstituted alkenyl group; a substituted or unsubstituted ester group; a substituted or unsubstituted aromatic group; and substituted or unsubstituted heterocyclic groups containing one or more elements selected from the group consisting of N, O, S and Se, or two adjacent substituents may form a condensed ring.

The compound represented by Chemical Formula 1 above may be selected from the following compounds, but is not limited thereto:

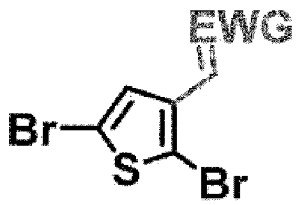 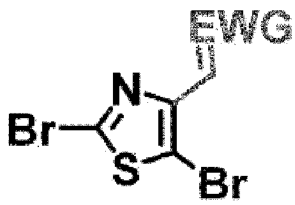 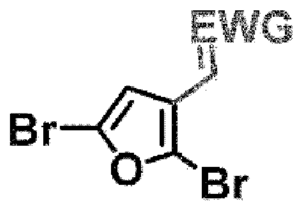

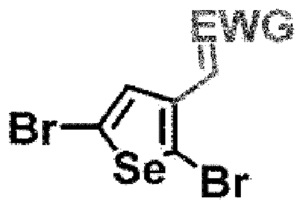 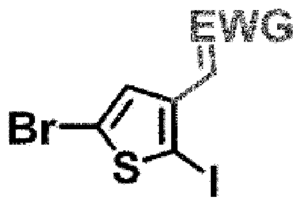

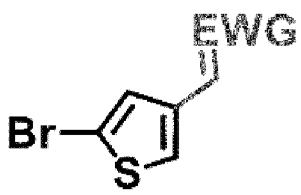 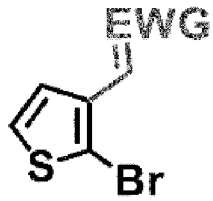 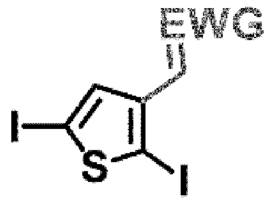

In the compounds, EWG is as defined above.

In addition, the compound represented by Chemical Formula 1 above may be selected from the following compounds, but is not limited thereto:

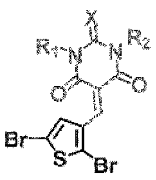 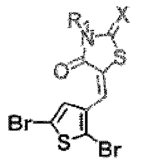 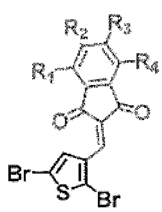 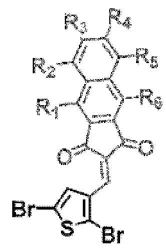 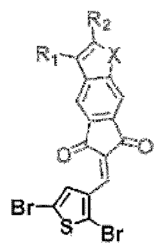

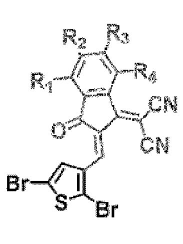
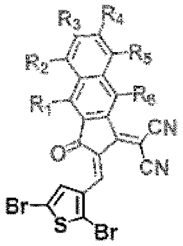
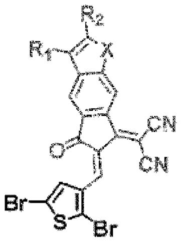
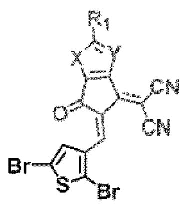
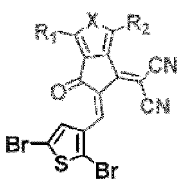

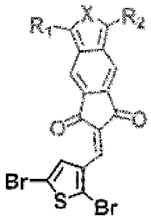
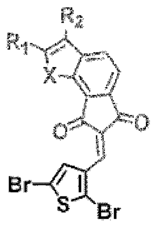
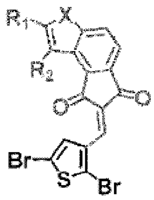
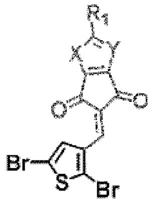
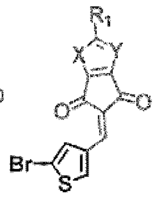
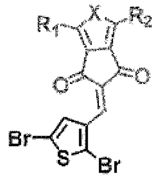

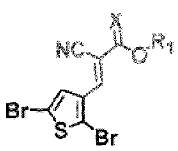
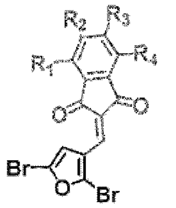
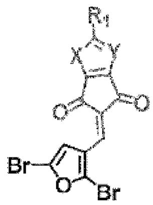
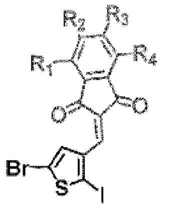
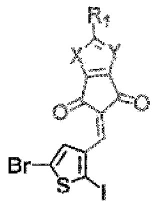

In the compounds, R and $R_1$ to $R_4$ are as defined above.

In addition, the compound represented by Chemical Formula 1 above may be selected from the following compounds, but is not limited thereto:

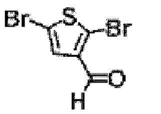
4
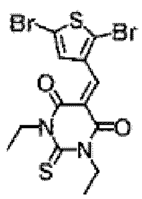
5
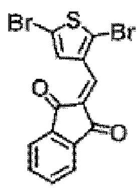
6
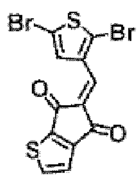
7
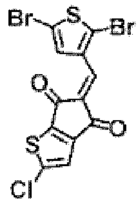
8
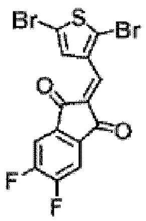
9

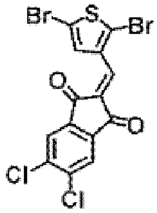
10
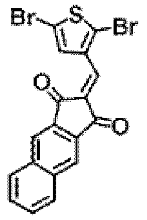
11
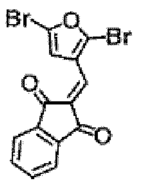
13
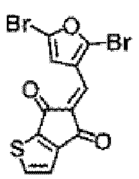
14
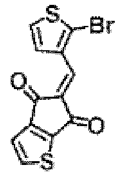
15
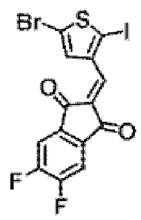
18

The present disclosure also relates to an organic semiconductor compound represented by the following Chemical Formula 2-1:

[Chemical Formula 2-1]

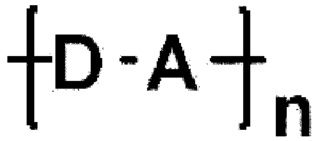

In Chemical Formula 2-1 above, A is an electron acceptor unit which is a compound represented by Chemical Formula 1 according to the present disclosure, n is an integer of 1 to 10,000, and D is an electron donor selected from compounds represented by the following structures, and

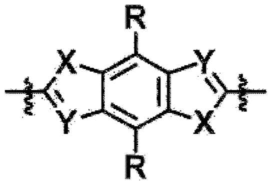
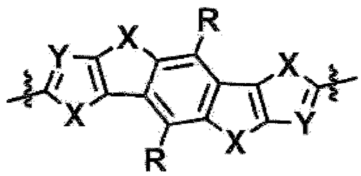

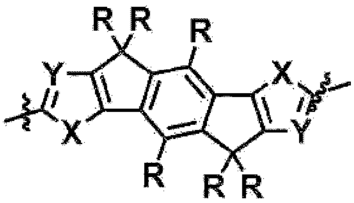
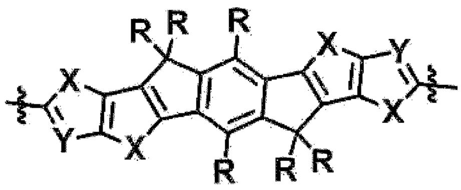

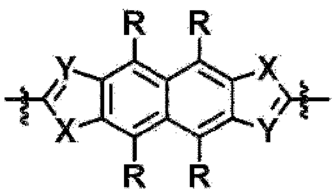
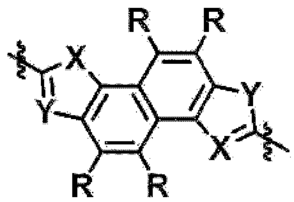

in the structures, X and Y are identical to or different from each other, and each independently chalcogens, wherein the chalcogen is selected from the group consisting of N, N-R, C, O, S, and Se, and R is identical to or different from each other, and each independently selected from the group consisting of hydrogen; a cyano group; a halogen group; a substituted or unsubstituted alkyl group; a substituted or unsubstituted cycloalkyl group; a substituted or unsubstituted aryloxy group; a substituted or unsubstituted arylsulfoxy group; a substituted or unsubstituted boron group; a substituted or unsubstituted alkylamine group; a substituted or unsubstituted aralkylamine group; a substituted or unsubstituted arylamine group; a substituted or unsubstituted heteroarylamine group; a substituted or unsubstituted aryl group; a substituted or unsubstituted fluorenyl group; a substituted or unsubstituted carbazole group; a substituted or unsubstituted alkoxy group; a substituted or unsubstituted thionyl group; a substituted or unsubstituted silyl group; a substituted or unsubstituted alkenyl group; a substituted or unsubstituted ester group; a substituted or unsubstituted aromatic group; and substituted or unsubstituted heterocyclic groups containing one or more elements selected from the group consisting of N, O, S and Se, or two adjacent substituents may form a condensed ring.

The organic semiconductor compound represented by Chemical Formula 2-1 above may be selected from compounds represented by the following Chemical Formulas 3-1 to 3-6, but is not limited thereto:

[Chemical Formula 3-1]

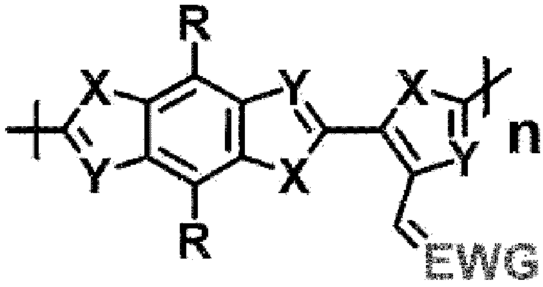

[Chemical Formula 3-2]

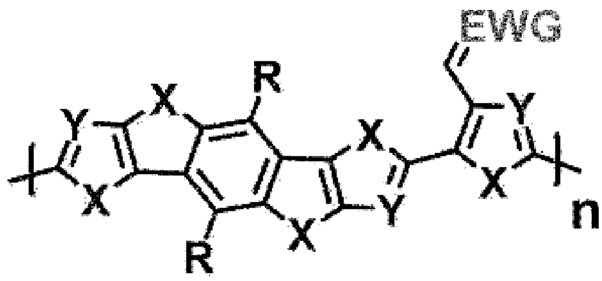

[Chemical Formula 3-3]

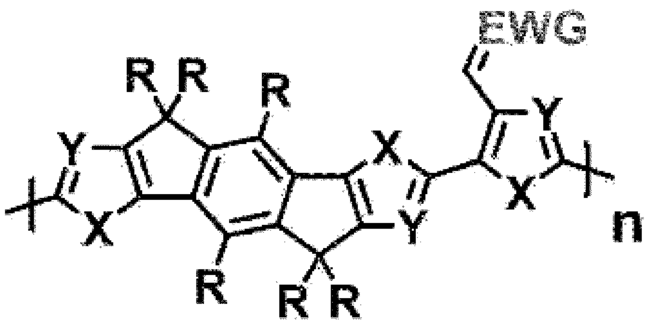

[Chemical Formula 3-4]

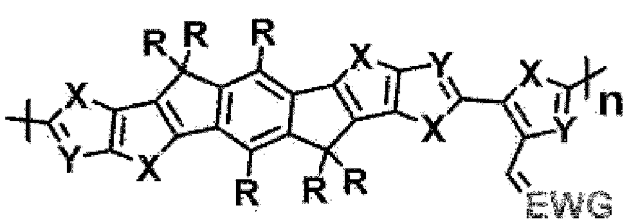

[Chemical Formula 3-5]

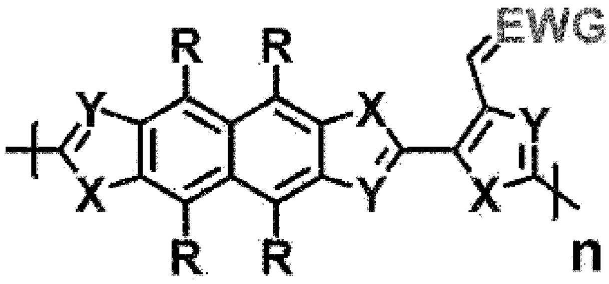

[Chemical Formula 3-6]

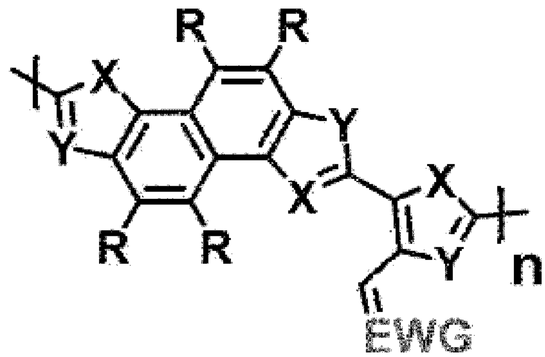

In Chemical Formulas 3-1 to 3-6 above, n is an integer of 1 to 10,000, and R, X, Y, and EWG are as defined above.

The organic semiconductor compound represented by Chemical Formula 2-1 above may be selected from the following compounds, but is not limited thereto:

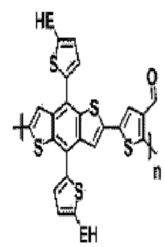

P(3CHO)

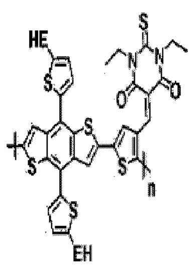

P(3BA)

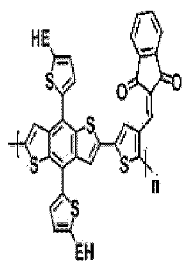

P(3IN)

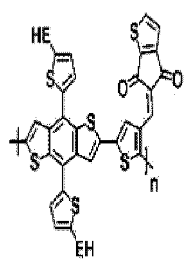

P(3TC)

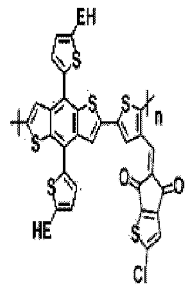

P(3TC1Cl)

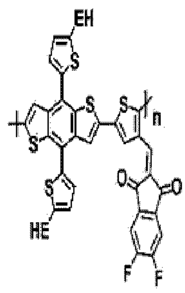

P(3IN2F)

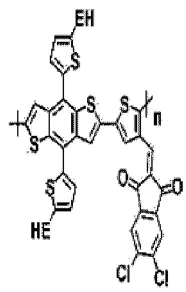

P(3IN2Cl)

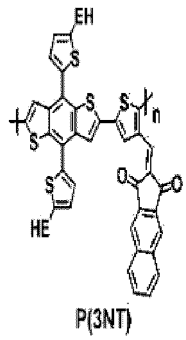

P(3NT)

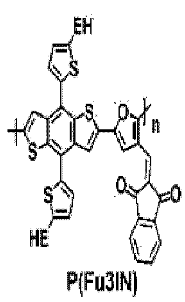

P(Fu3IN)

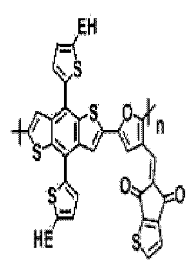

P(Fu3TC)

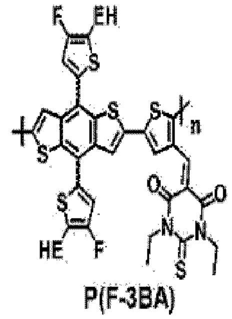
P(F-3BA)

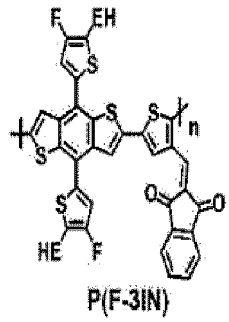
P(F-3IN)

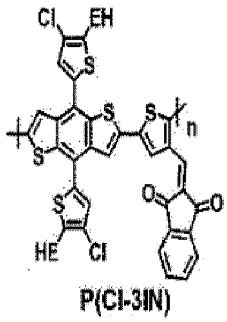
P(Cl-3IN)

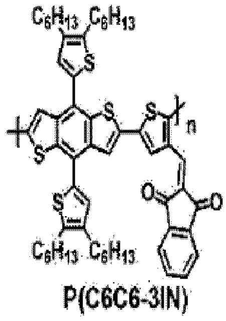
P(C6C6-3IN)

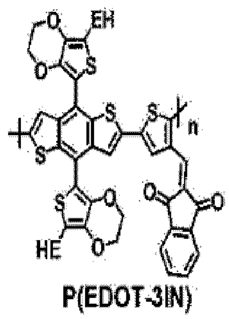
P(EDOT-3IN)

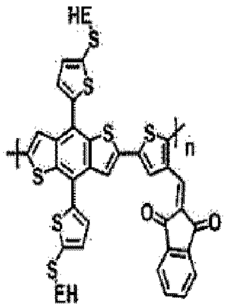
P(SEH-3IN)

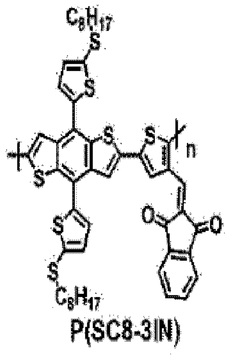
P(SC8-3IN)

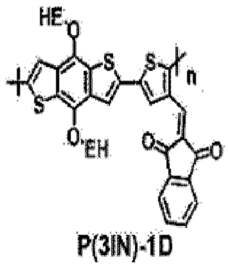
P(3IN)-1D

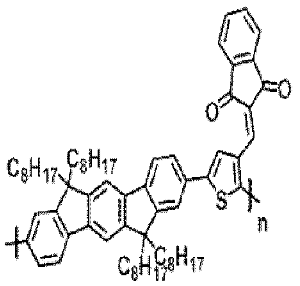
P(IFL-3IN)

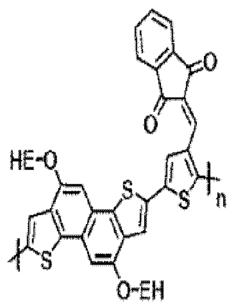
P(NDT-3IN)

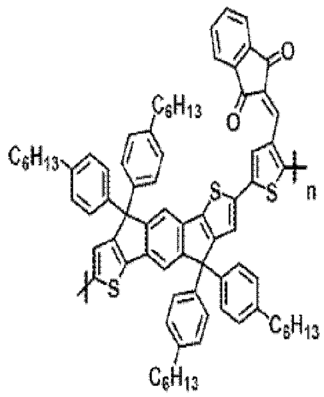
P(IDT-3IN)

The present disclosure also relates to an organic semiconductor compound represented by the following Chemical Formula 4-1:

[Chemical Formula 4-1]

$$\left[ A' - A \right]_n$$

In Chemical Formula 4-1 above, A is an electron acceptor unit which is a compound represented by Chemical Formula 1 according to the present disclosure, n is an integer of 1 to 10,000, and A' is an electron acceptor selected from compounds represented by the following structures, and

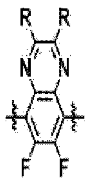 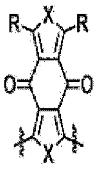 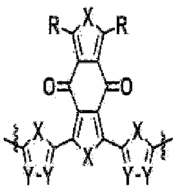 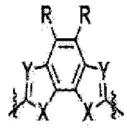 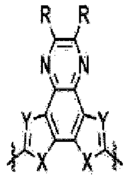

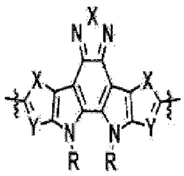 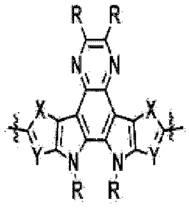 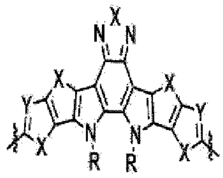 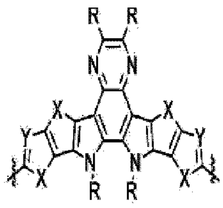

in the structures, X and Y are identical to or different from each other, and each independently chalcogens, wherein the chalcogen is selected from the group consisting of N, N-R, C, O, S, and Se, and R is identical to or different from each other, and each independently selected from the group consisting of hydrogen; a cyano group; a halogen group; a substituted or unsubstituted alkyl group; a substituted or unsubstituted cycloalkyl group; a substituted or unsubstituted aryloxy group; a substituted or unsubstituted arylsulfoxy group; a substituted or unsubstituted boron group; a substituted or unsubstituted alkylamine group; a substituted or unsubstituted aralkylamine group; a substituted or unsubstituted arylamine group; a substituted or unsubstituted heteroarylamine group; a substituted or unsubstituted aryl group; a substituted or unsubstituted fluorenyl group; a substituted or unsubstituted carbazole group; a substituted or unsubstituted alkoxy group; a substituted or unsubstituted thionyl group; a substituted or unsubstituted silyl group; a substituted or unsubstituted alkenyl group; a substituted or unsubstituted ester group; a substituted or unsubstituted aromatic group; and substituted or unsubstituted heterocyclic groups containing one or more elements selected from the group consisting of N, O, S and Se, or two adjacent substituents may form a condensed ring.

The organic semiconductor compound represented by Chemical Formula 4-1 above may be selected from compounds represented by the following Chemical Formulas 5-1 to 5-8, but is not limited thereto:

[Chemical Formula 5-1]

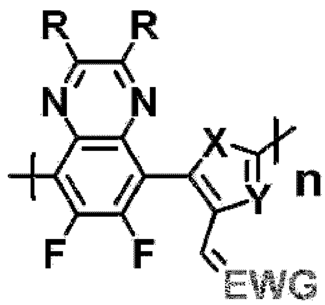

[Chemical Formula 5-2]

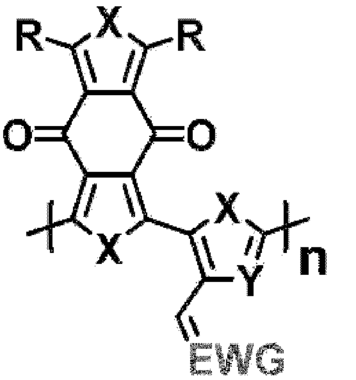

[Chemical Formula 5-3]

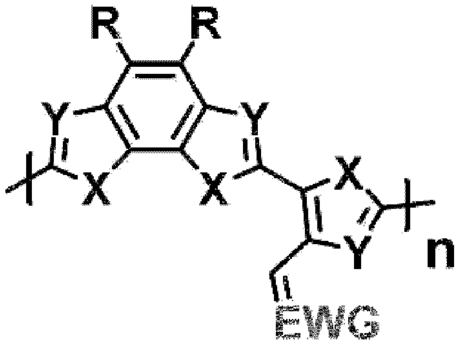

[Chemical Formula 5-4]

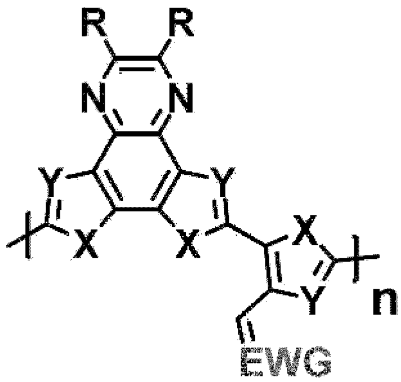

[Chemical Formula 5-5]

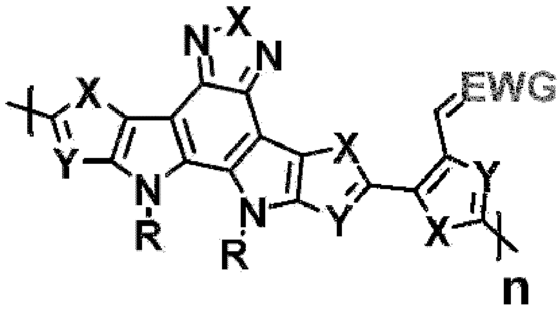

[Chemical Formula 5-6]

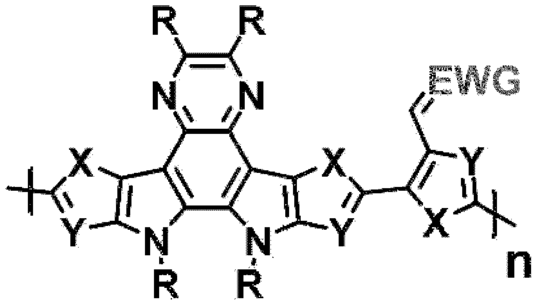

[Chemical Formula 5-7]

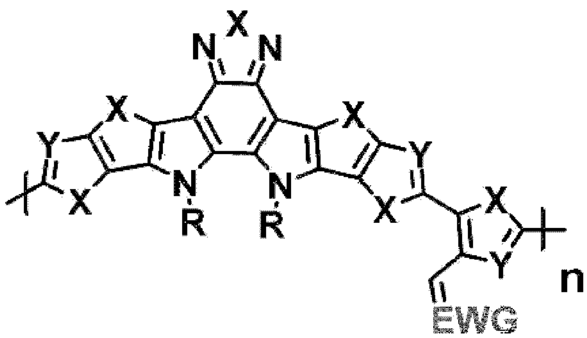

[Chemical Formula 5-8]

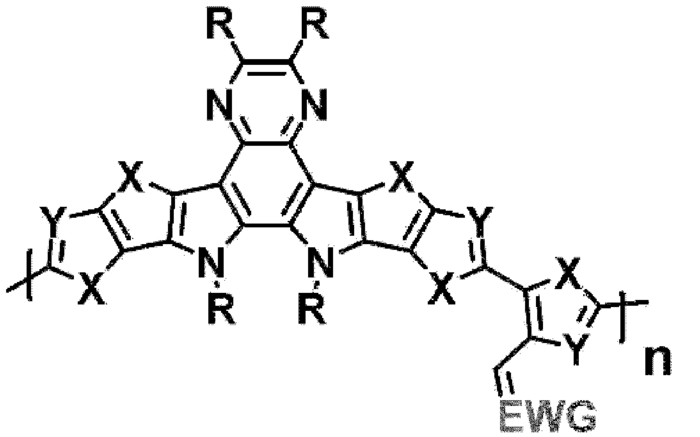

In Chemical Formulas 5-1 to 5-8 above, n is an integer of 1 to 10,000, and R, X, Y, and EWG are as defined above.

The organic semiconductor compound represented by Chemical Formula 4-1 above may include the following compounds, but is not limited thereto:

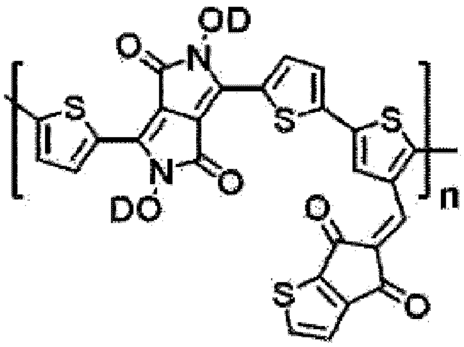

P(DPP-3TC)

The present disclosure also relates to an organic semiconductor compound selected from compounds represented by the following Chemical Formulas 6-1 to 6-5:

[Chemical Formula 6-1]

$$\left[\left[D_1\text{-}A_1\right]_l\left[D_1\text{-}A_2\right]_m\right]_n$$

[Chemical Formula 6-2]

$$\left[\left[D_1\text{-}A_1\right]_l\left[D_2\text{-}A_1\right]_m\right]_n$$

**[00303]** [Chemical Formula 6-3]

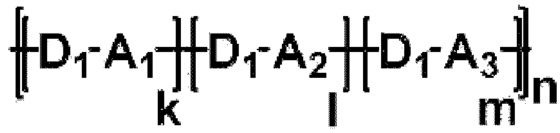

[Chemical Formula 6-4]

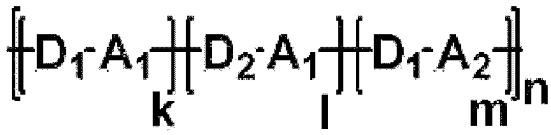

[Chemical Formula 6-5]

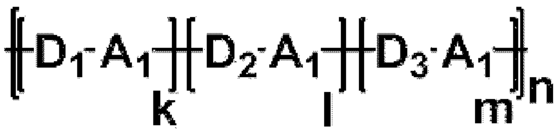

In Chemical Formulas 6-1 to 6-5 above, $D_1$, $D_2$ and $D_3$ are identical to or different from each other and are electron donors selected from compounds represented by the following structures,

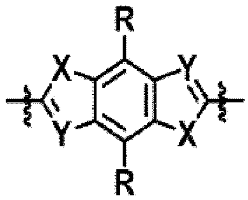

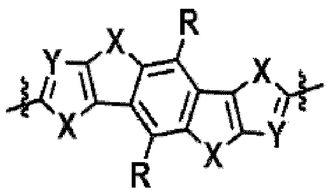

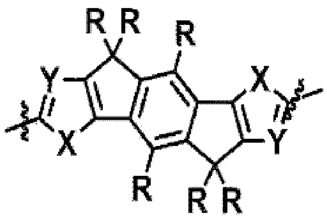

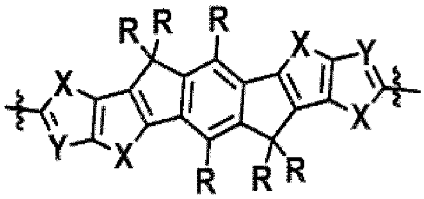

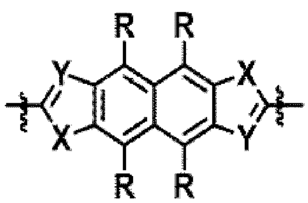

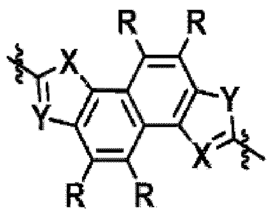

$A_1$, $A_2$, and $A_3$ are identical to or different from each other, and at least one thereof is an electron acceptor unit which is a compound represented by Chemical Formula 1 according to the present disclosure, and the rest are relative electron acceptors selected from compounds represented by the following structures, and

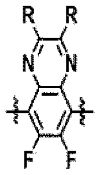 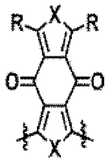 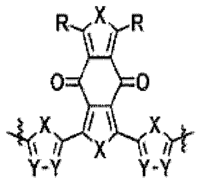 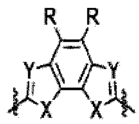 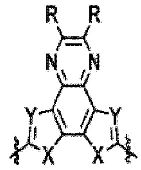

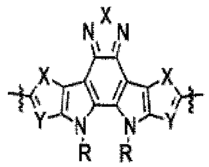 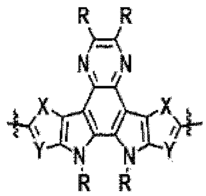 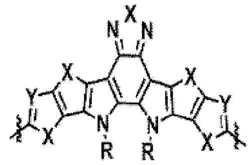 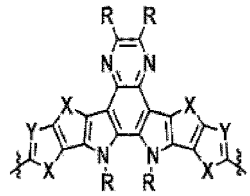

in the structures, X and Y are identical to or different from each other, and each independently chalcogens, wherein the chalcogen is selected from the group consisting of N, N-R, C, O, S, and Se, and R is identical to or different from each other, and each independently selected from the group consisting of hydrogen; a cyano group; a halogen group; a substituted or unsubstituted alkyl group; a substituted or unsubstituted cycloalkyl group; a substituted or unsubstituted aryloxy group; a substituted or unsubstituted arylsulfoxy group; a substituted or unsubstituted boron group; a substituted or unsubstituted alkylamine group; a substituted or unsubstituted aralkylamine group; a substituted or unsubstituted arylamine group; a substituted or unsubstituted heteroarylamine group; a substituted or unsubstituted aryl group; a substituted or unsubstituted fluorenyl group; a substituted or unsubstituted carbazole group; a substituted or unsubstituted alkoxy group; a substituted or unsubstituted thionyl group; a substituted or unsubstituted silyl group; a substituted or unsubstituted alkenyl group; a substituted or unsubstituted ester group; a substituted or unsubstituted aromatic group; and substituted or unsubstituted heterocyclic groups containing one or more elements selected from the group consisting of N, O, S and Se, or two adjacent substituents may form a condensed ring.

k represents a mole fraction, which is a real number with $0 \leq k < 1$, 1 represents a mole fraction, which is a real number with $0 \leq 1 < 1$, m represents a mole fraction, which is a real number with $0 < m \leq 1$, $k + 1 + m = 1$, and n is an integer of 1 to 10,000.

The organic semiconductor compounds represented by Chemical Formulas 6-1 to 6-5 above may be selected from compounds represented by the following Chemical Formulas 7-1 to 7-5, but is not limited thereto:

[Chemical Formula 7-1]

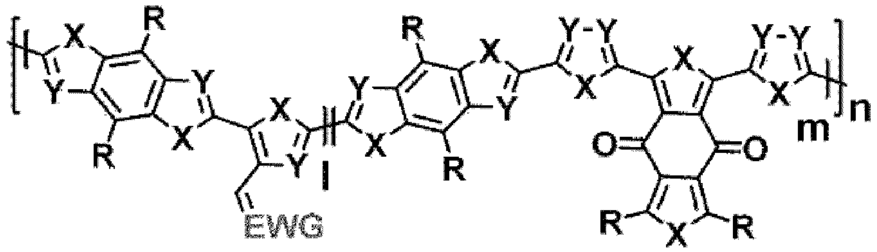

[Chemical Formula 7-2]

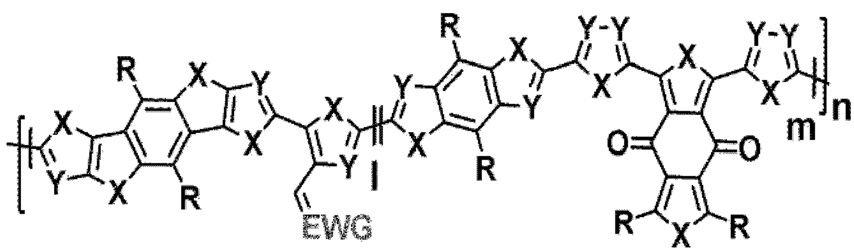

[Chemical Formula 7-3]

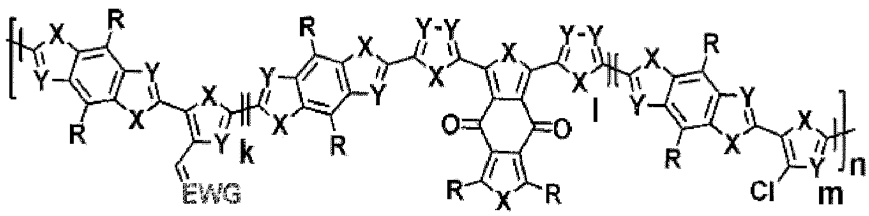

[Chemical Formula 7-4]

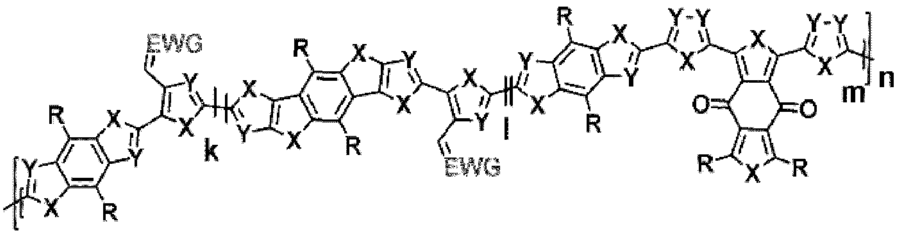

[Chemical Formula 7-5]

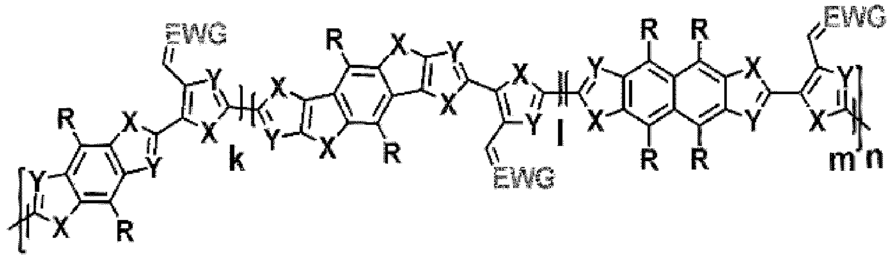

In Chemical Formulas 7-1 to 7-5 above, k, 1, m and n are as defined above, and R, X, Y and EWG are as defined above.

The organic semiconductor compounds represented by Chemical Formulas 6-1 to 6-5 above may be selected from the following compounds, but are not limited thereto:

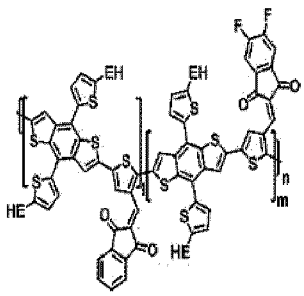

P(3IN)(3IN2F=0.5) (l=0.5, m=0.5)

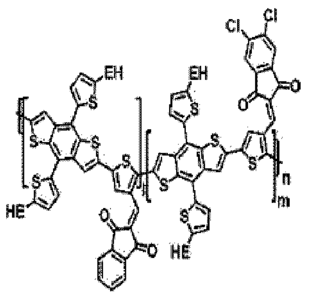

P(3IN)(3IN2Cl=0.5) (l=0.5, m=0.5)

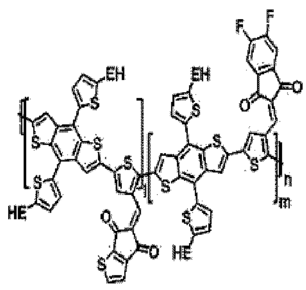

P(3TC)(3IN2F=0.5) (l=0.5, m=0.5)

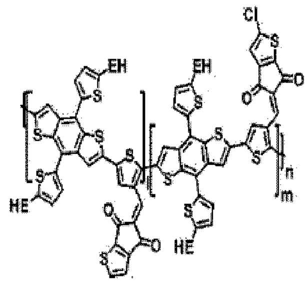

P(3TC)(3TC1Cl=0.5) (l=0.5, m=0.5)

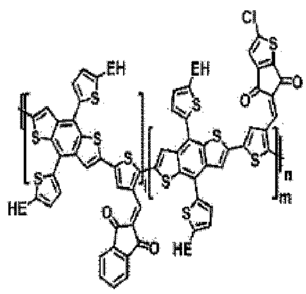

P(3IN)(3TC1Cl=0.4) (l=0.6, m=0.4)

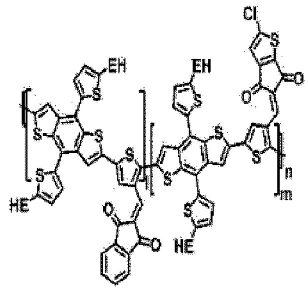

P(3IN)(3TC1Cl=0.2) (l=0.8, m=0.2)

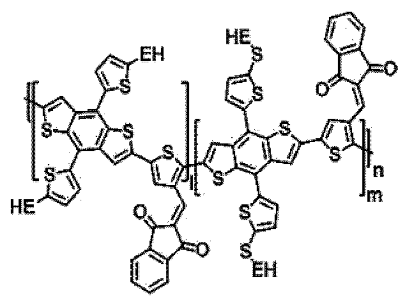

P(3IN)(SEH=0.5) (l=0.5, m=0.5)

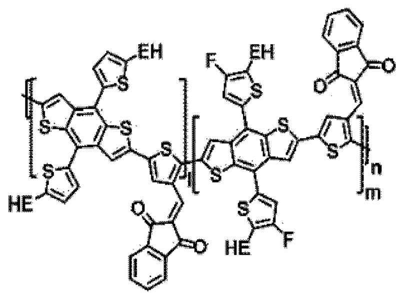

P(3IN)(F-2DBDT=0.5) (l=0.5, m=0.5)

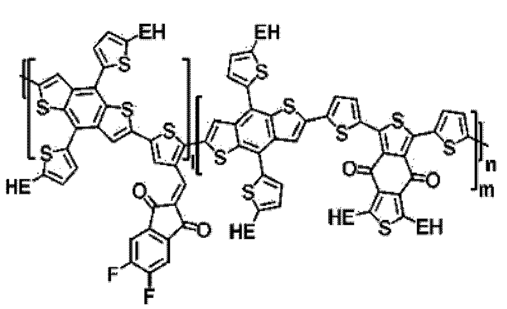

P(3IN2F)(BDD=0.5) (l=0.5, m=0.5)

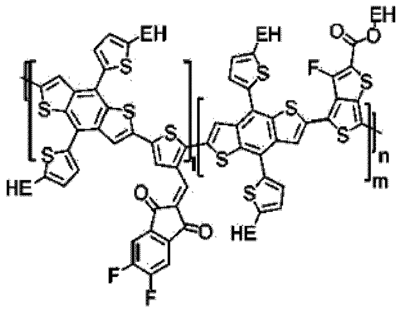

P(3IN2F)(FTT=0.2) (l=0.8, m=0.2)

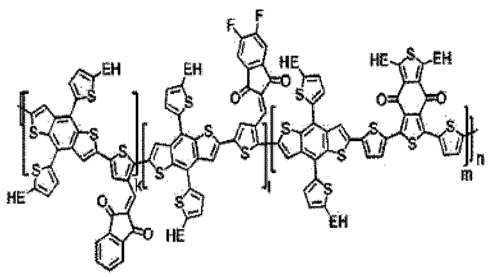

P(3IN=0.3)(3IN2F=0.5)(BDD=0.2) (k=0.3, l=0.5, m=0.2)

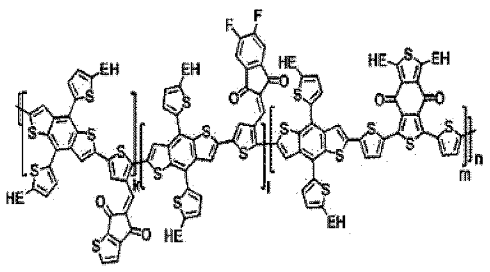

P(3TC=0.3)(3IN2F=0.5)(BDD=0.2) (k=0.3, l=0.5, m=0.2)

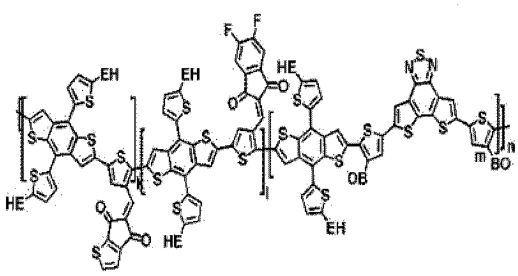

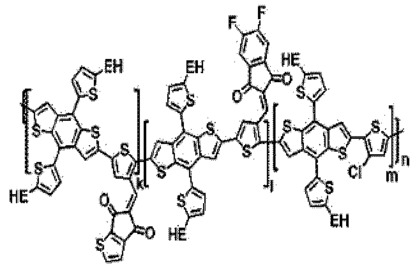

P(3TC=0.3)(3IN2F=0.5)(D18=0.2) (k=0.3, l=0.5, m=0.2) P(3TC=0.3)(3IN2F=0.5)(3ClTh=0.2) (k=0.3, l=0.5, m=0.2)

The present disclosure also provides an organic semiconductor compound selected from compounds represented by the following Chemical Formulas 8-1 to 8-4:

[Chemical Formula 8-1]

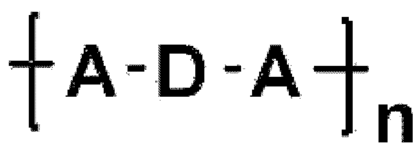

[Chemical Formula 8-2]

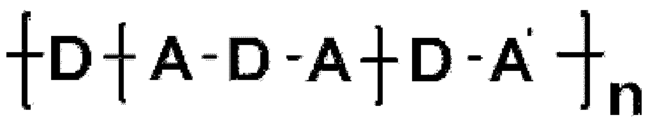

[Chemical Formula 8-3]

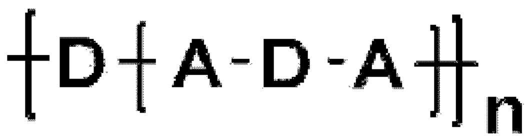

[Chemical Formula 8-4]

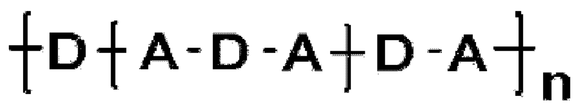

In Chemical Formulas 8-1 to 8-4 above, A is an electron acceptor unit which is a compound represented by Chemical Formula 1 according to the present disclosure, n is an integer of 1 to 10,000, and A' is a relative electron acceptor selected from compounds represented by the following structures,

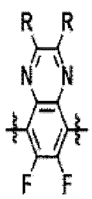

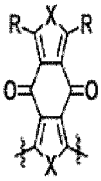

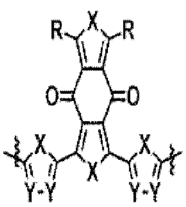

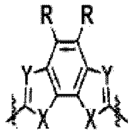

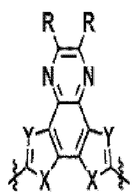

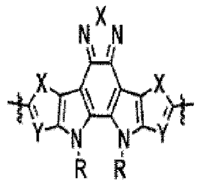 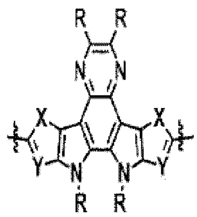 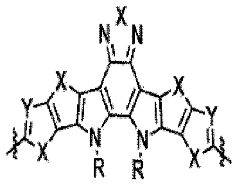 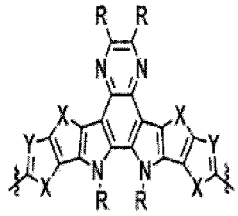

D is an electron donor selected from compounds represented by the following structures, and

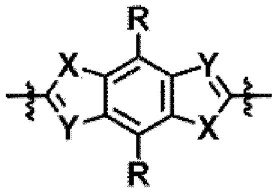 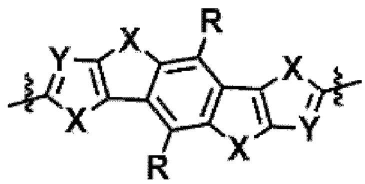

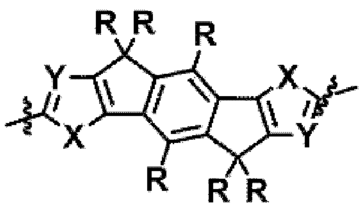 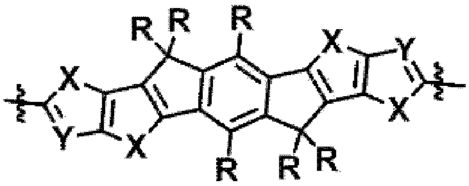

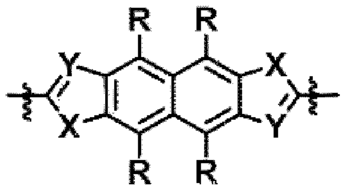 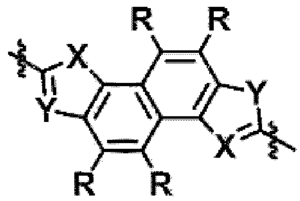

in the structures, X and Y are identical to or different from each other, and each independently chalcogens, wherein the chalcogen is selected from the group consisting of N, N-R, C, O, S, and Se, and R is identical to or different from each other, and each independently selected from the group consisting of hydrogen; a cyano group; a halogen group; a substituted or unsubstituted alkyl group; a substituted or unsubstituted cycloalkyl group; a substituted or unsubstituted aryloxy group; a substituted or unsubstituted arylsulfoxy group; a substituted or unsubstituted boron group; a substituted or unsubstituted alkylamine group; a substituted or unsubstituted aralkylamine group; a substituted or unsubstituted arylamine group; a substituted or unsubstituted heteroarylamine group; a substituted or unsubstituted aryl group; a substituted or unsubstituted fluorenyl group; a substituted or unsubstituted carbazole group; a substituted or unsubstituted alkoxy group; a substituted or unsubstituted thionyl group; a substituted or unsubstituted silyl group; a substituted or unsubstituted alkenyl group; a substituted or unsubstituted ester group; a substituted or unsubstituted aromatic group; and substituted or unsubstituted heterocyclic groups containing one or more elements selected from the group consisting of N, O, S and Se, or two adjacent substituents may form a condensed ring.

The compound represented by Chemical Formula 8-1 above may be selected from the following compounds, but is not limited thereto:

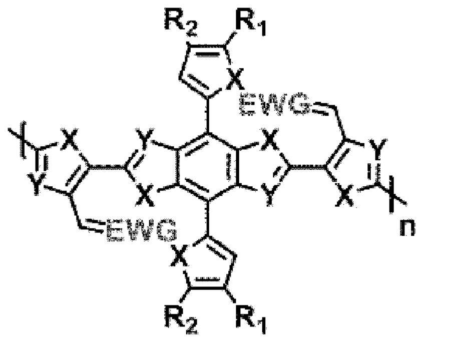

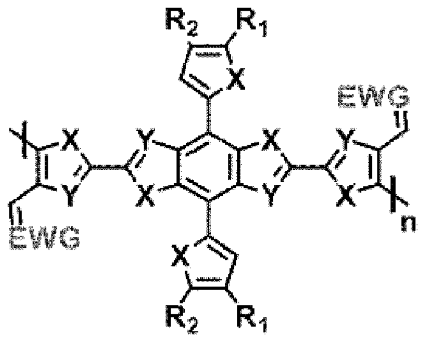

The compound represented by Chemical Formula 8-2 above may be selected from the following compounds, but is not limited thereto:

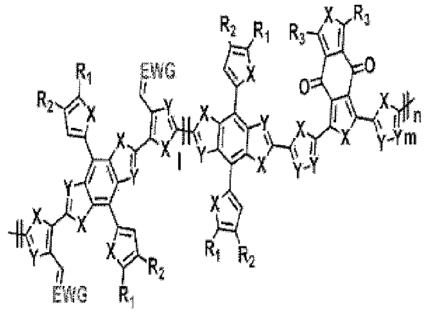

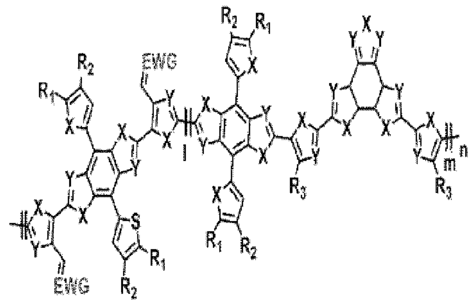

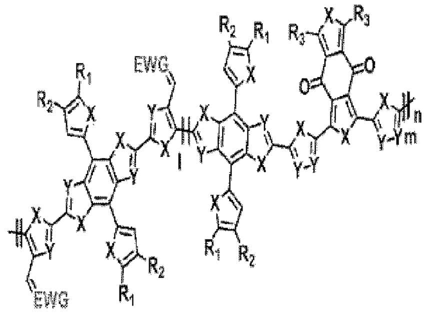

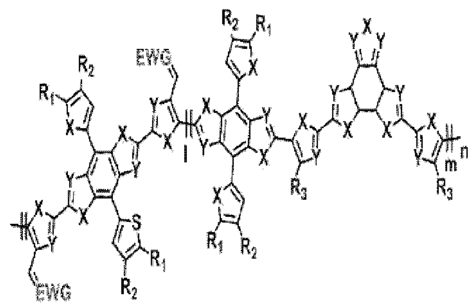

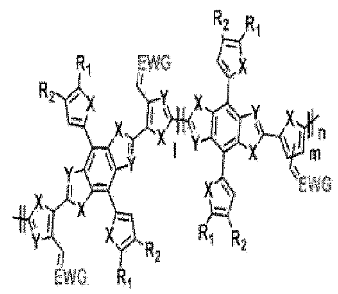

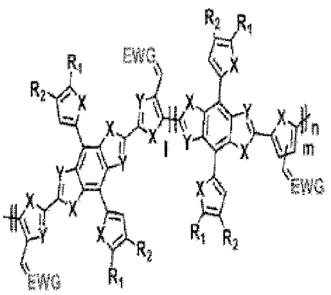

In the structures, 1, m and n are as defined above, and X, Y, $R_1$ to $R_3$ and EWG are as defined above.

The organic semiconductor compounds represented by Chemical Formulas 8-1 to 8-4 above may be selected from the following compounds, but are not limited thereto:

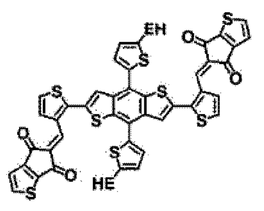

RR-2DBDT-3TC

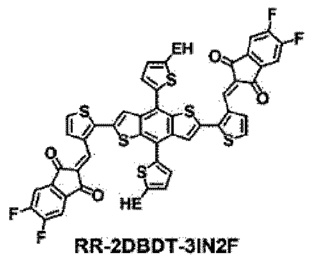

RR-2DBDT-3IN2F

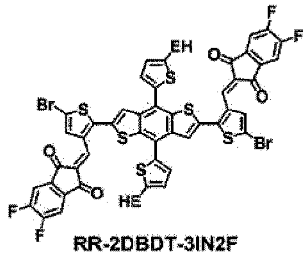

RR-2DBDT-3IN2F

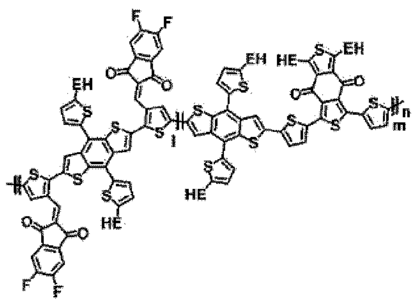

RR-P(3IN2F)(BDD=0.5)

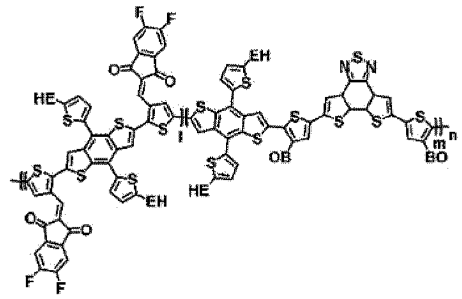

RR-P(3IN2F)(D18=0.5)

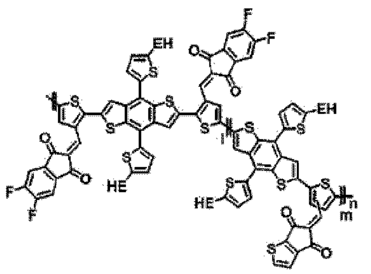

P(RR-3IN2F)(3TC=0.5)

In addition, the present disclosure provides an organic solar cell and an organic electronic device including an organic semiconductor compound according to the present disclosure.

The organic solar cell may include a lower substrate through which electricity may flow; a hole transport layer laminated on the lower substrate and including poly(3,4-ethylenedioxythiophene) polystyrene sulfonate (PEDOT:PSS); a photoactive layer laminated on the hole transport layer and including an electron donor and an electron acceptor, wherein the electron donor is the novel unit-based organic semiconductor compound according to the present disclosure; an organic electron transport layer laminated on the photoactive layer; and an electrode layer laminated on the organic electron transport layer.

The organic solar cell may include a lower substrate through which electricity may flow; an inorganic electron transport layer laminated on the lower substrate; a photoactive layer laminated on the inorganic electron transport layer and including an electron donor and an electron acceptor, wherein the electron donor is the novel unit-based organic semiconductor compound according to the present disclosure; a hole transport layer laminated on the photoactive layer and including a metal oxide; and an electrode layer laminated on the hole transport layer.

The lower substrate is an ITO substrate, the organic electron transport layer includes 2,9-Bis[3-[[3-(dimethylamino)propyl]amino]propyl]-anthra[2,1,9-def:6,5,10-d'e'f']diisoquinoline-1,3,8,10(2H,9H)-tetrone (PDINN), Poly[[2,7-bis(2-ethylhexyl)-1,2,3,6,7,8-hexahydro-1,3,6,8-tetraoxobenzo[lmn][3,8]phenanthroline-4,9-diyl]-2,5-thiophenediyl[9,9-bis[3-(dimethylamino)propyl]-9H-fluorene-2,7-diyl]-2,5-thiophenediyl] Other Names: PF3N-2TNDI(=PNDIT-F3N), etc., and the electrode layer may include at least one selected from the group consisting of silver (Ag) and aluminum (Al).

The lower substrate may be an ITO substrate, the inorganic electron transport layer may include zinc oxide (ZnO), the metal oxide may include molybdenum oxide ($MoO_3$), and the electrode layer may include at least one selected from the group consisting of silver (Ag) and aluminum (Al).

**[Modes]**

Hereinafter, preferred Examples, Experimental Examples, and Preparation Examples will be presented in order

to assist the understanding of the present disclosure. However, the following Examples, Experimental Examples, and Preparation Examples are just provided to more easily understand the present disclosure and the contents of the present disclosure are not limited by these Examples.

**<Example 1> Synthesis of 4H-chloropenta[b]thiophene-4,6(5H)-dione (1) [see FIG. 1]**

A 2-neck round bottom flask and a condenser were prepared. Vacuum and nitrogen were replaced. 11.37 g (85.27 mmol) of aluminum chloride, 100 ml of 1,2-dichloroethane, and 5.0 g (34.11 mmol) of 2-thiophenecarbonyl chloride were added in sequence, and then added dropwise with 10.57 g (75.04 mmol) of malonyl chloride in an ice bath, and stirred vigorously. A reactant was stirred for 12 hours while rising to 80°C. The reactant was poured into cold water and stirred with chloroform for 12 hours. The extracted organic layer was extracted with diluted hydrochloric acid solution (10%, 150 ml) and water (200 ml), and after removing moisture, column purification (chloroform:ethyl acetate = 9:1) was performed. Yellow powder was obtained. (Yield = 34.0%) $^1$H NMR (500 MHz, $CDCl_3$): δ (ppm) = 8.0 (d, 1H), 7.39 (d, 1H), 3.48 (s, 2H) [see FIG. 2] GC-MS (EI) m/z: Calcd. for $C_7H_4O_2S$ : 152.17; Found 152.19 [see FIG. 3].

**<Example 2> Synthesis of 2-chloro-4H-chloropenta[b]thiophene-4,6(5H)-dione (2) [see FIG. 1]**

Under nitrogen replacement, a 100 mL 2-neck round bottom flask was added with 14.7 g (110 mmol) of aluminum chloride and 12.1 ml (74.5 mmol) of malonyl chloride, and then added with 50 ml of dichloromethane. At this time, the temperature was adjusted to 0°C. 3.0 g (16.5 mmol) of 5-chlorothiophene-2-carbonyl chloride and 40 ml of dichloromethane were added and stirred at 39°C for 8 hours. After cooling to room temperature, the mixture was added with water until no gas came out, and then quenched with an aqueous oxalic acid solution. Thereafter, the pH was adjusted to 7 with a sodium bicarbonate aqueous solution. The mixture was extracted with dichloromethane and water. At this time, sodium chloride was used to remove salt. After removing moisture, column purification (hexane:dichloromethane = 4:6) was performed. Yellow powder was obtained. (Yield = 28.0%) $^1$H NMR (500 MHz, $CDCl_3$): δ (ppm) = 7.28 (s, 1H), 3.36 (s, 2H) [see FIG. 4] GC-MS (EI) m/z: Calcd. for $C_7H_3ClO_2S$ : 186.61; Found 186.06 [see FIG. 5].

**<Example 3> Synthesis of 1H-cyclopenta[b]naphthalene-1,3(2H)-dione (3) [see FIG. 1]**

A flame-dried 50 mL 2-neck round bottom flask equipped with a stir bar and a reflux condenser was added with 1 (2.5 g, 5.2 mmol), EtOAc (6.8 mL), and NaH (60% dispersion in mineral oil, 369 mg, 22.7 mmol), and then filled with nitrogen. The mixture was heated under reflux for 3 hours. The suspension was cooled to room temperature and diluted with hexane (5 mL). The mixture was filtered through a Buchner funnel and washed with an additional amount of hexane (5 mL) to obtain light yellow powder. The yellow powder was resuspended in a concentrated HCl aqueous solution (1 M, 25 mL) and heated at 80°C for 1 hour. The suspension was filtered through a Buchner funnel and washed with water to obtain ivory powder. (Yield = 33.0%) $^1$H NMR (500 MHz, $CDCl_3$): δ (ppm) = 8.52 (s, 2H), 8.14-8.13 (d, 2H), 7.76-7.73 (d, 2H), 3.39 (s, 2H) [see FIG. 6] GC-MS (EI) m/z: Calcd. for $C_{13}H_8O_2$ : 196.21; Found 196.18 [see FIG. 7].

**<Example 4> Synthesis of 2,5-dibromo-3-formylthiophene (4) [see FIG. 1]**

At 0°C, a solution mixed with 14.96 g (93.62 mmol) of bromine and 14.65 ml (48 %) of an aqueous hydrogen bromide solution was added dropwise to a solution mixed with 5.0 g (44.58 mmol) of thiophene-3-carboxyaldehyde, 19.53 ml (48%) of a hydrogen bromide aqueous solution, and 19.53 ml of diethyl ether. The mixture was all added and then heated at 50°C overnight. The mixture was added with 50 ml of a sodium bisulfite aqueous solution and quenched. The organic layer was extracted with ethyl acetate. The extracted product was dried with magnesium sulfate. The product was obtained by column (dichloromethane: hexane = 1:1). Ivory powder was obtained. (Yield = 90.0%) $^1$H NMR (500 MHz, $CDCl_3$): δ (ppm) = 9.75 (s, 1H), 7.21 (s, 2H) [see FIG. 8] GC-MS (EI) m/z: Calcd. for $C_5H_2Br_2OS$ : 269.94; Found 269.0 [see FIG. 9].

**<Example 5> Synthesis of 5-((2,5-dibromothiophene-3-yl)methylene)-1,3-diethyl-2-thioxodihydropyridine-4,6(1H,5H)-dione (5) [see FIG. 1]**

0.54 g (2.0 mmol) of 2,5-dibromothiophene-3-carbaldehyde and 0.38 g (1.9 mmol) of 1,3-diethyl-2-thioxodihydropyridine-4,6(1H,5H)-dione were added to 20.0 ml of HPLC ethanol and stirred vigorously at 78°C for 10 hours. The mixture was washed with ethanol and filtered. Orange powder was obtained. (Yield = 83.0%) $^1$H NMR (500 MHz, $CDCl_3$): δ (ppm) = 8.42 (s, 1H), 8.28 (s, 1H), 4.58-4.52 (m, 4H), 1.33-1.29 (m, 6H) [see FIG. 10] GC-MS (EI) m/z: Calcd. for $C_{13}H_{12}Br_2N_2O_2S_2$ : 452.18; Found 371.97 (detected with one Br removed during measurement) [see FIG. 11].

**<Example 6> Synthesis of 2-((2,5-dibromothiophene-3-yl)methylene)-1H-indene-1,3(2H)-dione (6) [see FIG. 1]**

1.59 g (5.89 mmol) of 2,5-dibromothiophene-3-carbaldehyde, 1.00 g (6.84 mmol) of 1H-indene-1,3(2H)-dione, and 79.5 ml of HPLC ethanol were added, vacuumed for 30 minutes, added dropwise with 0.2 ml of piperidine, vacuumed for 30 minutes, and then replaced with nitrogen. The mixture was refluxed for 24 hours. Column purification (hexane:chloroform = 1:1) was performed. Yellow solid was obtained. (Yield = 92.9%) $^1$H NMR (500 MHz, $CDCl_3$): δ (ppm) = 8.76 (s, 1H), 8.02-8.0 (m, 2H), 7.85-7.83 (m, 2H), 7.80 (s, 1H) [see FIG. 12] $^{13}$C NMR (100 MHz, $CDCl_3$): δ (ppm) = 189.79, 189.11, 142.61, 140.39, 135.84, 135.64, 133.55, 133.87, 133.25, 127.87, 126.90, 123.50, 123.46, 111.89 [see FIG. 13] GC-MS (EI) m/z: Calcd. for $C_{14}H_6Br_2O_2S$ : 398.07; Found 319.02 (detected with one Br removed during measurement) [see FIG. 14] MS (MALDI-TOF) m/z: Calcd. for $C_{14}H_6Br_2O_2S$ : 398.07; Found 319.71(detected with one Br removed during measurement) [see FIG. 15].

**<Example 7> Synthesis of (Z)-5-((2,5-dibromothiophene-3-yl)methylene)-4H-cyclopenta[b]thiophene-4,6(5H)-dione (7) [see FIG. 1]**

1.61 g (5.97 mmol) of 2,5-dibromothiophene-3-carbaldehyde, 1.00 g (6.57 mmol) of 4H-chloropenta[b]thiophene-4,6(5H)-dione, and 80.5 ml of HPLC ethanol were added, vacuumed for 30 minutes, added dropwise with 0.2 ml of piperidine, vacuumed for 30 minutes, and then replaced with nitrogen. The mixture was refluxed for 24 hours. Column purification (hexane:chloroform = 1:1) was performed. Yellow solid was obtained. (Yield = 96.0%) $^1$H NMR (500 MHz, $CDCl_3$): δ (ppm) = 8.58-8.57 (m, 1H), 7.96-7.94 (m, 1H), 7.62-7.60 (m, 1H), 7.47-7.46 (m, 1H) [see FIG. 16] $^{13}$C NMR (100 MHz, $CDCl_3$): δ (ppm) = 189.77, 189.06, 142.46, 140.25, 135.70, 135.57, 135.49, 133.81, 133.12, 127.68, 126.90, 123.40, 123.36, 111.81 [see FIG. 17] GC-MS (EI) m/z: Calcd. for $C_{12}H_6Br_2O_2S_2$ : 404.09; Found 324.97(detected with one Br removed during measurement) [see FIG. 18] MS (MALDI-TOF) m/z: Calcd. for $C_{12}H_6Br_2O_2S_2$ : 404.09; Found 324.92 (detected with one Br removed during measurement) [see FIG. 19].

**<Example 8> Synthesis of (Z)-2-chloro-5-((2,5-dibromothiophene-3-yl)methylene)-4H-cyclopenta[b]thiophene-4,6(5H)-dione (8) [see FIG. 1]**

0.39 g (1.46 mmol) of 2,5-dibromothiophene-3-carbaldehyde, 0.30 g (1.61 mmol) of 2-chloro-4H-chloropenta[b]thiophene-4,6(5H)-dione, and 14.6 ml of HPLC ethanol were added, vacuumed for 30 minutes, added dropwise with 0.06 ml of piperidine, vacuumed for 30 minutes, and then replaced with nitrogen. The mixture was refluxed for 24 hours. Column purification (hexane:chloroform = 1:1) was performed. Light yellow solid was obtained. (Yield = 82.9%) $^1$H NMR (500 MHz, $CDCl_3$): δ (ppm) = 8.55 (s, 1H), 7.61 (s, 1H), 7.34 (s, 1H) [see FIG. 20] $^{13}$C NMR (100 MHz, $CDCl_3$): δ (ppm) = 182.68, 182.07, 181.28, 180.77, 155.62, 154.10, 153.34, 150.97, 146.78, 146.46, 135.14, 133.08, 131.73, 129.40, 129.23, 125.82, 120.78, 120.72, 111.90 [see FIG. 21] GC-MS (EI) m/z: Calcd. for $C_{12}H_3Br_2ClO_2S_2$ : 438.53; Found 358.86(detected with one Br removed during measurement) [see FIG. 22] MS (MALDI-TOF) m/z: Calcd. for $C_{12}H_3Br_2ClO_2S_2$ : 438.53; Found 358.46 (detected with one Br removed during measurement) [see FIG. 23].

**<Example 9> Synthesis of 2-((2,5-dibromothiophene-3-yl)methylene)-5,6-difluoro-1H-indene-1,3(2H)-dione (9) [see FIG. 1]**

1.62 g (6.0 mmol) of 2,5-dibromothiophene-3-carbaldehyde, 1.20 g (6.6 mmol) of 5,6-difluoro-1H-indene-1,3(2H)-dione, and 60.0 ml of HPLC ethanol were added, vacuumed for 30 minutes, added dropwise with 0.2 ml of piperidine, vacuumed for 30 minutes, and then replaced with nitrogen. The mixture was refluxed for 24 hours. Column purification (hexane:dichloromethane = 1:1) was performed. (Yield = 73.0%) $^1$H NMR (500 MHz, $CDCl_3$): δ (ppm) = 8.67 (s, 1H), 7.81-7.77 (m, 2H), 7.75 (s, 1H) [see FIG. 24] $^{13}$C NMR (100 MHz, $CDCl_3$): δ (ppm) = 187.74, 186.61, 156.59, 156.47, 154.48, 139.71, 135.42, 134.36, 132.88, 127.84, 126.50, 112.47, 112.40, 112.32, 112.25, 112.06 [see FIG. 25] GC-MS (EI) m/z: Calcd. for $C_{14}H_4Br_2F_2O_2S$ : 434.05; Found 354.96(detected with one Br removed during measurement) [see FIG. 26] MS (MALDI-TOF) m/z: Calcd. for $C_{14}H_4Br_2F_2O_2S$ : 438.53; Found 354.85 (detected with one Br removed during measurement) [see FIG. 27].

**<Example 10> Synthesis of 2-((2,5-dibromothiophene-3-yl)methylene)-5,6-dichloro-1H-indene-1,3(2H)-dione (10) [see FIG. 1]**

1.62 g (6.0 mmol) of 2,5-dibromothiophene-3-carbaldehyde, 1.42 g (6.6 mmol) of 5,6-difluoro-1H-indene-1,3(2H)-dione, and 60.0 ml of HPLC ethanol were added, vacuumed for 30 minutes, added dropwise with 0.2 ml of piperidine, vacuumed for 30 minutes, and then replaced with nitrogen. The mixture was refluxed for 24 hours. Column purification (hexane:dichloromethane = 1:1) was performed. (Yield = 86.1%) $^1$H NMR (500 MHz, $CDCl_3$): δ (ppm) = 8.70 (s,

1H), 8.09-8.06 (ss, 2H), 7.81 (1H) [see FIG. 28].

**<Example 11> Synthesis of 2-((2,5-dibromothiophene-3-yl)methylene)-1H-cyclopenta[b]naphthalene-1,3(2H)-dione (11) [see FIG. 1]**

0.54 g (2.0 mmol) of 2,5-dibromothiophene-3-carbaldehyde, 0.43 g (2.2 mmol) of 1H-cyclopenta[b]naphthalene-1,3(2H)-dione, and 20.0 ml of HPLC ethanol were added, vacuumed for 30 minutes, added dropwise with 0.1 ml of piperidine, vacuumed for 30 minutes, and then replaced with nitrogen. The mixture was refluxed for 24 hours. Column purification (hexane:dichloromethane = 1:1) was performed. (Yield = 56.8%) $^1$H NMR (500 MHz, $CDCl_3$): δ (ppm) = 8.86 (s, 1H), 8.55-8.52 (ss, 2H), 8.13-8.12 (m, 2H), 7.88 (s, 1H), 7.74-7.72 (m, 2H) [see FIG. 29].

**<Example 12> Synthesis of 2,5-dibromofuran-3-carbaldehyde (12) [see** FIG. 1]

At 0°C, 11.12 g (62.5 mmol) of N-bromosuccimide was added in a small portion to a solution mixed with 3.0 g (31.0 mmol) of furan-3-carbaldehyde and 60.0 ml of anhydrous chloroform. The mixture was all added and then heated at 50°C overnight. The mixture was added with 50 ml of a sodium bisulfite aqueous solution and quenched. The organic layer was extracted with ethyl acetate. The extracted product was dried with magnesium sulfate. The product was obtained by a column (dichloromethane: hexane = 2:8). Ivory powder was obtained. (Yield = 16.0%) $^1$H NMR (500 MHz, $CDCl_3$): δ (ppm) = 9.80 (s, 1H), 6.74 (s, 2H) [see FIG. 30] GC-MS (EI) m/z: Calcd. for $C_5H_2Br_2O_2$ : 253.88; Found 253.96 [see FIG. 31].

**<Example 13> Synthesis of 2-((2,5-dibromofuran-3-yl)methylene)-1H-indene-1,3(2H)-dione (13) [see FIG. 1]**

0.51 g (2.0 mmol) of 2,5-dibromothiophene-3-carbaldehyde, 0.32 g (2.2 mmol) of 1H-indene-1,3(2H)-dione, and 20.0 ml of HPLC ethanol were added, vacuumed for 30 minutes, added dropwise with 0.1 ml of piperidine, vacuumed for 30 minutes, and then replaced with nitrogen. The mixture was refluxed for 24 hours. Column purification (hexane:dichloromethane = 1:1) was performed. (Yield = 66.8%) $^1$H NMR (500 MHz, $CDCl_3$): δ (ppm) = 8.04 (s, 1H), 8.03-7.99 (m, 2H), 7.84-7.83 (m, 2H), 7.55 (s, 1H) [see FIG. 32].

**<Example 14> Synthesis of 2-((2,5-dibromothiophene-3-yl)methylene)-1H-cyclopenta[b]naphthalene-1,3(2H)-dione (14) [see FIG. 1]**

0.51 g (2.0 mmol) of 2,5-dibromofuran-3-carbaldehyde, 0.33 g (2.2 mmol) of 4H-chloropenta[b]thiophene-4,6(5H)-dione, and 20.0 ml of HPLC ethanol were added, vacuumed for 30 minutes, added dropwise with 0.1 ml of piperidine, vacuumed for 30 minutes, and then replaced with nitrogen. The mixture was refluxed for 24 hours. Column purification (hexane:dichloromethane = 1:1) was performed. (Yield = 59.9%) $^1$H NMR (500 MHz, $CDCl_3$): δ (ppm) = 7.97-7.89 (m, 2H), 7.47-7.45 (s, 1H), 7.36-7.34 (m, 1H) [see FIG. 33].

**<Example 15> Synthesis of 2-((2,5-dibromothiophene-3-yl)methylene)-1H-cyclopenta[b]naphthalene-1,3(2H)-dione (15) [see FIG. 1]**

0.38 g (2.0 mmol) of 2-bromothiophene-3-carbaldehyde, 0.33 g (2.2 mmol) of 4H-chloropenta[b]thiophene-4,6(5H)-dione, and 20.0 ml of HPLC ethanol were added, vacuumed for 30 minutes, added dropwise with 0.1 ml of piperidine, vacuumed for 30 minutes, and then replaced with nitrogen. The mixture was refluxed for 24 hours. Column purification (hexane:dichloromethane = 1:1) was performed. (Yield = 87.8%) $^1$H NMR (500 MHz, $CDCl_3$): δ (ppm) = 8.52-8.50 (d, 1H), 7.95-7.92 (d, 1H), 7.75-7.73 (d, 1H), 7.47-7.46 (s, 1H), 7.35-7.34 (d, 1H) [see FIG. 34].

**<Example 16> Synthesis of 5-bromothiophene-3-carbaldehyde (16) [see FIG. 1]**

5.56 g (31.25 mmol) of N-bromosuccimide was added in a small portion to a solution mixed with 3.5 g (31.0 mmol) of thiophene-3-carbaldehyde and 60.0 ml of anhydrous dimethylformamide. The mixture was all added and then left at room temperature overnight. The mixture was added with 50 ml of a sodium bisulfite aqueous solution and quenched. The organic layer was extracted with ethyl acetate. The extracted product was dried with magnesium sulfate. The product was obtained by a column (dichloromethane: hexane = 2:8). Light yellow oil was obtained. (Yield = 77.5%) $^1$H NMR (500 MHz, $CDCl_3$): δ (ppm) = 9.78 (s, 1H), 8.01 (s, 1H), 7.50 (s, 1H) [see FIG. 35].

**<Example 17> Synthesis of 5-bromo-2-iodothiophene-3-carbaldehyde (17) [see FIG. 1]**

At 0°C, 3.47 g (10.77 mmol) of PhI(OAc) and 2.53 g (9.97 mmol) of iodine were added in small portions to a

solution mixed with 3.81 g (19.94 mmol) of 5-bromothiophene-3-carbaldehyde and 44.0 ml of anhydrous dimethyl chloride. The mixture was all added and then stirred at room temperature for 3 hours while kept in the dark. The mixture was added with 50 ml of a sodium thiosulfate aqueous solution and quenched. The organic layer was extracted with ethyl acetate. The extracted product was dried with magnesium sulfate. The product was obtained by a column (dichloromethane:hexane = 4:6). Light yellow solid was obtained. (Yield = 80.3%) $^1H$ NMR (500 MHz, $CDCl_3$): δ (ppm) = 9.54 (s, 1H), 7.26 (s, 1H) [see FIG. 36].

**<Example 18> Synthesis of 2-((5-bromo-2-iodothiophene-3-yl)methylene)-5,6-difluoro-1H-indene-1,3(2H)-dione (18) [see FIG. 1]**

0.38 g (2.0 mmol) of 5-bromo-2-iodothiophene-3-carbaldehyde, 0.4 g (2.2 mmol) of 5,6-difluoro-1H-indene-1,3(2H)-dione, and 20.0 ml of HPLC ethanol were added, vacuumed for 30 minutes, added dropwise with 0.1 ml of piperidine, vacuumed for 30 minutes, and then replaced with nitrogen. The mixture was refluxed for 24 hours. Column purification (hexane:dichloromethane = 1:1) was performed. (Yield = 77.8%) $^1H$ NMR (500 MHz, $CDCl_3$): δ (ppm) = 8.65 (s, 1H), 7.82-7.76 (m, 2H), 7.62 (s, 1H) [see FIG. 37].

**<Example 19> Synthesis of D-A structural polymer P(3CHO) [see FIG. 38]**

In 10 to 20 mL of a microwave auxiliary vial, 2,5-dibromo-3-formylthiophene (4) (54.0 mg, 0.20 mmol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 24 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (24 hours) to remove all solvents, and then reprecipitated in methanol to obtain 120.5 mg (87.4% yield) of a red material.

$^1H$ NMR (500 MHz, $CDCl_3$): δ (ppm) [see FIG. 39].

**<Example 20> Synthesis of D-A structural polymer P(3BA) [see FIG. 38]**

In 10 to 20 mL of a microwave auxiliary vial, 5-((2,5-dibromothiophene-3-yl)methylene)-1,3-diethyl-2-thioxodihydropyrimidine-4,6(1H,5H)-dione (5) (90.4 mg, 0.20 mmol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 24 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), and hexane (24 hours) to remove oligomers and residual catalyst. Finally, the product was dissolved in chloroform (24 hours) to remove all solvents, and then reprecipitated in methanol to obtain 118.1 mg (67.8% yield) of a brown material.

$^1H$ NMR (500 MHz, $CDCl_3$): δ (ppm) [see FIG. 40].

**<Example 21> Synthesis of D-A structural polymer P(3IN) [see FIG. 38]**

In 10 to 20 mL of a microwave auxiliary vial, 2-((2,5-dibromothiophene-3-yl)methylene)-1H-indene-1,3(2H)-dione (6) (79.6 mg, 0.20 mmol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 24 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (24 hours) to remove all solvents, and then reprecipitated in methanol to obtain 151.0 mg (92.4% yield) of a black-green material.

$^1H$ NMR (500 MHz, $CDCl_3$): δ (ppm) [see FIG. 41].

**<Example 22> Synthesis of D-A structural polymer P(3TC) [see FIG. 38]**

In 10 to 20 mL of a microwave auxiliary vial, (Z)-5-((2,5-dibromothiophene-3-yl)methylene)-4H-cyclopenta[b]thiophene-4,6(5H)-dione (7) (80.8 mg, 0.20 mmol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 24 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (24 hours) to remove all solvents, and then reprecipitated in methanol to obtain 147.9 mg (89.8% yield) of a black-brown material.

$^1H$ NMR (500 MHz, $CDCl_3$): $\delta$ (ppm) [see FIG. 42].

**<Example 23> Synthesis of D-A structural polymer P(3TC1Cl) [see FIG. 38]**

In 10 to 20 mL of a microwave auxiliary vial, (Z)-2-chloro-5-((2,5-dibromothiophene-3-yl)methylene)-4H-cyclopenta[b]thiophene-4,6(5H)-dione (8) (87.7 mg, 0.20 mmol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 12 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (24 hours) to remove all solvents, and then reprecipitated in methanol to obtain 112.5 mg (65.6% yield) of a black-brown material.

$^1H$ NMR (500 MHz, $CDCl_3$): $\delta$ (ppm) [see FIG. 43].

**<Example 24> Synthesis of D-A structural polymer P(3IN2F) [see FIG. 38]**

In 10 to 20 mL of a microwave auxiliary vial, 2-((2,5-dibromothiophene-3-yl)methylene)-5,6-difluoro-1H-indene-1,3(2H)-dione (9) (86.8 mg, 0.20 mmol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 18 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (24 hours) to remove all solvents, and then reprecipitated in methanol to obtain 138.8 mg (81.3% yield) of a black-green material.

$^1H$ NMR (500 MHz, $CDCl_3$): $\delta$ (ppm) [see FIG. 44].

**<Example 25> Synthesis of D-A structural polymer P(3IN2Cl) [see FIG. 38]**

In 10 to 20 mL of a microwave auxiliary vial, 2-((2,5-dibromothiophene-3-yl)methylene)-5,6-dichloro-1H-indene-1,3(2H)-dione (10) (93.4 mg, 0.20 mmol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 12 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (24 hours) to remove all solvents, and then reprecipitated in methanol to obtain 136.3 mg (76.9% yield) of a black-green material.

$^1H$ NMR (500 MHz, $CDCl_3$): $\delta$ (ppm) [none; insoluble after reprecipitation].

**<Example 26> Synthesis of D-A structural polymer P(3NT) [see FIG. 38]**

In 10 to 20 mL of a microwave auxiliary vial, 2-((2,5-dibromothiophene-3-yl)methylene)-1H-cyclopenta[b]naphthalene-1,3(2H)-dione (11) (89.6 mg, 0.20 mmol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 12 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (24 hours) to remove all solvents, and then reprecipitated in methanol to obtain 97.3 mg (56.1% yield) of a black-green material.

$^1$H NMR (500 MHz, $CDCl_3$): $\delta$ (ppm) [none; insoluble after reprecipitation].

**<Example 27> Synthesis of D-A structural polymer P(Fu3IN) [see FIG. 38]**

In 10 to 20 mL of a microwave auxiliary vial, 2-((2,5-dibromofuran-3-yl)methylene)-1H-indene-1,3(2H)-dione (13) (57.3 mg, 0.15 mmol), 2DBDT (135.6 mg, 0.15 mmol), and $Pd(PPh_3)_4$ (5.3 mg) were added and capped. After vacuuming for 30 minutes or more, $N_2$ was changed and blown strongly for 15 minutes, and then added with dry toluene (3.8 mL) and dissolved completely. Thereafter, the reaction mixture was heated to 110°C and stirred for 24 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (24 hours) to remove all solvents, and then reprecipitated in methanol to obtain 64.1 mg (53.3% yield) of a green material.

$^1$H NMR (500 MHz, $CDCl_3$): $\delta$ (ppm) [see FIG. 45].

**<Example 28> Synthesis of D-A structural polymer P(Fu3TC) [see FIG. 38]**

In 10 to 20 mL of a microwave auxiliary vial, (Z)-5-((2,5-dibromofuran-3-yl)methylene)-4H-cyclopenta[b]thiophene-4,6(5H)-dione (14) (58.2 mg, 0.15 mmol), 2DBDT (135.6 mg, 0.15 mmol), and $Pd(PPh_3)_4$ (5.3 mg) were added and capped. After vacuuming for 30 minutes or more, $N_2$ was changed and blown strongly for 15 minutes, and then added with dry toluene (3.8 mL) and dissolved completely. Thereafter, the reaction mixture was heated to 110°C and stirred for 24 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (24 hours) to remove all solvents, and then reprecipitated in methanol to obtain 67.5 mg (53.9% yield) of a green-brown material.

$^1$H NMR (500 MHz, $CDCl_3$): $\delta$ (ppm) [see FIG. 46].

**<Example 29> Synthesis of D-A structural polymer P(F-3BA) [see FIG. 47]**

In 10 to 20 mL of a microwave auxiliary vial, 5-((2,5-dibromothiophene-3-yl)methylene)-1,3-diethyl-2-thioxodihydropyrimidine-4,6(1H,5H)-dione (4) (90.5 mg, 0.20 mmol), F-2DBDT (188.0 mg, 0.20 mmol), and $Pd(PPh_3)_4$ (6.6 mg) were added and capped. After vacuuming for 30 minutes or more, $N_2$ was changed and blown strongly for 15 minutes, and then added with dry toluene (5.5 mL) and dissolved completely. Thereafter, the reaction mixture was heated to 110°C and stirred for 16 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (24 hours) to remove all solvents, and then reprecipitated in methanol to obtain 109.9 mg (60.6% yield) of a brown material.

$^1$H NMR (500 MHz, $CDCl_3$): $\delta$ (ppm) [see FIG. 48].

**<Example 30> Synthesis of D-A structural polymer P(F-3IN) [see FIG. 47]**

In 10 to 20 mL of a microwave auxiliary vial, 2-((2,5-dibromothiophene-3-yl)methylene)-1H-indene-1,3(2H)-dione (6) (79.6 mg, 0.20 mmol), F-2DBDT (188.0 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 16 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (24 hours) to remove all solvents, and then reprecipitated in methanol to obtain 92.4 mg (54.1% yield) of a black-green material.

$^1H$ NMR (500 MHz, $CDCl_3$): δ (ppm) [see FIG. 49].

**<Example 31> Synthesis of D-A structural polymer P(Cl-3IN) [see FIG. 47]**

In 10 to 20 mL of a microwave auxiliary vial, 2-((2,5-dibromothiophene-3-yl)methylene)-1H-indene-1,3(2H)-dione (6) (79.6 mg, 0.20 mmol), Cl-2DBDT (194.6 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 20 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (24 hours) to remove all solvents, and then reprecipitated in methanol to obtain 79.6 mg (44.9% yield) of a black-green material.

$^1H$ NMR (500 MHz, $CDCl_3$): δ (ppm) [see FIG. 50].

**<Example 32> Synthesis of D-A structural polymer P(C6C6-3IN) [see FIG. 47]**

In 10 to 20 mL of a microwave auxiliary vial, 2-((2,5-dibromothiophene-3-yl)methylene)-1H-indene-1,3(2H)-dione (6) (79.6 mg, 0.20 mmol), C6C6-2DBDT (203.4 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 24 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (24 hours) to remove all solvents, and then reprecipitated in methanol to obtain 88.8 mg (46.4% yield) of a black-green material.

$^1H$ NMR (500 MHz, $CDCl_3$): δ (ppm) [see FIG. 51].

**<Example 33> Synthesis of D-A structural polymer P(EDOT-3IN) [see FIG. 47]**

In 10 to 20 mL of a microwave auxiliary vial, 2-((2,5-dibromothiophene-3-yl)methylene)-1H-indene-1,3(2H)-dione (6) (79.6 mg, 0.20 mmol), EDOT-2DBDT (204.0 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 24 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (24 hours) to remove all solvents, and then reprecipitated in methanol to obtain 68.2 mg (37.7% yield) of a black-green material.

$^1H$ NMR (500 MHz, $CDCl_3$): δ (ppm) [see FIG. 52].

**<Example 34> Synthesis of D-A structural polymer P(SEH-3IN) [see FIG. 47]**

In 10 to 20 mL of a microwave auxiliary vial, 2-((2,5-dibromothiophene-3-yl)methylene)-1H-indene-1,3(2H)-dione (6) (38.8 mg, 0.10 mmol), SEH-2DBDT (96.9 mg, 0.10 mmol), and $Pd(PPh_3)_4$ (3.5 mg) were added and capped. After vacuuming for 30 minutes or more, $N_2$ was changed and blown strongly for 15 minutes, and then added

with dry toluene (3.0 mL) and dissolved completely. Thereafter, the reaction mixture was heated to 110°C and stirred for 24 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (24 hours) to remove all solvents, and then reprecipitated in methanol to obtain 71.1 mg (80.7% yield) of a black-green material.

$^1$H NMR (500 MHz, $CDCl_3$): δ (ppm) [none; insoluble after reprecipitation].

**<Example 35> Synthesis of D-A structural polymer P(SC8-3IN) [see FIG. 47]**

In 10 to 20 mL of a microwave auxiliary vial, 2-((2,5-dibromothiophene-3-yl)methylene)-1H-indene-1,3(2H)-dione (6) (38.8 mg, 0.10 mmol), SC8-2DBDT (96.9 mg, 0.10 mmol), and $Pd(PPh_3)_4$ (3.5 mg) were added and capped. After vacuuming for 30 minutes or more, $N_2$ was changed and blown strongly for 15 minutes, and then added with dry toluene (3.0 mL) and dissolved completely. Thereafter, the reaction mixture was heated to 110°C and stirred for 24 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (24 hours) to remove all solvents, and then reprecipitated in methanol to obtain 58.0 mg (65.8% yield) of a black-green material.

$^1$H NMR (500 MHz, $CDCl_3$): δ (ppm) [none; insoluble after reprecipitation].

**<Example 36> Synthesis of D-A structural polymer P(3IN)-1D [see FIG. 47]**

In 10 to 20 mL of a microwave auxiliary vial, 2-((2,5-dibromothiophene-3-yl)methylene)-1H-indene-1,3(2H)-dione (6) (39.8 mg, 0.10 mmol), 1DBDT (77.2 mg, 0.10 mmol), and $Pd(PPh_3)_4$ (3.5 mg) were added and capped. After vacuuming for 30 minutes or more, $N_2$ was changed and blown strongly for 15 minutes, and then added with dry toluene (3.0 mL) and dissolved completely. Thereafter, the reaction mixture was heated to 110°C and stirred for 24 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (24 hours) to remove all solvents, and then reprecipitated in methanol to obtain 52.0 mg (75.9% yield) of a green-brown material.

$^1$H NMR (500 MHz, $CDCl_3$): δ (ppm) [see FIG. 53].

**<Example 37> Synthesis of D-A structural polymer P(IFL-3IN) [see FIG. 54]**

In 10 to 20 mL of a microwave auxiliary vial, 2-((2,5-dibromothiophene-3-yl)methylene)-1H-indene-1,3(2H)-dione (6) (79.6 mg, 0.20 mmol), IFL (191.0 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (3.0 mL), dry hydrofuran (0.5 mL), and 2 M $K_2CO_3$ (1.5 mL) and dissolved completely. Thereafter, the reaction mixture was heated to 110°C and stirred for 24 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (24 hours) to remove all solvents, and then reprecipitated in methanol to obtain 122.0 mg (64.8% yield) of a red-brown material.

$^1$H NMR (500 MHz, $CDCl_3$): δ (ppm) [see FIG. 55].

**<Example 38> Synthesis of D-A structural polymer P(NDT-3IN) [see FIG. 54]**

In 10 to 20 mL of a microwave auxiliary vial, 2-((2,5-dibromothiophene-3-yl)methylene)-1H-indene-1,3(2H)-dione (6) (39.8 mg, 0.10 mmol), NDT (82.2 mg, 0.10 mmol), and $Pd(PPh_3)_4$ (3.5 mg) were added and capped. After vacuuming for 30 minutes or more, $N_2$ was changed and blown strongly for 15 minutes, and then added with dry toluene (2.5 mL) and dissolved completely. Thereafter, the reaction mixture was heated to 110°C and stirred for 24 hours. After end-capping using 2 to 3 drops of 2-bromothiophene, the mixture was further stirred for about 1 hour. The reactant

was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (24 hours) to remove all solvents, and then reprecipitated in methanol to obtain 63.8 mg (86.8% yield) of a brown material.

$^1H$ NMR (500 MHz, $CDCl_3$): δ (ppm) [none; insoluble after reprecipitation].

**<Example 39> Synthesis of D-A structural polymer P(IDT-3IN) [see FIG. 54]**

In 10 to 20 mL of a microwave auxiliary vial, 2-((2,5-dibromothiophene-3-yl)methylene)-1H-indene-1,3(2H)-dione (6) (79.6 mg, 0.20 mmol), IDT (246.6 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 24 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (24 hours) to remove all solvents, and then reprecipitated in methanol to obtain 179.7 mg (78.4% yield) of a brown material.

$^1H$ NMR (500 MHz, $CDCl_3$): δ (ppm) [see FIG. 56].

**<Example 40> Synthesis of $D_1$-$A_1$-$D_1$-$A_2$ structural ternary polymer P(3IN)(3IN2F=0.5) [see FIG. 57]**

In 10 to 20 mL of a microwave auxiliary vial, 2-((2,5-dibromothiophene-3-yl)methylene)-1H-indene-1,3(2H)-dione (6) (39.8 mg, 0.10 mmol), 2-((2,5-dibromothiophene-3-yl)methylene)-5,6-difluoro-1H-indene-1,3(2H)-dione (9) (43.4 mg, 0.10 mol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 18 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (12 hours) to obtain 153.1 mg (91.7% yield) of a black-green material.

$^1H$ NMR (400 MHz, $CDCl_3$) : δ (ppm) [see FIG. 58].

**<Example 41> Synthesis of $D_1$-$A_1$-$D_1$-$A_2$ structural ternary polymer P(3IN)(3IN2Cl=0.5) [see FIG. 57]**

In 10 to 20 mL of a microwave auxiliary vial, 2-((2,5-dibromothiophene-3-yl)methylene)-1H-indene-1,3(2H)-dione (6) (39.8 mg, 0.10 mmol), 2-((2,5-dibromothiophene-3-yl)methylene)-5,6-dichloro-1H-indene-1,3(2H)-dione (10) (46.7 mg, 0.10 mol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 18 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (12 hours) to obtain 148.5 mg (87.2% yield) of a black-green material.

$^1H$ NMR (400 MHz, $CDCl_3$) : δ (ppm) [none; insoluble after reprecipitation].

**<Example 42> Synthesis of $D_1$-$A_1$-$D_1$-$A_2$ structural ternary polymer P(3TC)(3IN2F=0.5) [see FIG. 57]**

In 10 to 20 mL of a microwave auxiliary vial, (Z)-5-((2,5-dibromothiophene-3-yl)methylene)-4H-cyclopenta[b]thiophene-4,6(5H)-dione (7) (40.4 mg, 0.10 mmol), 2-((2,5-dibromothiophene-3-yl)methylene)-5,6-difluoro-1H-indene-1,3(2H)-dione (9) (43.4 mg, 0.10 mol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 18 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24

hours), and dichloromethane (24 hours) to remove oligomers and residual catalyst. Finally, the product was dissolved in chloroform (12 hours) to obtain 149.0 mg (88.9% yield) of a green-brown material.

$^1$H NMR (400 MHz, $CDCl_3$) : δ (ppm) [see FIG. 59].

### <Example 43> Synthesis of $D_1$-$A_1$-$D_1$-$A_2$ structural ternary polymer P(3TC)(3TC1Cl=0.5) [see FIG. 57]

In 10 to 20 mL of a microwave auxiliary vial, (Z)-5-((2,5-dibromothiophene-3-yl)methylene)-4H-cyclopenta[b]thiophene-4,6(5H)-dione (7) (40.4 mg, 0.10 mmol), (Z)-2-chloro-5-((2,5-dibromothiophene-3-yl)methylene)-4H-cyclopenta[b]thiophene-4,6(5H)-dione (8) (43.9 mg, 0.10 mol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 18 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (12 hours) to obtain 120.1 mg (71.4% yield) of a brown material.

$^1$H NMR (400 MHz, $CDCl_3$) : δ (ppm) [see FIG. 60].

### <Example 44> Synthesis of $D_1$-$A_1$-$D_1$-$A_2$ structural ternary polymer P(3IN)(3TC1Cl=0.4) [see FIG. 57]

In 10 to 20 mL of a microwave auxiliary vial, 2-((2,5-dibromothiophene-3-yl)methylene)-1H-indene-1,3(2H)-dione (6) (47.8 mg, 0.12 mmol), (Z)-2-chloro-5-((2,5-dibromothiophene-3-yl)methylene)-4H-cyclopenta[b]thiophene-4,6(5H)-dione (8) (35.1 mg, 0.08 mol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 18 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (12 hours) to obtain 139.7 mg (83.4% yield) of a black-brown material.

$^1$H NMR (400 MHz, $CDCl_3$) : δ (ppm) [see FIG. 61].

### <Example 45> Synthesis of $D_1$-$A_1$-$D_1$-$A_2$ structural ternary polymer P(3IN)(3TC1Cl=0.2) [see FIG. 57]

In 10 to 20 mL of a microwave auxiliary vial, 2-((2,5-dibromothiophene-3-yl)methylene)-1H-indene-1,3(2H)-dione (6) (63.7 mg, 0.16 mmol), (Z)-2-chloro-5-((2,5-dibromothiophene-3-yl)methylene)-4H-cyclopenta[b]thiophene-4,6(5H)-dione (8) (17.5 mg, 0.04 mol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 24 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (12 hours) to obtain 143.3 mg (86.8% yield) of a brown material.

$^1$H NMR (400 MHz, $CDCl_3$) : δ (ppm) [none; insoluble after reprecipitation].

### <Example 46> Synthesis of $D_1$-$A_1$-$D_2$-$A_1$ structural ternary polymer P(3IN)(SEH-2DBDT=0.5) [see FIG. 62]

In 10 to 20 mL of a microwave auxiliary vial, 2-((2,5-dibromothiophene-3-yl)methylene)-1H-indene-1,3(2H)-dione (6) (79.6 mg, 0.20 mmol), 2DBDT (90.4 mg, 0.10 mmol), SEH-2DBDT (96.8 mg, 0.10 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 24 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (12 hours) to obtain 153.2 mg (90.2% yield) of a black-green material.

$^1$H NMR (400 MHz, $CDCl_3$) : δ (ppm) [none; insoluble after reprecipitation].

**<Example 47> Synthesis of $D_1$-$A_1$-$D_2$-$A_1$ structural ternary polymer P(3IN)(F-2DBDT=0.5) [see FIG. 62]**

In 10 to 20 mL of a microwave auxiliary vial, 2-((2,5-dibromothiophene-3-yl)methylene)-1H-indene-1,3(2H)-dione (6) (79.6 mg, 0.20 mmol), 2DBDT (90.4 mg, 0.10 mmol), F-2DBDT (94.0 mg, 0.10 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 24 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (12 hours) to obtain 128.8 mg (77.1% yield) of a black-green material.

$^1H$ NMR (400 MHz, $CDCl_3$) : $\delta$ (ppm) [see FIG. 63].

**<Example 48> Synthesis of $D_1$-$A_1$-$D_1$-$A_2$ structural ternary polymer P(3IN2F)(BDD=0.5) [see FIG. 64]**

In 10 to 20 mL of a microwave auxiliary vial, 2-((2,5-dibromothiophene-3-yl)methylene)-5,6-difluoro-1H-indene-1,3(2H)-dione (9) (43.4 mg, 0.10 mmol), BDD (76.7 mg, 0.10 mmol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 24 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (12 hours) to obtain 177.5 mg (87.0% yield) of a black-purple material.

$^1H$ NMR (400 MHz, $CDCl_3$) : $\delta$ (ppm) [see FIG. 65].

**<Example 49> Synthesis of $D_1$-$A_1$-$D_1$-$A_2$ structural ternary polymer P(3IN2F)(FTT=0.2) [see FIG. 64]**

In 10 to 20 mL of a microwave auxiliary vial, 2-((2,5-dibromothiophene-3-yl)methylene)-5,6-difluoro-1H-indene-1,3(2H)-dione (9) (69.4 mg, 0.16 mmol), FTT (18.9 mg, 0.04 mmol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 24 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (12 hours) to obtain 139.3 mg (80.9% yield) of a black-blue material.

$^1H$ NMR (400 MHz, $CDCl_3$) : $\delta$ (ppm) [see FIG. 66].

**<Example 50> Synthesis of $D_1$-$A_1$-$D_1$-$A_2$-$D_1$-$A_3$ structural quaternary polymer P(3IN=0.3)(3IN2F=0.5)(BDD=0.2) [see FIG. 67]**

In 10 to 20 mL of a microwave auxiliary vial, 2-((2,5-dibromothiophene-3-yl)methylene)-1H-indene-1,3(2H)-dione (6) (23.9 mg, 0.06 mmol), 2-((2,5-dibromothiophene-3-yl)methylene)-5,6-difluoro-1H-indene-1,3(2H)-dione (9) (43.4 mg, 0.10 mmol), BDD (30.67 mg, 0.04 mmol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 20 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (12 hours) to obtain 167.2 mg (92.0% yield) of a black-light green material.

$^1H$ NMR (400 MHz, $CDCl_3$) : $\delta$ (ppm) [see FIG. 68].

**<Example 51> Synthesis of $D_1$-$A_1$-$D_1$-$A_2$-$D_1$-$A_3$ structural quaternary polymer P(3TC=0.3)(3IN2F=0.5)(BDD=0.2) [see FIG. 67]**

In 10 to 20 mL of a microwave auxiliary vial, (Z)-5-((2,5-dibromothiophene-3-yl)methylene)-4H-cyclopenta[b]thiophene-4,6(5H)-dione (7) (24.2 mg, 0.06 mmol), 2-((2,5-dibromothiophene-3-yl)methylene)-5,6-difluoro-1H-indene-1,3(2H)-dione (9) (43.4 mg, 0.10 mmol), BDD (30.67 mg, 0.04 mmol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 18 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (12 hours) to obtain 169.9 mg (93.3% yield) of a black-light brown material.

$^1H$ NMR (400 MHz, $CDCl_3$) : δ (ppm) [see FIG. 69].

**<Example 52> Synthesis of $D_1$-$A_1$-$D_1$-$A_2$-$D_1$-$A_3$ structural quaternary polymer P(3TC=0.3)(3IN2F=0.5)(D18=0.2) [see FIG. 67]**

In 10 to 20 mL of a microwave auxiliary vial, (Z)-5-((2,5-dibromothiophene-3-yl)methylene)-4H-cyclopenta[b] thiophene-4,6(5H)-dione (7) (24.2 mg, 0.06 mmol), 2-((2,5-dibromothiophene-3-yl)methylene)-5,6-difluoro-1H-indene-1,3(2H)-dione (9) (43.4 mg, 0.10 mmol), D18 (36.3 mg, 0.04 mmol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 18 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (12 hours) to obtain 166.2 mg (88.5% yield) of a black-light purple material.

$^1H$ NMR (400 MHz, $CDCl_3$) : δ (ppm) [see FIG. 70].

**<Example 53> Synthesis of $D_1$-$A_1$-$D_1$-$A_2$-$D_1$-$A_3$ structural quaternary polymer P(3TC=0.3)(3IN2F=0.5) (3ClTh=0.2) [see FIG. 67]**

In 10 to 20 mL of a microwave auxiliary vial, (Z)-5-((2,5-dibromothiophene-3-yl)methylene)-4H-cyclopenta[b] thiophene-4,6(5H)-dione (7) (24.2 mg, 0.06 mmol), 2-((2,5-dibromothiophene-3-yl)methylene)-5,6-difluoro-1H-indene-1,3(2H)-dione (9) (43.4 mg, 0.10 mmol), 3ClTh (11.1 mg, 0.04 mmol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 18 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (12 hours) to obtain 148.7 mg (91.5% yield) of a black-light brown material.

$^1H$ NMR (400 MHz, $CDCl_3$) : δ (ppm) [see FIG. 71].

**<Example 54> Synthesis of A-D-A structural monomer RR-2DBDT-3TC [see FIG. 72]**

In 10 to 20 mL of a microwave auxiliary vial, (Z)-5-((2-bromothiophene-3-yl)methylene)-4H-cyclopenta[b] thiophene-4,6(5H)-dione (15) (541.4 mg, 1.665 mmol), 2DBDT (502.0 mg, 0.555 mmol), $Pd_2(dba)_3$ (23.0 mg), and P(*o*-toly)$_3$ (15.0 mg) were added and capped. After vacuuming for 30 minutes or more, $N_2$ was changed and blown strongly for 15 minutes, and then added with dry toluene (15.0 mL) and dissolved completely. Thereafter, the reaction mixture was heated to 110°C and stirred for 18 hours. After confirming the reaction course with TLC, a short column was put on dichloromethane using a silica pad, the solvent was removed, and the column purification (dichloromethane:hexane = 1:1) was performed. 518.5 mg (87.5% yield) of a red-brown material was obtained.

$^1H$ NMR (400 MHz, $CDCl_3$) : δ (ppm) = 8.44-8.42 (d, 2H), 8.05-8.03 (d, 2H), 7.94-7.92 (d, 2H), 7.75-7.74 (s, 2H), 7.49-7.48 (d, 2H), 7.46 (s, 2H), 7.40-7.39 (d, 2H), 6.88-6.87 (s, 2H), 2.81-2.79 (m, 4H), 1.58-1.56 (m, 2H), 1.34-1.25 (m, 16H), 0.86-0.83 (m, 12H) [see FIG. 73].

**<Example 55> Synthesis of A-D-A structural monomer RR-2DBDT-3IN2F [see FIG. 72]**

In 10 to 20 mL of a microwave auxiliary vial, 2-((2-bromothiophene-3-yl)methylene)-5,6-difluoro-1H-indene-1,3(2H)-dione (15') (591.3 mg, 1.665 mmol), 2DBDT (502.0 mg, 0.555 mmol), $Pd_2(dba)_3$ (23.0 mg), and P(*o*-toly)$_3$

(15.0 mg) were added and capped. After vacuuming for 30 minutes or more, $N_2$ was changed and blown strongly for 15 minutes, and then added with dry toluene (15.0 mL) and dissolved completely. Thereafter, the reaction mixture was heated to 110°C and stirred for 18 hours. After confirming the reaction course with TLC, a short column was put on dichloromethane using a silica pad, the solvent was removed, and the column purification (dichloromethane:hexane = 1:1) was performed. 533.0 mg (85.2% yield) of a black-brown material was obtained.

$^1$H NMR (400 MHz, $CDCl_3$) : δ (ppm) = 8.60-8.59 (d, 2H), 8.23 (s, 2H), 7.86-7.82 (m, 4H), 7.81-7.79 (s, 2H), 7.48-7.47 (d, 2H), 7.46-7.45 (d, 2H), 7.46 (s, 2H), 6.89-6.88 (d, 2H), 2.83-2.81 (m, 4H), 1.60-1.58 (m, 2H), 1.30-1.25 (m, 16H), 0.88-0.85 (m, 12H) [see FIG. 74].

**<Example 56> Synthesis of A-D-A structural monomer RR-2DBDT-3IN2F-2Br [see FIG. 72]**

In 10 to 20 mL of a microwave auxiliary vial, 2-((5-bromo-2-iodothiophene-3-yl)methylene)-5,6-difluoro-1H-indene-1,3(2H)-dione (18) (601.3 mg, 1.25 mmol), 2DBDT (452.3 mg, 0.50 mmol), and $Pd(PPh_3)_4$ (30.0 mg) were added and capped. After vacuuming for 30 minutes or more, $N_2$ was changed and blown strongly for 15 minutes, and then added with dry toluene (10.0 mL) and dimethylformamide (1.0 mL) and dissolved completely. Thereafter, the reaction mixture was heated to 110°C and stirred for 18 hours. After confirming the reaction course with TLC, a short column was put on dichloromethane using a silica pad, the solvent was removed, and the column purification (dichloromethane:hexane = 1:1) was performed. 485.0 mg (75.5% yield) of a black-brown material was obtained.

$^1$H NMR (400 MHz, $CDCl_3$) : δ (ppm) = 8.60 (s, 2H), 8.09 (s, 2H), 7.79-7.77 (m, 4H), 7.76 (s, 2H), 7.44-7.43 (s, 2H), 7.46 (s, 2H), 6.88 (s, 2H), 2.82-2.80 (m, 4H), 1.60-1.58 (m, 2H), 1.34-1.25 (m, 16H), 0.86-0.85 (m, 12H) [see FIG. 75].

**<Example 57> Synthesis of D-(A-D-A)-D-A' structure regioregular ternary polymer RR-P(3IN2F)(BDD=0.5) [see FIG. 76]**

In 10 to 20 mL of a microwave auxiliary vial, RR-2DBDT-3IN2F-2Br (128.5 mg, 0.10 mmol), BDD (76.7 mg, 0.10 mmol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 12 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (12 hours) to obtain 178.8 mg (77.4% yield) of a black-purple material.

$^1$H NMR (400 MHz, $CDCl_3$) : δ (ppm) [see FIG. 77].

**<Example 58> Synthesis of D-(A-D-A)-D-A' structure regioregular ternary polymer RR-P(3IN2F)(D18=0.5) [see FIG. 76]**

In 10 to 20 mL of a microwave auxiliary vial, RR-2DBDT-3IN2F-2Br (128.5 mg, 0.10 mmol), D18 (76.7 mg, 0.10 mmol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 12 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (12 hours) to obtain 196.5 mg (80.0% yield) of a black-purple material.

$^1$H NMR (400 MHz, $CDCl_3$) : δ (ppm) [see FIG. 78].

**<Example 59> Synthesis of A'-A structural polymer P(DPP-3TC) [see FIG. 150]**

In 10 to 20 mL of a microwave auxiliary vial, (Z)-5-((2,5-dibromothiophene-3-yl)methylene)-4H-cyclopenta[b]thiophene-4,6(5H)-dione (7) (40.4 mg, 0.10 mmol), 2,5-bis(2-octyldodecyl)-3,6-bis(5-(trimethylstannyl)thiophene-2-yl)-2,5-dihydropyrrolo[3,4-c]pyrrole-1,4-dione (DPP) (118.7 mg, 0.10 mmol), and $Pd(PPh_3)_4$ (3.5 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 20 hours. After end-capping using 2 to 3 drops of 2-bromothiophene, the mixture was further stirred for about 1 hour. The reactant was precipitated in methanol and stirred for about 30 minutes, and then the precipitates were filtered through a

colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (12 hours) to obtain 79.0 mg (71.4% yield) of a black-light green material.

$^1H$ NMR (400 MHz, $CDCl_3$) : δ (ppm) [see FIG. 151].

**<Example 60> Synthesis of D-(A-D-A) or D-(A-D-A)-D-A structural ternary polymer P(RR-3IN2F)(3TC=0.5) [see FIG. 148]**

In 10 to 20 mL of a microwave auxiliary vial, (Z)-5-((2,5-dibromothiophene-3-yl)methylene)-4H-cyclopenta[b] thiophene-4,6(5H)-dione (7) (40.4 mg, 0.10 mmol), RR-2DBDT-3IN2F-2Br (128.5 mg, 0.10 mmol), 2DBDT (180.9 mg, 0.20 mmol), and $Pd(PPh_3)_4$ (7.0 mg) were added and capped. After vacuuming for 30 minutes or more, $N_2$ was changed and blown strongly for 15 minutes, and then added with dry toluene (5.0 mL) and dissolved completely. Thereafter, the reaction mixture was heated to 110°C and stirred for 15 hours. After end-capping using 2 to 3 drops of 2-bromothiophene, the mixture was further stirred for about 1 hour. The reactant was precipitated in methanol and stirred for about 30 minutes, and then the precipitates were filtered through a colander. Then, Soxhlet purification was performed in the order of methanol (24 hours), acetone (24 hours), hexane (24 hours), and dichloromethane (24 hours) to remove oligomers and residual catalyst. Finally, the product was dissolved in chloroform (12 hours) to obtain 168.9 mg (86.7% yield) of a black-light brown material.

$^1H$ NMR (400 MHz, $CDCl_3$) : δ (ppm) [see FIG. 149].

**<Example 61> Design, synthesis and application of efficient organic semiconductor for electron donor based on 3-vinylene heterocycling introduced with high-planar electron withdrawer: Development of novel acceptor unit with low synthetic complexity and high scalability factor and novel polymer introduced with novel acceptor unit, computing simulation calculation and theoretical analysis [see FIGS. 79, 80, 81, 82, and 83]**

Comparison of synthetic complexity of acceptor units of existing highperformance polymers (e.g., PM6/7, D18, PTB7-Th, etc.) and newly developed 3-vinylene heterocycling units with highly planar electron withdrawers: Synthetic complexity (SC; Macromolecules 2015, 48, 3, 453-461) was defined as Equation evaluated by the Ricardo Po. research team in 2015 for the commercialization index of organic solar cell materials. The Equation was the sum of five parameters in a normalized database with weights of 35%, 25%, 15%, 15%, and 10% in sequence, wherein the parameters were Number of synthetic steps (NSS), Reciprocal yield (RY), Number of operation units for the isolation/purification (NUO), Number of column chromatographies for the isolation/purification (NCC), and Number of hazardous chemicals (NHC).

Therefore, the closer the SC was to 0, the higher the commercialization potential and the better the price competitiveness. However, estimating the SC of a material may be somewhat difficult due to various interpretations and assumptions about components that configure the separation and purification process, and inconsistencies in the number and characteristics of hazardous factor statements listed for the same chemical by different chemical suppliers. Therefore, in 2021, the Iain McCulloch research team took these considerations into account and proposed a simpler and more reliable method to calculate a scalability factor (SF; Adv. Energy Master. 2021, 11, 2100056) of specific organic solar cell materials considering only NSS and RY of a synthetic pathway, as an equation newly evaluated for commercialization indexes of the organic solar cell materials. Starting materials were defined similarly to previous literatures, i.e. "chemicals found on the market in amounts sufficient to produce hundreds of kilograms of polymer". Therefore, a synthetic pathway was always tracked until these starting materials were generated, and if possible, the starting materials were defined as starting materials in previous literatures. In this way, the scalability factors of BDD and D18 units, which constituted high-efficiency polymers such as PM6/7 and D18 well-known based on reliability, and newly developed 3-vinylene heterocycling units introduced with high-planar electron withdrawers were compared and analyzed.

As may be seen in FIG. 79, the BDD and D18 units exhibited NSS of 5 and 10 steps and RY of 4.97 and 4.76, respectively. On the other hand, as shown in FIG. 80, newly developed 3CHO, 3BA, 3IN, 3TC, 3TC1Cl, 3IN2F, 3IN2Cl, etc. showed lower values than the acceptor units of pre-commercialized high-efficiency polymers in all parameters with NSS of steps 1 to 4 and RY of 1.11 to 3.93.

Therefore, polymers composed of the newly developed 3-vinylene heterocycling units introduced with the high-planar electron withdrawers may have high scalability factors with much lower synthetic complexity. In other words, it may be evaluated that there is sufficient price competition if only efficiency is guaranteed. (a detailed comparison of the efficiency-scalability factors of the newly developed polymers will be discussed in detail later.)

Computing simulation calculations and analyses of newly developed polymers based on 3-vinylene heterocycling units introduced with high-planar electron withdrawers: Next, density functional theory (DFT) calculations were performed using Gaussian16 and computating simulation software, to determine the change trend of frontier energy levels when novel units were introduced into the polymer backbone. The functions used were b3lyp, and 6-31G(d).

The aforementioned BDD and D18 units are high-planar derivatives with intermediate electron-withdrawing

characteristics, and introduced with two long branched chains of 2-ethylhexyl and 2-butyloctyl to induce a thermal-dependent aggregation (TDA) effect in the polymer backbone, which has an advantage of simultaneously obtaining high solubility and polymerization degree in general organic solvents.

On the other hand, in the case of the novel units, the band gap of the polymer may be lowered more effectively due to groups with relatively strong electron-withdrawing characteristics linked to 3-vinylene, and unlike general side chains, the novel units have a single linked plate-like structure to generate a large tiling angle with regiorandom (RA) between adjacent 2DBDT donor derivatives, thereby cause a TDA effect like BDD and D18. In addition, since an opposite adjacent 2DBDT has a relatively recovered plane angle as much as the large tilting angle formed, it is expected that a face-on structure will be formed on a substrate. In addition, due to high aromaticity resulting from the sharing and hybridization of electron clouds linked to 3-vinylene, the novel units may be designed to have a deep HOMO energy level of the polymer at the same time. More importantly, since these novel monomers have an asymmetric structure, the novel monomers may impart a larger dipole moment to the polymer than BDD and D18, and despite the relatively low number of side chain substitutions, high solubility and high polymerization degree may be expected simultaneously in general organic solvents.

As may be seen in FIG. 81, as may be seen from the Gaussian result of n = 1 as a repeating unit (n) of model compounds having 2DBDT and 3IN derivatives as the main backbones, the effects of halogenation on the dihedral angle, dipole moment (D), HOMO, LUMO energy levels, and band gap could be investigated. First, as previously described, the novel acceptor units formed an RA backbone with an asymmetric structure. Therefore, the results in P(3IN)-in/out were different for all DFT calculation parameters. It was noteworthy that the dihedral angle was greatly tilted to 41.0° in an in structure, whereas the planarity was restored to 12.9° in an out structure, and thus it could be expected that an actual structure will have a flexible backbone as expected. On the other hand, when halogenation, especially fluorination, was introduced to 2DBDT and 3IN, there was no significant change in the dihedral angle, but there was a very large difference in frontier energy levels. Finally, it was found that introducing chlorination rather than fluorination into the polymer had a deeper HOMO energy level and a lower band gap.

In FIG. 82, the Gaussian calculation results at n = 1 were shown for model compounds having 2DBDT and 3TC derivatives as the main backbone. Even like P(3IN)-in/out, the dihedral angle and the frontier energy level were different in P(3TC)-in/out. However, the dipole moment showed similar values, which was considered to be analyzed through more detailed structural analysis and simulation calculations of possible structures. In particular, since the direction of S on the TC side may be oriented in RA in two directions, the total possible structures for 3TC are four, which is twice as many as for 3IN. Halogenation is also possible in 3TC, and as in the results of chlorinated 3TC1Cl, all DFT calculation parameters except for the dihedral angle are upward, and in other words, the dipole moment increases, the LUMO and HOMO energy levels become deeper, and the band gap also becomes smaller.

Finally, in order to more accurately estimate the energy levels of the actual polymer in FIG. 83, the DFT calculation results of P(3IN)-in, in/in, out/out, and out structures at n = 2 were compared and analyzed. As the repeating unit expanded into a dimer, the molecular hybridization energy level was stabilized, and thus all of the three structures exhibited similar LUMO, HOMO, and band gap. On the other hand, in the case of dihedral angles, it was observed that the planarity of the polymer backbone changed significantly depending on the direction in which 3IN was oriented in 2DBDT. It was noteworthy that the dihedral angles were alternately distorted and then restored between the 2DBDT and 3IN units, and it could be expected that the in and out structures will have a higher-planar polymer backbone when formed. This is because the polymer may have an inherently high $\pi$-$\pi$ stacking to have a structure in which a face-on structure is dominant.

<Example 62> Design, synthesis and application of efficient organic semiconductor for electron donor based on 3-vinylene heterocycling introduced with high-planar electron withdrawer: Physical, thermal, optical and electrochemical characteristics analysis [see FIGS. 84, 85, 86, 87, 88, 89, 90, 91, 92, 93, 94, 95, 96, 97, 98, 99, 100, 101, 102, 103]

Physical and thermal characteristics of polymers: All newly synthesized polymers/monomers were finally successfully synthesized via a Stille C-C coupling reaction. Each synthesis yield was manually recorded in the synthetic method of Example. All polymers, except for some polymers having insolubility after reprecipitation, showed excellent solubility in general organic solvents.

As shown in Table 1, the relative molecular weights were measured by Gel permeation chromatography (GPC) using polystyrene as a standard material, and as a result, it was confirmed that most of polymers formed appropriate molecular weights with a weight average molecular weight (Mw) of 30,000 or higher and a polydispersity index (PDI) of 2 points. Therefore, it was enough to investigate a structure-property relationship of polymers.

As the structure expanded from binary to ternary and quaternary polymer forms, the molecular weight gradually increased, which was because the rigidity of the polymer decreased relatively due to the structural complexity as the regiorandomnity in the molecular chain increased and the number of formable structures increased.

As may be seen in FIG. 84, as a result of thermo-gravimetric analysis (TGA), it was shown that all four polymers had a decomposition temperature $T_d$ (a temperature at which reduction of the sample weight was 5%) of 300°C or higher. This indicates that all four structures have sufficient thermal stability to be applicable to organic electronics applications

such as organic solar cells. In addition, it was observed that the thermal stability tended to increase as the halogenation within the structure increased or when 3TC was introduced rather than 3IN, and it was shown that the binary, ternary, and finally quaternary structures had the highest thermal stability. Detailed contents about the measurement results were summarized in Table 1.

The crystalline characteristics of the molecular structures were investigated by differential scanning calorimetry (DSC) measurements, and all polymers showed no obvious thermal transition between 25 and 300°C.

[Table 1]

| **Results of analyzing physical and thermal charateristics of novel polymers** | | | | |
|---|---|---|---|---|
| | **$M_n$ [Da]** | **$M_w$ [Da]** | **PDI** | **$T_d$ [°C]** |
| **P(3CHO)** | 18705 | 59354 | 3.17 | 368 |
| **P(3BA)** | 13048 | 24983 | 1.91 | 307 |
| **P(3IN)** | 19447 | 25515 | 1.31 | 336 |
| **P(3TC)** | 21261 | 28527 | 1.34 | 346 |
| **P(3TC1Cl)** | 18653 | 41595 | 2.22 | 350 |
| **P(3IN2F)** | 16573 | 38656 | 2.33 | 348 |
| **P(Fu3IN)** | 9453 | 11643 | 1.23 | - |
| **P(Fu3TC)** | 9569 | 12226 | 1.28 | - |
| **P(F-3BA)** | 16413 | 37445 | 2.28 | - |
| **P(EDOT-31N)** | 7419 | 10157 | 1.37 | - |
| **P(3IN)-1D** | 10700 | 13179 | 1.23 | - |
| **P(IFL-3IN)** | 7851 | 12811 | 1.63 | - |
| **P(IDT-3IN)** | 5039 | 8066 | 1.60 | - |
| **P(3IN)(3IN2F=0.5)** | 31740 | 64906 | 2.04 | 342 |
| **P(3TC)(3IN2F=0.5)** | 29203 | 65494 | 2.24 | 349 |
| **P(3IN)(3TC1Cl=0.4)** | 25902 | 78132 | 3.02 | 344 |
| **P(31N)(F-2DBDT=0.5)** | 20092 | 53351 | 2.66 | - |
| **P(3IN2F)(BDD=0.5)** | 43755 | 65154 | 1.49 | 354 |
| **P(3IN=0.3)(3IN2F=0.5)(BDD=0.2)** | 37461 | 78408 | 2.09 | 353 |
| **P(3TC=0.3)(3IN2F=0.5)(BDD=0.2)** | 39715 | 78729 | 1.98 | 357 |
| **P(3TC=0.3)(3IN2F=0.5)(D18 =0.2)** | 42815 | 81021 | 1.89 | - |
| **P(3TC=0.3)(3IN2F=0.5)(3ClTh=0.2)** | 28903 | 61660 | 2.13 | 375 |
| **RR-P(3IN2F)(BDD=0.5)** | 36607 | 42660 | 1.17 | - |
| **RR-P(3IN2F)(D18=0.5)** | 39874 | 55086 | 1.38 | - |

Optical and electrochemical characteristics of polymers: The optical and electrochemical characteristics of major polymers were studied by UV-Vis spectroscopy and cyclic voltammetry (CV).

As may be seen in FIGS. 85, 86, 87, and 88, it was shown that the absorption wavelengths in the normalized film states of all polymers had a narrower band gap, while a strong 0-0 peak disappeared around 550 to 600 nm, which had been clearly shown in the P(3CHO) polymer.

While P(3CHO) showed a UV behavior with a general donor-acceptor pushpull structure, all the polymers except for P(3CHO) showed unique $\pi$-$\pi$* transition characteristics around 300 to 400 nm and 450 to 550 nm, and the peaks indicating intramolecular charge transfer (ICT) were not distinct and appeared while being drafted toward a long-wavelength region.

This means that the electron flow varies along a main chain unlike a structural form between general donor-acceptor, and it maybe expected that Y6 derivatives with strong absorption bands in the range of 700 to 950 nm, and especially BTP-eC9 acceptors, may have complementary characteristics. The optical band gaps ($E_g^{opt}$) of the polymers

were calculated from each absorption onset in a thin film state and summarized in Table 2. Among novel acceptor units excluding 3CHO, 3BA, 3TC1Cl, 3TC, 3IN, 3IN2F, and 3IN2Cl tended to have narrower band gaps in the order. It was also shown when the structures were expanded from binary polymers to ternary and quaternary polymers.

In FIGS. 89, 90, 91, 92, 93, 94, 95, 96, 97, 98, and 99, the TDA characteristics of representative polymers developed in the art, including representative commercial polymers PM6 and PTB7-Th, were compared and analyzed. It was observed that P(3BA) had strong aggregation behavior at room temperature in all polymers except for P(3IN)-1D, and then the aggregation behavior was released as the temperature gradually increased. The polymers with these characteristics may be arranged and aggregated in a regular manner, and have the advantage of enabling more efficient charge transfer when configuring organic solar cell devices and orientation according to the polymer substrate.

As in FIGS. 100, 101, 102, and 103, the frontier energy levels of the polymers may be calculated from each redox curve obtained through a CV curve. In more detail, the HOMO ($E_{HOMO}$) and LUMO energy levels ($E_{LUMO}$) may be obtained by the following electrochemical equations for onset oxidation potential ($E_{ox}^{onset}$) and reduction potential ($E_{red}^{onset}$), respectively.

$E_{HOMO}$ or $E_{LUMO}$ = -4.8 - ($E_{ox or red}^{onset}$ - $E_{1/2}$, ferrocene), wherein $E_{1/2}$, ferrocene = 0.44 to 0.48 eV (ferrocene data obtained for each measurement). In this study, a method for determining a more reasonable and accurate trend was adopted by obtaining only the HOMO energy levels of all polymers through CV than obtaining the LUMO energy levels through the optical band gap and difference. All of the polymers are expected to have well-aligned frontier energy levels when mixed with Y6 derivatives, especially the BTP-eC9 acceptor, which enables efficient charge transfer.

Additionally, considering that the HOMO and LUMO energy levels of BTP-eC9, a near-infrared (NIR) acceptor, are -5.68 eV and -4.05 eV, all of the polymers are expected to have a minimized energy offset corresponding to 0.23 to 0 to have less energy loss.

In particular, among the novel acceptor units, as 3BA, 3IN, 3TC, 3IN2F, 3CHO, and 3IN2Cl are sequentially introduced into the polymers, it may be expected that the open-circuit voltage ($V_{oc}$) will increase due to the deep HOMO energy level. Detailed values representing the optical and electrochemical characteristics were summarized in Table 2.

[Table 2]

**Results of analyzing optical and electrochemical charateristics of novel polymers**

| Classification | UV-Vis spectroscopy | Cyclic voltammetry | | |
|---|---|---|---|---|
| | $E_g^{opt, a)}$ [eV] | $E_{ox}^{onsat}$ [V] | $E_{HOMO}^{b)}$ [eV] | $E_{LUMO}^{b)}$ [eV] |
| P(3CHO) | 1.92 | 1.33 | -5.58 | -3.66 |
| P(3BA) | 1.65 | 1.02 | -5.37 | -3.72 |
| P(3IN) | 1.68 | 1.09 | -5.44 | -3.76 |
| P(3TC) | 1.73 | 1.05 | -5.40 | -3.67 |
| P(3TC1Cl) | 1.64 | 1.22 | -5.53 | -3.89 |
| P(3IN2F) | 1.61 | 1.35 | -5.66 | -4.05 |
| P(3IN2Cl) | 1.58 | 1.36 | -5.68 | -4.10 |
| P(3IN)(3IN2F=0.5) | 1.62 | 1.27 | -5.58 | -3.96 |
| P(3IN)(3TC1Cl=0.4) | 1.69 | 1.24 | -5.55 | -3.86 |
| P(3IN2F)(BDD=0.5) | 1.61 | 1.27 | -5.58 | -3.97 |
| P(3IN)(F-2DBDT=0.5) | 1.74 | 1.21 | -5.57 | -3.83 |
| P(3IN=0.3)(3IN2F=0.5)(BDD=0.2) | 1.57 | 1.25 | -5.56 | -3.99 |
| P(3TC=0.3)(3IN2F=0.5)(BDD=0.2) | 1.62 | 1.23 | -5.54 | -3.92 |
| P(3TC=0.3)(3IN2F=0.5)(3ClTh=0.2) | 1.60 | 1.20 | -5.51 | -3.91 |
| RR-P(3IN2F)(D18=0.5) | 1.58 | 1.30 | -5.65 | -4.07 |

a) Calculated near tangent intersection on low energy edge of the spectrum in baseline
b) $E_{HOMO or LUMO}$ = -4.8 - [$E_{ox or red}^{onset}$ (vs. Ag/AgCl) - $E_{1/2}$($Fc/Fc^+$ vs. Ag/AgCl)] ($E_{1/2}$($Fc/Fc^+$ vs. Ag/AgCl) = 0.48 eV for polymers; 0.44 eV for terpolymers; 0.46 eV for quarterpolymers and regioregular polymer).

**<Example 63> Design, synthesis and application of efficient organic semiconductor for electron donor based on 3-vinylene heterocycling introduced with high-planar electron withdrawer: Fabrication of organic solar cells, crystalization/orientation, morphology analysis, contact angle analysis [see FIGS. 104, 105, 106, 107, 108, 109, 110, 111, 112, 113, 114, 115, 116, 117, 118, 119, 120, 121, 122, 123, 124, 125, 126, 127, 128, 129]**

Optimization for regular-structural organic solar cells by polymers: Newly developed representative polymers have optical and electrochemical characteristics that are highly complementary to those of BTP-eC9 having a band gap of 1.34 eV, and thus fabrication of devices based on the polymers is very rational.

The organic solar cell structure used in the present disclosure was a regular structure, and laminated with ITO/PEDOT:PSS (30 nm)/Polymer:BTP-eC9/PDINN (10 nm or less) or PNDIT-F3N (10 nm or less)/Ag (100 nm) to observe the photovoltaic performance.

Each device was optimized through a donor-acceptor ratio, a heat treatment temperature, and a solvent/additive control. All mixed films had a ratio of polymers and BTP-eC9 of 1:1.2 and an optimized thickness of 90 to 100 nm. An effective photoactive measurement area covering a mask was confirmed to be 0.04 $cm^2$ using an optical microscope.

As shown in FIGS. 104, 105, 106, 107, 108, 109, 110, and 111, J-V and External quantum efficiency (EQE) waveforms of each optimized organic solar cell device of representative binary, ternary, quaternary, and regioregular polymers were shown. First, the binary polymers were optimized using all halogen solvents as parent materials (chloroform, CF with 0.5% 1,8-diiodooctane, DIO for P(3CHO), P(3BA); chlorobenzene, CB with 0.5% DIO for P(3IN), P(3TC1Cl), P(3IN2F); CB with 3.5% 1-chloronaphthalene, CN for P(3TC)) due to limited solubility. It was noteworthy that like the trend of HOMO energy level change according to introduction of the novel acceptor units into the polymer backbone as described above, an open circuit voltage ($V_{oc}$) was formed, and all polymers except for P(3BA) showed high efficiency of 10% or higher. In particular, in the case of P(3TC), the highest efficiency of 11.7% was achieved among binary polymers with an excellent short circuit current density ($J_{sc}$) of 24 mA/$cm^2$ or higher. This is due to a photoreaction of 70% or more in the entire spectrum of 400 to 850 nm, which is the main photon energy band of sunlight.

As a morphology of a donor-acceptor building block expanded from binary polymers to ternary polymers, the regiorandomnity and structural complexity of moieties within the polymer backbone further increased to improve the processability of the polymer by using relatively eco-friendly solvents such as toluene and xylene as parent solvents. The optimized solvent conditions were as follows: o-xylene, XY with 0.8% 1-phenylnaphtalene, PN for P(3IN)(3IN2F=0.5), P(3IN)(3TC1Cl=0.5); toluene with 0.8% PN for P(3IN2F)(BDD=0.5). Despite the optimization of devices under eco-friendly solvent conditions, improved efficiency was achieved compared to binary polymers, and in particular, photoelectric conversion efficiency of up to 13.0% was achieved due to an increase in ICT effect by relatively introducing 50% of BDD units as acceptors in a P(3IN)(BDD=0.5) structure. However, the introduction of 50% BDD resulted in a loss in cost and further reduced transmittance in the visible light spectrum, so that structural changes were absolutely necessary.

As described above, since the effect of the ternary building block was evident, the results of organic solar cells using the quaternary polymers were expected. Likewise, the quaternary polymers also showed excellent solubility in eco-friendly solvents and were optimized under the following conditions: XY with 0.8% PN for P(3IN=0.3)(3IN2F=0.5)(BDD=0.2), P(3TC=0.3)(3IN2F=0.5)(BDD=0.2), P(3TC=0.3)(3IN2F=0.5)(3ClTh=0.2). All three quaternary polymers exhibited high photoelectric conversion efficiency of 13.7% or more, and as may be seen in the EQE graph, the quaternary polymers exhibited photoreaction approaching 80% in the entire spectrum of 450 to 850 nm. More importantly, the cost, efficiency, and permeability may all be increased simultaneously even though the BDD introduced about 50% in the ternary polymer was reduced to 20%. Furthermore, when introducing a 3ClTh acceptor unit with a simpler structure than BDD, lower cost may be achieved while maintaining the efficiency. Among the quaternary polymers, P(3IN=0.3)(3IN2F=0.5)(BDD=0.2) showed the best efficiency of 14.0% by obtaining the most balanced solar cell parameter values.

[Table 3]

| Results of optimized regular-structural organic solar cell devices using novel polymers | | | | | |
|---|---|---|---|---|---|
| | **Annealing[a)] [°C]** | **$V_{oc}$ M** | **$J_{sc}$ [mA $cm^{-2}$]** | **FF [%]** | **PCE [%]** |
| **P(3CHO):BTP-eC9** | 80 | 0.855 | 21.68 | 56.6 | 10.5 |
| **P(3BA):BTP-eC9** | 100 | 0.791 | 9.00 | 39.7 | 2.8 |
| **P(3IN):BTP-eC9** | 80 | 0.816 | 20.09 | 64.8 | 10.6 |
| **P(3TC):BTP-eC9** | 100 | 0.807 | 24.27 | 59.7 | 11.7 |
| **P(3TC1Cl):BTP-eC9** | 80 | 0.838 | 20.74 | 57.5 | 10.0 |
| **P(3IN2F):BTP-eC9** | 80 | 0.878 | 22.31 | 51.7 | 10.1 |
| **P(3IN)(3IN2F=0.5):BTP-eC9** | 100 | 0.846 | 24.14 | 61.6 | 12.6 |

(continued)

| Results of optimized regular-structural organic solar cell devices using novel polymers | | | | | |
|---|---|---|---|---|---|
| | Annealing[a)] [°C] | $V_{oc}$ M | $J_{sc}$ [mA cm$^{-2}$] | FF [%] | PCE [%] |
| P(3IN)(3TC1Cl=0.4):BTP -eC9 | 100 | 0.841 | 23.2 | 63.0 | 12.3 |
| P(3IN2F)(BDD=0.5):BTP -eC9 | 100 | 0.830 | 25.27 | 62.0 | 13.0 |
| P(3IN=0.3)(3IN2F=0.5) (BDD=0.2):BTP-eC9 | 80 | 0.825 | 25.42 | 66.8 | 14.0 |
| P(3TC=0.3)(3IN2F=0.5) (BDD=0.2):BTP-eC9 | 80 | 0.823 | 25.70 | 65.9 | 13.9 |
| P(3TC=0.3)(3IN2F=0.5) (3ClTh=0.2):BTP-eC9 | 80 | 0.807 | 25.37 | 66.7 | 13.7 |
| RR-P(3IN2F)(BDD=0.5): BTP-eC9 | 80 | 0.858 | 20.59 | 58.0 | 10.2 |
| a) Heat treatment was performed for 10 minutes after coating. | | | | | |

To analyze the photovoltaic characteristics of polymers in detail, the crystal and orientation characteristics of the polymers in a pristine film or mixed film state were investigated by Grazing-Incidence Wide-Angle X-ray Scattering (GIWAXS).

As may be seen in FIG. 112, first, among the binary polymers, P(3CHO) was found to have a stacking distance in the tightest direction when viewed in an out-of-plane direction. This means that P(3CHO) has an orientation that may be expected to have high charge transfer and crystallinity when mixed with BTP-eC9. On the other hand, the crystallization and orientation characteristics were found to weaken and then strengthen again in the order of P(3BA), P(3IN), and P(3TC). In particular, in the case of P(3BA), the low efficiency of the device was understood because an amorphous phase was dominant in a pristine polymer film. Although weaker than P(3CHO), high charge transfer could be expected as the face-on structure was restored in the P(3IN) and P(3TC) structures.

As shown in FIGS. 113 and 114, the BTP-eC9 and mixed film of each of P(3CHO), P(3IN), and P(3TC) polymers, which had excellent crystallization and orientation characteristics, were analyzed. As a result, it was confirmed that P(3CHO) in (a) lost its original strong face-on structure characteristic, whereas P(3IN) in (b) strongly formed an edge-on crystal structure, and finally, P(3TC) in (c) showed the most dominant face-on crystal structure. It was analyzed that for each solar cell parameter, P(3CHO) achieved 10.5% with high $V_{oc}$ even though the crystal collapsed, P(3IN) had a relatively weakest face-on structure, but achieved 10.6% with high FF due to strong (100) characteristics, and P(3CHO) had efficiency of 11.7% with high $J_{sc}$ due to strong $\pi$-$\pi$ stacking.

In FIG. 115, changes in the crystallization and orientation characteristics of P(3TC1Cl) and P(3IN2F) were observed according to halogenation of P(3TC) and P(3IN) polymers. Although it was difficult to be compared in parallel with previous data because X-ray related analysis equipment used was different, the trends and effects in the ternary polymers developed using these novel halogenated units were observed. As may be seen in FIG. 116, when the P(3IN)(3IN2F=0.5) ternary polymer had a relatively weaker face-on structure than P(3IN2F) introduced with 100% 3IN2F, it could be seen that the fluorination effect of 3IN had a positive effect on the orientation of the polymer. Similarly, it was found that chlorination in P(3TC1Cl) also had a similar trend. However, while P(3IN2F) and P(3TC1Cl) had excellent characteristics in terms of crystallization and orientation, P(3IN2F) and P(3TC1Cl) exhibited several issues, such as relatively low molecular weight, strong molecular aggregation behavior, limited solubility, etc.

On the other hand, ternary polymers such as P(3IN)(3IN2F=0.5) and P(3IN)(3TC1Cl=0.4) showed results in which the physical, optical, and electrochemical characteristics were fully reflected to the device efficiency despite the use of eco-friendly solvents. This was because the polymers had a strong face-on structure and enabled smooth charge transfer when mixed with BTP-eC9. Finally, the P(3IN2F)(BDD=0.5) polymer, in which 3IN2F 50% and BDD 50% as acceptor units were introduced into the polymer backbone, exhibited the strongest face-on structure, which was caused by the highest efficiency of 13%. More specifically, as may be seen in FIGS. 117 and 118, both polymers, P(3IN)(3IN2F=0.5) and P(3IN2F)(BDD=0.5), showed further increased crystallinity when mixed with BTP-eC9 than each pristine film, and reached high efficiencies of 12.6% and 13.0% with relatively high $J_{sc}$ characteristics.

Finally, as shown in FIGS. 119 and 120, the P(3IN=0.3)(3IN2F=0.5)(BDD=0.2) polymer, which was further expanded into a quaternary building block, exhibited a stronger face-on structure in the pristine film despite a relatively 30% reduction in BDD in the backbone compared to the P(3IN2F)(BDD=0.5) polymer, which was confirmed to have crystal and orientation characteristics that were the opposite of those of a commercial polymer PM6, in which an edge-on structure was dominated. In the quaternary structure, since the rigidity was reduced compared to the ternary polymer and the miscibility was relatively increased, the highest efficiency of 14.0% was achieved among the synthesized polymers with a high FF of 66.8% when mixed with BTP-eC9.

In photovoltaic performance, the morphology of the active layer is closely related to charge transport and collection. Therefore, the surfaces of the optimized mixed films were closely observed through atomic force microscopy

(AFM).

As may be seen in FIGS. 121, 122, 123, and 124, most polymers exhibited smooth and good morphologies with BTP-eC9 in a microscale. It was noteworthy that as the aggregates were relatively reduced from binary polymers to ternary and quaternary polymers, the root-mean-square (RMS) roughness gradually decreased from around 3.0 nm to 0.7-0.8 nm, which was an appropriate value for efficient charge transfer.

In addition, the change trends of the FF and $J_{sc}$ values of the devices were able to be understood as observed in each of 2D/3D topography and phase images. In particular, all three polymers, P(3IN)(3IN2F=0.5), P(3IN2F)(BDD=0.5), and P(3IN=0.3)(3IN2F=0.5)(BDD=0.2), which showed high efficiency of 12.6%, 13.0%, and 14.0%, formed donor-acceptor interpenetrating networks and had clear phase images, which indicated that bicontinuous phase separation was possible at a nanoscale.

Finally, as shown in FIGS. 125, 126, 127, 128, and 129, the surface contact angles of each material in diiodomethane (DIM) and water were measured using DSA100 equipment, and as a result, a correlation between newly synthesized donor polymer and the miscibility with BTP-eC9 could be understood and calculated. In brief, from the contact angle measurement results, a Owens, Wendt, Rabel and Kaelble (OWRK) model was used to determine the surface energy of each material. In the case of polymers that showed high efficiency, including a commercial polymer PM6, it was analyzed that a surface energy difference with BTP-eC9 was relatively increased to form a purer domain than in the mixed film and the donor and the acceptor were properly mixed while maintaining high crystallinity to exhibit high photovoltaic characteristics. Detailed values thereof were summarized in Table 4.

[Table 4]

| **Results of surface energy analysis accoridng to measurment of contact angles of novel polymers** | | | | | |
|---|---|---|---|---|---|
| | **$CA_{DIM}$ [°]** | **$CA_{Water}$ [°]** | **SFE [mN/m]** | **Polar [mN/m]** | **Disperse [mN/m]** |
| **P(3CHO)** | 48.83 | 97.08 | 35.29 | 0.37 | 34.93 |
| **P(3BA)** | 44.20 | 92.99 | 38.13 | 0.69 | 37.43 |
| **P(3IN)** | 39.07 | 93.13 | 40.54 | 0.46 | 40.08 |
| **P(3TC)** | 41.27 | 97.32 | 39.10 | 0.13 | 38.96 |
| **P(3IN2F)** | 47.95 | 95.47 | 35.93 | 0.52 | 35.41 |
| **P(3IN)(3IN2F=0.5)** | 44.73 | 94.39 | 37.68 | 0.52 | 37.16 |
| **P(3IN)(3TC1Cl=0.4)** | 41.84 | 94.37 | 39.09 | 0.41 | 38.67 |
| **P(3TC1Cl)** | 41.97 | 92.41 | 39.28 | 0.67 | 38.61 |
| **P(3IN2F)(BDD=0.5)** | 52.51 | 99.35 | 33.13 | 0.27 | 32.86 |
| **P(3IN=0.3)(3IN2F=0.5) (BDD=0.2)** | 51.59 | 97.41 | 33.82 | 0.44 | 33.38 |
| **PM6 (Commercial)** | 57.55 | 104.98 | 30.03 | 0.04 | 29.99 |
| **BTP-eC9** | 39.84 | 93.52 | 40.28 | 0.43 | 39.84 |

**<Example 64> Design, synthesis and application of efficient organic semiconductor for electron donor based on 3-vinylene heterocycling introduced with high-planar electron withdrawer: Optimization of organic solar cells by controlling ratio of ternary-based donors of P(3IN=0.3)(3IN2F=0.5)(BDD=0.2) polymer, external certification and fabrication of semitransparent high-efficiency organic solar cell devices/modules [see FIGS. 130, 131, 132, 133, 134, 135, 136, 137, 138, 139, 140, 141, 142, 143, 144, 145, 146, 147]**

Based on an excellent structure-property relationship, crystallization/orientation, and morphology characteristics of the P(3IN=0.3)(3IN2F=0.5)(BDD=0.2) polymer, ternary organic solar cells were first fabricated for higher efficiency. In order to improve FF by increasing edge-on orientation in a conventional P(3IN=0.3)(3IN2F=0.5)(BDD=0.2):BTP-eC9 mixed film, the PM6 polymer was adopted and introduced as a ternary donor, and devices with a regular structure were fabricated and evaluated under the same eco-friendly solvent conditions. In particular, high-crystallinity film formation was induced during coating and annealing by selecting TL instead of an XY solvent. The optimized ternary organic solar cell device results were summarized in Table 5, and the J-V and EQE waveforms of the optimized ternary organic solar cell devices were shown in FIGS. 130 and 131. As the ratio of PM6 gradually increased instead of P(3IN=0.3)(3IN2F=0.5)(BDD=0.2), $J_{sc}$ and FF were improved, and in particular, the highest PCE of 16.5% could be reached at the ratio of P(3IN=0.3)(3IN2F=0.5)(BDD=0.2):PM6=0.4:0.6 as FF increased dramatically. However, as 0.6

or higher or PM6 was introduced proportionally instead of P(3IN=0.3)(3IN2F=0.5)(BDD=0.2), $J_{sc}$ was decreased significantly, resulting in relatively low efficiency of 13.9% at PM6:BTP-eC9=1:1.2. To verify the highest cell efficiency, an external evaluation was conducted at the Nano Convergence Practical Application Center (NCPAC) in Daegu, Korea, and as a result, high PCE of 16.564% was certified, similar to the laboratory. Even the efficiency deviation within one cell was less than 0.1%, and the average value of all four points within the device was 16.469%, confirming high uniformity. (see FIG. 132)

[Table 5]

| **Results of optimized regular-structural ternary-based organic solar cell devices** | | | | | |
|---|---|---|---|---|---|
| | **Annealing[a) ] [°C]** | **$V_{oc}$ [V]** | **$J_{sc}$ [mA cm$^{-2}$]** | **FF [%]** | **PCE [%]** |
| **P(3IN=0.3)(3IN2F=0.5)(BDD=0.2):PM6:BTP-eC9= 1.0:0:1.2 (XY)** | 80 | 0.825 | 25.42 | 66.8 | 14.0 |
| **P(3IN=0.3)(3IN2F=0.5)(BDD=0.2):PM6:BTP-eC9= 1.0:0:1.2 (TL)** | 80 | 0.823 | 25.70 | 65.4 | 13.8 |
| **P(3IN=0.3)(3IN2F=0.5)(BDD=0.2):PM6:BTP-eC9= 0.9:0.1:1.2 (TL)** | 80 | 0.824 | 25.90 | 66.1 | 14.1 |
| **P(3IN=0.3)(3IN2F=0.5)(BDD=0.2):PM6:BTP-eC9= 0.8:0.2:1.2 (TL)** | 80 | 0.829 | 26.10 | 67.9 | 14.7 |
| **P(3IN=0.3)(3IN2F=0.5)(BDD=0.2):PM6:BTP-eC9= 0.6:0.4:1.2 (TL)** | 80 | 0.827 | 26.36 | 70.3 | 15.3 |
| **P(3IN=0.3)(3IN2F=0.5)(BDD=0.2):PM6:BTP-eC9= 0.5:0.5:1.2 (TL)** | 80 | 0.827 | 26.41 | 73.3 | 16.0 |
| **P(3IN=0.3)(3IN2F=0.5)(BDD=0.2):PM6:BTP-eC9= 0.4:0.6:1.2 (TL)** | 80 | 0.827 | 26.25 | 75.8 | 16.5 |
| **Certified data** | | 0.828 | 26.709 | 74.885 | 16.564 |
| **P(3IN=0.3)(3IN2F=0.5)(BDD=0.2):PM6:BTP-eC9 = 0.2:0.8:1.2 (TL)** | 80 | 0.794 | 25.36 | 74.8 | 15.1 |
| **P(3IN=0.3)(3IN2F=0.5)(BDD=0.2):PM6:BTP-eC9= 0:1.0:1.2 (TL)** | 80 | 0.776 | 23.36 | 76.5 | 13.9 |
| **PM6:BTP-eC9=1:1.2 (CF 0.25% DIO)** | 100 | 0.845 | 26.0 | 76.8 | 16.9 |
| **PM6:BTP-eC9 (Ref.)[c)]** | 100 | 0.839 | 26.2 | 81.1 | 17.8 |

[a)] Heat treatment was performed for 10 minutes after coating.
[b)] P(3IN=0.3)(3IN2F=0.5)(BDD=0.2):PM6:BTP-eC9=0.4:0.6:1.2 (TL)-based device were certified for efficiency after external evaluation from Nano Convergence Practical Application Center (NCPAC) in Korea.
[c)] Adv. Mater. 2020, 1908205.

As designed above for the device structure, GIWAXS measurements were performed to understand the results of the ternary organic solar cell devices along with the crystallinity and orientation trends. First, as may be seen in FIG. 133, P(3IN=0.3)(3IN2F=0.5)(BDD=0.2) and PM6 pristine thin films were investigated, and as a result, P(3IN=0.3)(3IN2F=0.5)(BDD=0.2) showed a face-on dominant structure, while PM6 showed an edge-on dominant structure, which showed opposing crystallization and orientation characteristics. As shown in FIG. 134, the crystallinity and orientation of the mixed films were observed according to the ratio between the two donors, and as a result, the results of the optimized ternary organic solar cell devices could be understood. As the PM6 ratio gradually increased instead of P(3IN=0.3)(3IN2F=0.5)(BDD=0.2), the edge-on crystallinity was improved and the ratio of face-on to edge-on structures was balanced at the 0.4:0.6 condition. On the other hand, similar to the device results, the face-on structures showed a collapse pattern due to excessive edge-on orientation as the ratio of PM6 increased above 0.6. The ternary organic solar cell that had the highest efficiency using transmission electron microscopy (TEM) was compared with that of PM6:BTP-eC9 (PCE = 16.9%) fabricated with CF through surface and elemental analysis in the nanoscale region. As shown in FIG. 135, the ternary organic solar cell film exhibited uniform and continuous thin film characteristics than PM6:BTP-eC9 in the 100 nm scale region. When analyzed by further expanding to the 10 nm scale, it was found that PM6:BTP-eC9 formed very small and uniform domains that could overcome the exciton diffusion length of 10 nm or less, and it was found that PM6:BTP-eC9

also formed high-quality domains even in the ternary organic solar cells to have excellent thin film characteristics. As shown in FIG. 136, as a result of comparing a closer donor-acceptor mixing distribution through elemental analysis (F signal: P(3IN=0.3)(3IN2F=0.5)(BDD=0.2) and PM6; N, Cl signals: BTP-eC9), it could be seen that both films formed dense and uniform bi-continous D-A networks with smooth charge transfer.

Based on the optimized device results, P(3IN=0.3)(3IN2F=0.5)(BDD=0.2) and P(3IN=0.3)(3IN2F=0.5)(BDD=0.2):PM6-based semitransparent organic solar cells were designed. First, as shown in FIG. 137, the UV-Vis measurement results of each polymer at a concentration of $10^{-5}$ M showed that P(3IN=0.3)(3IN2F=0.5)(BDD=0.2) had higher transmittance in the average visible transmittance (AVT; Joule 2019, 3, 1803-1809) of 380 to 780 nm than PM6. This is because the transmittance differs greatly in the photopic response region, which is most important for distinguishing eye color. In other words, as a result of calculating the absorption coefficient of the solution as the average value according to the Beer-Lambert equation at 555 nm, PM6 had an absorbance of approximately 50,000, while P(3IN=0.3)(3IN2F=0.5)(BDD=0.2) showed a relatively smaller absorbance of 30,000. The low absorbance in this region suggests that it is more advantageous for application as transparent solar cells, and means that the ability and probability of humans discerning objects through windows manufactured based thereon increase. As a result of comparative analysis of the absorption coefficient in the form of a thin film to be applied to an actual solar cell, as shown in FIG. 138, it was confirmed that the same result as the solution was shown. Actually, as shown in FIG. 139, thin films of the two polymers were formed to have the same thickness of 30, 50, 85, and 135 nm, respectively, and then the identification of words with rainbow colors was compared and analyzed. As a result, it was seen that P(3IN=0.3)(3IN2F=0.5)(BDD=0.2) had better characteristics than PM6. In particular, at the same thickness of 135 nm, PM6 showed difficulty in both word and color identification, whereas P(3IN=0.3)(3IN2F=0.5)(BDD=0.2) still showed the ability to identificate both words and colors.

Next, changes in the polarization characteristics of light of the two polymers were confirmed using an Ellipsometer, and the complex refractive indexes of the materials were measured according to the wavelength of light. Based on this, as shown in FIG. 140, the optical electric field was analyzed using optical simulation, and as a result, at 300 to 500 nm, PM6 showed a slightly lower electric field than P(3IN=0.3)(3IN2F=0.5)(BDD=0.2) to have high light transmittance in this region. However, at 500 to 700 nm including a photopic response (maximum at 555 nm), PM6 showed relatively high light transmittance with the lowest electric field of almost 0.4 to have the same trend as the UV result. It was confirmed that this trend was maintained even under mixed conditions with BTP-eC9. Even in the simulations laminated with the same thickness as the optimized device structure based on each polymer, it was suggested that P(3IN=0.3)(3IN2F=0.5)(BDD=0.2):BTP-eC9 showed a lower electric field than PM6:BTP-eC9 in the 555 nm range to fabricate a transparent solar cell device with high light transmittance actually.

As shown in FIGS. 141 and 142, the absorbance and transmittance of the optimized ternary mixed films based on P(3IN=0.3)(3IN2F=0.5)(BDD=0.2) measured via UV-Vis were compared and analyzed, and as a result, a trade-off relationship between the absorbance and the transmittance was clearly confirmed, and based on the result, the AVT was calculated according to a ratio between the two donors. As a result, in increasing order of the PM6 ratio, the P(3IN=0.3)(3IN2F=0.5)(BDD=0.2):PM6:BTP-eC9 mixture had the transmittance of 62.0%, 60.9%, 59.4%, 56.6%, 53.7%, and 50.7%, which was confirmed to have a relative advantage in terms of transmittance compared to the transmittance of 47.6% of the PM6:BTP-eC9 mixed phase.

Based on the remarkable results and advantages of transmittance in opaque ternary organic solar cells, semitransparent organic solar cells were fabricated with the following regular structure, ITO/PEDOT:PSS(30 nm)/P(3IN=0.3)(3IN2F=0.5)(BDD=0.2):PM6:BTP-eC9/PDINN(10 nm)/Ag(10 or 13 nm)/WO3(25 nm). As a result, a high-performance semitransparent organic solar cell with both high efficiency and high transmittance was obtained, as shown in Table 6. The J-V and EQE waveforms of the optimized semitransparent organic solar cell devices were shown in FIGS. 143 and 144, and the AVT calculations of these devices were performed through UV-Vis measurement as shown in FIG. 145. In summary, among devices introduced with Ag(13 nm)/WO3(25 nm) as an upper transparent electrode, as seen above, in terms of transmittance, P(3IN=0.3)(3IN2F=0.5)(BDD=0.2):BTP-eC9=1:1.2 showed the highest AVT of 35.1%, and in terms of efficiency, P(3IN=0.3)(3IN2F=0.5)(BDD=0.2):PM6:BTP-eC9=0.4:0.6:1.2 showed the highest PCE of 12.7%. The semitransparent device of PM6:BTP-eC9=1:1.2 showed higher efficiency of 13.2%, which was a result of using CF, a toxic solvent, and showed the lowest AVT of 17.0%. By making an Ag electrode slightly thinner under the conditions that showed the highest light transmittance and efficiency, as a result optimized by introducing Ag(10 nm)/WO3(25 nm), the PCE and AVT of P(3IN=0.3)(3IN2F=0.5)(BDD=0.2):BTP-eC9=1:1.2 were 9.8% and 40.7%, respectively, showing the highest light utilization efficiency (LUE=PCE x AVT) of 3.99%. In addition, P(3IN=0.3)(3IN2F=0.5)(BDD=0.2):PM6:BTP-eC9=0.4:0.6:1.2 had PCE and AVT of 11.6% and 32.4%, respectively, which was a significant increase of 0.61% in LUE compared to a device introduced with Ag(13 nm)/$WO_3$(25 nm) with LUE of 3.76%. When an advanced transparent electrode technology is introduced instead of a metal/metal oxide combination, it is expected that the highest LUE may be achieved with higher PCE and AVT under the conditions of P(3IN=0.3)(3IN2F=0.5)(BDD=0.2):PM6:BTP-eC9=0.4:0.6:1.2. FIG. 146 shows real images of the best semitransparent devices introduced with Ag(10 or 13 nm)/$WO_3$(25 nm). The photovoltaic performance and transmittance of the transparent organic solar cell cells are remarkable achievements that may be marked as top research when compared with recent literature.

[Table 6]

| Results of optimized regular-structural ternary-based semitransparent organic solar cell devices | | | | | | |
|---|---|---|---|---|---|---|
| | Annealing[a)] [°C] | $V_{oc}$ [V] | $J_{sc}$ [mA $cm^{-2}$] | FF [%] | PCE [%] | AVT [%] |
| P(3IN=0.3)(3IN2F=0.5)(BDD=0.2):PM6:BTP-eC9=1.0:0:1.2 (XY)[b)] | 80 | 0.836 | 19.77 | 63.7 | 10.5 | 35.1 |
| P(3IN=0.3)(3IN2F=0.5)(BDD=0.2):PM6:BTP-eC9=1.0:0:1.2 (XY)[c)] | 80 | 0.836 | 18.83 | 63.5 | 9.8 | 40.7 |
| P(3IN=0.3)(3IN2F=0.5)(BDD=0.2):PM6:BTP-eC9=0.8:0.2:1.2 (TL)[b)] | 80 | 0.823 | 19.85 | 67.4 | 11.0 | 30.3 |
| P(3IN=0.3)(3IN2F=0.5)(BDD=0.2):PM6:BTP-eC9=0.6:0.4:1.2 (TL)[b)] | 80 | 0,821 | 20.14 | 71.6 | 11.8 | - |
| P(3IN=0.3)(3IN2F=0.5)(BDD=0.2):PM6:BTP-eC9=0.4:0.6:1.2 (TL)[b)] | 80 | 0.818 | 20.75 | 74.6 | 12.7 | 24.8 |
| P(3IN=0.3)(3IN2F=0.5)(BDD=0.2):PM6:BTP-eC9=0.4-0.6:1.2 (TL)[c)] | 80 | 0.819 | 19.09 | 74.2 | 11.6 | 32.4 |
| P(3IN=0.3)(3IN2F=0.5)(BDD=0.2):PM6:BTP-eC9=0.2:0.8:1.2 (TL)[b)] | 80 | 0.810 | 19.60 | 73.6 | 11.7 | - |
| PM6:BTP-eC9=1:1.2 (CF 0.25% DIO)[b)] | 100 | 0.817 | 20.87 | 77.7 | 13.2 | 17.0 |
| [a)] Heat treatment was performed for 10 minutes after coating.<br>[b)] Ag(13 nm)/$WO_3$(25 nm),<br>[c)] Ag(10 nm)/$WO_3$(25 nm). | | | | | | |

Finally, in order to verify the potential as building-integrated photovoltaics (BIPVs), a key application of the future transparent solar cell market, and windows for building facades, a large-area P(3IN=0.3)(3IN2F=0.5)(BDD=0.2) based opaque/semitransparent organic solar cell module with a size of 15 x 15 $cm^2$ was fabricated as a prototype and evaluated. A real photograph of the fabricated opaque/semitransparent organic solar cell module was shown in FIG. 147. The opaque/semitransparent organic solar cell adopted an inverse structure due to relative instability of an organic ETL on a large area, which was the same as ITO/ZnO(20 nm)/P(3IN=0.3)(3IN2F=0.5)(BDD=0.2):BTP-eC9=1:1.2/$MoO_3$(10 nm)/Ag(15 nm)/$MoO_3$(30 nm). In the case of the fabricated opaque/translucent modules, the efficiency was less than 1% due to insufficient large-area production technology and equipment, but $V_{oc}$ was 5 to 7 V or higher, and thus the modules were formed relatively stably, so that a propeller of 3 to 5 V or higher could rotate at high speed when light was irradiated. Real photographs of the opaque/semitransparent organic solar cell modules were shown in FIG. 144. Considering the technologies so far, it is expected that when advanced large-area technologies are satisfied along with upper transparent electrode technologies in the future, it will be expected to gain an advantage not only in the BIPV market but also in automotive-integrated photovoltaics (AIPV), Internet of Things (IoT) sensors, etc.

In the present disclosure, the methods for designing, synthesizing and applying the efficient organic semiconductor for the electron donor based on 3-vinylene heterocycling introduced with the high-planar electron withdrawer have been described through the above-described Examples, but there is no need to specifically limit the methods, and any fabrication method satisfying the reaction formulas above may be used.

In addition, the organic solar cell device of the present disclosure may be manufactured in the order of anode/hole transport layer/photoelectric conversion layer/electron transport layer/cathode as described above, or may be manufactured in the opposite order, that is, cathode/electron transport layer/photoelectric conversion layer/hole transport layer/anode.

Hereinabove, the present disclosure has been described with reference to preferred embodiments thereof. It will

be understood to those skilled in the art that the present disclosure may be implemented as modified forms without departing from an essential characteristic of the present disclosure. Therefore, the disclosed embodiments should be considered in an illustrative viewpoint rather than a restrictive viewpoint. The scope of the present disclosure is illustrated by the appended claims rather than by the foregoing description, and all differences within the scope of equivalents thereof should be construed as being included in the present disclosure.

## Claims

1. A compound represented by the following Chemical Formula 1:

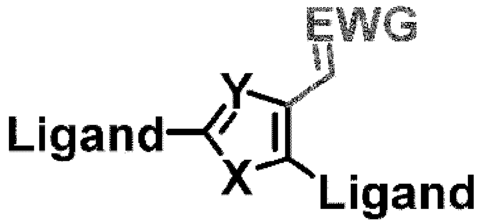

In Chemical Formula 1,

Ligands are reactive ligands which are identical to or different from each other, and each independently represents H, halogen or pseudohalogen, wherein the halogen is selected from the group consisting of Cl, Br and I, and the pseudohalogen is selected from the group consisting of OTf, $OPO(OR)_2$ and an acetoxy group,
X and Y are identical to or different from each other, and each independently chalcogens, wherein the chalcogen is selected from the group consisting of N, N-R, C, O, S, and Se,
EWG is selected from the group consisting of the following structures, and

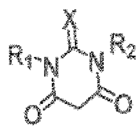 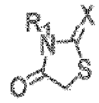 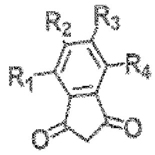 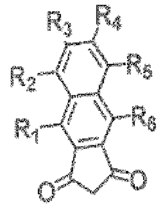 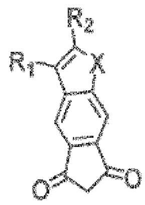

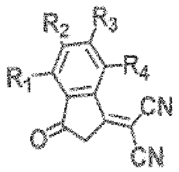 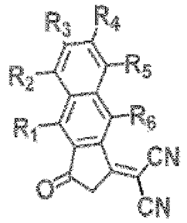 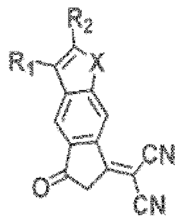 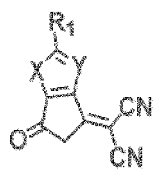 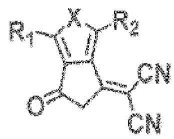

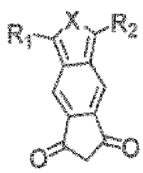 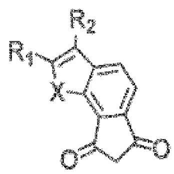 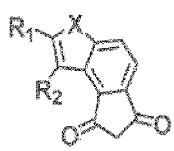 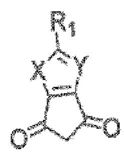 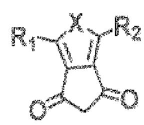

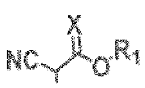 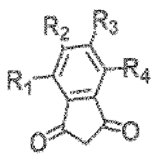 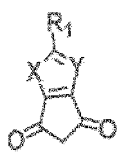 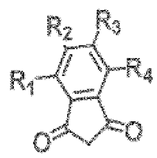 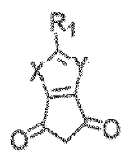

in Chemical Formula above, R and $R_1$ to $R_4$ are identical to or different from each other, and each independently selected from the group consisting of hydrogen; a cyano group; a halogen group; a substituted or unsubstituted alkyl group; a substituted or unsubstituted cycloalkyl group; a substituted or unsubstituted aryloxy group; a substituted or unsubstituted arylsulfoxy group; a substituted or unsubstituted boron group; a substituted or unsubstituted alkylamine group; a substituted or unsubstituted aralkylamine group; a substituted or unsubstituted arylamine group; a substituted or unsubstituted heteroarylamine group; a substituted or unsubstituted aryl group; a substituted or unsubstituted fluorenyl group; a substituted or unsubstituted carbazole group; a substituted or unsubstituted alkoxy group; a substituted or unsubstituted thionyl group; a substituted or unsubstituted silyl group; a substituted or unsubstituted alkenyl group; a substituted or unsubstituted ester group; a substituted or unsubstituted aromatic group; and substituted or unsubstituted heterocyclic groups containing one or more elements selected from the group consisting of N, O, S and Se, or two adjacent substituents may form a condensed ring.

**2.** The compound of claim 1, wherein the compound is selected from the following compounds:

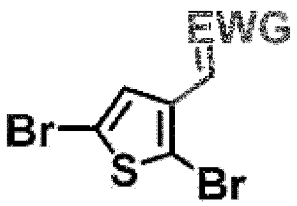
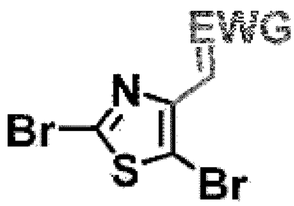
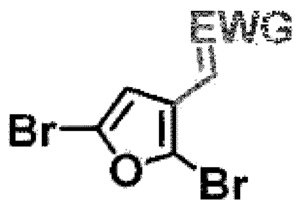

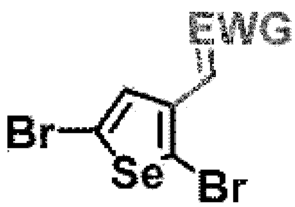
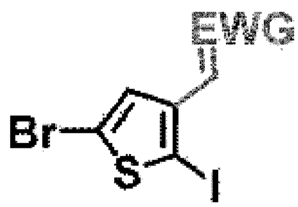

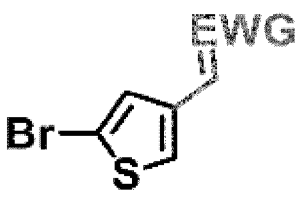
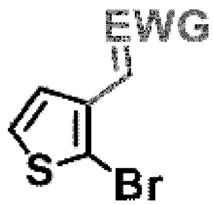
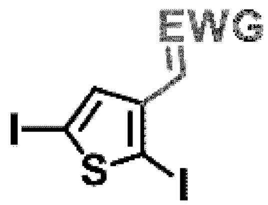

in the compounds, EWG is as defined in claim 1.

**3.** An organic semiconductor compound represented by the following Chemical Formula 2-1:

[Chemical Formula 2-1]

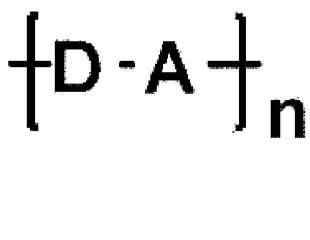

In Chemical Formula 2-1,

A is an electron acceptor unit, which is a compound of claim 1,
n is an integer of 1 to 10,000,
D is an electron donor selected from compounds represented by the following structures,

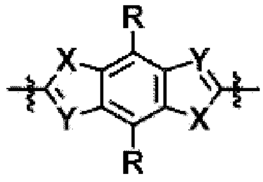

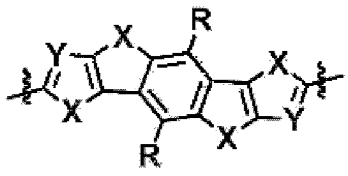

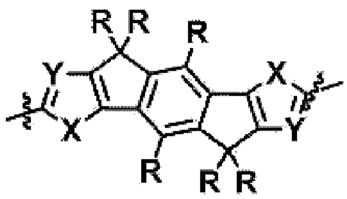

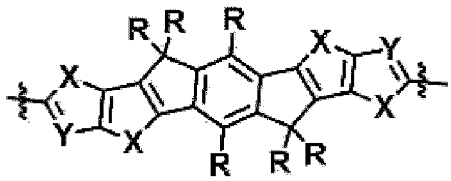

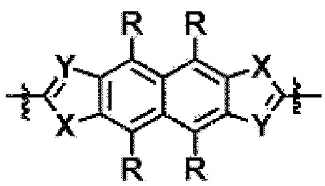

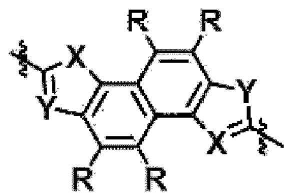

in the structures, X and Y are identical to or different from each other, and each independently chalcogens, wherein the chalcogen is selected from the group consisting of N, N-R, C, O, S, and Se, and
R is identical to or different from each other, and each independently selected from the group consisting of hydrogen; a cyano group; a halogen group; a substituted or unsubstituted alkyl group; a substituted or unsubstituted cycloalkyl group; a substituted or unsubstituted aryloxy group; a substituted or unsubstituted arylsulfoxy group; a substituted or unsubstituted boron group; a substituted or unsubstituted alkylamine group; a substituted or unsubstituted aralkylamine group; a substituted or unsubstituted arylamine group; a substituted or unsubstituted heteroarylamine group; a substituted or unsubstituted aryl group; a substituted or unsubstituted fluorenyl group; a substituted or unsubstituted carbazole group; a substituted or unsubstituted alkoxy group; a substituted or unsubstituted thionyl group; a substituted or unsubstituted silyl group; a substituted or unsubstituted alkenyl group; a substituted or unsubstituted ester group; a substituted or unsubstituted aromatic group; and substituted or unsubstituted heterocyclic groups containing one or more elements selected from the group consisting of N, O, S and Se, or two adjacent substituents may form a condensed ring.

4. The organic semiconductor compound of claim 3, wherein the organic semiconductor compound is selected from compounds represented by the following Chemical Formulas 3-1 to 3-6:

[Chemical Formula 3-1]

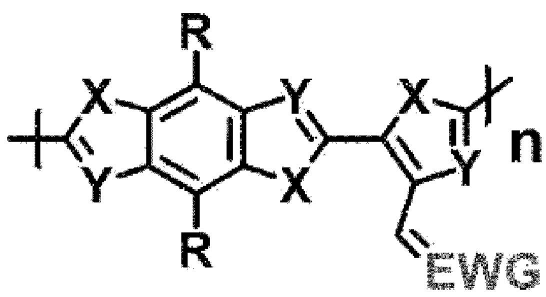

[Chemical Formula 3-2]

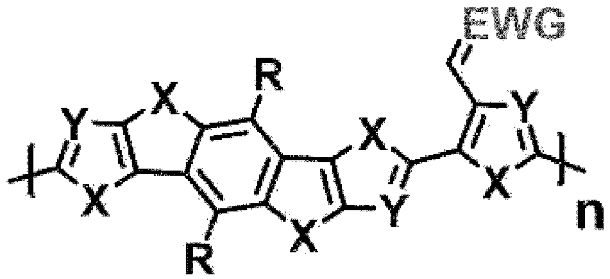

[Chemical Formula 3-3]

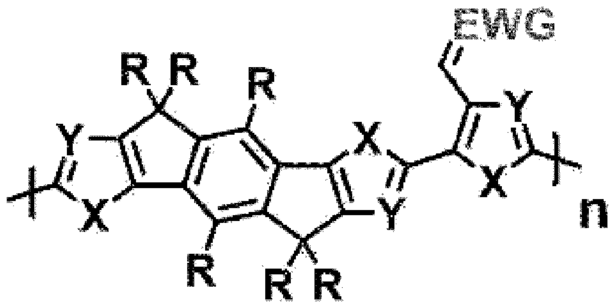

[Chemical Formula 3-4]

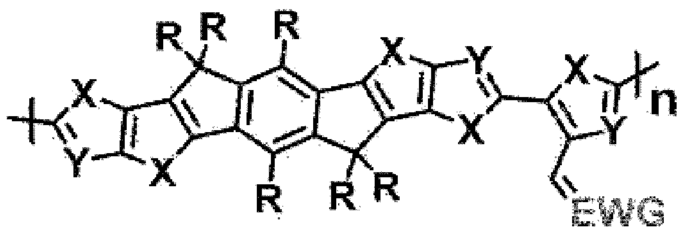

[Chemical Formula 3-5]

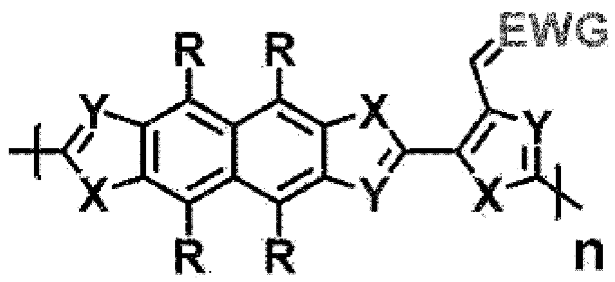

[Chemical Formula 3-6]

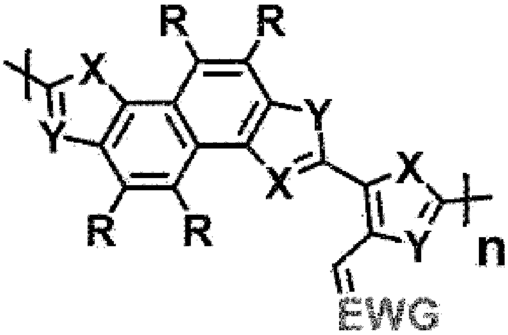

In Chemical Formulas 3-1 to 3-6 above,

n is an integer of 1 to 10,000, and
R, X, Y and EWG are as defined in claim 1.

**5.** An organic semiconductor compound represented by the following Chemical Formula 4-1:

[Chemical Formula 4-1]

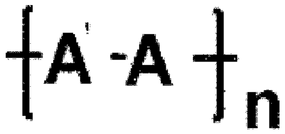

In Chemical Formula 4-1 above,

A is an electron acceptor unit, which is a compound of claim 1,
n is an integer of 1 to 10,000,
A' is an electron acceptor selected from compounds represented by the following structures,

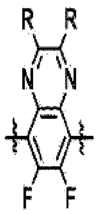
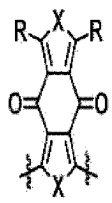
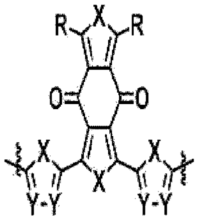
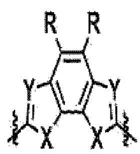
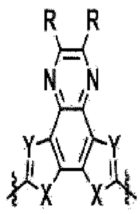

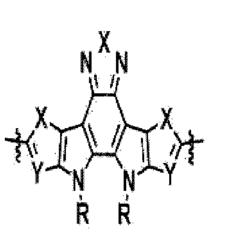
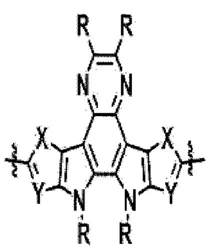
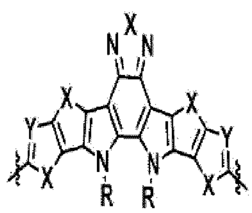
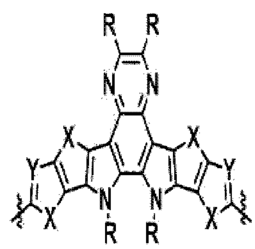

in the structures, X and Y are identical to or different from each other, and each independently chalcogens, wherein the chalcogen is selected from the group consisting of N, N-R, C, O, S, and Se, and
R is identical to or different from each other, and each independently selected from the group consisting of

hydrogen; a cyano group; a halogen group; a substituted or unsubstituted alkyl group; a substituted or unsubstituted cycloalkyl group; a substituted or unsubstituted aryloxy group; a substituted or unsubstituted arylsulfoxy group; a substituted or unsubstituted boron group; a substituted or unsubstituted alkylamine group; a substituted or unsubstituted aralkylamine group; a substituted or unsubstituted arylamine group; a substituted or unsubstituted heteroarylamine group; a substituted or unsubstituted aryl group; a substituted or unsubstituted fluorenyl group; a substituted or unsubstituted carbazole group; a substituted or unsubstituted alkoxy group; a substituted or unsubstituted thionyl group; a substituted or unsubstituted silyl group; a substituted or unsubstituted alkenyl group; a substituted or unsubstituted ester group; a substituted or unsubstituted aromatic group; and substituted or unsubstituted heterocyclic groups containing one or more elements selected from the group consisting of N, O, S and Se, or two adjacent substituents may form a condensed ring.

**6.** The organic semiconductor compound of claim 5, wherein the organic semiconductor compound is selected from compounds represented by the following Chemical Formulas 5-1 to 5-8:

[Chemical Formula 5-1]

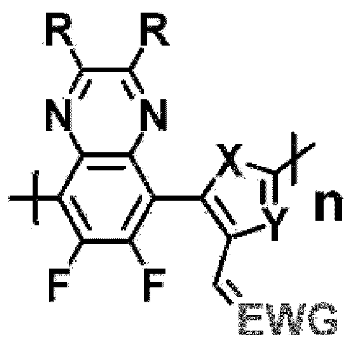

[Chemical Formula 5-2]

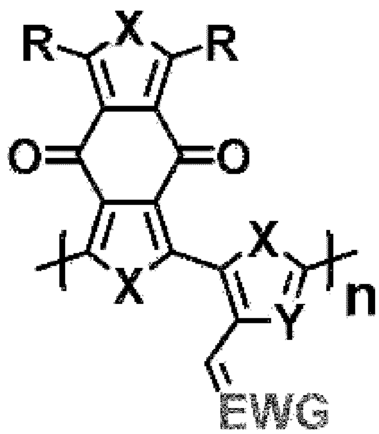

[Chemical Formula 5-3]

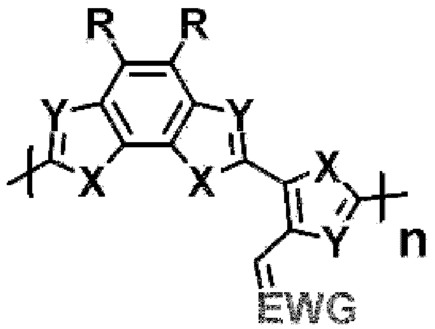

[Chemical Formula 5-4]

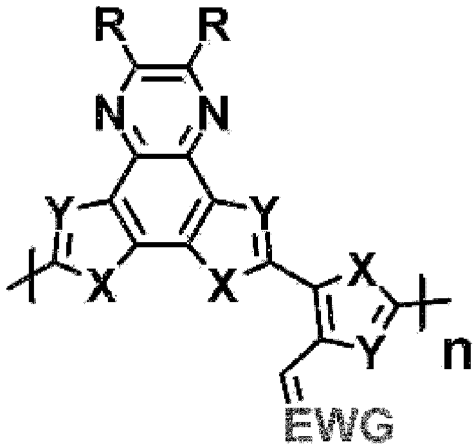

[Chemical Formula 5-5]

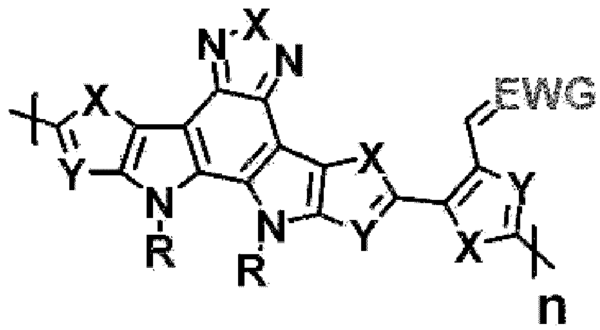

[Chemical Formula 5-6]

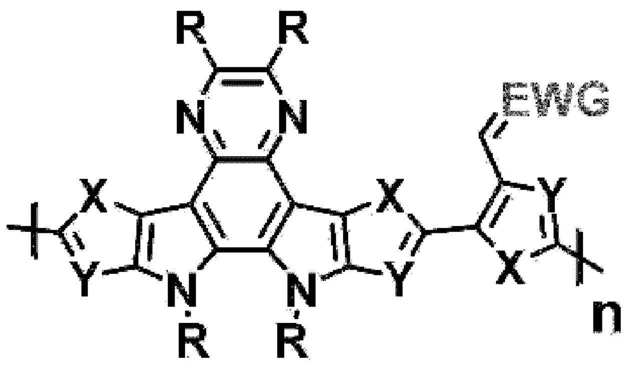

[Chemical Formula 5-7]

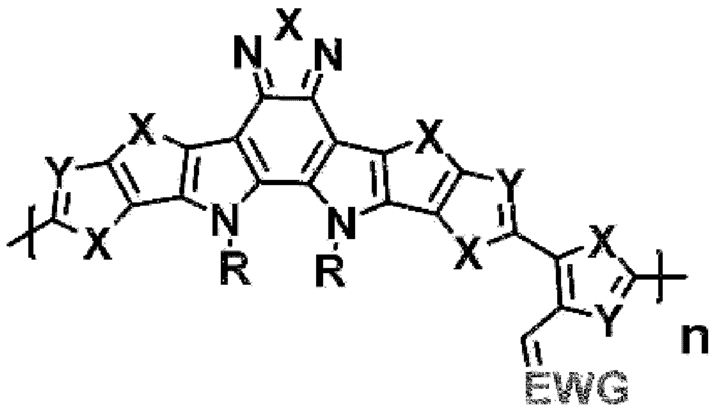

[Chemical Formula 5-8]

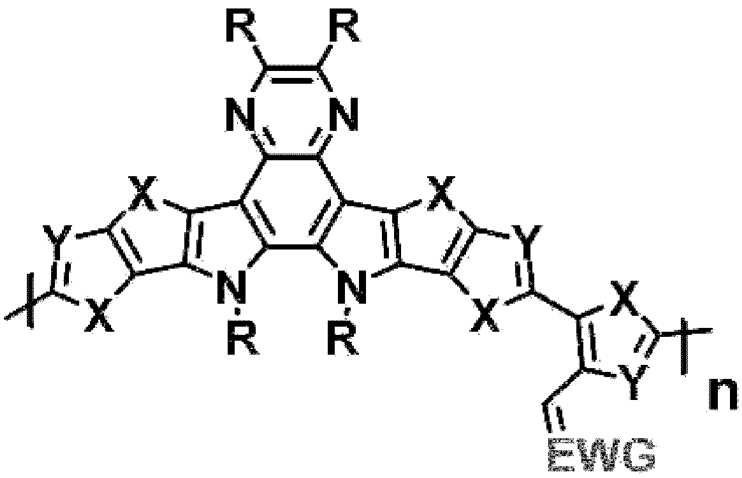

In Chemical Formulas 5-1 to 5-8 above,

n is an integer of 1 to 10,000, and
R, X, Y and EWG are as defined in claim 1.

**7.** An organic semiconductor compound selected from compounds represented by the following Chemical Formulas 6-1 to 6-5:

[Chemical Formula 6-1]

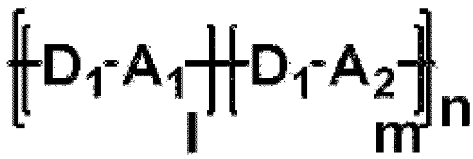

[Chemical Formula 6-2]

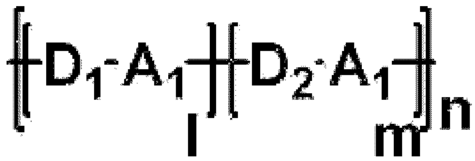

[Chemical Formula 6-3]

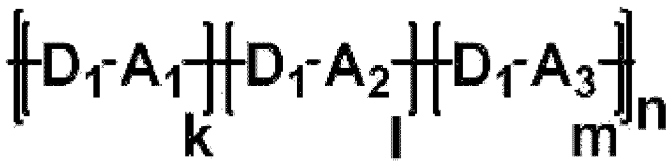

[Chemical Formula 6-4]

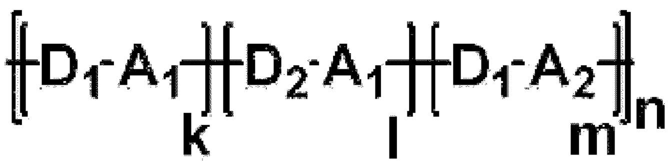

[Chemical Formula 6-5]

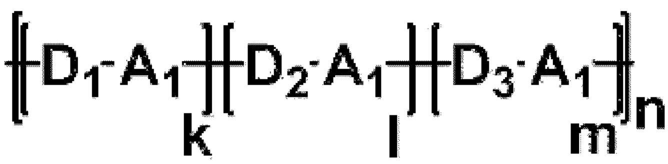

In Chemical Formulas 6-1 to 6-5 above,

$D_1$, $D_2$ and $D_3$ are identical to or different from each other and electron donors selected from compounds represented by the following structures,

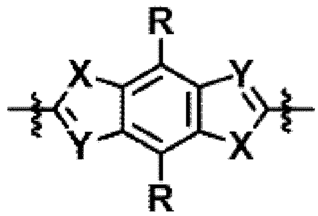
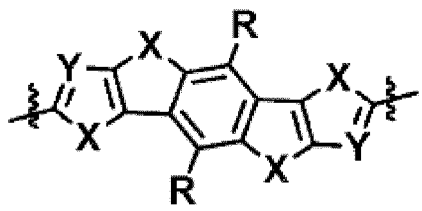
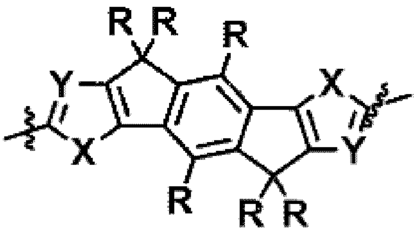
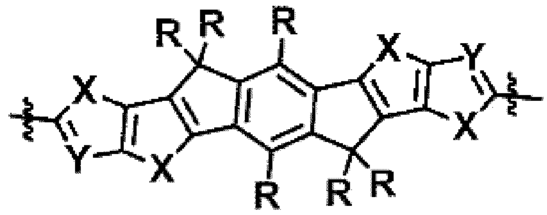
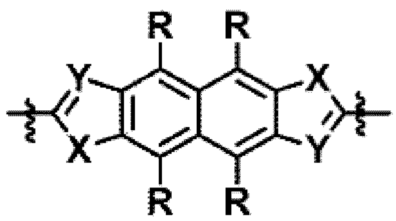
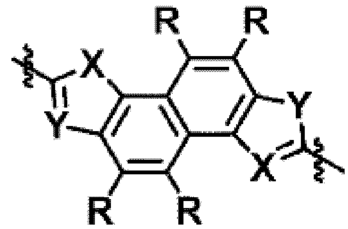

$A_1$, $A_2$, and $A_3$ are identical to or different from each other, and at least one thereof is an electron acceptor unit which is a compound of claim 1, and the rest are relative electron acceptors selected from compounds represented by the following structures,

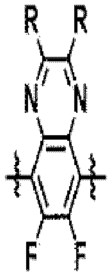
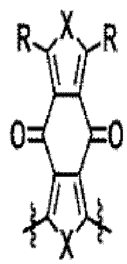
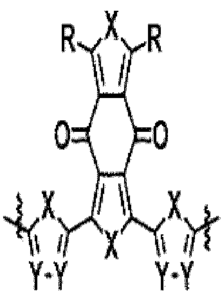
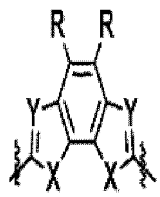
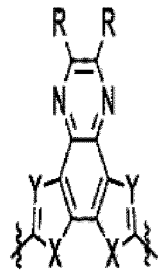
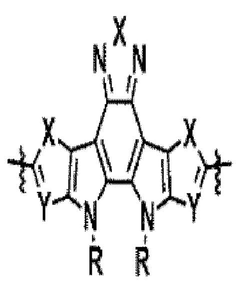
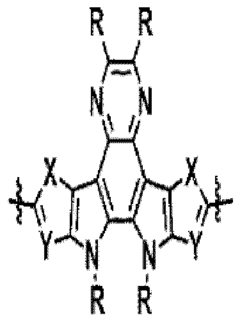
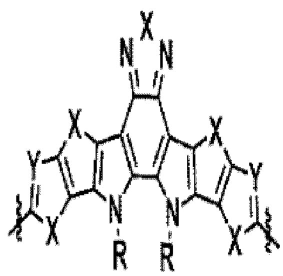
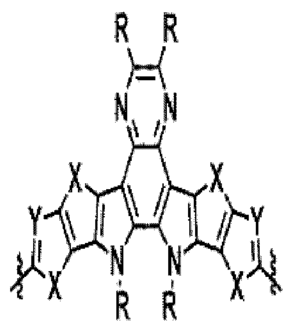

in the structures, X and Y are identical to or different from each other, and each independently chalcogens, wherein the chalcogen is selected from the group consisting of N, N-R, C, O, S, and Se,
R is identical to or different from each other, and each independently selected from the group consisting of hydrogen; a cyano group; a halogen group; a substituted or unsubstituted alkyl group; a substituted or

unsubstituted cycloalkyl group; a substituted or unsubstituted aryloxy group; a substituted or unsubstituted arylsulfoxy group; a substituted or unsubstituted boron group; a substituted or unsubstituted alkylamine group; a substituted or unsubstituted aralkylamine group; a substituted or unsubstituted arylamine group; a substituted or unsubstituted heteroarylamine group; a substituted or unsubstituted aryl group; a substituted or unsubstituted fluorenyl group; a substituted or unsubstituted carbazole group; a substituted or unsubstituted alkoxy group; a substituted or unsubstituted thionyl group; a substituted or unsubstituted silyl group; a substituted or unsubstituted alkenyl group; a substituted or unsubstituted ester group; a substituted or unsubstituted aromatic group; and substituted or unsubstituted heterocyclic groups containing one or more elements selected from the group consisting of N, O, S and Se, or two adjacent substituents may form a condensed ring,
k represents a mole fraction, which is a real number with $0 \leq k < 1$,
l represents a mole fraction, which is a real number with $0 \leq l < 1$,
m represents a mole fraction, which is a real number with $0 < m \leq 1$,

$$k + l + m = 1,$$

and
n is an integer of 1 to 10,000.

**8.** The organic semiconductor compound of claim 7, wherein the organic semiconductor compound is selected from compounds represented by the following Chemical Formulas 7-1 to 7-5:

[Chemical Formula 7-1]

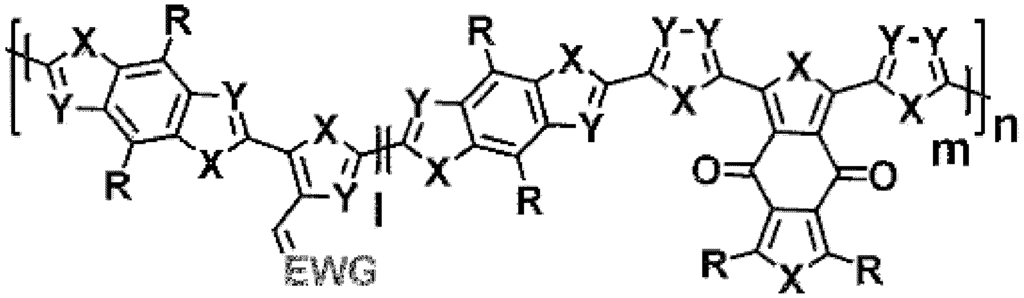

[Chemical Formula 7-2]

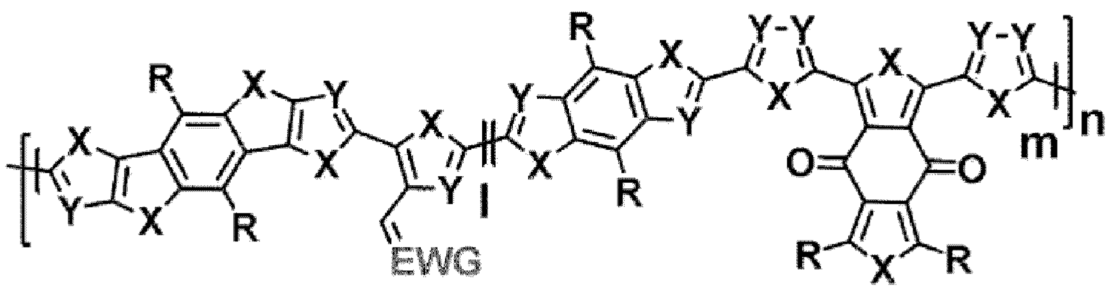

[Chemical Formula 7-3]

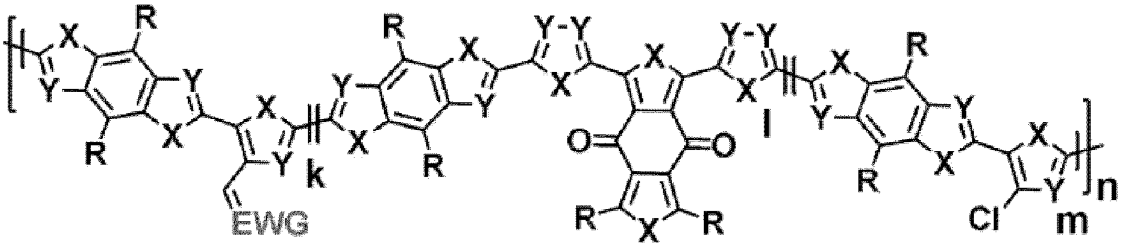

[Chemical Formula 7-4]

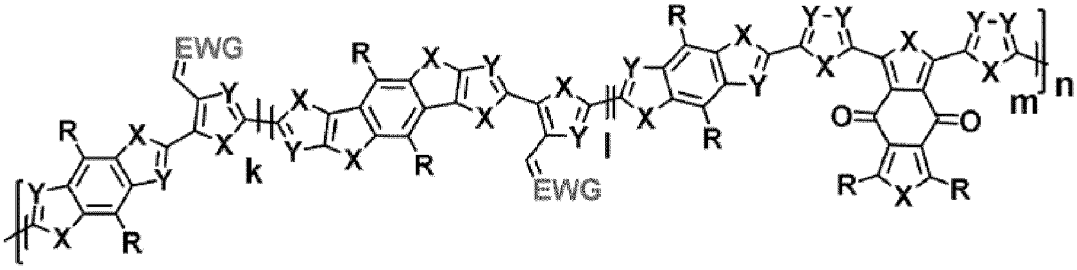

[Chemical Formula 7-5]

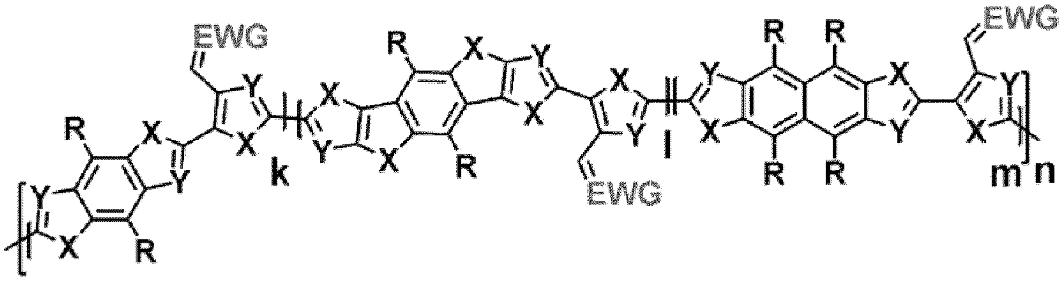

In Chemical Formulas 7-1 to 7-5 above,

k, l, m and n are as defined in claim 7, and
R, X, Y and EWG are as defined in claim 1.

**9.** An organic semiconductor compound selected from compounds represented by the following Chemical Formulas 8-1 to 8-4:

[Chemical Formula 8-1]

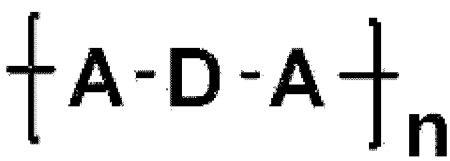

[Chemical Formula 8-2]

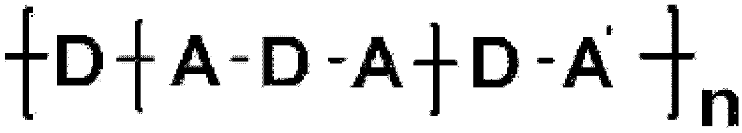

[Chemical Formula 8-3]

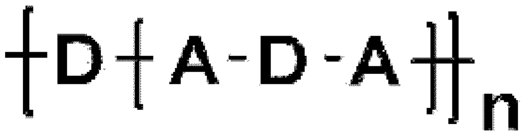

[Chemical Formula 8-4]

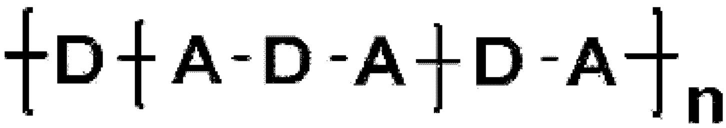

In Chemical Formulas 8-1 to 8-4 above,

A is an electron acceptor unit, which is a compound of claim 1,
n is an integer of 1 to 10,000,
A' is a relative electron acceptor selected from compounds represented by the following structures,

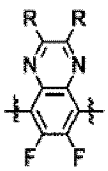
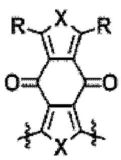
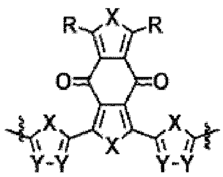
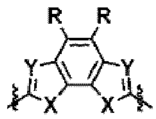
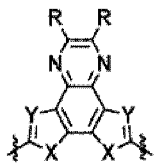

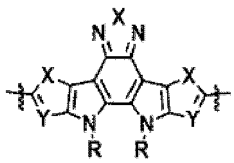
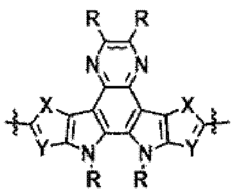
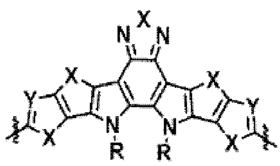
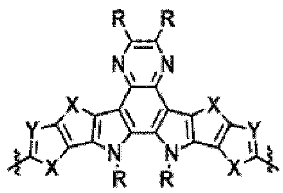

D is an electron donor selected from compounds represented by the following structures,

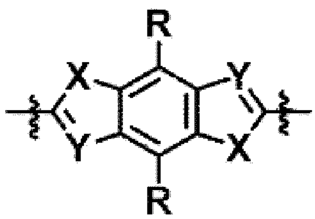
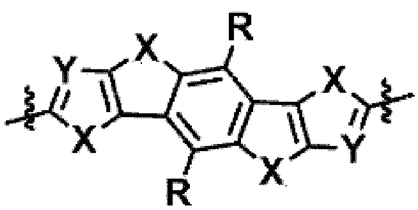

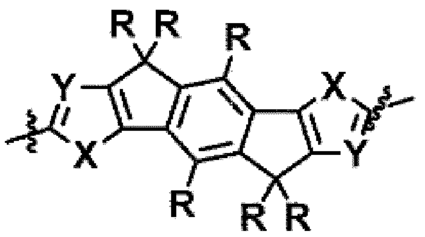

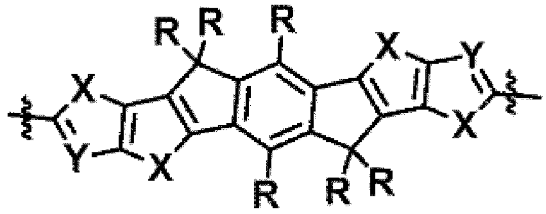

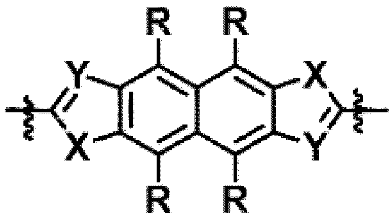

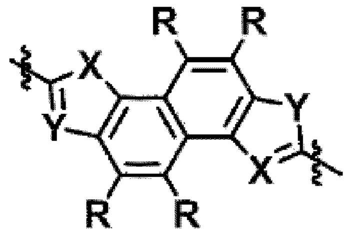

in the structures, X and Y are identical to or different from each other, and each independently chalcogens, wherein the chalcogen is selected from the group consisting of N, N-R, C, O, S, and Se, and
R is identical to or different from each other, and each independently selected from the group consisting of hydrogen; a cyano group; a halogen group; a substituted or unsubstituted alkyl group; a substituted or unsubstituted cycloalkyl group; a substituted or unsubstituted aryloxy group; a substituted or unsubstituted arylsulfoxy group; a substituted or unsubstituted boron group; a substituted or unsubstituted alkylamine group; a substituted or unsubstituted aralkylamine group; a substituted or unsubstituted arylamine group; a substituted or unsubstituted heteroarylamine group; a substituted or unsubstituted aryl group; a substituted or unsubstituted fluorenyl group; a substituted or unsubstituted carbazole group; a substituted or unsubstituted alkoxy group; a substituted or unsubstituted thionyl group; a substituted or unsubstituted silyl group; a substituted or unsubstituted alkenyl group; a substituted or unsubstituted ester group; a substituted or unsubstituted aromatic group; and substituted or unsubstituted heterocyclic groups containing one or more elements selected from the group consisting of N, O, S and Se, or two adjacent substituents may form a condensed ring.

**10.** The organic semiconductor compound of claim 9, wherein the compound represented by Chemical Formula 8-1 above is selected from compounds having the following structures:

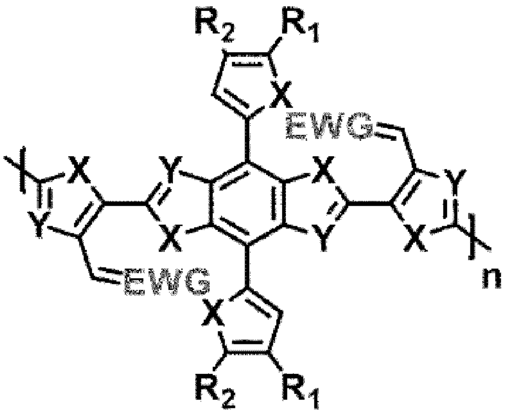

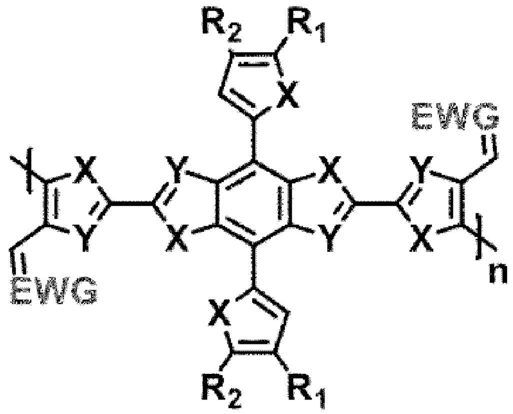

in the structures, $R_1$, $R_2$, X, Y and EWG are as defined in claim 1.

**11.** The organic semiconductor compound of claim 9, wherein the compound represented by Chemical Formula 8-2 above is selected from compounds having the following structures:

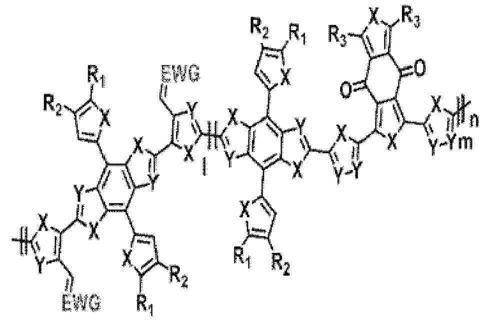

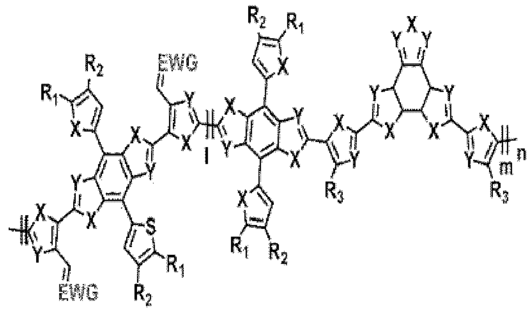

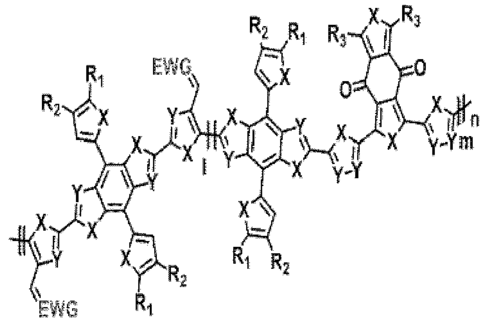

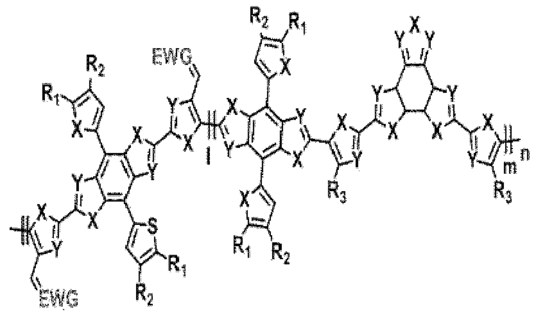

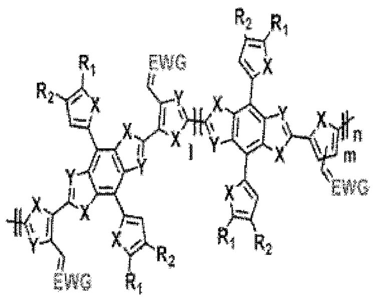

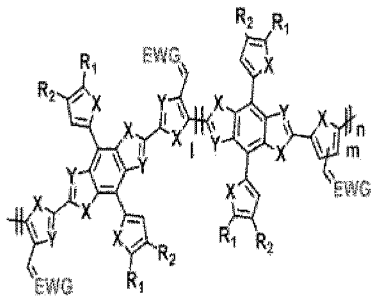

In the structures, l, m and n are as defined in claim 7, and $R_1$, $R_2$, $R_3$, X, Y and EWG are as defined in claim 1.

**12.** An organic solar cell comprising an organic semiconductor compound according to any one of claims 3 to 11.

**13.** An organic electronic device comprising an organic semiconductor compound according to any one of claims 3 to 11.

【Fig. 1】

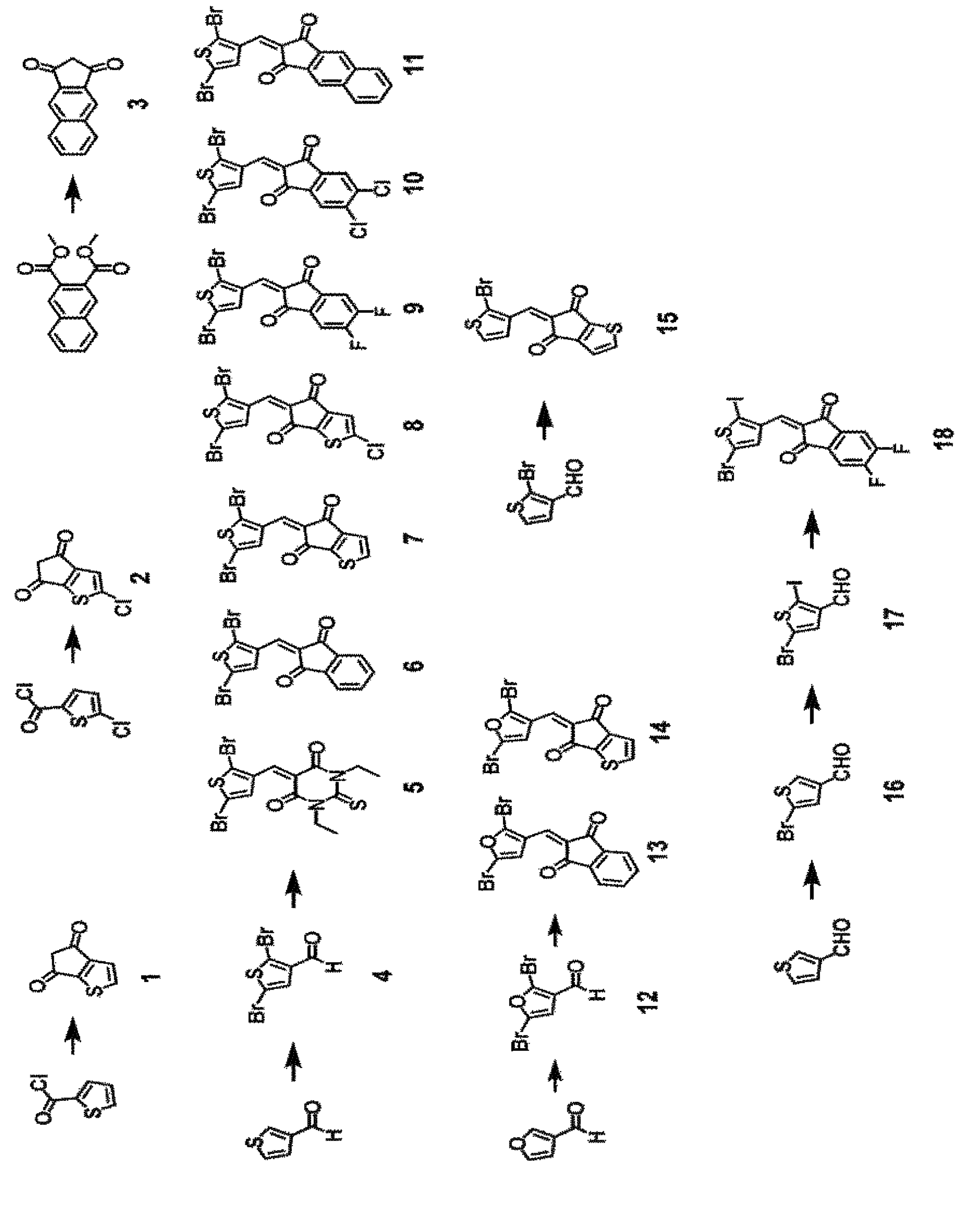

1 2 3 4 5 6 7 8 9 10 11 12 13 14 15 16 17 18

【Fig. 2】

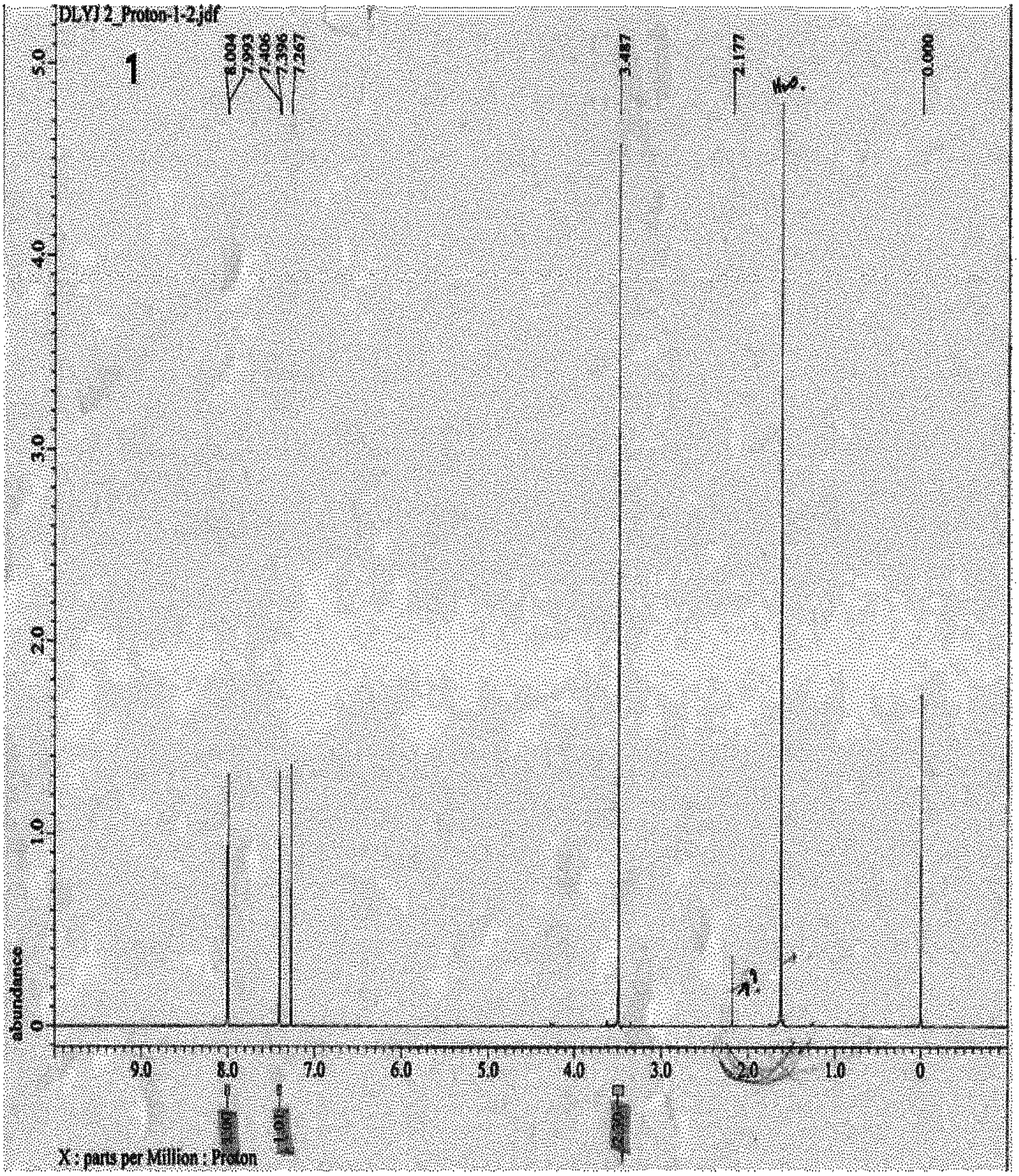
DLVJ 2_Proton-1-2.jdf
1
8.004
7.993
7.406
7.396
7.267
3.487
2.177
0.000
abundance
0
1.0
2.0
3.0
4.0
5.0
9.0
8.0
7.0
6.0
5.0
4.0
3.0
2.0
1.0
0
X : parts per Million : Proton

【Fig. 3】

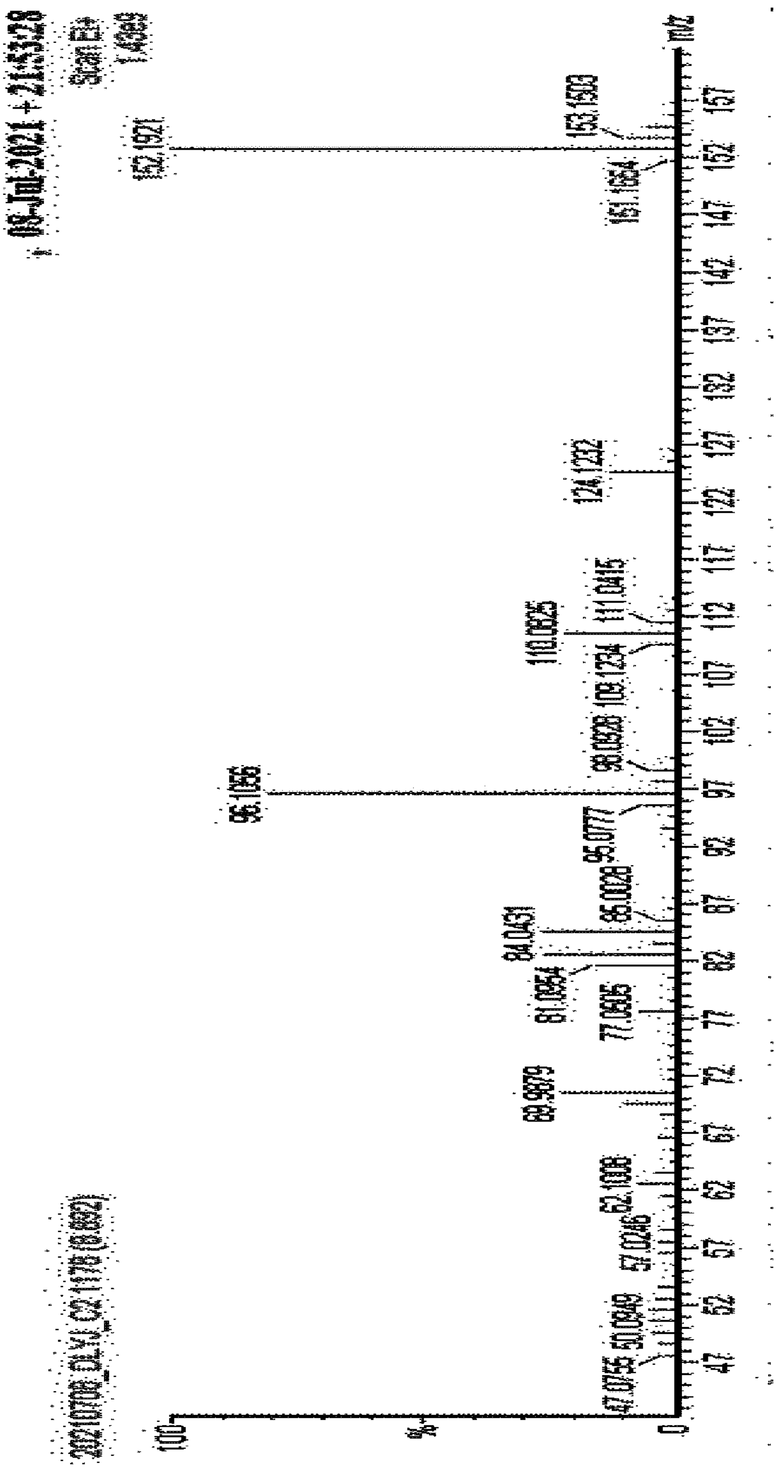
20210708_DLYJ_C2 1176 (8.892)
08-Jul-2021 + 21:53:28
Scan EI+
1.43e9
100
%
0
m/z
152.1921
153.1503
151.1654
124.1232
110.0625
111.0415
109.1234
96.1056
98.0928
95.0777
95.0028
84.0431
81.0954
77.0605
69.9879
62.1006
57.0246
50.0949
47.0755

【Fig. 4】

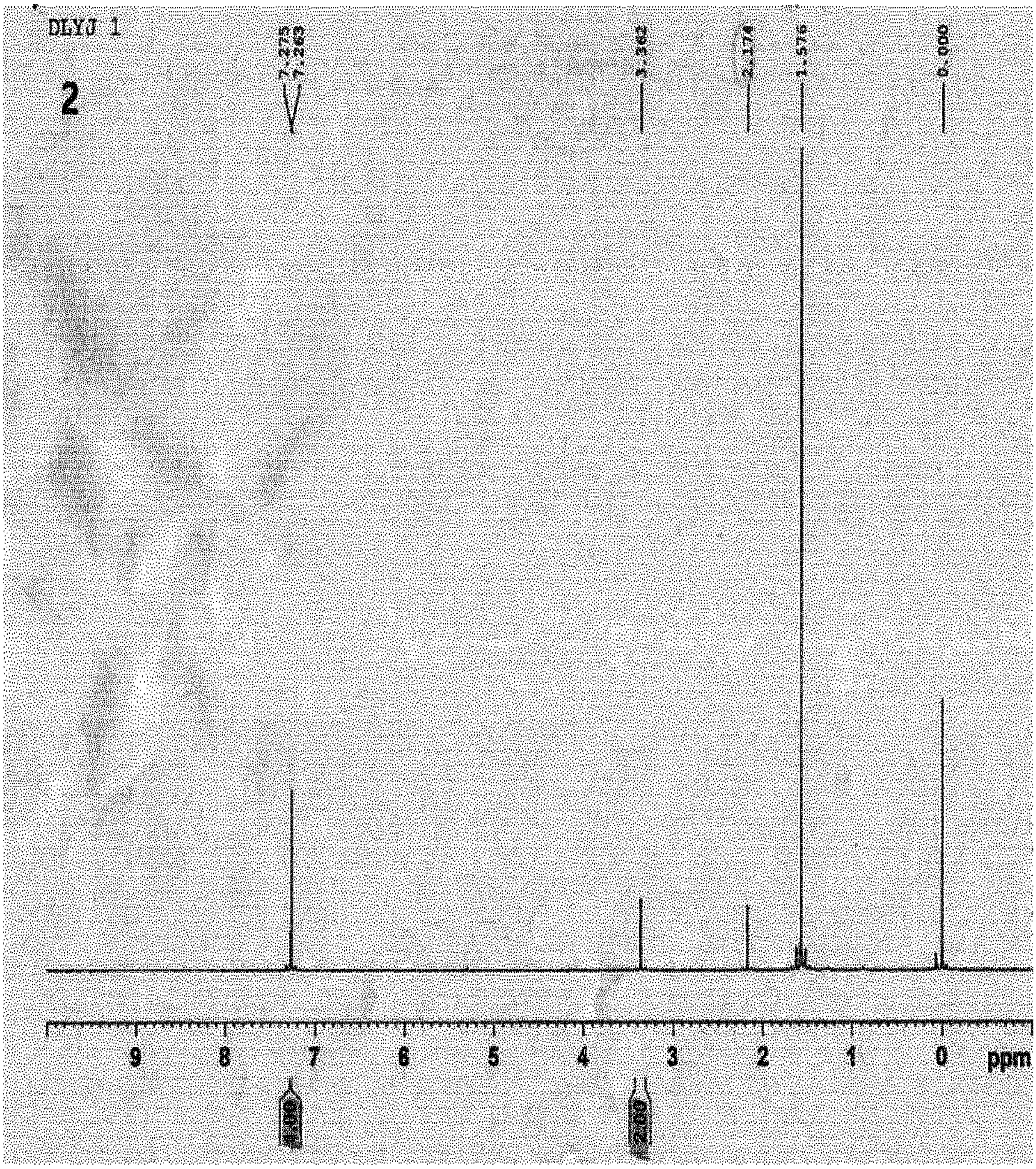

【Fig. 5】

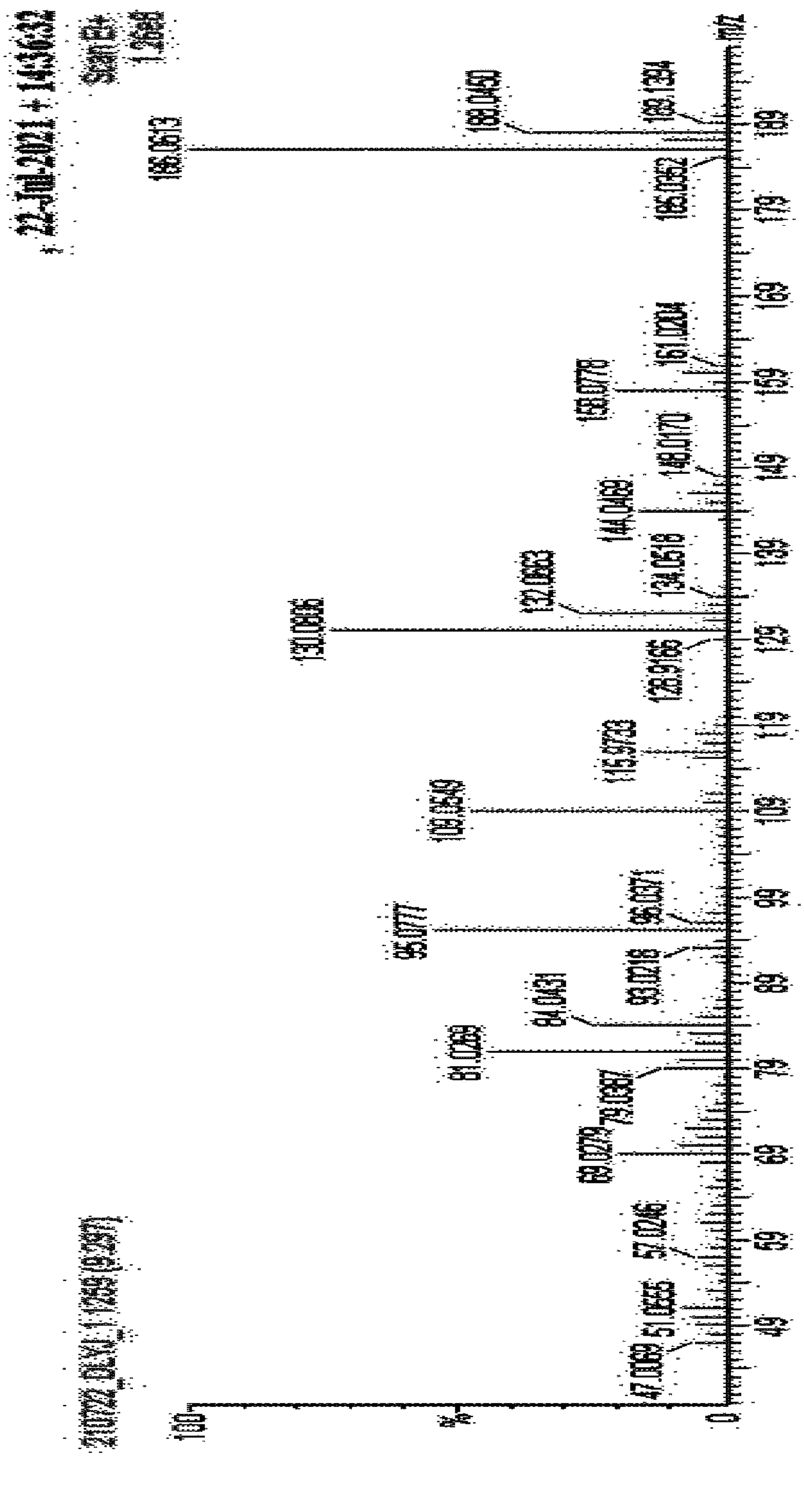
22-Jul-2021 + 14:36:32
Scan EI+
1.26e6
186.0613
188.0450
189.1354
185.0352
161.0204
158.0778
148.0170
144.0469
134.0518
132.0663
130.0806
128.9165
115.9733
109.0549
95.0777
96.0371
93.0218
84.0431
81.0269
79.0387
69.0279
57.0246
51.0555
47.0069
100
%
0
m/z

【Fig. 6】

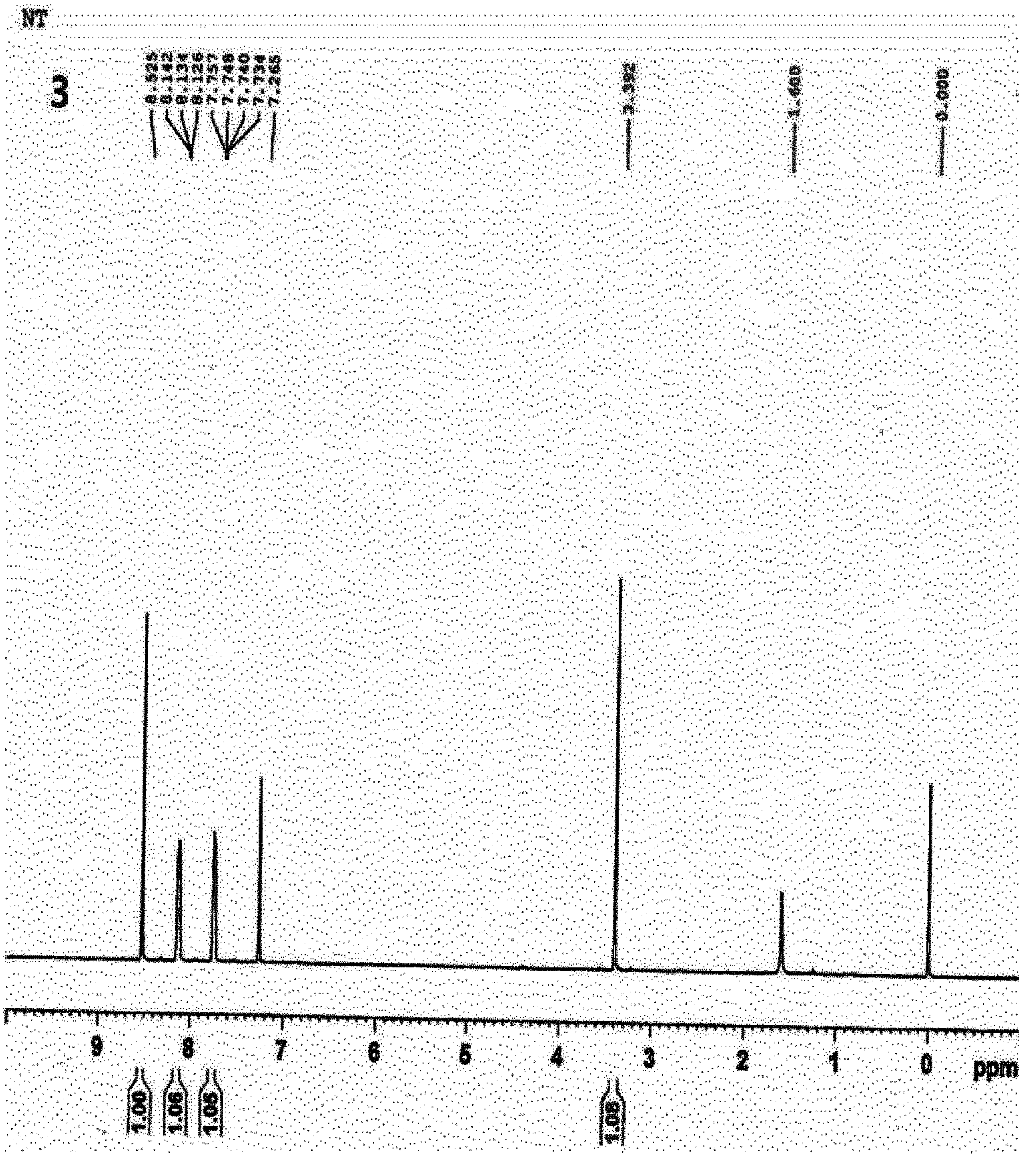
NT
3
8.525
8.142
8.134
8.126
7.757
7.748
7.740
7.734
7.265
3.392
1.600
0.000
9
8
7
6
5
4
3
2
1
0
ppm
1.00
1.06
1.05
1.08

【Fig. 7】

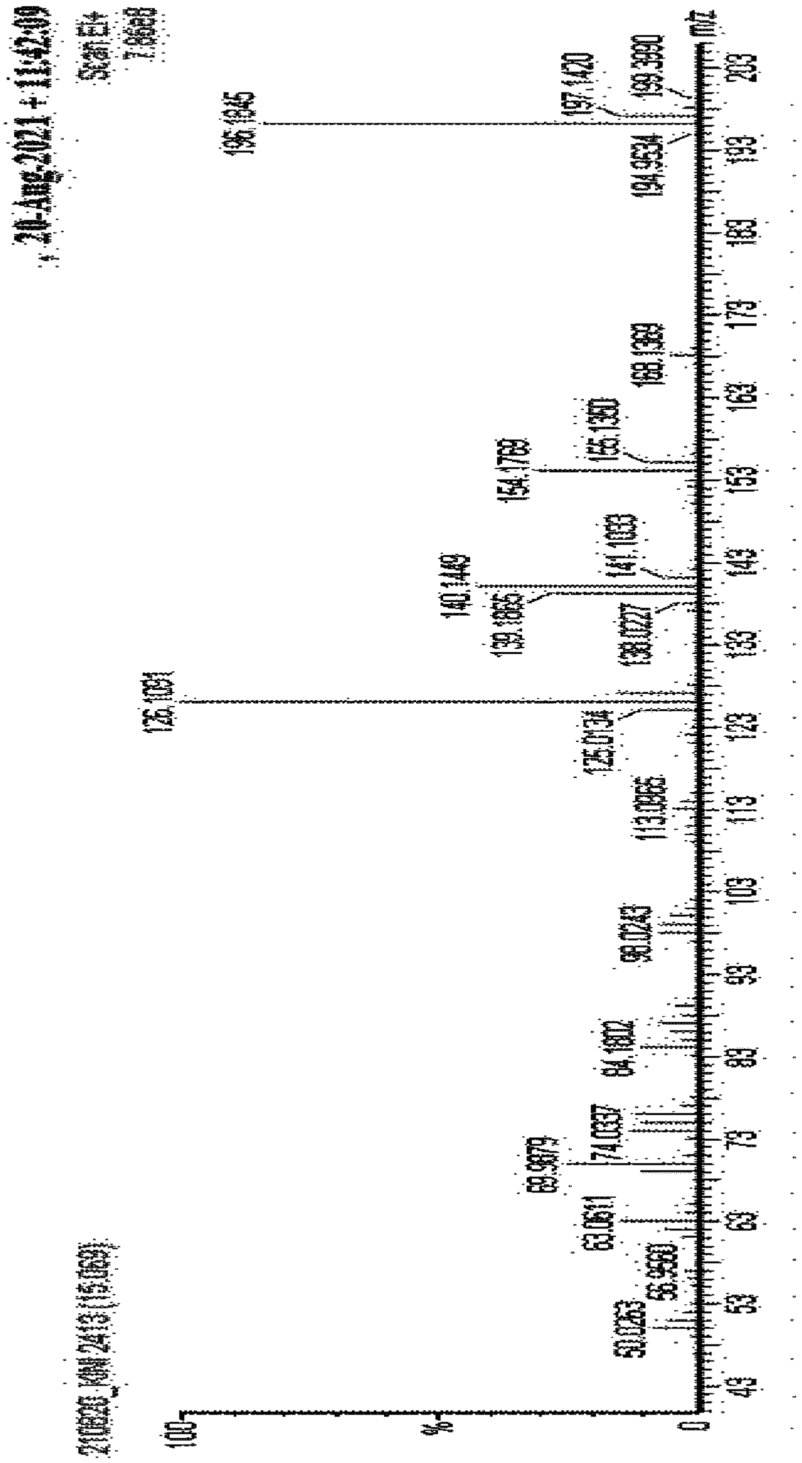
20-Aug-2021 + 11:42:09
Scan EI+
7.96e8
100
%
0
196.1945
197.1420
199.3990
194.9534
168.1369
155.1380
154.1769
141.1033
140.1449
139.1865
138.0227
126.1031
125.0134
113.0965
98.0243
84.1802
74.0337
69.9879
63.0611
56.9560
50.0263
43
53
63
73
83
93
103
113
123
133
143
153
163
173
183
193
203
m/z

【Fig. 8】

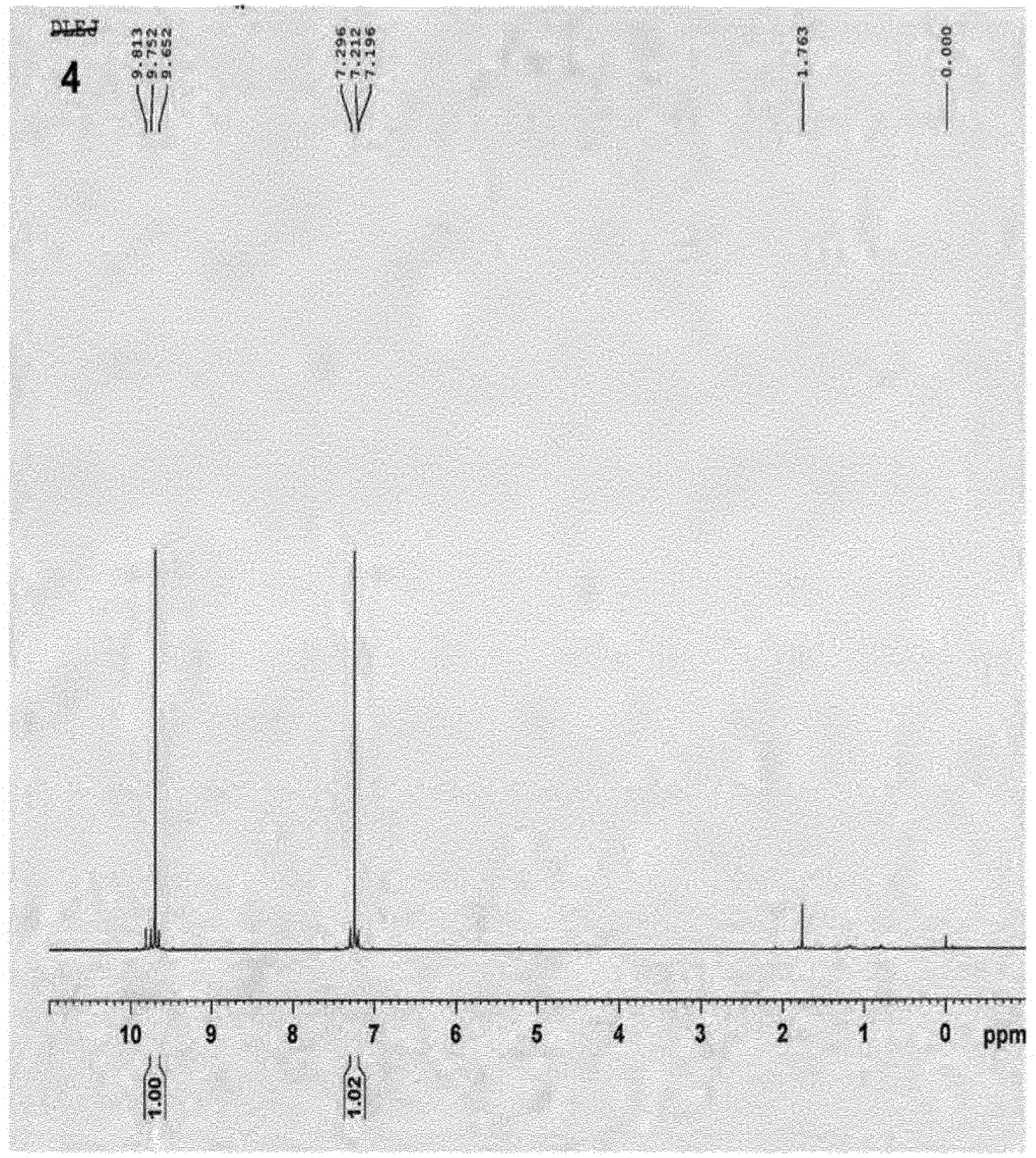
DLEJ
4
9.813
9.752
9.652
7.296
7.212
7.196
1.763
0.000
10
9
8
7
6
5
4
3
2
1
0
ppm
1.00
1.02

【Fig. 9】

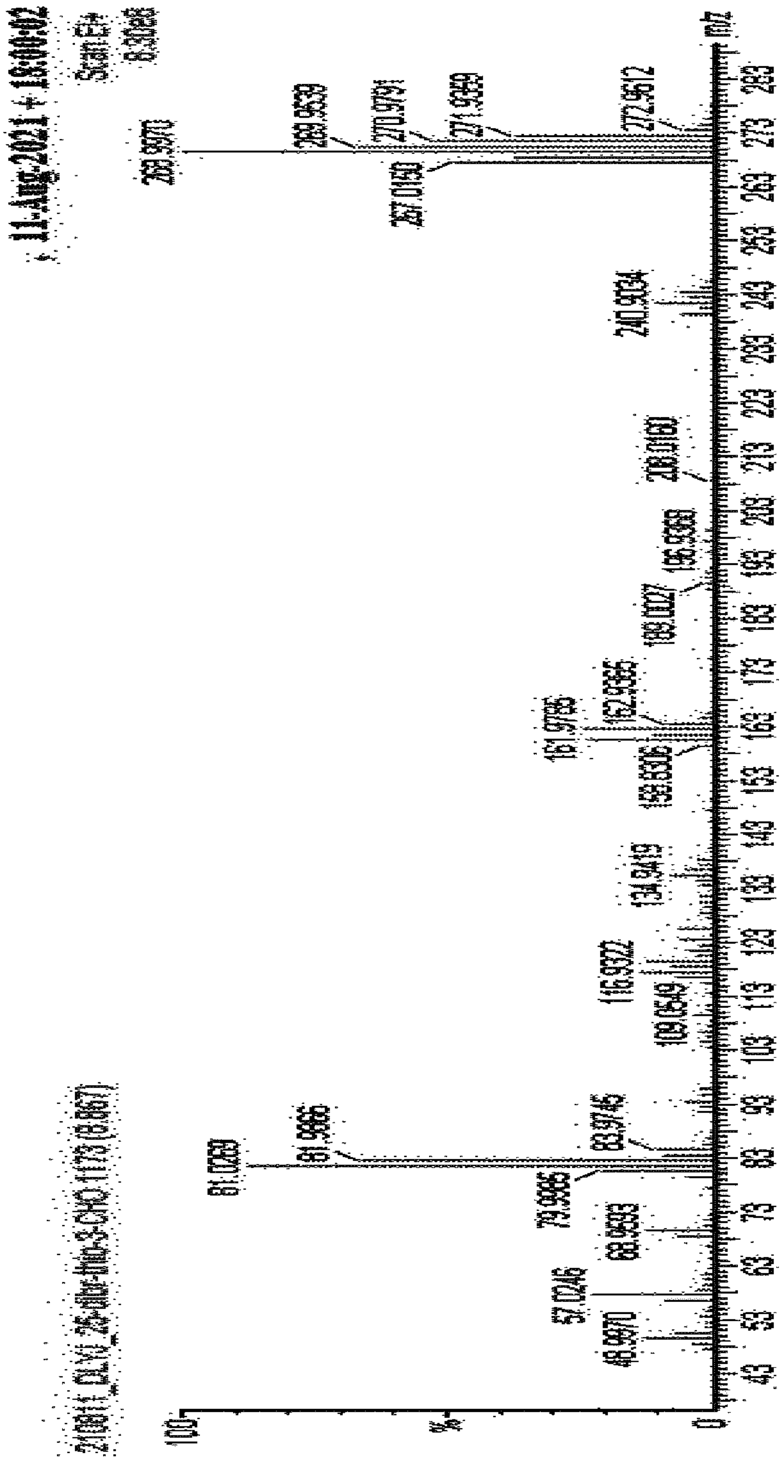

【Fig. 10】

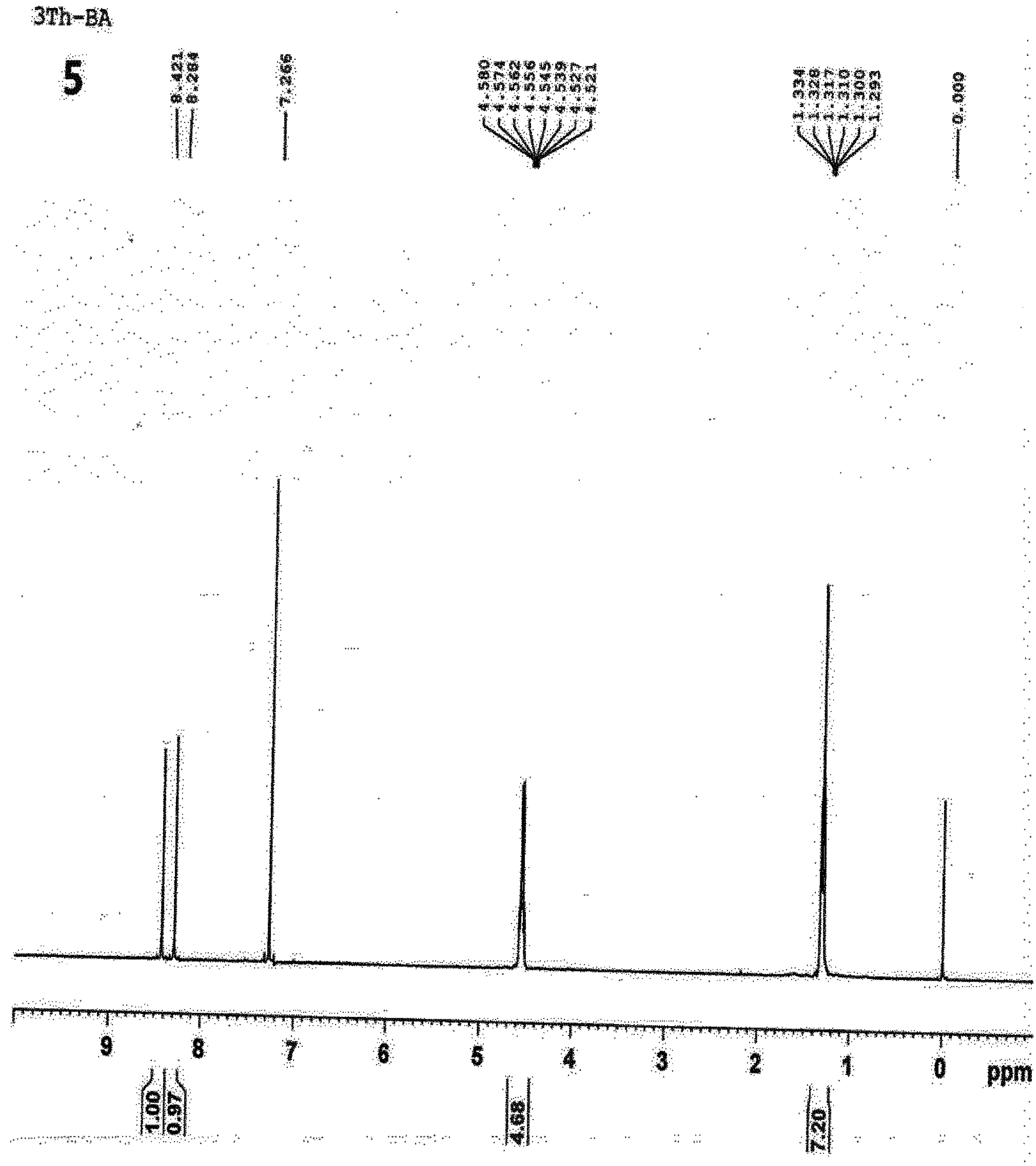
3Th-BA
5
8.421
8.284
7.266
4.580
4.574
4.562
4.556
4.545
4.539
4.527
4.521
1.334
1.328
1.317
1.310
1.300
1.293
0.000
9
8
7
6
5
4
3
2
1
0
ppm
1.00
0.97
4.68
7.20

【Fig. 11】

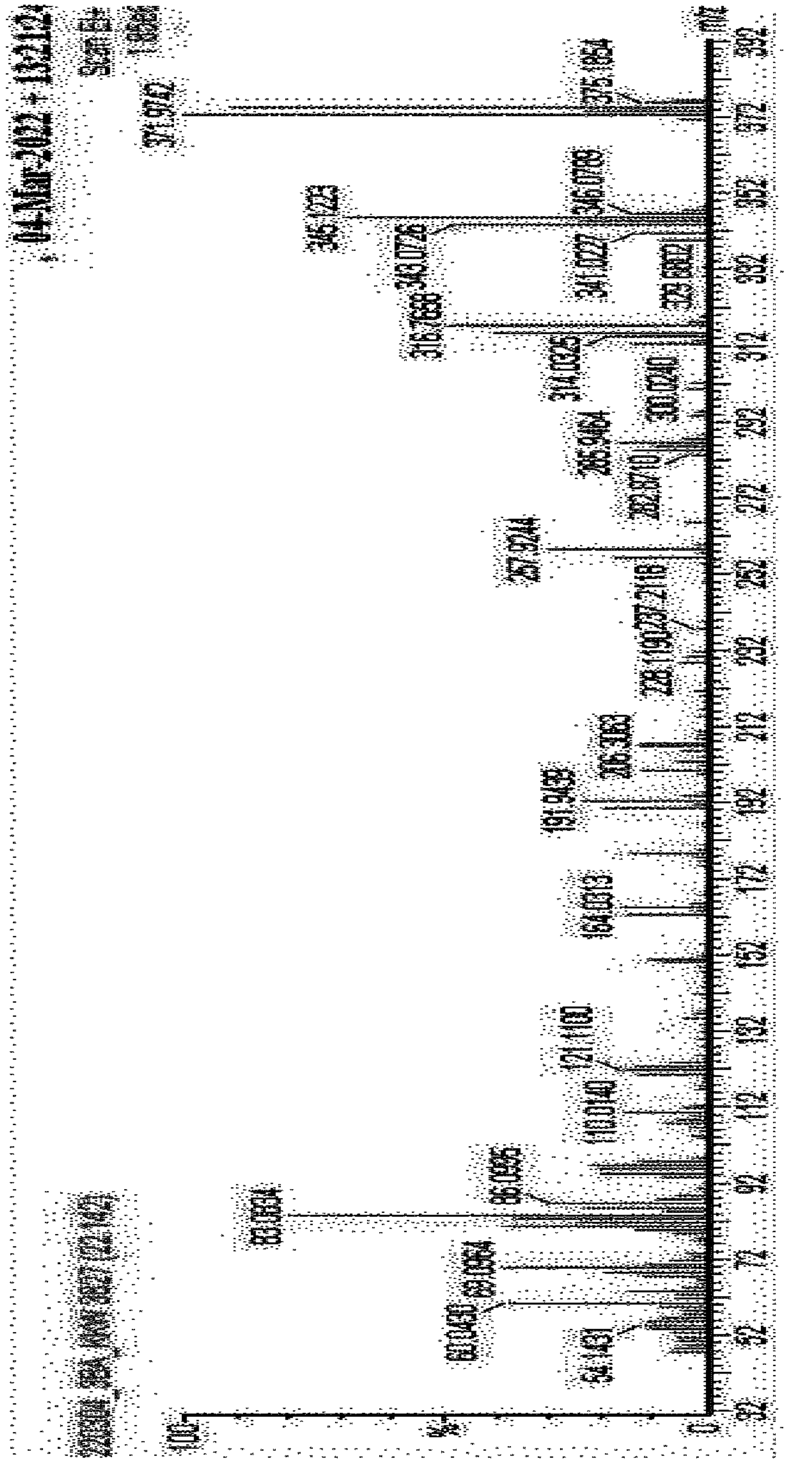

【Fig. 12】

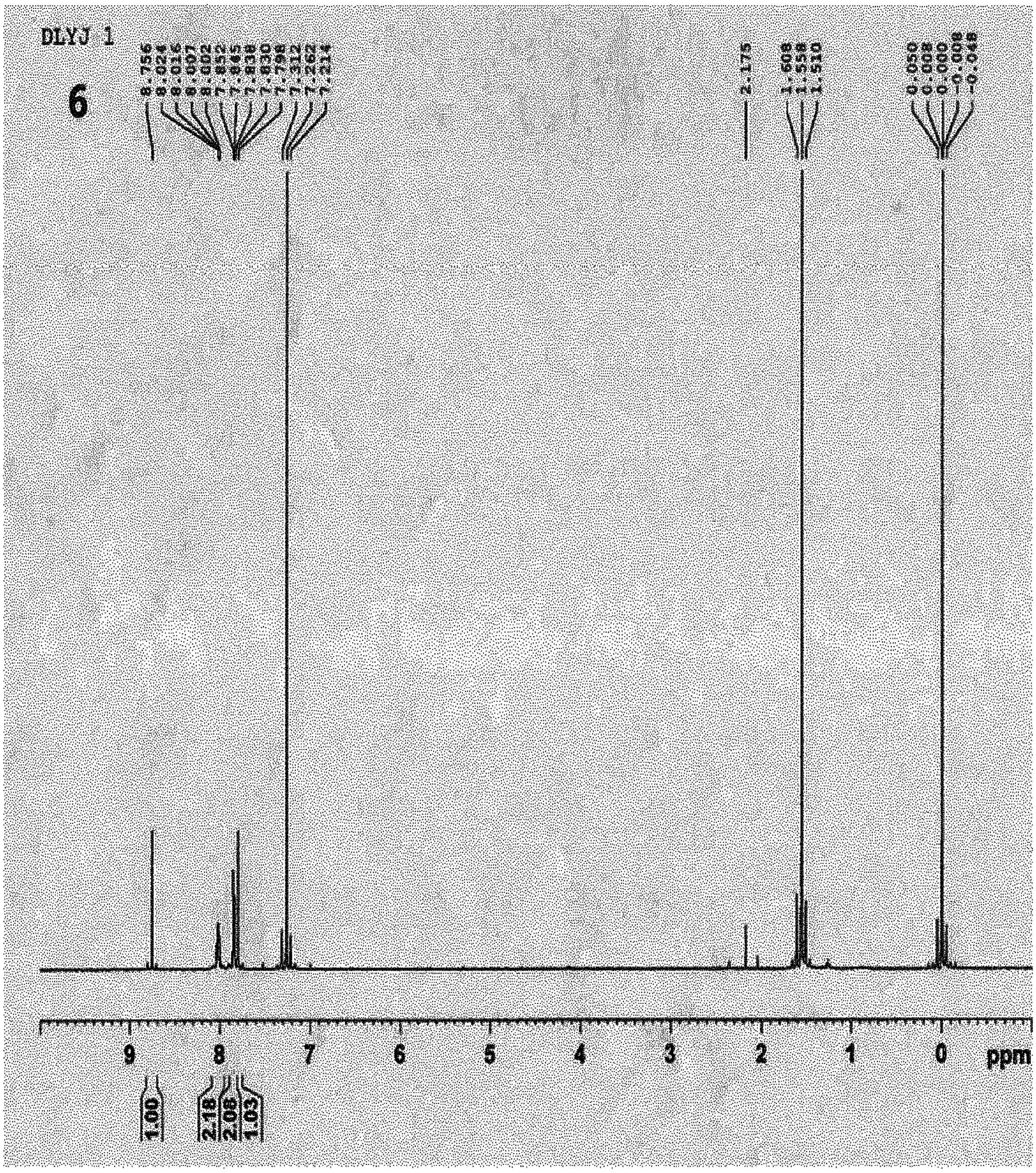
DLYJ 1
6
8.756
8.024
8.016
8.007
8.002
7.852
7.845
7.838
7.830
7.798
7.312
7.262
7.214
2.175
1.608
1.558
1.510
0.050
0.008
0.000
-0.008
-0.048
9
8
7
6
5
4
3
2
1
0
ppm
1.00
2.18
2.08
1.03

【Fig. 13】

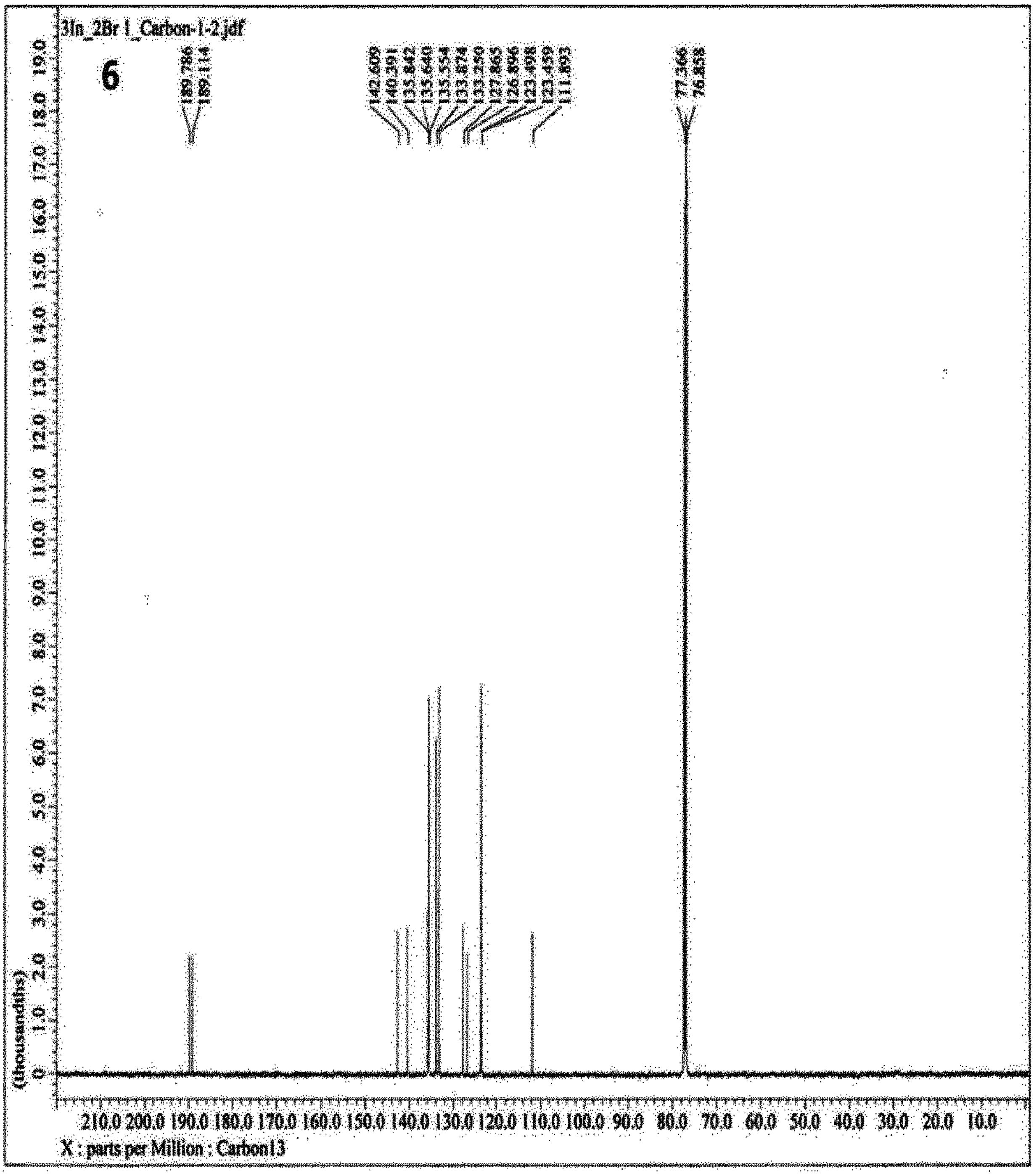
3In_2Br 1_Carbon-1-2.jdf
6
189.786
189.114
142.609
140.391
135.842
135.640
135.554
133.874
133.250
127.865
126.896
123.498
123.459
111.893
77.366
76.858
(thousandths)
0
1.0
2.0
3.0
4.0
5.0
6.0
7.0
8.0
9.0
10.0
11.0
12.0
13.0
14.0
15.0
16.0
17.0
18.0
19.0
210.0 200.0 190.0 180.0 170.0 160.0 150.0 140.0 130.0 120.0 110.0 100.0 90.0 80.0 70.0 60.0 50.0 40.0 30.0 20.0 10.0
X : parts per Million : Carbon13

【Fig. 14】

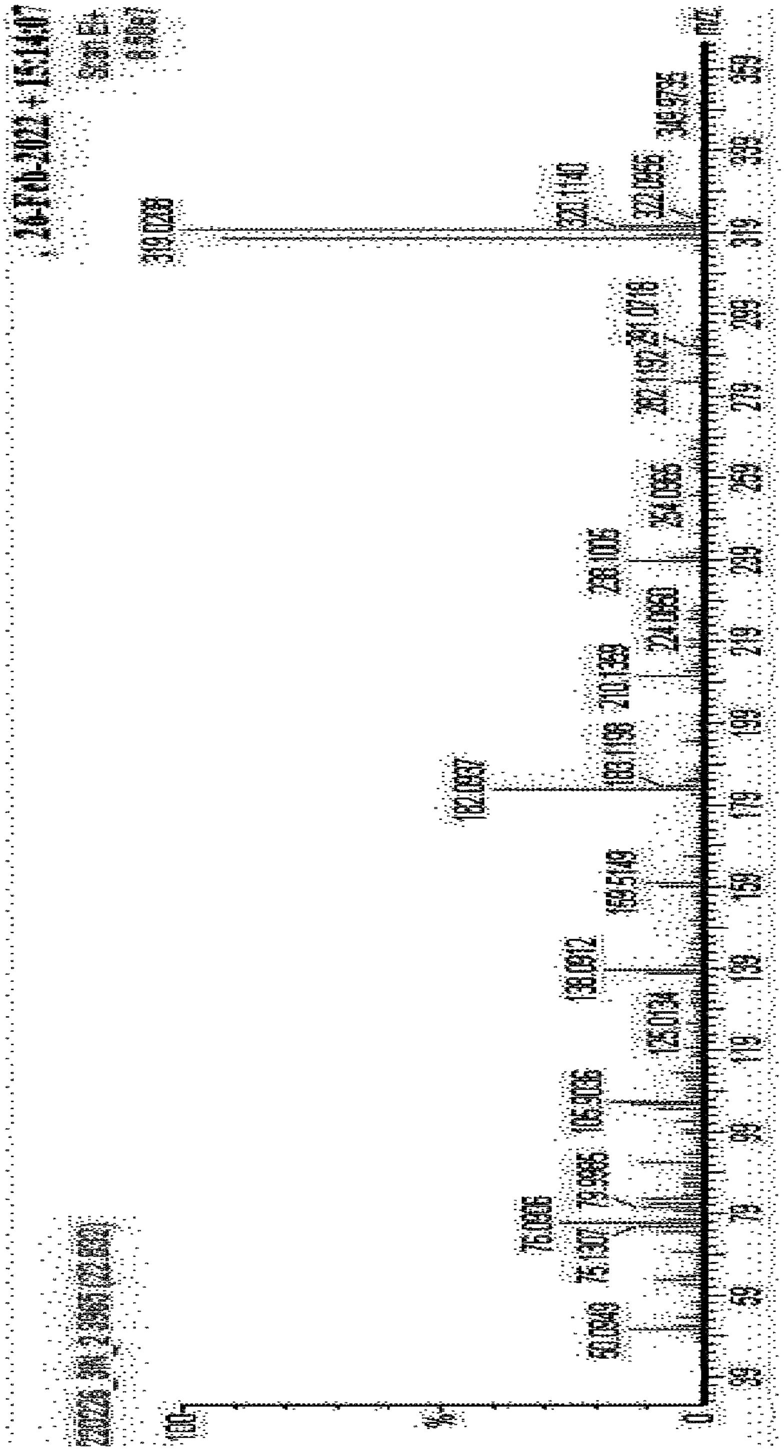

【Fig. 15】

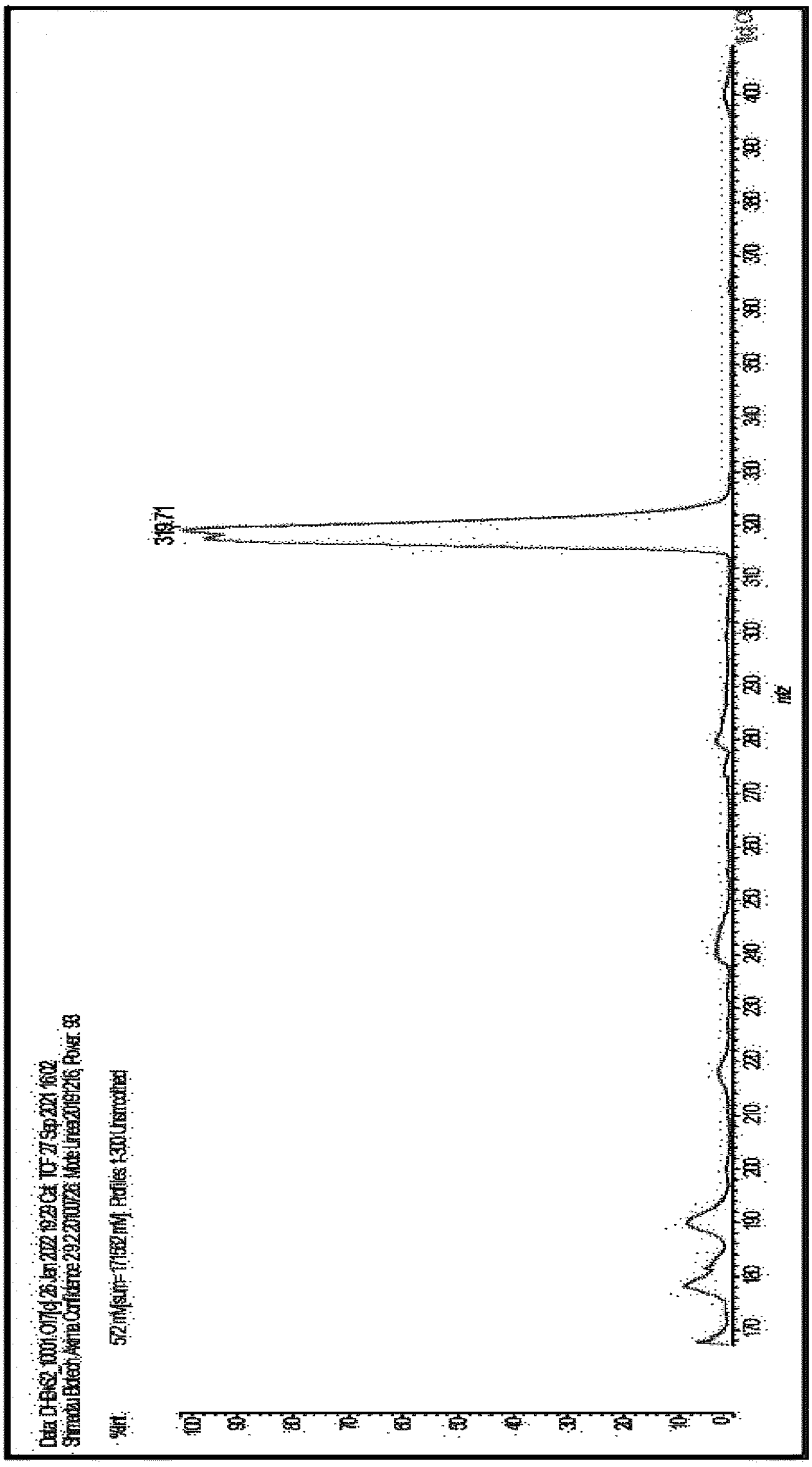

【Fig. 17】

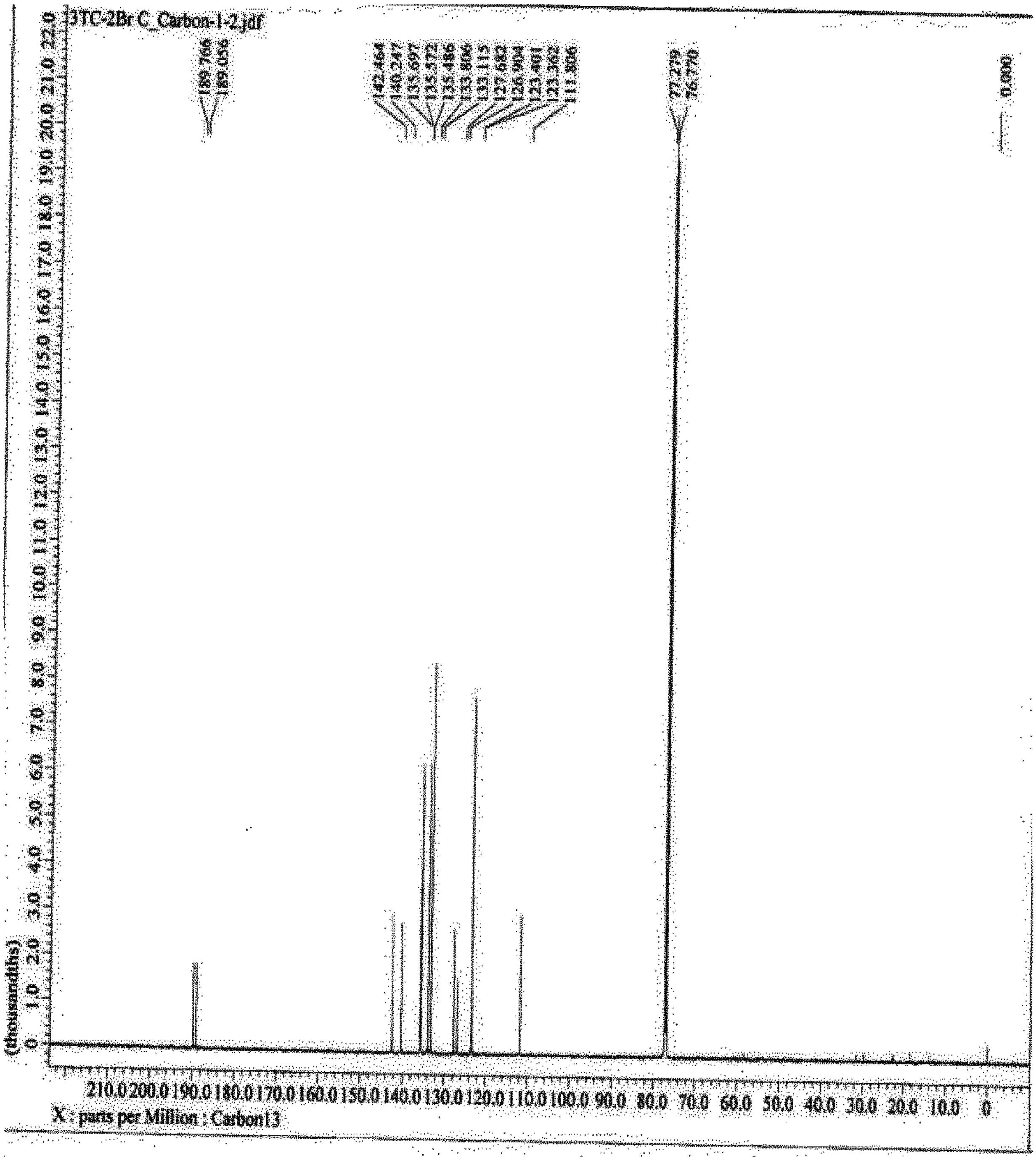

JTC-2Br C_Carbon-1-2.jdf
189.766
189.056
142.464
140.247
135.697
135.572
135.486
133.806
133.115
127.682
126.904
123.401
123.362
111.806
77.279
76.770
0.000
(thousandths)
X : parts per Million : Carbon13

【Fig. 18】

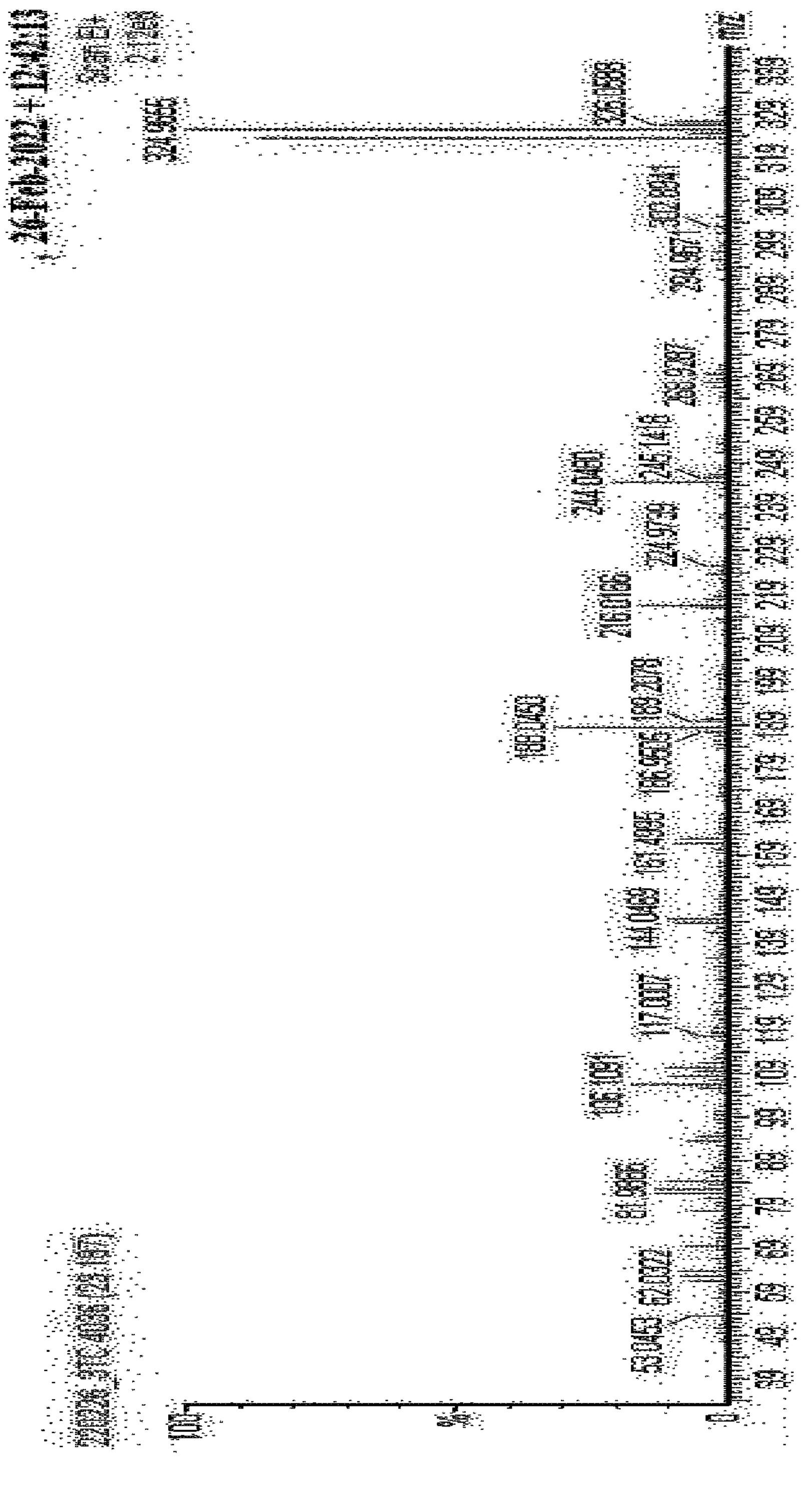

【Fig. 19】

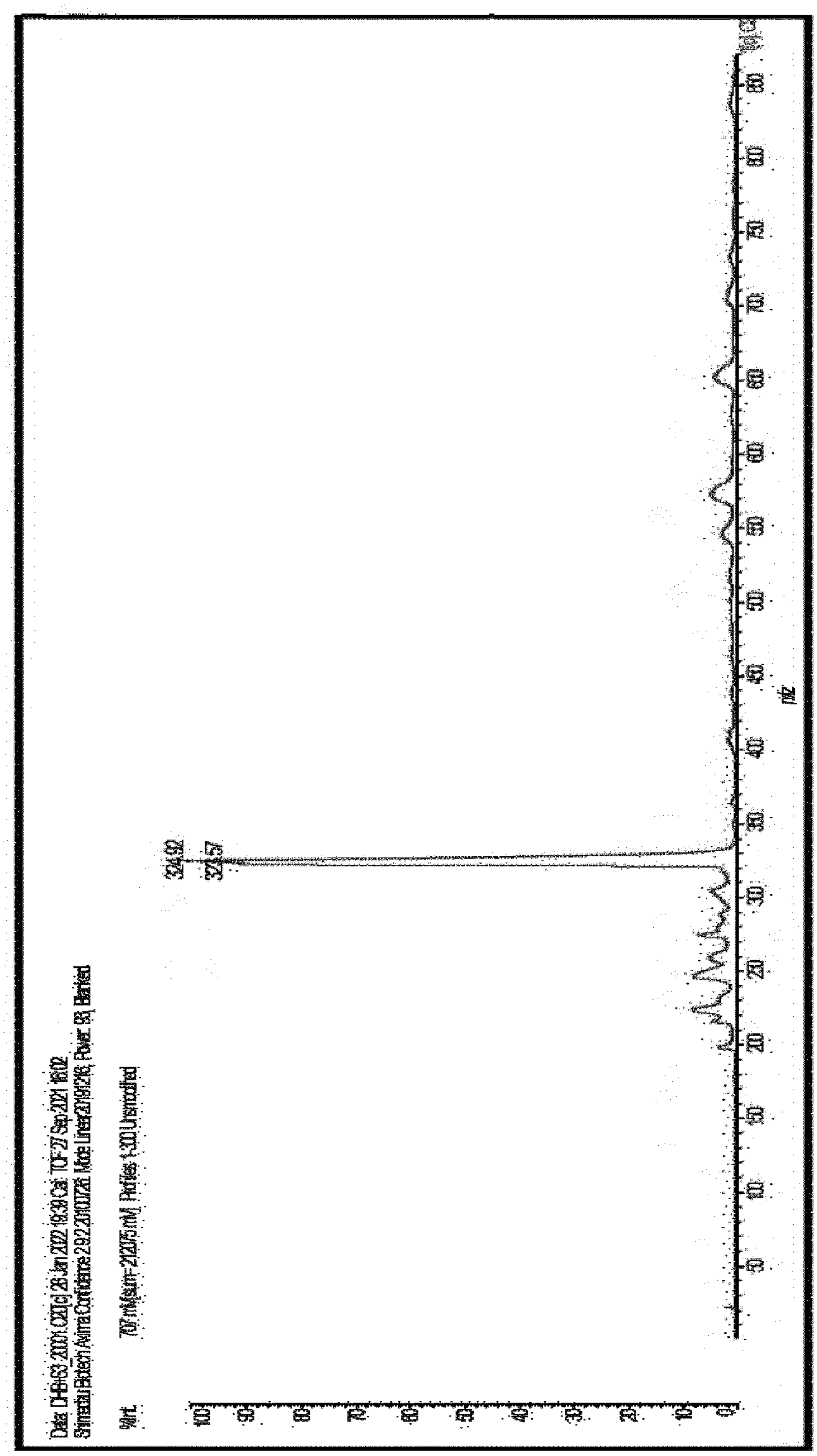

【Fig. 20】

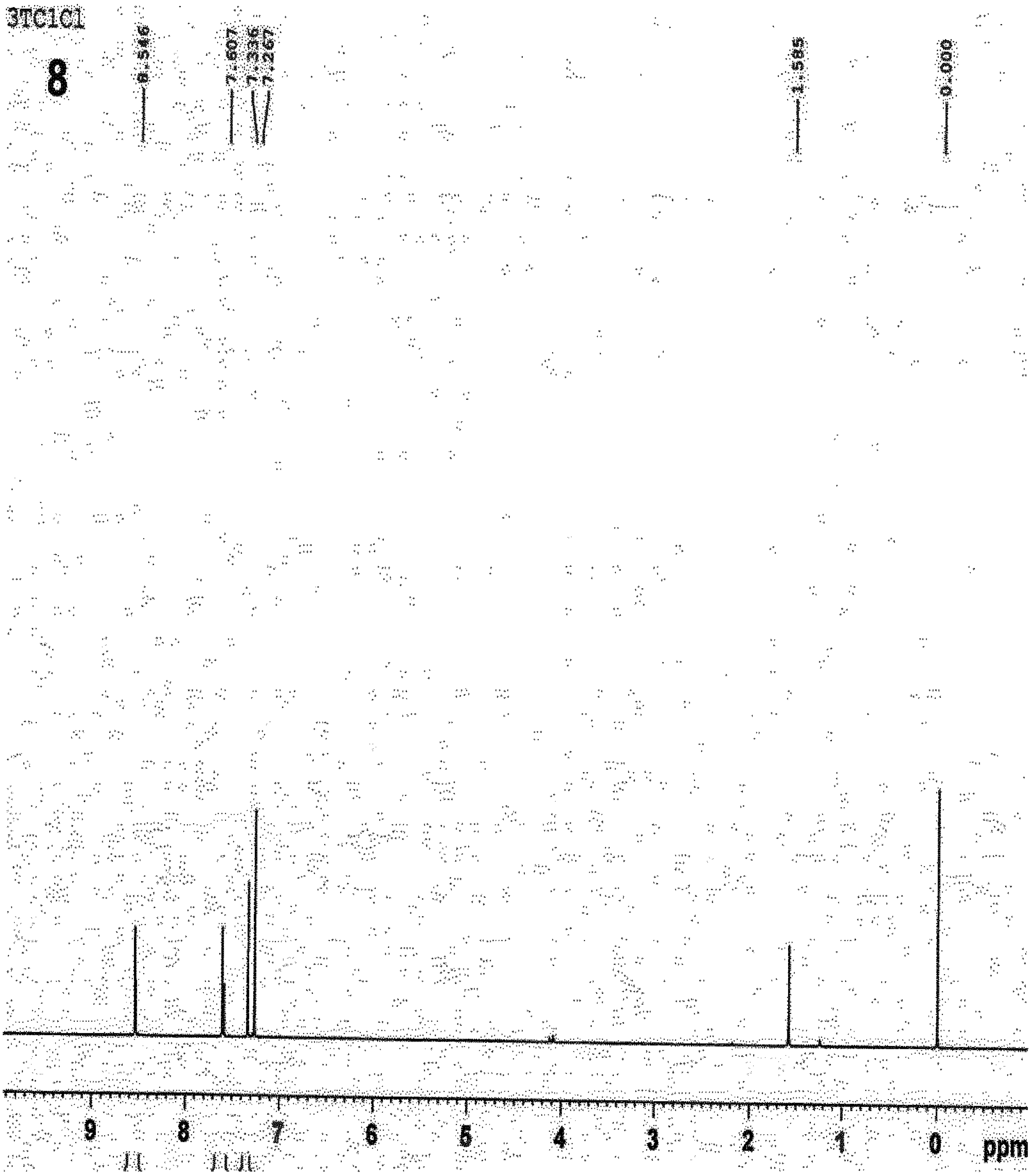

【Fig. 21】

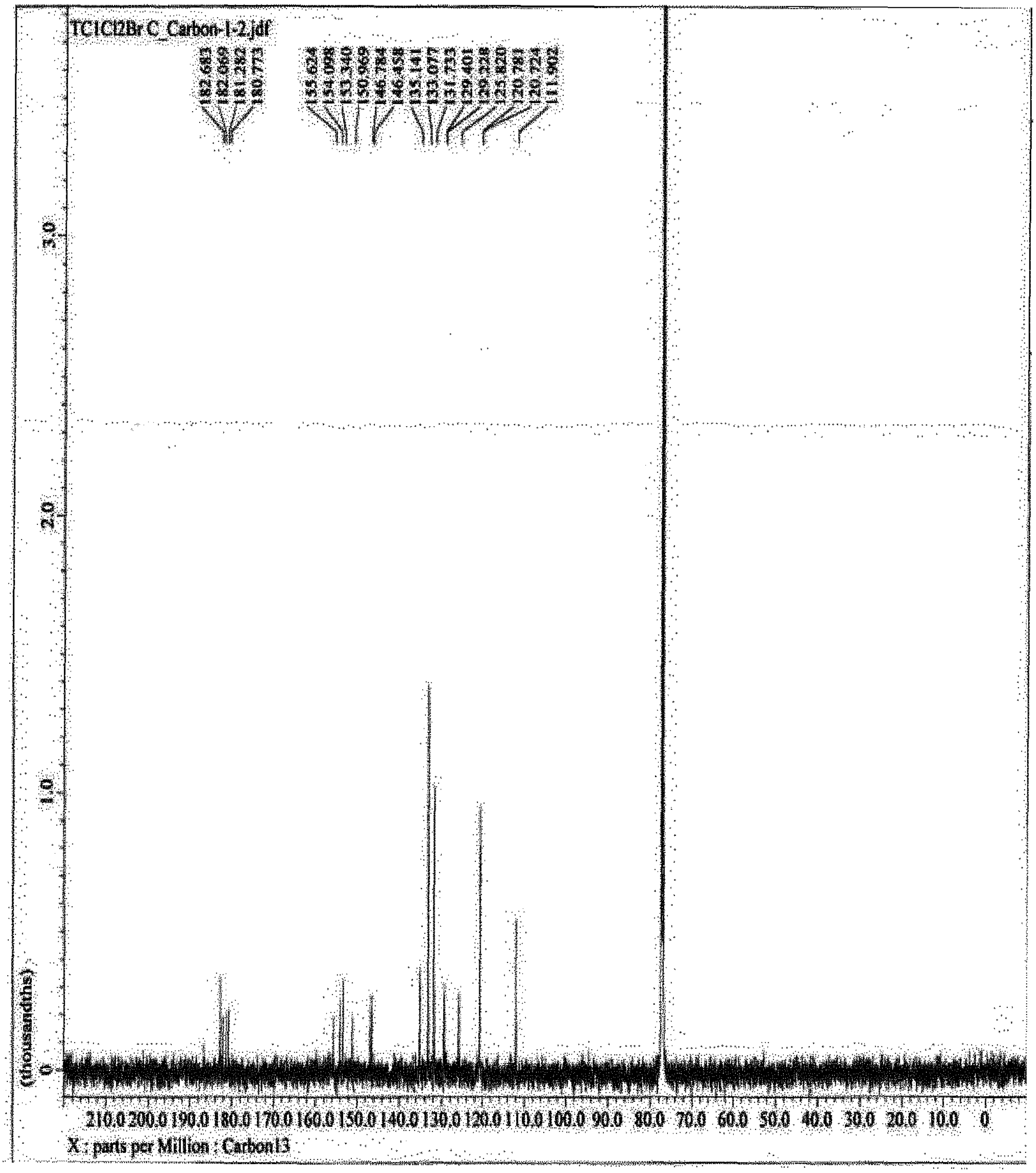
TC1Cl2Br C_Carbon-1-2.jdf
182.683
182.069
181.282
180.773
155.624
154.008
153.340
150.969
146.784
146.458
135.141
133.077
131.733
129.401
129.228
125.820
120.781
120.724
111.902
(thousandths)
3.0
2.0
1.0
0
210.0 200.0 190.0 180.0 170.0 160.0 150.0 140.0 130.0 120.0 110.0 100.0 90.0 80.0 70.0 60.0 50.0 40.0 30.0 20.0 10.0 0
X : parts per Million : Carbon13

【Fig. 22】

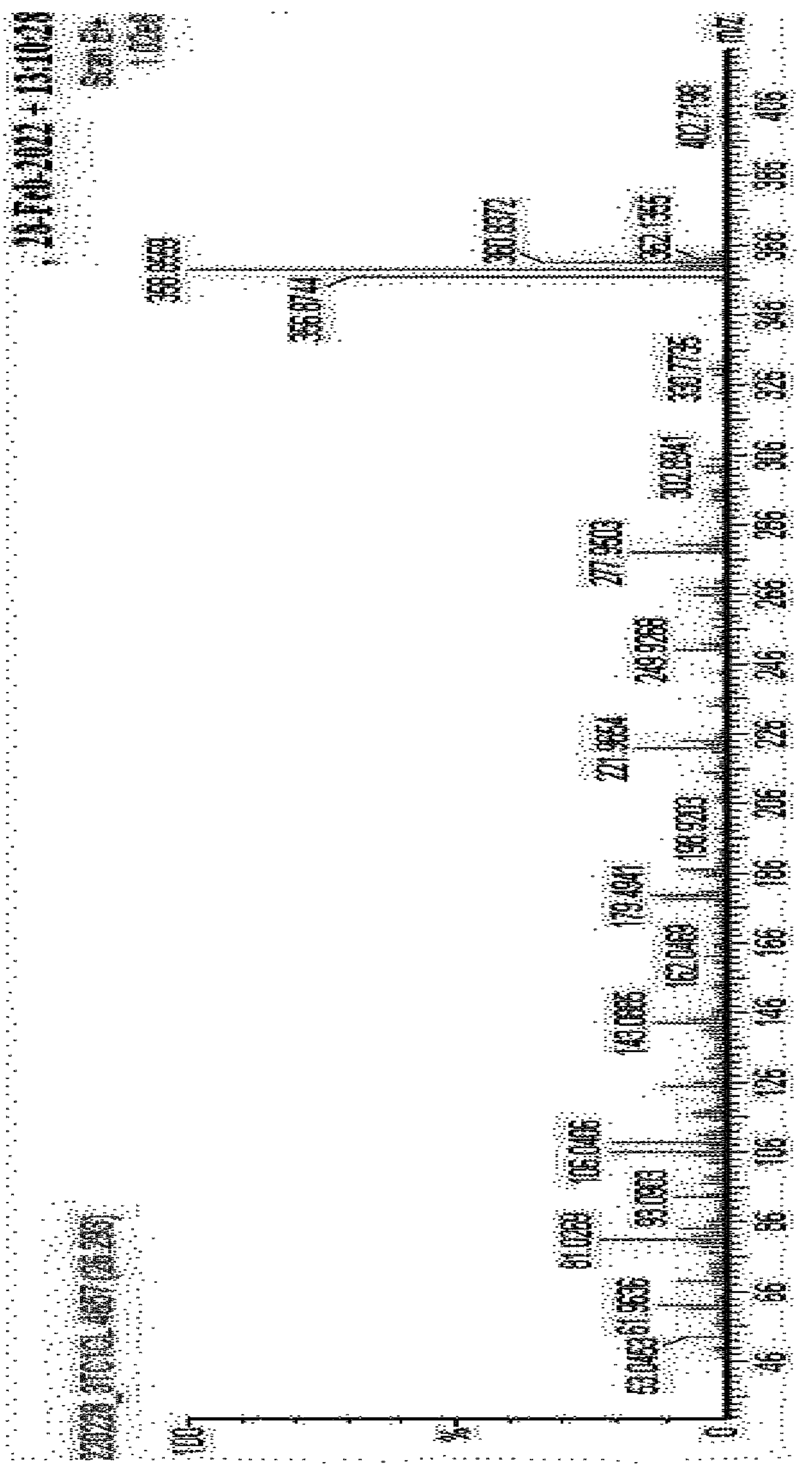

【Fig. 23】

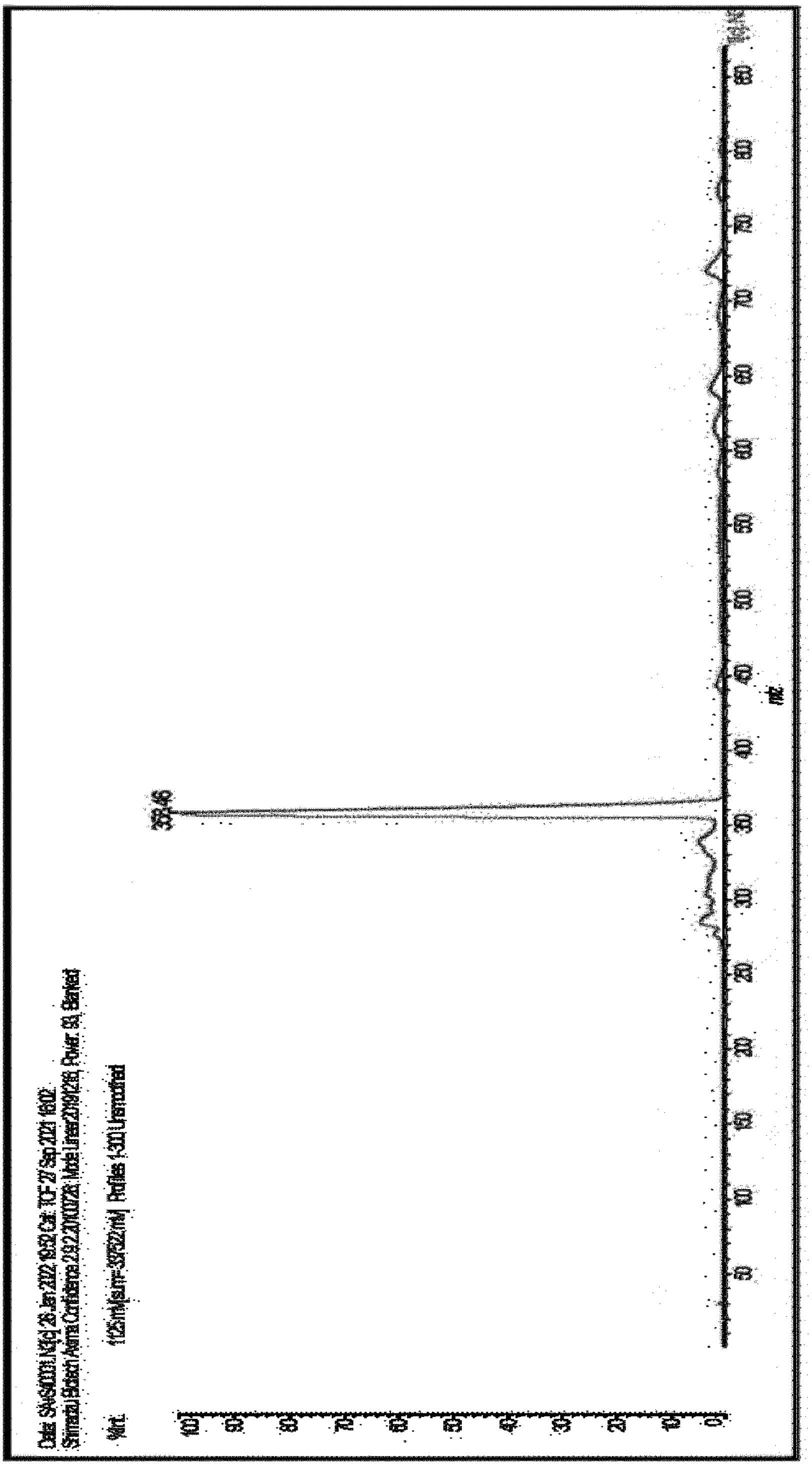

【Fig. 24】

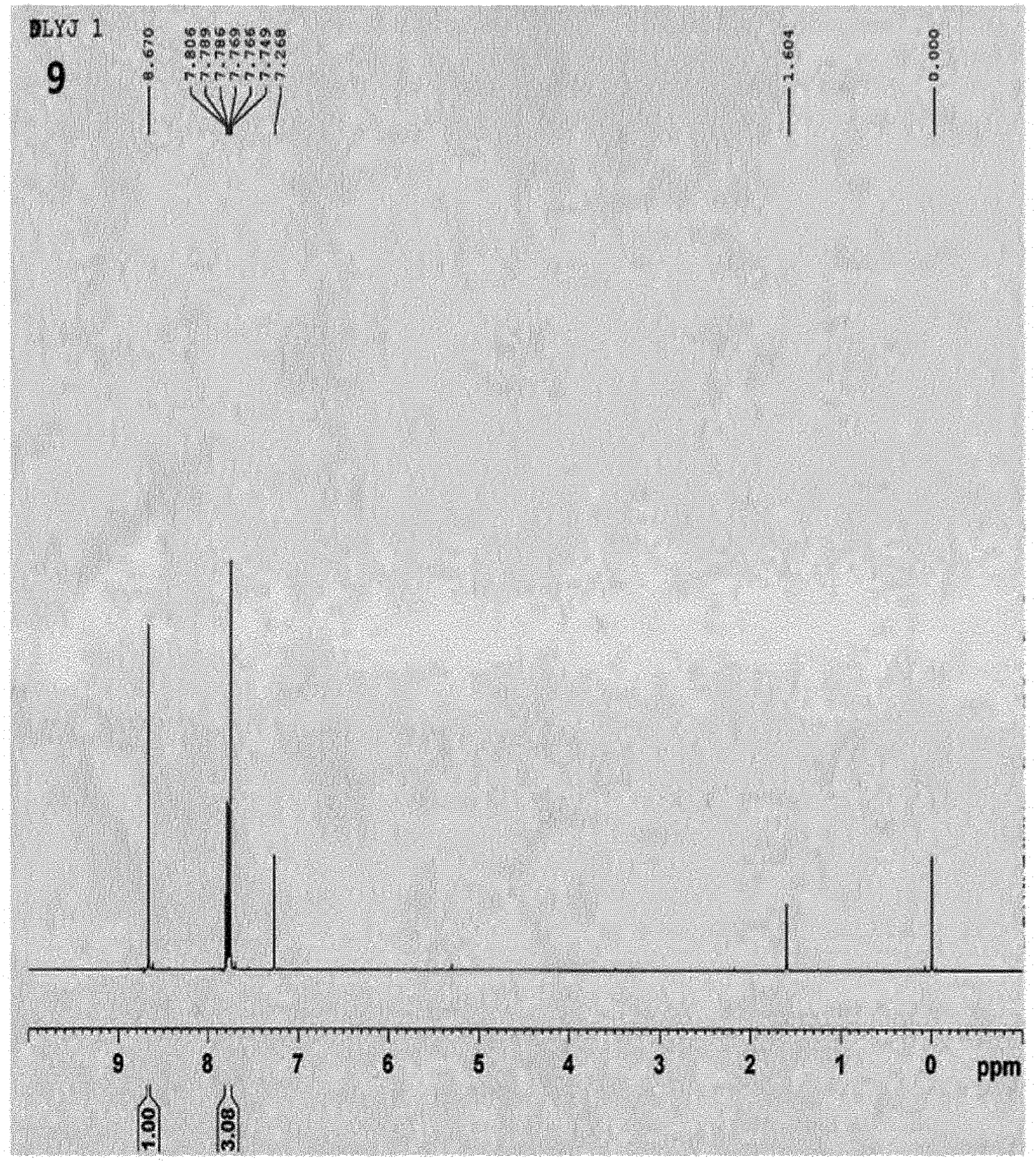
LYJ 1
9
8.670
7.806
7.789
7.786
7.769
7.766
7.749
7.268
1.604
0.000
9 8 7 6 5 4 3 2 1 0 ppm
1.00
3.08

【Fig. 25】

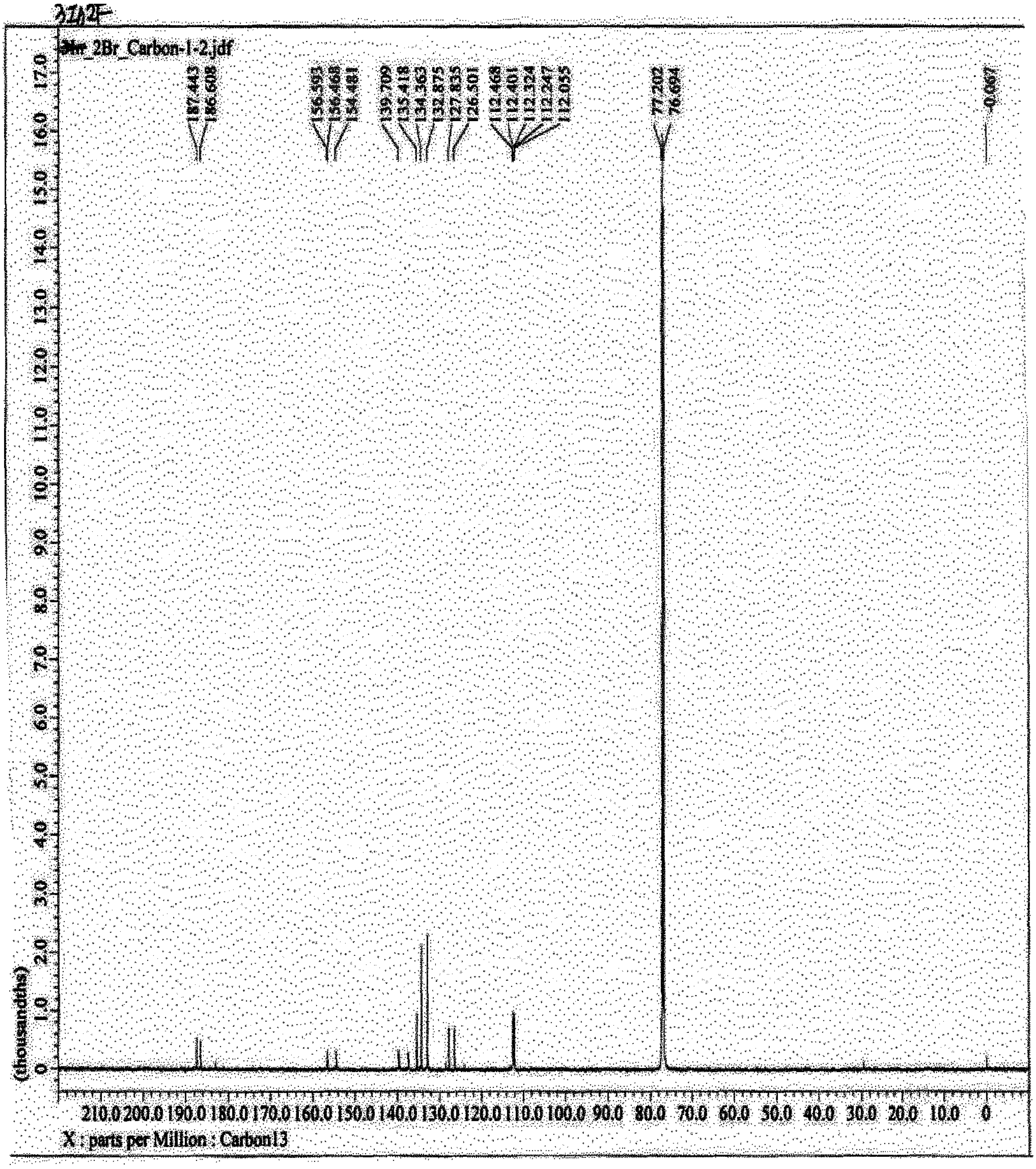
Br_2Br_Carbon-1-2.jdf
187.443
186.608
156.593
156.468
154.481
139.709
135.418
134.363
132.875
127.835
126.501
112.468
112.401
112.324
112.247
112.055
77.202
76.694
-0.067
(thousandths)
X : parts per Million : Carbon13

【Fig. 26】

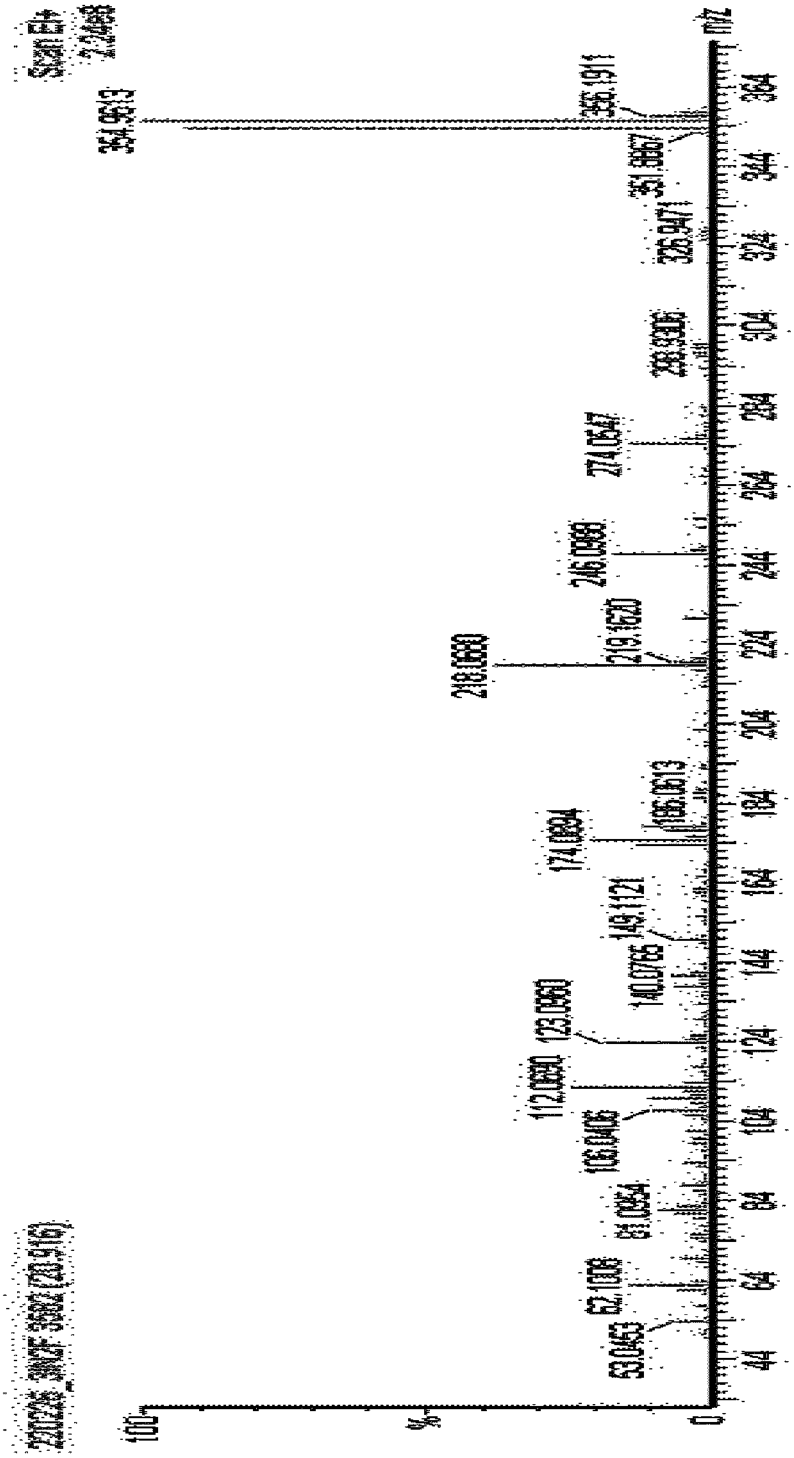
Scan EI+
2.24e8
354.9613
355.1911
351.9867
326.9471
298.9306
274.0547
246.0989
218.0693
219.1620
174.0894
186.0613
149.1121
140.0765
123.0860
112.0890
106.0406
91.0854
67.1006
53.0453
100
%
0
m/z

【Fig. 27】

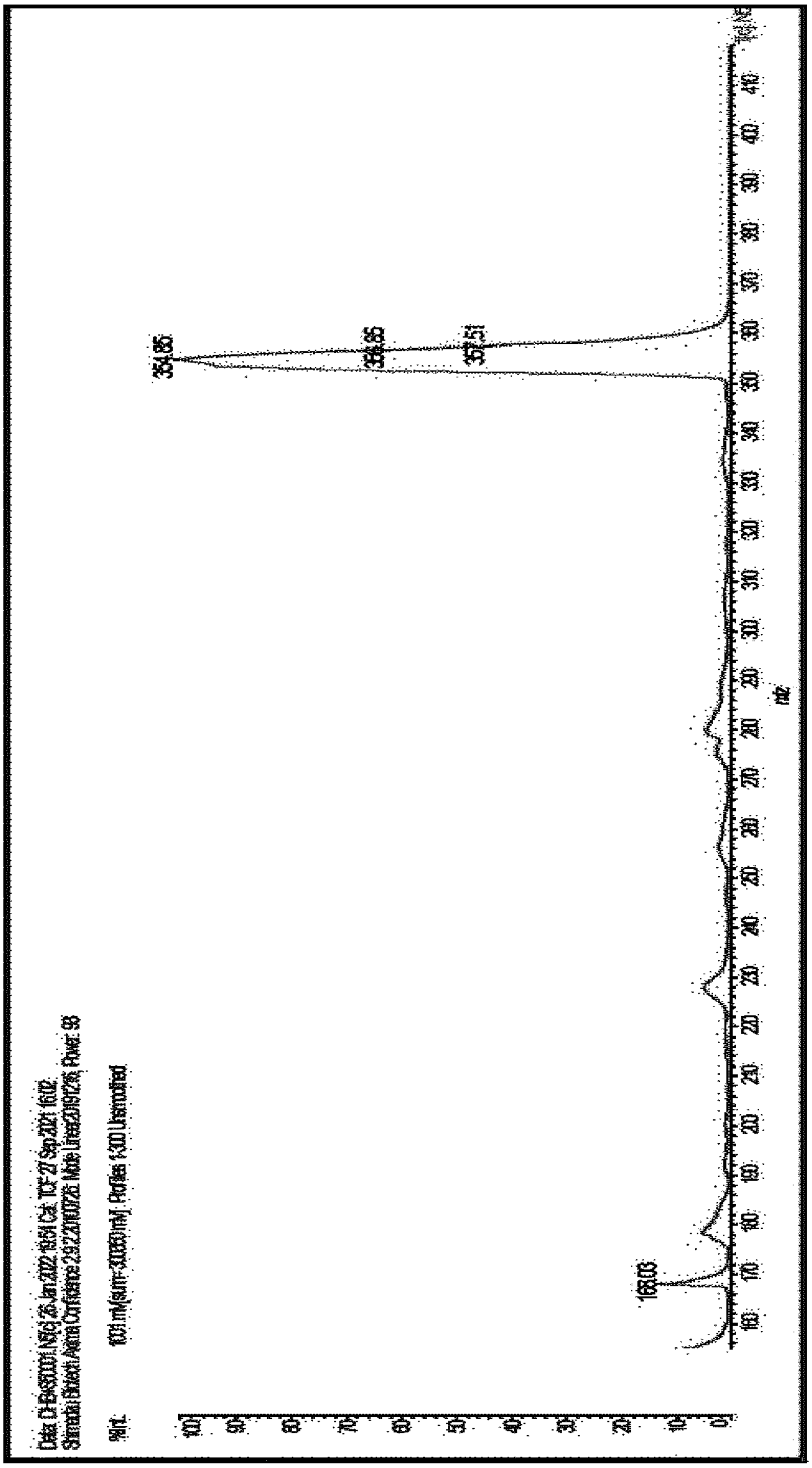

【Fig. 28】

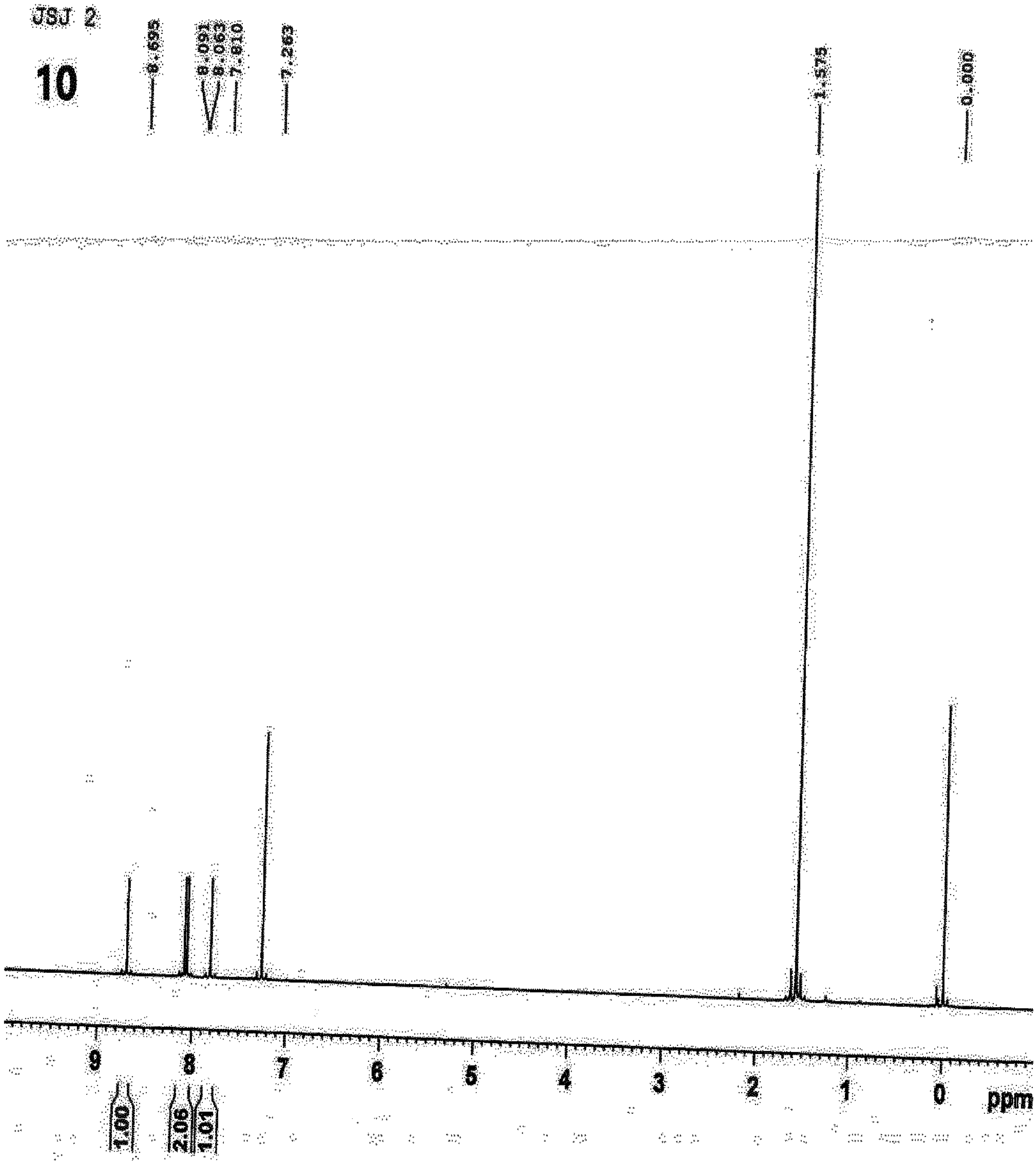
JSJ 2
10
8.695
8.091
8.063
7.810
7.263
1.575
0.000
9
8
7
6
5
4
3
2
1
0
ppm
1.00
2.06
1.01

【Fig. 29】

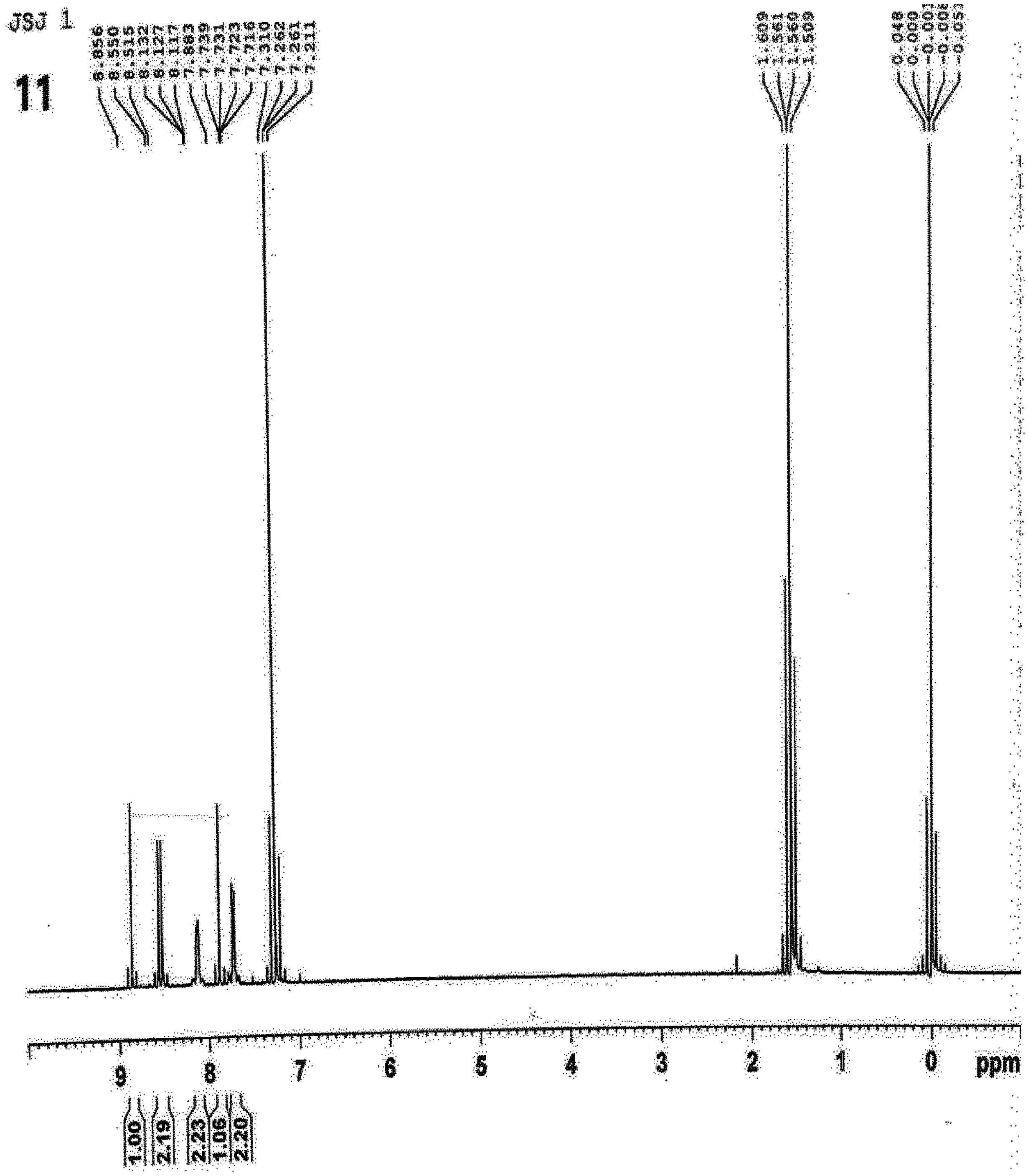
JSJ 1
11
8.856
8.550
8.515
8.132
8.127
8.117
7.883
7.739
7.731
7.723
7.716
7.310
7.262
7.261
7.211
1.609
1.561
1.560
1.509
0.048
0.000
-0.001
-0.008
-0.051
9
8
7
6
5
4
3
2
1
0
ppm
1.00
2.19
2.23
1.06
2.20

【Fig. 30】

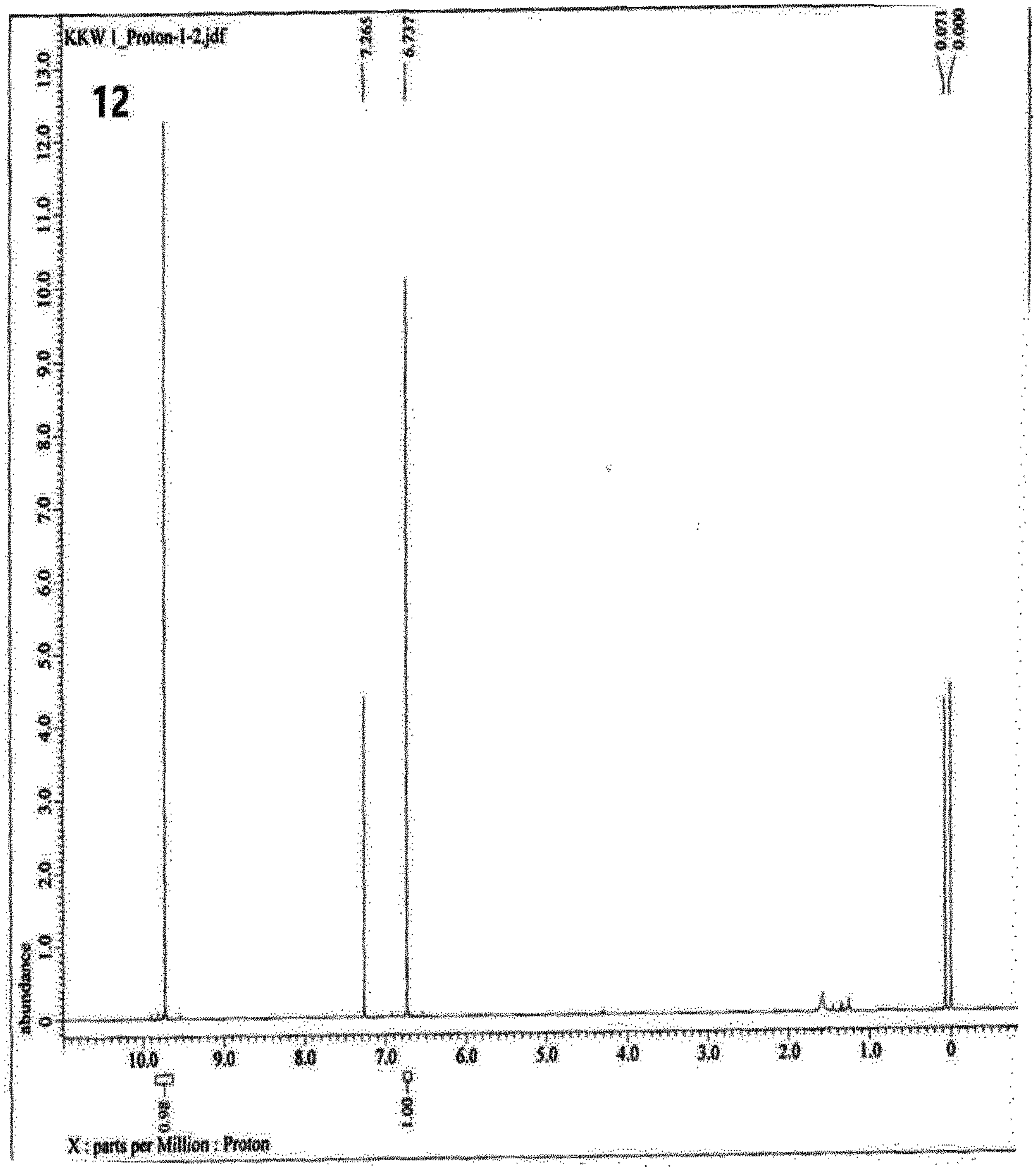
KKW I_Proton-1-2.jdf
12
7.265
6.737
0.071
0.000
abundance
0.98
1.00
X : parts per Million : Proton

【Fig. 31】

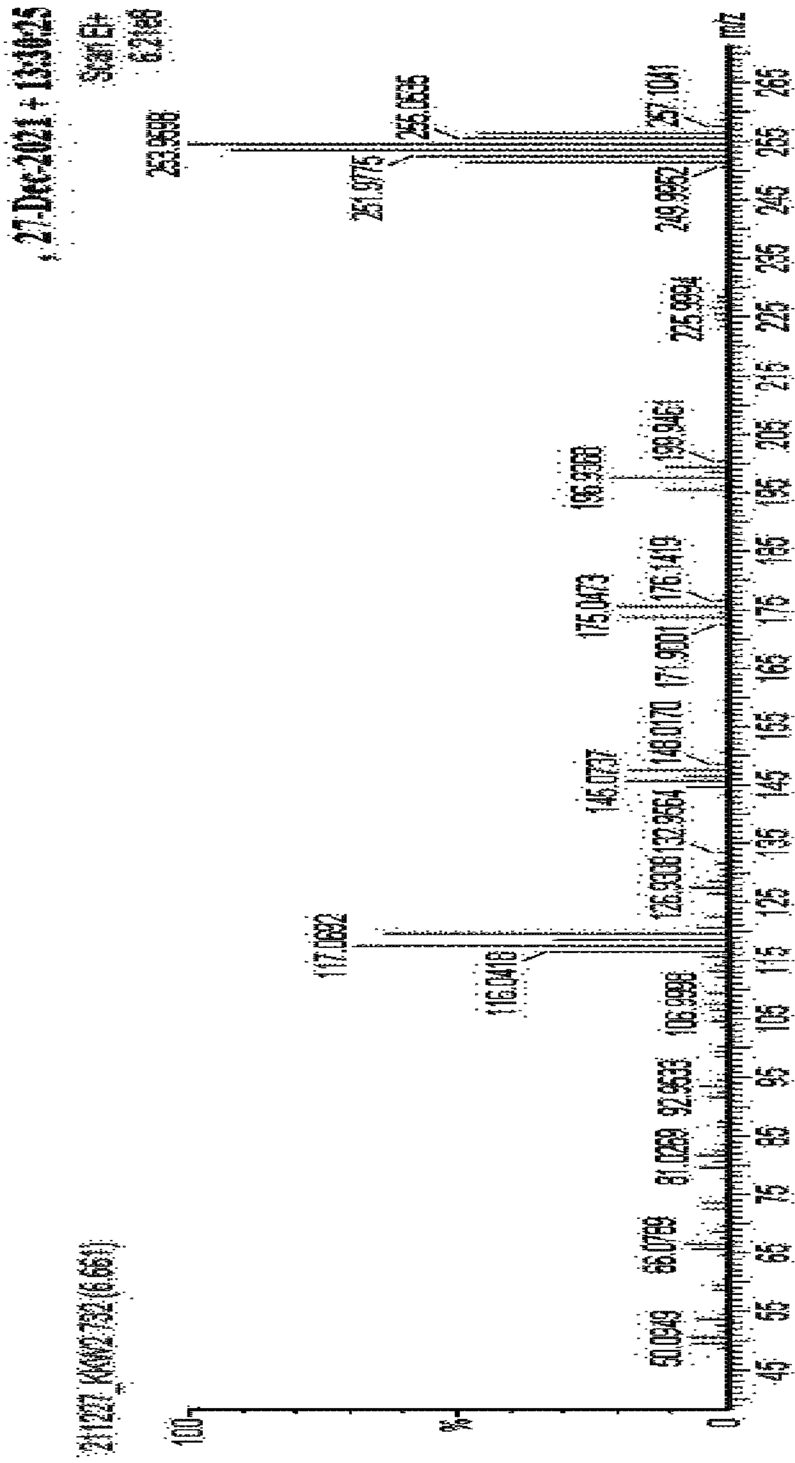
211227_KAW2 732 (6.661)
27-Dec-2021 + 13:10:25
Scan EI+
6.21e6
253.9698
251.9775
249.9952
257.1041
225.9994
199.9461
196.9363
175.0473
176.1419
171.9001
148.0170
145.0737
132.9564
126.9308
117.0692
116.0418
106.9996
92.9533
81.0269
66.0789
50.0349
100
%
0
m/z

【Fig. 32】

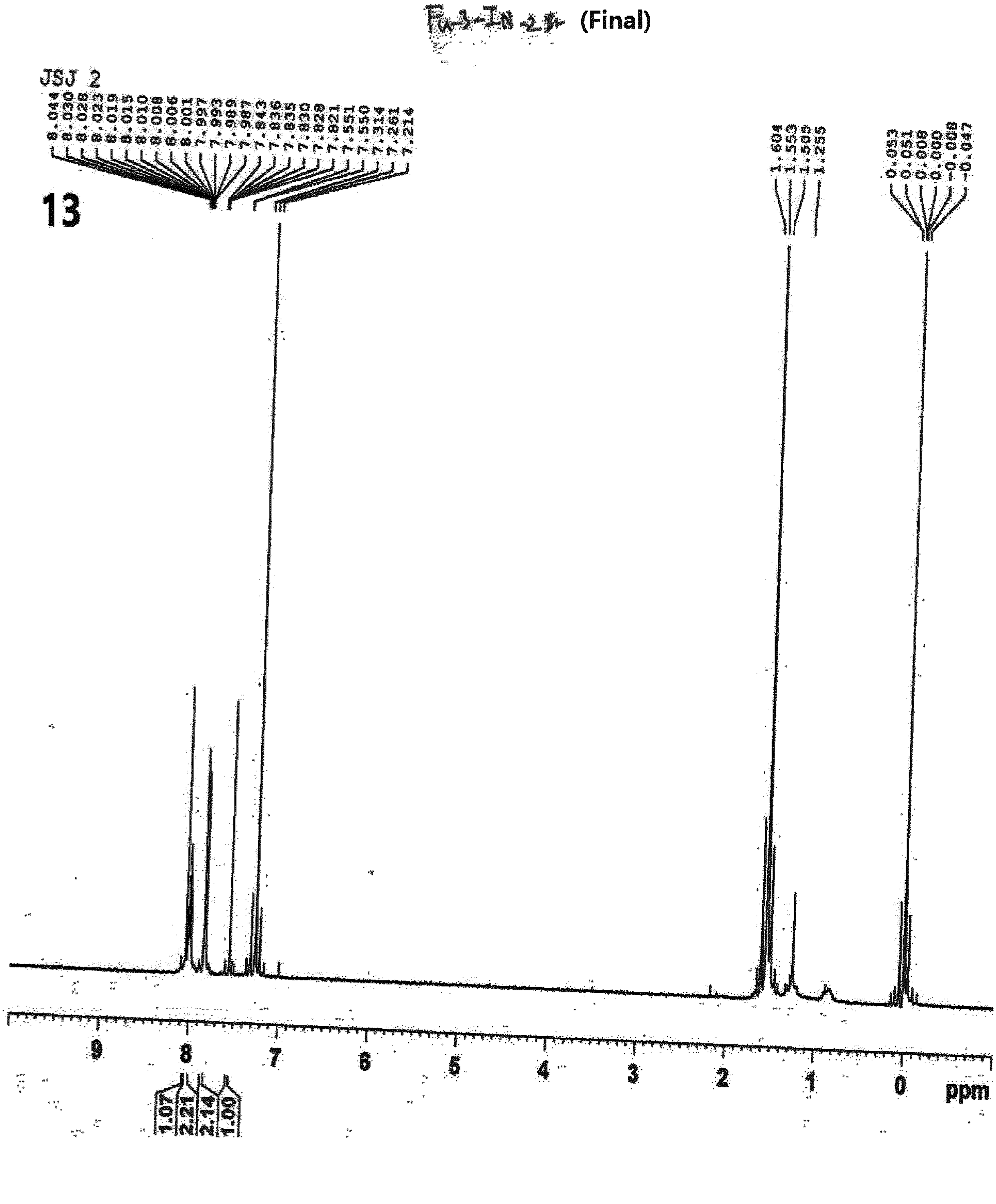
(Final)
JSJ 2
8.044
8.030
8.028
8.023
8.019
8.015
8.010
8.008
8.006
8.001
7.997
7.993
7.989
7.987
7.843
7.836
7.835
7.830
7.828
7.821
7.551
7.550
7.314
7.261
7.214
1.604
1.553
1.505
1.255
0.053
0.051
0.008
0.000
-0.008
-0.047
13
9
8
7
6
5
4
3
2
1
0
ppm
1.07
2.21
2.14
1.00

【Fig. 33】

Fu3TC

14

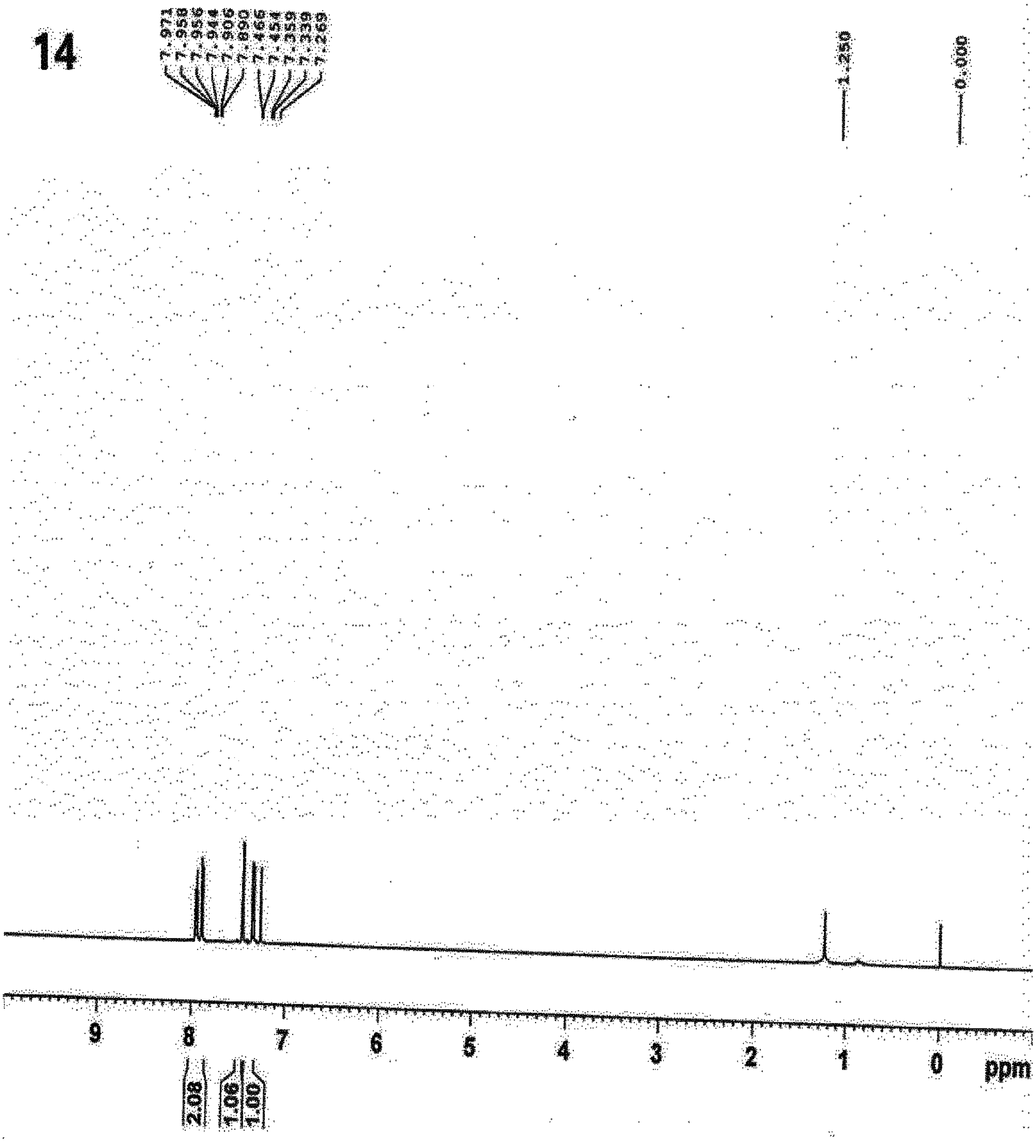

【Fig. 34】

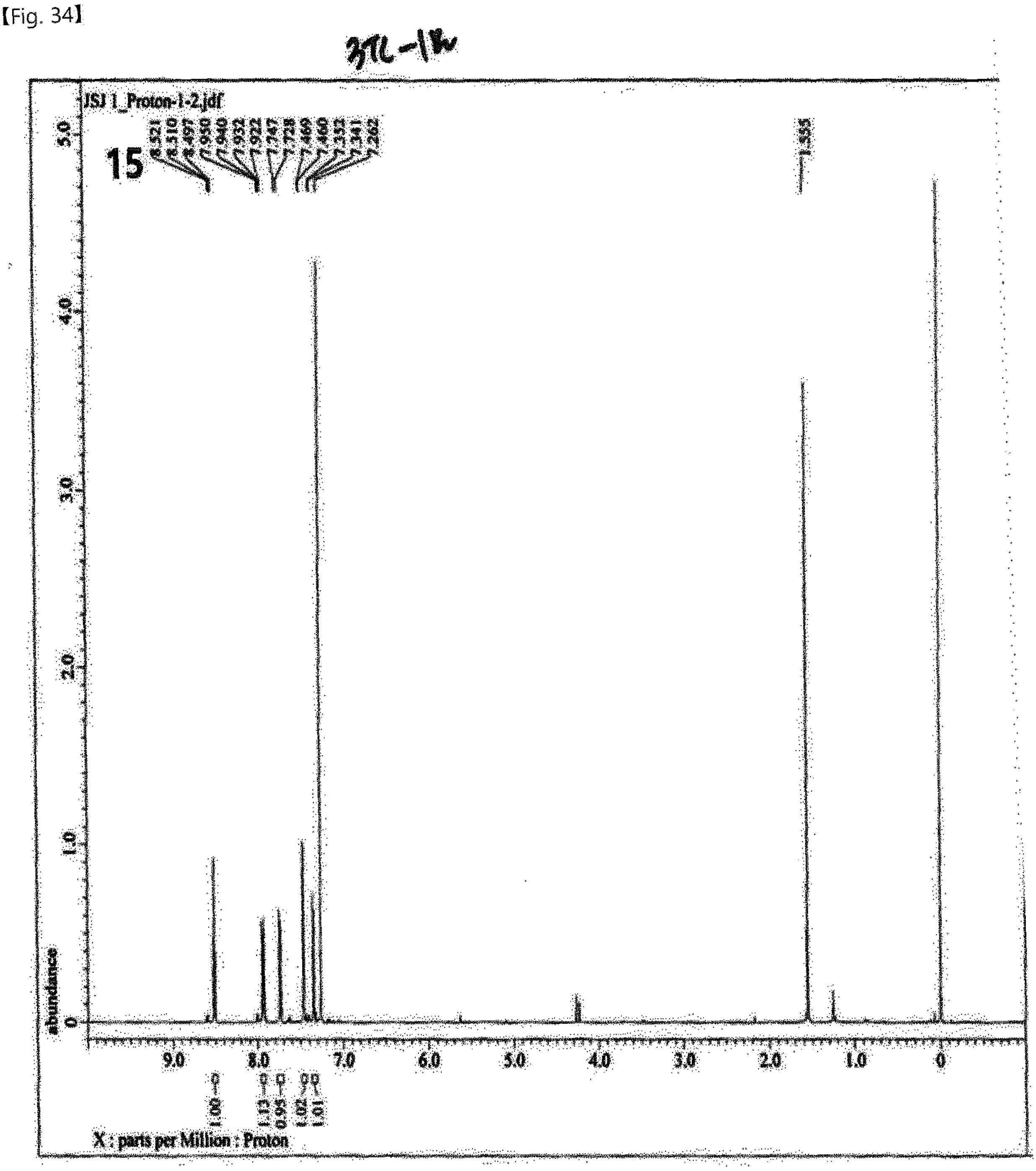
JSJ 1_Proton-1-2.jdf
15
8.521
8.510
8.497
7.950
7.940
7.932
7.922
7.747
7.728
7.469
7.460
7.352
7.341
7.262
1.555
abundance
X : parts per Million : Proton
1.00
1.13
0.95
1.02
1.01

【Fig. 35】

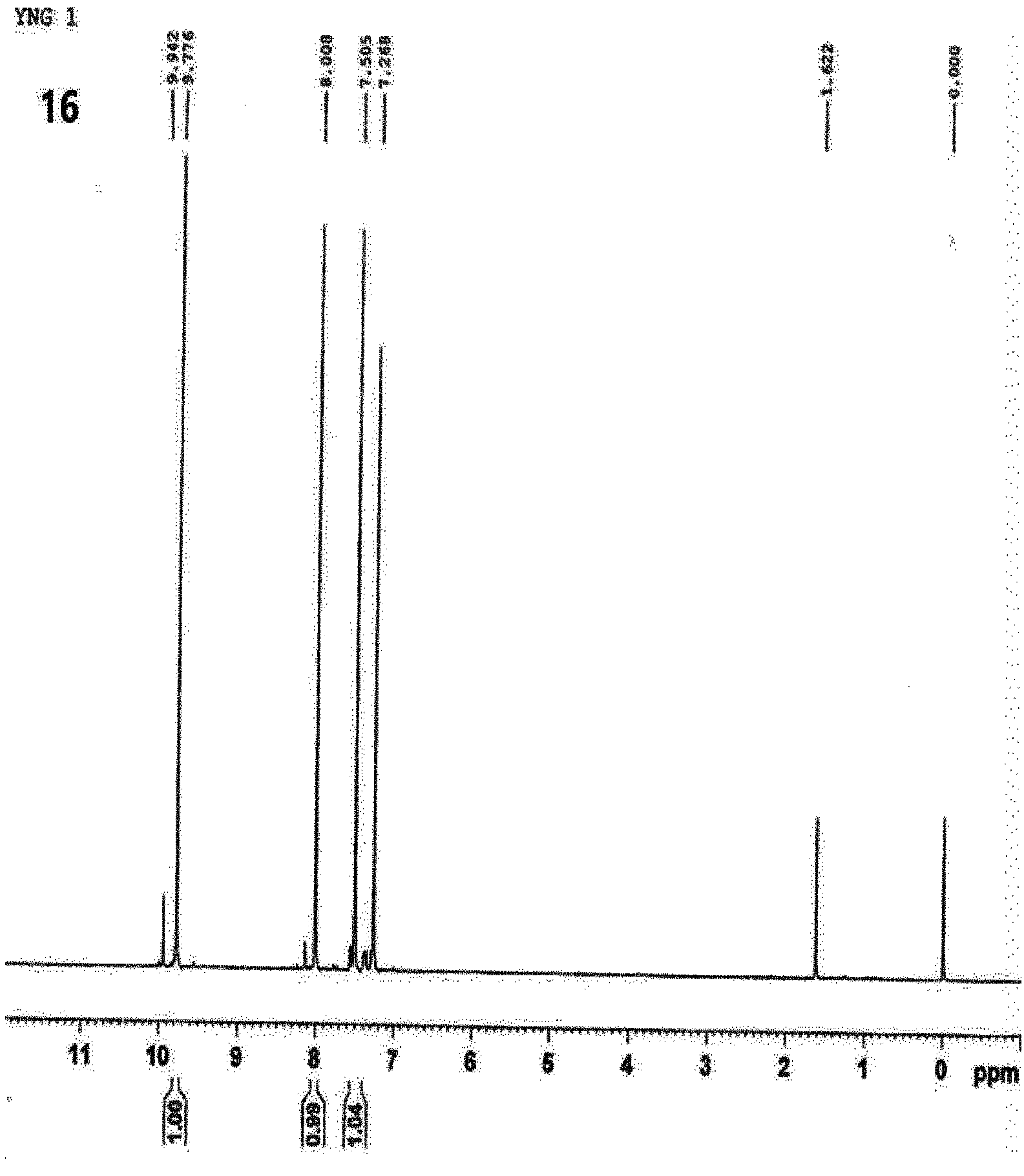
YNG 1
16
9.942
9.776
8.008
7.505
7.268
1.622
0.000
11
10
9
8
7
6
5
4
3
2
1
0
ppm
1.00
0.99
1.04

【Fig. 36】

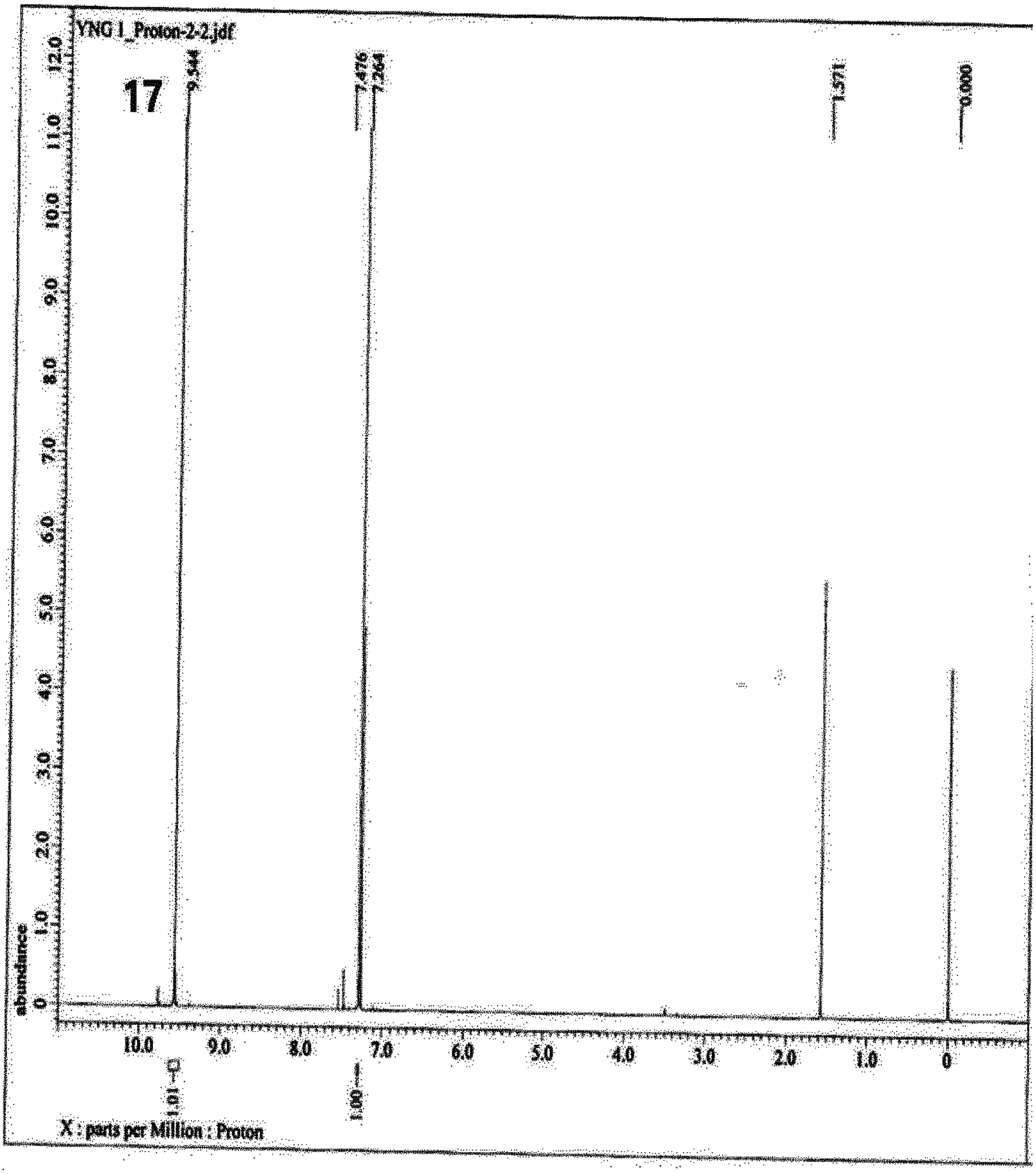
YNG 1_Proton-2-2.jdf
17
9.544
7.476
7.264
1.571
0.000
abundance
0
1.0
2.0
3.0
4.0
5.0
6.0
7.0
8.0
9.0
10.0
11.0
12.0
10.0
9.0
8.0
7.0
6.0
5.0
4.0
3.0
2.0
1.0
0
1.01
1.00
X : parts per Million : Proton

【Fig. 37】

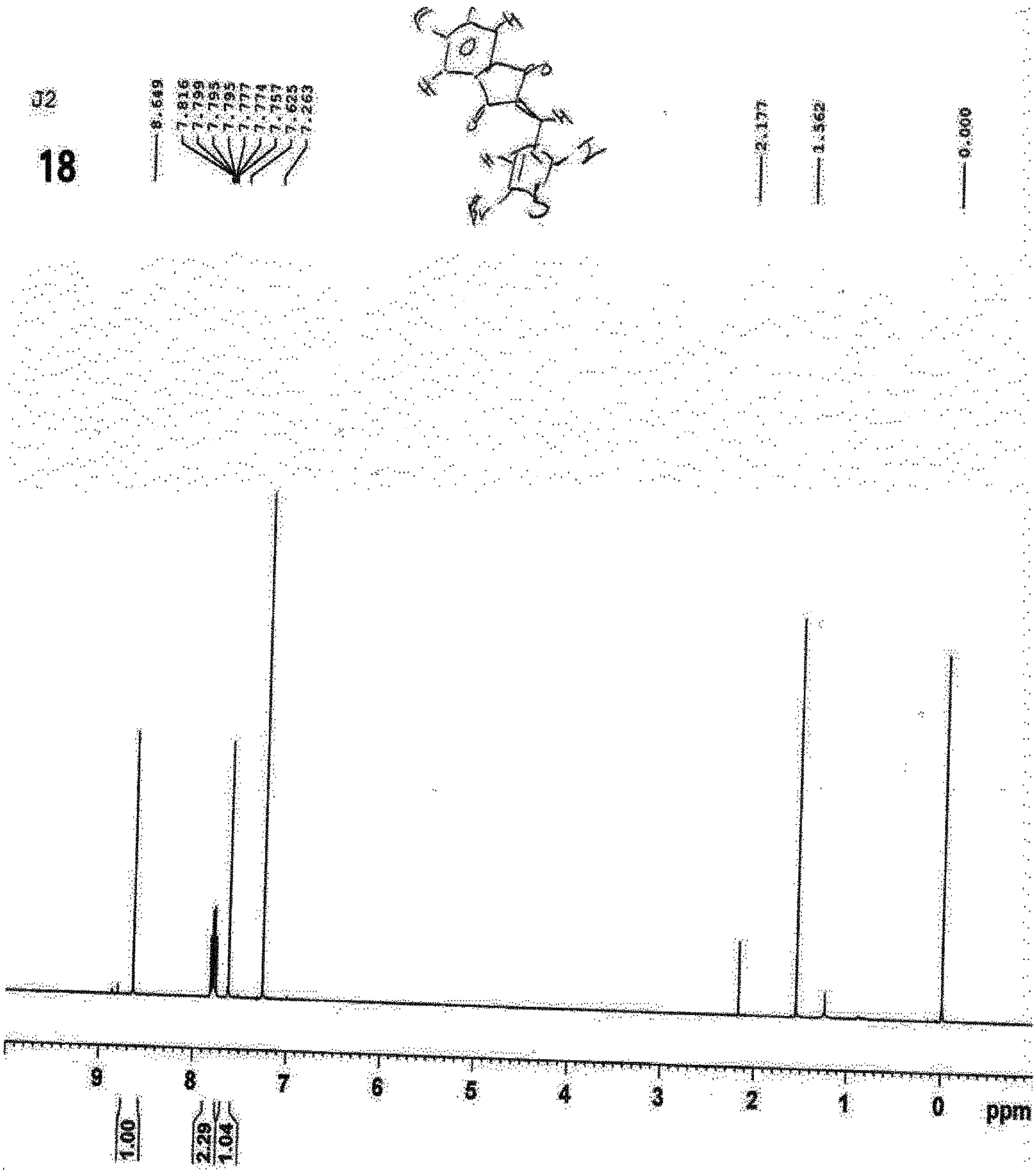
J2
18
8.649
7.816
7.799
7.795
7.795
7.777
7.777
7.774
7.757
7.625
7.263
2.177
1.562
0.000
9
8
7
6
5
4
3
2
1
0
ppm
1.00
2.29
1.04

【Fig. 38】

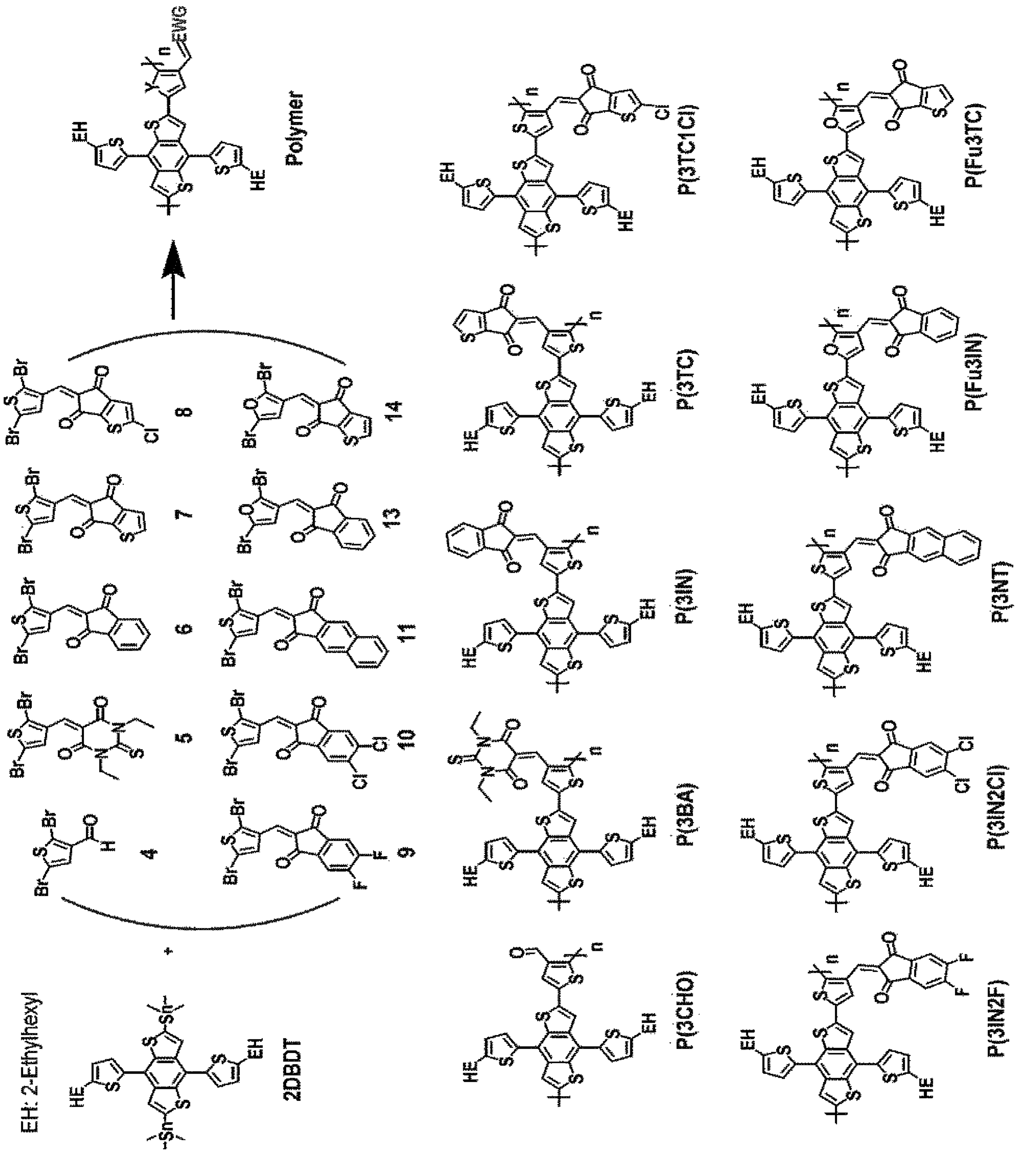


2DBDT

4

5

6

7

8

9

10

11

13

14

Polymer

P(3CHO)

P(3BA)

P(3IN)

P(3TC)

P(3TC1Cl)

P(3IN2F)

P(3IN2Cl)

P(3NT)

P(Fu3IN)

P(Fu3TC)

【Fig. 39】

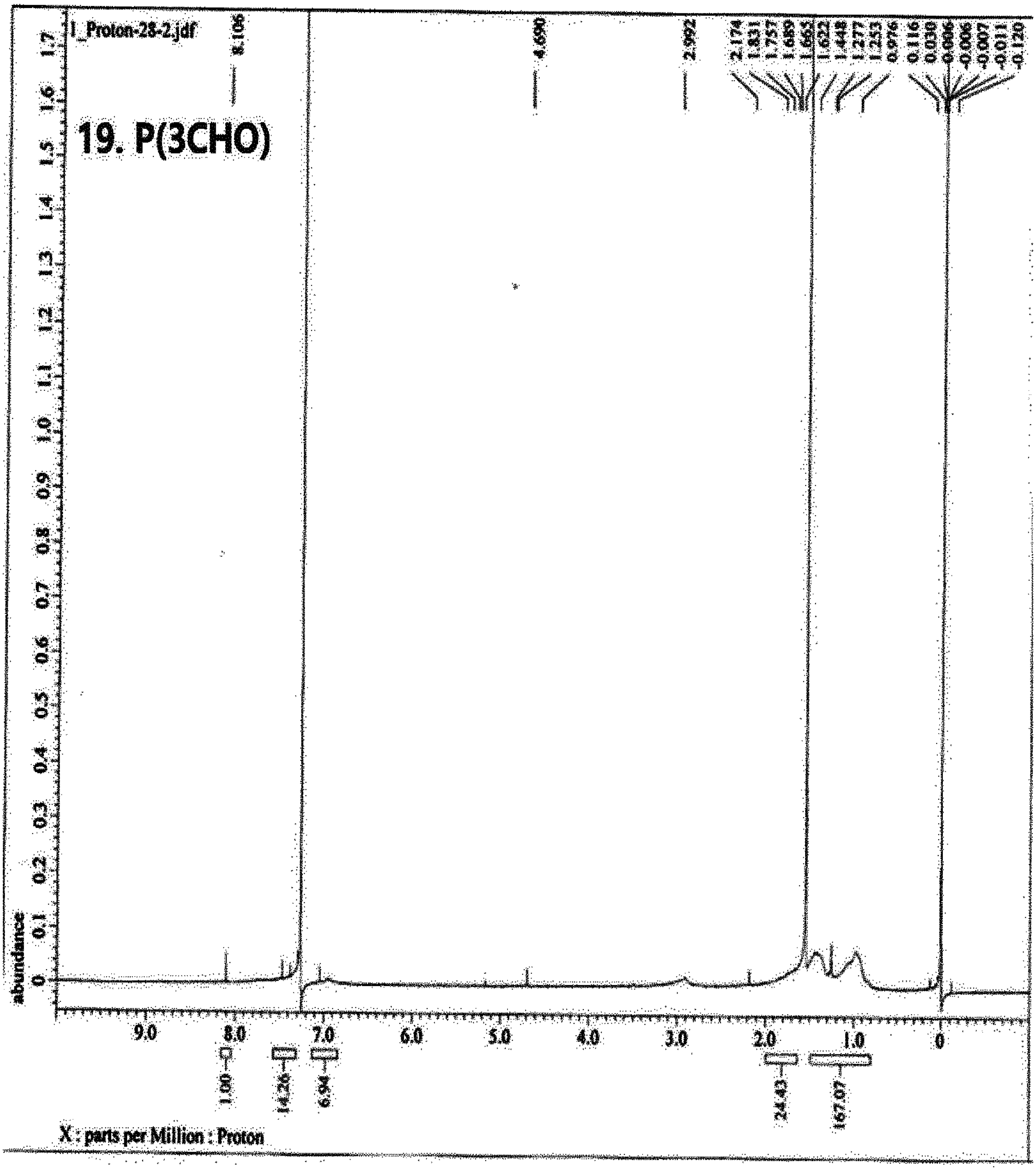
1_Proton-28-2.jdf
19. P(3CHO)
8.106
4.690
2.992
2.174
1.831
1.757
1.689
1.665
1.622
1.448
1.277
1.253
0.976
0.116
0.030
0.006
-0.006
-0.007
-0.011
-0.120
abundance
1.00
14.26
6.94
24.43
167.07
X : parts per Million : Proton

【Fig. 40】

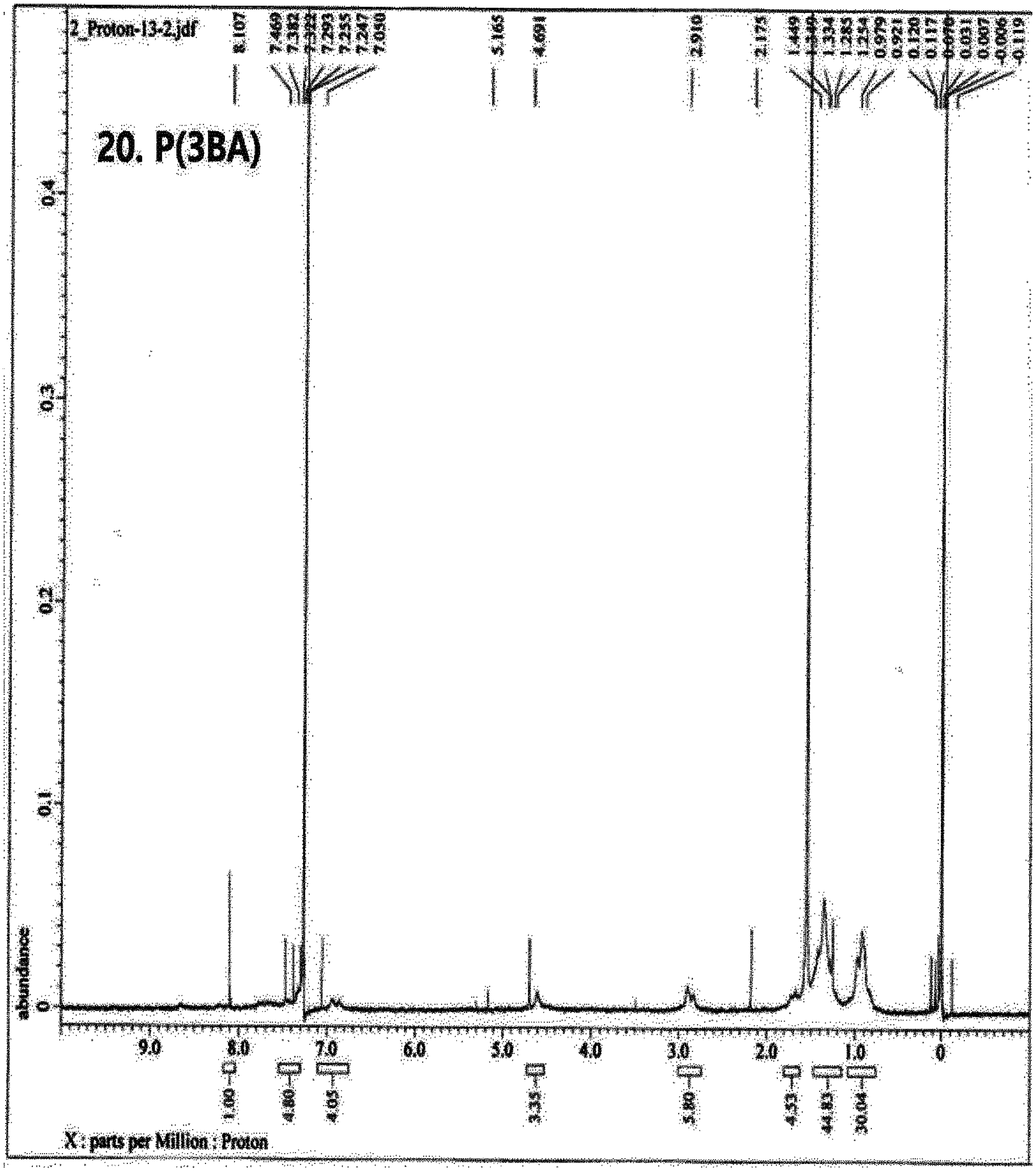

2_Proton-13-2.jdf
20. P(3BA)
8.107
7.469
7.382
7.322
7.293
7.255
7.247
7.050
5.165
4.691
2.910
2.175
1.449
1.334
1.285
1.234
0.979
0.921
0.120
0.117
0.031
0.007
-0.006
-0.119
abundance
0
0.1
0.2
0.3
0.4
9.0
8.0
7.0
6.0
5.0
4.0
3.0
2.0
1.0
0
1.00
4.80
4.05
3.35
5.80
4.53
44.83
30.04
X : parts per Million : Proton

【Fig. 41】

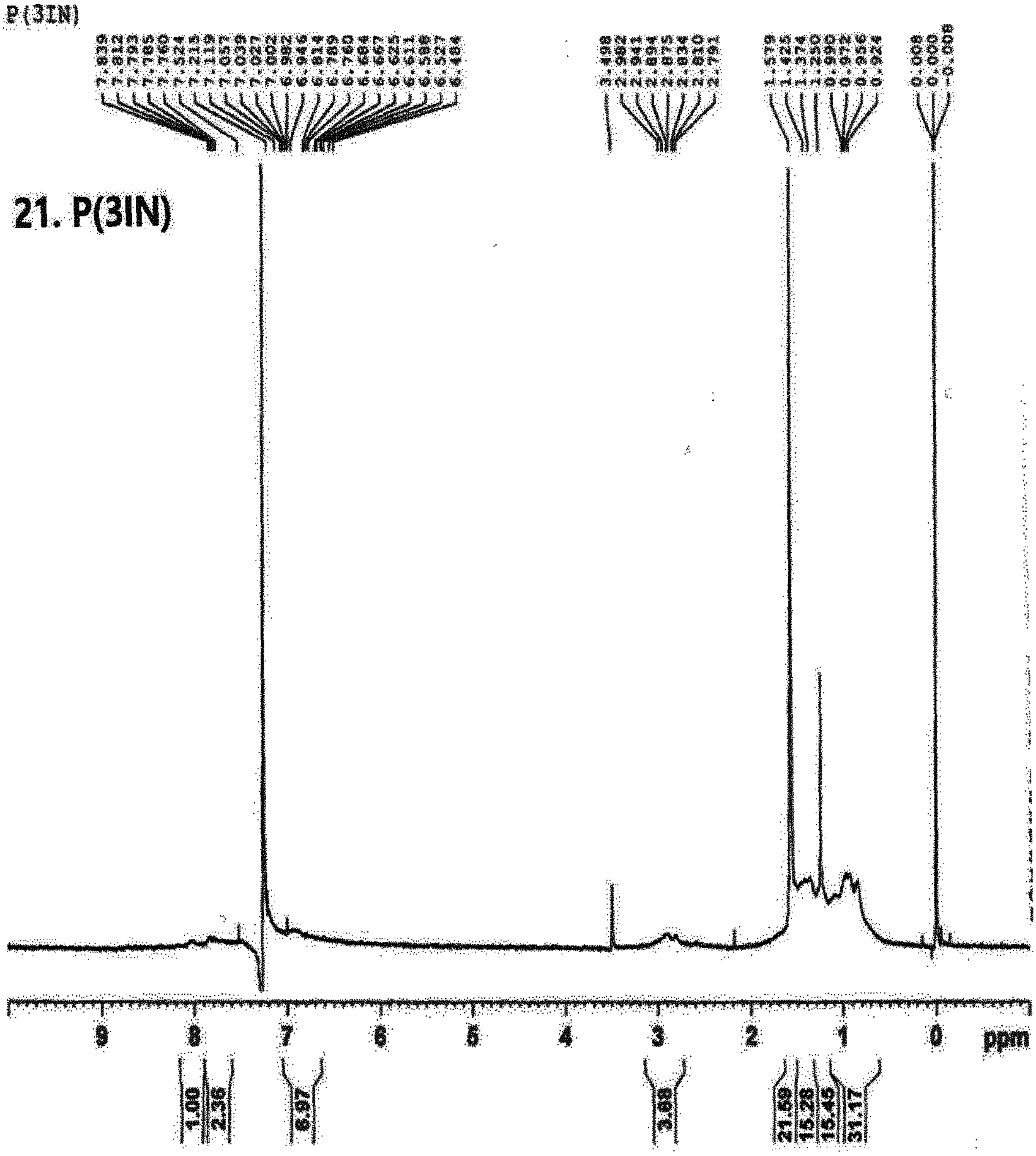
P(3IN)
7.839
7.812
7.793
7.785
7.760
7.524
7.215
7.119
7.057
7.039
7.027
7.002
6.982
6.946
6.814
6.789
6.760
6.684
6.667
6.625
6.611
6.586
6.527
6.484
3.498
2.982
2.941
2.894
2.875
2.834
2.810
2.791
1.579
1.425
1.374
1.250
0.990
0.972
0.956
0.924
0.008
0.000
-0.008
21. P(3IN)
9
8
7
6
5
4
3
2
1
0
ppm
1.00
2.36
6.97
3.68
21.59
15.28
15.45
31.17

【Fig. 42】

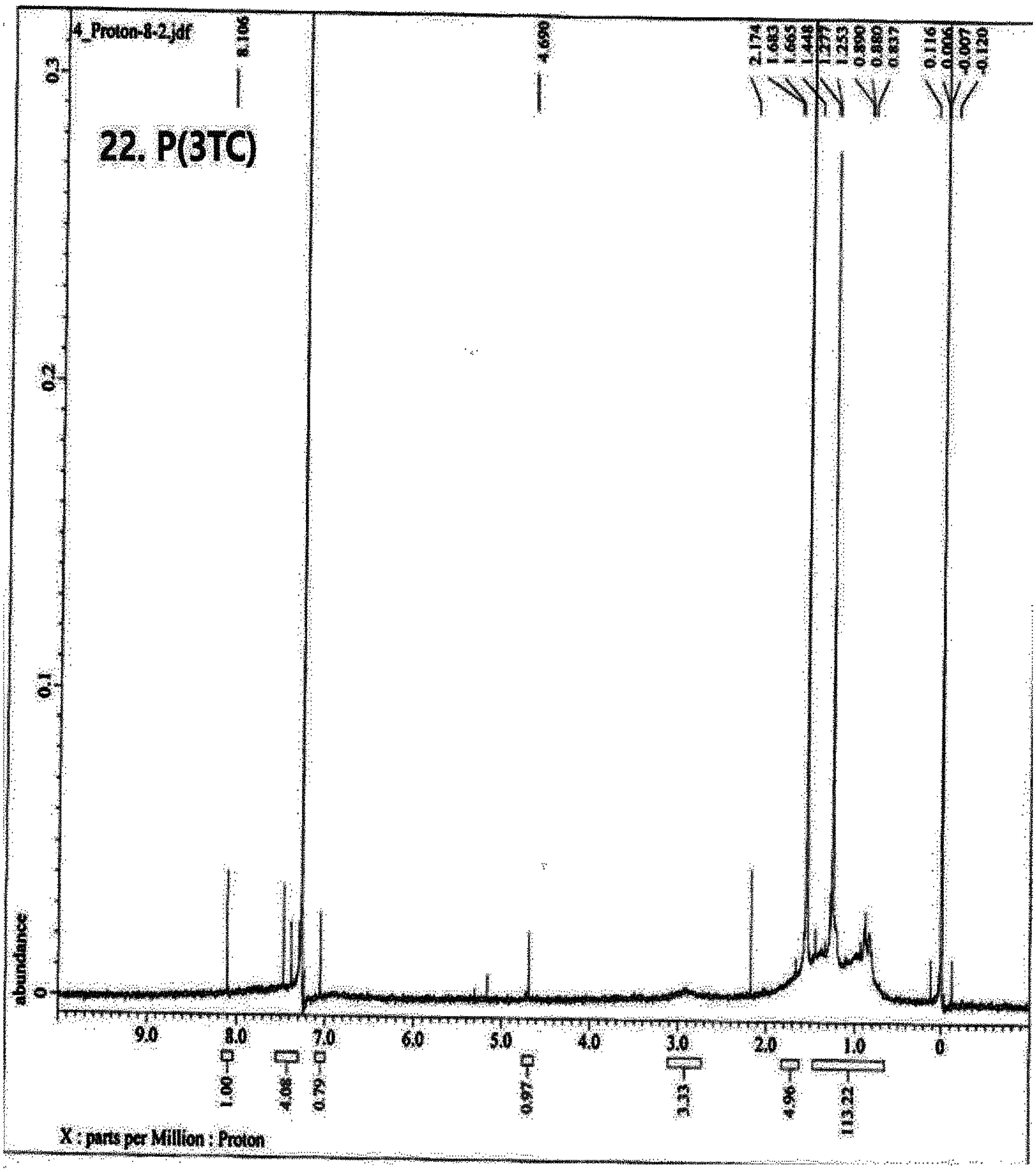
4_Proton-8-2.jdf
22. P(3TC)
8.106
4.690
2.174
1.683
1.665
1.448
1.277
1.253
0.890
0.880
0.857
0.116
0.006
-0.007
-0.120
abundance
0.3
0.2
0.1
0
9.0
8.0
7.0
6.0
5.0
4.0
3.0
2.0
1.0
0
1.00
4.08
0.79
0.97
3.33
4.96
113.22
X : parts per Million : Proton

【Fig. 43】

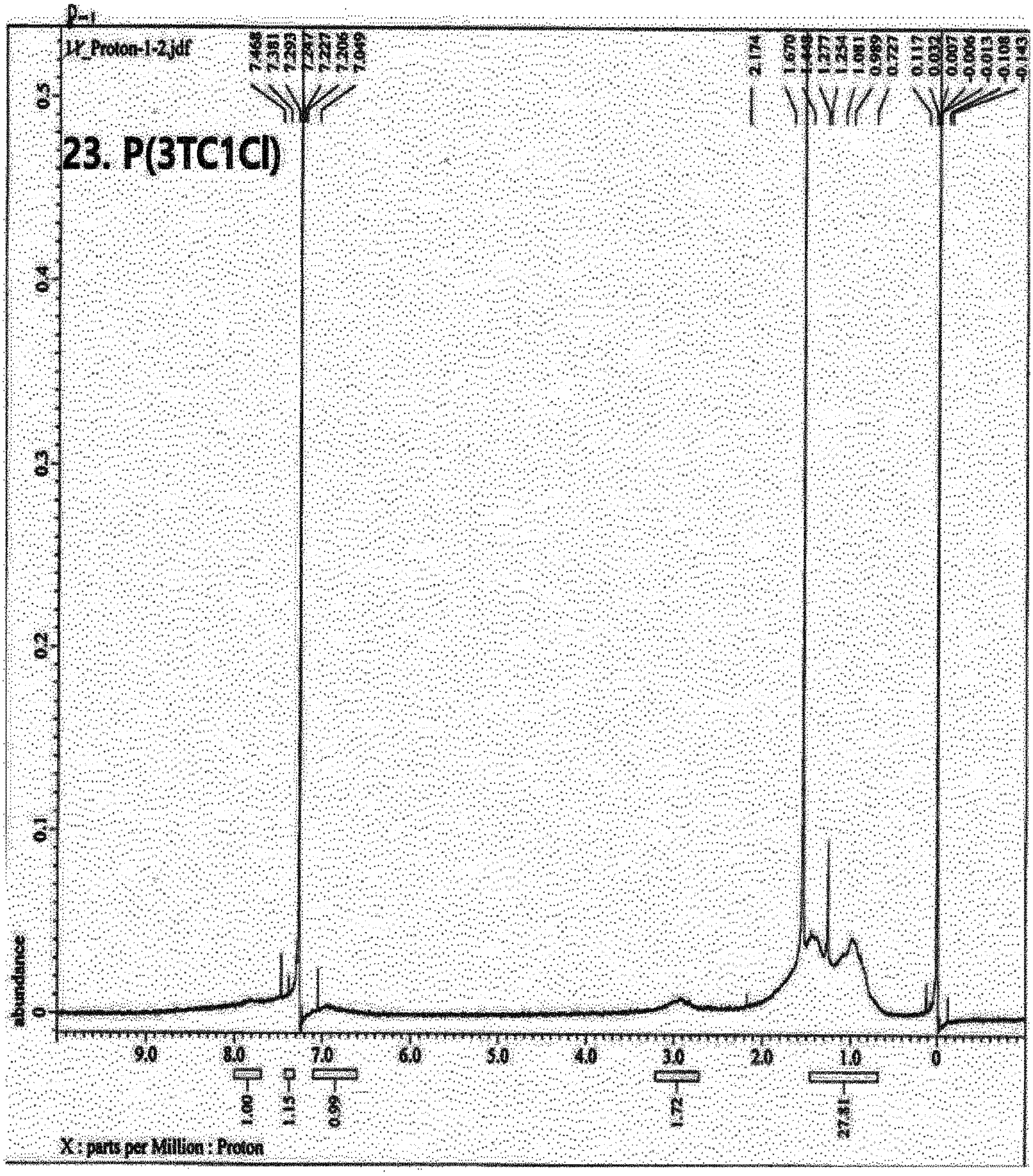
1V_Proton-1-2.jdf
23. P(3TC1Cl)
7.468
7.381
7.293
7.287
7.227
7.206
7.049
2.174
1.670
1.468
1.277
1.254
1.081
0.989
0.727
0.117
0.032
0.007
-0.006
-0.013
-0.108
-0.143
abundance
0
0.1
0.2
0.3
0.4
0.5
9.0
8.0
7.0
6.0
5.0
4.0
3.0
2.0
1.0
0
1.00
1.15
0.99
1.72
27.81
X : parts per Million : Proton

【Fig. 44】

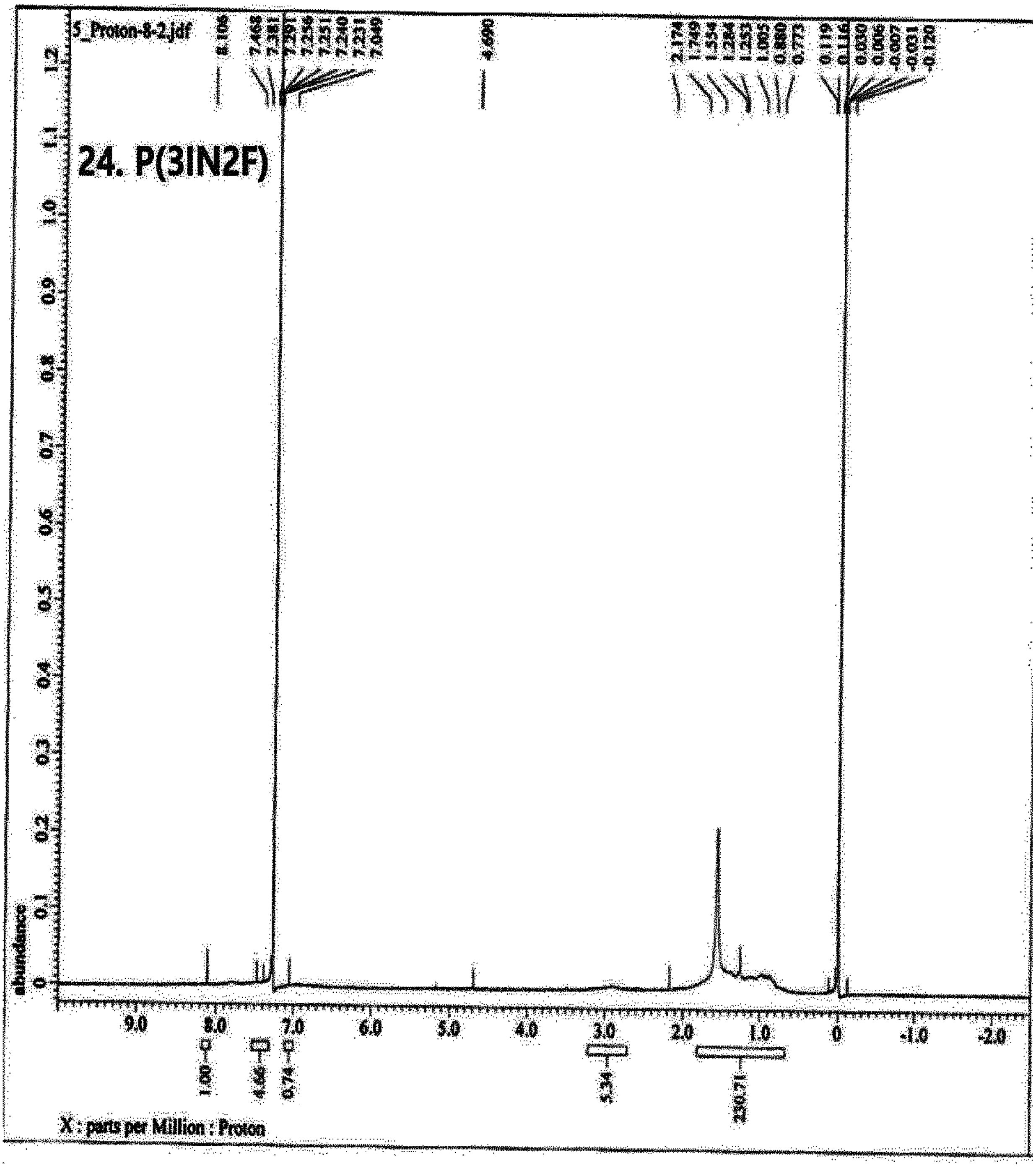
5_Proton-8-2.jdf
24. P(3IN2F)
abundance
X : parts per Million : Proton

【Fig. 45】

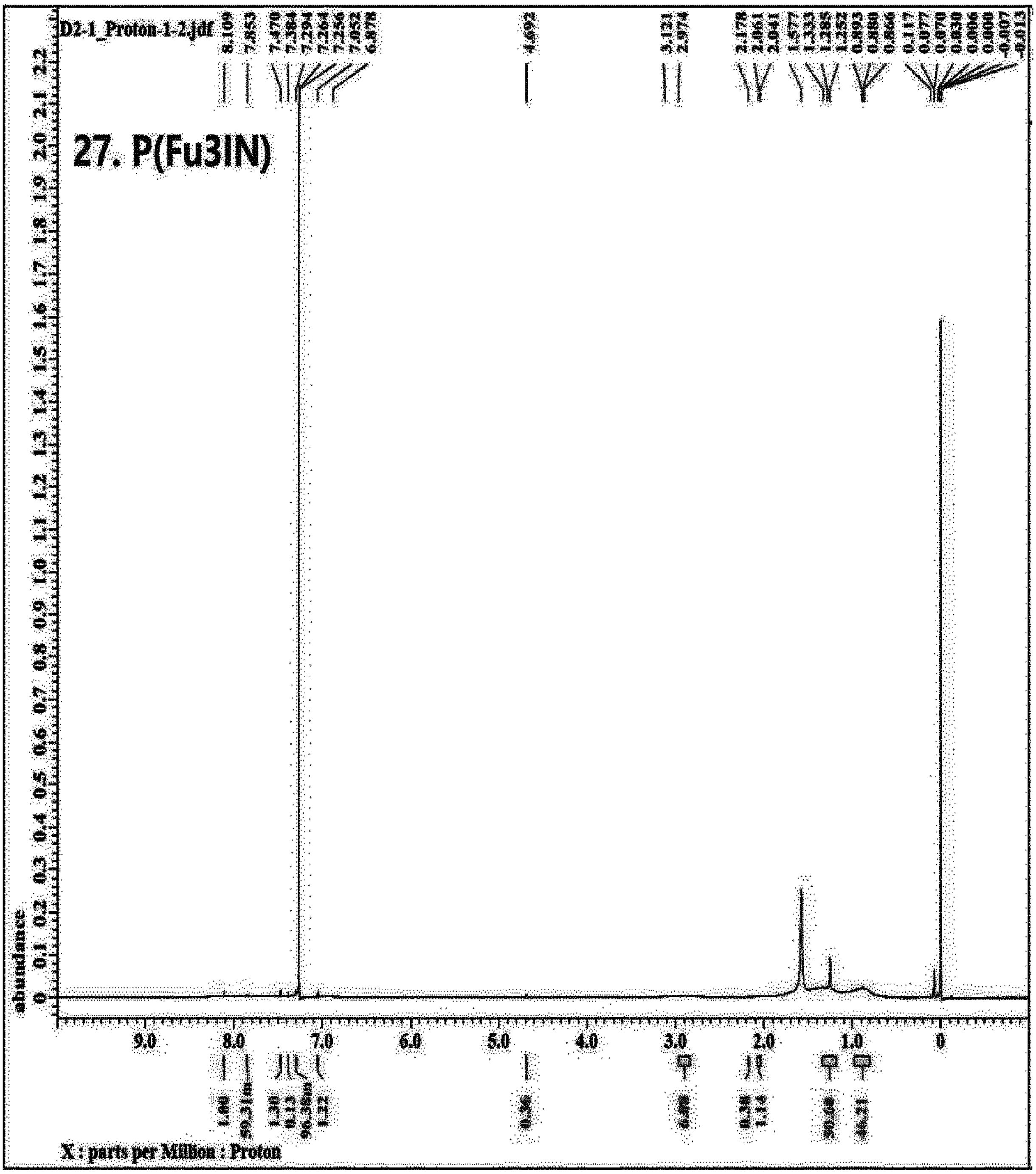
D2-1_Proton-1-2.jdf
27. P(Fu3IN)
abundance
X : parts per Million : Proton

【Fig. 46】

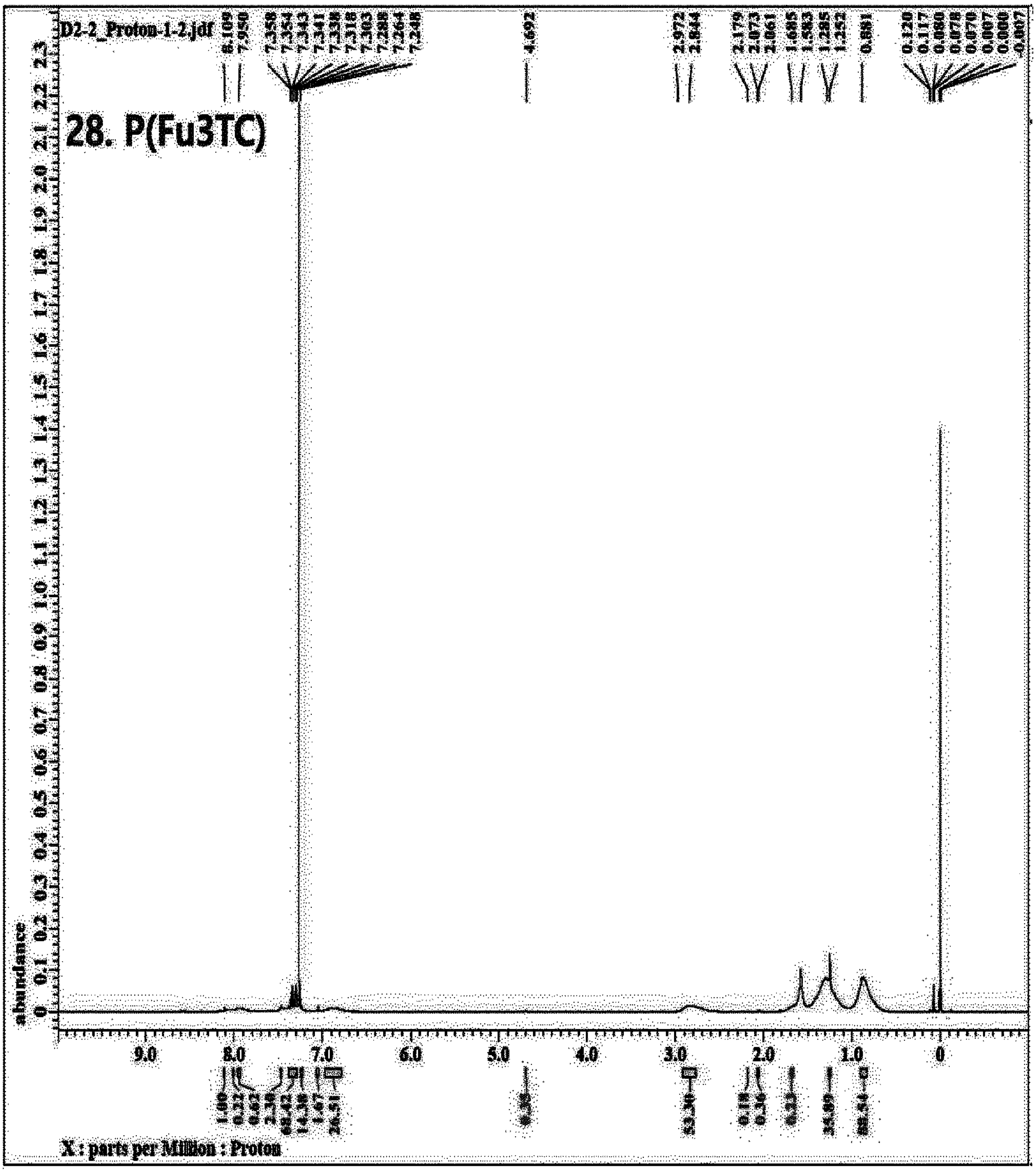
D2-2_Proton-1-2.jdf
28. P(Fu3TC)
abundance
X : parts per Million : Proton

【Fig. 47】

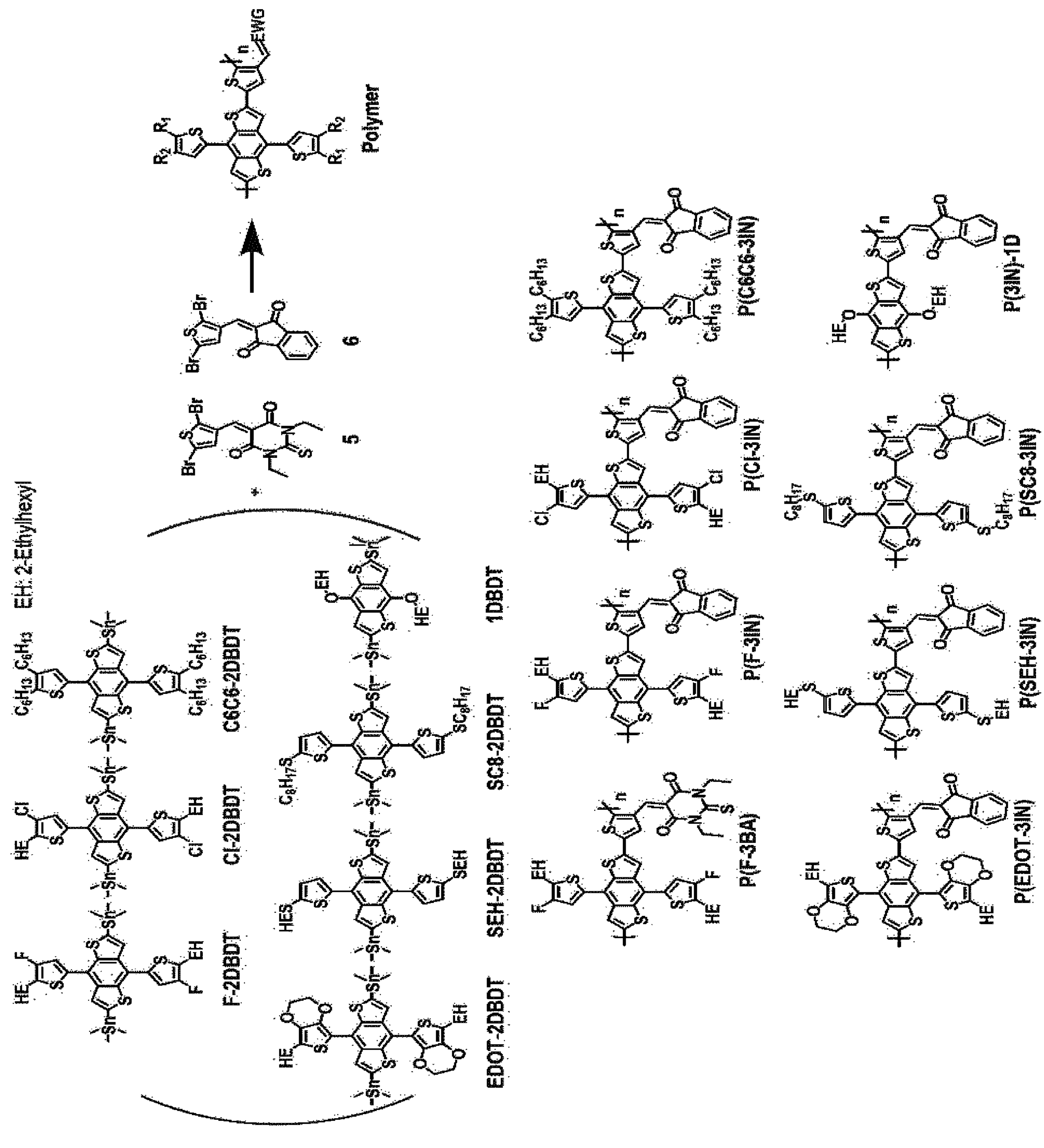

EH: 2-Ethylhexyl

F-2DBDT

Cl-2DBDT

C6C6-2DBDT

EDOT-2DBDT

SEH-2DBDT

SC8-2DBDT

1DBDT

5

6

Polymer

P(F-3BA)

P(F-3IN)

P(Cl-3IN)

P(C6C6-3IN)

P(EDOT-3IN)

P(SEH-3IN)

P(SC8-3IN)

P(3IN)-1D

【Fig. 48】

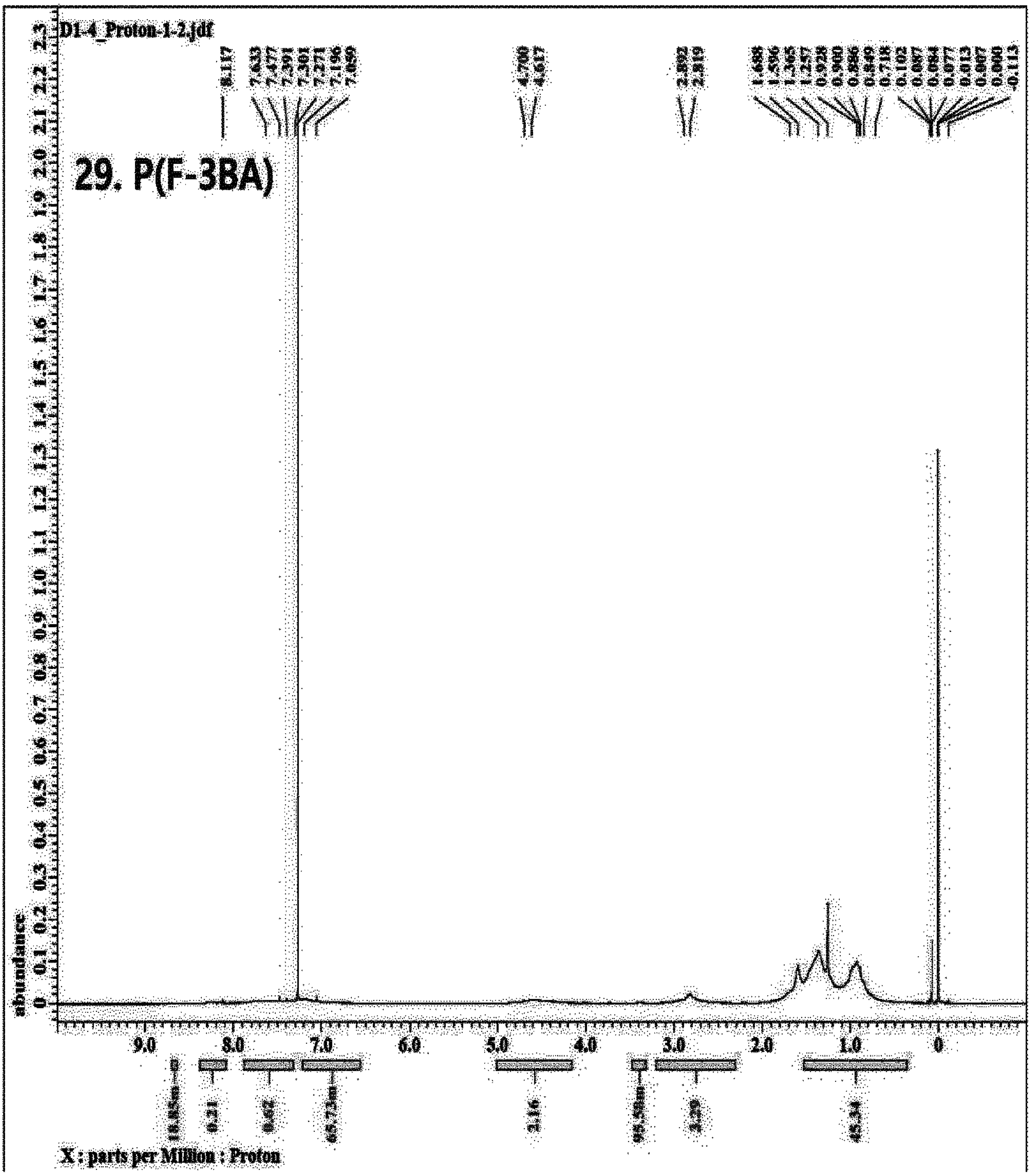
D1-4_Proton-1-2.jdf
29. P(F-3BA)
abundance
X : parts per Million : Proton

【Fig. 49】

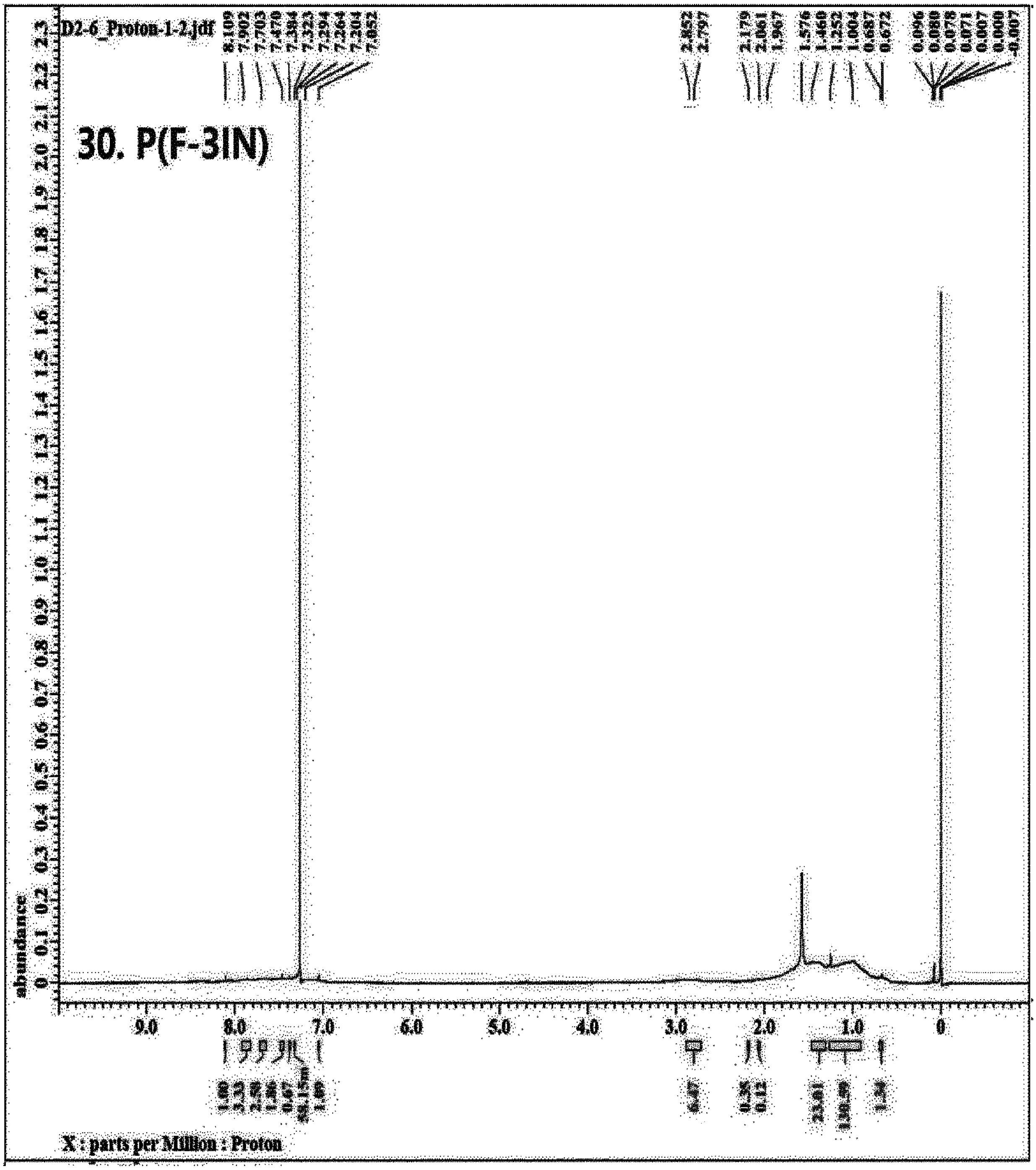
D2-6_Proton-1-2.jdf
30. P(F-3IN)
abundance
X : parts per Million : Proton

【Fig. 50】

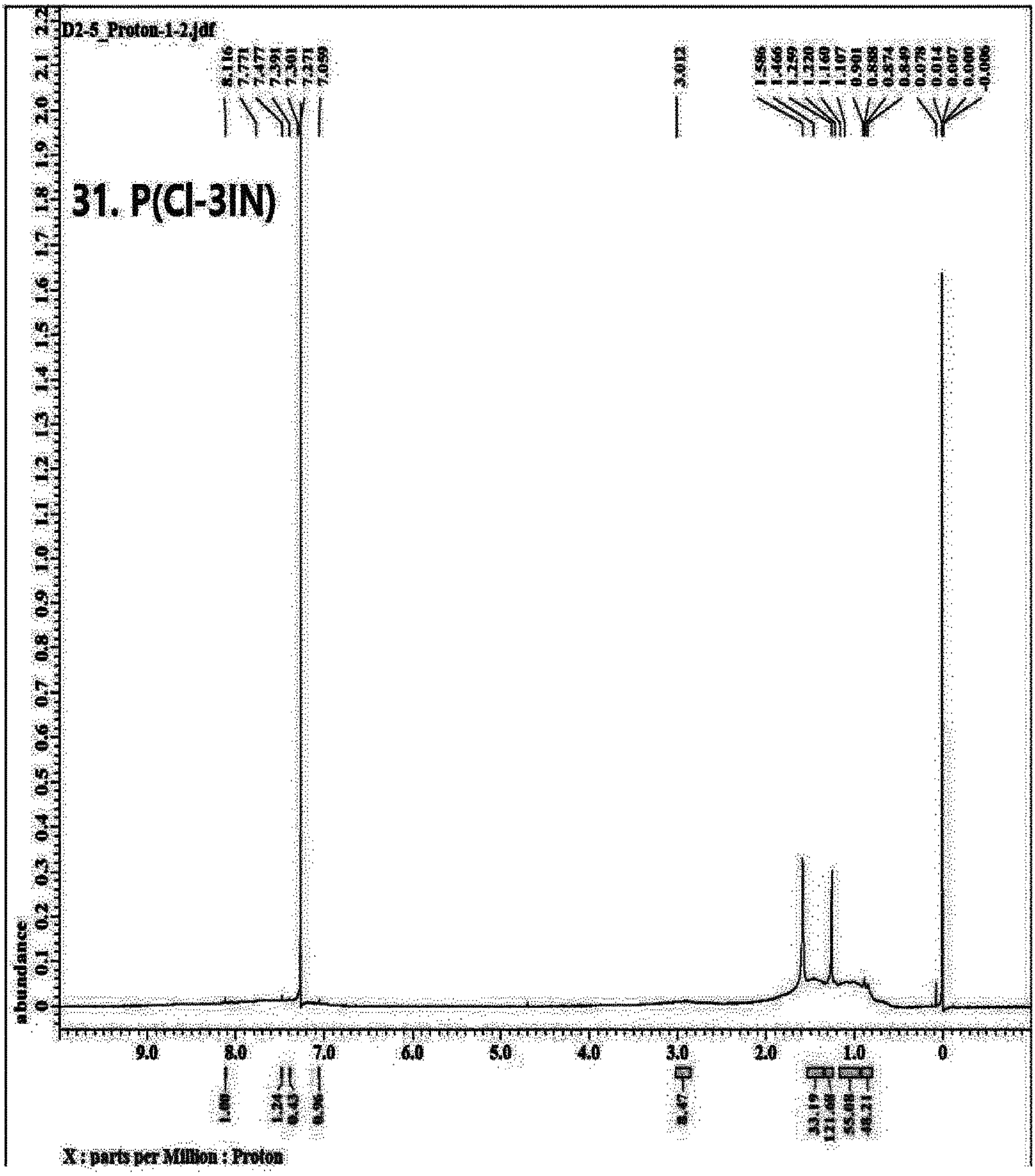
D2-5_Proton-1-2.jdf
31. P(Cl-3IN)
abundance
X : parts per Million : Proton

【Fig. 51】

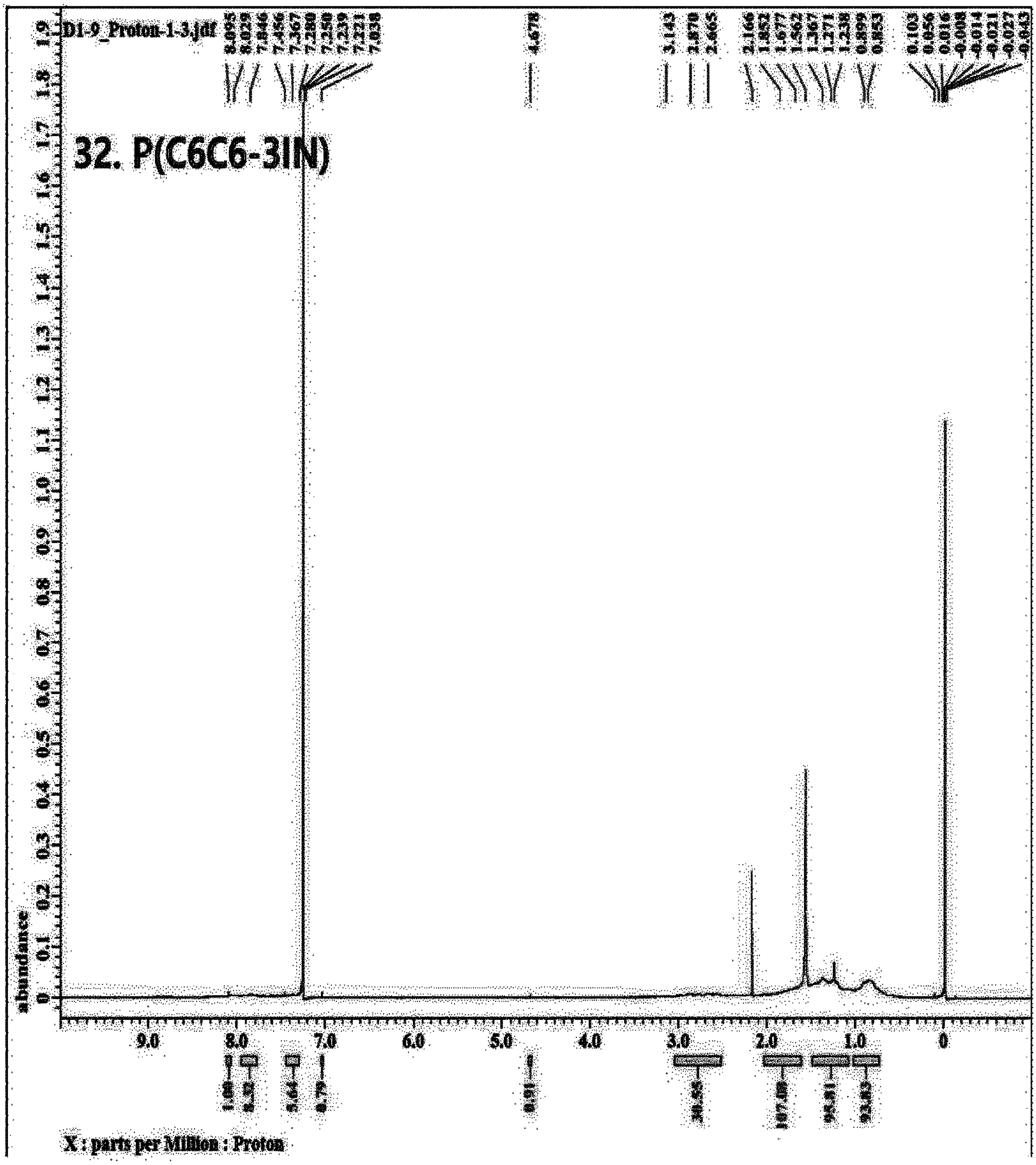
D1-9_Proton-1-3.jdf
32. P(C6C6-3IN)
abundance
X : parts per Million : Proton

【Fig. 52】

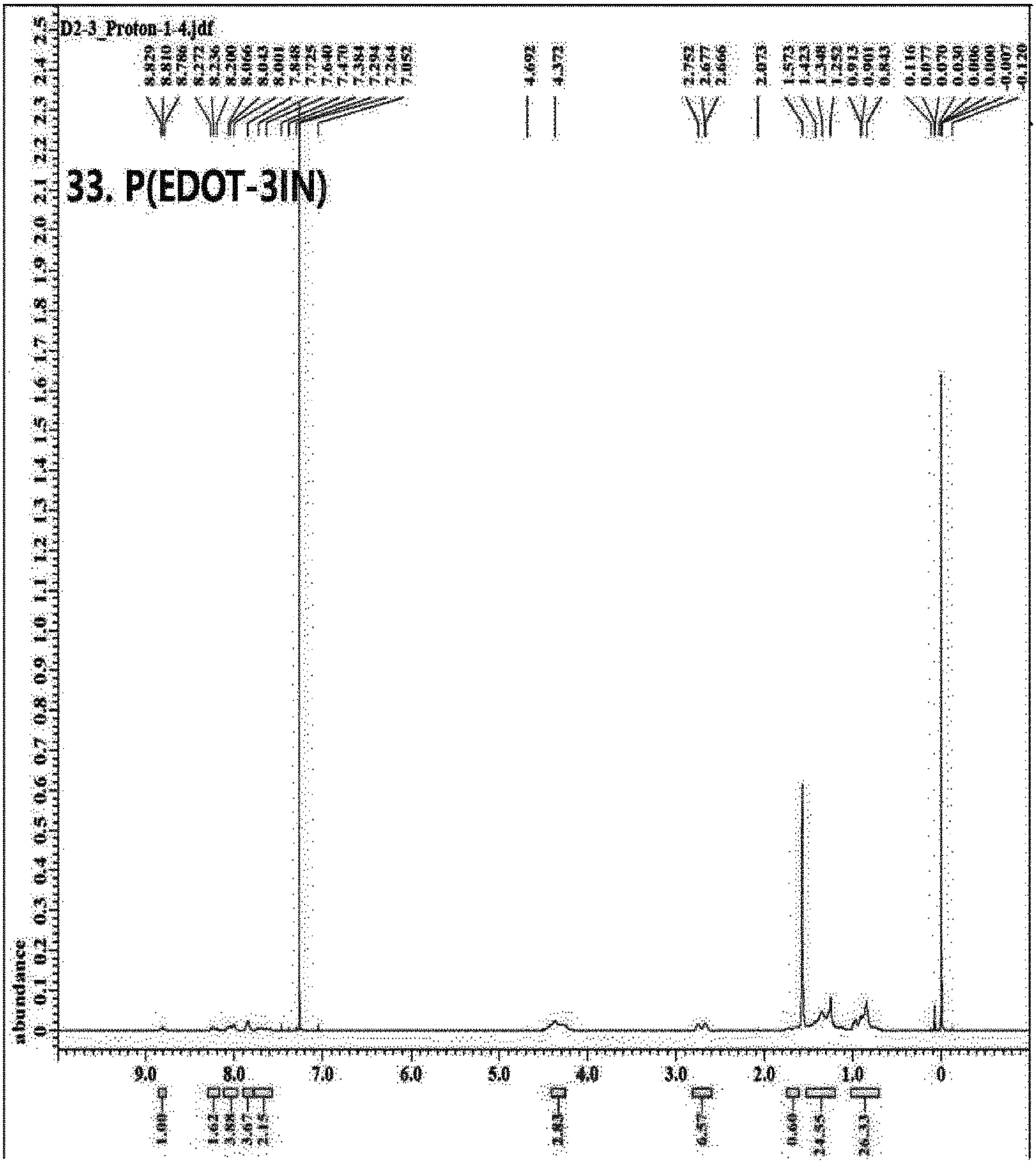
D2-3_Proton-1-4.jdf
33. P(EDOT-3IN)
8.829 8.810 8.786 8.272 8.236 8.200 8.066 8.043 8.001 7.848 7.725 7.640 7.470 7.384 7.294 7.264 7.052
4.692 4.372
2.752 2.677 2.666
2.073
1.573 1.423 1.348 1.252 0.913 0.901 0.843
0.116 0.077 0.070 0.030 0.006 0.000 -0.007 -0.120
abundance
0 0.1 0.2 0.3 0.4 0.5 0.6 0.7 0.8 0.9 1.0 1.1 1.2 1.3 1.4 1.5 1.6 1.7 1.8 1.9 2.0 2.1 2.2 2.3 2.4 2.5
9.0 8.0 7.0 6.0 5.0 4.0 3.0 2.0 1.0 0
1.00 1.62 3.88 3.67 2.15 3.83 6.57 0.60 24.55 26.33

【Fig. 53】

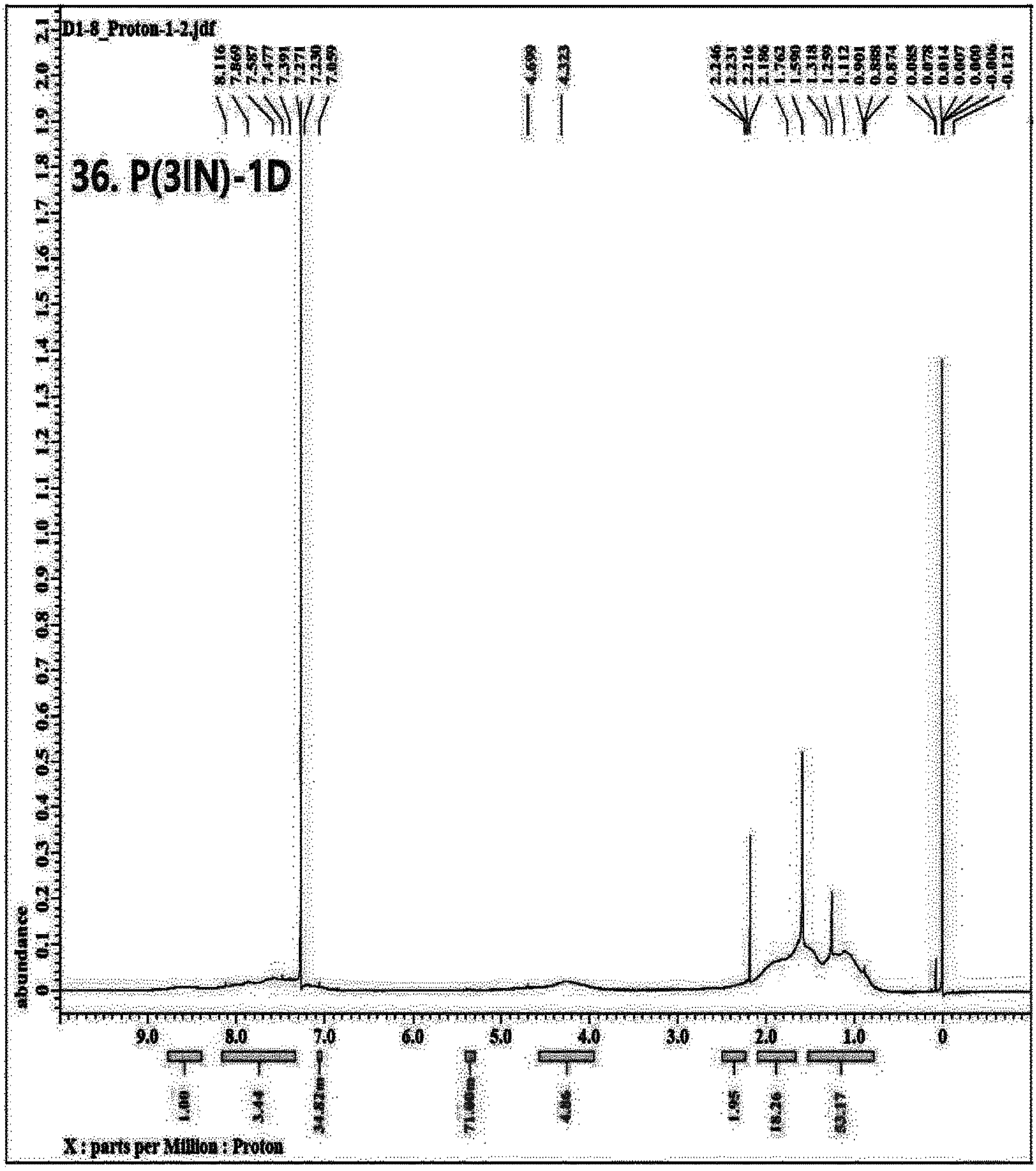
D1-8_Proton-1-2.jdf
36. P(3IN)-1D
abundance
X : parts per Million : Proton

【Fig. 54】

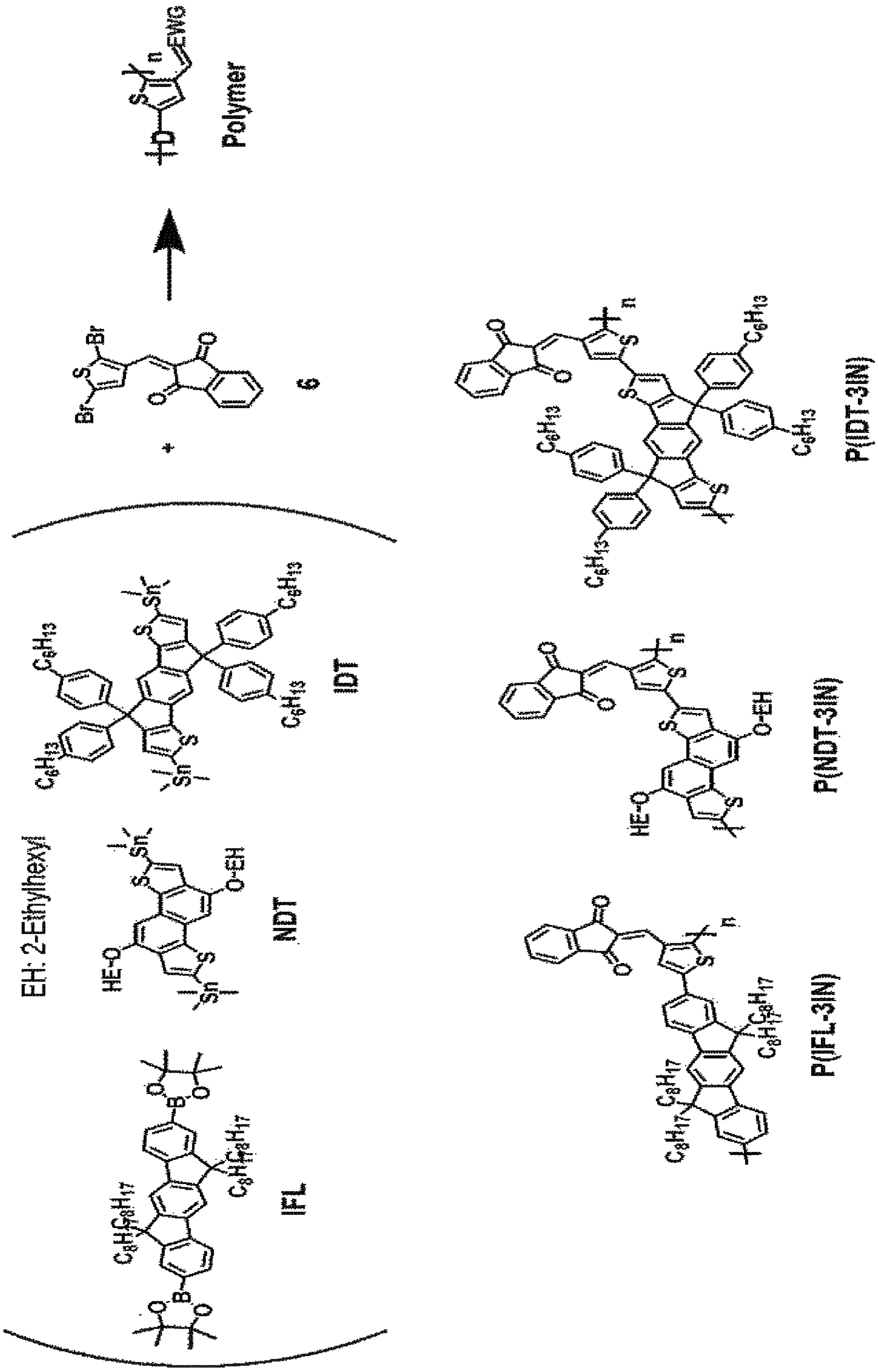

EH: 2-Ethylhexyl

IFL

NDT

IDT

6

Polymer

P(IFL-3IN)

P(NDT-3IN)

P(IDT-3IN)

【Fig. 55】

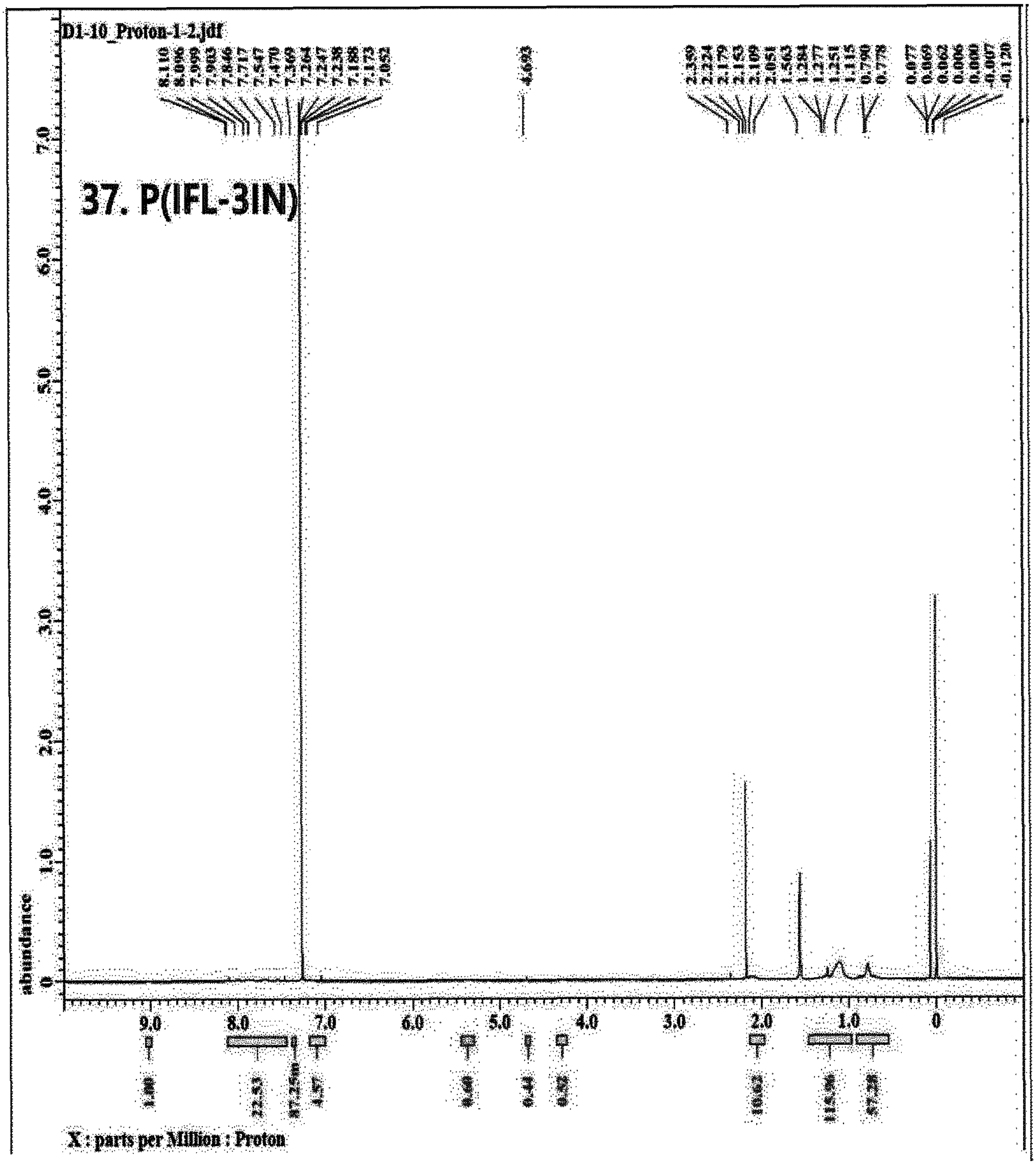
D1-10_Proton-1-2.jdf
37. P(IFL-3IN)
abundance
X : parts per Million : Proton

【Fig. 56】

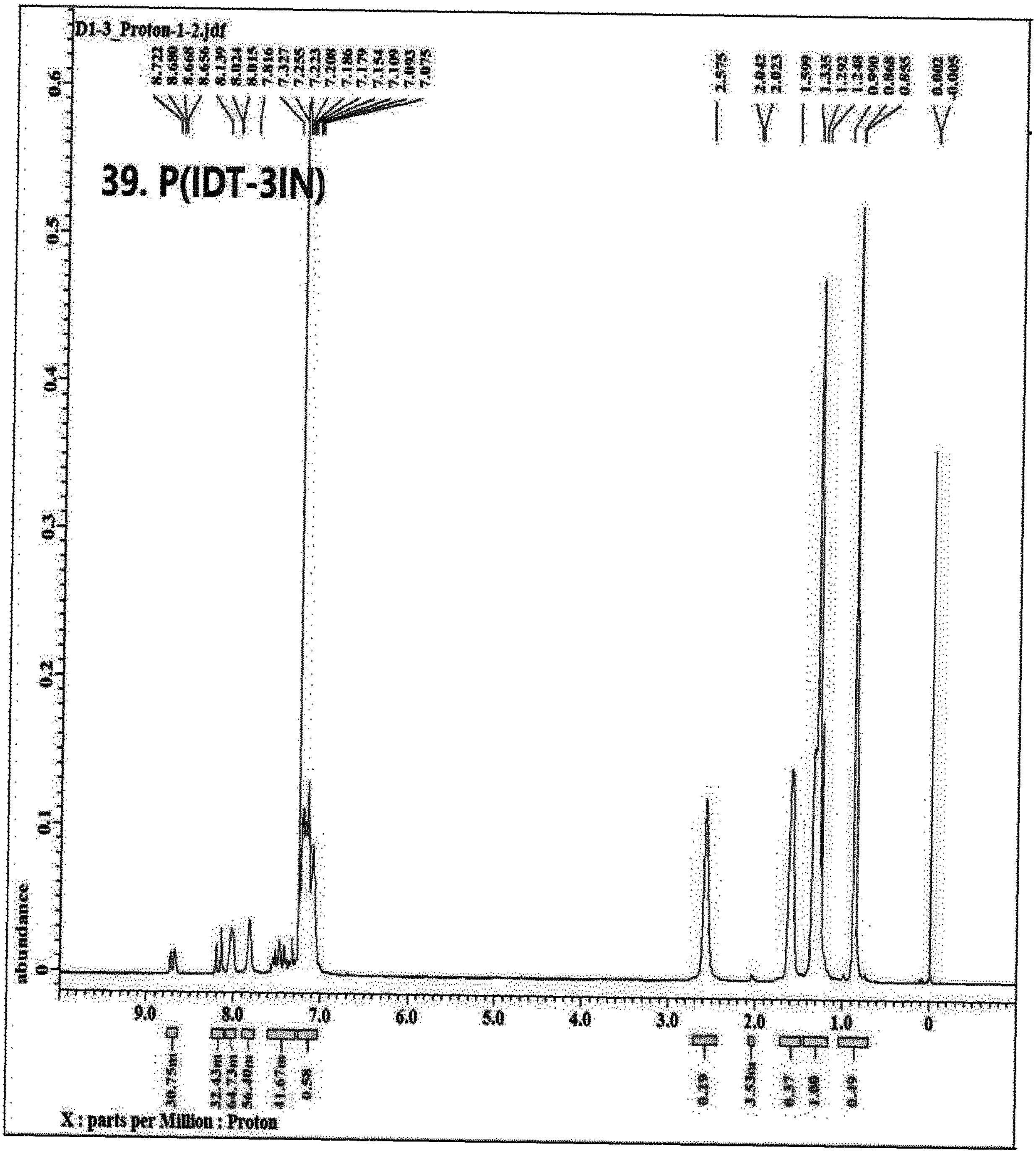
D1-3_Proton-1-2.jdf
39. P(IDT-3IN)
abundance
X : parts per Million : Proton

【Fig. 57】

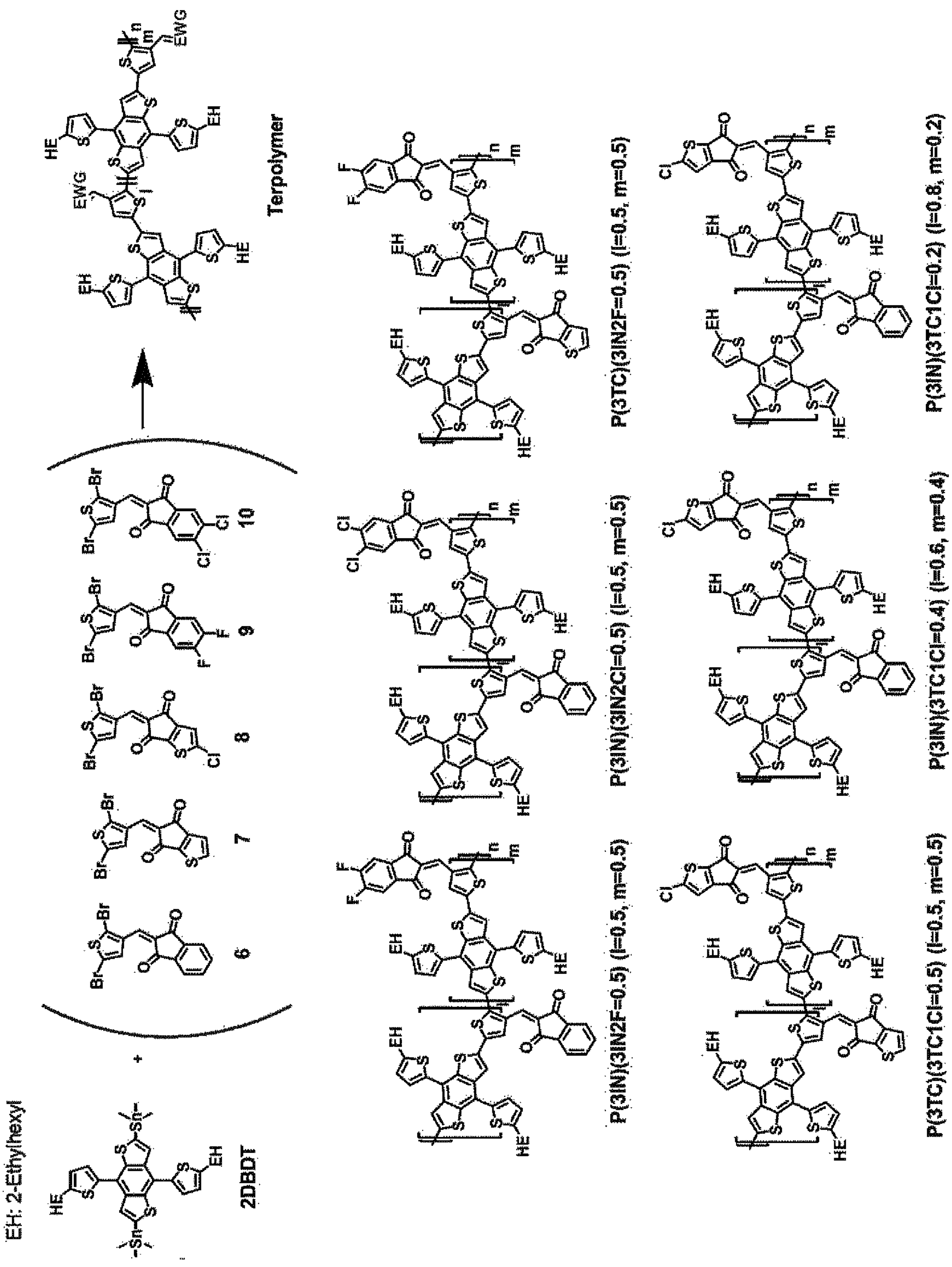

EH: 2-Ethylhexyl

2DBDT

6

7

8

9

10

Terpolymer

P(3IN)(3IN2F=0.5) (l=0.5, m=0.5)

P(3IN)(3IN2Cl=0.5) (l=0.5, m=0.5)

P(3TC)(3IN2F=0.5) (l=0.5, m=0.5)

P(3TC)(3TC1Cl=0.5) (l=0.5, m=0.5)

P(3IN)(3TC1Cl=0.4) (l=0.6, m=0.4)

P(3IN)(3TC1Cl=0.2) (l=0.8, m=0.2)

【Fig. 58】

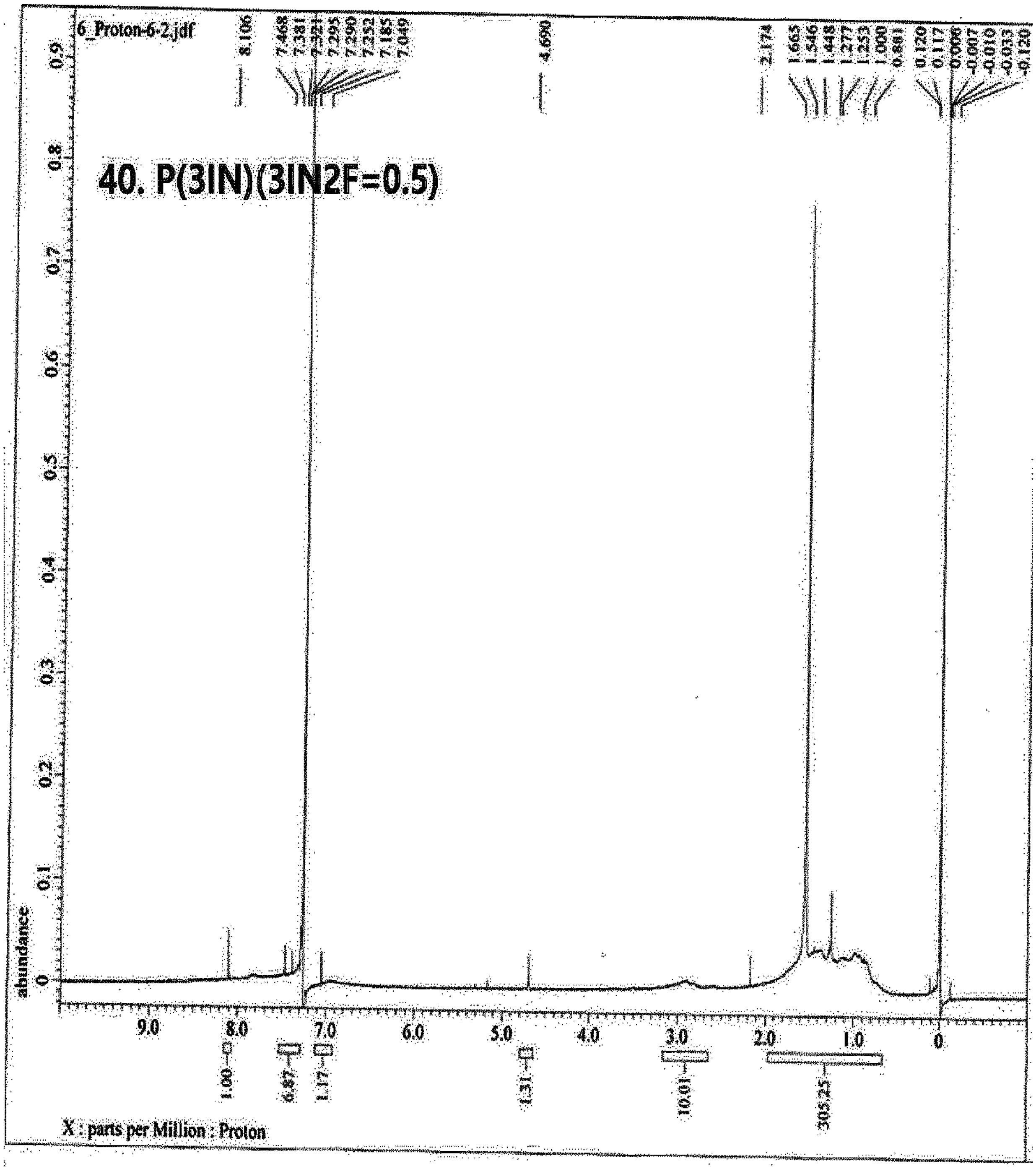
6_Proton-6-2.jdf
40. P(3IN)(3IN2F=0.5)
8.106
7.468
7.381
7.321
7.295
7.290
7.252
7.185
7.049
4.690
2.174
1.665
1.546
1.448
1.277
1.253
1.000
0.881
0.120
0.117
0.006
-0.007
-0.010
-0.033
-0.120
abundance
0.9
0.8
0.7
0.6
0.5
0.4
0.3
0.2
0.1
0
9.0
8.0
7.0
6.0
5.0
4.0
3.0
2.0
1.0
0
1.00
6.87
1.17
1.31
10.01
305.25
X : parts per Million : Proton

【Fig. 59】

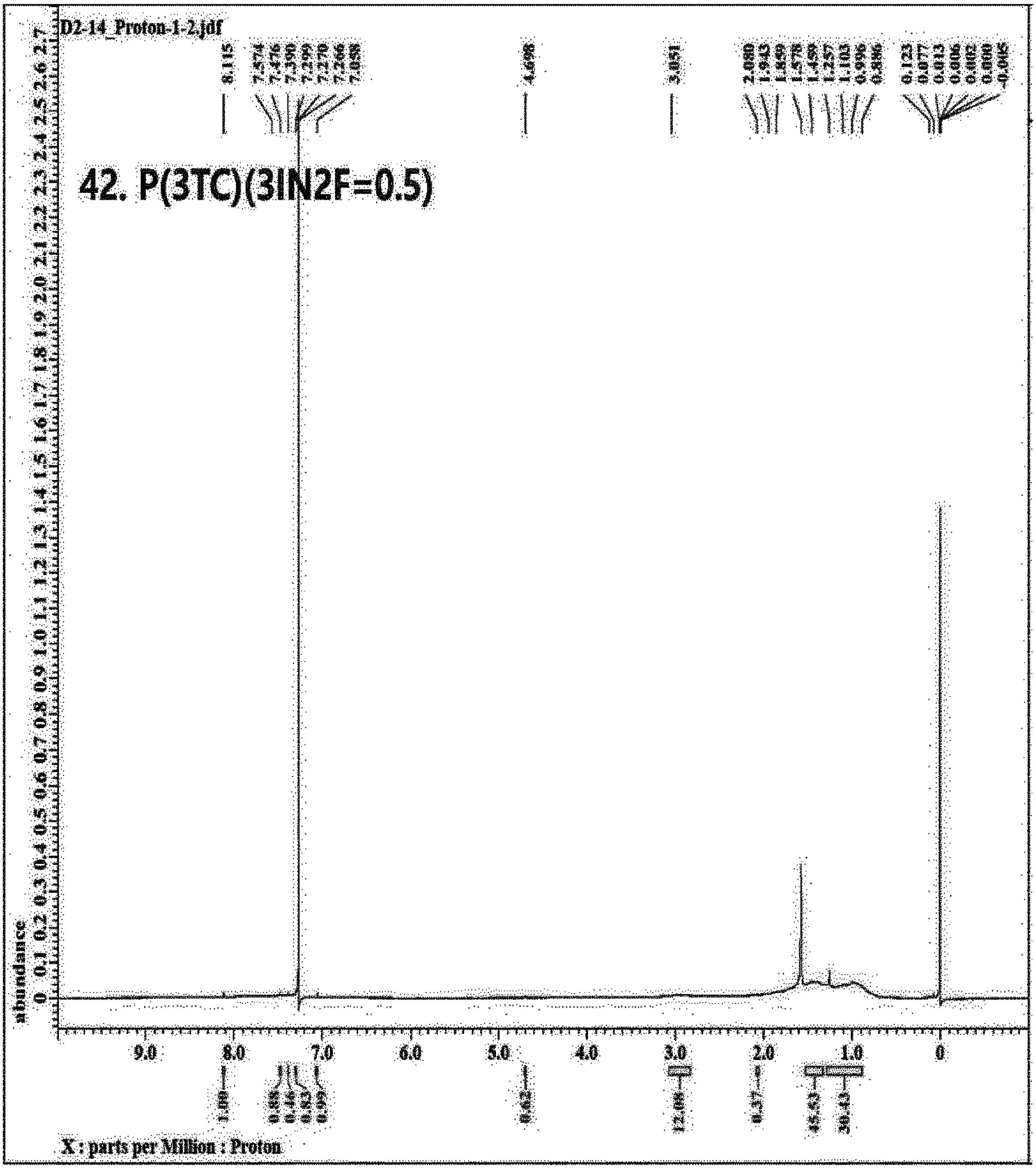
D2-14_Proton-1-2.jdf
42. P(3TC)(3IN2F=0.5)
abundance
X : parts per Million : Proton

【Fig. 60】

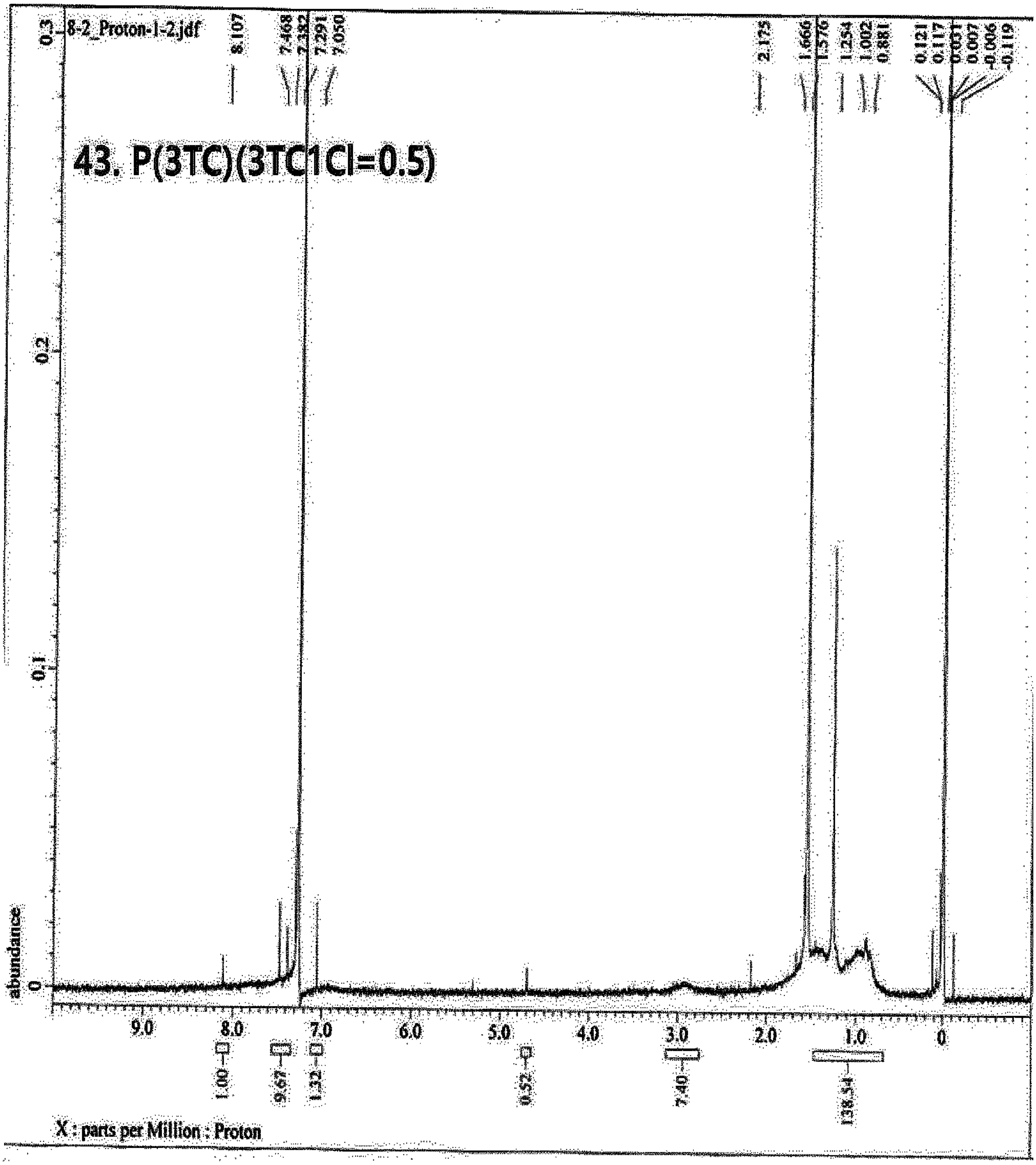
8-2_Proton-1-2.jdf
43. P(3TC)(3TC1Cl=0.5)
8.107
7.468
7.382
7.291
7.050
2.175
1.666
1.576
1.254
1.002
0.881
0.121
0.117
0.031
0.007
-0.006
-0.119
abundance
0
0.1
0.2
0.3
9.0
8.0
7.0
6.0
5.0
4.0
3.0
2.0
1.0
0
1.00
9.67
1.32
0.52
7.40
138.34
X : parts per Million : Proton

【Fig. 61】

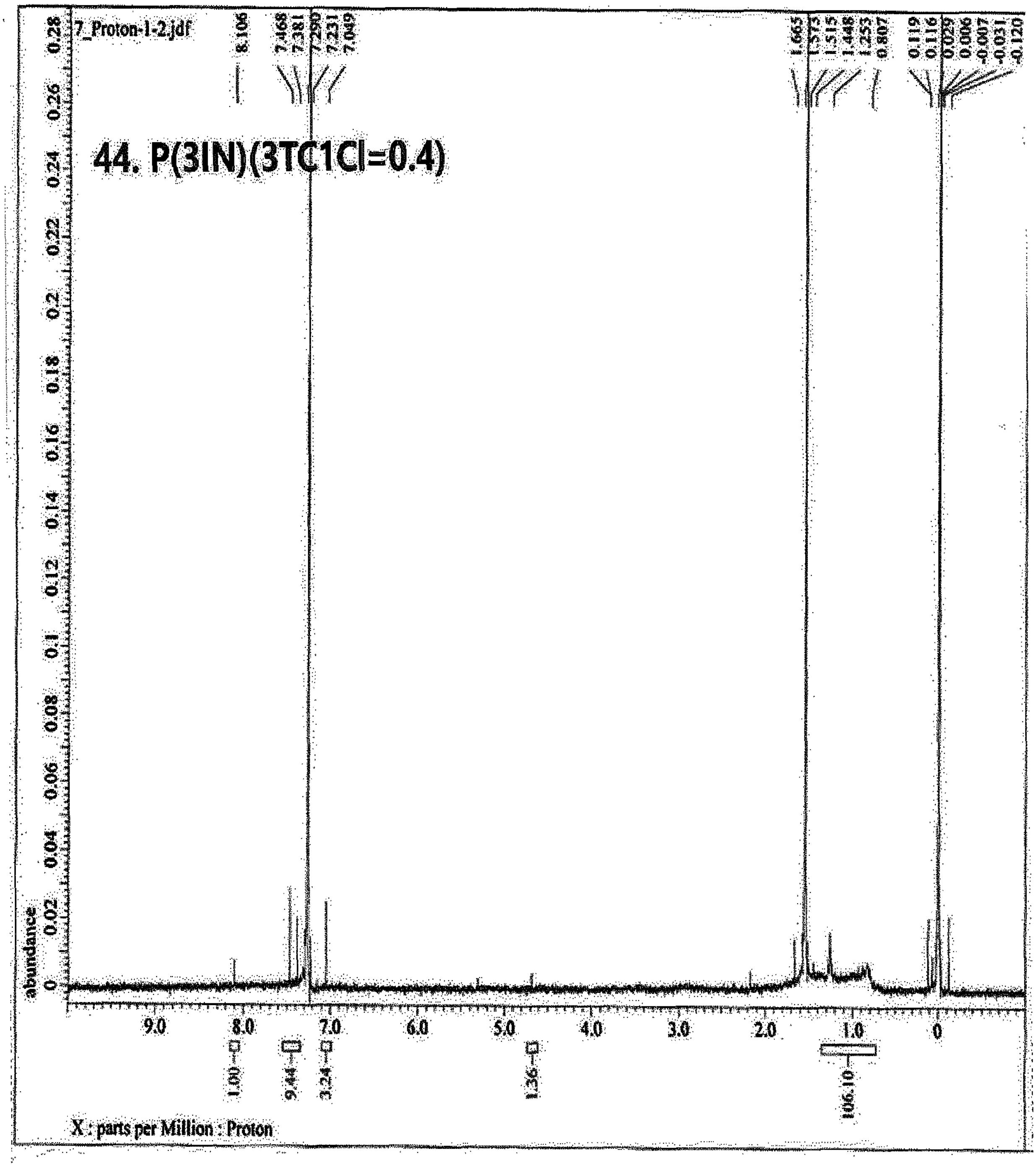

7_Proton-1-2.jdf
44. P(3IN)(3TC1Cl=0.4)
8.106
7.468
7.381
7.290
7.231
7.049
1.665
1.573
1.515
1.448
1.253
0.807
0.119
0.116
0.029
0.006
-0.007
-0.031
-0.120
abundance
0
0.02
0.04
0.06
0.08
0.1
0.12
0.14
0.16
0.18
0.2
0.22
0.24
0.26
0.28
9.0
8.0
7.0
6.0
5.0
4.0
3.0
2.0
1.0
0
1.00
9.44
3.24
1.36
106.10
X : parts per Million : Proton

【Fig. 62】

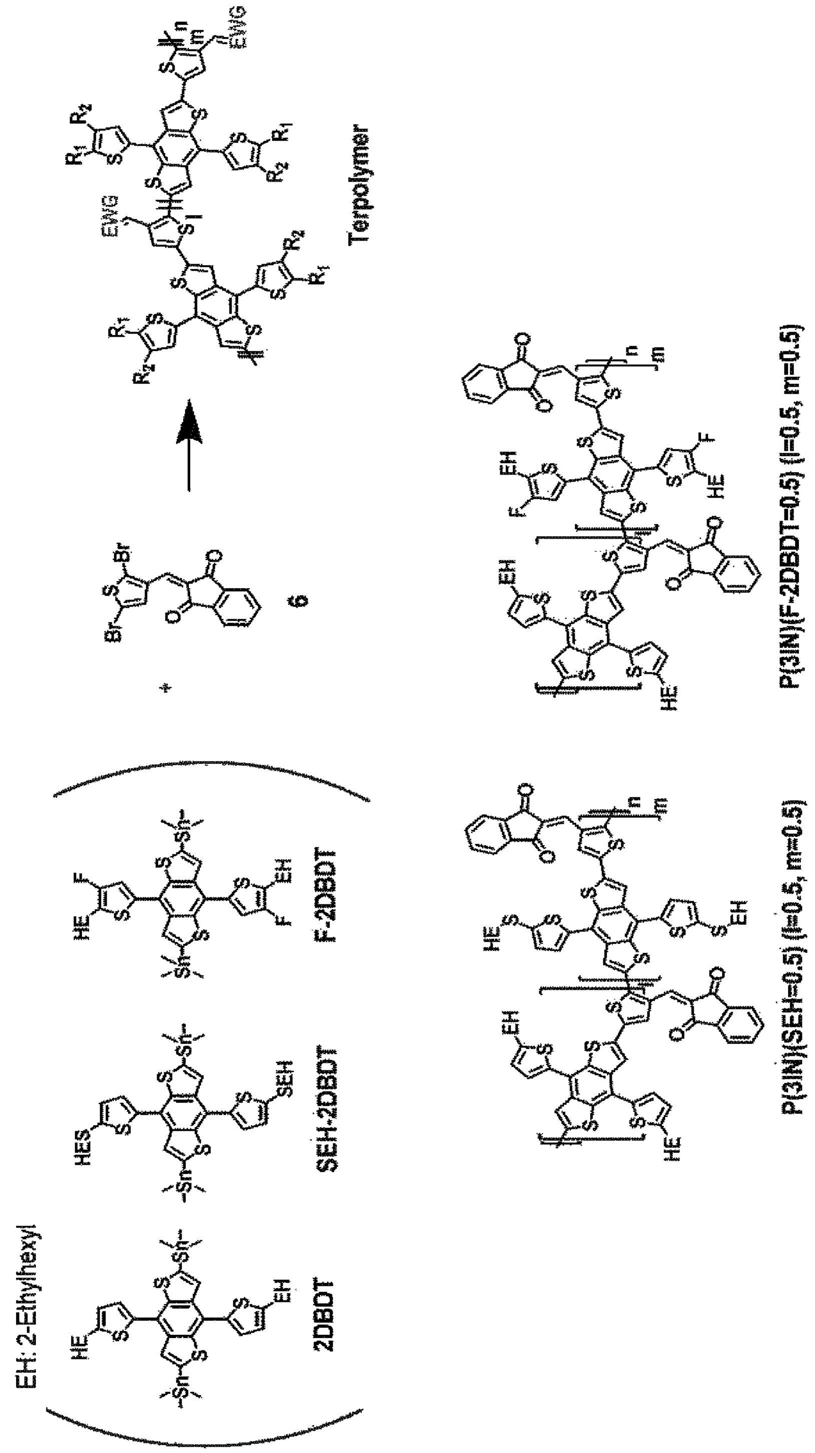
EH: 2-Ethylhexyl
2DBDT
SEH-2DBDT
F-2DBDT
6
Terpolymer
P(3IN)(SEH=0.5) (l=0.5, m=0.5)
P(3IN)(F-2DBDT=0.5) (l=0.5, m=0.5)

【Fig. 63】

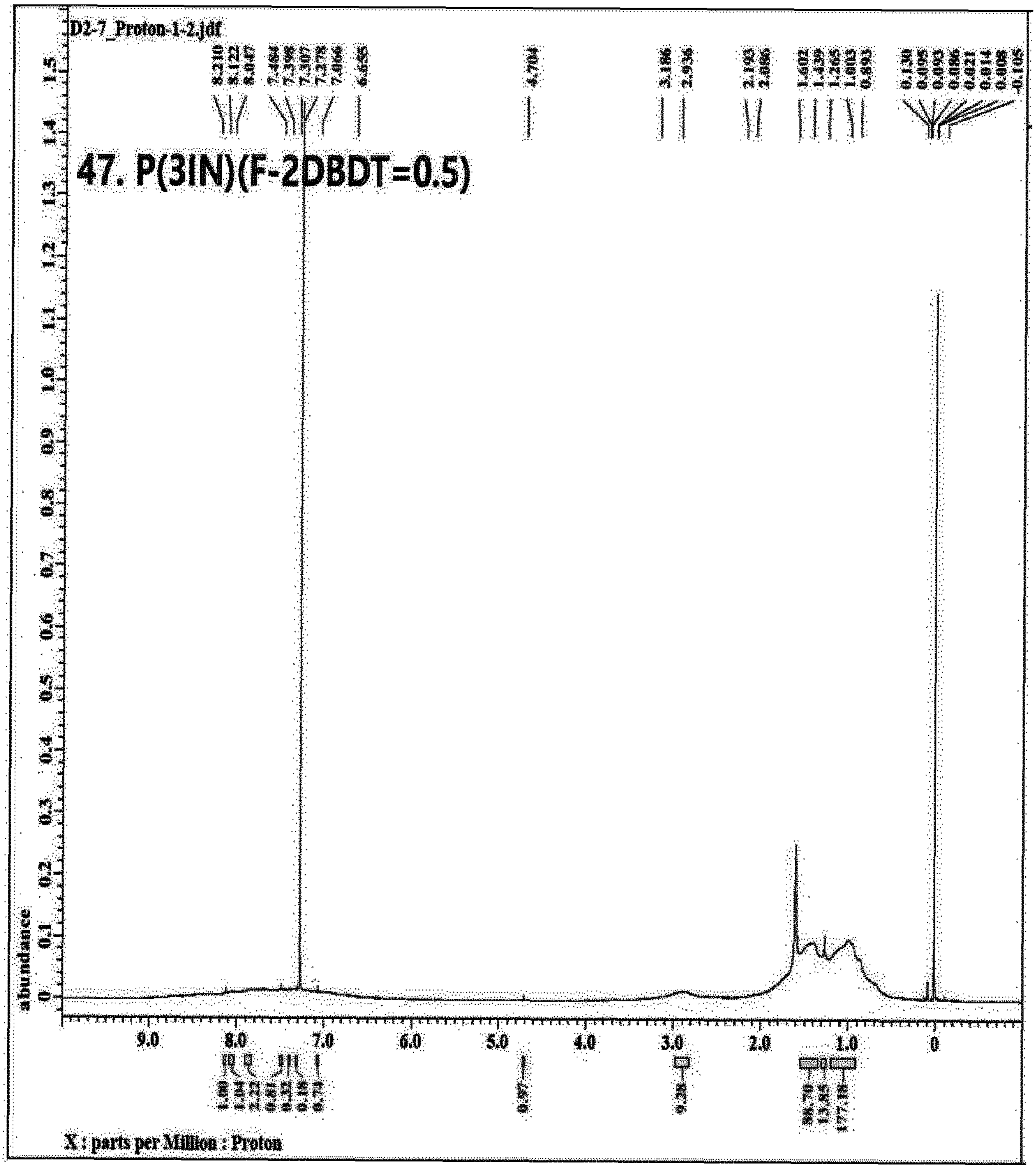
D2-7_Proton-1-2.jdf
47. P(3IN)(F-2DBDT=0.5)
8.210
8.122
8.047
7.484
7.398
7.307
7.278
7.066
6.655
4.704
3.186
2.936
2.193
2.086
1.602
1.439
1.265
1.003
0.893
0.130
0.095
0.093
0.086
0.021
0.014
0.008
-0.105
abundance
X : parts per Million : Proton

【Fig. 64】

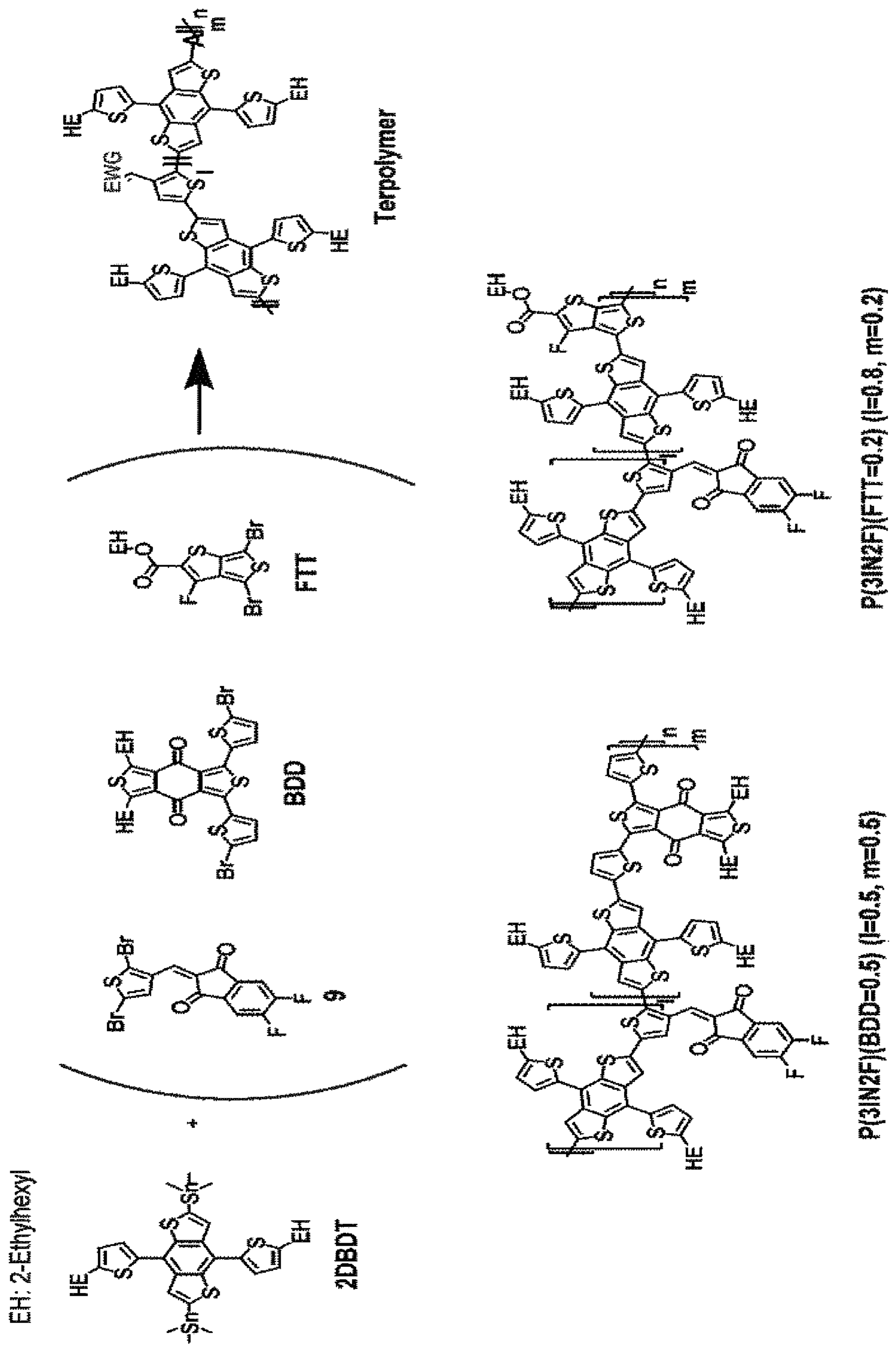

EH: 2-Ethylhexyl

2DBDT

9

BDD

FTT

Terpolymer

P(3IN2F)(BDD=0.5) (l=0.5, m=0.5)

P(3IN2F)(FTT=0.2) (l=0.8, m=0.2)

【Fig. 65】

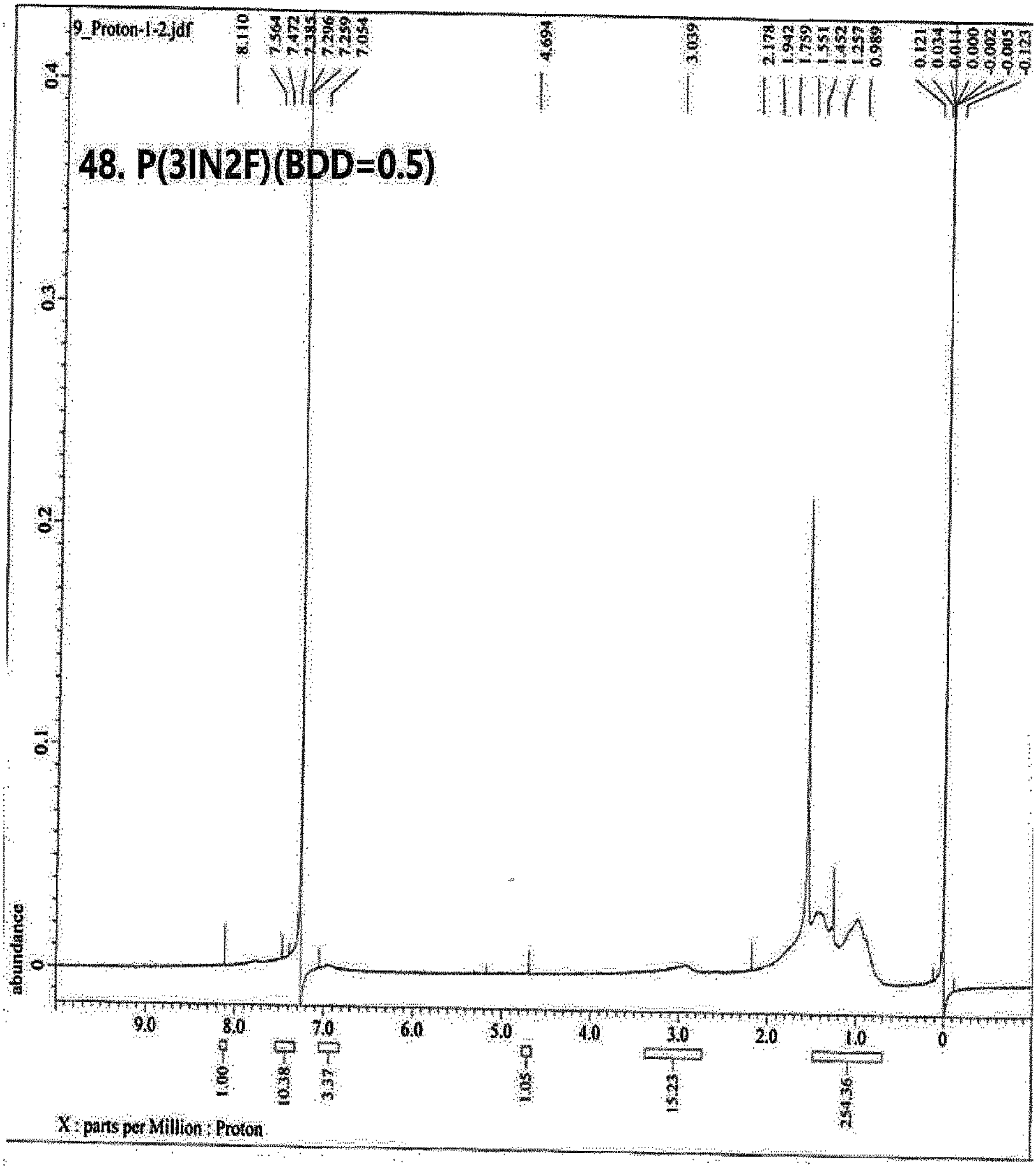
9_Proton-1-2.jdf
8.110
7.564
7.472
7.385
7.206
7.259
7.054
4.694
3.039
2.178
1.942
1.759
1.551
1.452
1.257
0.989
0.121
0.034
0.014
0.000
-0.002
-0.005
-0.123
48. P(3IN2F)(BDD=0.5)
abundance
0
0.1
0.2
0.3
0.4
9.0
8.0
7.0
6.0
5.0
4.0
3.0
2.0
1.0
0
1.00
10.38
3.37
1.05
15.23
254.36
X : parts per Million : Proton

【Fig. 66】

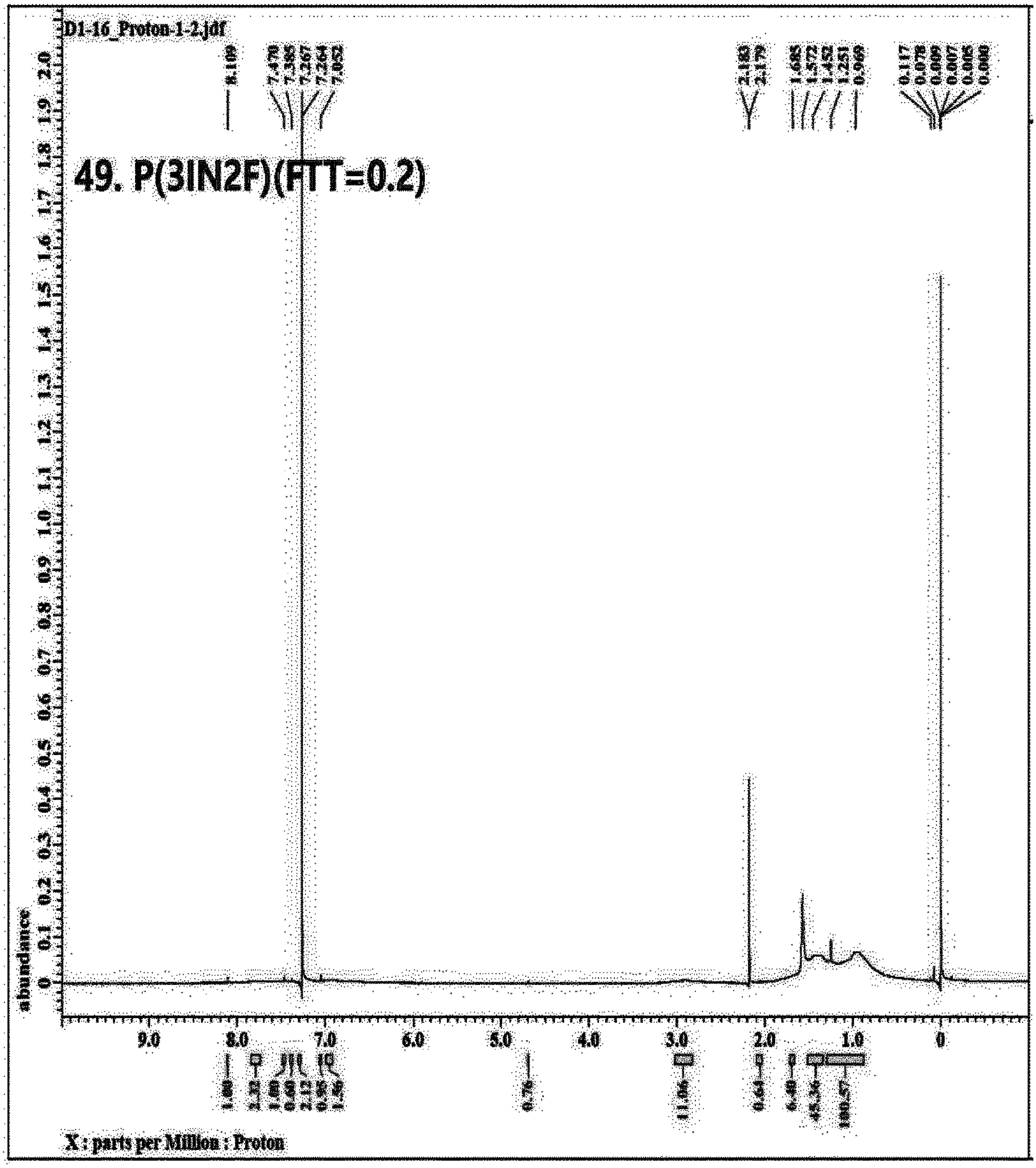
D1-16_Proton-1-2.jdf
49. P(3IN2F)(FTT=0.2)
abundance
X : parts per Million : Proton

【Fig. 67】

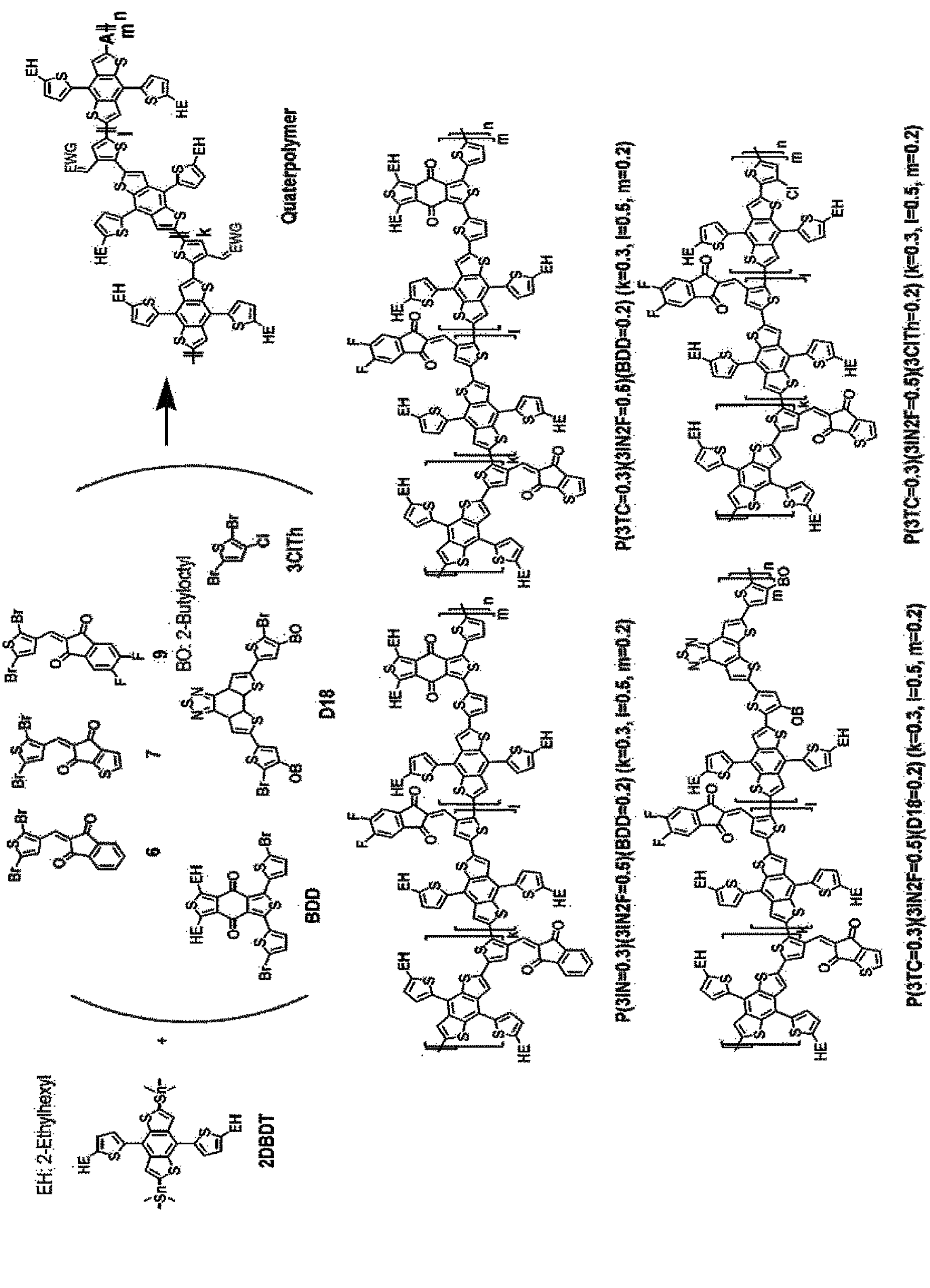

EH: 2-Ethylhexyl

2DBDT

6

7

9

BO: 2-Butyloctyl

BDD

D18

3ClTh

Quaterpolymer

P(3IN=0.3)(3IN2F=0.5)(BDD=0.2) (k=0.3, l=0.5, m=0.2)

P(3TC=0.3)(3IN2F=0.5)(BDD=0.2) (k=0.3, l=0.5, m=0.2)

P(3TC=0.3)(3IN2F=0.5)(D18=0.2) (k=0.3, l=0.5, m=0.2)

P(3TC=0.3)(3IN2F=0.5)(3ClTh=0.2) (k=0.3, l=0.5, m=0.2)

【Fig. 68】

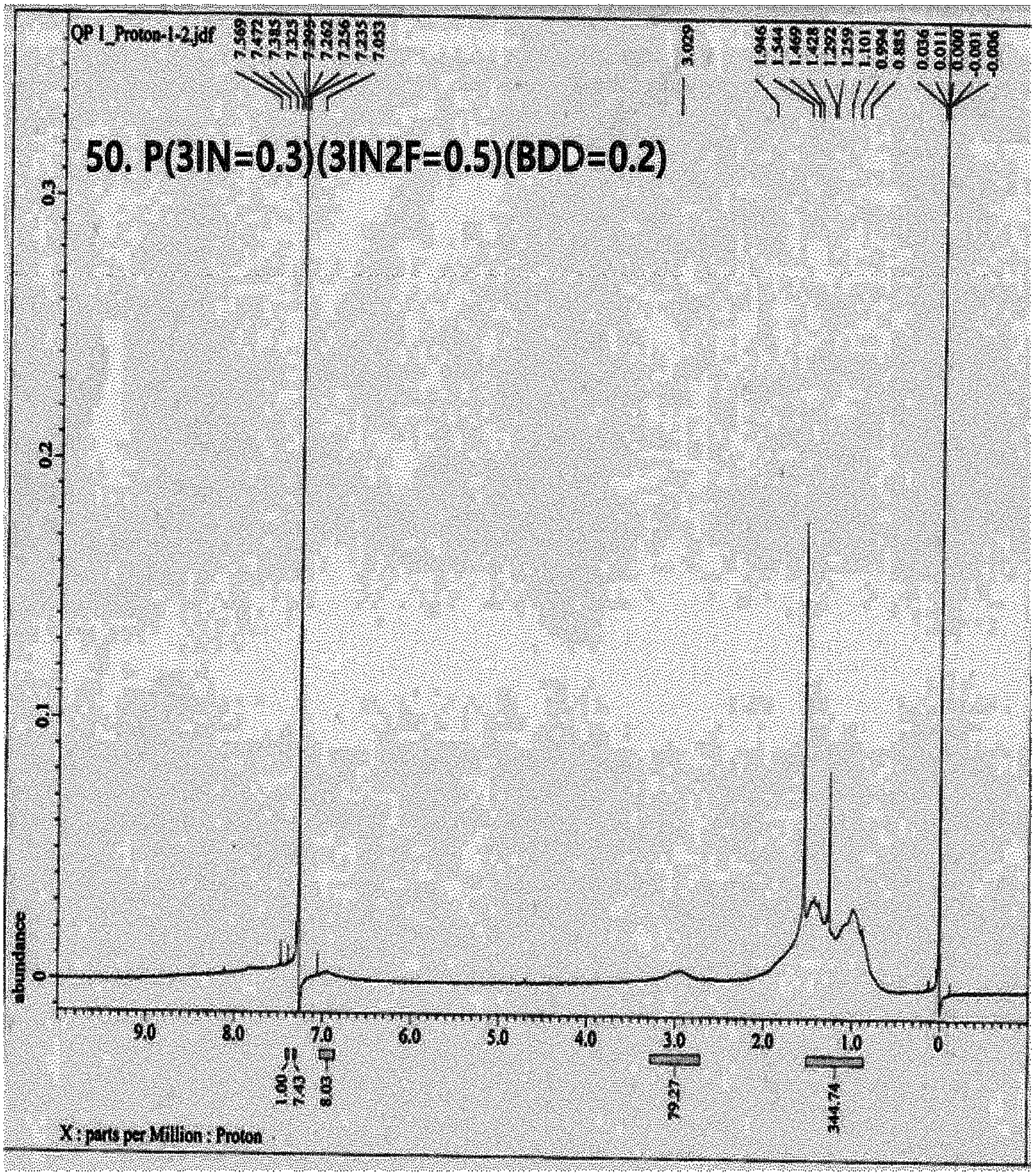
QP I_Proton-1-2.jdf
50. P(3IN=0.3)(3IN2F=0.5)(BDD=0.2)
7.569
7.472
7.385
7.325
7.296
7.262
7.236
7.235
7.053
3.029
1.946
1.544
1.469
1.428
1.292
1.259
1.101
0.994
0.885
0.036
0.011
0.000
-0.001
-0.006
abundance
0
0.1
0.2
0.3
9.0
8.0
7.0
6.0
5.0
4.0
3.0
2.0
1.0
0
1.00
7.43
8.03
79.27
344.74
X : parts per Million : Proton

【Fig. 69】

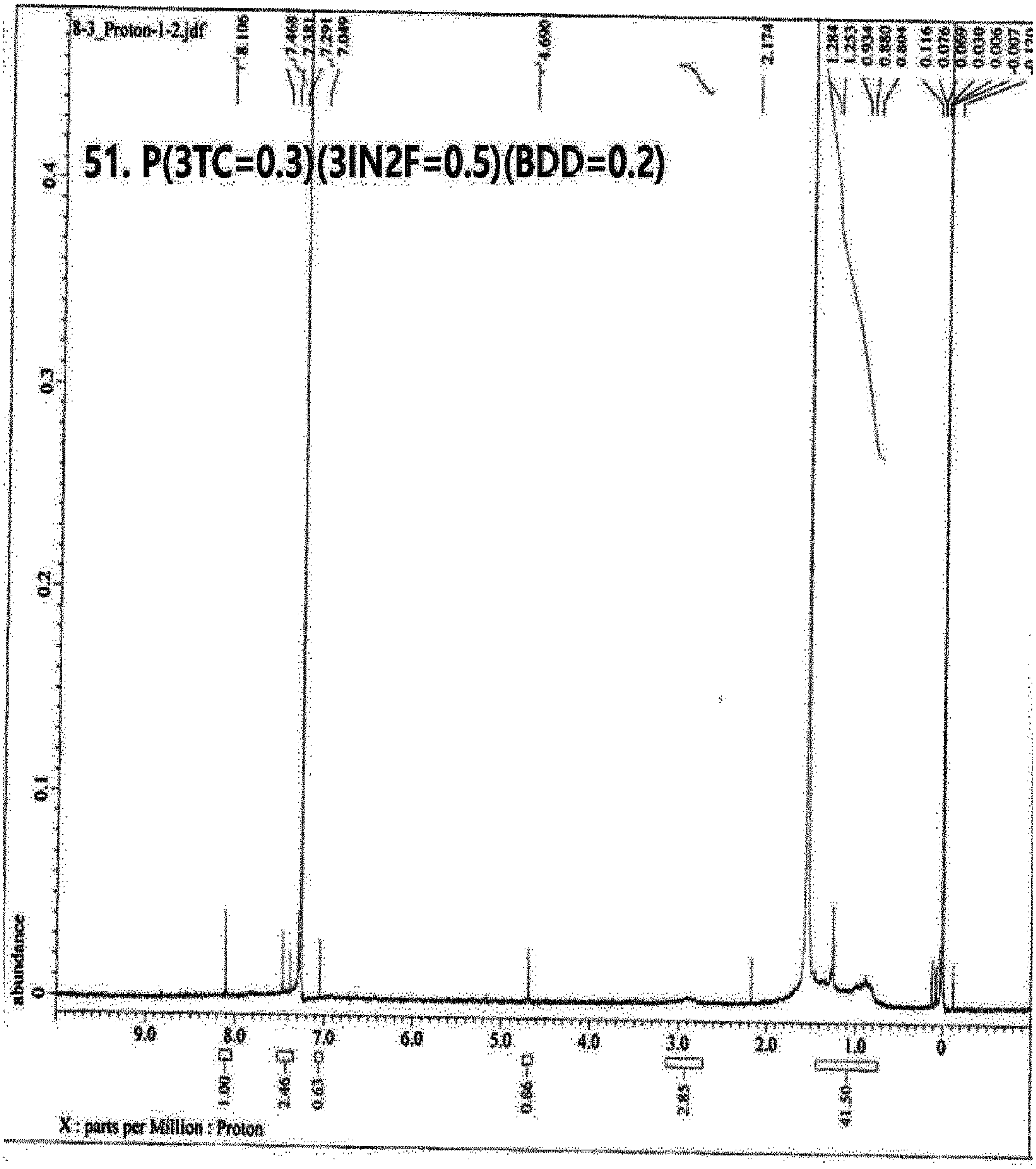
8-3_Proton-1-2.jdf
51. P(3TC=0.3)(3IN2F=0.5)(BDD=0.2)
8.106
7.468
7.381
7.291
7.049
4.690
2.174
1.284
1.253
0.934
0.880
0.804
0.116
0.076
0.069
0.030
0.006
-0.007
abundance
0.4
0.3
0.2
0.1
0
9.0
8.0
7.0
6.0
5.0
4.0
3.0
2.0
1.0
0
1.00
2.46
0.63
0.86
2.85
41.50
X : parts per Million : Proton

【Fig. 70】

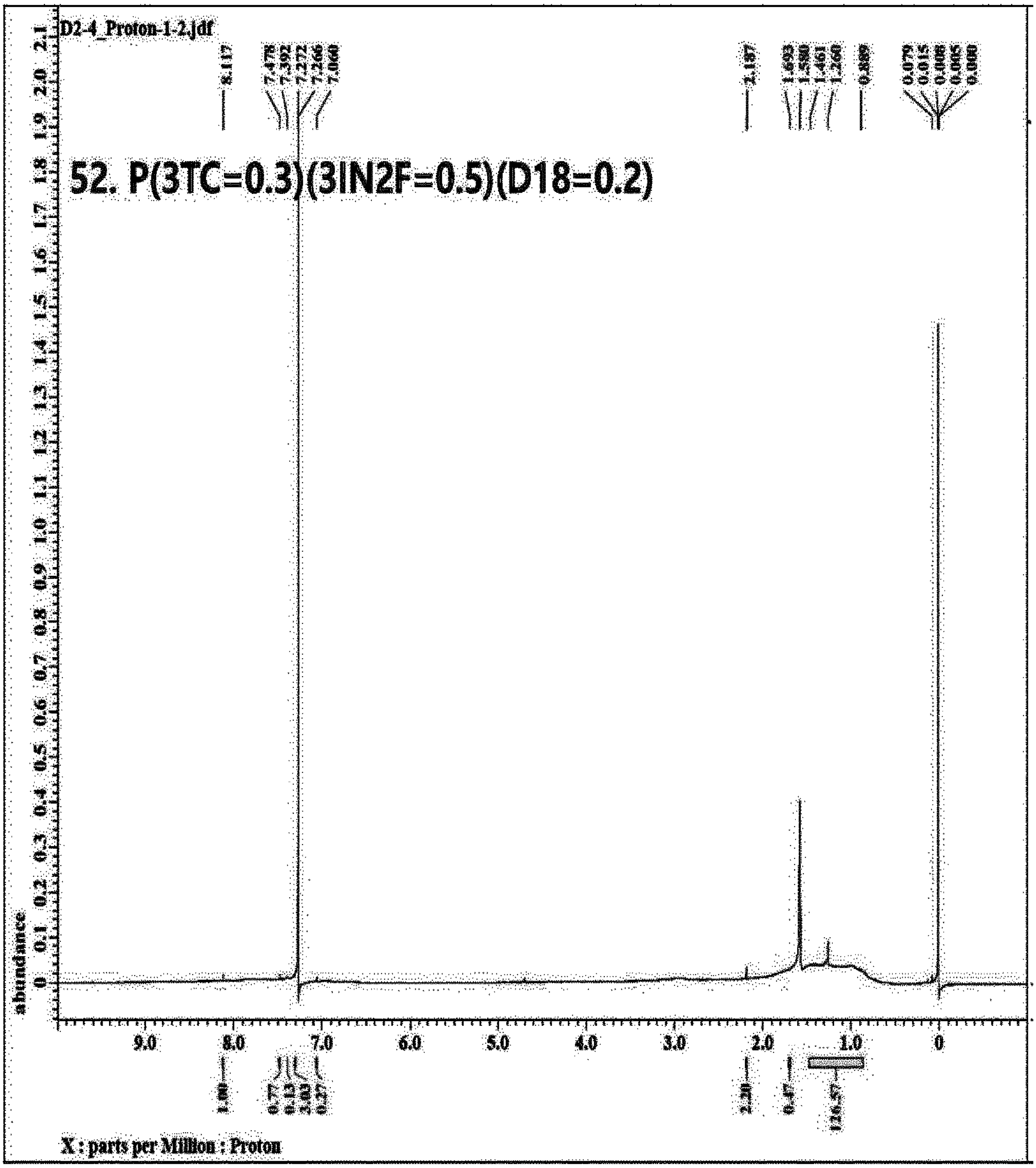
D2-4_Proton-1-2.jdf
52. P(3TC=0.3)(3IN2F=0.5)(D18=0.2)
8.117
7.478
7.392
7.272
7.266
7.060
2.187
1.603
1.580
1.461
1.260
0.889
0.079
0.015
0.008
0.005
0.000
abundance
X : parts per Million : Proton
1.00
0.77
0.13
3.03
0.27
2.20
0.47
126.57

【Fig. 71】

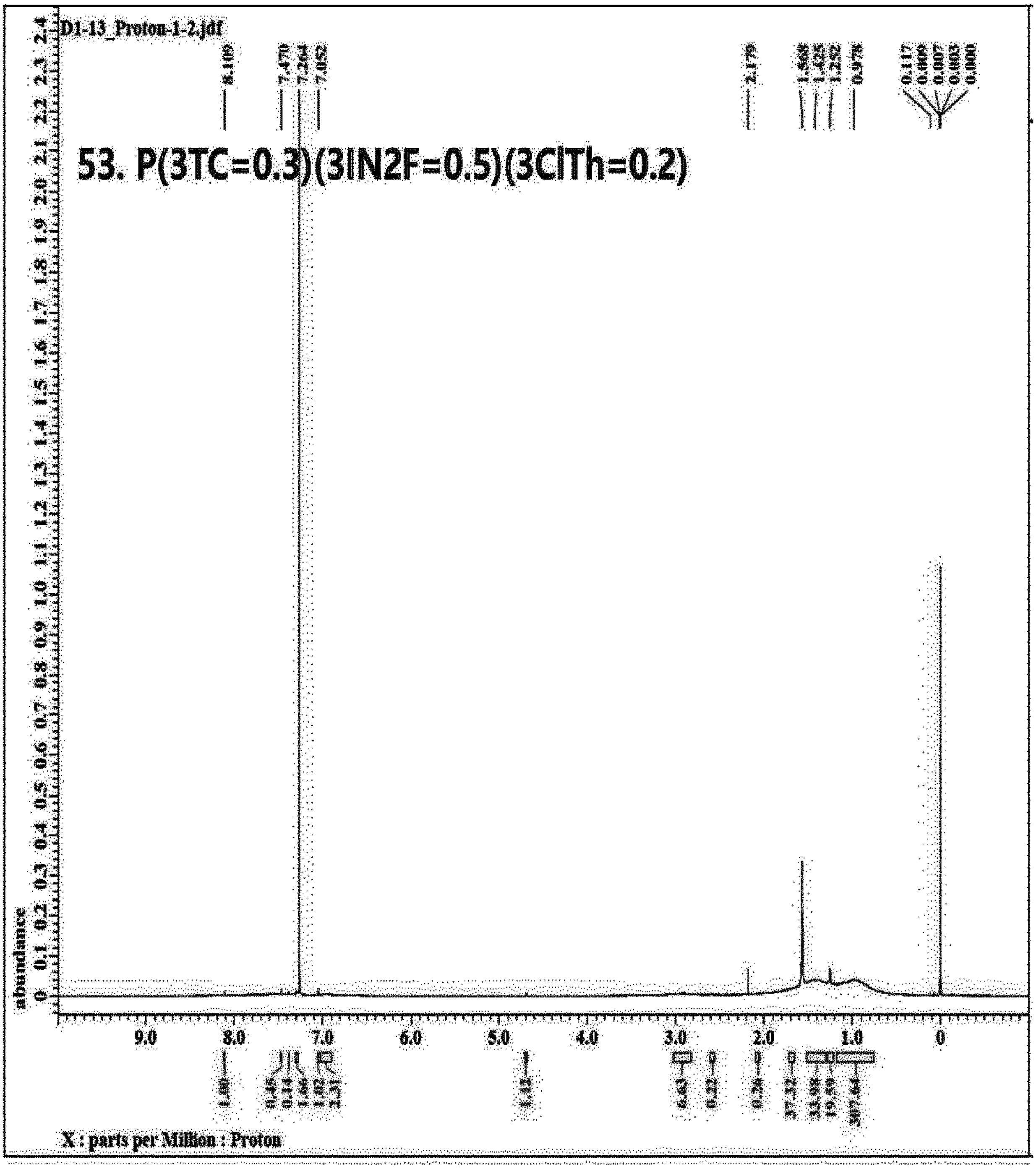
D1-13_Proton-1-2.jdf
53. P(3TC=0.3)(3IN2F=0.5)(3ClTh=0.2)
8.109
7.470
7.264
7.052
2.179
1.568
1.425
1.252
0.978
0.117
0.009
0.007
0.003
0.000
abundance
X : parts per Million : Proton

【Fig. 72】

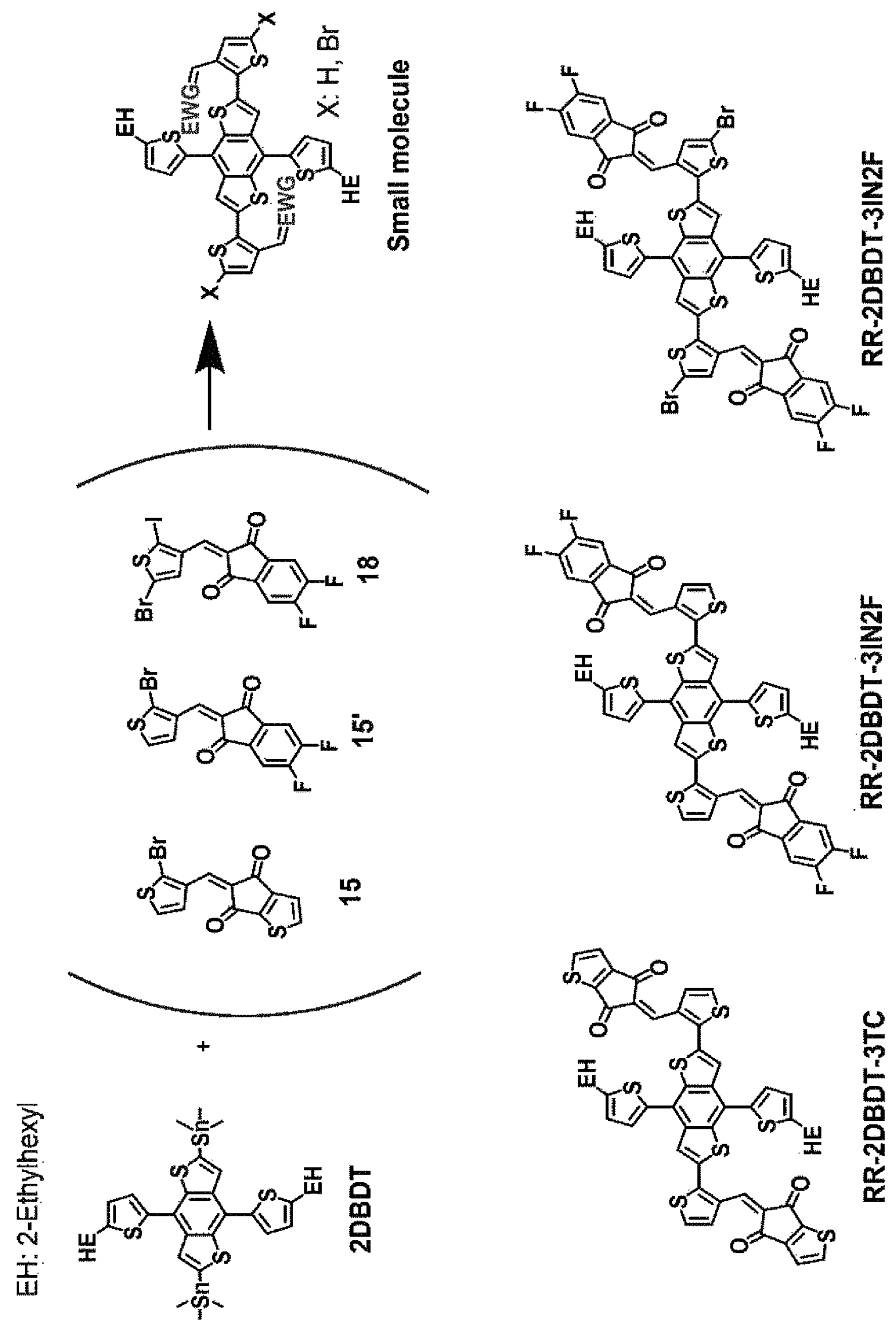

EH: 2-Ethylhexyl

2DBDT

15

15'

18

Small molecule

X: H, Br

RR-2DBDT-3TC

RR-2DBDT-3IN2F

RR-2DBDT-3IN2F

【Fig. 73】

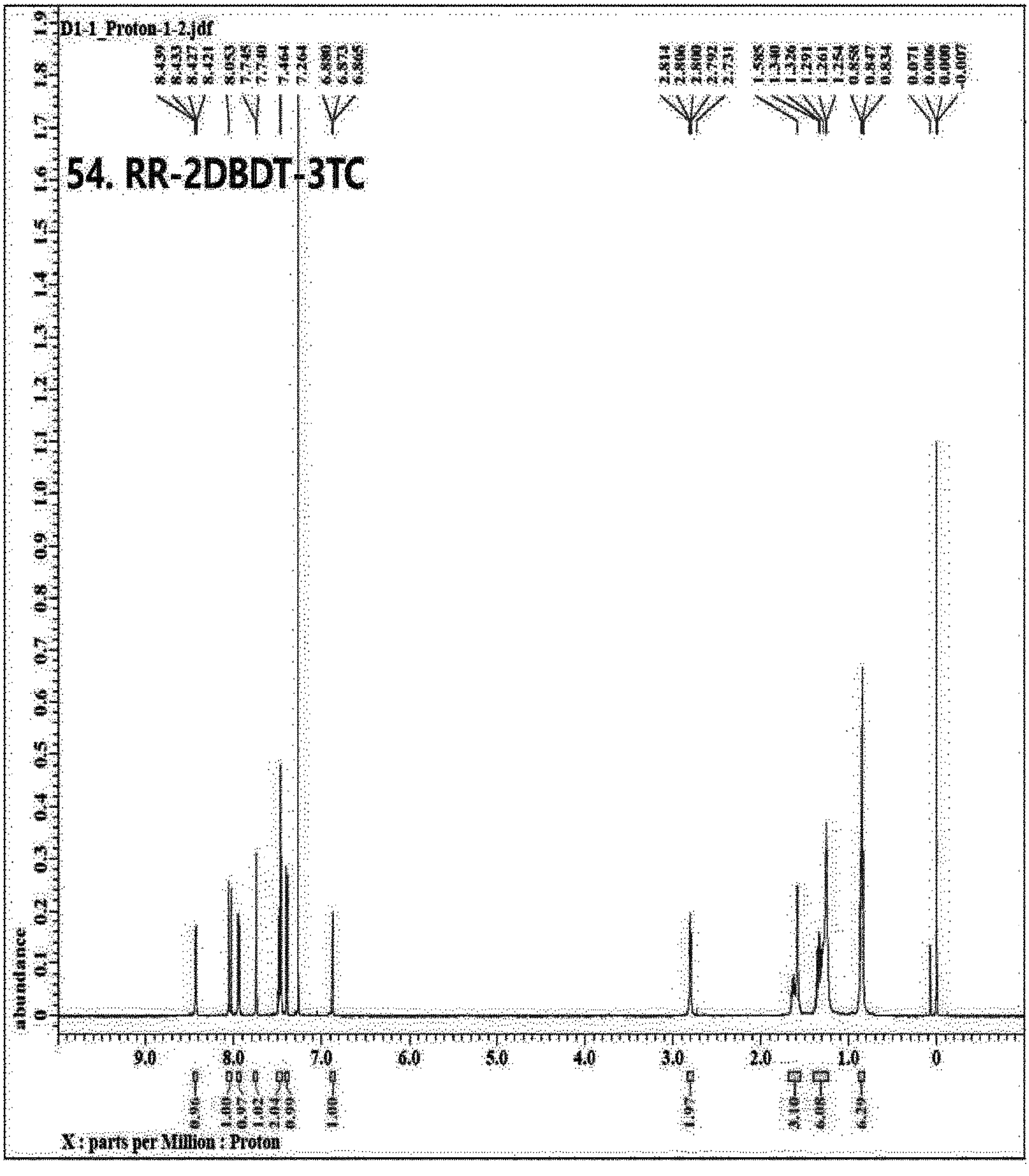
D1-1_Proton-1-2.jdf
54. RR-2DBDT-3TC
abundance
X : parts per Million : Proton

【Fig. 74】

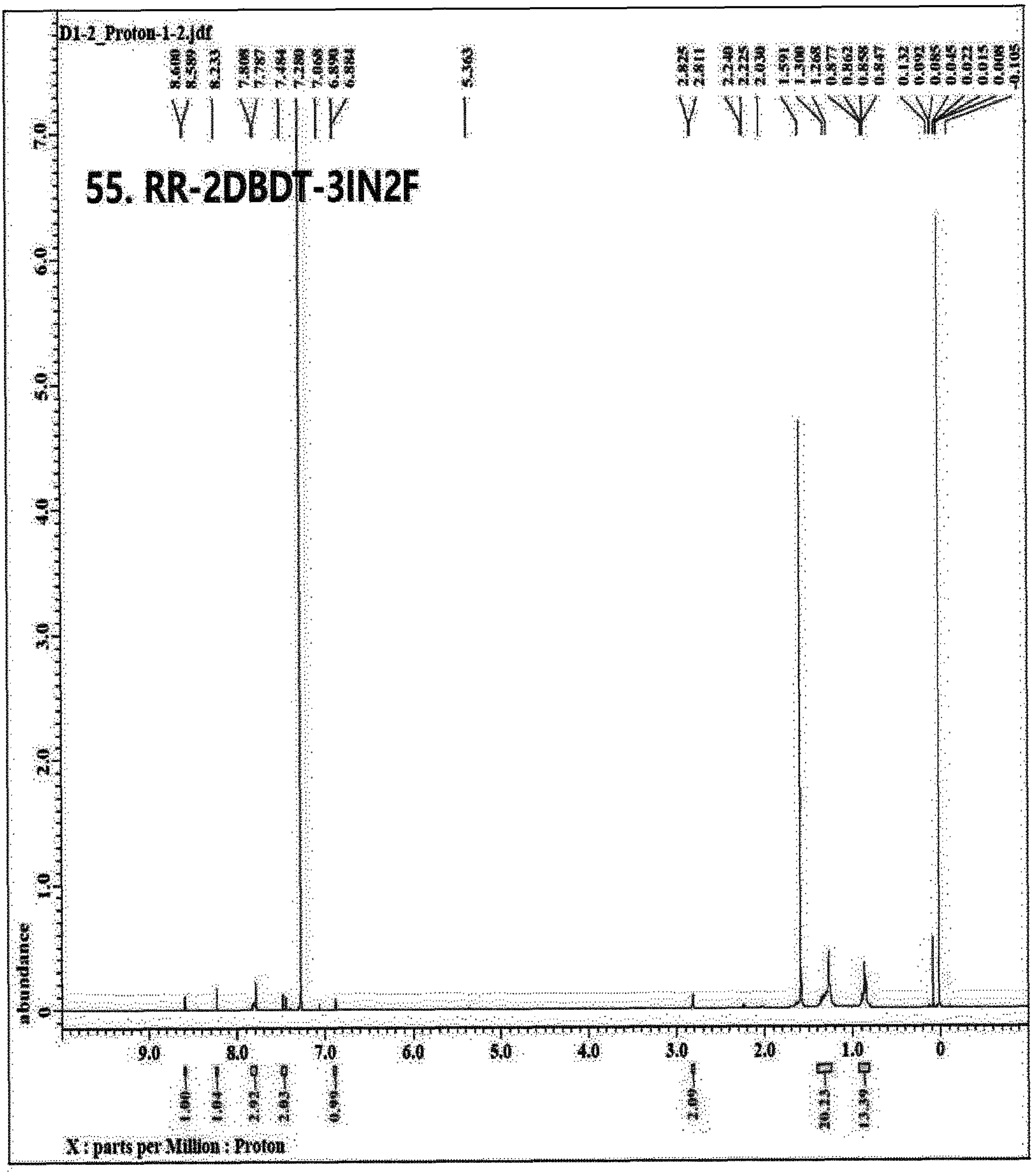
D1-2_Proton-1-2.jdf
55. RR-2DBDT-3IN2F
abundance
X : parts per Million : Proton

【Fig. 75】

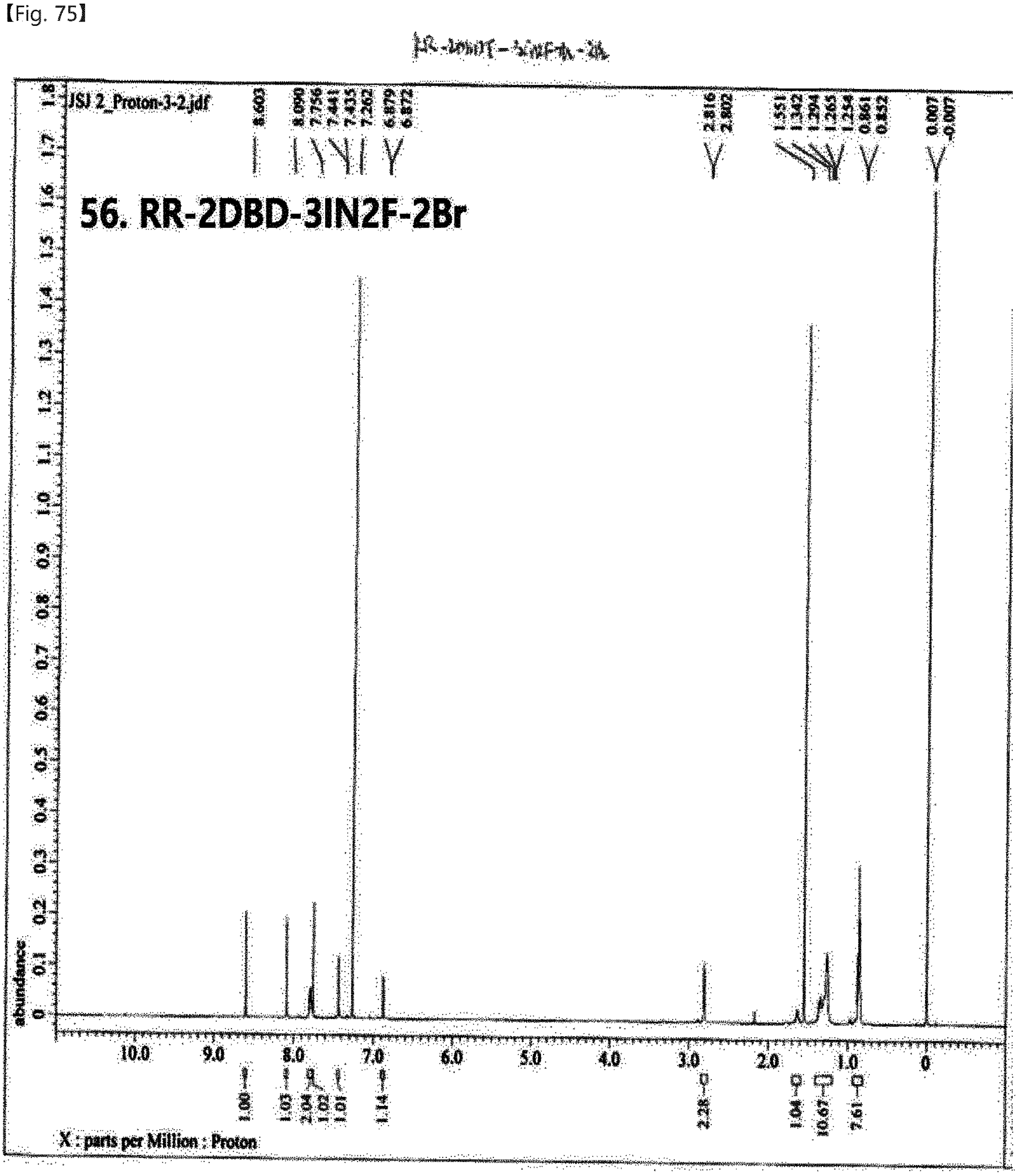
JSJ 2_Proton-3-2.jdf
56. RR-2DBD-3IN2F-2Br
8.603
8.090
7.756
7.441
7.435
7.262
6.879
6.872
2.816
2.802
1.551
1.342
1.294
1.265
1.234
0.861
0.852
0.007
-0.007
abundance
1.00
1.03
2.04
1.02
1.01
1.14
2.28
1.04
10.67
7.61
X : parts per Million : Proton

【Fig. 76】

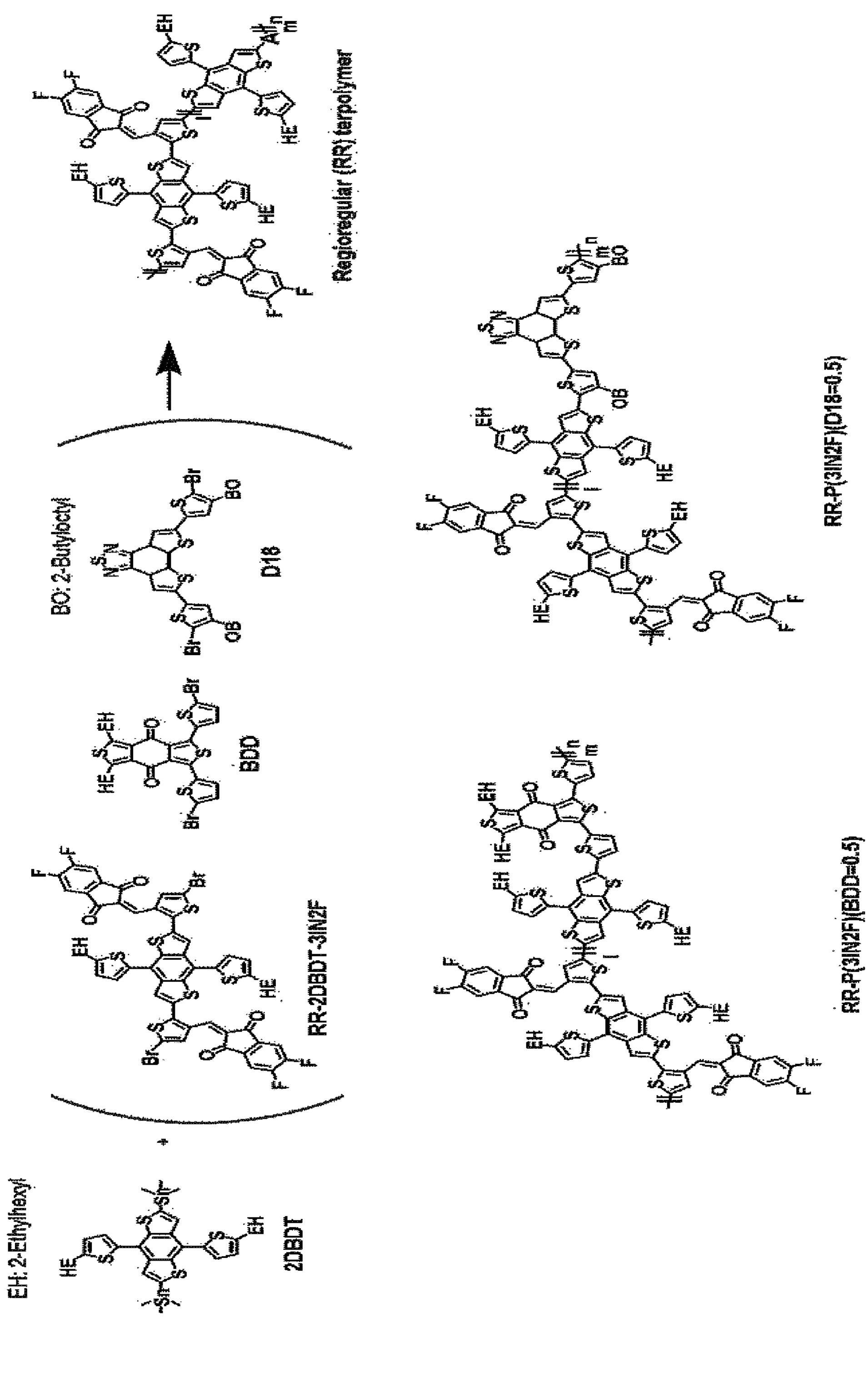
EH: 2-Ethylhexyl
2DBDT
RR-2DBDT-3IN2F
BDD
BO: 2-Butyloctyl
D18
Regioregular (RR) terpolymer
RR-P(3IN2F)(BDD=0.5)
RR-P(3IN2F)(D18=0.5)

【Fig. 77】

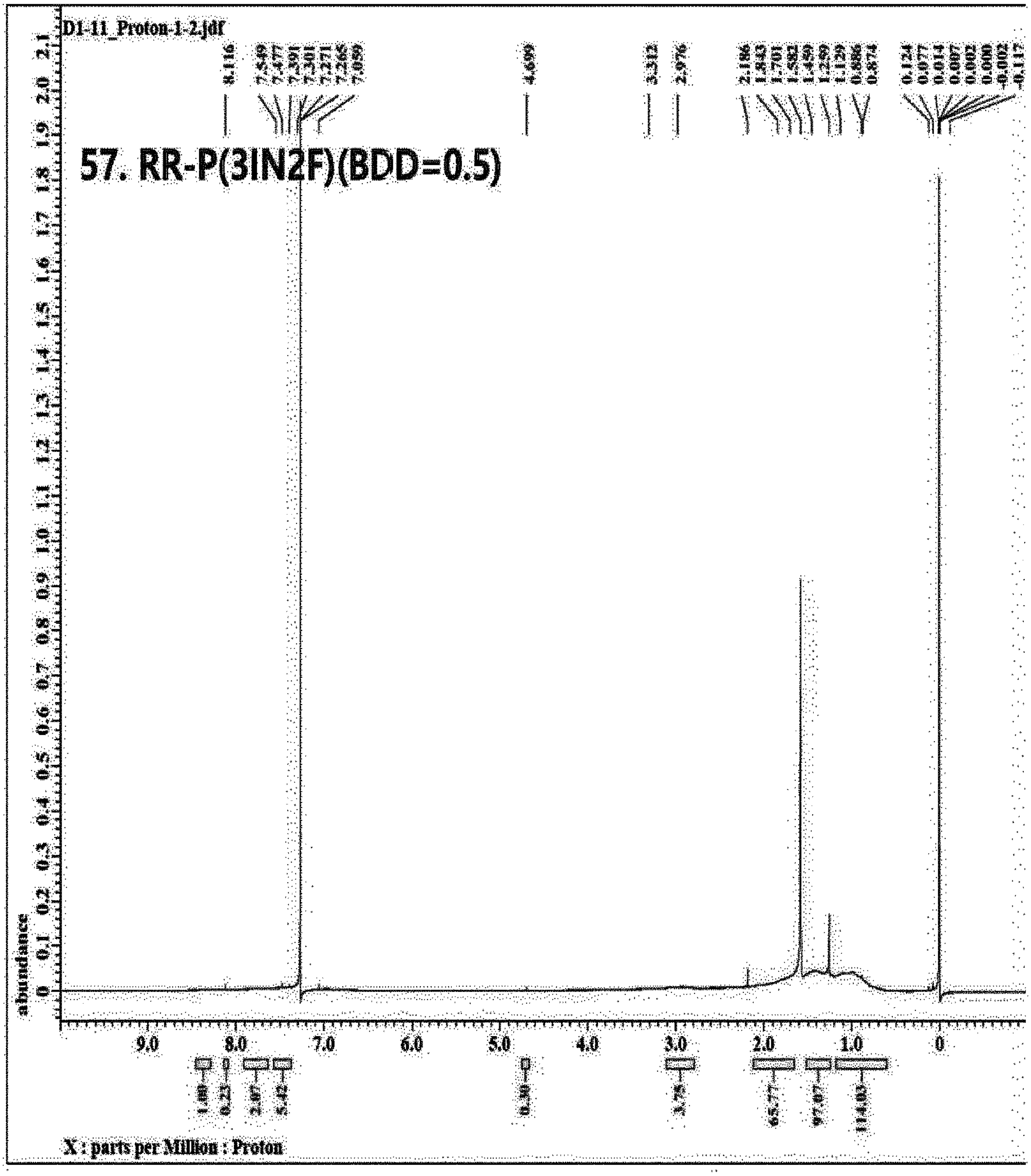
D1-11_Proton-1-2.jdf
57. RR-P(3IN2F)(BDD=0.5)
abundance
X : parts per Million : Proton

【Fig. 78】

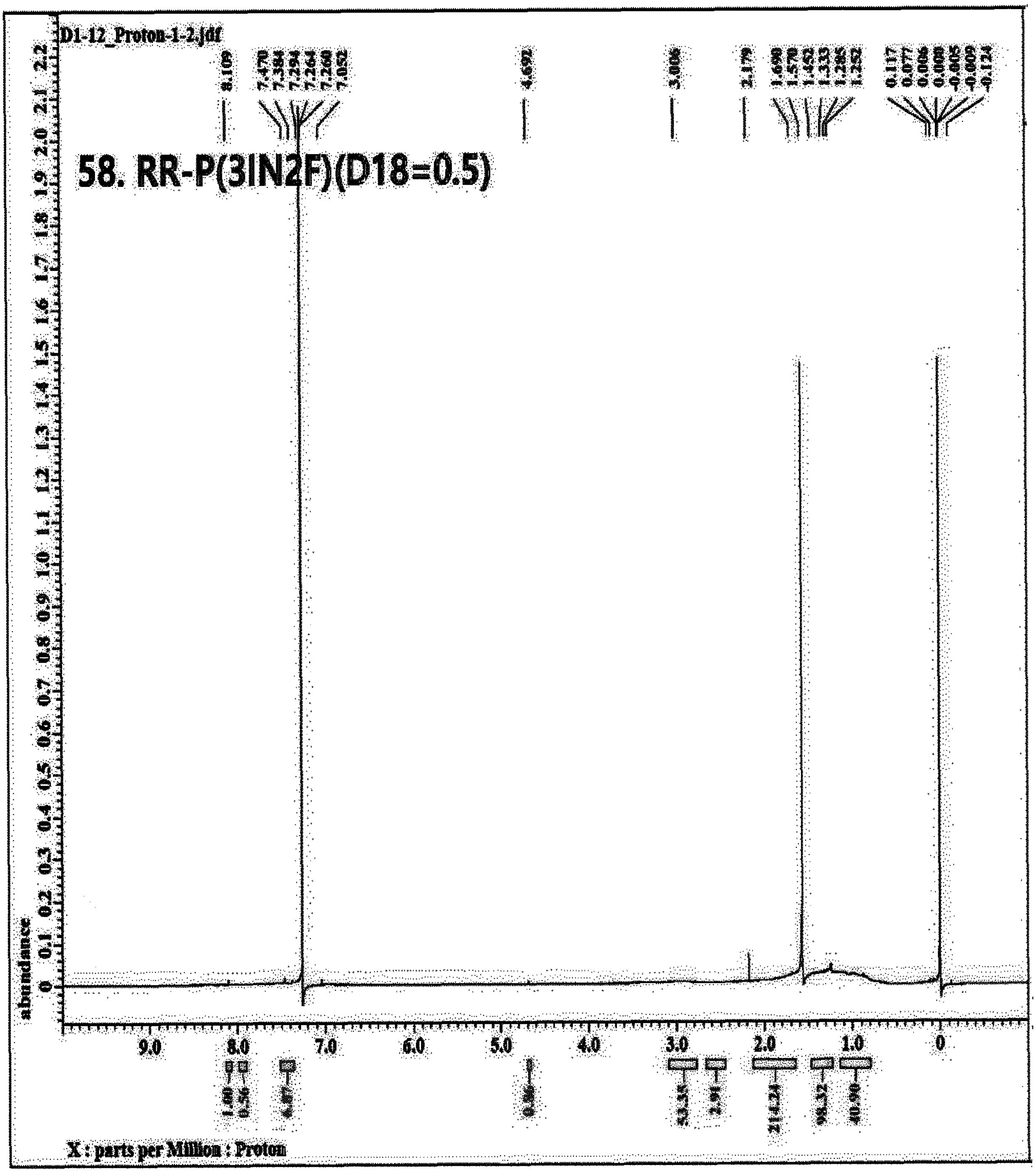
D1-12_Proton-1-2.jdf
58. RR-P(3IN2F)(D18=0.5)
abundance
X : parts per Million : Proton

【Fig. 79】

Ref.) Acceptor unit of high-efficiency polymer PM6/7,
Number of synthetic steps(NSS) and Reciprocal yield(RY) of BDD(=B11)

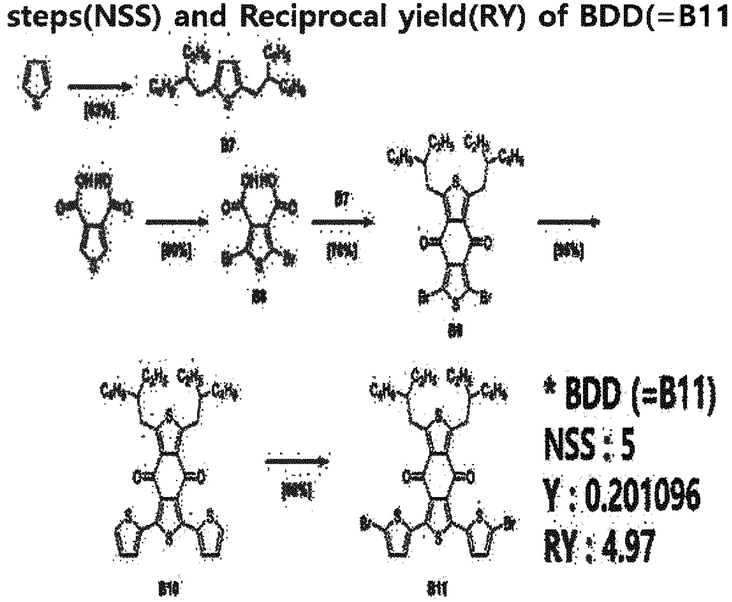

* BDD (=B11)
NSS : 5
Y : 0.201096
RY : 4.97

Ref.) Acceptor unit of high-efficiency polymer D18,
Number of synthetic steps(NSS) and Reciprocal yield(RY) of D18(=E19)

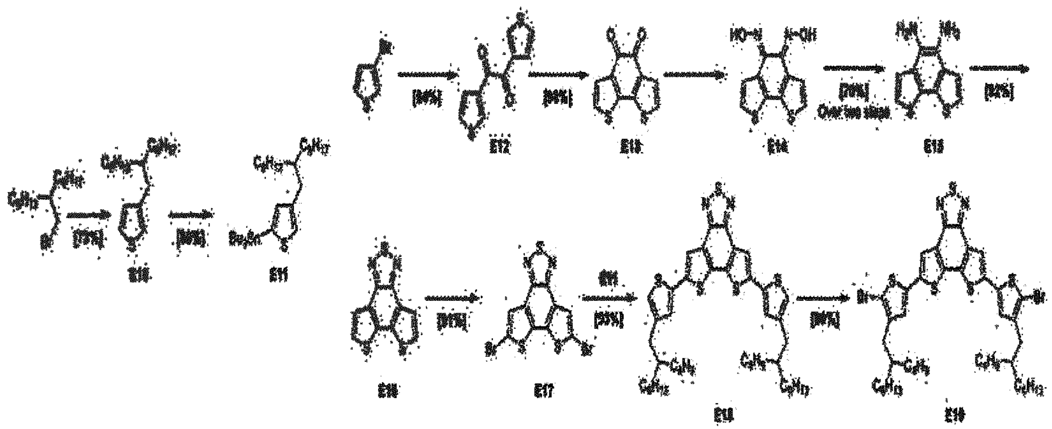

* D18 (=E19)
NSS : 10
Y : 0.2102
RY : 4.76

【Fig. 80】

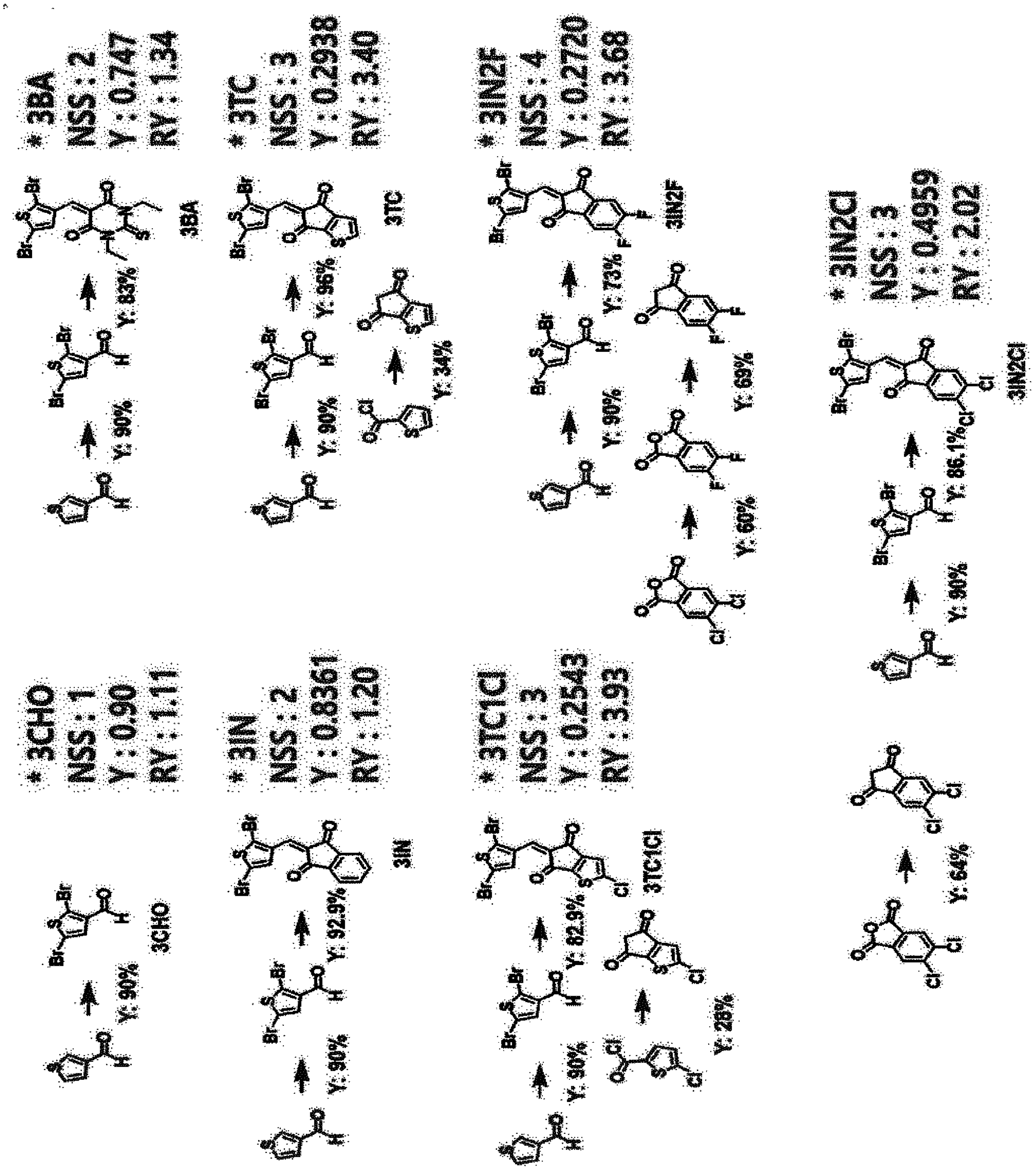

* 3BA
NSS : 2
Y : 0.747
RY : 1.34
Y: 90%
Y: 83%
3BA
* 3TC
NSS : 3
Y : 0.2938
RY : 3.40
Y: 90%
Y: 96%
Y: 34%
3TC
* 3IN2F
NSS : 4
Y : 0.2720
RY : 3.68
Y: 90%
Y: 73%
Y: 60%
Y: 69%
3IN2F
* 3IN2Cl
NSS : 3
Y : 0.4959
RY : 2.02
Y: 90%
Y: 86.1%
Y: 64%
3IN2Cl
* 3CHO
NSS : 1
Y : 0.90
RY : 1.11
Y: 90%
3CHO
* 3IN
NSS : 2
Y : 0.8361
RY : 1.20
Y: 90%
Y: 92.9%
3IN
* 3TC1Cl
NSS : 3
Y : 0.2543
RY : 3.93
Y: 90%
Y: 82.9%
Y: 28%
3TC1Cl

【Fig. 81】

P(F-3IN) & P(3IN)-in/out & P(3IN2F) & P(3IN2Cl)

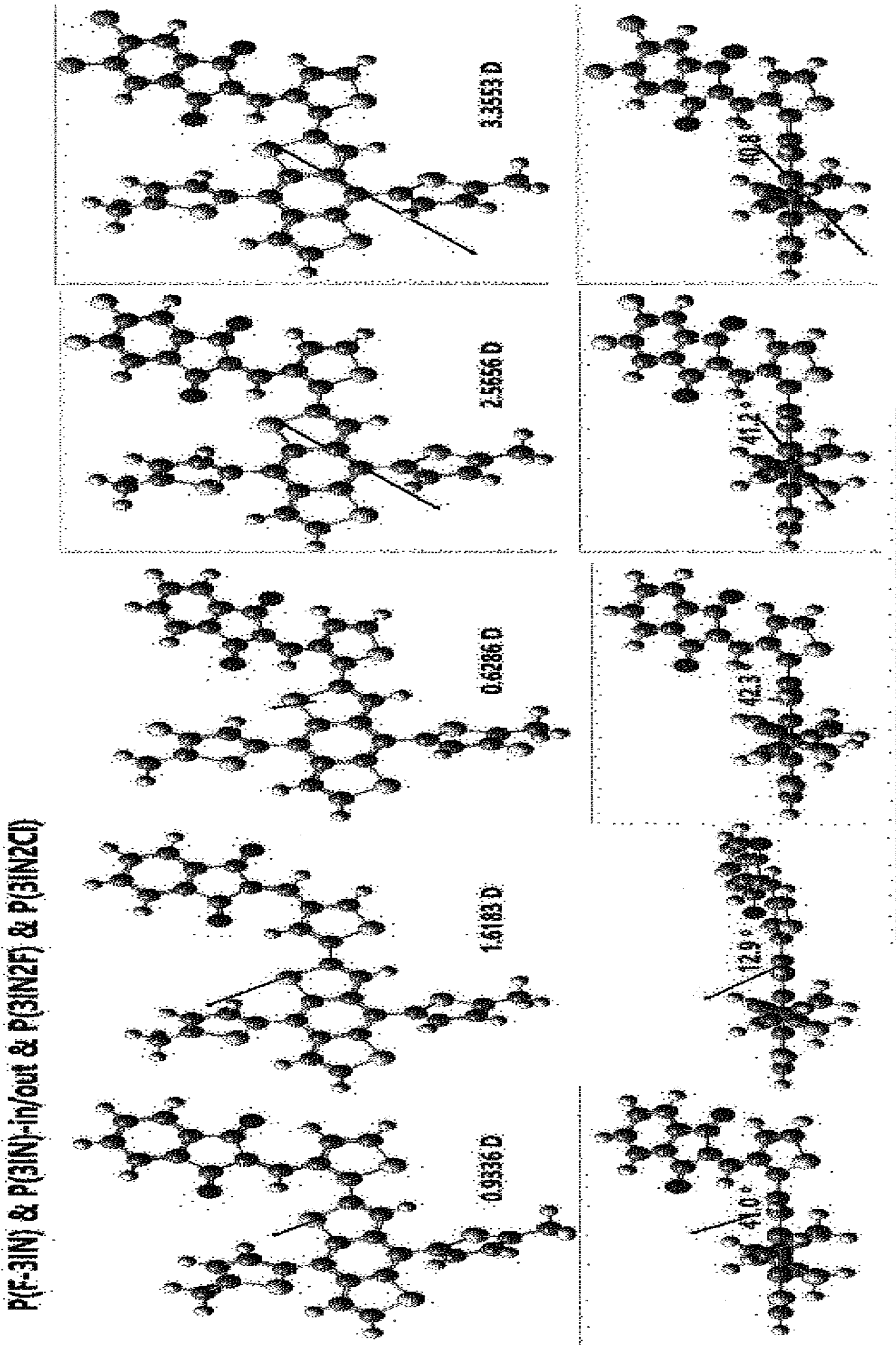


| n = 1 | θ [°] | D | $E_{LUMO}^{cal}$ [eV] | $E_{HOMO}^{cal}$ [eV] | $E_g^{cal}$ [eV] |
|---|---|---|---|---|---|
| P(3IN)-in | 41.0 | 0.9336 | -2.577 | -5.213 | 2.64 |
| P(3IN)-out | 12.9 | 1.6183 | -2.498 | -5.150 | 2.65 |
| P(F-3IN) | 42.3 | 0.6286 | -2.644 | -5.391 | 2.75 |
| P(3IN2F) | 41.2 | 2.5656 | -2.733 | -5.210 | 2.48 |
| P(3IN2Cl) | 40.8 | 3.3553 | -2.839 | -5.311 | 2.47 |

【Fig. 82】

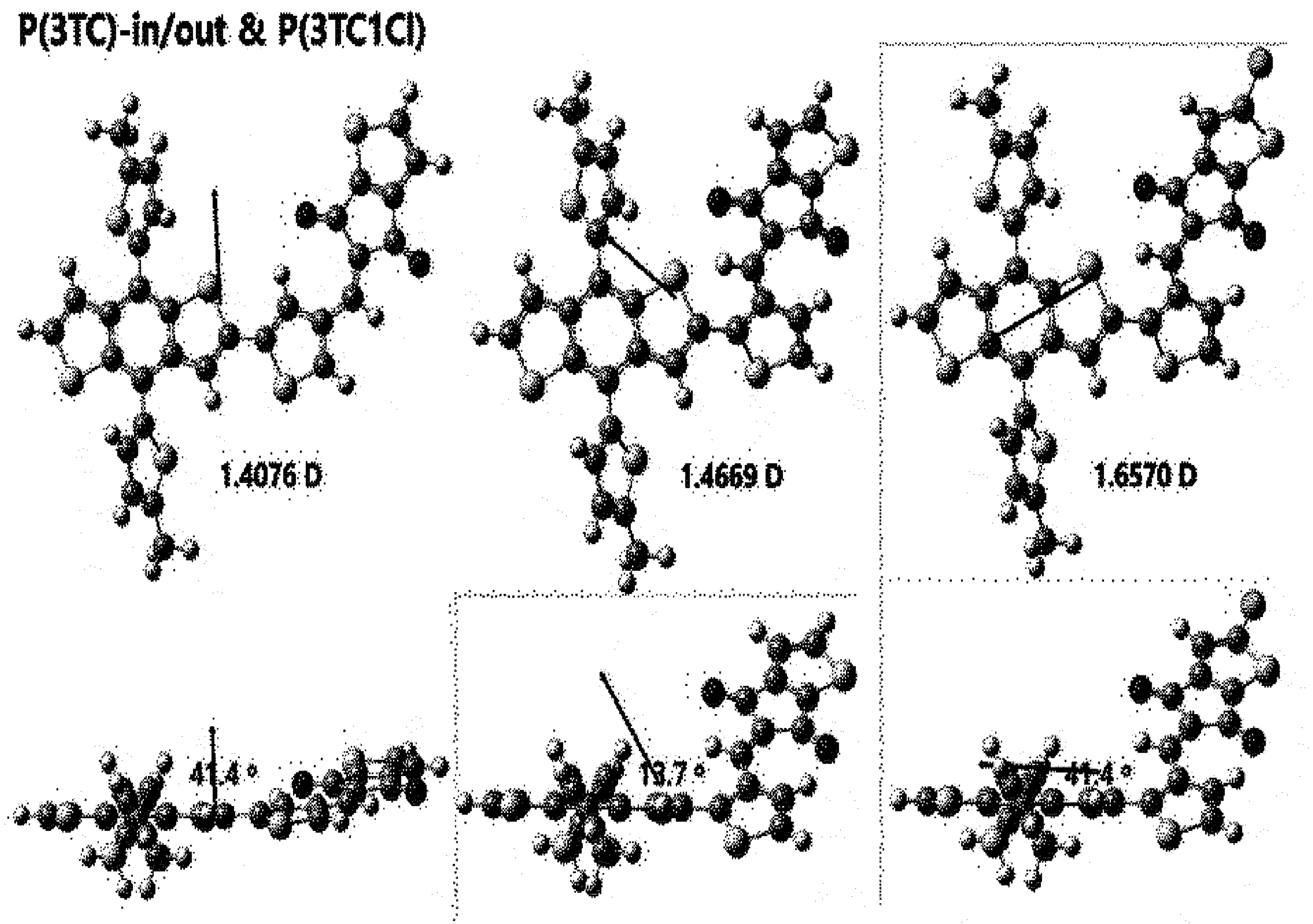


| n = 1 | θ [°] | D | $E_{LUMO}^{cal}$ [eV] | $E_{HOMO}^{cal}$ [eV] | $E_g^{cal}$ [eV] |
|---|---|---|---|---|---|
| P(3TC)-In | 41.4 | 1.4669 | -2.477 | -5.207 | 2.73 |
| P(3TC)-out | 13.7 | 1.4076 | -2.431 | -5.137 | 2.71 |
| P(3TC1Cl) | 41.4 | 1.6570 | -2.606 | -5.251 | 2.64 |

【Fig. 83】

P(3IN)-in,in & P(3IN)-in,out & P(3IN)-out,out

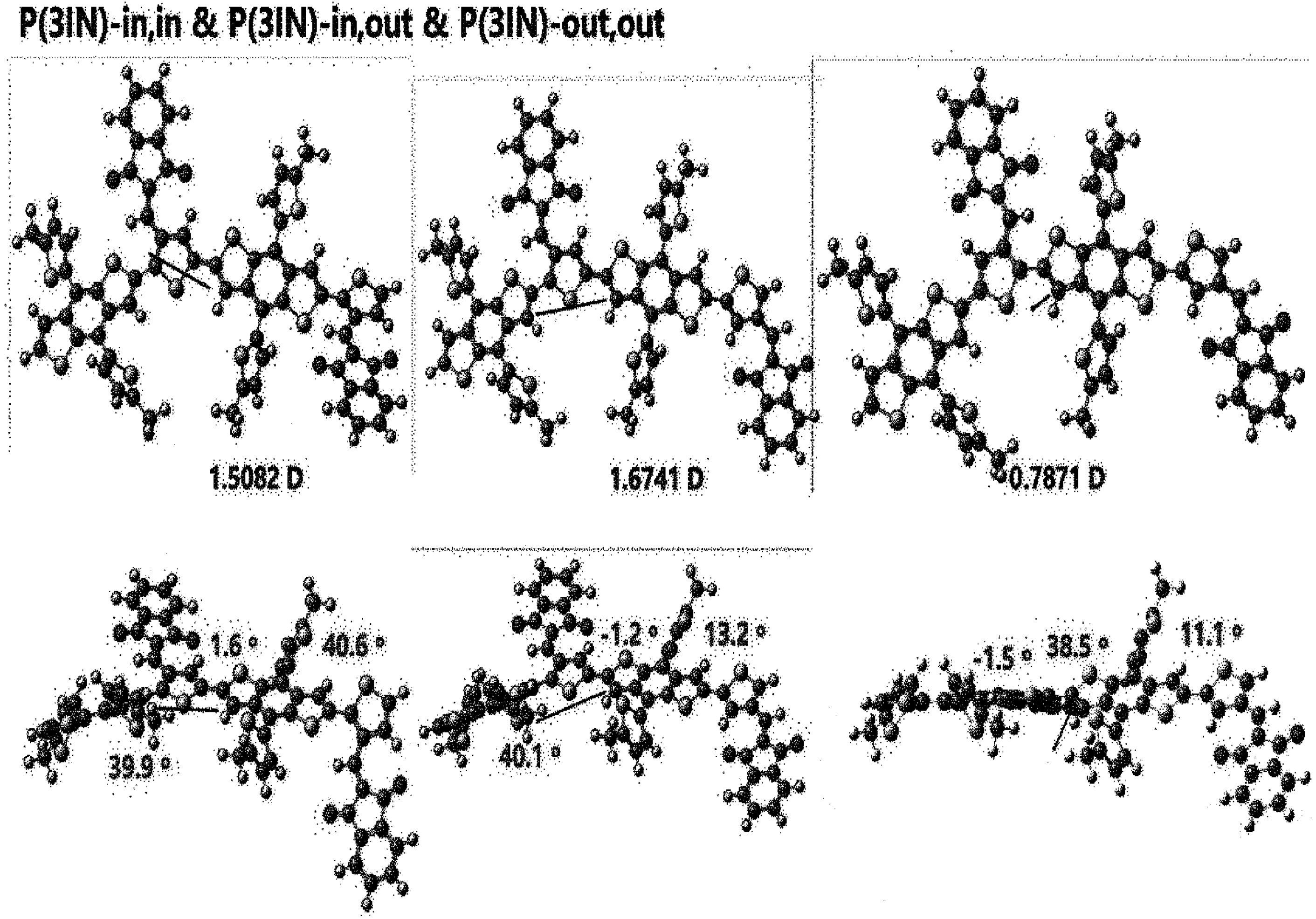


| n = 2 | $\theta_1$ [°] | $\theta_2$ [°] | $\theta_3$ [°] | D | $E_{LUMO}^{cal}$ [eV] | $E_{HOMO}^{cal}$ [eV] | $E_g^{cal}$ [eV] |
|---|---|---|---|---|---|---|---|
| P(3IN)-in,in | 39.9 | 1.6 | 40.6 | 1.5082 | -2.647 | -5.088 | 2.44 |
| P(3IN)-in,out | 40.1 | -1.2 | 13.2 | 1.6741 | -2.616 | -5.037 | 2.42 |
| P(3IN)-out,out | -1.5 | 38.5 | 11.1 | 0.7871 | -2.640 | -5.045 | 2.41 |

【Fig. 84】

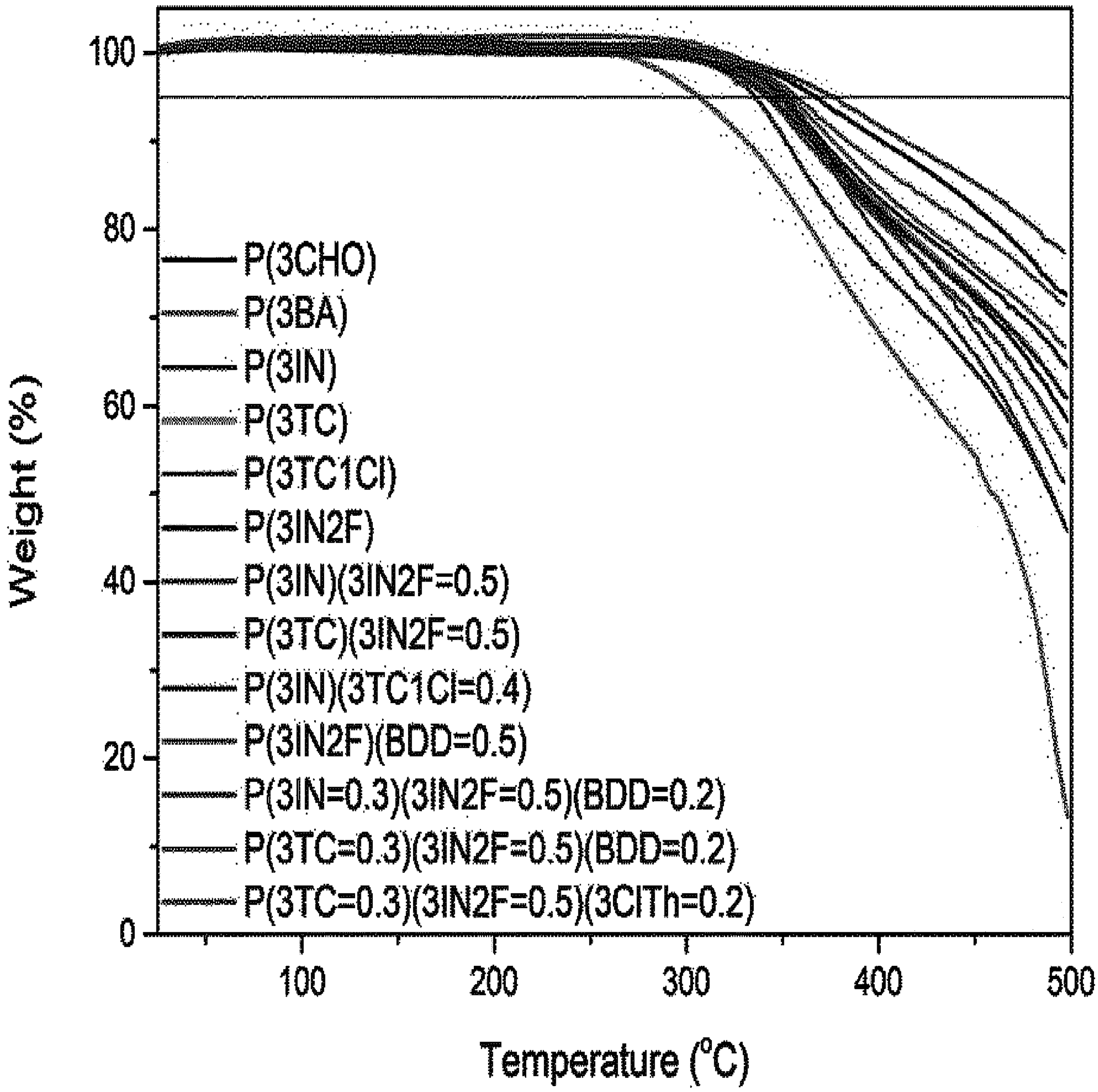
100
80
60
40
20
0
Weight (%)
100
200
300
400
500
Temperature (°C)
P(3CHO)
P(3BA)
P(3IN)
P(3TC)
P(3TC1Cl)
P(3IN2F)
P(3IN)(3IN2F=0.5)
P(3TC)(3IN2F=0.5)
P(3IN)(3TC1Cl=0.4)
P(3IN2F)(BDD=0.5)
P(3IN=0.3)(3IN2F=0.5)(BDD=0.2)
P(3TC=0.3)(3IN2F=0.5)(BDD=0.2)
P(3TC=0.3)(3IN2F=0.5)(3ClTh=0.2)

【Fig. 85】

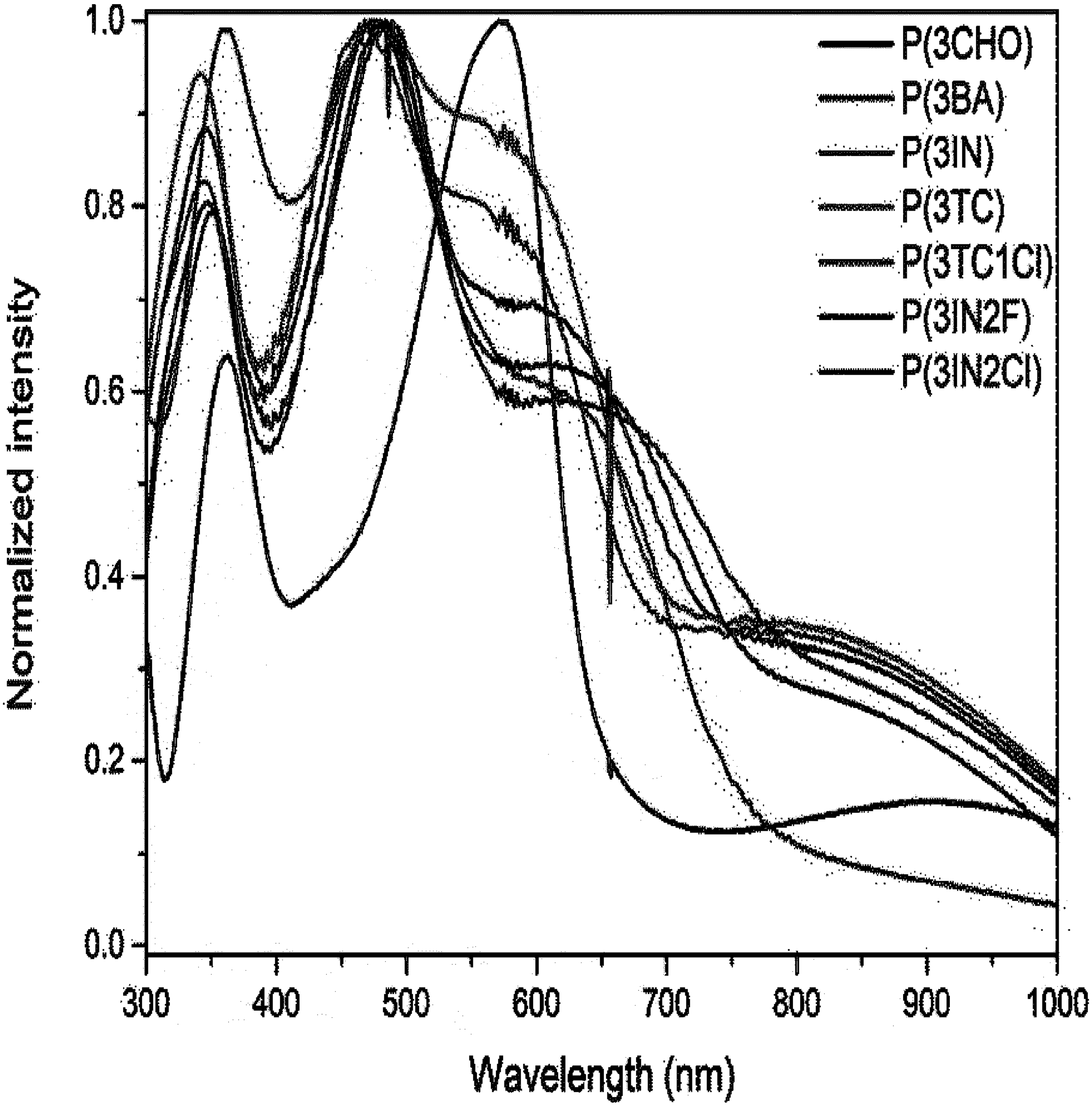
Normalized intensity
Wavelength (nm)
P(3CHO)
P(3BA)
P(3IN)
P(3TC)
P(3TC1Cl)
P(3IN2F)
P(3IN2Cl)
0.0
0.2
0.4
0.6
0.8
1.0
300
400
500
600
700
800
900
1000

【Fig. 86】

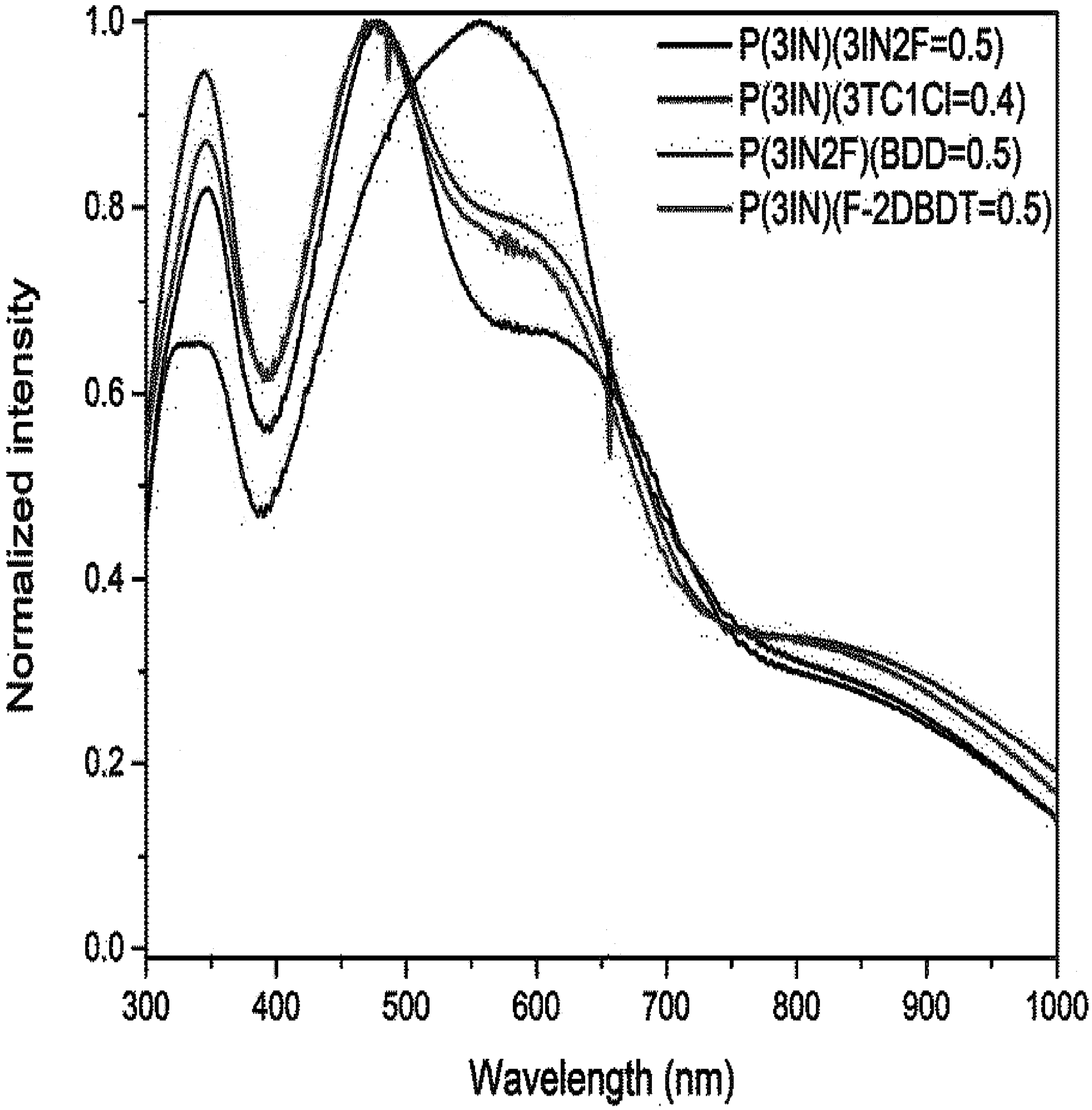
P(3IN)(3IN2F=0.5)
P(3IN)(3TC1Cl=0.4)
P(3IN2F)(BDD=0.5)
P(3IN)(F-2DBDT=0.5)
Normalized intensity
1.0
0.8
0.6
0.4
0.2
0.0
300
400
500
600
700
800
900
1000
Wavelength (nm)

【Fig. 87】

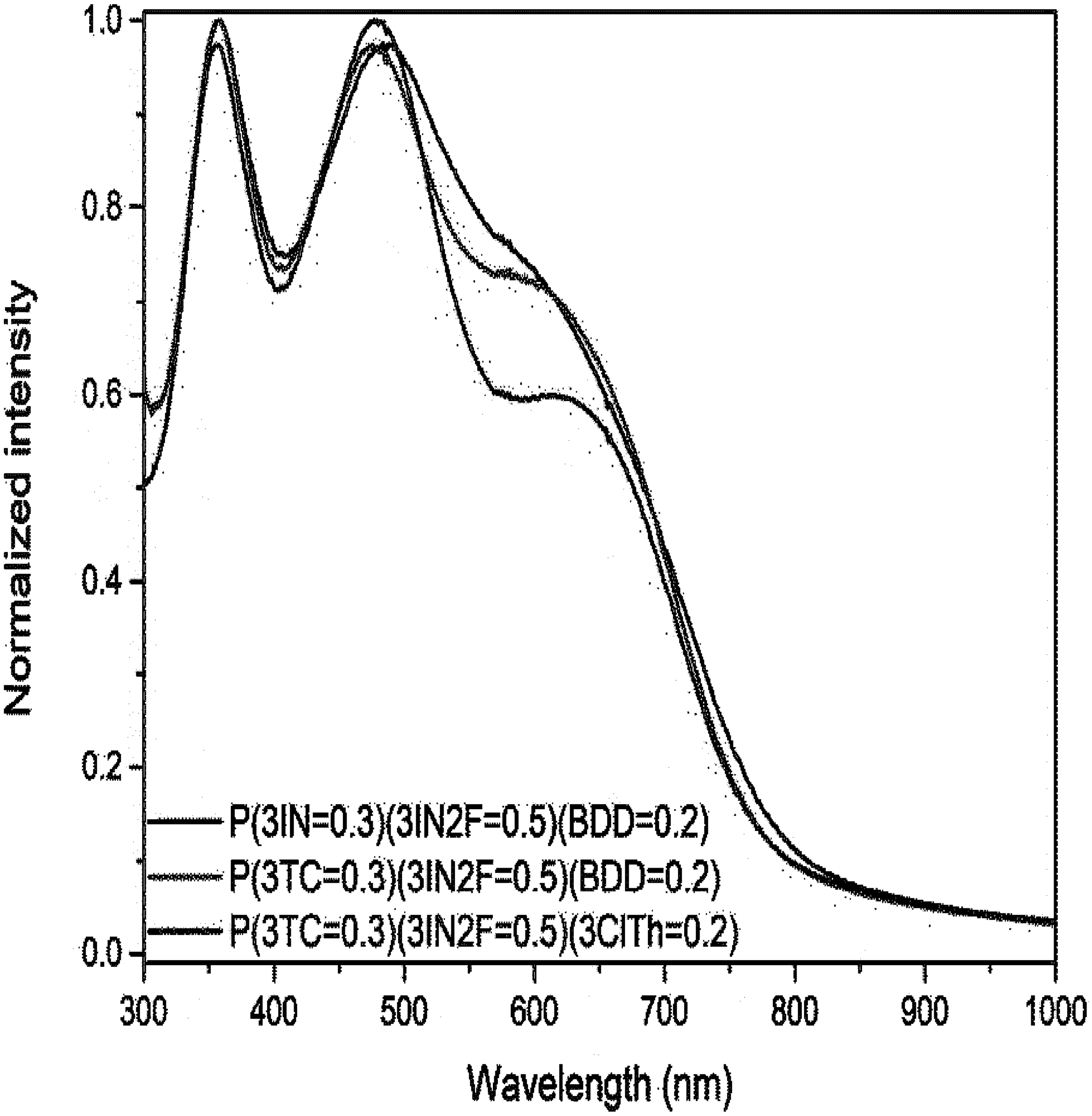
1.0
0.8
0.6
0.4
0.2
0.0
Normalized intensity
300
400
500
600
700
800
900
1000
Wavelength (nm)
P(3IN=0.3)(3IN2F=0.5)(BDD=0.2)
P(3TC=0.3)(3IN2F=0.5)(BDD=0.2)
P(3TC=0.3)(3IN2F=0.5)(3ClTh=0.2)

【Fig. 88】

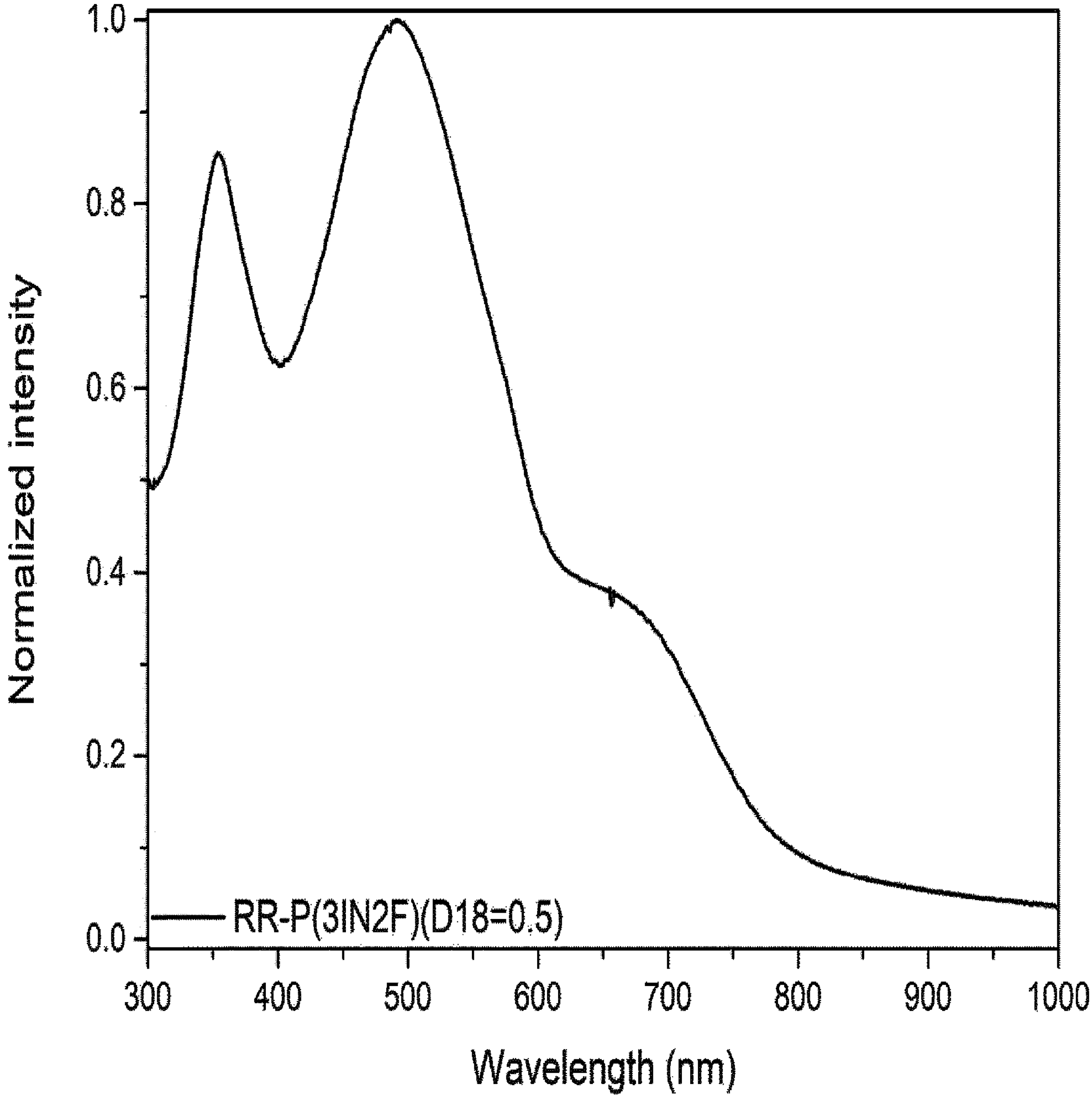
Normalized intensity
1.0
0.8
0.6
0.4
0.2
0.0
300
400
500
600
700
800
900
1000
Wavelength (nm)
RR-P(3IN2F)(D18=0.5)

【Fig. 89】

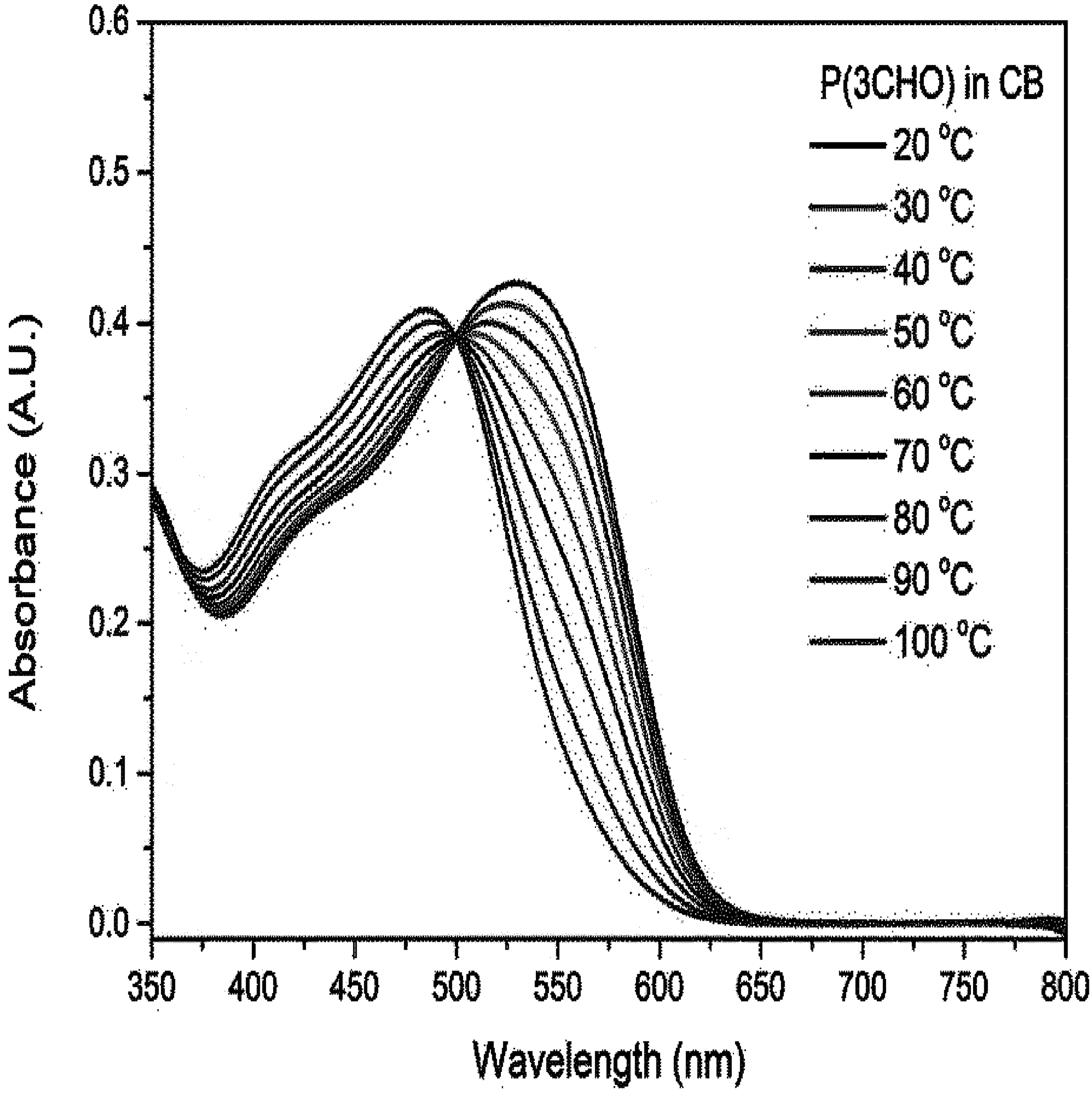
P(3CHO) in CB
20 °C
30 °C
40 °C
50 °C
60 °C
70 °C
80 °C
90 °C
100 °C
Absorbance (A.U.)
Wavelength (nm)
0.0
0.1
0.2
0.3
0.4
0.5
0.6
350
400
450
500
550
600
650
700
750
800

【Fig. 90】

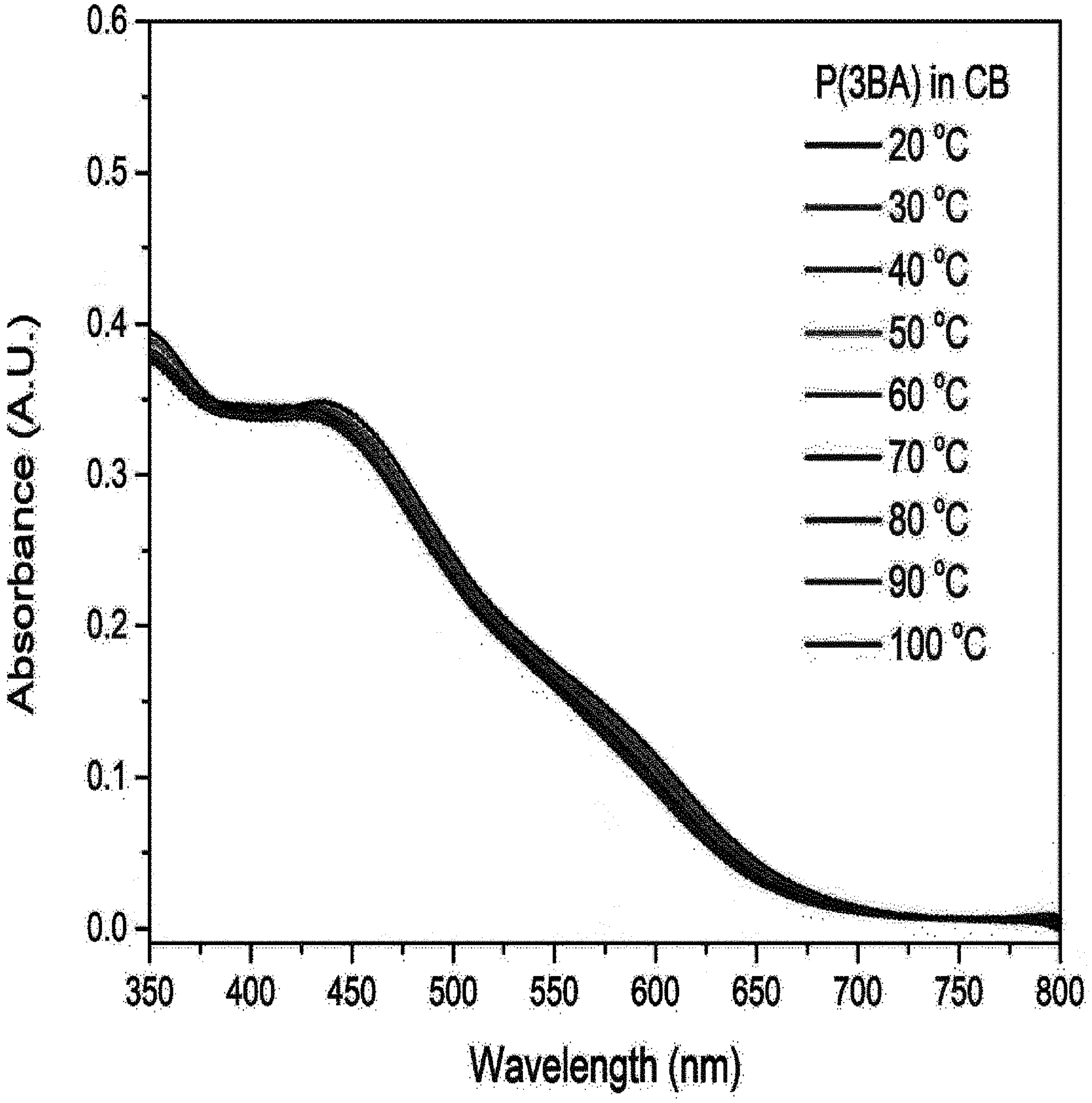
P(3BA) in CB
20 °C
30 °C
40 °C
50 °C
60 °C
70 °C
80 °C
90 °C
100 °C
Absorbance (A.U.)
0.0
0.1
0.2
0.3
0.4
0.5
0.6
350
400
450
500
550
600
650
700
750
800
Wavelength (nm)

【Fig. 91】

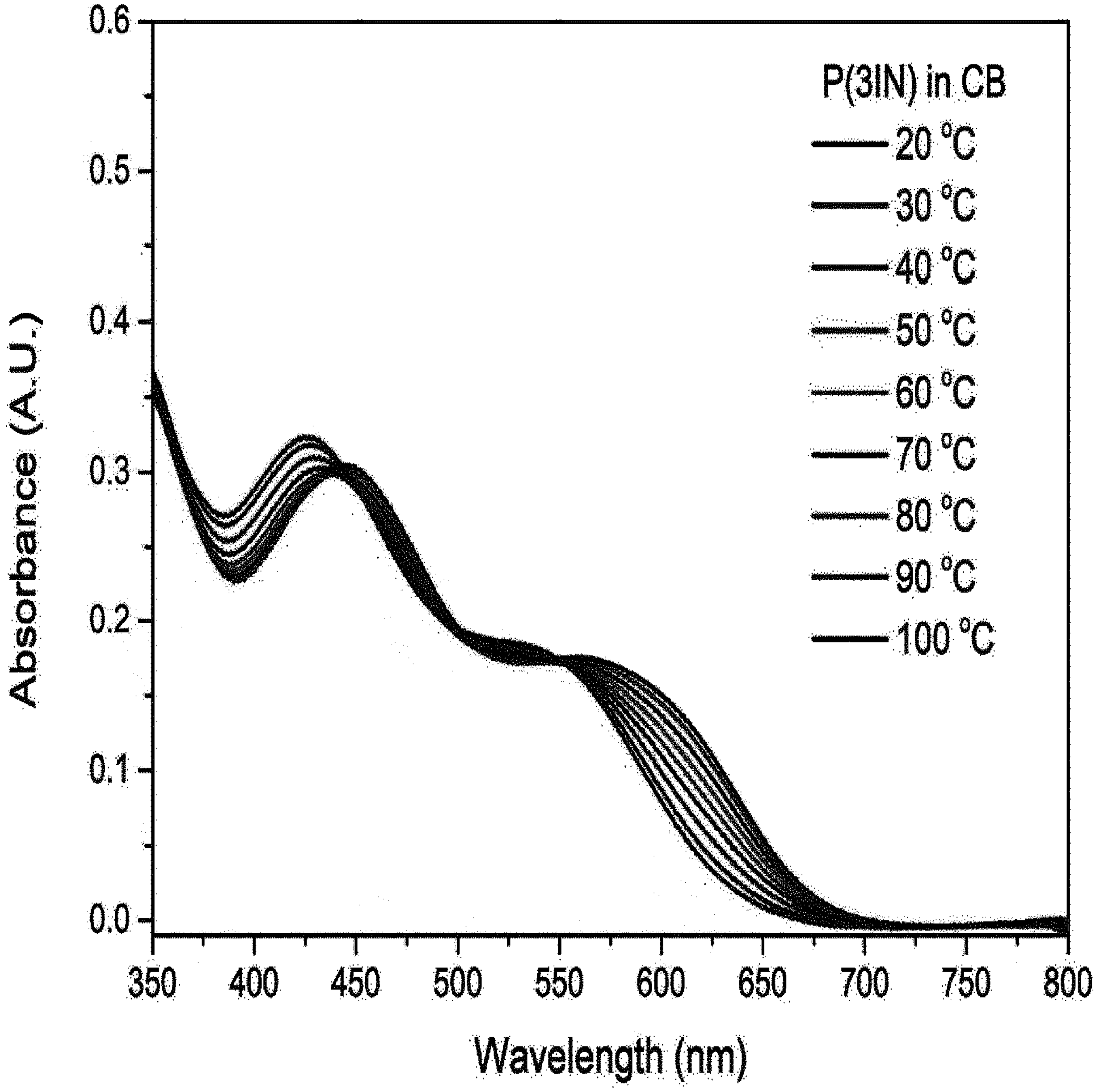
0.6
0.5
0.4
0.3
0.2
0.1
0.0
Absorbance (A.U.)
350
400
450
500
550
600
650
700
750
800
Wavelength (nm)
P(3IN) in CB
20 °C
30 °C
40 °C
50 °C
60 °C
70 °C
80 °C
90 °C
100 °C

【Fig. 92】

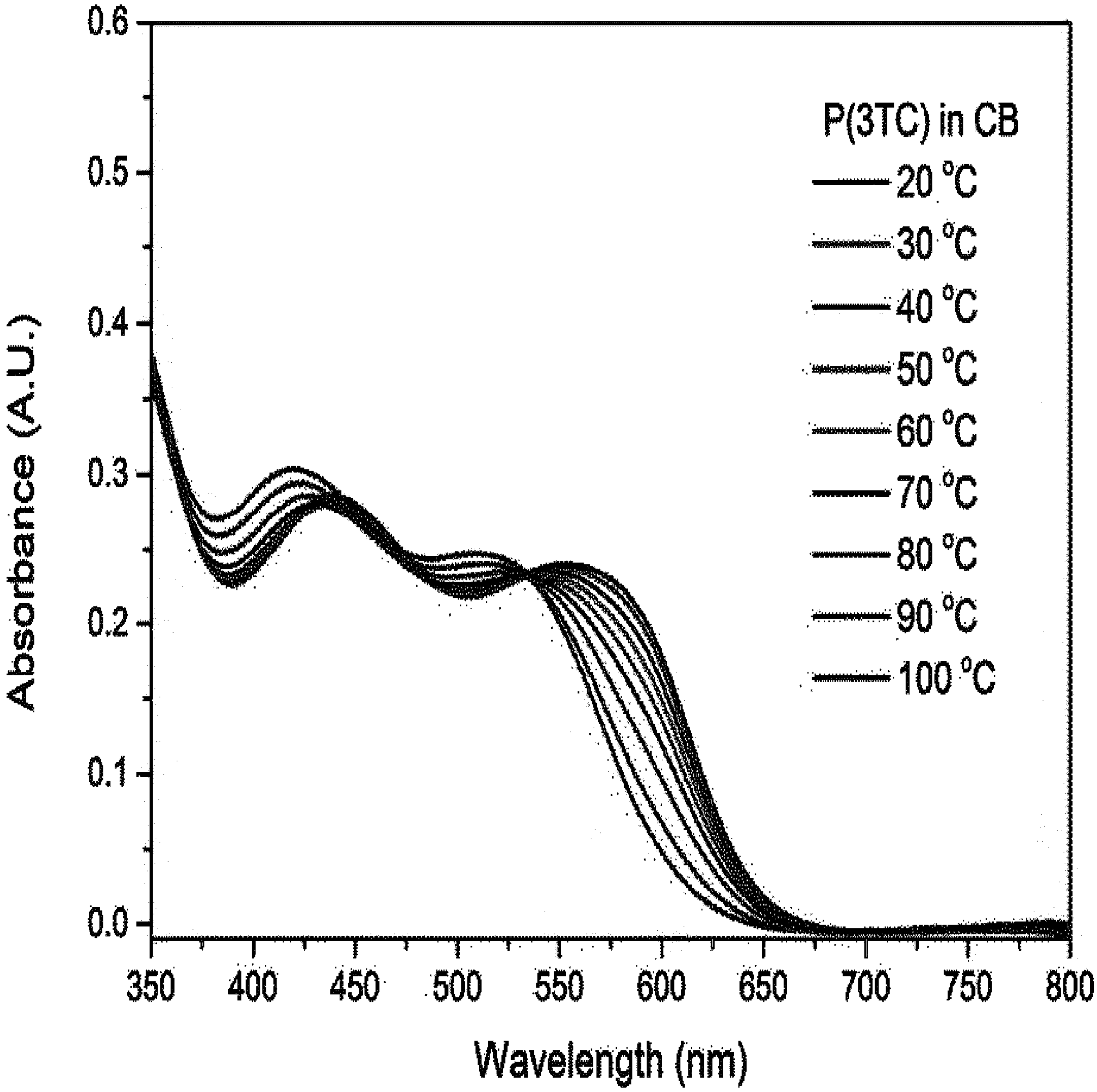
P(3TC) in CB
20 °C
30 °C
40 °C
50 °C
60 °C
70 °C
80 °C
90 °C
100 °C
Absorbance (A.U.)
0.6
0.5
0.4
0.3
0.2
0.1
0.0
350
400
450
500
550
600
650
700
750
800
Wavelength (nm)

【Fig. 93】

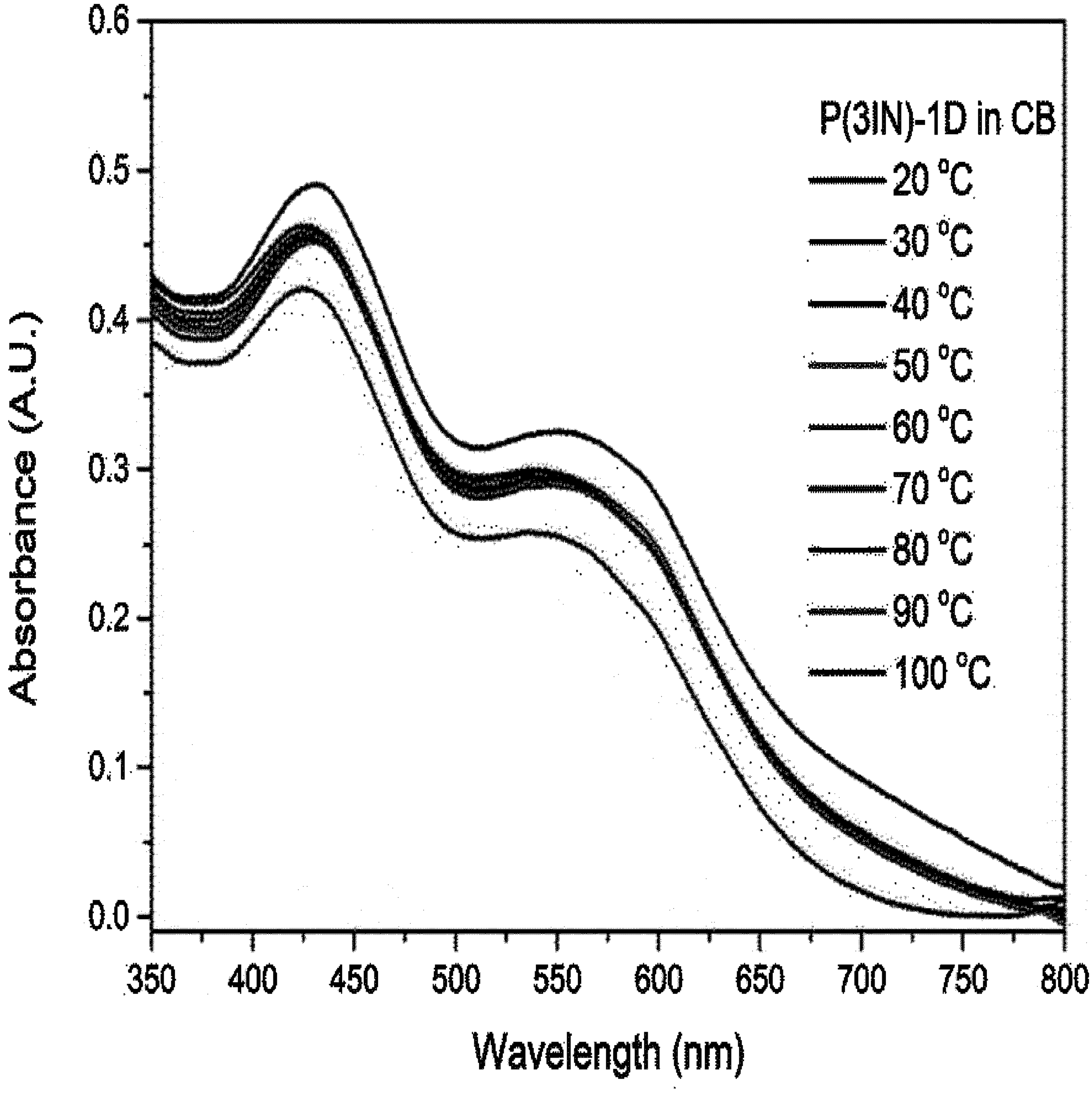
P(3IN)-1D in CB
20 °C
30 °C
40 °C
50 °C
60 °C
70 °C
80 °C
90 °C
100 °C
Absorbance (A.U.)
0.0
0.1
0.2
0.3
0.4
0.5
0.6
Wavelength (nm)
350
400
450
500
550
600
650
700
750
800

【Fig. 94】

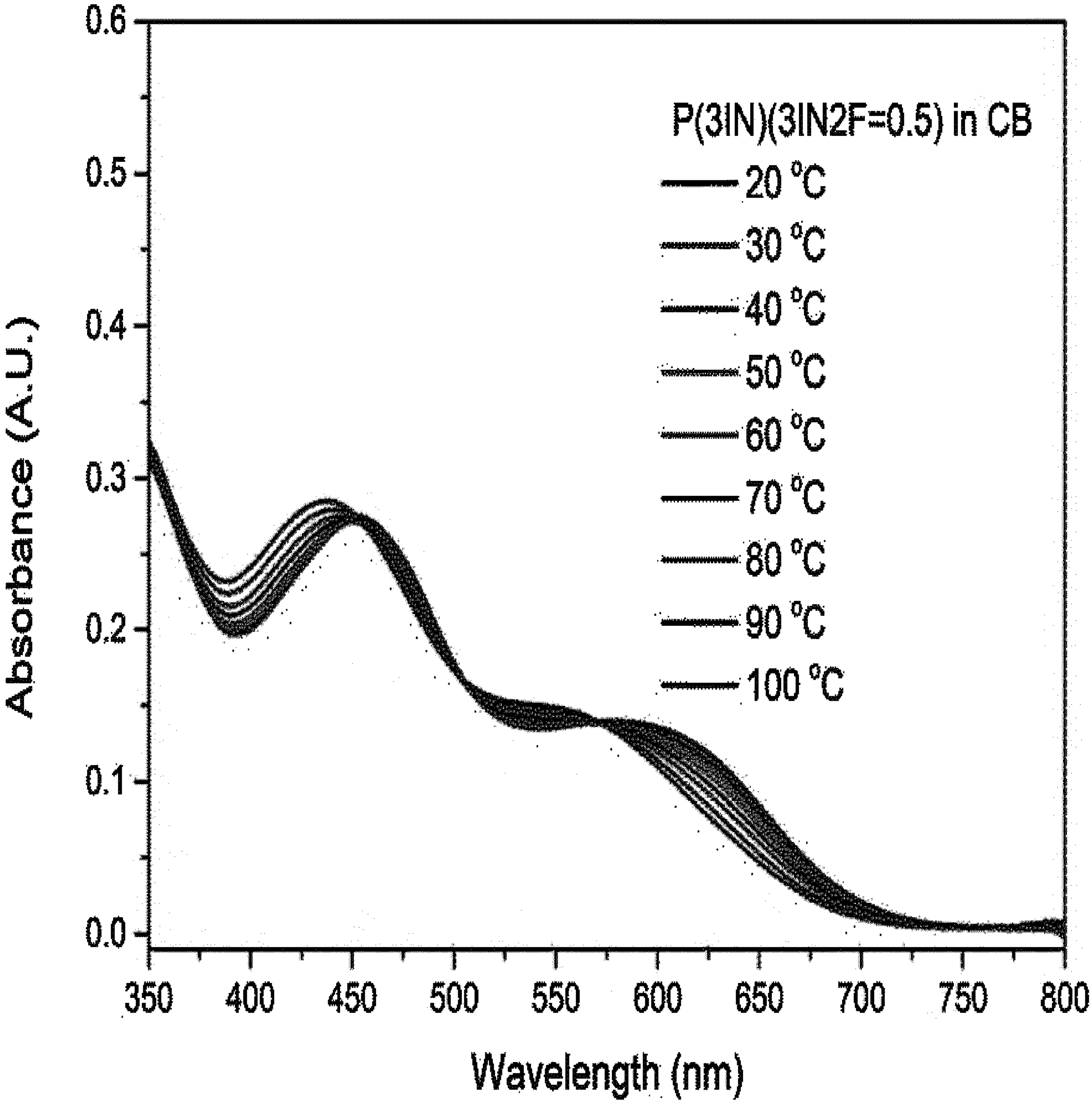
P(3IN)(3IN2F=0.5) in CB
20 °C
30 °C
40 °C
50 °C
60 °C
70 °C
80 °C
90 °C
100 °C
Absorbance (A.U.)
0.0
0.1
0.2
0.3
0.4
0.5
0.6
350
400
450
500
550
600
650
700
750
800
Wavelength (nm)

【Fig. 95】

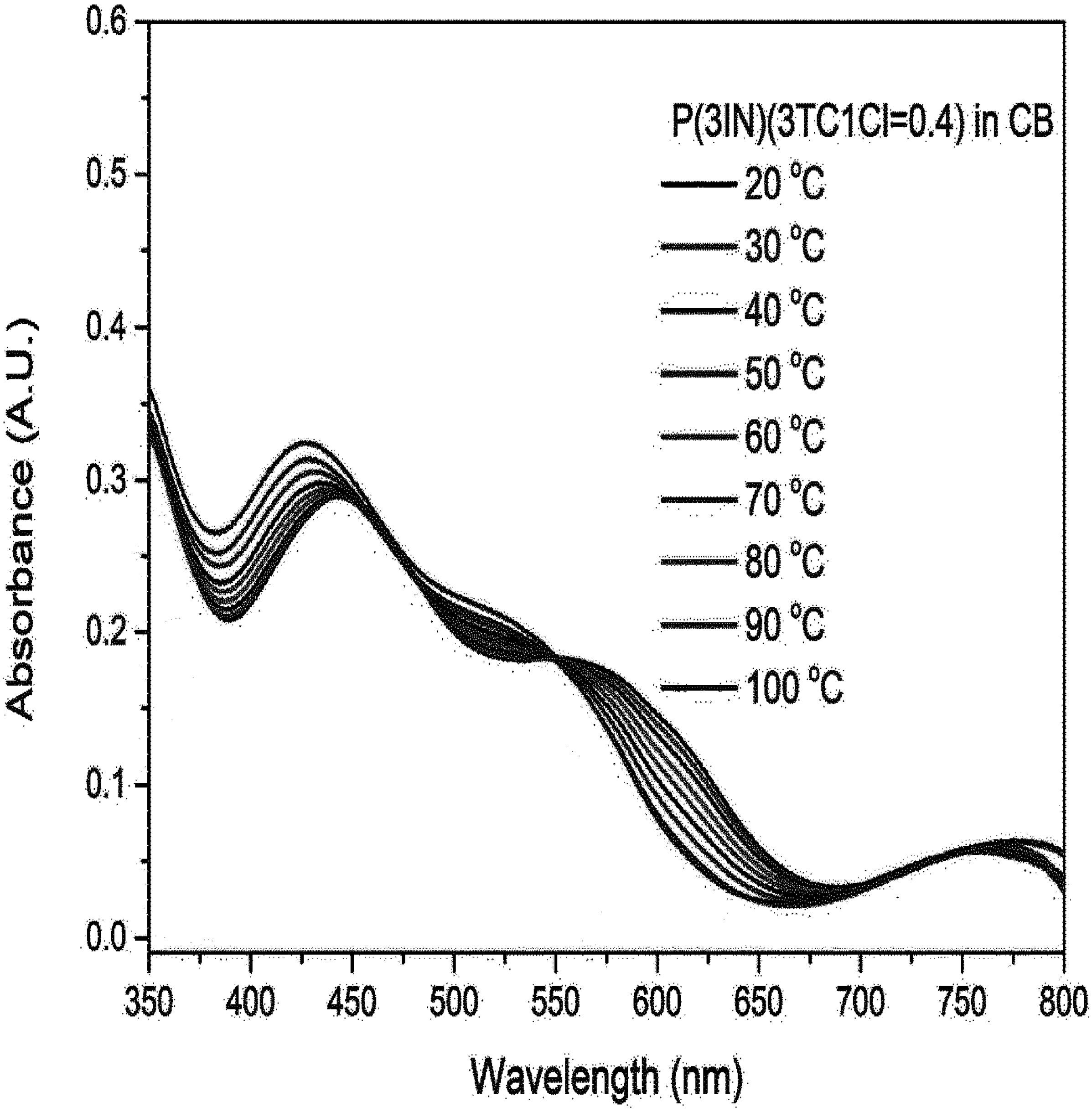
P(3IN)(3TC1Cl=0.4) in CB
20 °C
30 °C
40 °C
50 °C
60 °C
70 °C
80 °C
90 °C
100 °C
Absorbance (A.U.)
Wavelength (nm)
0.0
0.1
0.2
0.3
0.4
0.5
0.6
350
400
450
500
550
600
650
700
750
800

【Fig. 96】

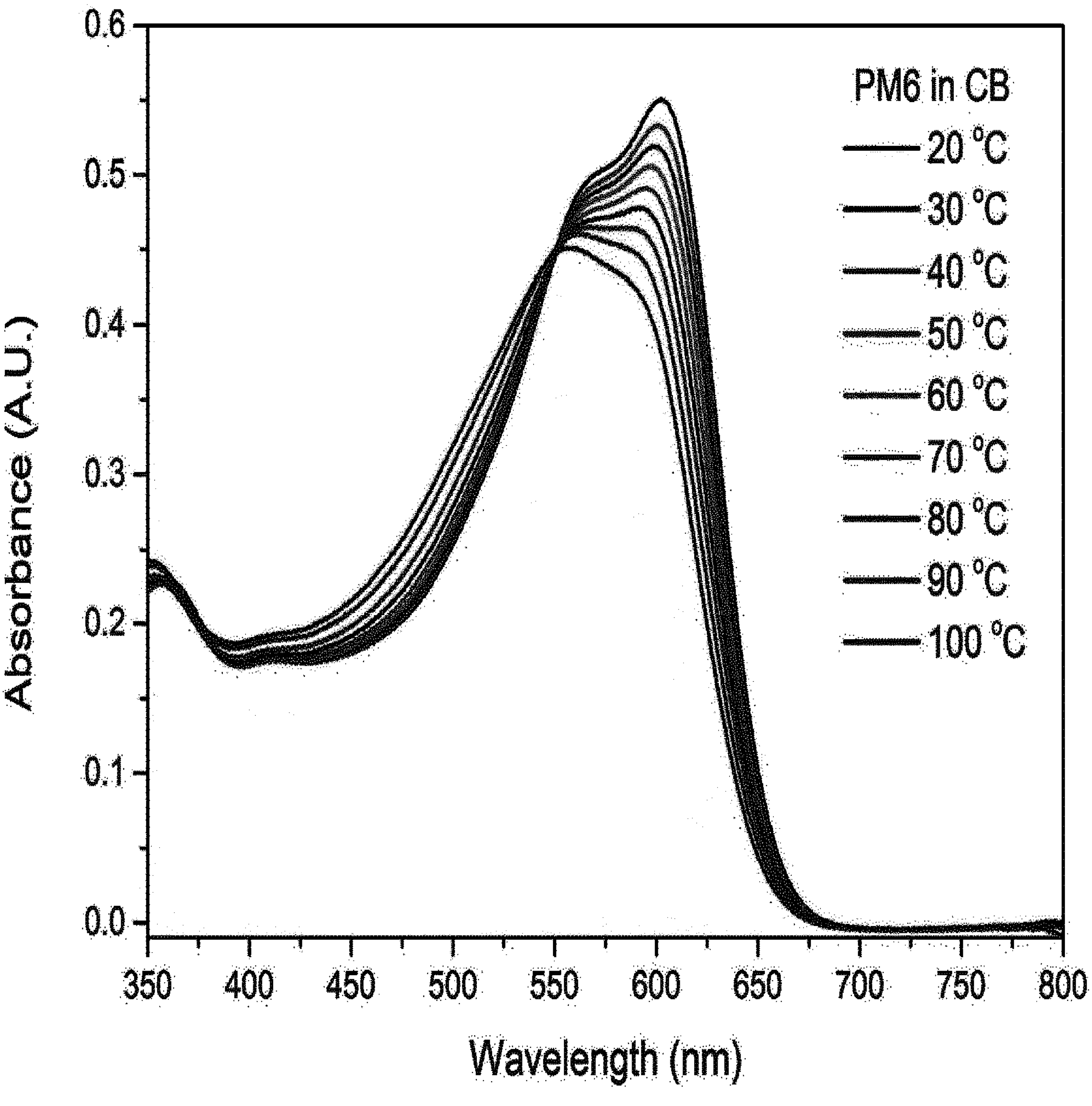
PM6 in CB
20 °C
30 °C
40 °C
50 °C
60 °C
70 °C
80 °C
90 °C
100 °C
Absorbance (A.U.)
Wavelength (nm)
0.0
0.1
0.2
0.3
0.4
0.5
0.6
350
400
450
500
550
600
650
700
750
800

【Fig. 97】

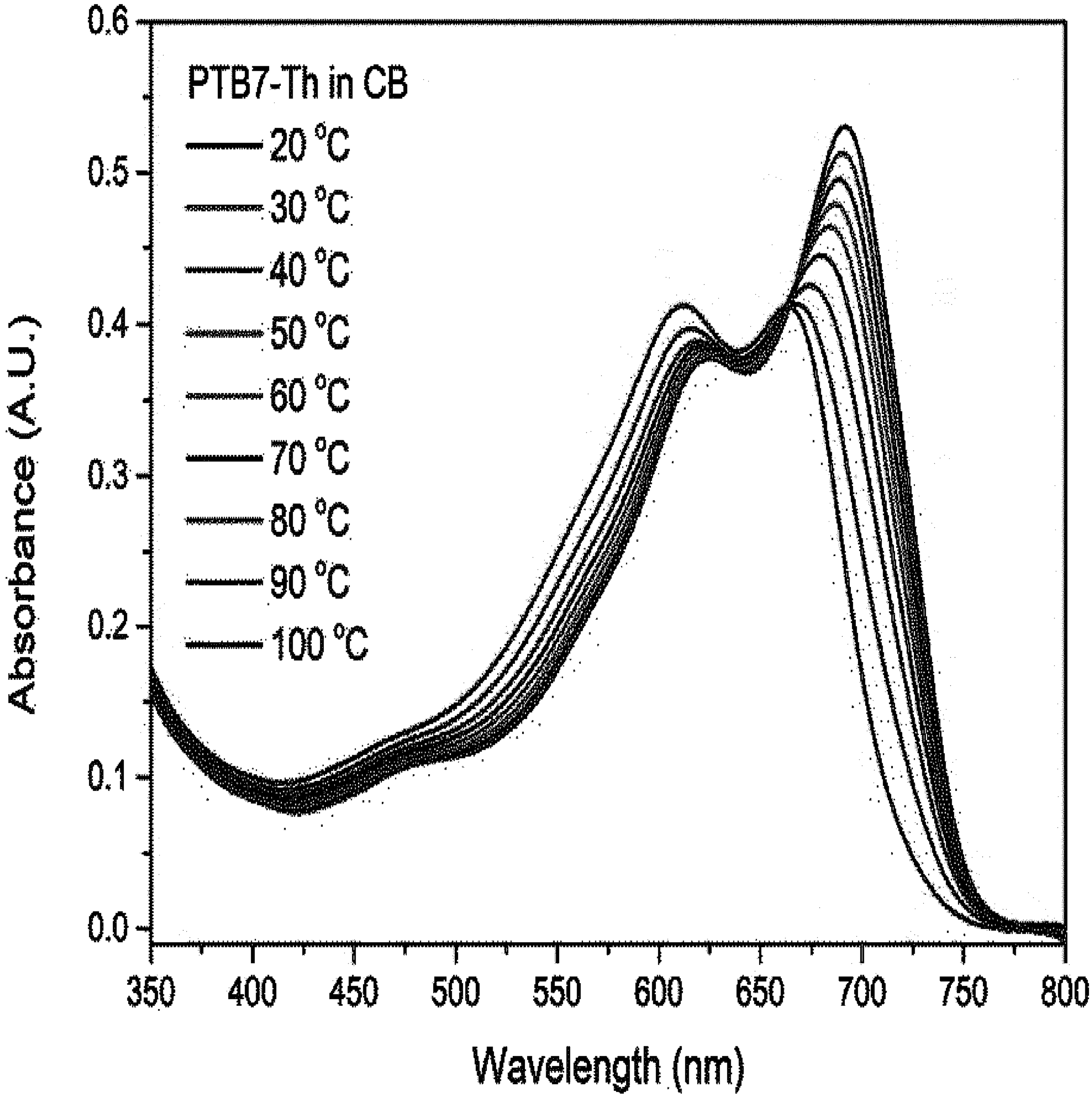
PTB7-Th in CB
20 °C
30 °C
40 °C
50 °C
60 °C
70 °C
80 °C
90 °C
100 °C
Absorbance (A.U.)
Wavelength (nm)
0.0
0.1
0.2
0.3
0.4
0.5
0.6
350
400
450
500
550
600
650
700
750
800

【Fig. 98】

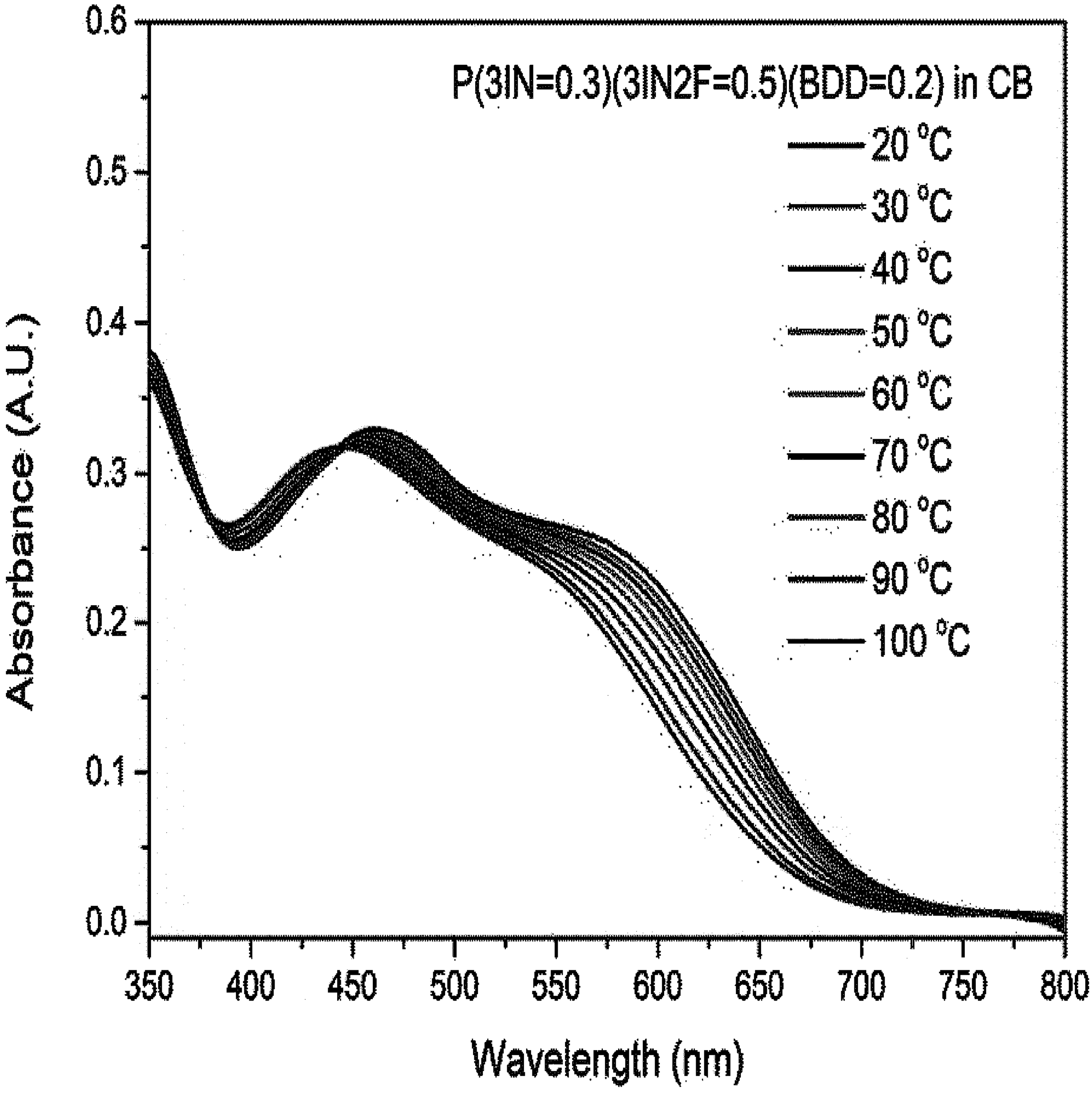
P(3IN=0.3)(3IN2F=0.5)(BDD=0.2) in CB
20 °C
30 °C
40 °C
50 °C
60 °C
70 °C
80 °C
90 °C
100 °C
Absorbance (A.U.)
0.0
0.1
0.2
0.3
0.4
0.5
0.6
Wavelength (nm)
350
400
450
500
550
600
650
700
750
800

【Fig. 99】

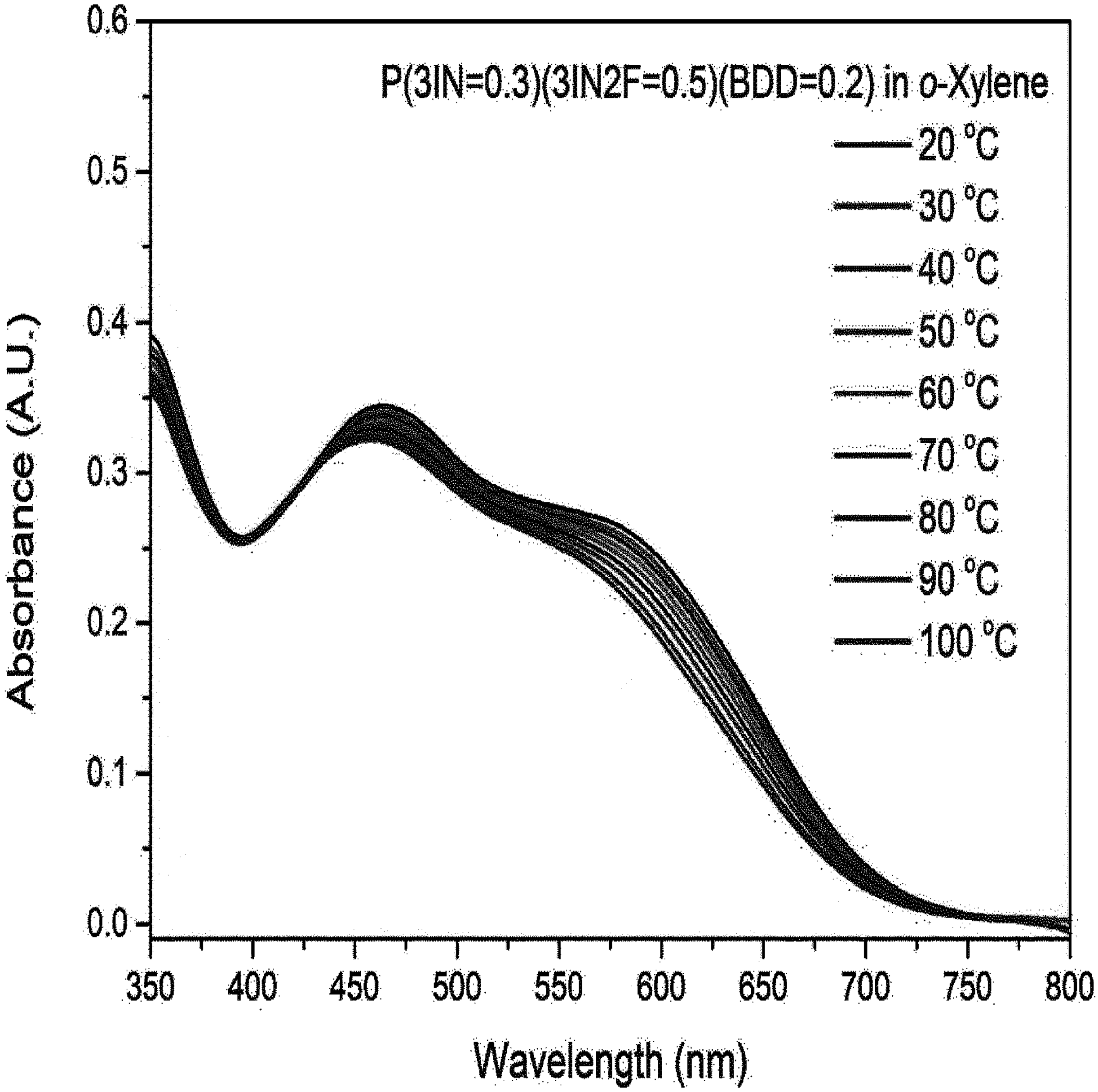
P(3IN=0.3)(3IN2F=0.5)(BDD=0.2) in o-Xylene
20 °C
30 °C
40 °C
50 °C
60 °C
70 °C
80 °C
90 °C
100 °C
Absorbance (A.U.)
0.6
0.5
0.4
0.3
0.2
0.1
0.0
350
400
450
500
550
600
650
700
750
800
Wavelength (nm)

【Fig. 100】

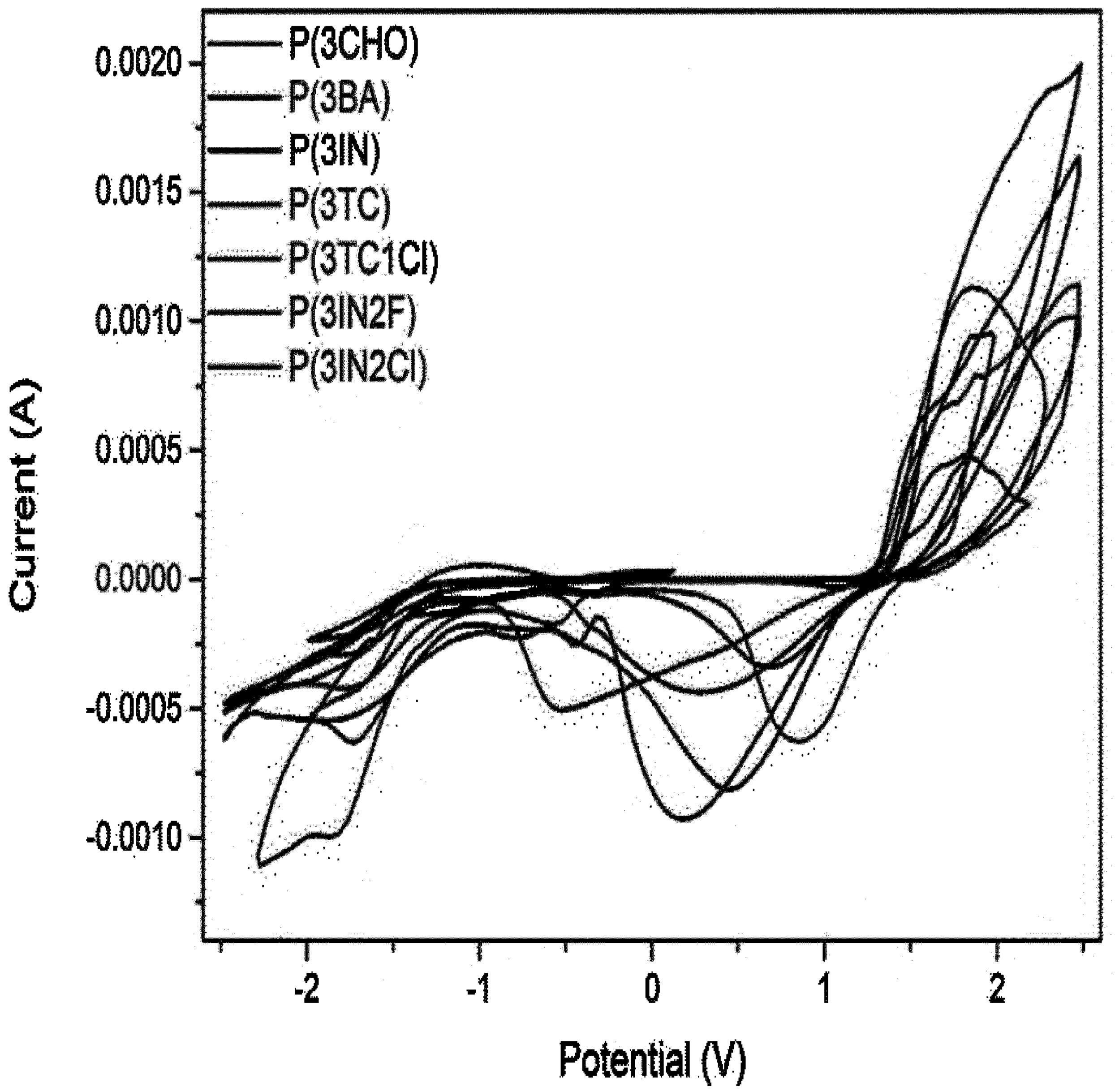
P(3CHO)
P(3BA)
P(3IN)
P(3TC)
P(3TC1Cl)
P(3IN2F)
P(3IN2Cl)
0.0020
0.0015
0.0010
0.0005
0.0000
-0.0005
-0.0010
Current (A)
-2
-1
0
1
2
Potential (V)

【Fig. 101】

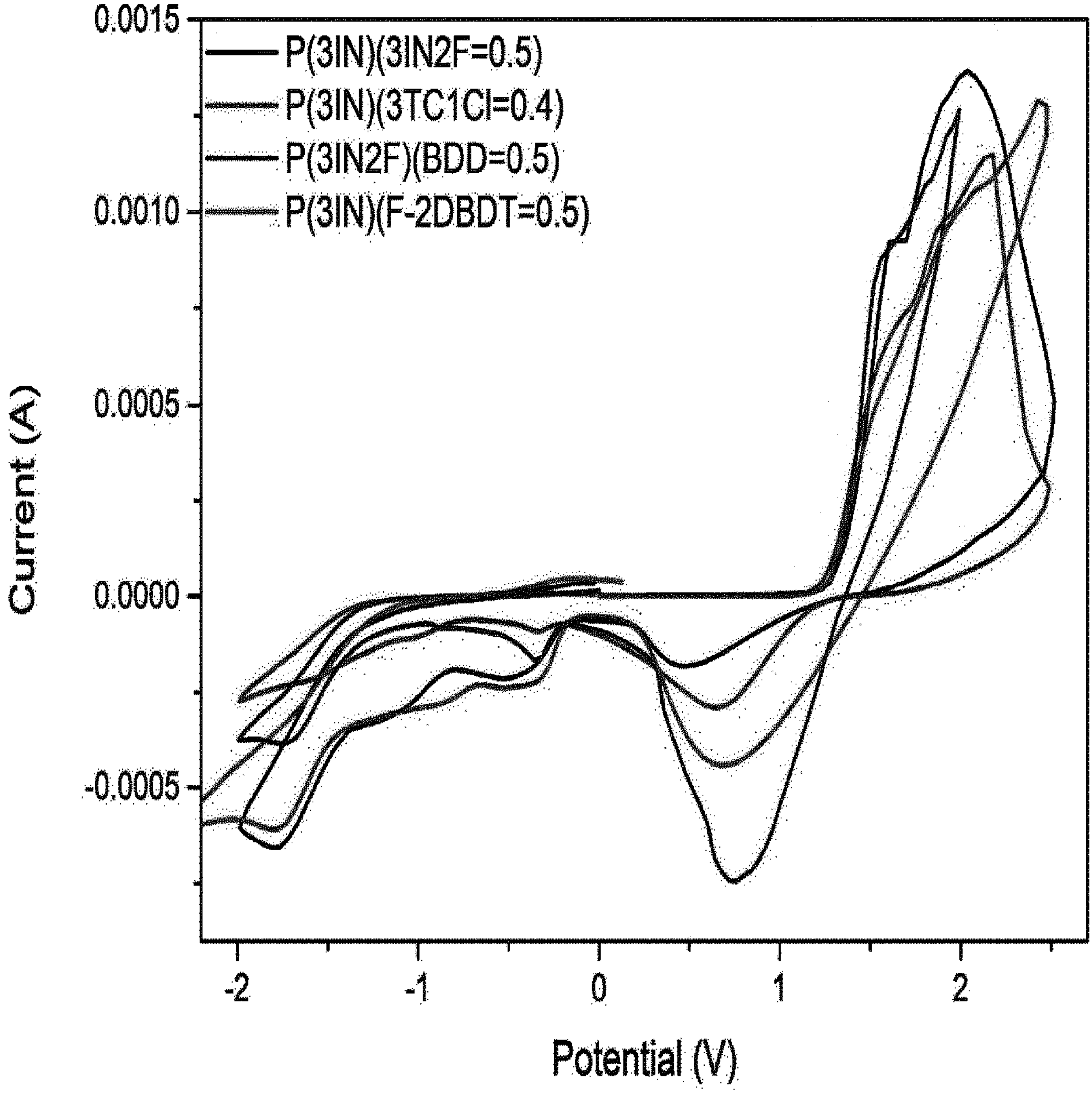
0.0015
0.0010
0.0005
0.0000
-0.0005
Current (A)
-2
-1
0
1
2
Potential (V)
P(3IN)(3IN2F=0.5)
P(3IN)(3TC1Cl=0.4)
P(3IN2F)(BDD=0.5)
P(3IN)(F-2DBDT=0.5)

【Fig. 102】

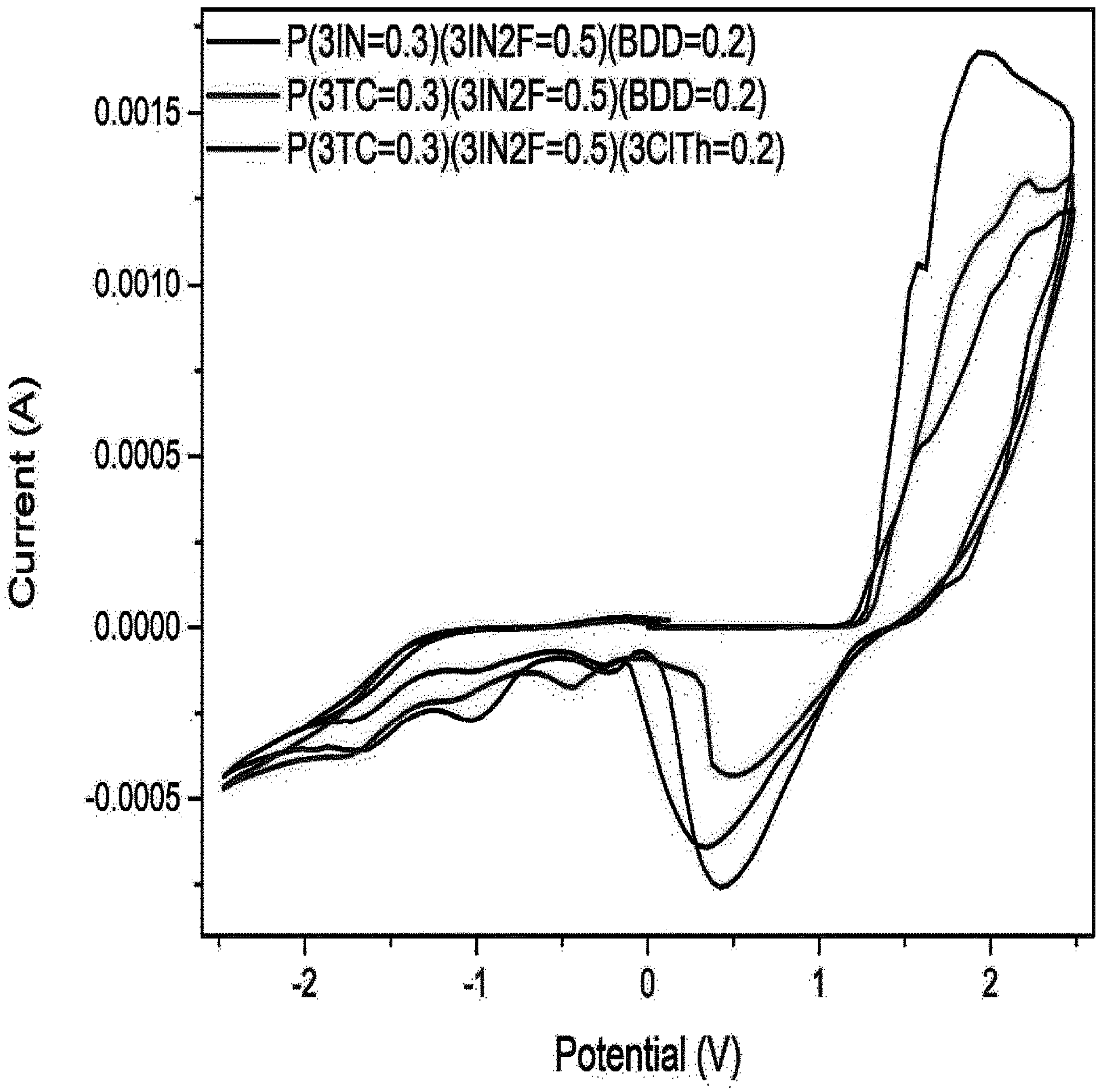
P(3IN=0.3)(3IN2F=0.5)(BDD=0.2)
P(3TC=0.3)(3IN2F=0.5)(BDD=0.2)
P(3TC=0.3)(3IN2F=0.5)(3ClTh=0.2)
0.0015
0.0010
0.0005
0.0000
-0.0005
Current (A)
-2
-1
0
1
2
Potential (V)

【Fig. 103】

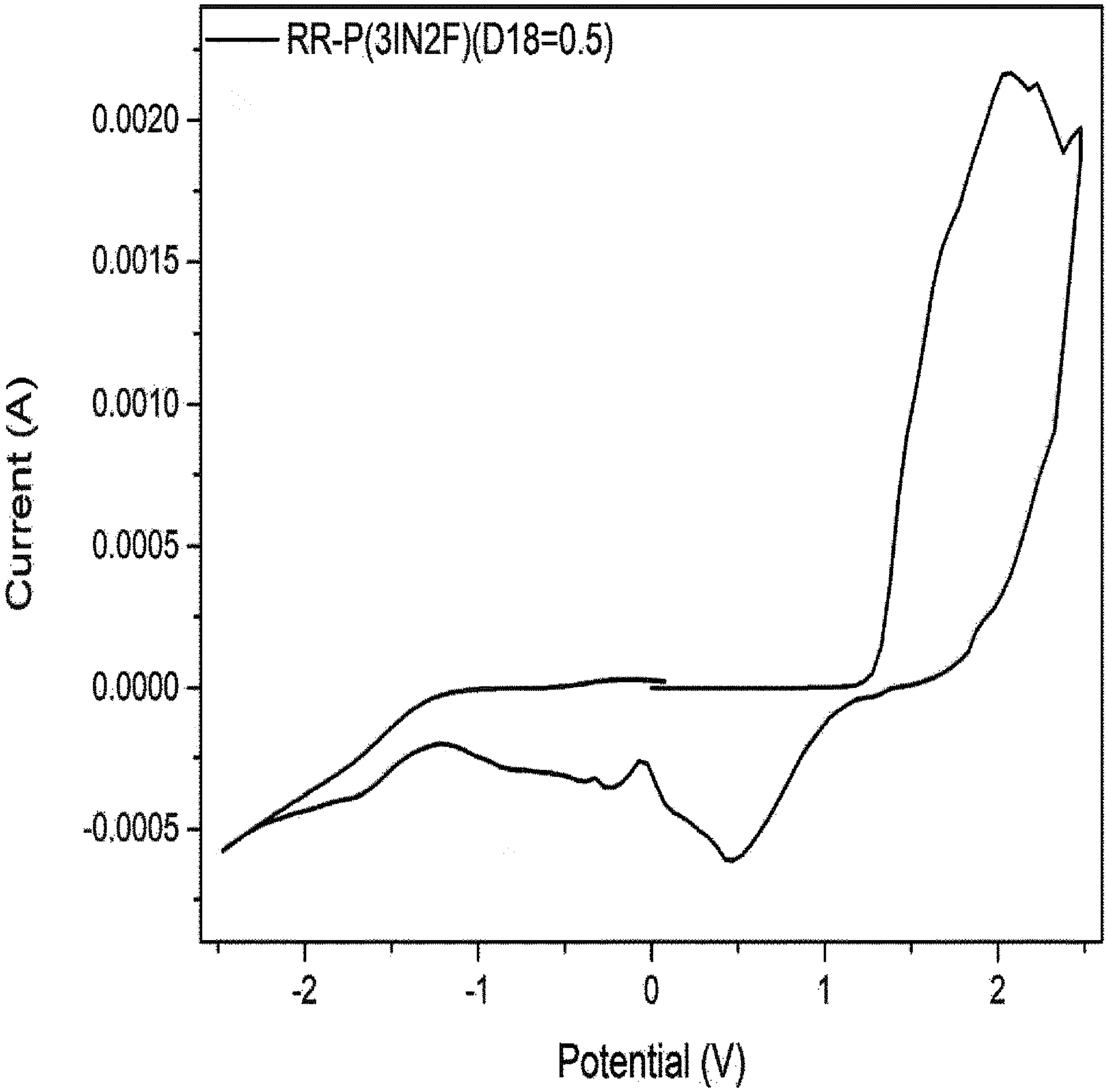
RR-P(3IN2F)(D18=0.5)
0.0020
0.0015
0.0010
0.0005
0.0000
-0.0005
Current (A)
-2
-1
0
1
2
Potential (V)

【Fig. 104】

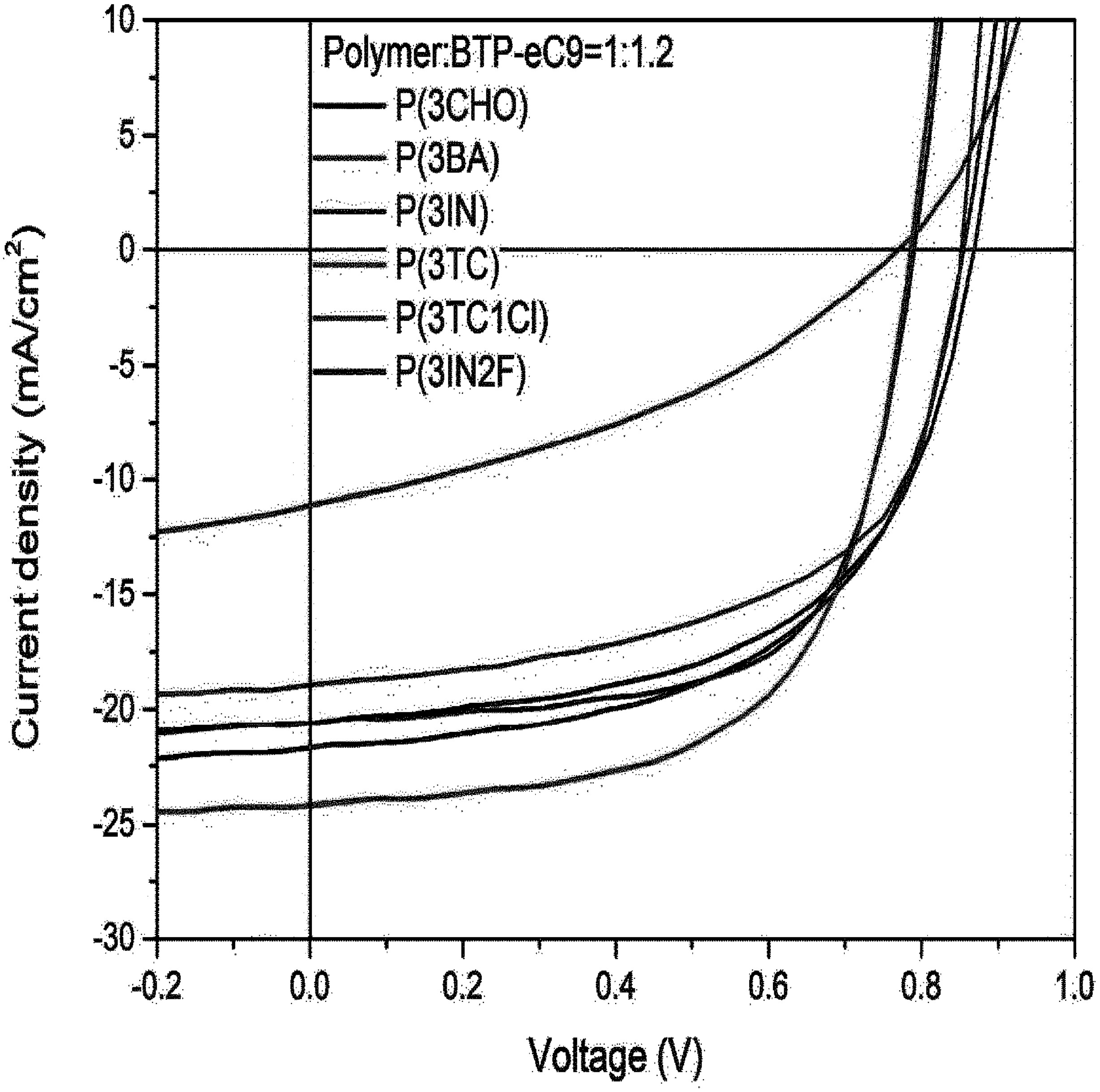
Polymer:BTP-eC9=1:1.2
P(3CHO)
P(3BA)
P(3IN)
P(3TC)
P(3TC1Cl)
P(3IN2F)
Current density (mA/cm2)
10
5
0
-5
-10
-15
-20
-25
-30
-0.2
0.0
0.2
0.4
0.6
0.8
1.0
Voltage (V)

【Fig. 105】

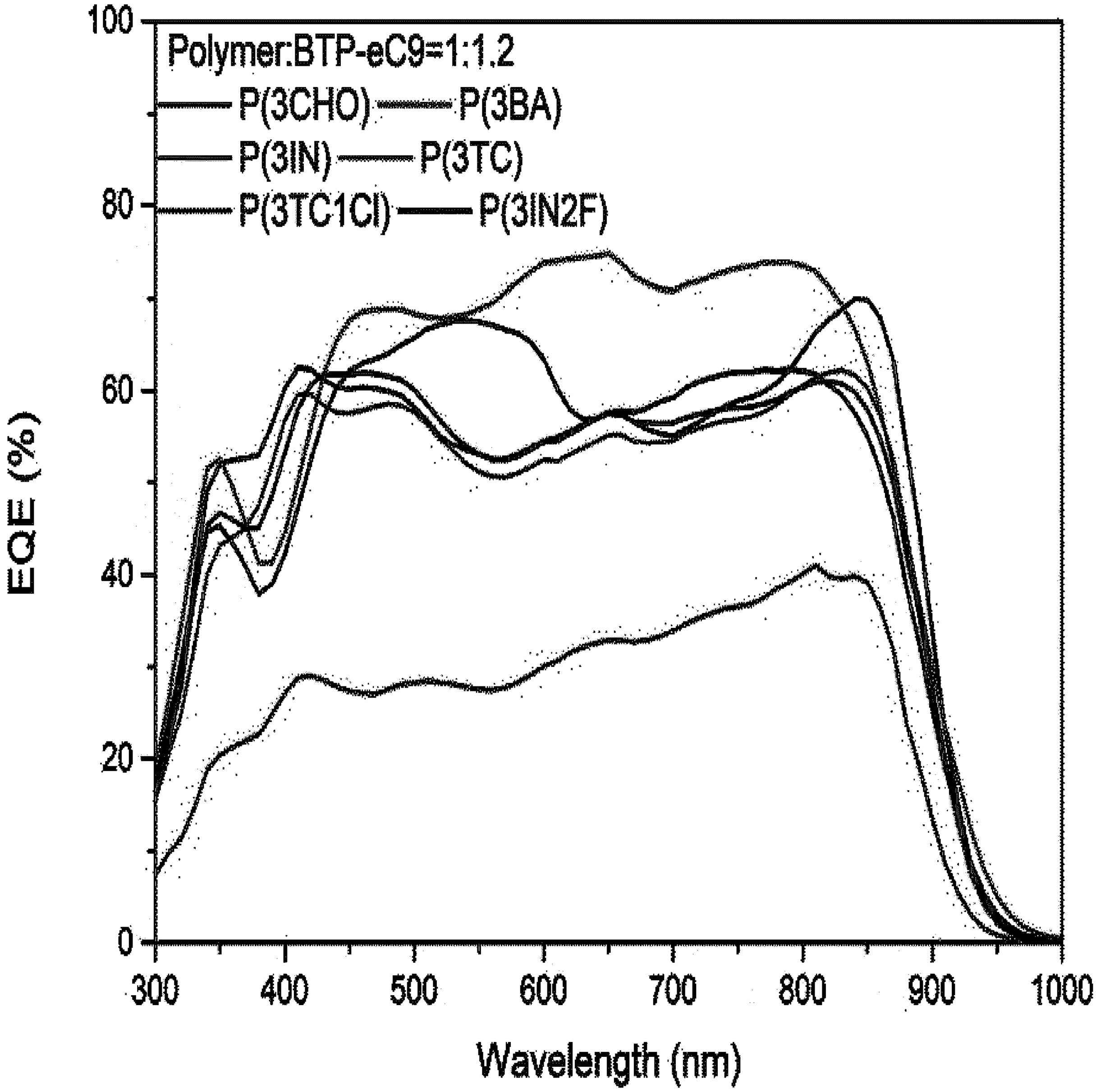

Polymer:BTP-eC9=1:1.2
P(3CHO)
P(3BA)
P(3IN)
P(3TC)
P(3TC1Cl)
P(3IN2F)
EQE (%)
Wavelength (nm)
0
20
40
60
80
100
300
400
500
600
700
800
900
1000

【Fig. 106】

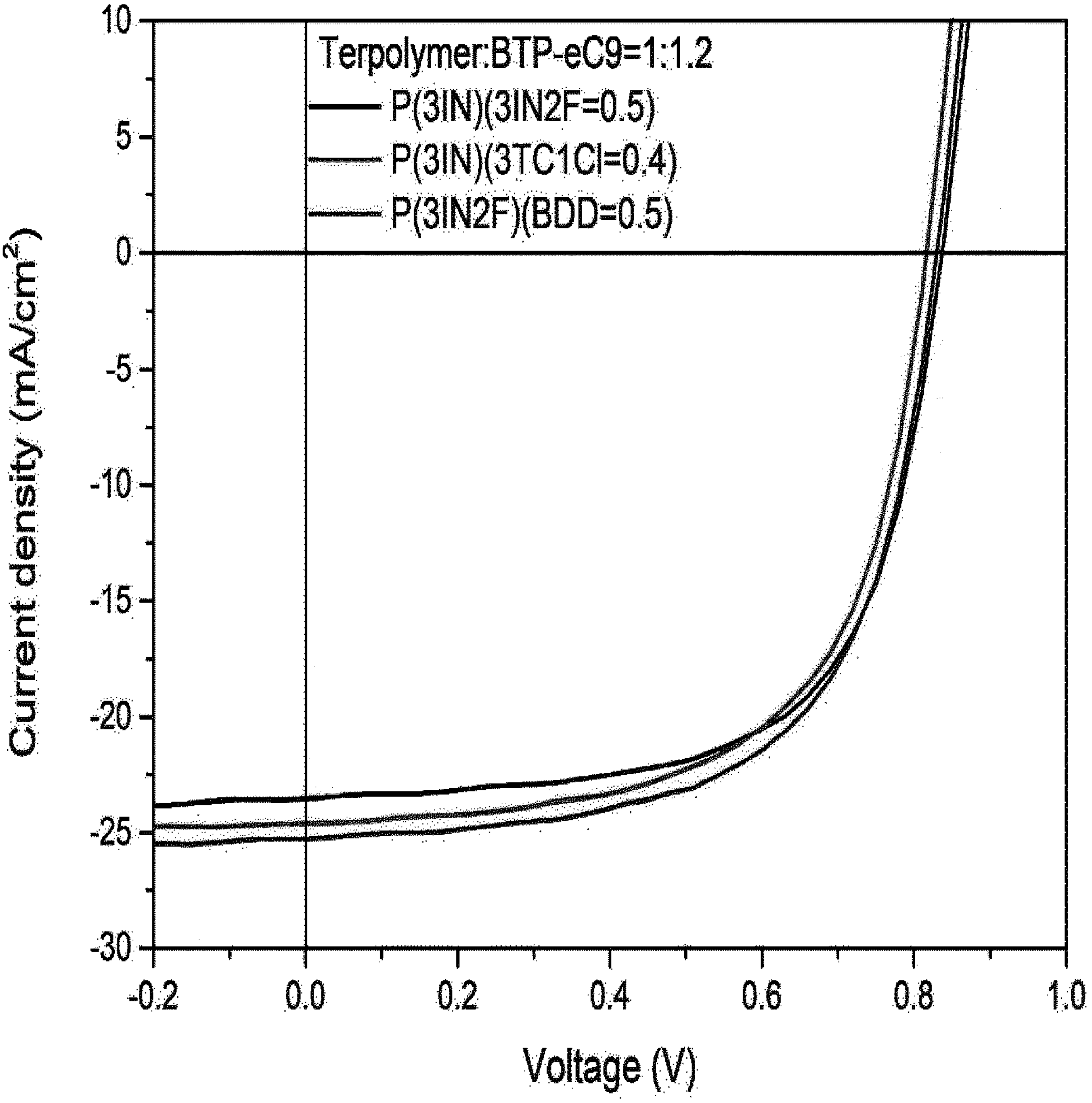
Terpolymer:BTP-eC9=1:1.2
P(3IN)(3IN2F=0.5)
P(3IN)(3TC1Cl=0.4)
P(3IN2F)(BDD=0.5)
Current density (mA/cm²)
Voltage (V)
10
5
0
-5
-10
-15
-20
-25
-30
-0.2
0.0
0.2
0.4
0.6
0.8
1.0

【Fig. 107】

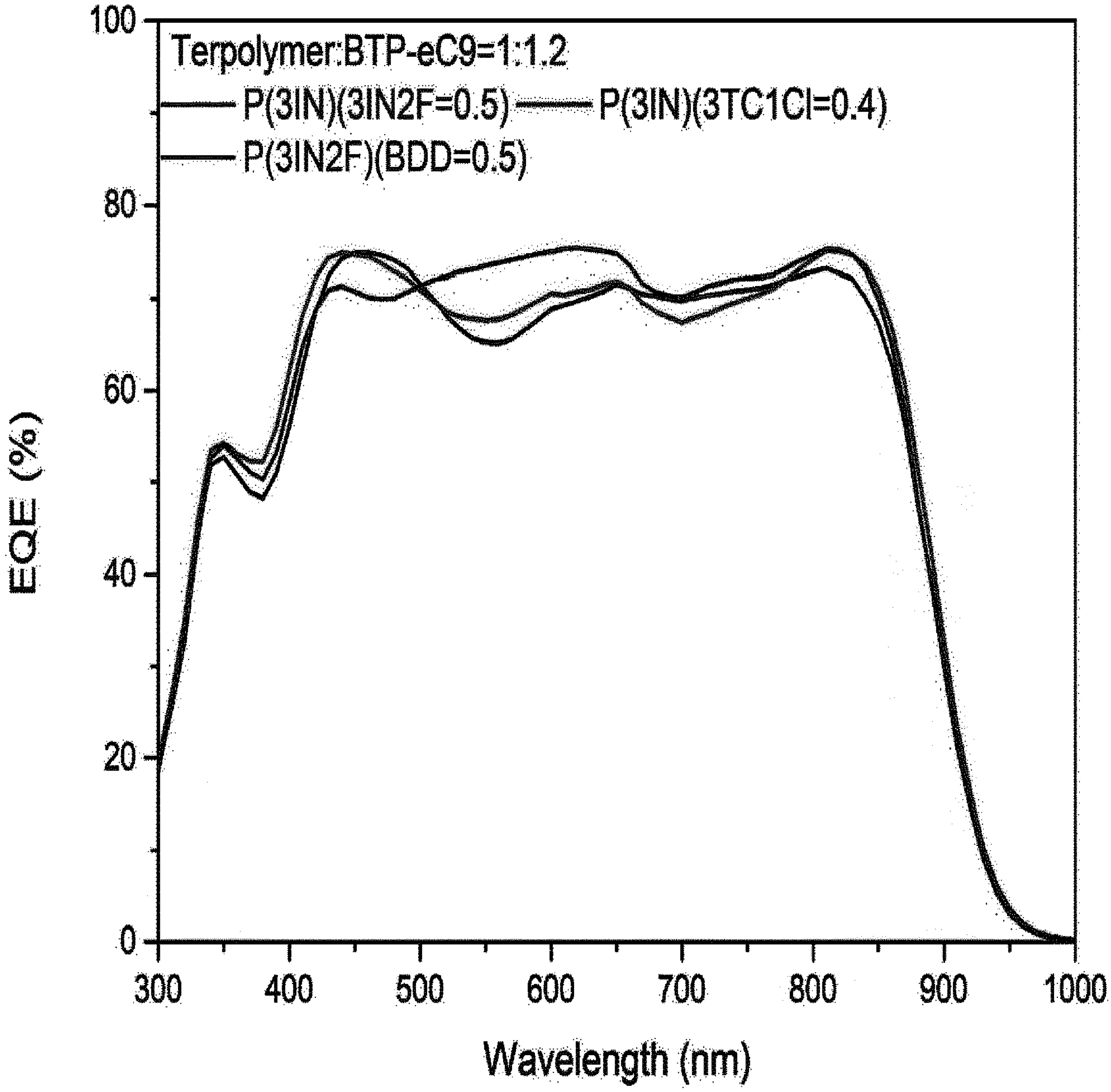
Terpolymer:BTP-eC9=1:1.2
P(3IN)(3IN2F=0.5)
P(3IN)(3TC1Cl=0.4)
P(3IN2F)(BDD=0.5)
EQE (%)
Wavelength (nm)
0
20
40
60
80
100
300
400
500
600
700
800
900
1000

【Fig. 108】

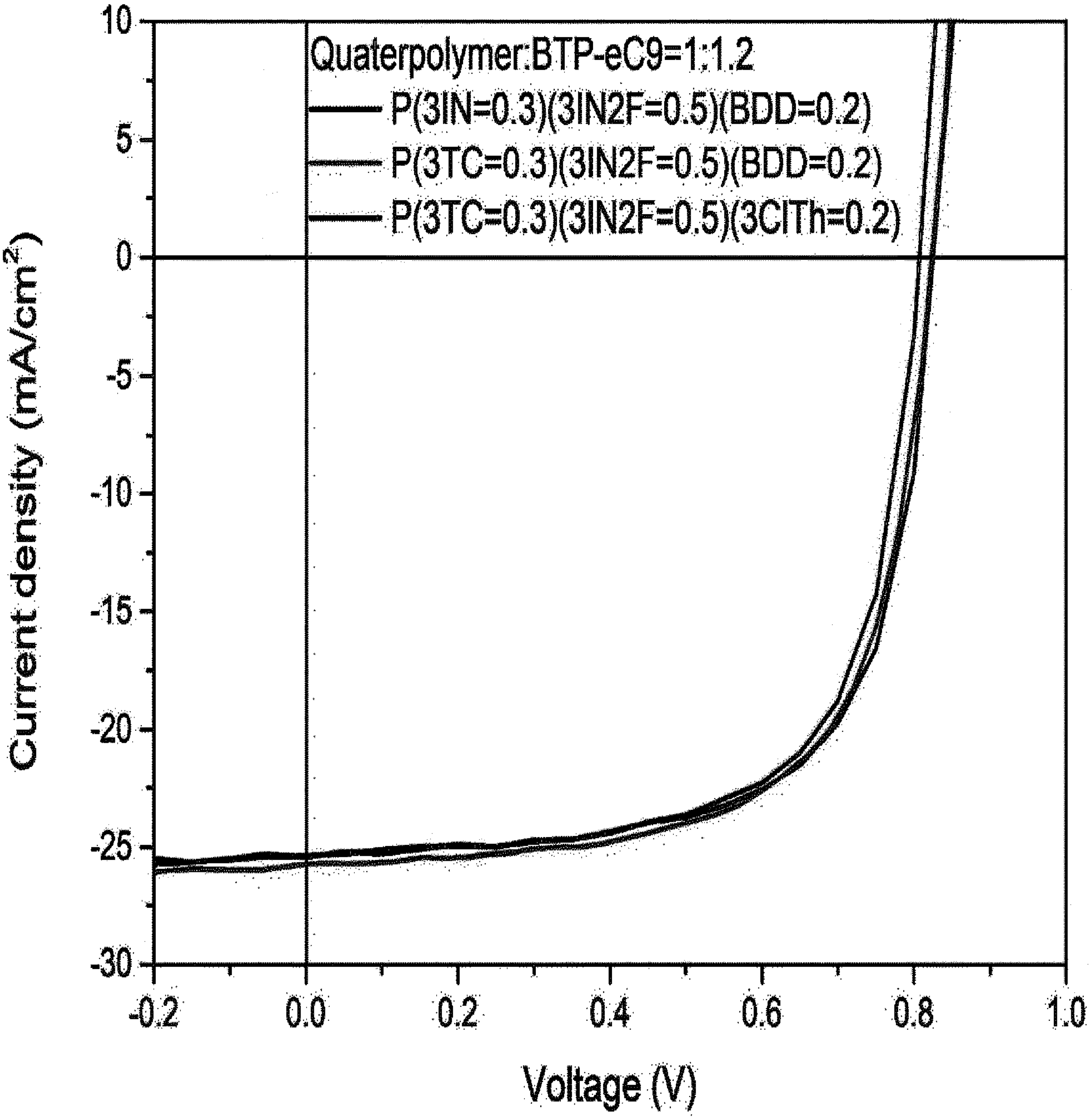
Quaterpolymer:BTP-eC9=1:1.2
P(3IN=0.3)(3IN2F=0.5)(BDD=0.2)
P(3TC=0.3)(3IN2F=0.5)(BDD=0.2)
P(3TC=0.3)(3IN2F=0.5)(3ClTh=0.2)
Current density (mA/cm2)
Voltage (V)
10
5
0
-5
-10
-15
-20
-25
-30
-0.2
0.0
0.2
0.4
0.6
0.8
1.0

【Fig. 109】

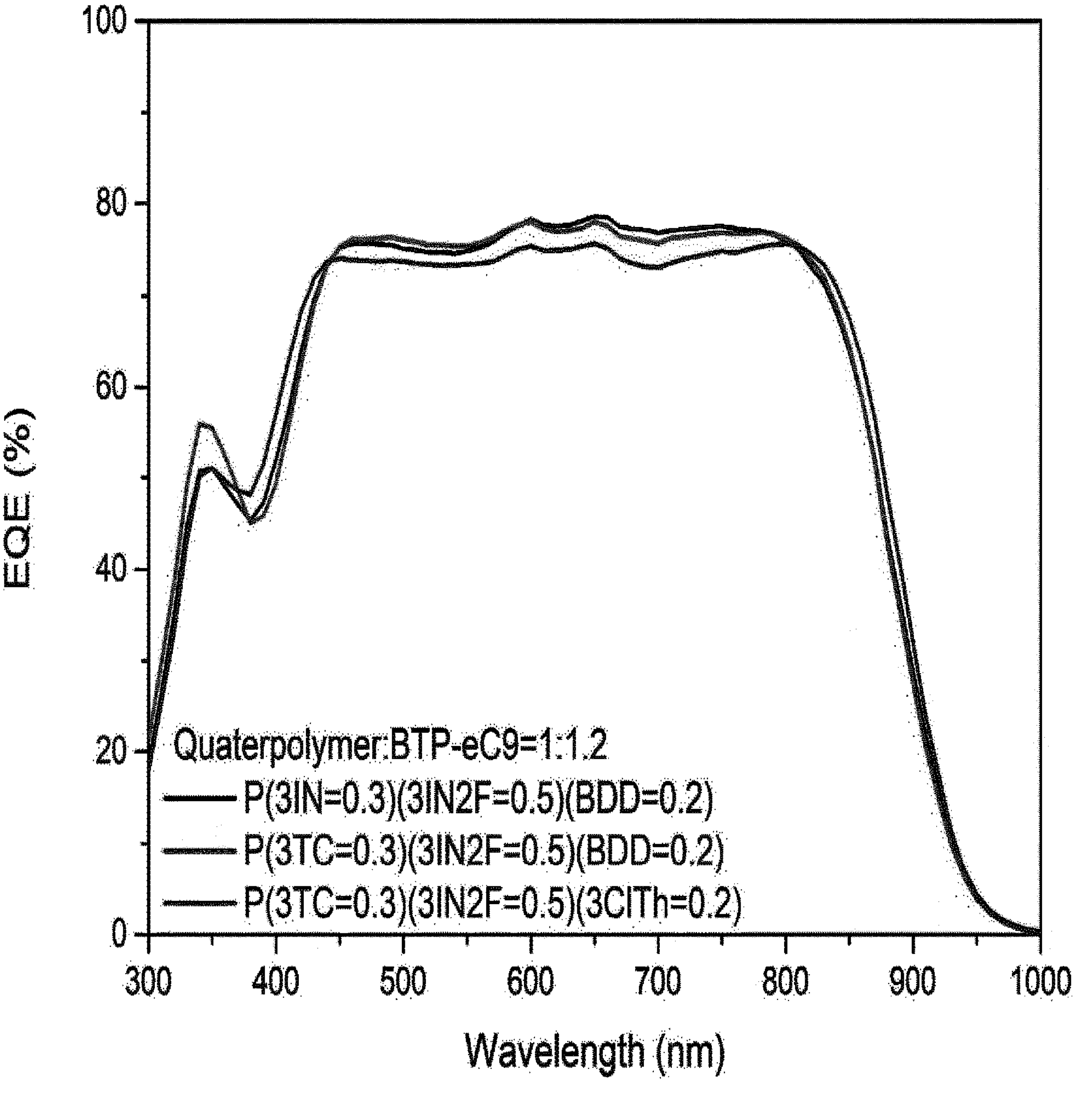
100
80
60
40
20
0
EQE (%)
300
400
500
600
700
800
900
1000
Wavelength (nm)
Quaterpolymer:BTP-eC9=1:1.2
P(3IN=0.3)(3IN2F=0.5)(BDD=0.2)
P(3TC=0.3)(3IN2F=0.5)(BDD=0.2)
P(3TC=0.3)(3IN2F=0.5)(3ClTh=0.2)

【Fig. 110】

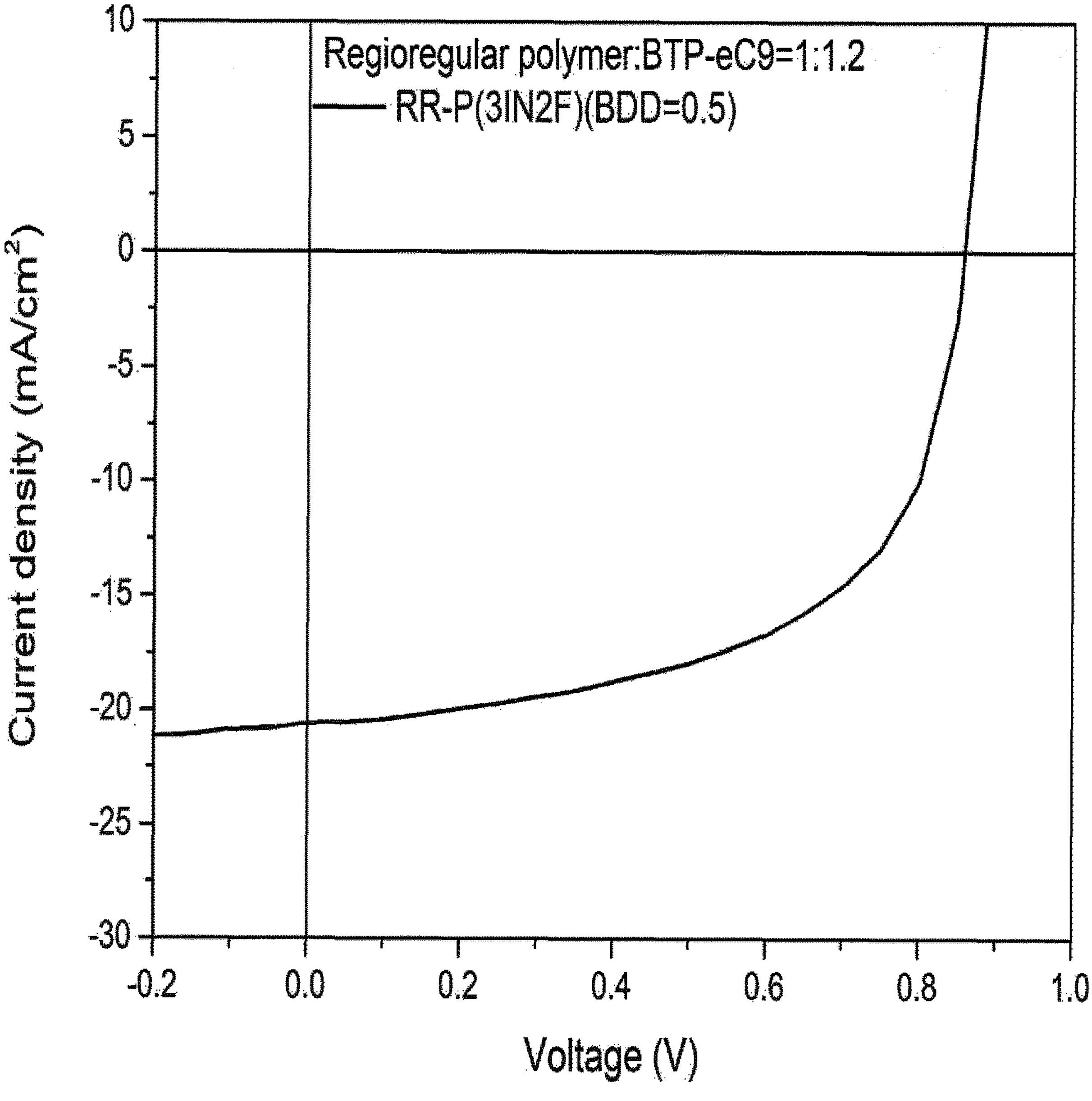
Regioregular polymer:BTP-eC9=1:1.2
RR-P(3IN2F)(BDD=0.5)
Current density (mA/cm2)
10
5
0
-5
-10
-15
-20
-25
-30
-0.2
0.0
0.2
0.4
0.6
0.8
1.0
Voltage (V)

【Fig. 111】

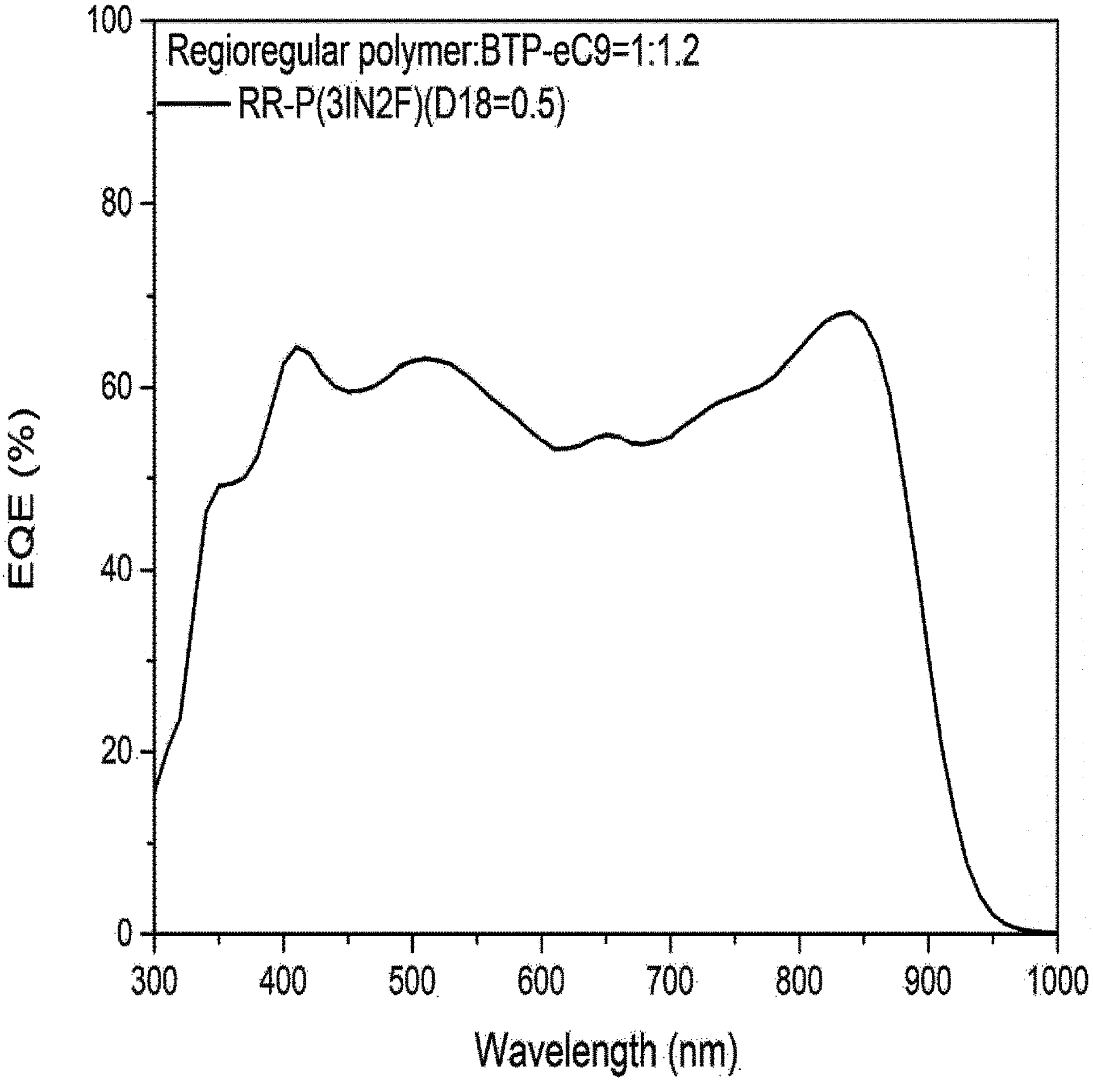
Regioregular polymer:BTP-eC9=1:1.2
RR-P(3IN2F)(D18=0.5)
EQE (%)
Wavelength (nm)
0
20
40
60
80
100
300
400
500
600
700
800
900
1000

【Fig. 112】

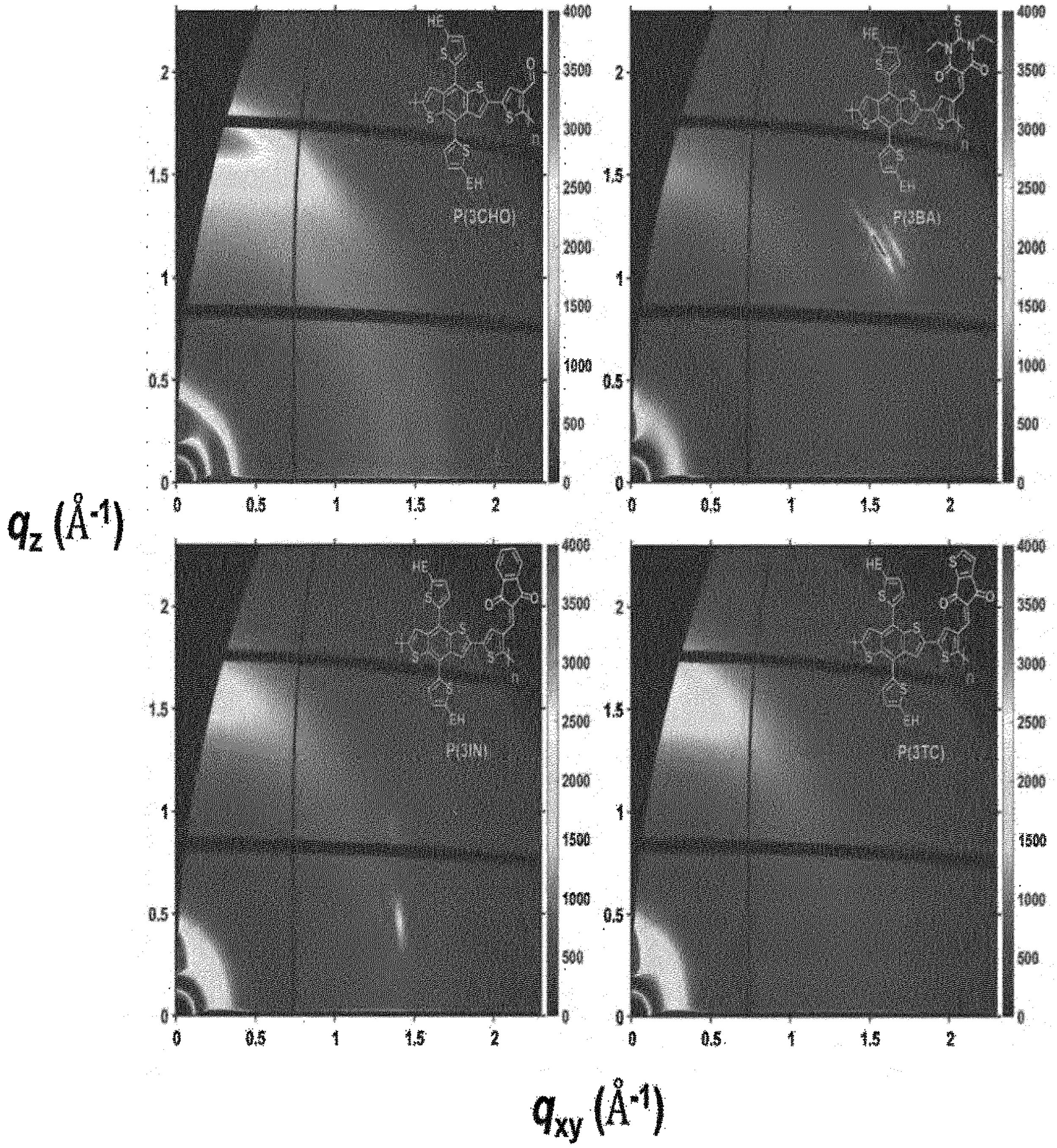
P(3CHO)
P(3BA)
P(3IN)
P(3TC)
$q_z$ (Å$^{-1}$)
$q_{xy}$ (Å$^{-1}$)
0
500
1000
1500
2000
2500
3000
3500
4000
0
0.5
1
1.5
2

【Fig. 113】

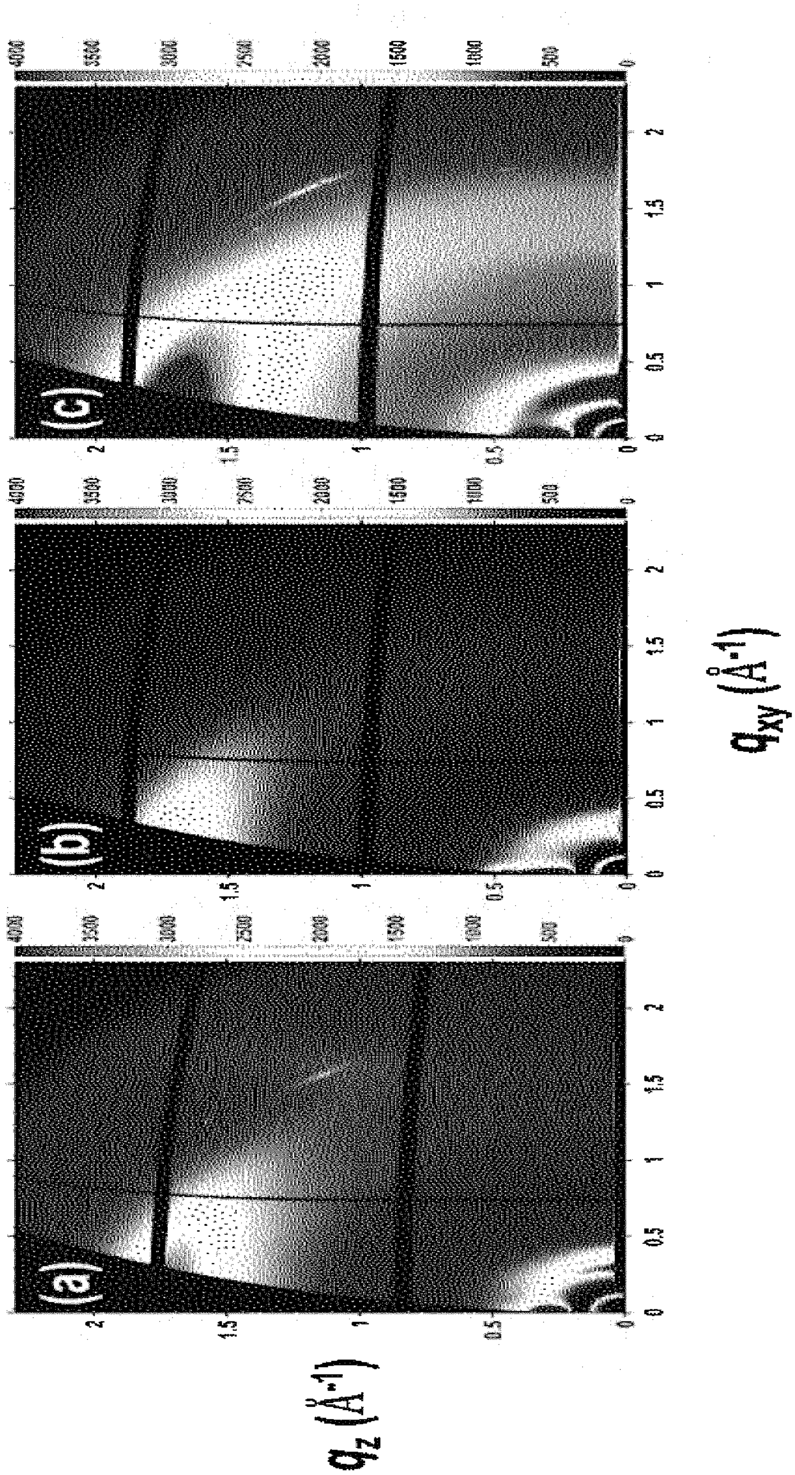

【Fig. 114】

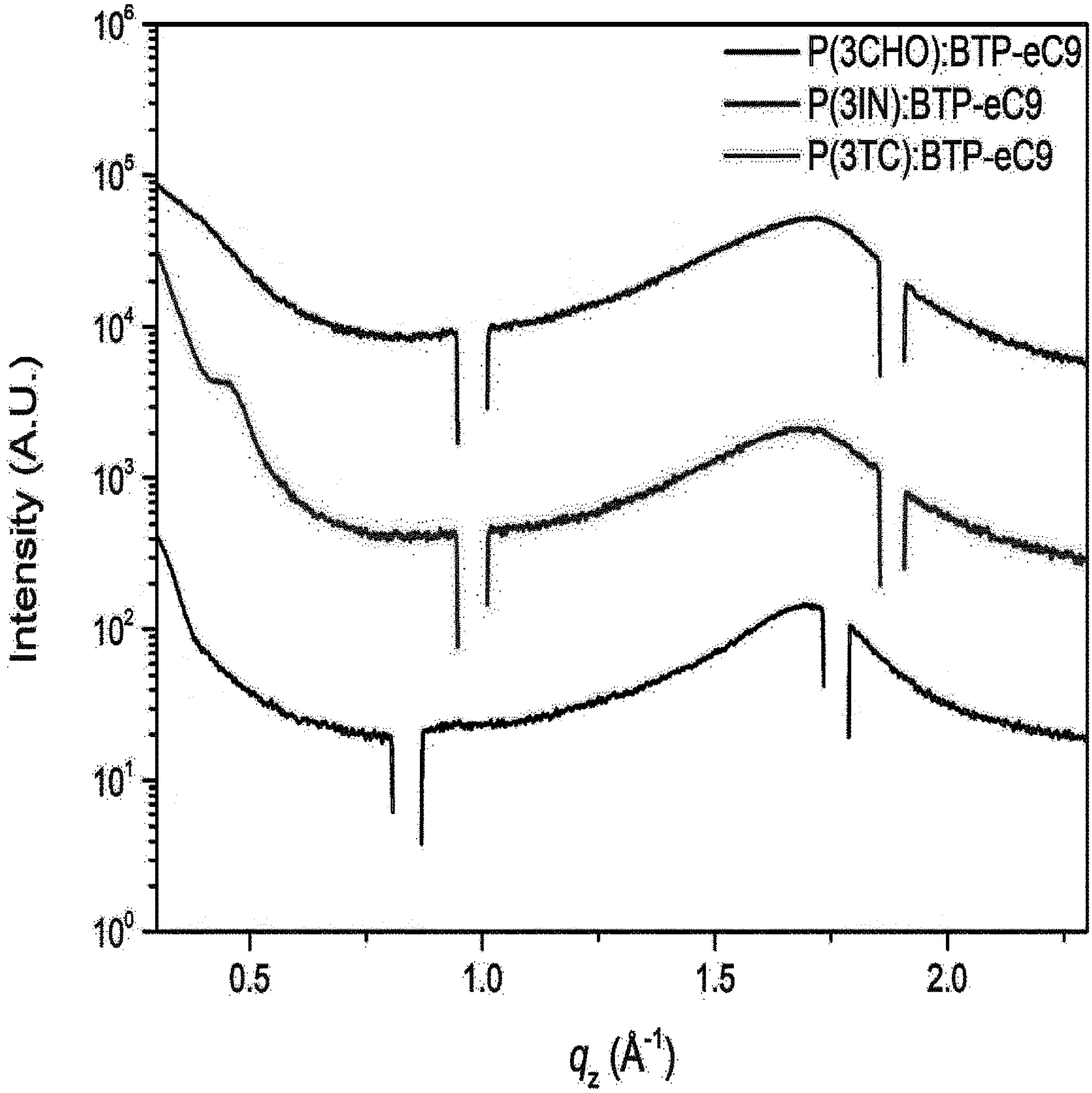
P(3CHO):BTP-eC9
P(3IN):BTP-eC9
P(3TC):BTP-eC9
Intensity (A.U.)
10^6
10^5
10^4
10^3
10^2
10^1
10^0
0.5
1.0
1.5
2.0
q_z (Å^-1)

【Fig. 115】

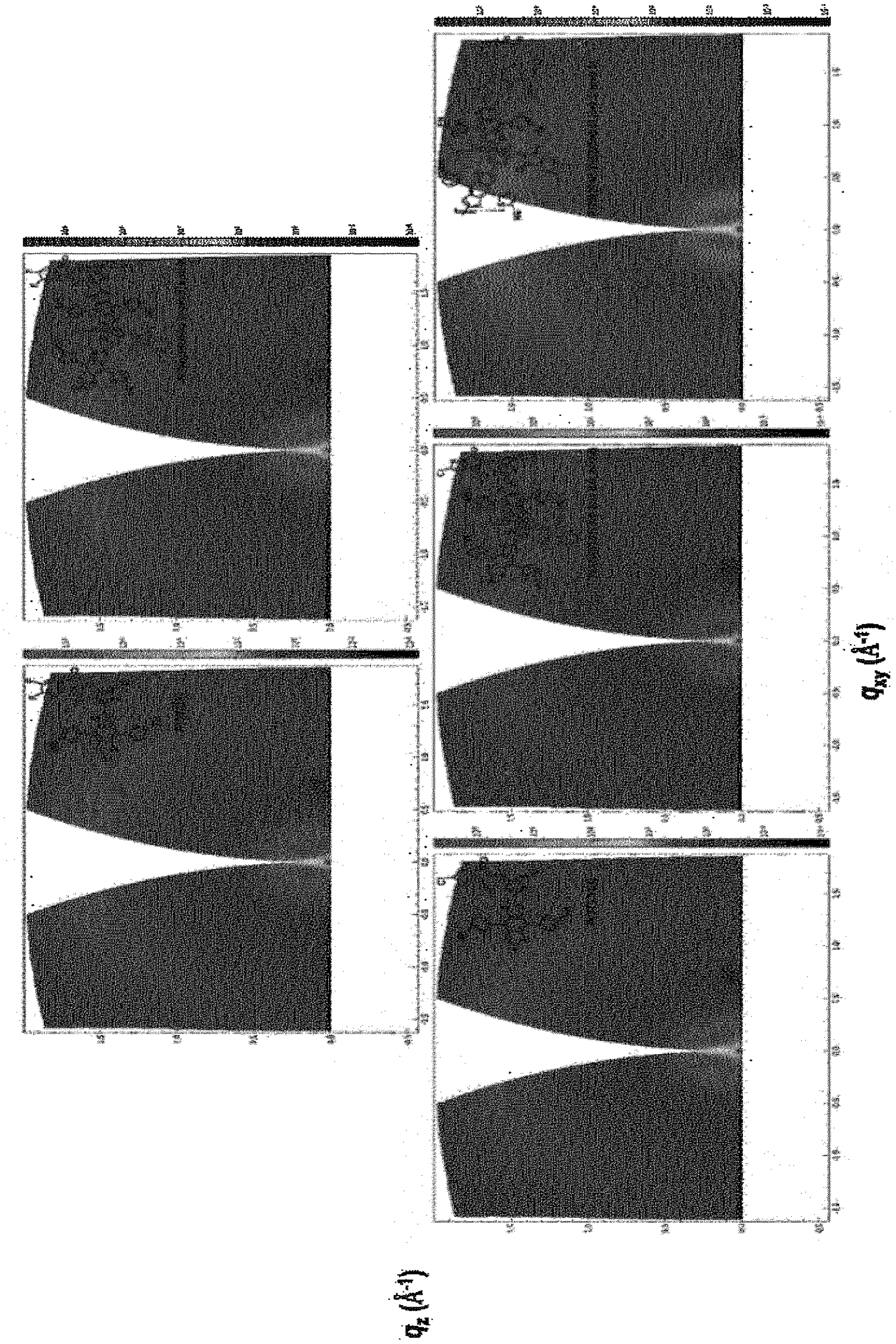

【Fig. 116】

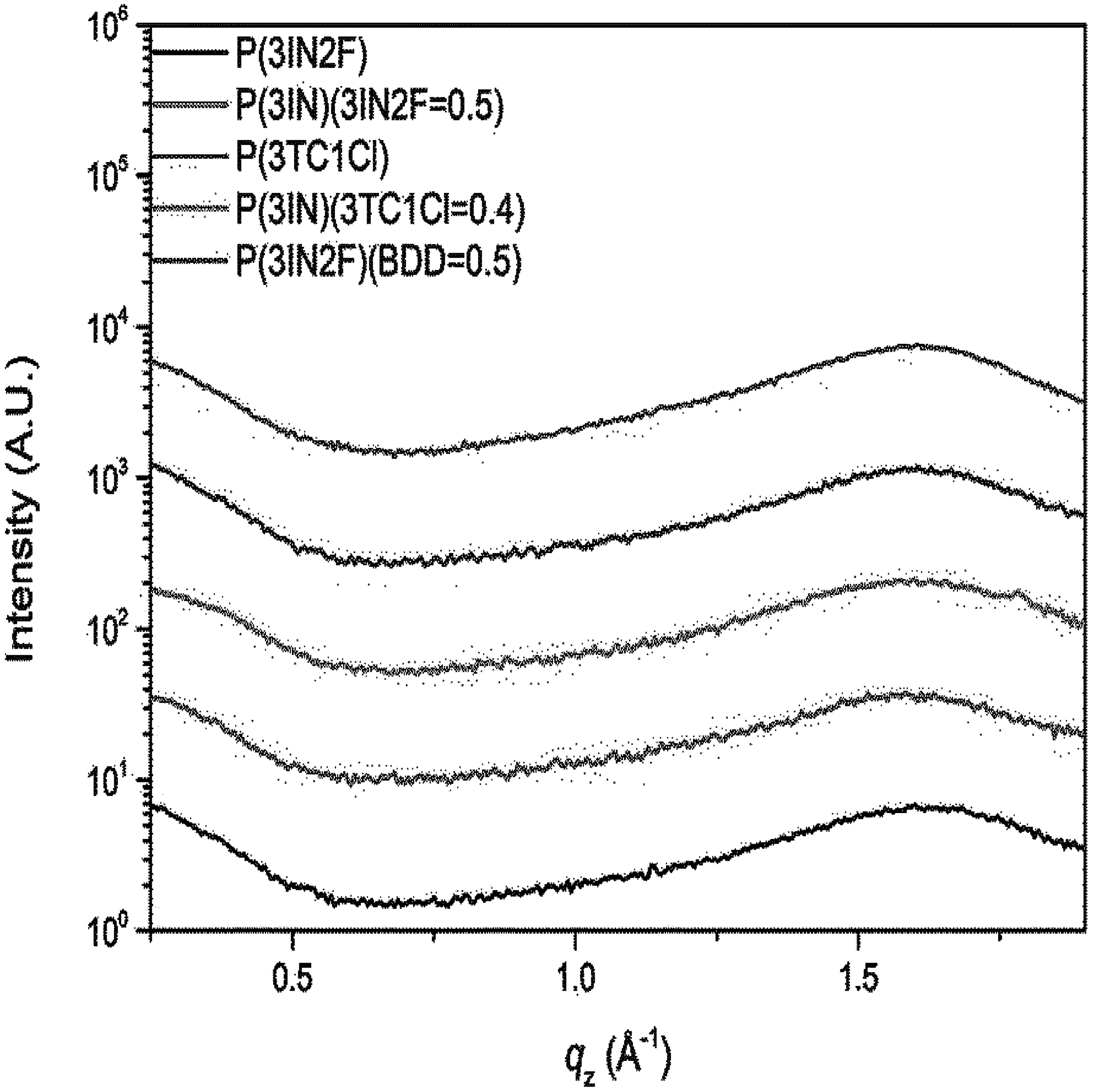
P(3IN2F)
P(3IN)(3IN2F=0.5)
P(3TC1Cl)
P(3IN)(3TC1Cl=0.4)
P(3IN2F)(BDD=0.5)
Intensity (A.U.)
$10^0$
$10^1$
$10^2$
$10^3$
$10^4$
$10^5$
$10^6$
0.5
1.0
1.5
$q_z$ ($Å^{-1}$)

【Fig. 117】

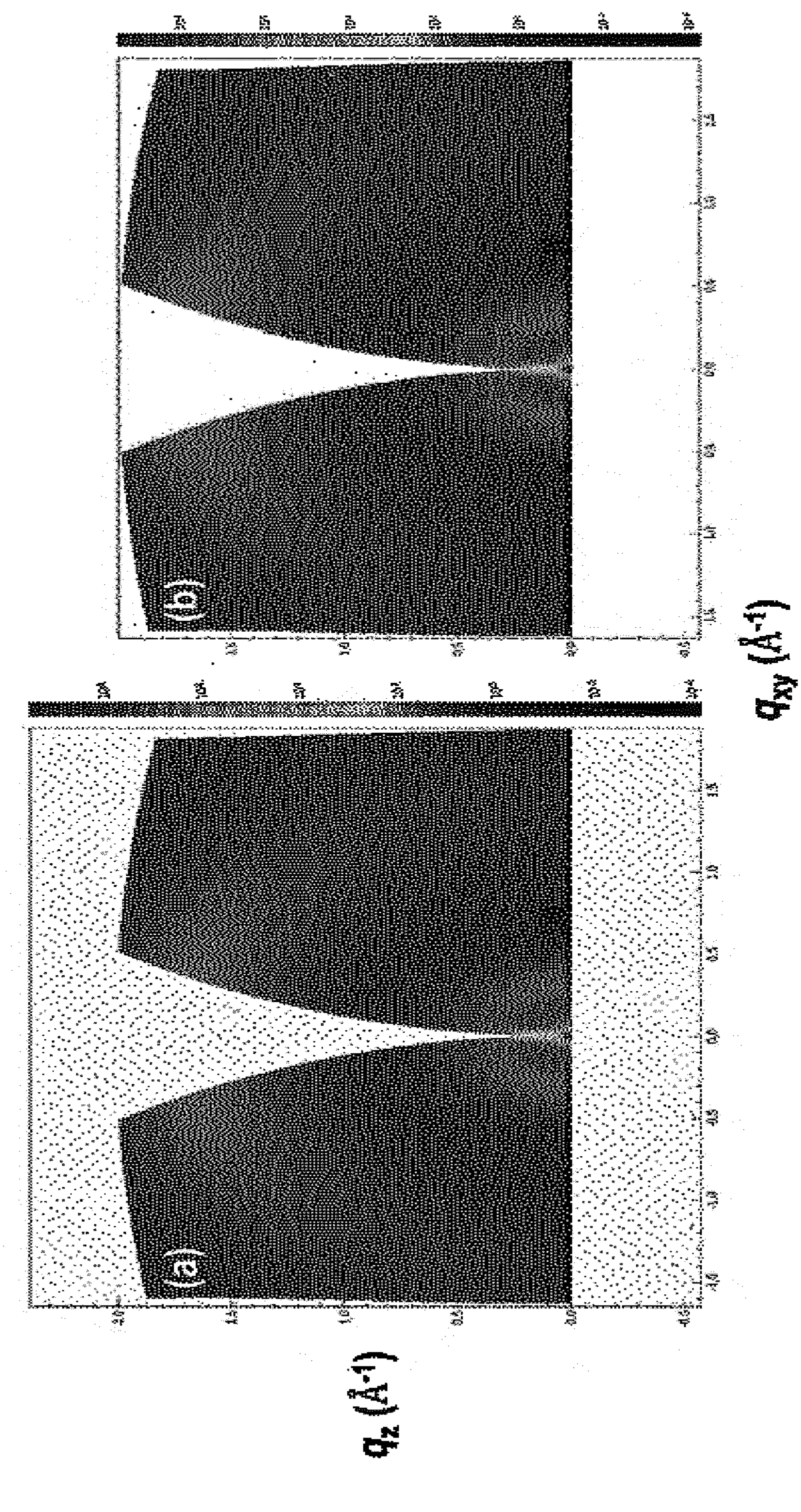

【Fig. 118】

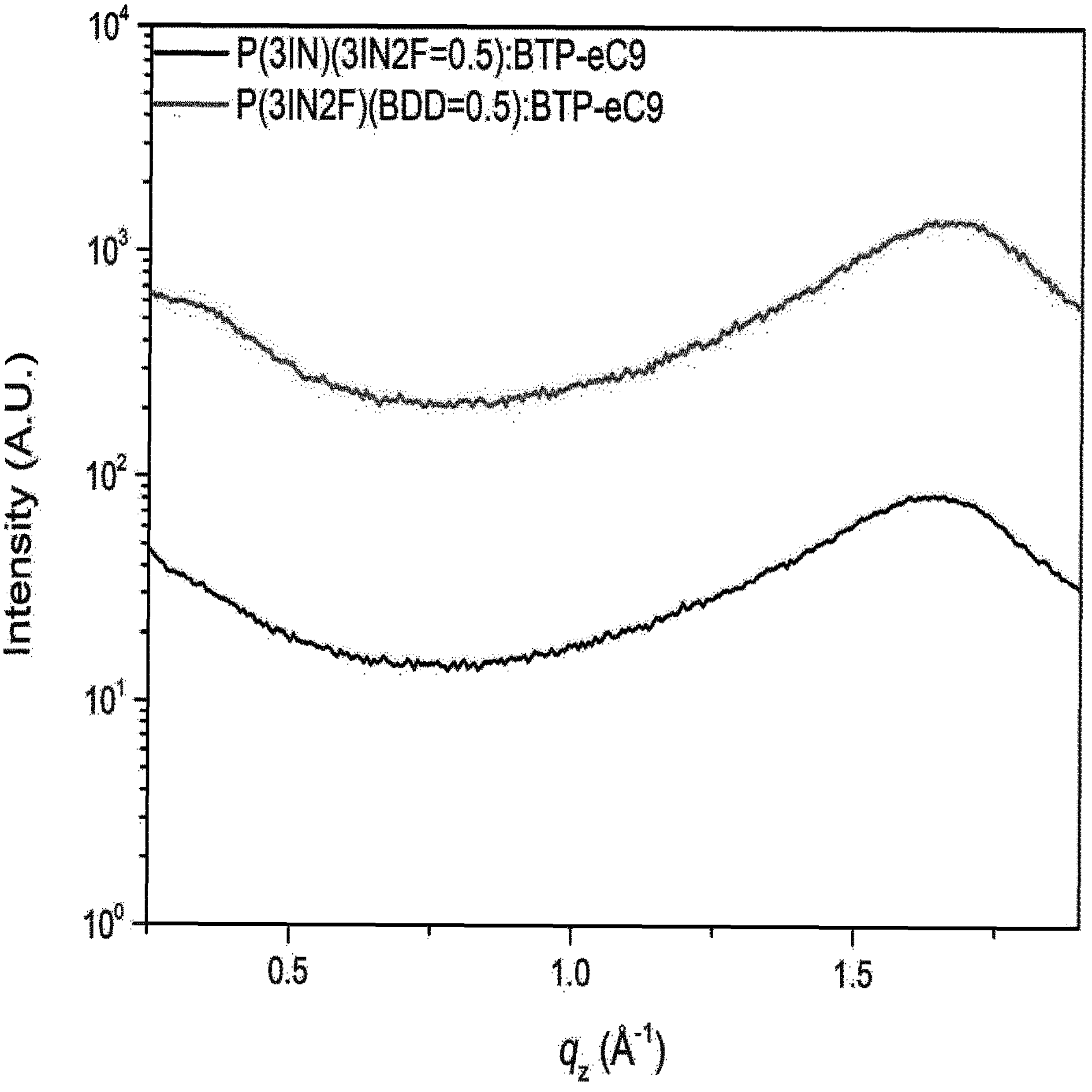
P(3IN)(3IN2F=0.5):BTP-eC9
P(3IN2F)(BDD=0.5):BTP-eC9
Intensity (A.U.)
10^4
10^3
10^2
10^1
10^0
0.5
1.0
1.5
q_z (Å^-1)

【Fig. 119】

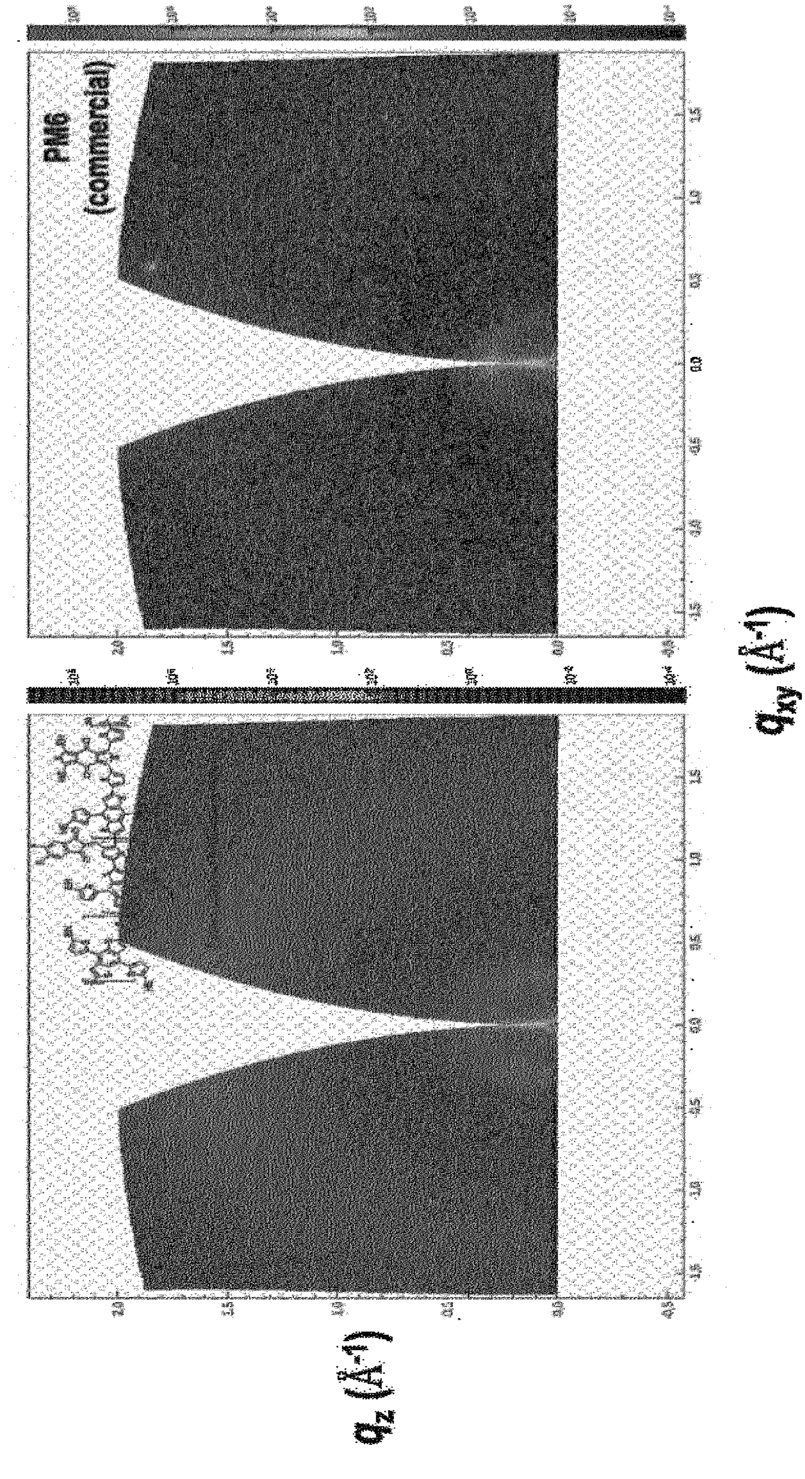

【Fig. 120】

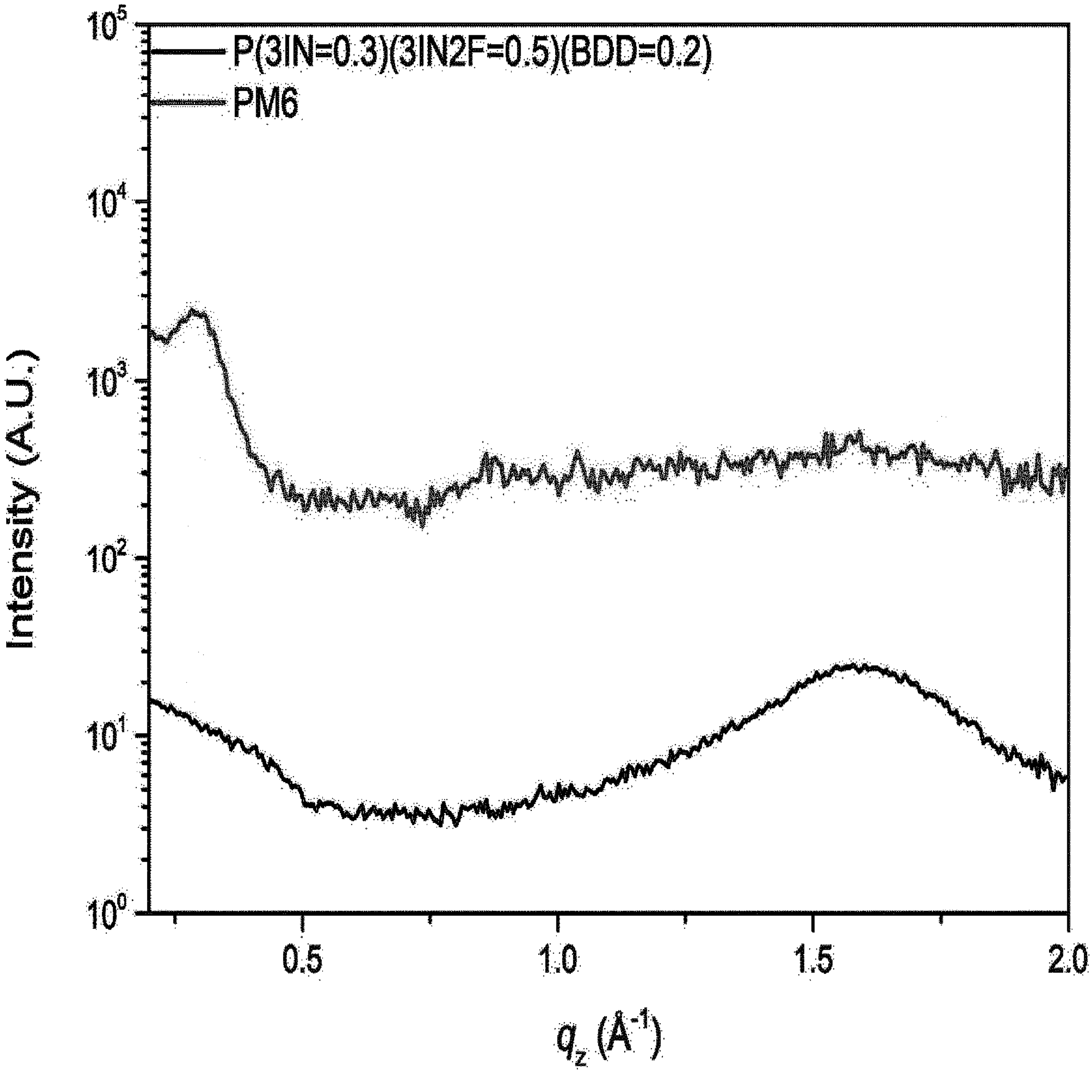
P(3IN=0.3)(3IN2F=0.5)(BDD=0.2)
PM6
Intensity (A.U.)
$10^5$
$10^4$
$10^3$
$10^2$
$10^1$
$10^0$
0.5
1.0
1.5
2.0
$q_z$ (Å$^{-1}$)

【Fig. 121】

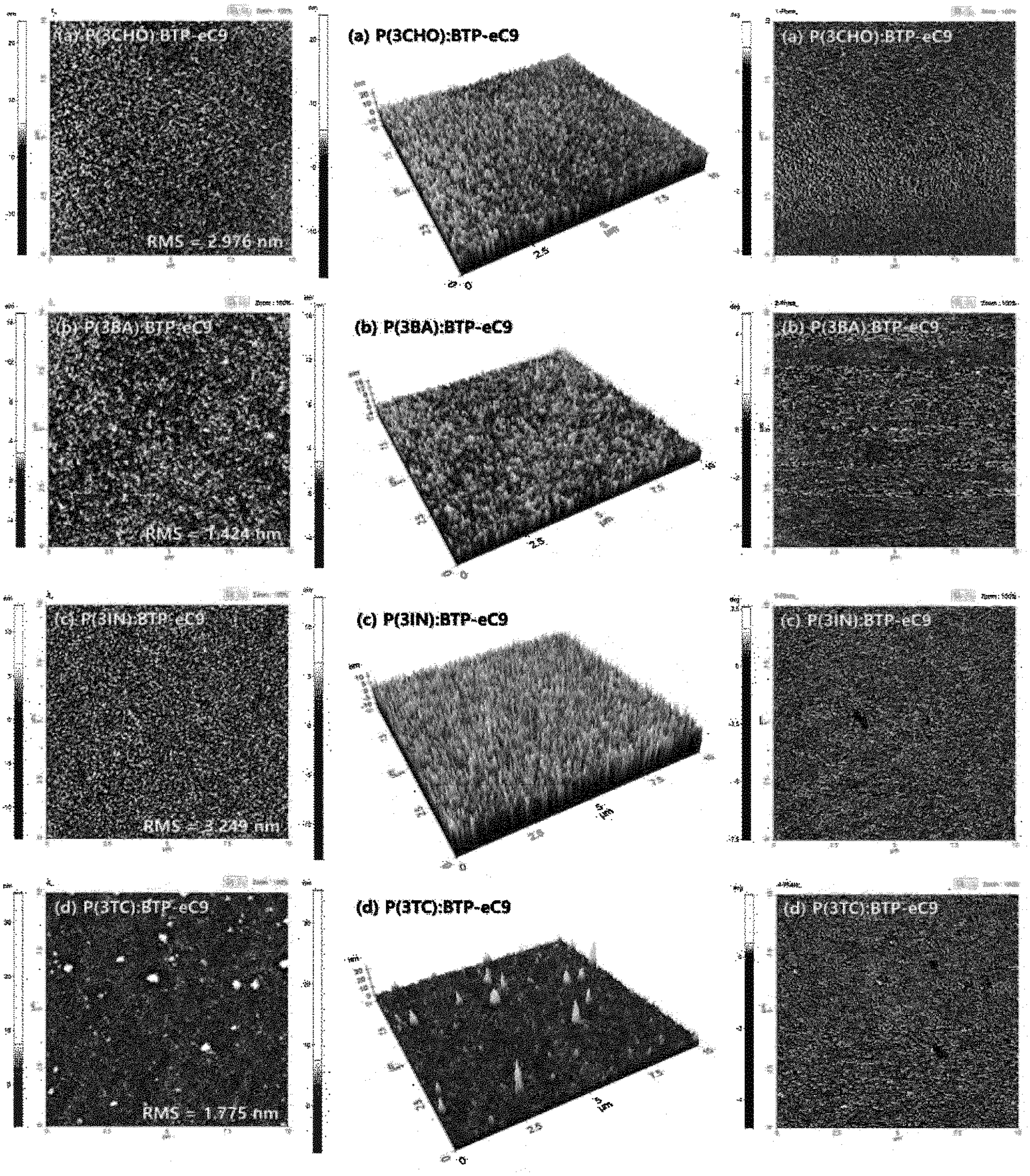
(a) P(3CHO):BTP-eC9
RMS = 2.976 nm
(a) P(3CHO):BTP-eC9
(a) P(3CHO):BTP-eC9
(b) P(3BA):BTP-eC9
RMS = 1.424 nm
(b) P(3BA):BTP-eC9
(b) P(3BA):BTP-eC9
(c) P(3IN):BTP-eC9
RMS = 3.249 nm
(c) P(3IN):BTP-eC9
(c) P(3IN):BTP-eC9
(d) P(3TC):BTP-eC9
RMS = 1.775 nm
(d) P(3TC):BTP-eC9
(d) P(3TC):BTP-eC9

【Fig. 122】

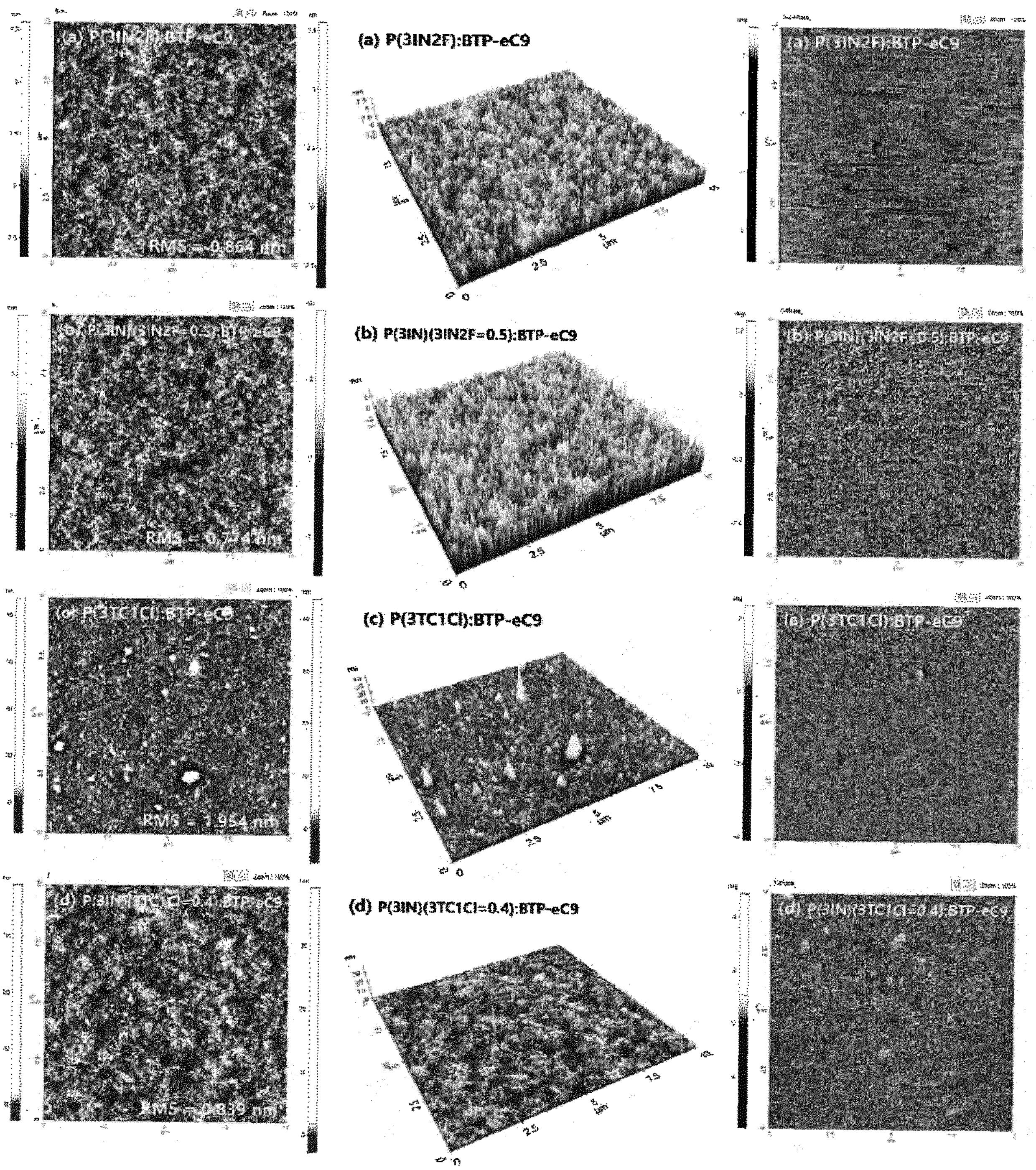
(a) P(3IN2F):BTP-eC9
RMS = 0.864 nm
(a) P(3IN2F):BTP-eC9
(a) P(3IN2F):BTP-eC9
(b) P(3IN)(3IN2F=0.5):BTP-eC9
RMS = 0.774 nm
(b) P(3IN)(3IN2F=0.5):BTP-eC9
(b) P(3IN)(3IN2F=0.5):BTP-eC9
(c) P(3TC1Cl):BTP-eC9
RMS = 1.954 nm
(c) P(3TC1Cl):BTP-eC9
(c) P(3TC1Cl):BTP-eC9
(d) P(3IN)(3TC1Cl=0.4):BTP-eC9
RMS = 0.839 nm
(d) P(3IN)(3TC1Cl=0.4):BTP-eC9
(d) P(3IN)(3TC1Cl=0.4):BTP-eC9

【Fig. 123】

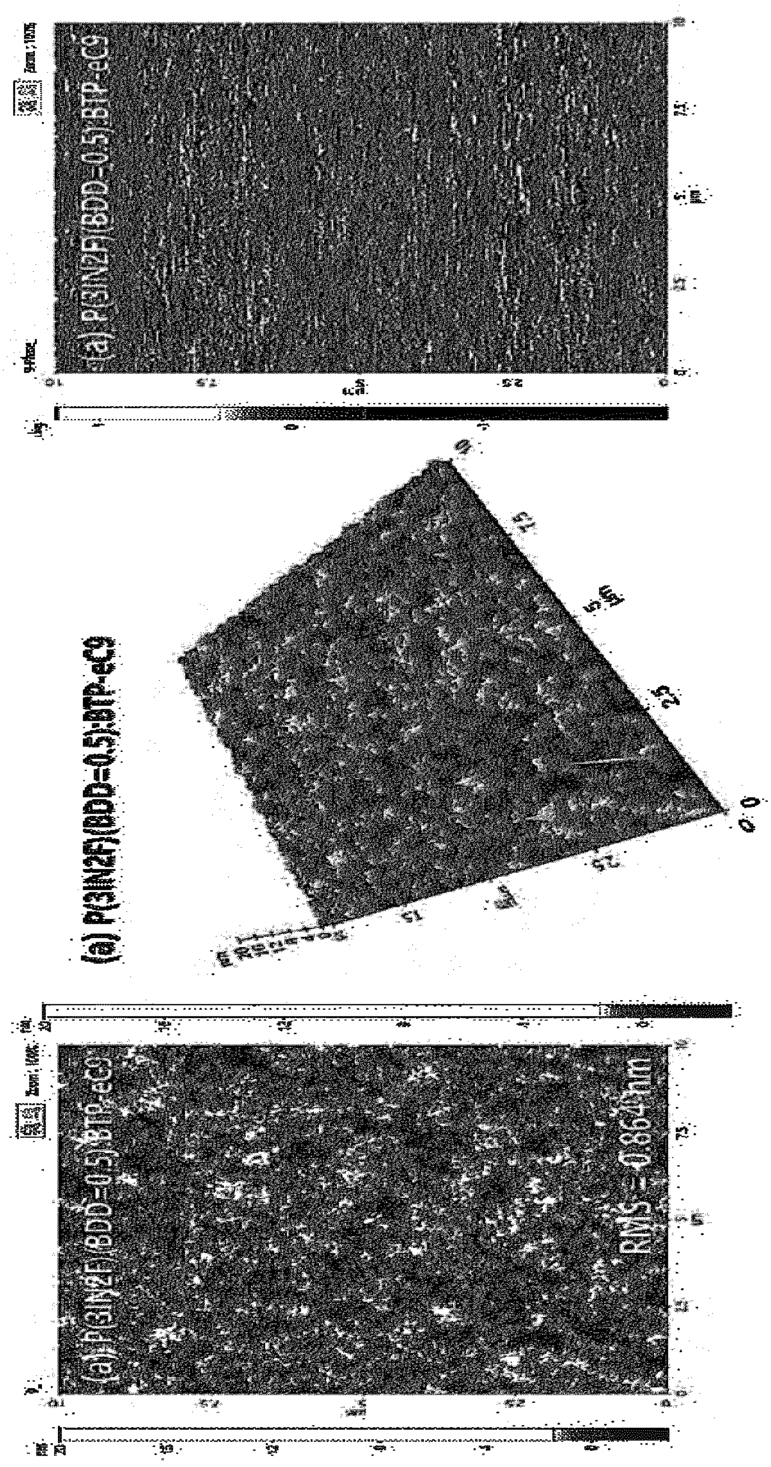
(a) P(3IN2F)(BDD-0.5):BTP-eC9
(a) P(3IN2F)(BDD-0.5):BTP-eC9
(a) P(3IN2F)(BDD-0.5):BTP-eC9
RMS = 0.864nm

【Fig. 124】

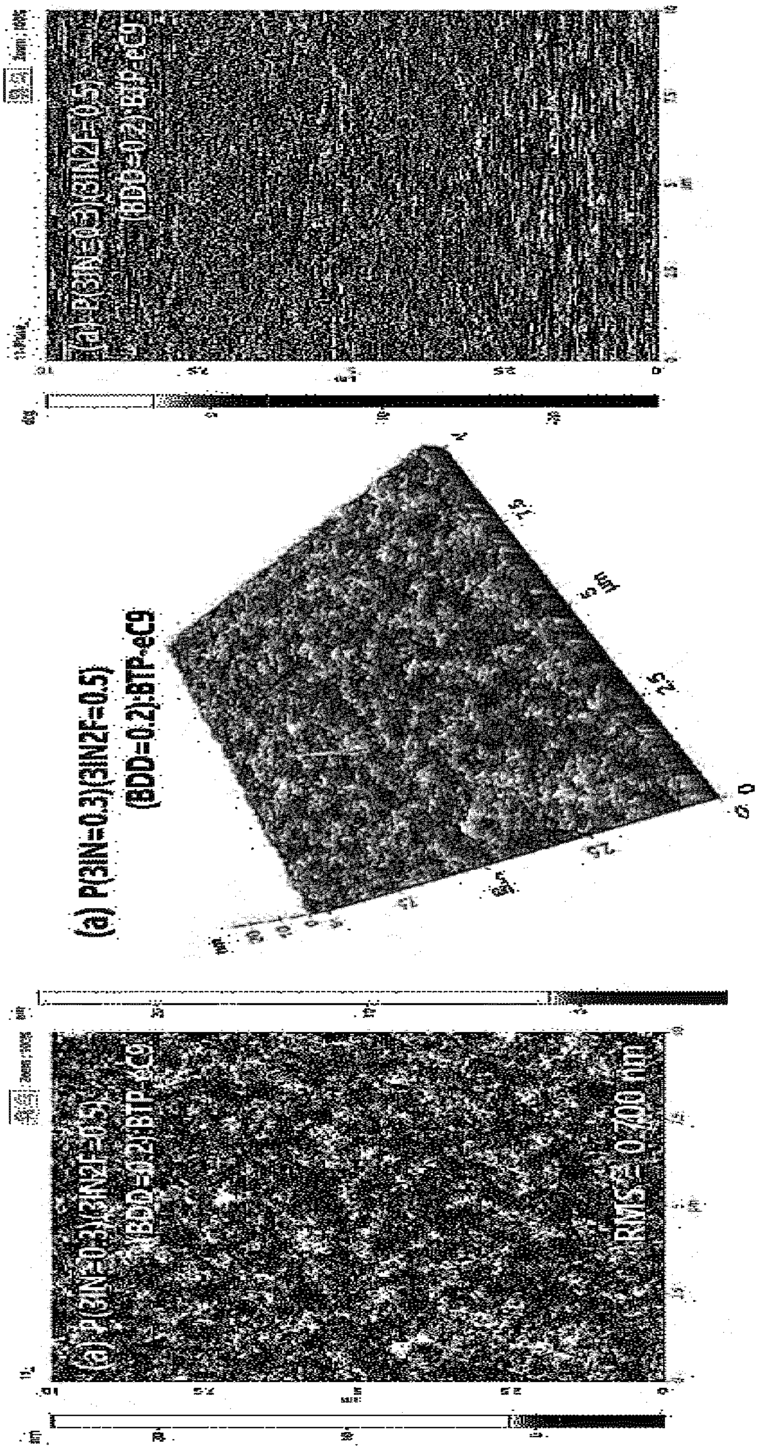

【Fig. 125】

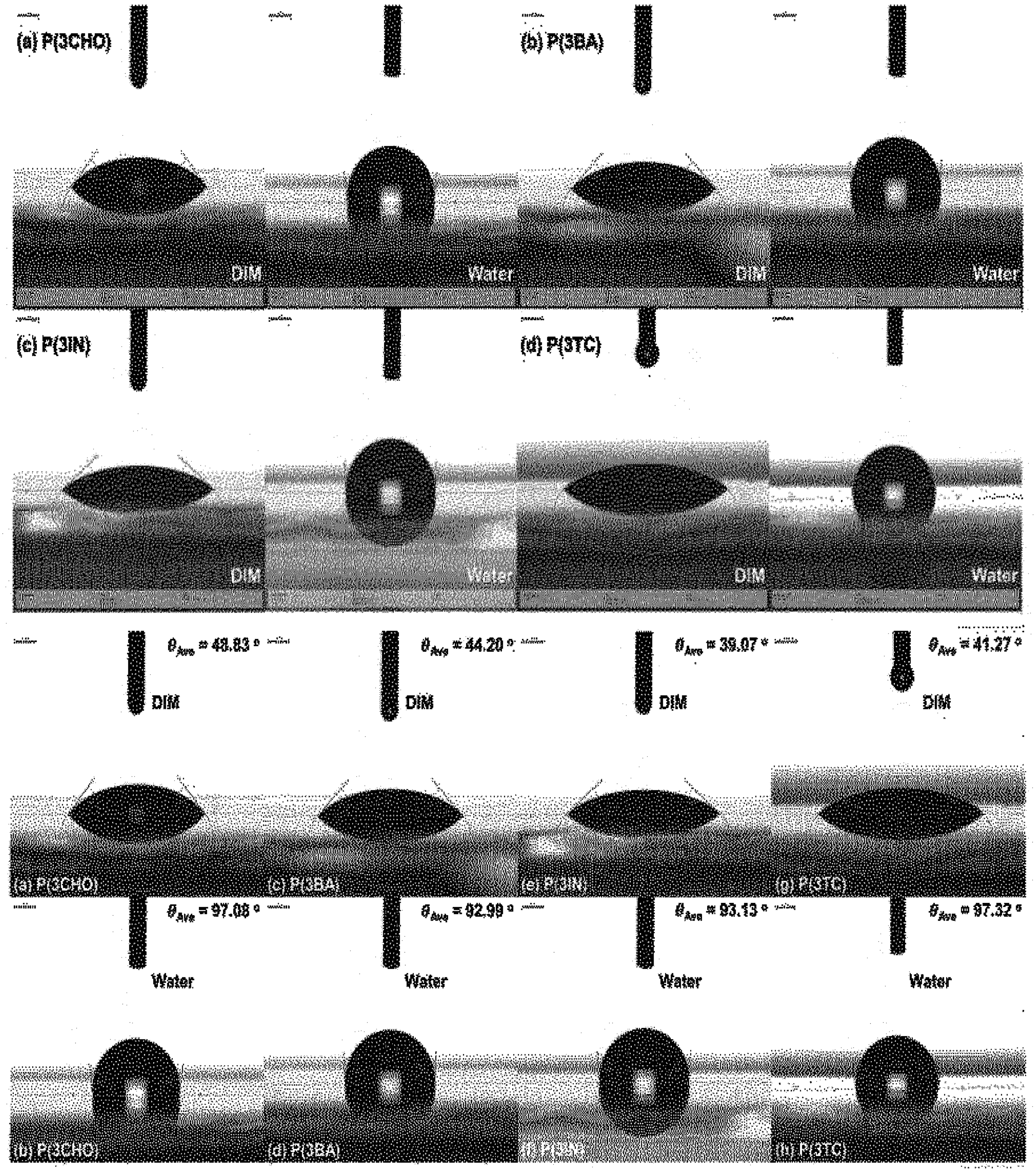

(a) P(3CHO)
DIM
Water
(b) P(3BA)
DIM
Water
(c) P(3IN)
DIM
Water
(d) P(3TC)
DIM
Water
$\theta_{Ave}$ = 48.83 °
DIM
$\theta_{Ave}$ = 44.20 °
DIM
$\theta_{Ave}$ = 39.07 °
DIM
$\theta_{Ave}$ = 41.27 °
DIM
(a) P(3CHO)
(c) P(3BA)
(e) P(3IN)
(g) P(3TC)
$\theta_{Ave}$ = 97.08 °
Water
$\theta_{Ave}$ = 92.99 °
Water
$\theta_{Ave}$ = 93.13 °
Water
$\theta_{Ave}$ = 97.32 °
Water
(b) P(3CHO)
(d) P(3BA)
(f) P(3IN)
(h) P(3TC)

【Fig. 126】

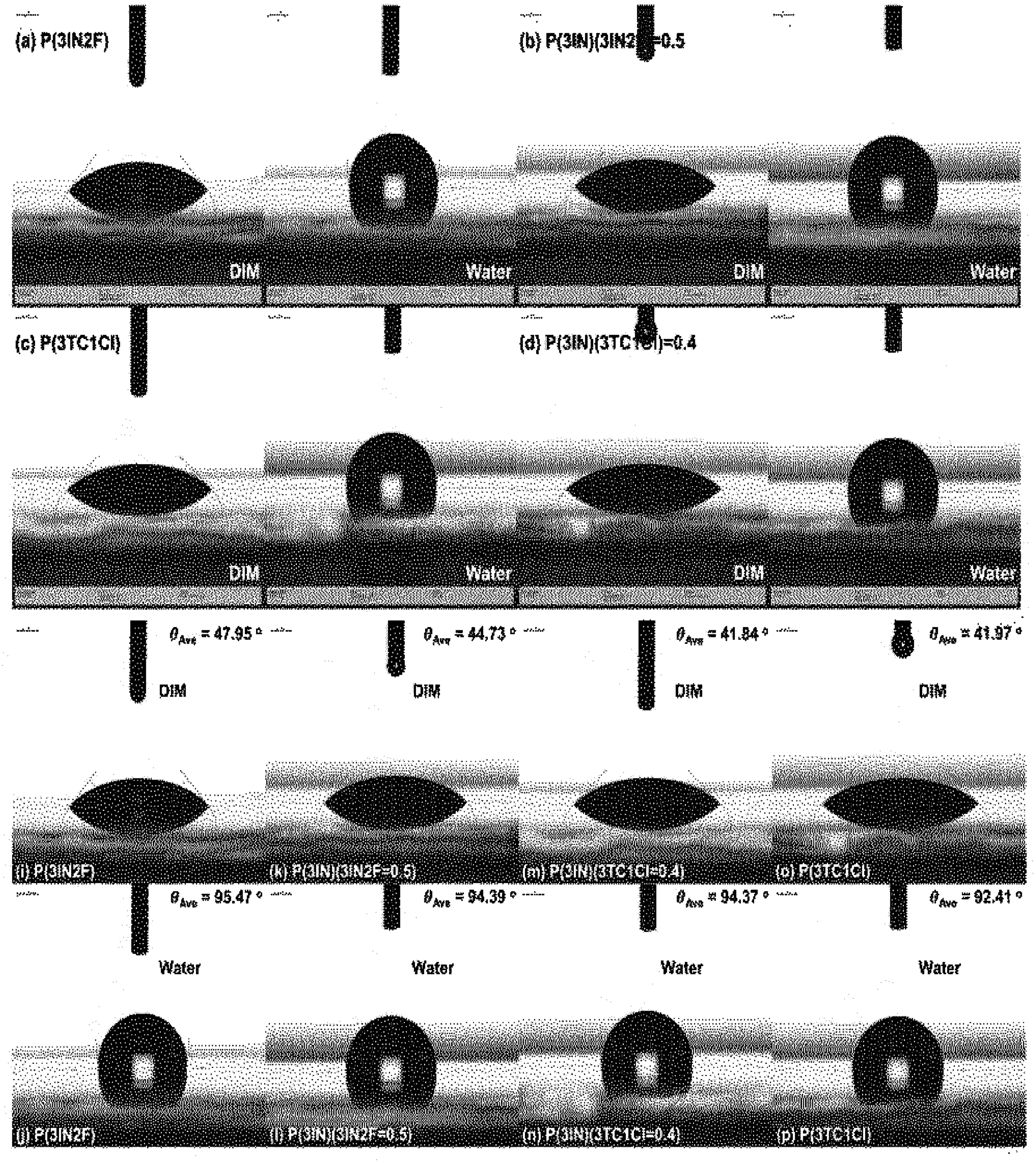
(a) P(3IN2F)
DIM
Water
(b) P(3IN)(3IN2F)=0.5
DIM
Water
(c) P(3TC1Cl)
DIM
Water
(d) P(3IN)(3TC1Cl)=0.4
DIM
Water
$\theta_{Ave}$ = 47.95 °
DIM
$\theta_{Ave}$ = 44.73 °
DIM
$\theta_{Ave}$ = 41.84 °
DIM
$\theta_{Ave}$ = 41.97 °
DIM
(i) P(3IN2F)
(k) P(3IN)(3IN2F=0.5)
(m) P(3IN)(3TC1Cl=0.4)
(o) P(3TC1Cl)
$\theta_{Ave}$ = 95.47 °
Water
$\theta_{Ave}$ = 94.39 °
Water
$\theta_{Ave}$ = 94.37 °
Water
$\theta_{Ave}$ = 92.41 °
Water
(j) P(3IN2F)
(l) P(3IN)(3IN2F=0.5)
(n) P(3IN)(3TC1Cl=0.4)
(p) P(3TC1Cl)

【Fig. 127】

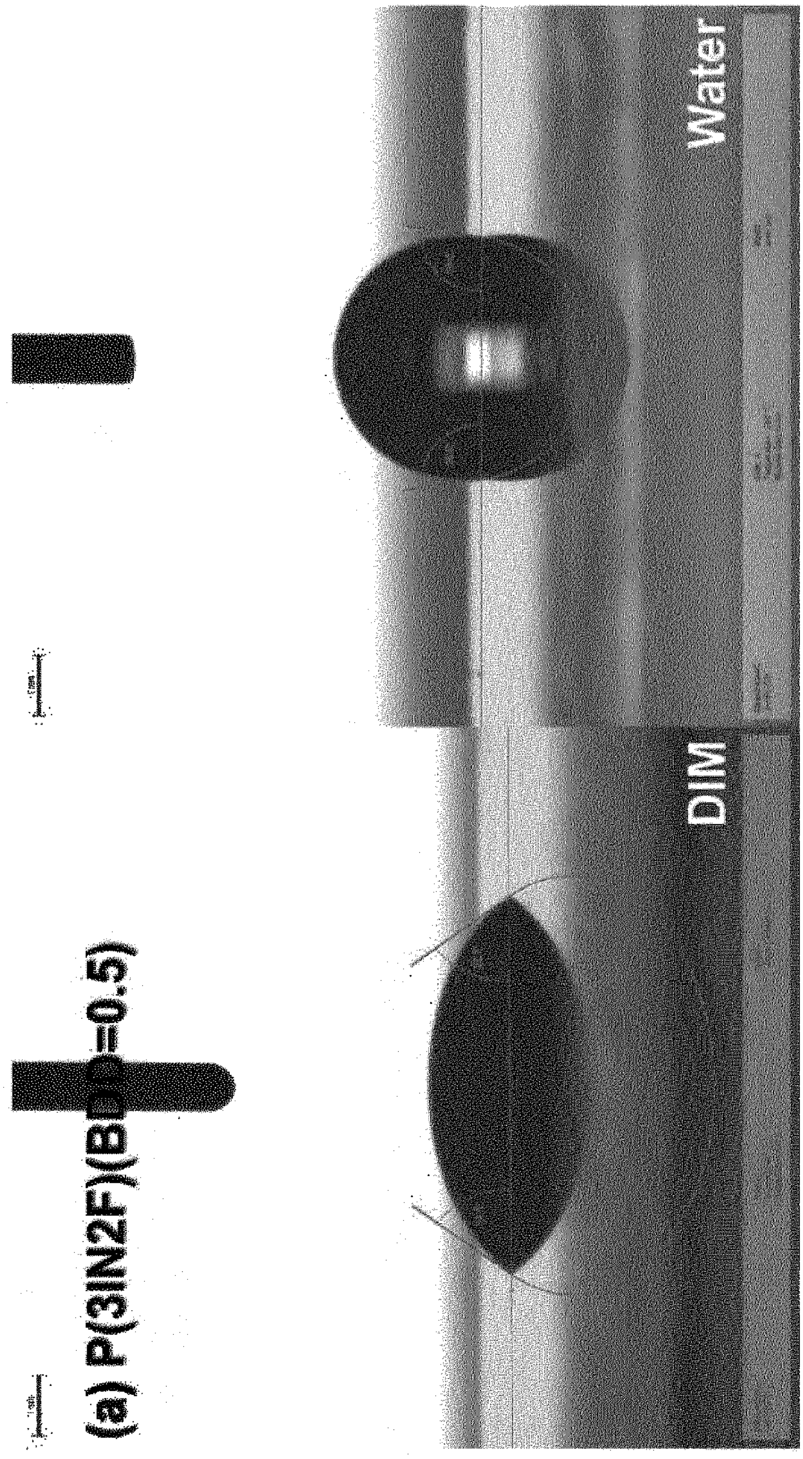

【Fig. 128】

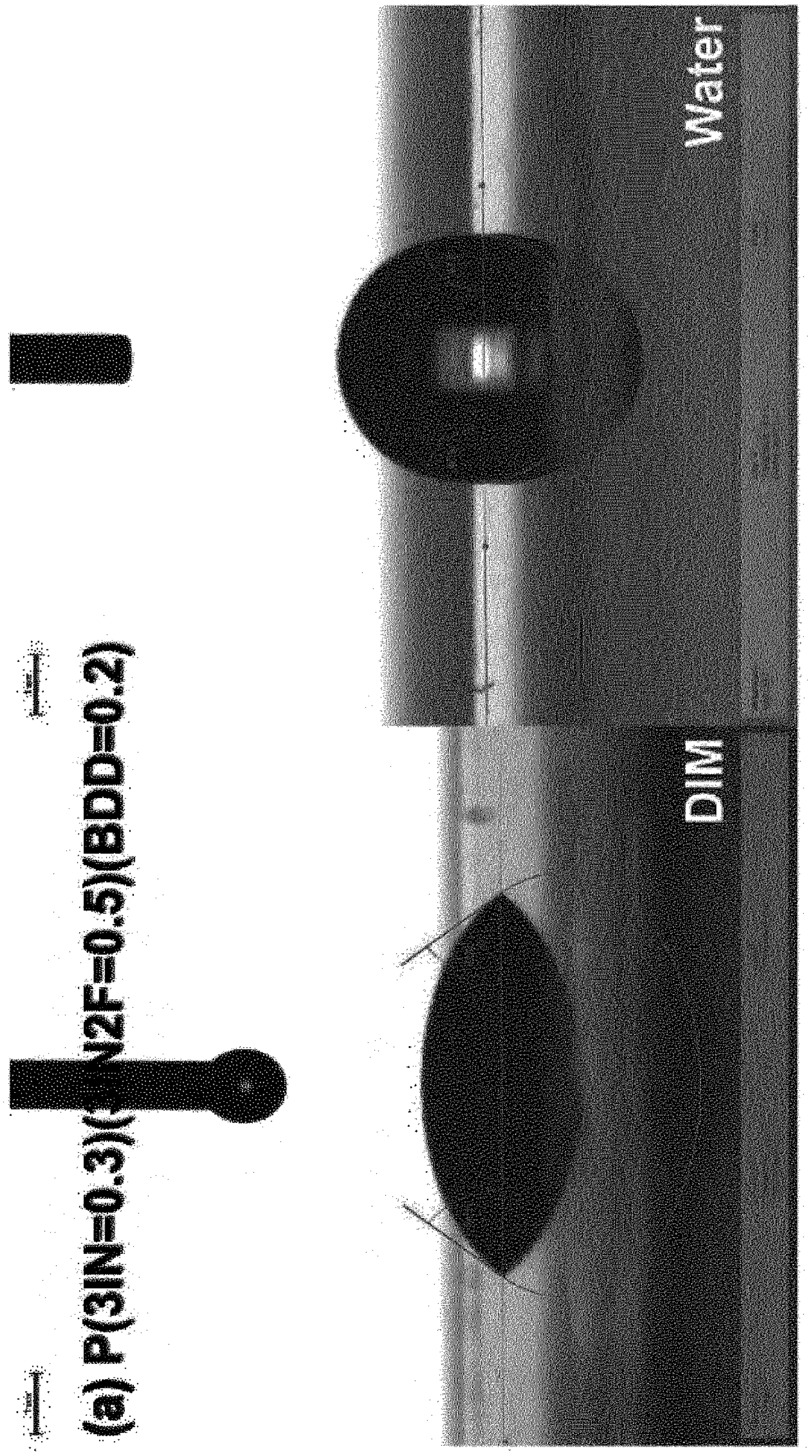

【Fig. 129】

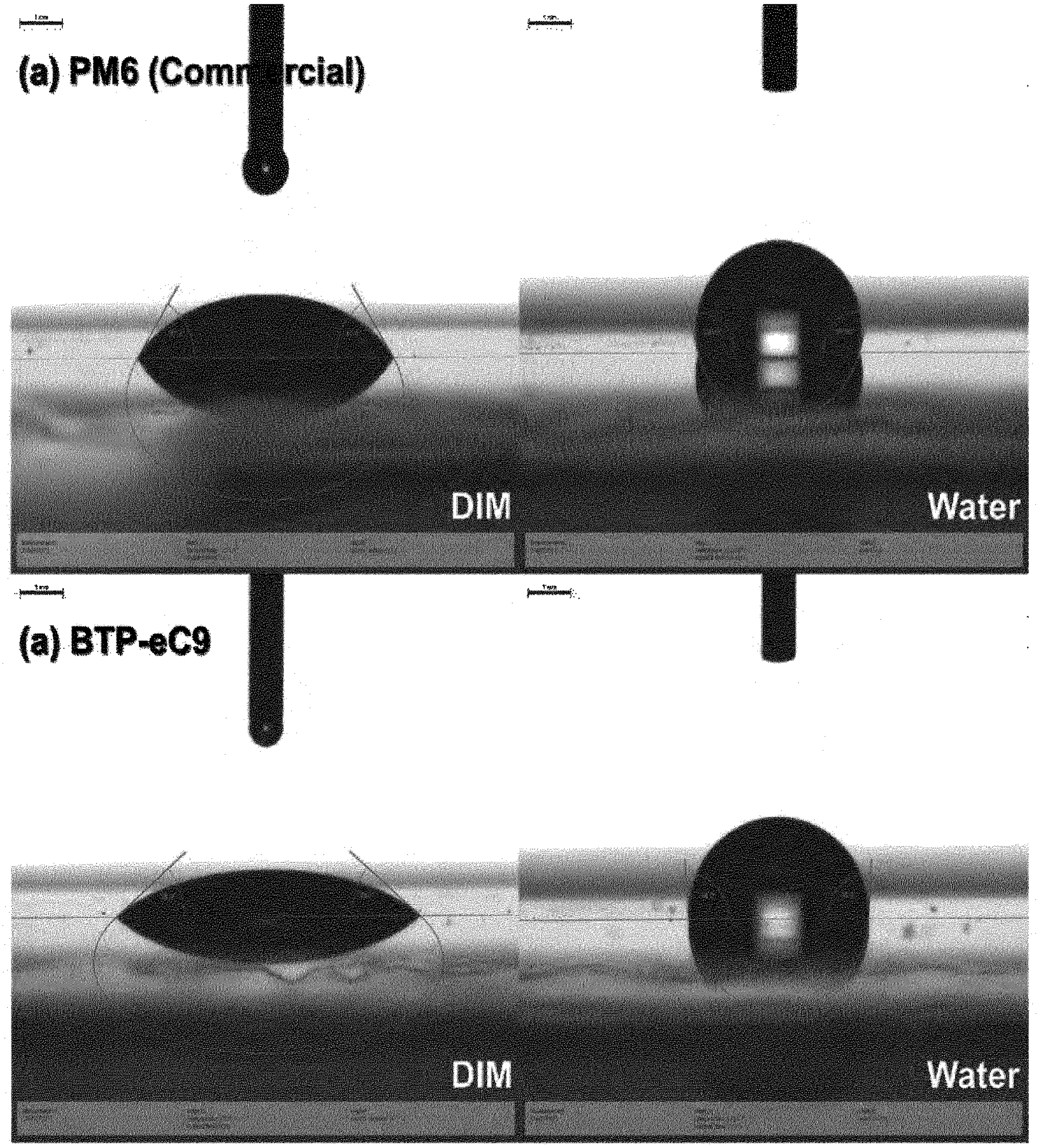

【Fig. 130】

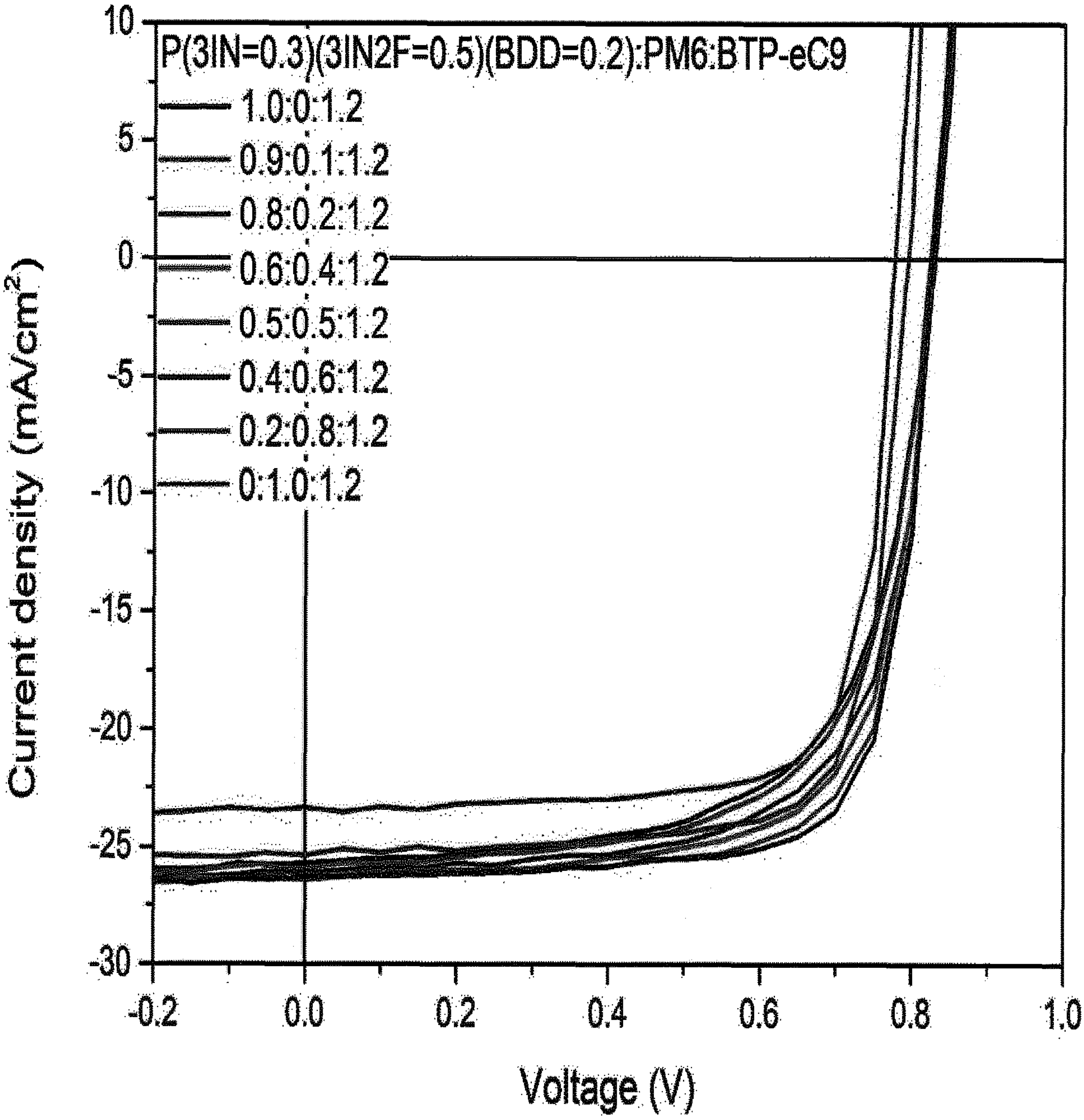
P(3IN=0.3)(3IN2F=0.5)(BDD=0.2):PM6:BTP-eC9
1.0:0:1.2
0.9:0.1:1.2
0.8:0.2:1.2
0.6:0.4:1.2
0.5:0.5:1.2
0.4:0.6:1.2
0.2:0.8:1.2
0:1.0:1.2
Current density (mA/cm²)
Voltage (V)
10
5
0
-5
-10
-15
-20
-25
-30
-0.2
0.0
0.2
0.4
0.6
0.8
1.0

【Fig. 131】

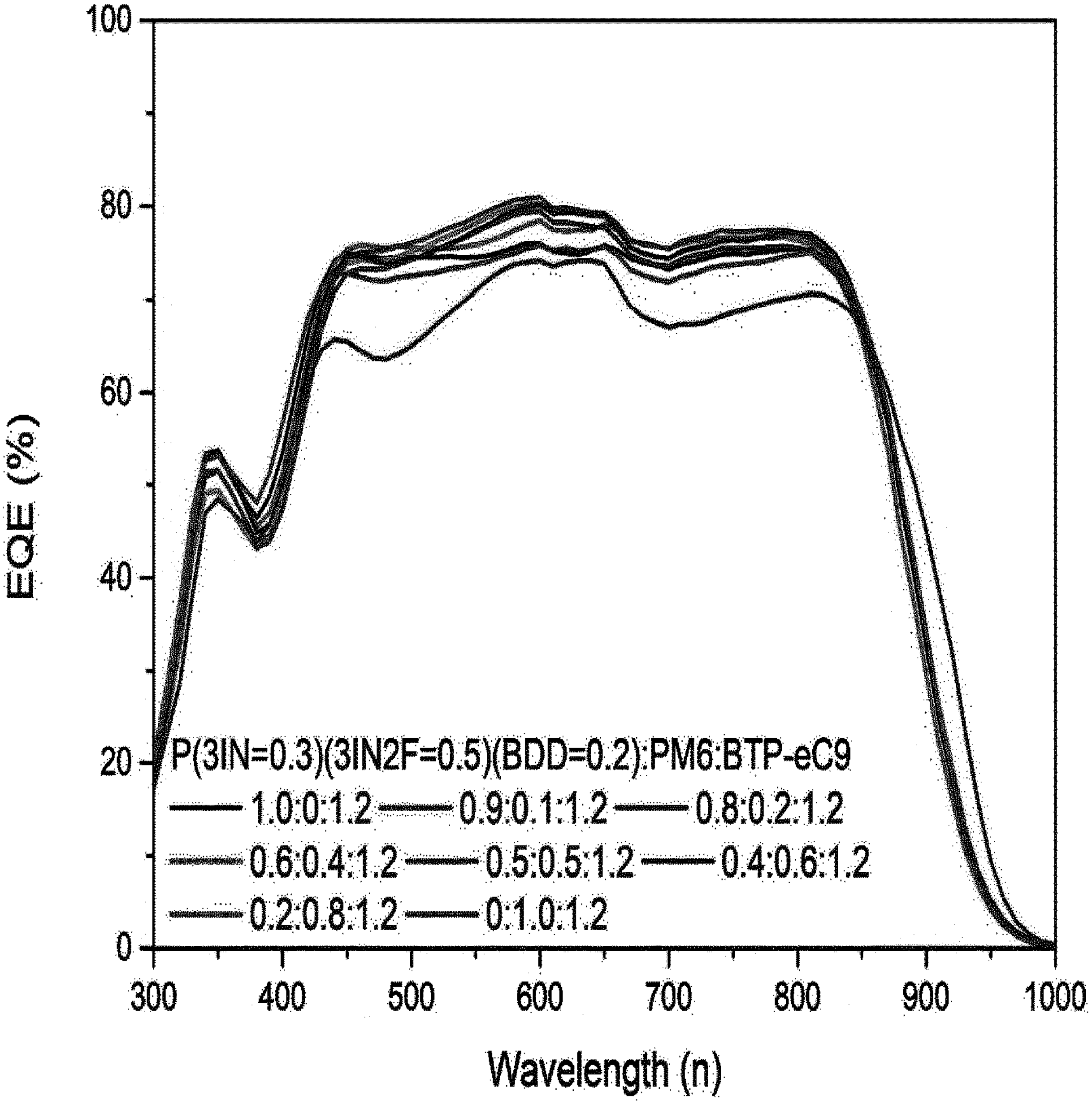
100
80
60
40
20
0
EQE (%)
300
400
500
600
700
800
900
1000
Wavelength (n)
P(3IN=0.3)(3IN2F=0.5)(BDD=0.2):PM6:BTP-eC9
1.0:0:1.2
0.9:0.1:1.2
0.8:0.2:1.2
0.6:0.4:1.2
0.5:0.5:1.2
0.4:0.6:1.2
0.2:0.8:1.2
0:1.0:1.2

【Fig. 132a】

DAEGU TECHNOPARK
Nano Convergence Practical Application Center
46-17, Seongseogongdan-ro, Dalseo-gu, Daegu, Korea
(Tel: +82 53 602 1860 Fax: +82 53 602 1872)
www.npac.or.kr

# TEST REPORT

Report No. : TP-NC-22S-0396

Page( 1 )/( 6 )Pages

1. Client
- Name : KONKUK University
- Address : 120 Neungdong-ro, Gwangjin-gu, Seoul, KOREA
- Tel : 010-9024-7073 (e-mail) yblhs@naver.com

2. Sample Description : KU8-2

3. Date of Test : 08.03.2022

4. Test method used : Refer to attachment

5. Test Results :

| |
|---|
| - Test equipment : Solar Cell Test System (Sun simulator-1kW) |
| - Manufacturer : Newport Stratford |
| - Model : #91192 1KW |
| - Attachment : report on test result |

☐ The results shown in this test report refer only to the sample(s) tested unless otherwise stated.
☐ This report is not related to KS Q ISO and KOLAS.

| Affirmation | Tested by<br>Name : Minji Kim (Signature) | Technical Manager<br>Name : kyeong su Jeon (Signature) |
|---|---|---|

08. 03. 2022

Daegu Technopark Nano Convergence Practical Application Center (signature)

Check Certificate of Authenticity : +82 53 602 1860

NPAC-QP-15-T-01(01) (재)대구테크노파크 나노융합실용화센터 A4(210 × 297)

【Fig. 132b】

DAEGU TECHNOPARK
Nano Convergence Practical Application Center
46-17, Seongseogongdan-ro, Dalseo-gu, Daegu, Korea
(Tel: +82 53 602 1860 Fax: +82 53 602 1872)
www.npac.or.kr

# TEST REPORT

Report No. : TP-NC-22S-0396

Page( 2 )/( 6 )Pages

복사본 COPY

- Test equipment

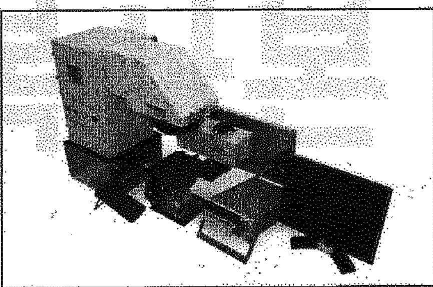

Solar Cell Test System(#91192 1KW)

- Test result
- Sample area : 0.04 ㎠

| | Sample name | $V_{oc}(V)$ | $I_{sc}$(mA) | $J_{sc}$(mA/㎠) | F.F(%) | $I_{max}$(mA) | $V_{max}(V)$ | $P_{max}$(㎽) | Eff.(%) |
|---|---|---|---|---|---|---|---|---|---|
| Test result | KU8-2-1 | 0.828 | 1.068 | 26.709 | 74.885 | 0.941 | 0.704 | 0.663 | 16.564 |
| | KU8-2-2 | 0.830 | 1.068 | 26.707 | 74.430 | 0.950 | 0.695 | 0.660 | 16.506 |
| | KU8-2-3 | 0.830 | 1.066 | 26.645 | 74.678 | 0.951 | 0.695 | 0.661 | 16.516 |
| | KU8-2-4 | 0.829 | 1.059 | 26.467 | 74.282 | 0.938 | 0.695 | 0.652 | 16.290 |
| | Aaverage Efficiency (%) | | | | | | | | 16.469 |

복사본 COPY

Check Certificate of Authenticity : +82 53 602 1860

【Fig. 132c】

DAEGU TECHNOPARK
Nano Convergence Practical Application Center
46-17, Seongseogongdan-ro, Dalseo-gu, Daegu, Korea
(Tel: +82 53 602 1860 Fax: +82 53 602 1872)
www.npac.or.kr

# TEST REPORT

Report No. : TP-NC-22S-0396
Page( 3 )/( 6 )Pages

- KU8-2-1 : Area 0.04 ㎠

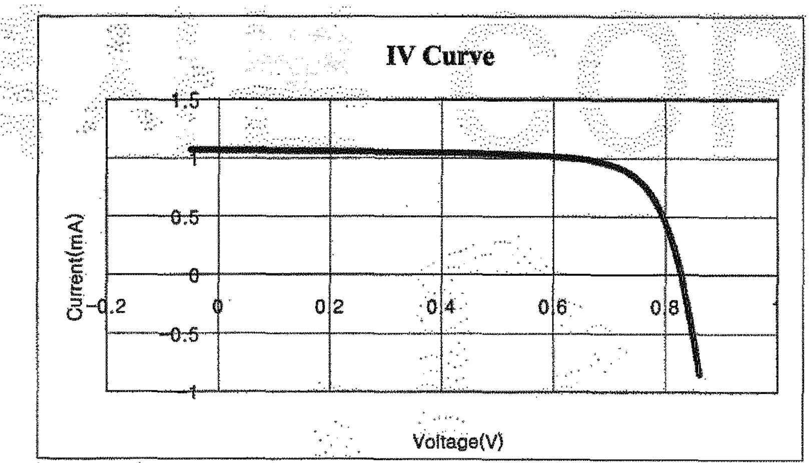


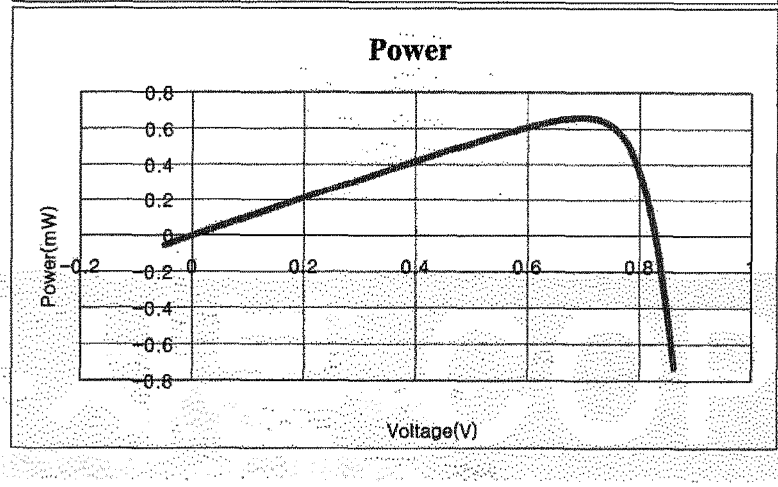


Check Certificate of Authenticity : +82 53 602 1860

NPAC-QP-15-T-02(00) (재)대구테크노파크 나노융합실용화센터 A4(210 × 297)

[redacted]

【Fig. 132d】

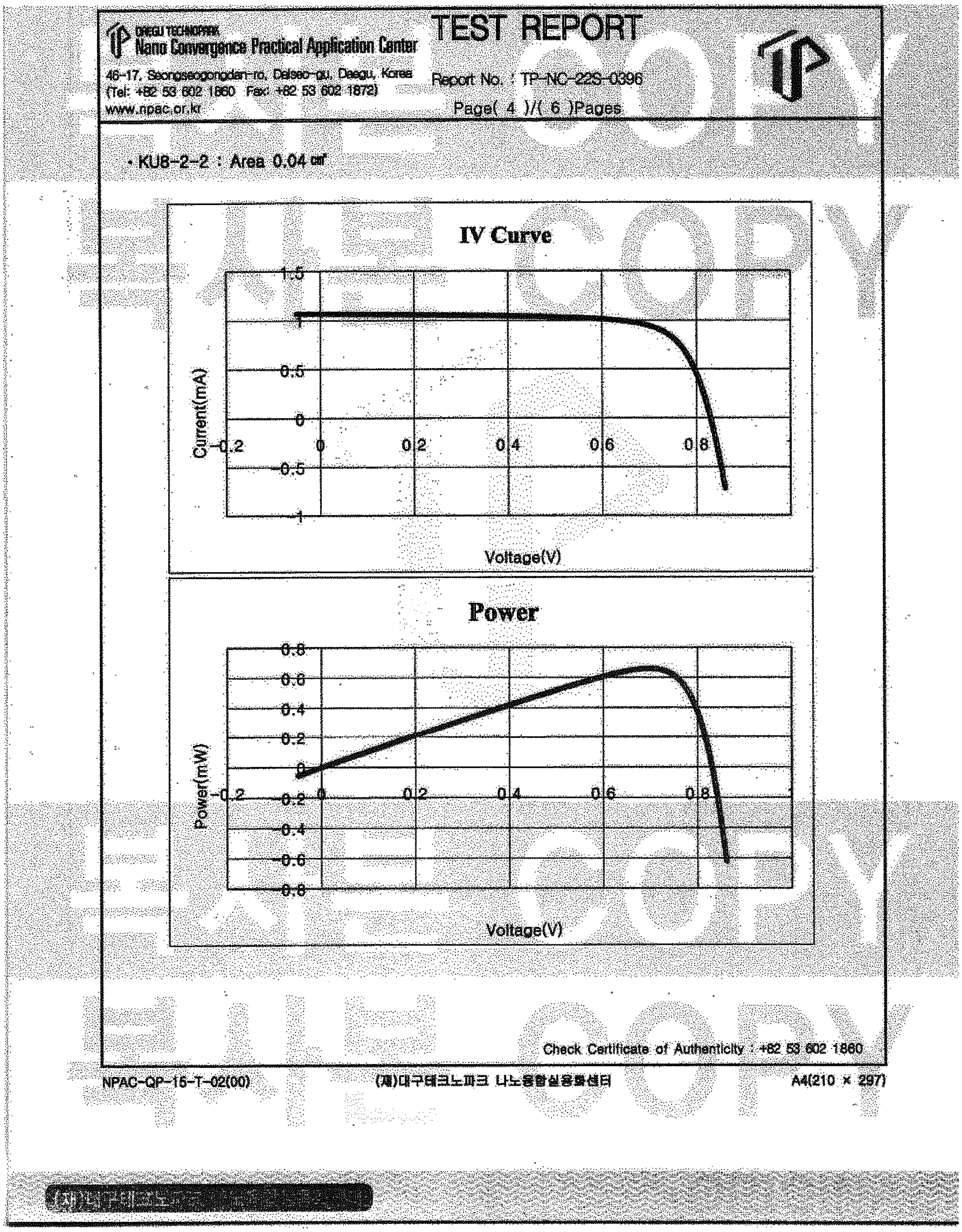

DAEGU TECHNOPARK
Nano Convergence Practical Application Center
46-17, Seongseogongdan-ro, Dalseo-gu, Daegu, Korea
(Tel: +82 53 602 1860 Fax: +82 53 602 1872)
www.npac.or.kr

TEST REPORT

Report No. : TP-NC-22S-0396

Page( 4 )/( 6 )Pages

· KU8-2-2 : Area 0.04 ㎠



Check Certificate of Authenticity : +82 53 602 1860

NPAC-QP-15-T-02(00) (재)대구테크노파크 나노융합실용화센터 A4(210 × 297)

복사본 COPY

【Fig. 132e】

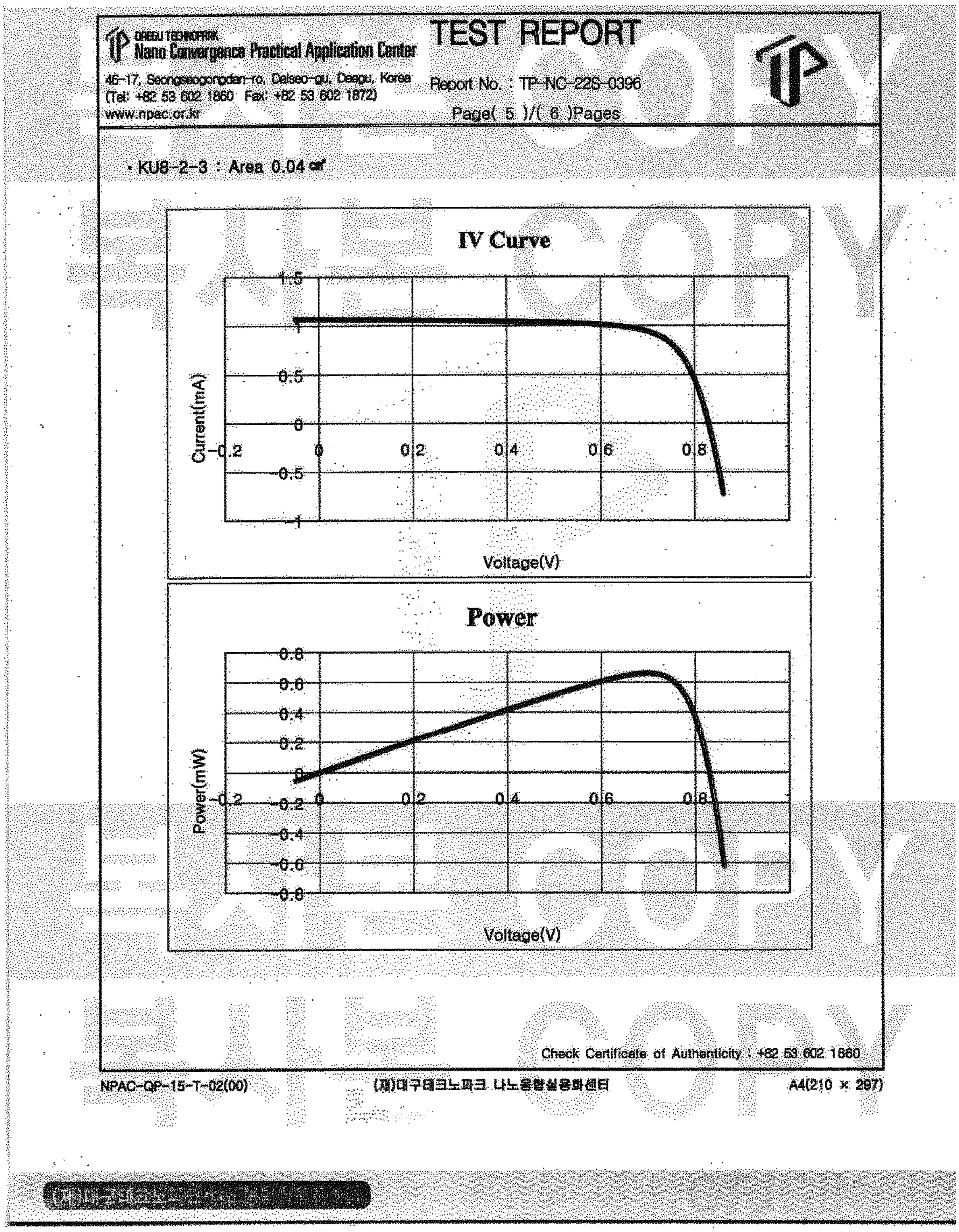
DAEGU TECHNOPARK
Nano Convergence Practical Application Center
46-17, Seongseogongdan-ro, Dalseo-gu, Daegu, Korea
(Tel: +82 53 602 1860 Fax: +82 53 602 1872)
www.npac.or.kr
TEST REPORT
Report No. : TP-NC-22S-0396
Page( 5 )/( 6 )Pages
· KU8-2-3 : Area 0.04 ㎠
IV Curve
Current(mA)
Voltage(V)
1.5
1
0.5
0
-0.5
-1
-0.2
0
0.2
0.4
0.6
0.8
Power
Power(mW)
Voltage(V)
0.8
0.6
0.4
0.2
0
-0.2
-0.4
-0.6
-0.8
Check Certificate of Authenticity : +82 53 602 1860
NPAC-QP-15-T-02(00)
(재)대구테크노파크 나노융합실용화센터
A4(210 × 297)
복사본 COPY

【Fig. 132f】

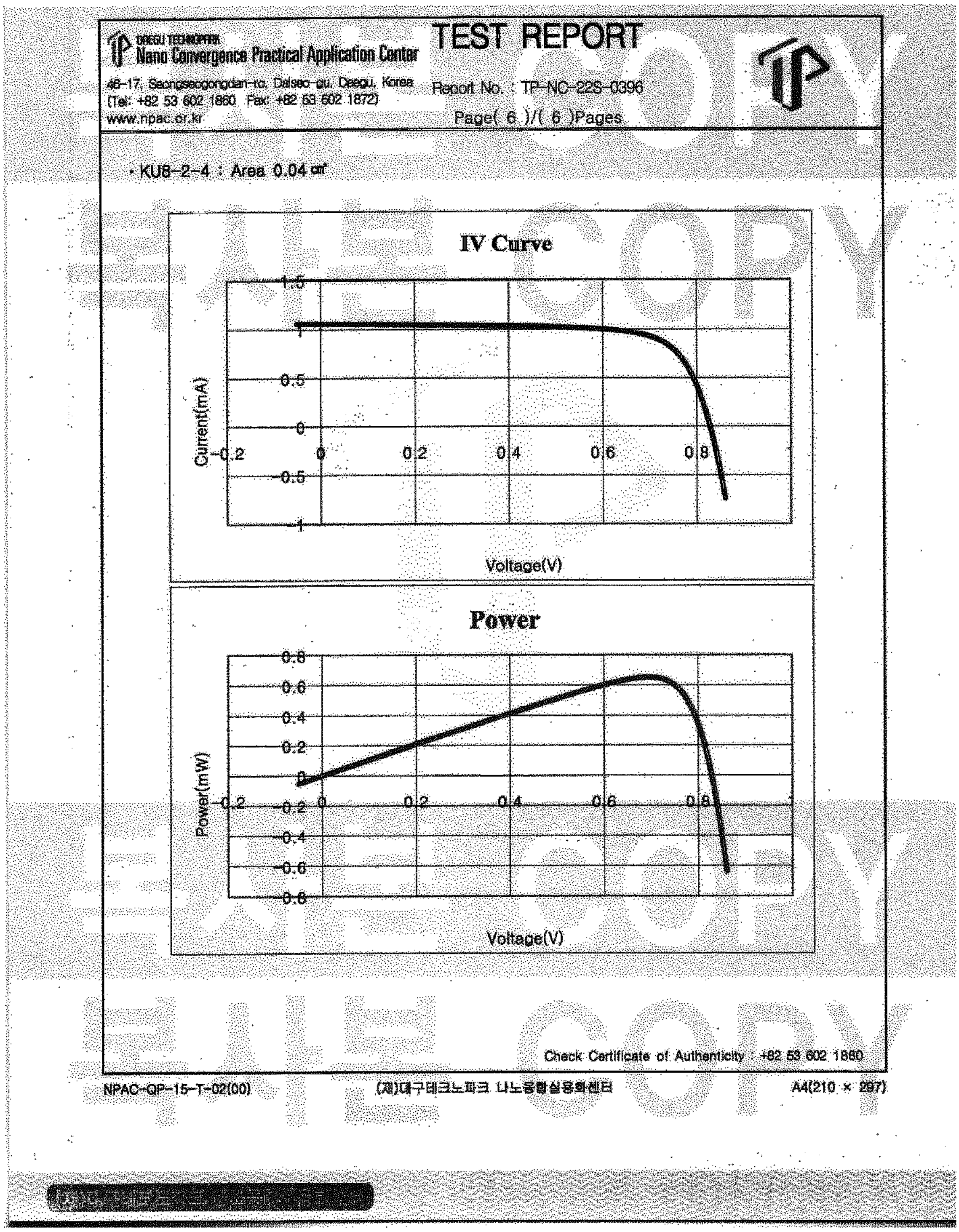
DAEGU TECHNOPARK
Nano Convergence Practical Application Center
46-17, Seongseogongdan-ro, Dalseo-gu, Daegu, Korea
(Tel: +82 53 602 1860 Fax: +82 53 602 1872)
www.npac.or.kr

TEST REPORT

Report No. : TP-NC-22S-0396

Page( 6 )/( 6 )Pages

· KU8-2-4 : Area 0.04 ㎠



Check Certificate of Authenticity : +82 53 602 1860

NPAC-QP-15-T-02(00) (재)대구테크노파크 나노융합실용화센터 A4(210 × 297)

【Fig. 133】

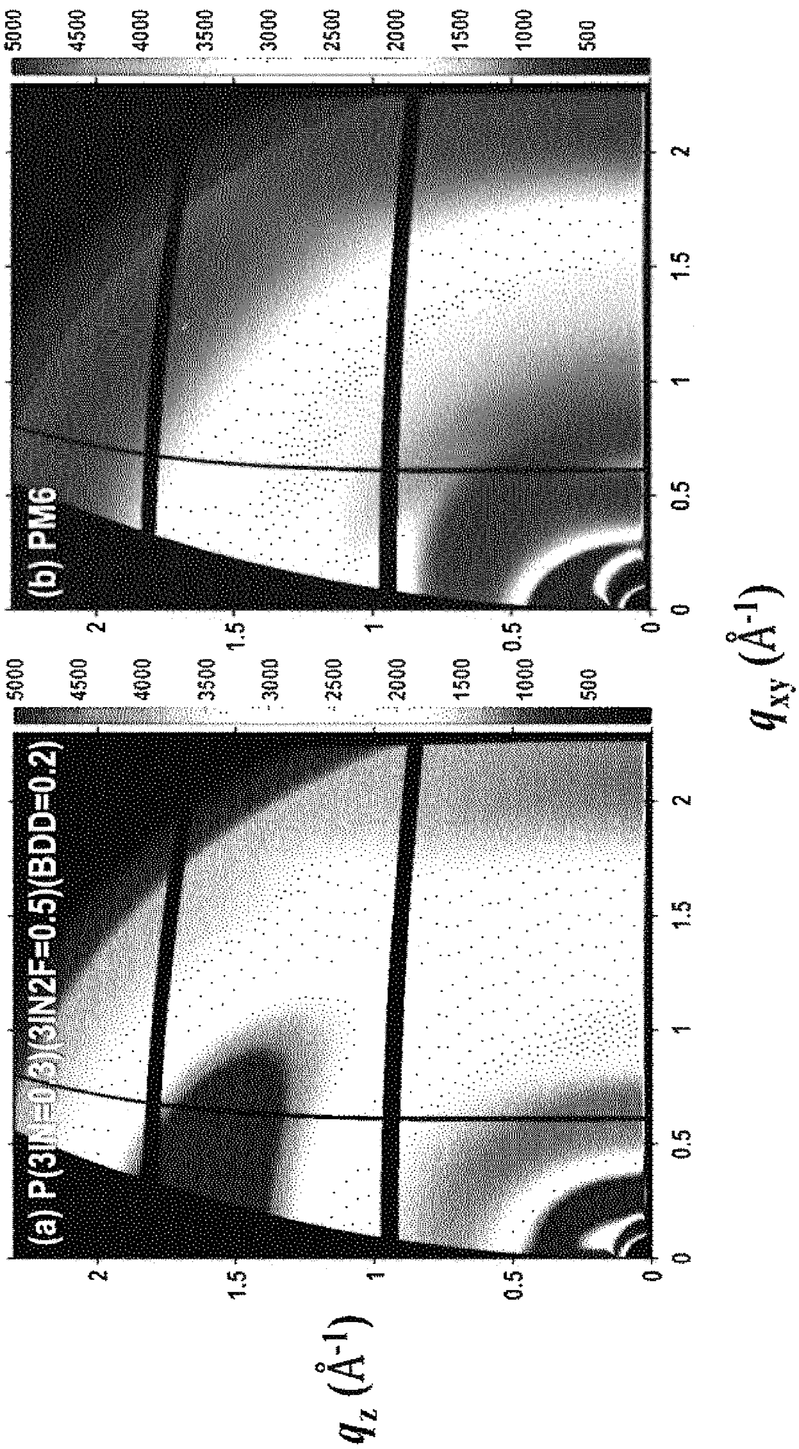
(b) PM6
5000
4500
4000
3500
3000
2500
2000
1500
1000
500
0
0.5
1
1.5
2
$q_z$ (Å$^{-1}$)
$q_{xy}$ (Å$^{-1}$)

【Fig. 134】

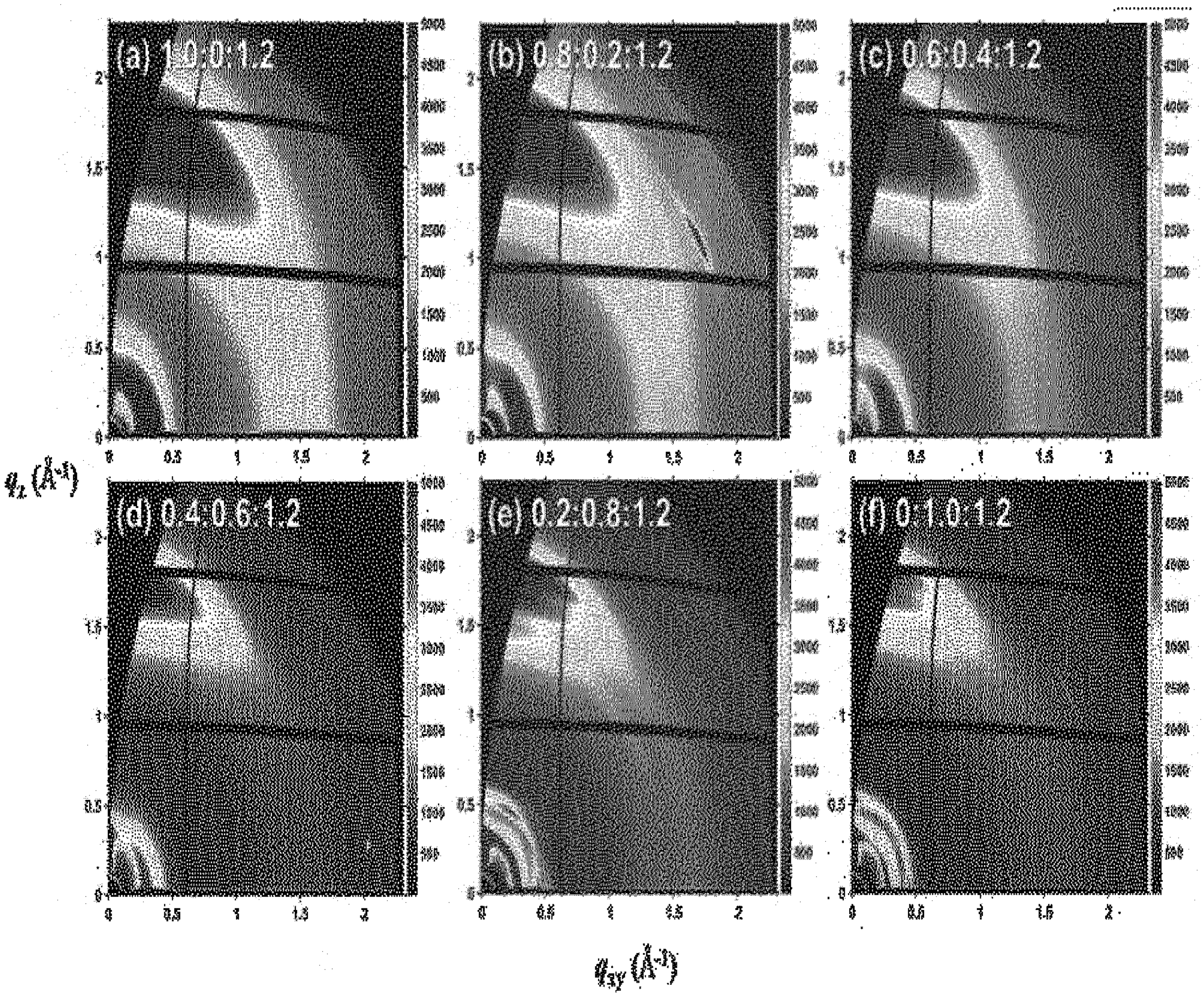
(a) 1.0:0:1.2
(b) 0.8:0.2:1.2
(c) 0.6:0.4:1.2
(d) 0.4:0.6:1.2
(e) 0.2:0.8:1.2
(f) 0:1.0:1.2
$q_z$ (Å$^{-1}$)
$q_{xy}$ (Å$^{-1}$)

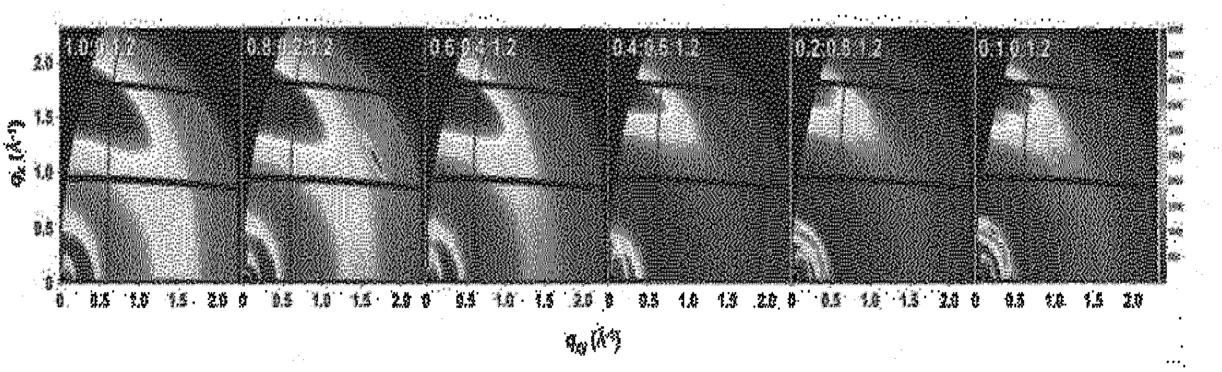
$q_z$ (Å$^{-1}$)
$q_{xy}$ (Å$^{-1}$)

【Fig. 135】

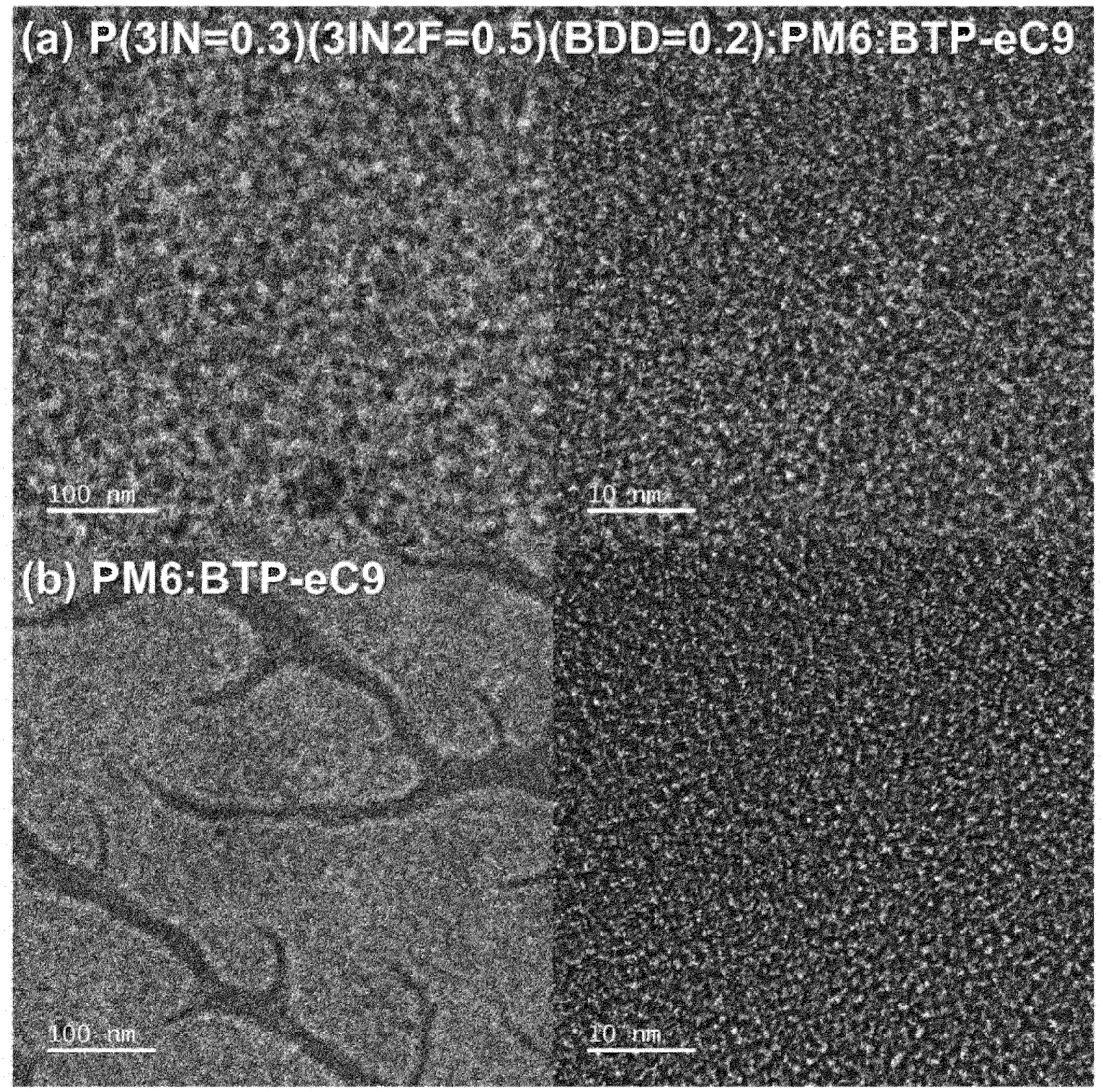
(a) P(3IN=0.3)(3IN2F=0.5)(BDD=0.2):PM6:BTP-eC9
100 nm
10 nm
(b) PM6:BTP-eC9
100 nm
10 nm

【Fig. 136】

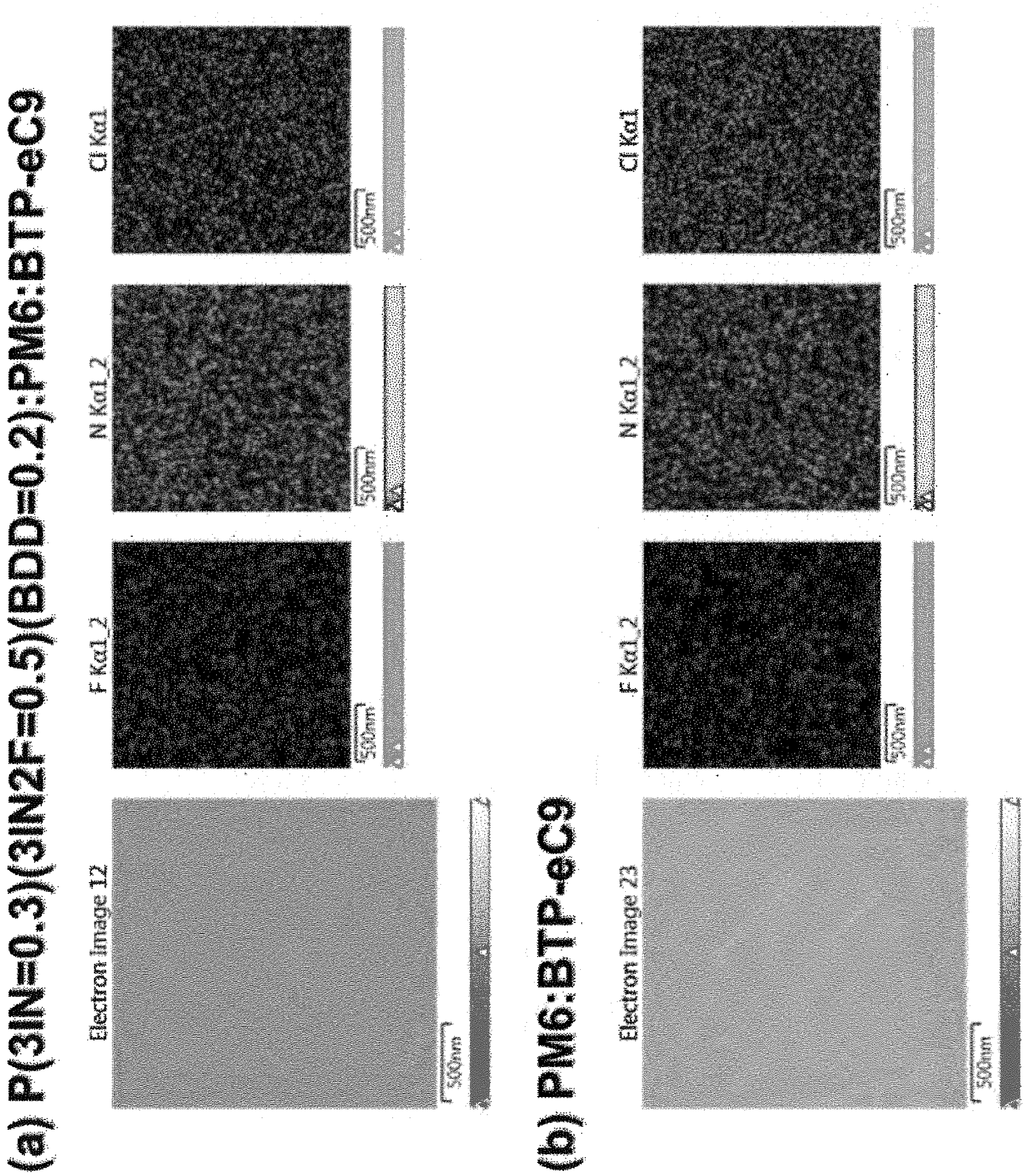
(a) P(3IN=0.3)(3IN2F=0.5)(BDD=0.2):PM6:BTP-eC9
Electron Image 12
F Kα1_2
N Kα1_2
Cl Kα1
500nm
(b) PM6:BTP-eC9
Electron Image 23
F Kα1_2
N Kα1_2
Cl Kα1
500nm

【Fig. 137】

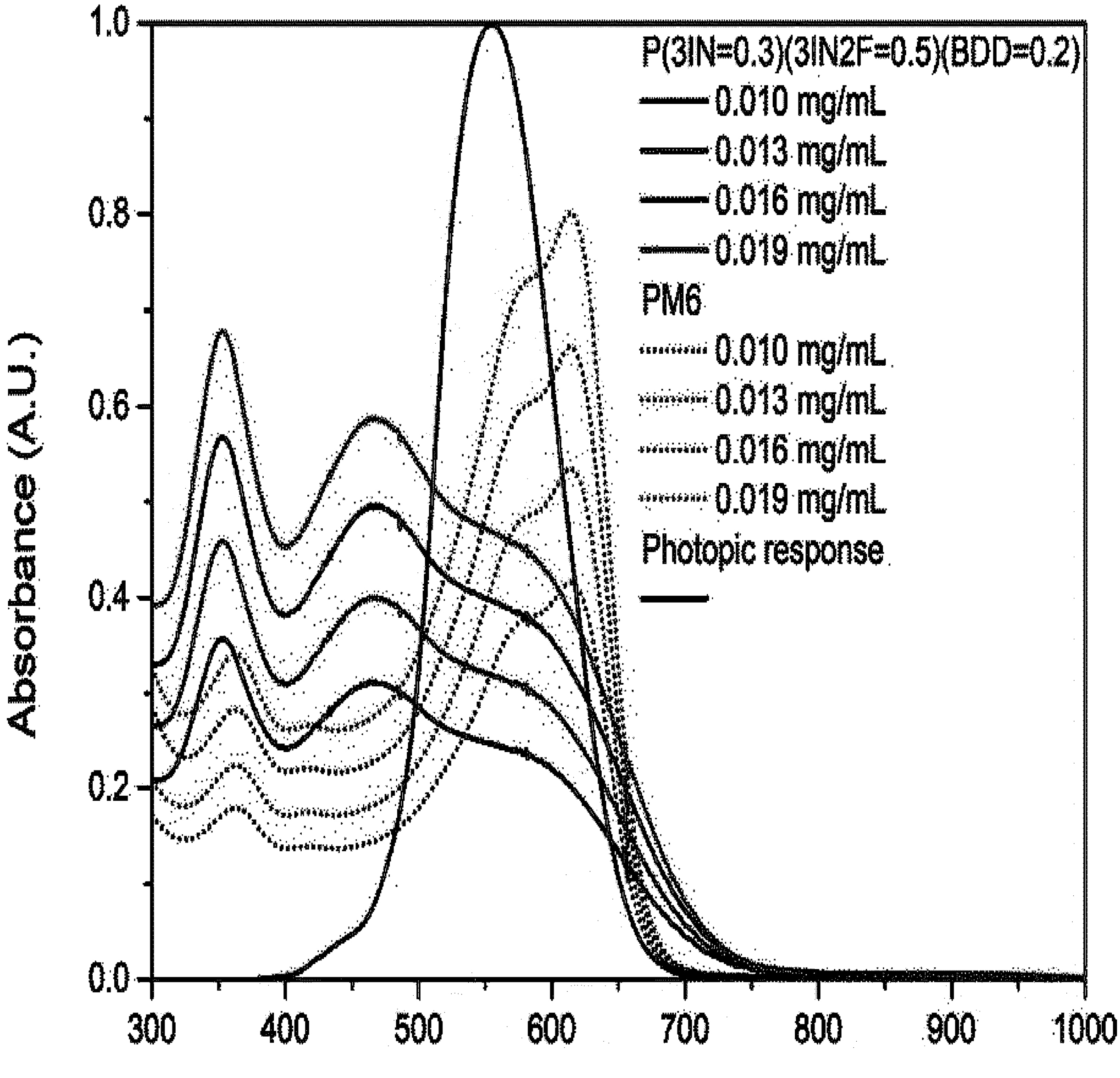
P(3IN=0.3)(3IN2F=0.5)(BDD=0.2)
0.010 mg/mL
0.013 mg/mL
0.016 mg/mL
0.019 mg/mL
PM6
0.010 mg/mL
0.013 mg/mL
0.016 mg/mL
0.019 mg/mL
Photopic response
Absorbance (A.U.)
Wavelength (nm)
1.0
0.8
0.6
0.4
0.2
0.0
300
400
500
600
700
800
900
1000

【Fig. 138】

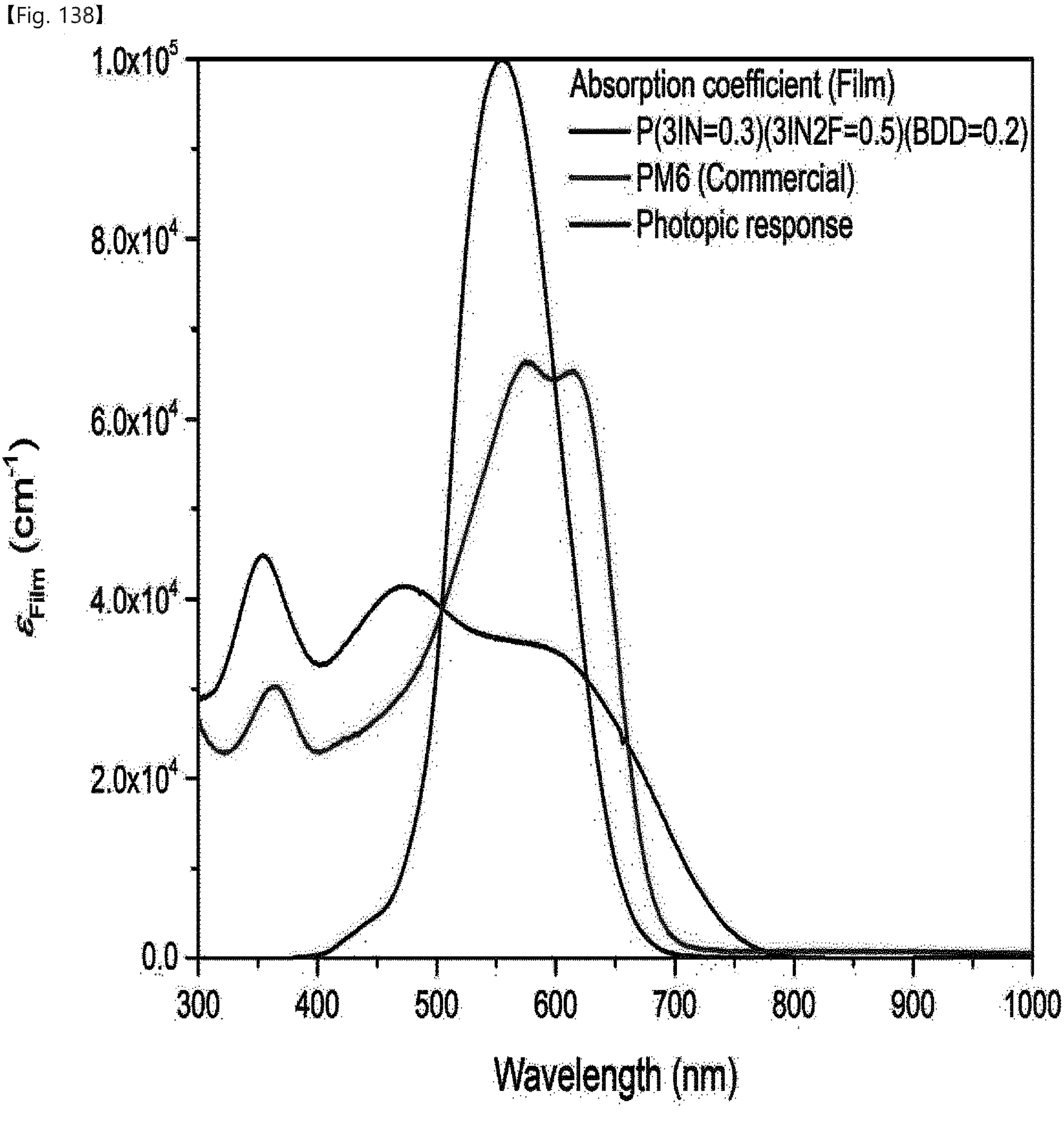
1.0x10^5
8.0x10^4
6.0x10^4
4.0x10^4
2.0x10^4
0.0
ε_Film (cm^-1)
300
400
500
600
700
800
900
1000
Wavelength (nm)
Absorption coefficient (Film)
P(3IN=0.3)(3IN2F=0.5)(BDD=0.2)
PM6 (Commercial)
Photopic response

【Fig. 139】

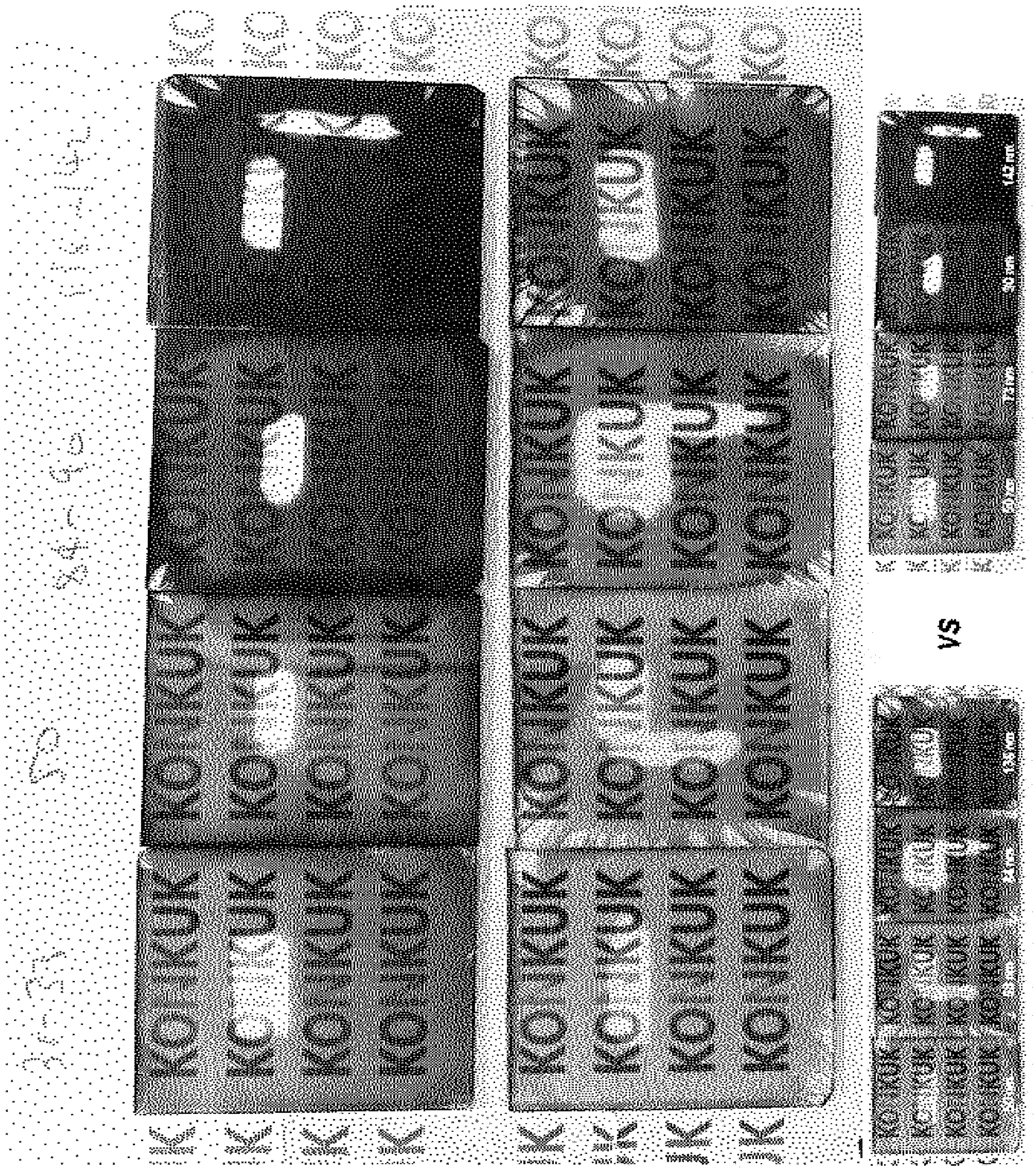

【Fig. 140】

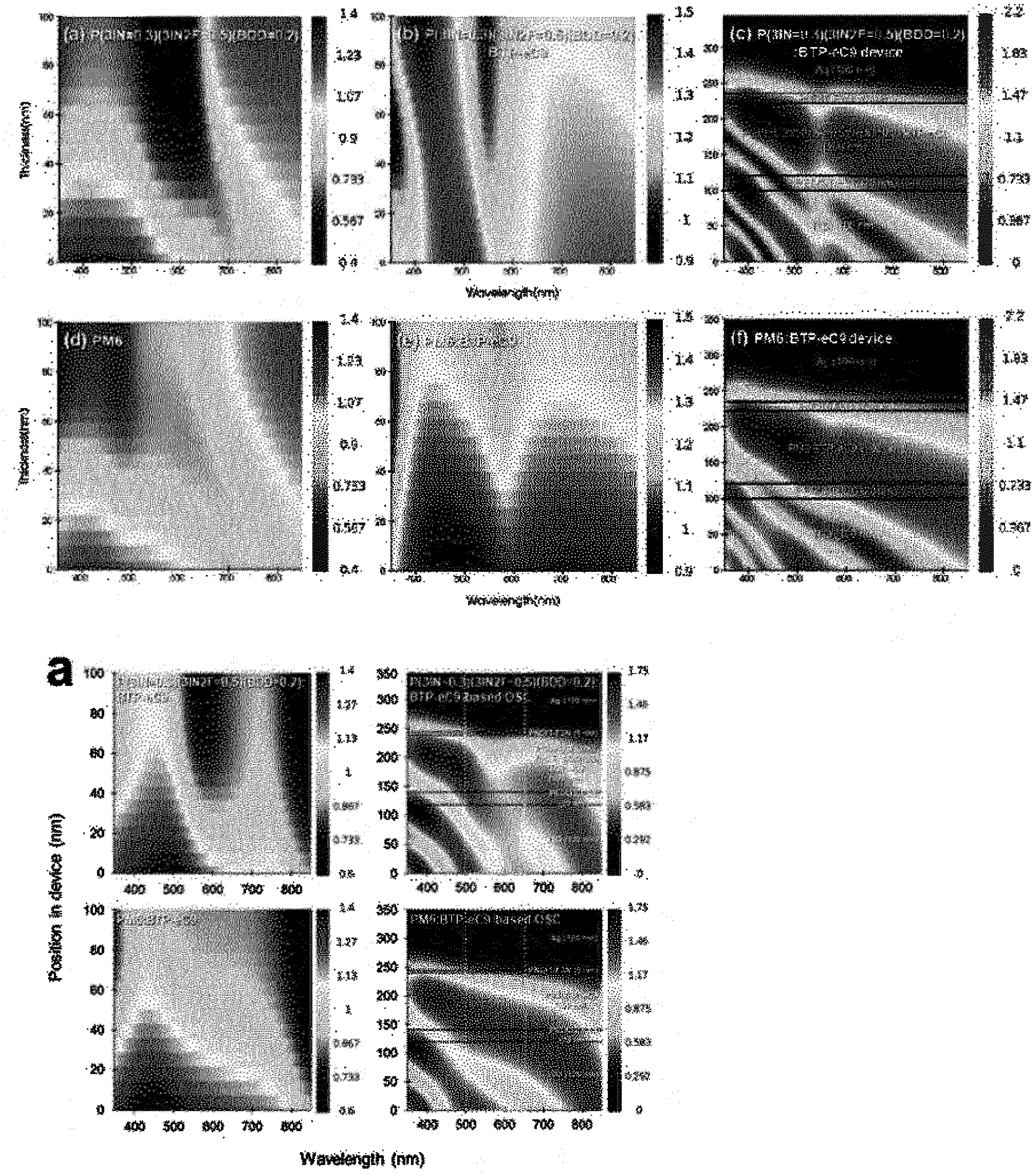
(c) P(3IN=0.3)(3IN2F=0.5)(BDD=0.2):BTP-eC9 device
(d) PM6
(f) PM6:BTP-eC9 device
Thickness(nm)
Wavelength(nm)
a
PM6:BTP-eC9 based OSC
Position in device (nm)
Wavelength (nm)

【Fig. 141】

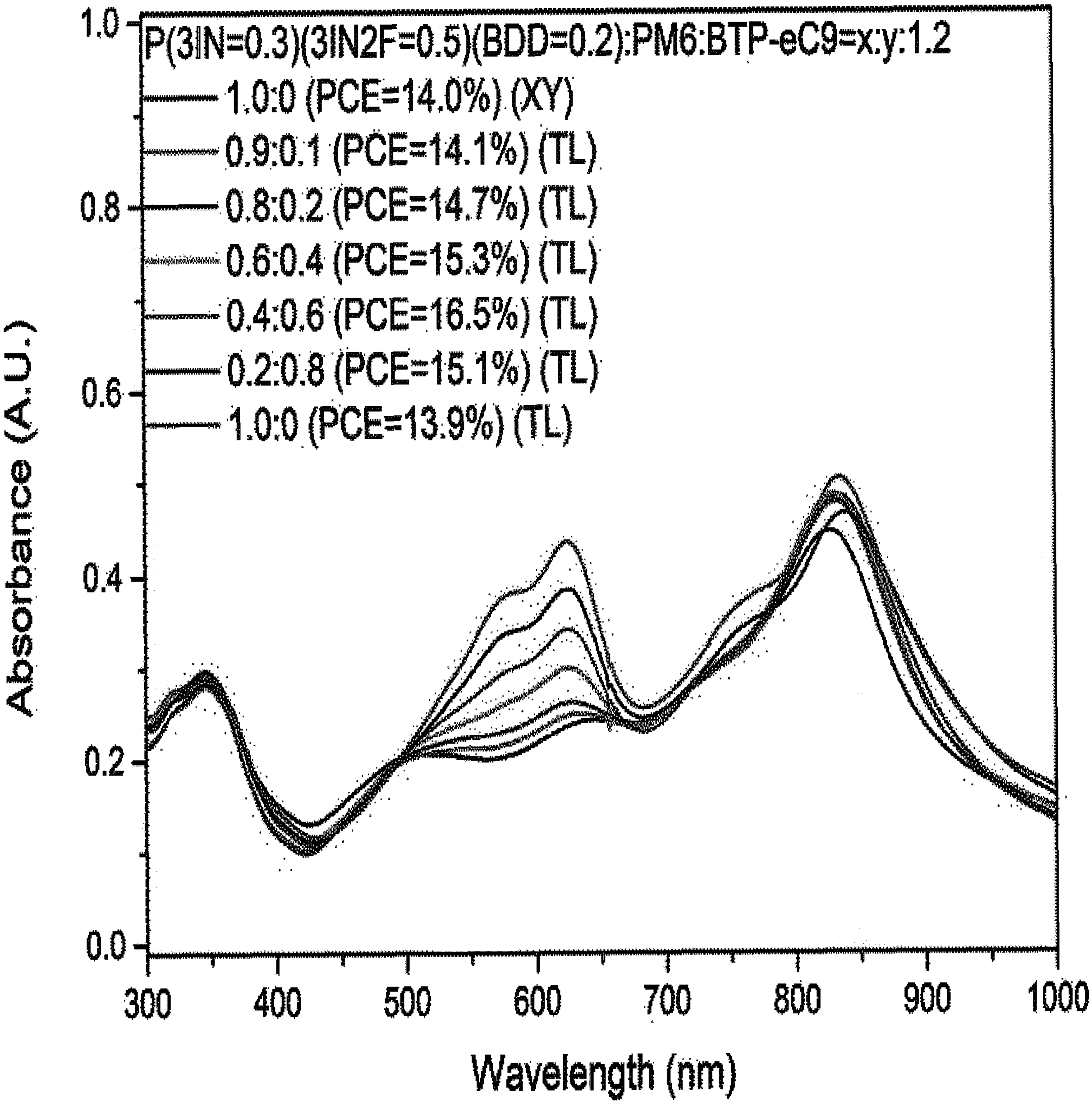
P(3IN=0.3)(3IN2F=0.5)(BDD=0.2):PM6:BTP-eC9=x:y:1.2
1.0:0 (PCE=14.0%) (XY)
0.9:0.1 (PCE=14.1%) (TL)
0.8:0.2 (PCE=14.7%) (TL)
0.6:0.4 (PCE=15.3%) (TL)
0.4:0.6 (PCE=16.5%) (TL)
0.2:0.8 (PCE=15.1%) (TL)
1.0:0 (PCE=13.9%) (TL)
Absorbance (A.U.)
Wavelength (nm)
0.0
0.2
0.4
0.6
0.8
1.0
300
400
500
600
700
800
900
1000

【Fig. 142】

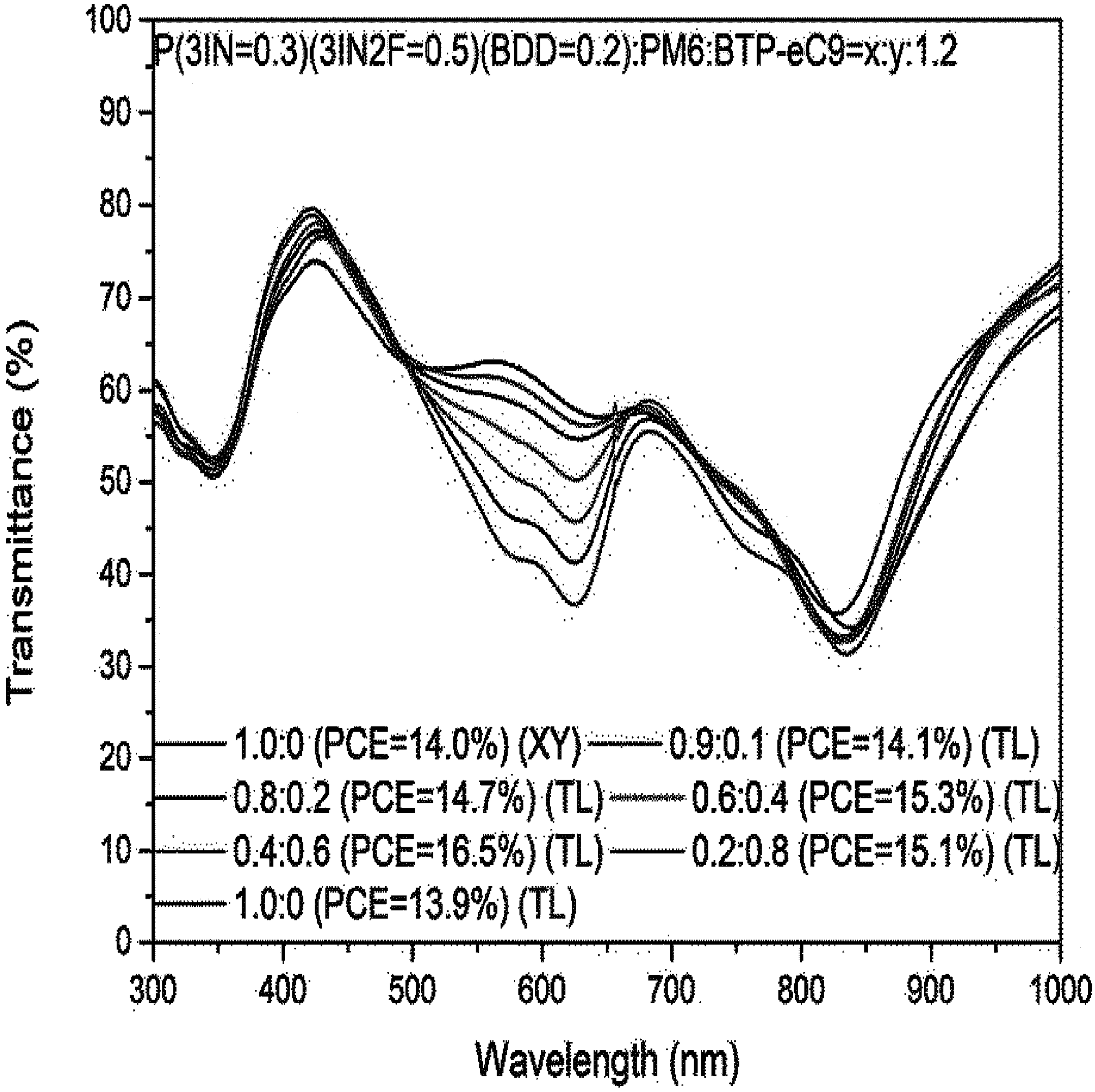
P(3IN=0.3)(3IN2F=0.5)(BDD=0.2):PM6:BTP-eC9=x:y:1.2
Transmittance (%)
Wavelength (nm)
100
90
80
70
60
50
40
30
20
10
0
300
400
500
600
700
800
900
1000
1.0:0 (PCE=14.0%) (XY)
0.9:0.1 (PCE=14.1%) (TL)
0.8:0.2 (PCE=14.7%) (TL)
0.6:0.4 (PCE=15.3%) (TL)
0.4:0.6 (PCE=16.5%) (TL)
0.2:0.8 (PCE=15.1%) (TL)
1.0:0 (PCE=13.9%) (TL)

【Fig. 143】

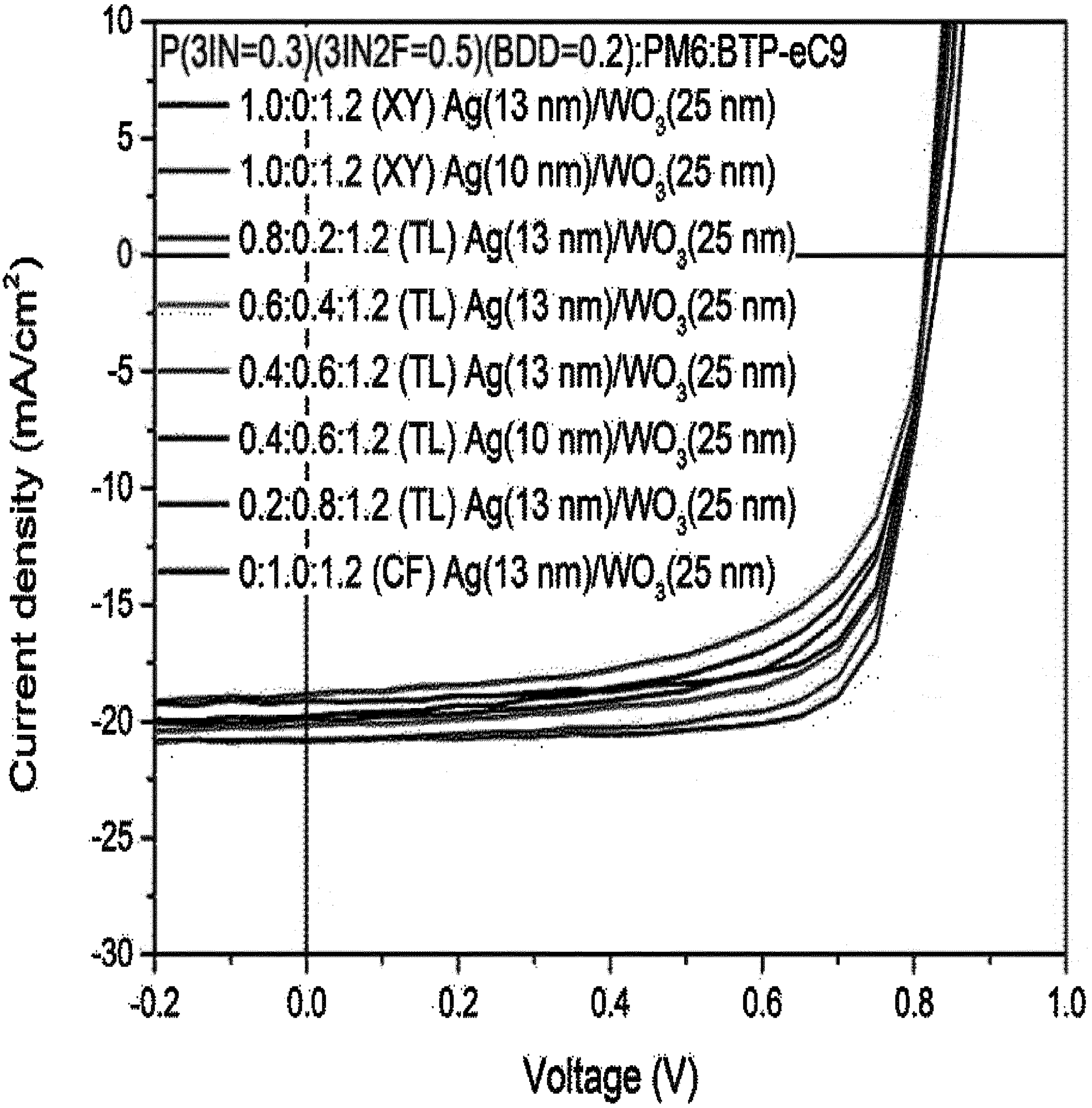
P(3IN=0.3)(3IN2F=0.5)(BDD=0.2):PM6:BTP-eC9
1.0:0:1.2 (XY) Ag(13 nm)/WO3(25 nm)
1.0:0:1.2 (XY) Ag(10 nm)/WO3(25 nm)
0.8:0.2:1.2 (TL) Ag(13 nm)/WO3(25 nm)
0.6:0.4:1.2 (TL) Ag(13 nm)/WO3(25 nm)
0.4:0.6:1.2 (TL) Ag(13 nm)/WO3(25 nm)
0.4:0.6:1.2 (TL) Ag(10 nm)/WO3(25 nm)
0.2:0.8:1.2 (TL) Ag(13 nm)/WO3(25 nm)
0:1.0:1.2 (CF) Ag(13 nm)/WO3(25 nm)
Current density (mA/cm2)
Voltage (V)
10
5
0
-5
-10
-15
-20
-25
-30
-0.2
0.0
0.2
0.4
0.6
0.8
1.0

【Fig. 144】

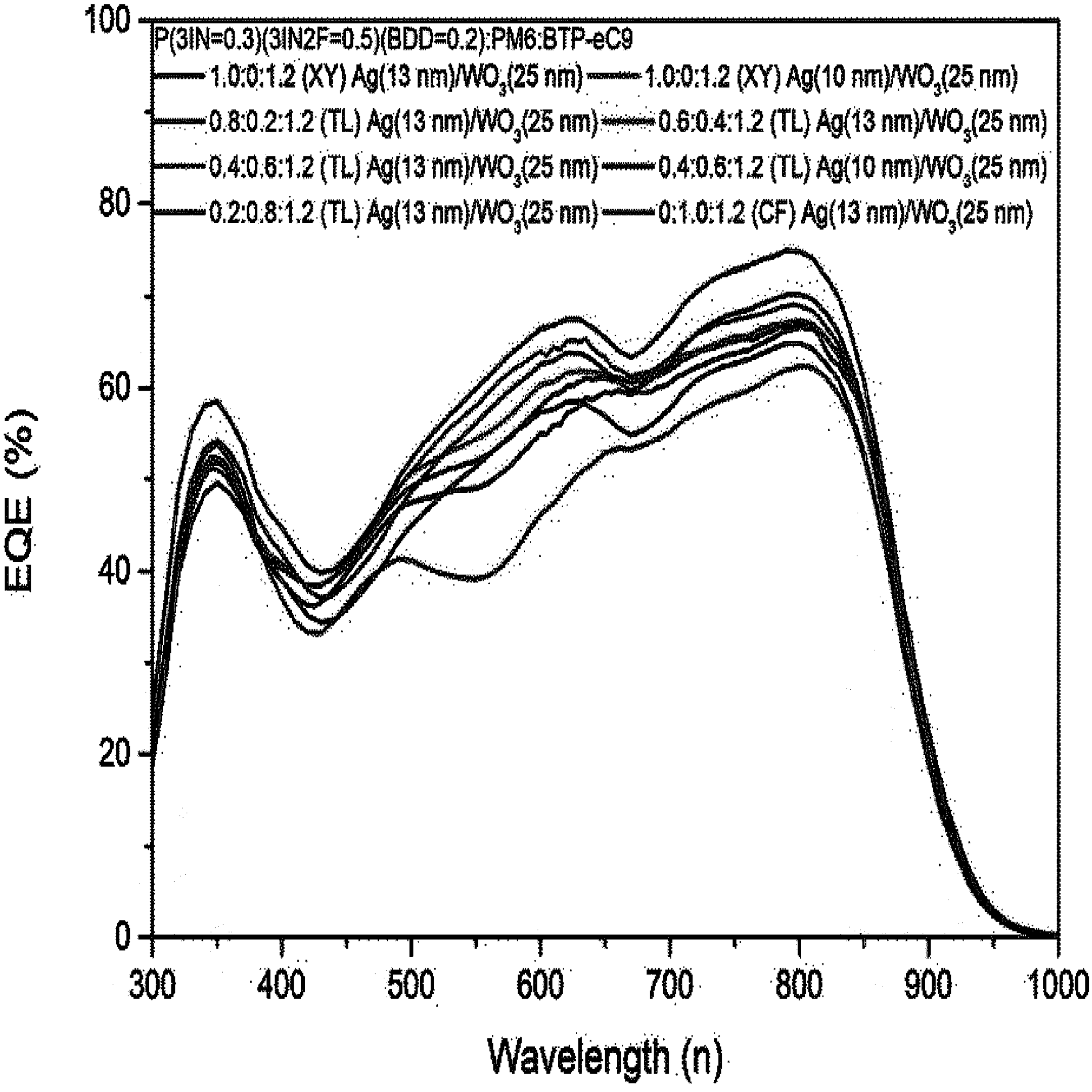
P(3IN=0.3)(3IN2F=0.5)(BDD=0.2):PM6:BTP-eC9
1.0:0:1.2 (XY) Ag(13 nm)/WO3(25 nm)
1.0:0:1.2 (XY) Ag(10 nm)/WO3(25 nm)
0.8:0.2:1.2 (TL) Ag(13 nm)/WO3(25 nm)
0.6:0.4:1.2 (TL) Ag(13 nm)/WO3(25 nm)
0.4:0.6:1.2 (TL) Ag(13 nm)/WO3(25 nm)
0.4:0.6:1.2 (TL) Ag(10 nm)/WO3(25 nm)
0.2:0.8:1.2 (TL) Ag(13 nm)/WO3(25 nm)
0:1.0:1.2 (CF) Ag(13 nm)/WO3(25 nm)
EQE (%)
Wavelength (n)
0
20
40
60
80
100
300
400
500
600
700
800
900
1000

【Fig. 145】

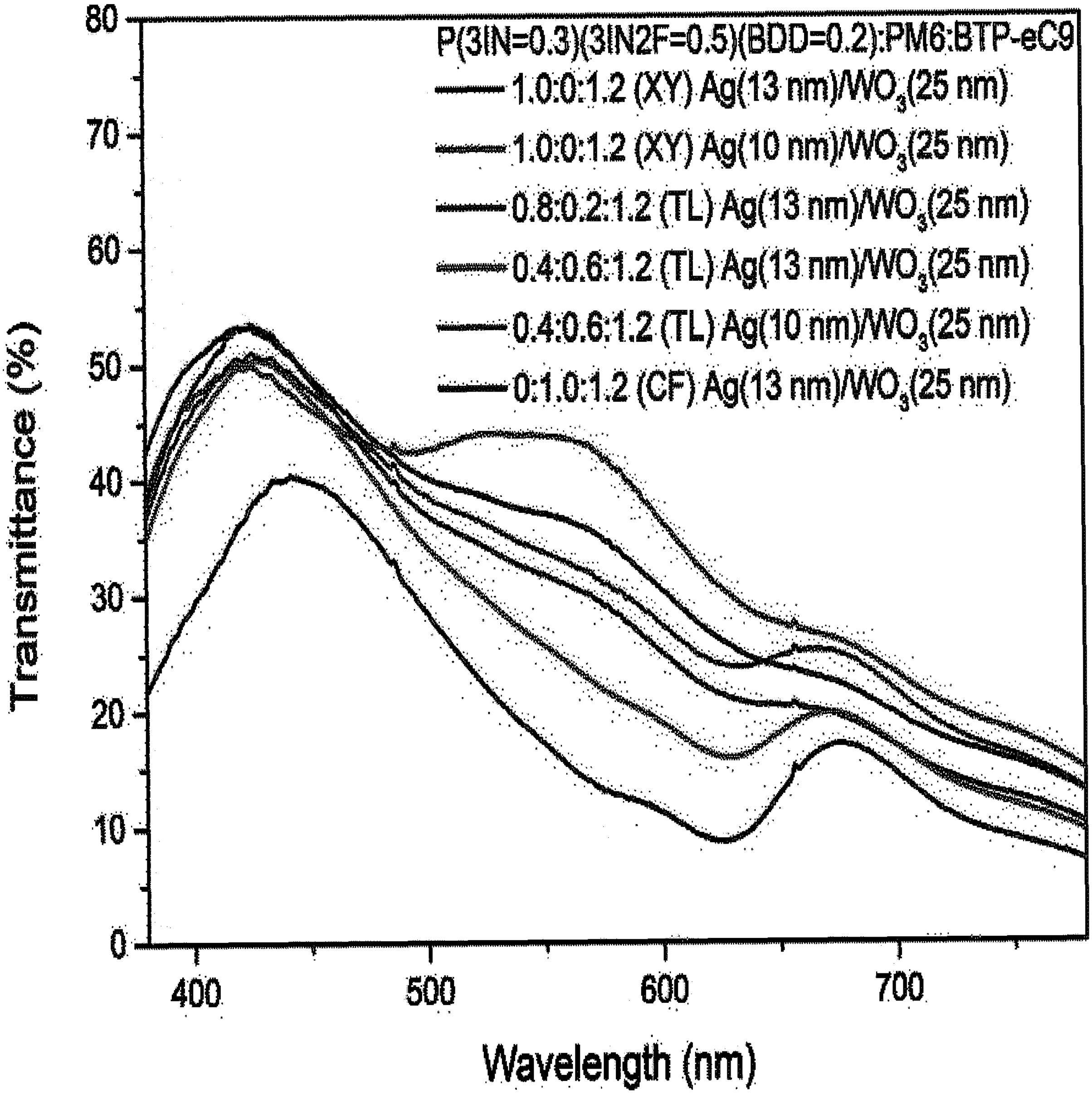
P(3IN=0.3)(3IN2F=0.5)(BDD=0.2):PM6:BTP-eC9
1.0:0:1.2 (XY) Ag(13 nm)/WO3(25 nm)
1.0:0:1.2 (XY) Ag(10 nm)/WO3(25 nm)
0.8:0.2:1.2 (TL) Ag(13 nm)/WO3(25 nm)
0.4:0.6:1.2 (TL) Ag(13 nm)/WO3(25 nm)
0.4:0.6:1.2 (TL) Ag(10 nm)/WO3(25 nm)
0:1.0:1.2 (CF) Ag(13 nm)/WO3(25 nm)
Transmittance (%)
Wavelength (nm)
0
10
20
30
40
50
60
70
80
400
500
600
700

【Fig. 146】

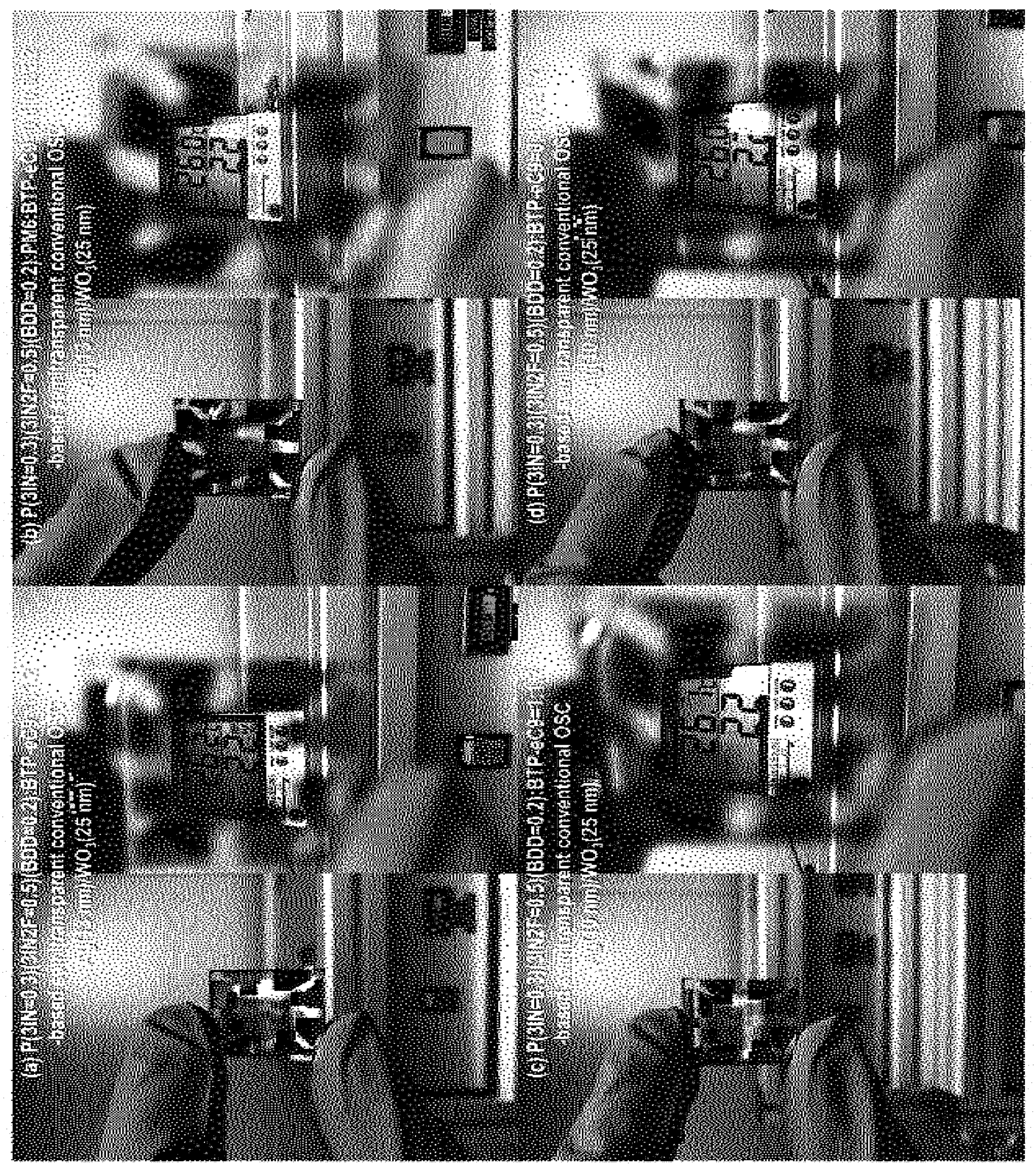

【Fig. 147】

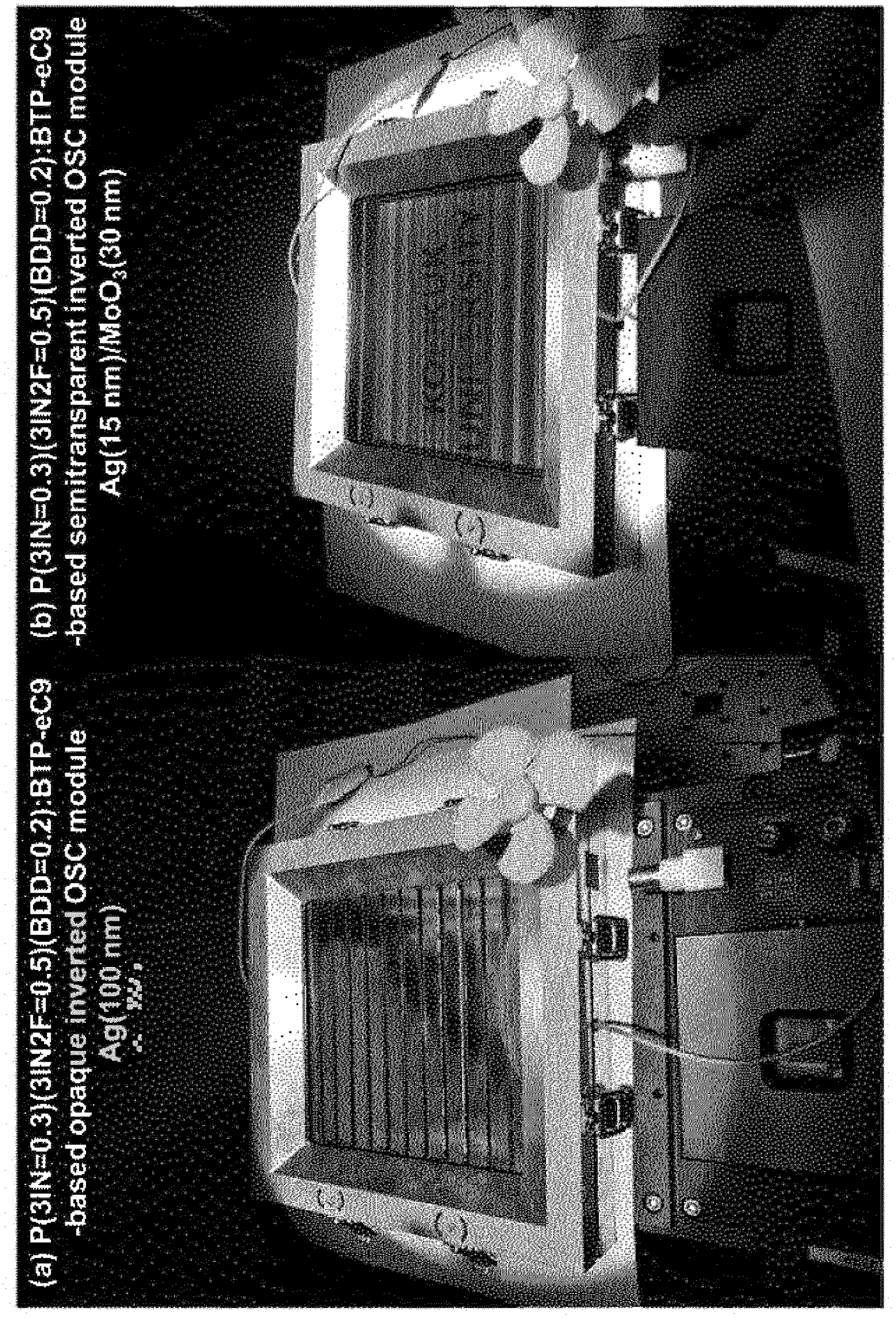
(a) P(3IN=0.3)(3IN2F=0.5)(BDD=0.2):BTP-eC9
-based opaque inverted OSC module
Ag(100 nm)
(b) P(3IN=0.3)(3IN2F=0.5)(BDD=0.2):BTP-eC9
-based semitransparent inverted OSC module
Ag(15 nm)/MoO3(30 nm)

【Fig. 148】

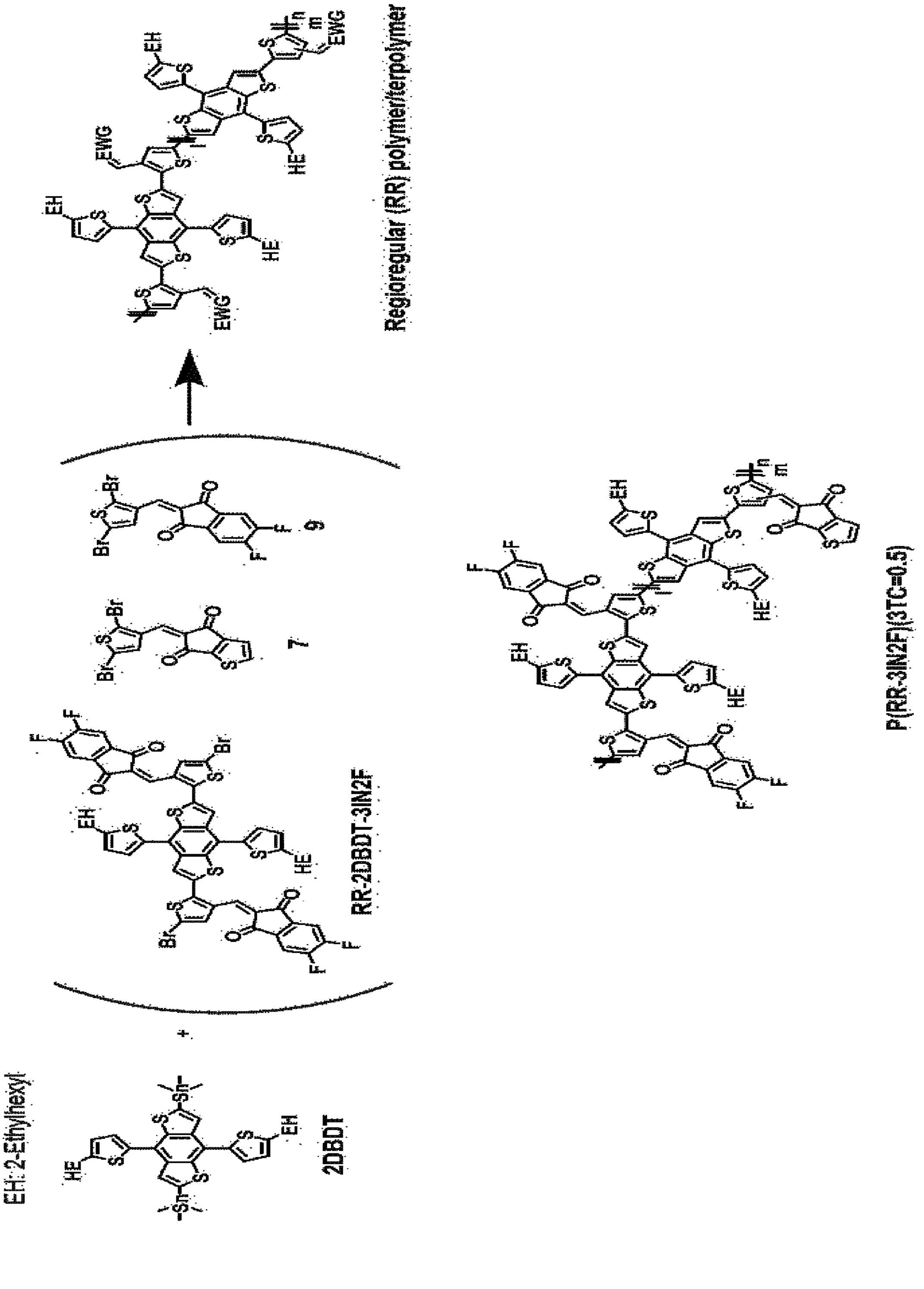

EH: 2-Ethylhexyl

2DBDT

+

RR-2DBDT-3IN2F

7

9

Regioregular (RR) polymer/terpolymer

P(RR-3IN2F)(3TC=0.5)

【Fig. 149】

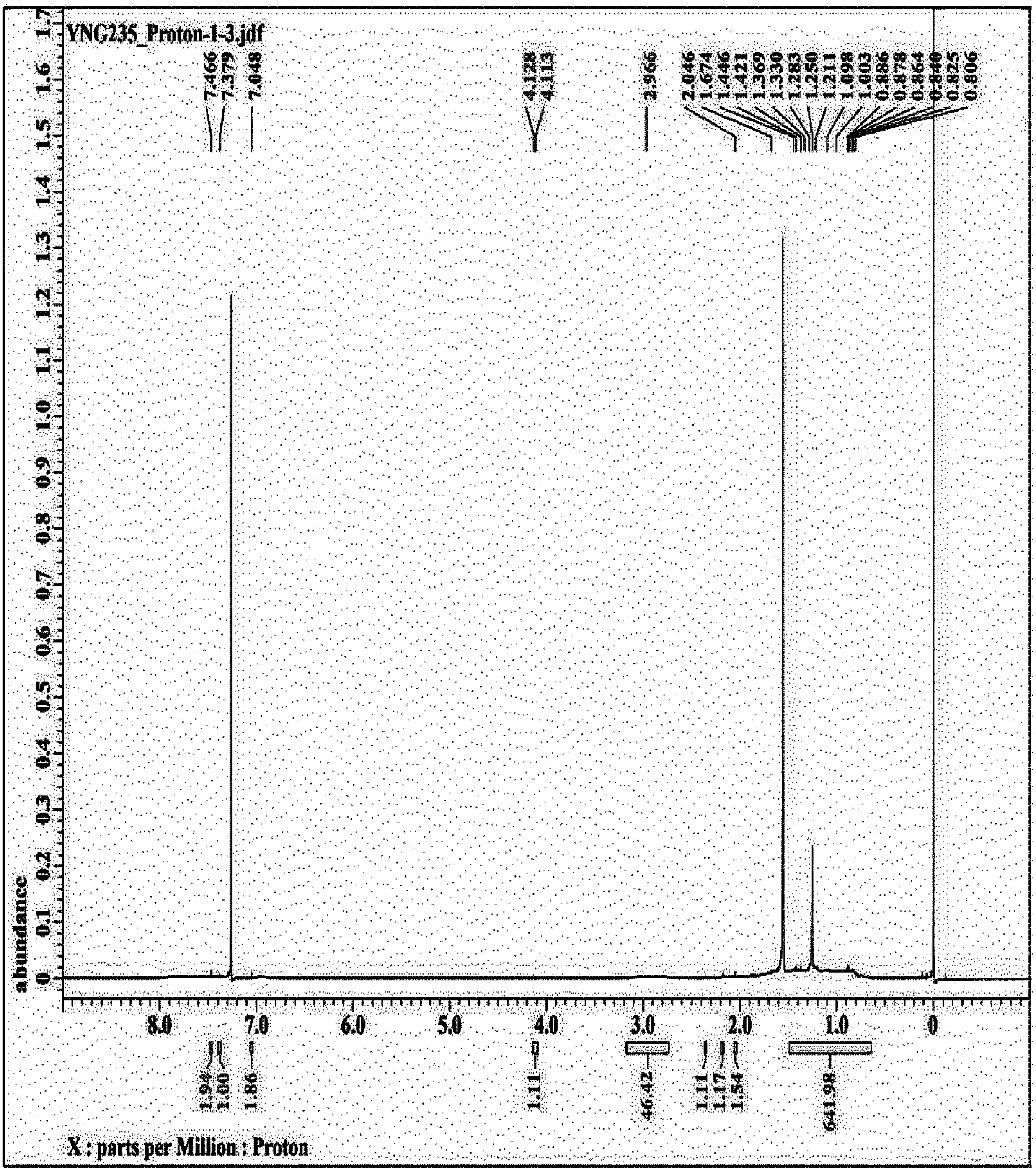
YNG235_Proton-1-3.jdf
7.466
7.379
7.048
4.128
4.113
2.966
2.046
1.674
1.446
1.421
1.369
1.330
1.283
1.250
1.211
1.098
1.003
0.886
0.878
0.864
0.840
0.825
0.806
abundance
0 0.1 0.2 0.3 0.4 0.5 0.6 0.7 0.8 0.9 1.0 1.1 1.2 1.3 1.4 1.5 1.6 1.7
8.0 7.0 6.0 5.0 4.0 3.0 2.0 1.0 0
1.94
1.00
1.86
1.11
46.42
1.11
1.17
1.54
641.98
X : parts per Million : Proton

【Fig. 150】

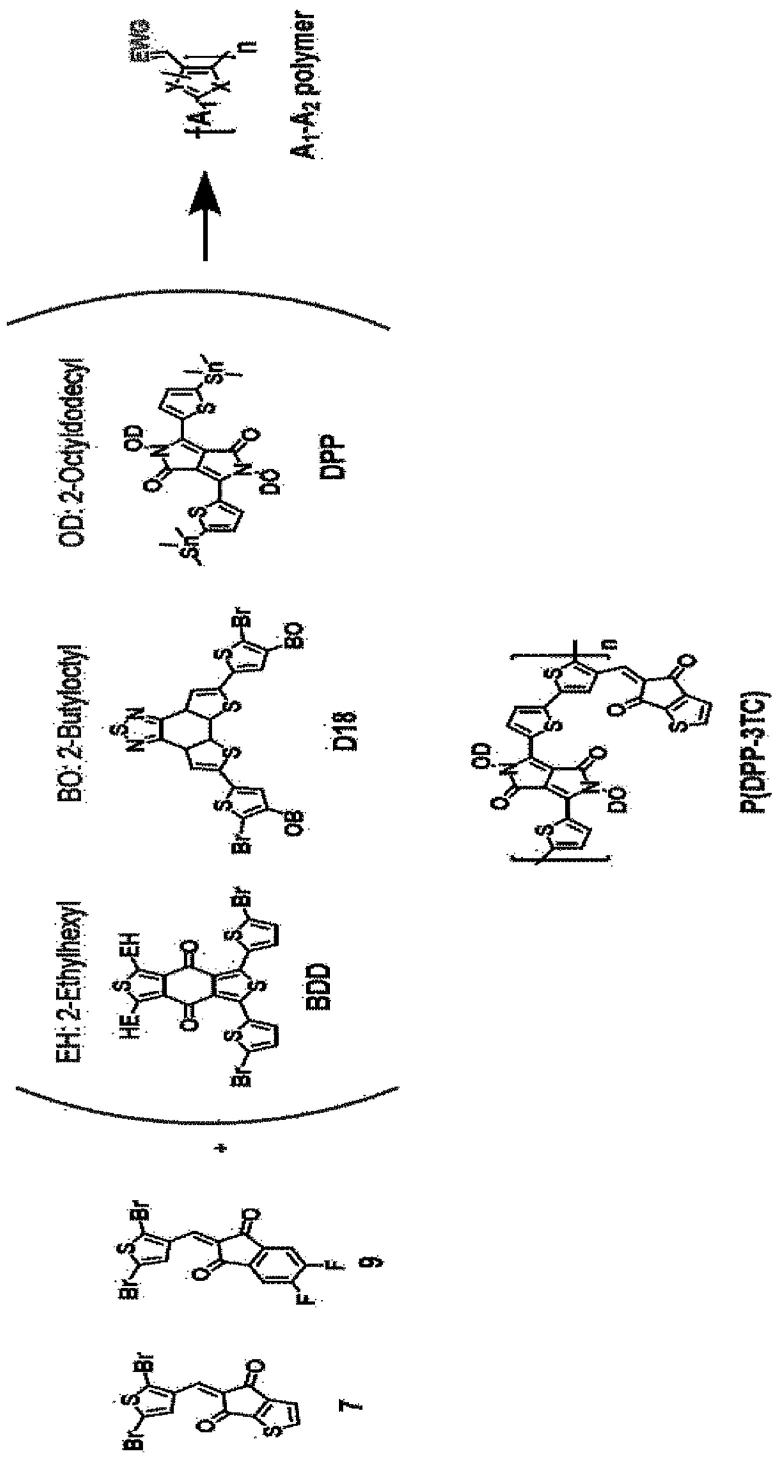
EH: 2-Ethylhexyl
BO: 2-Butyloctyl
OD: 2-Octyldodecyl
7
9
BDD
D18
DPP
$A_1$-$A_2$ polymer
P(DPP-3TC)

【Fig. 151】

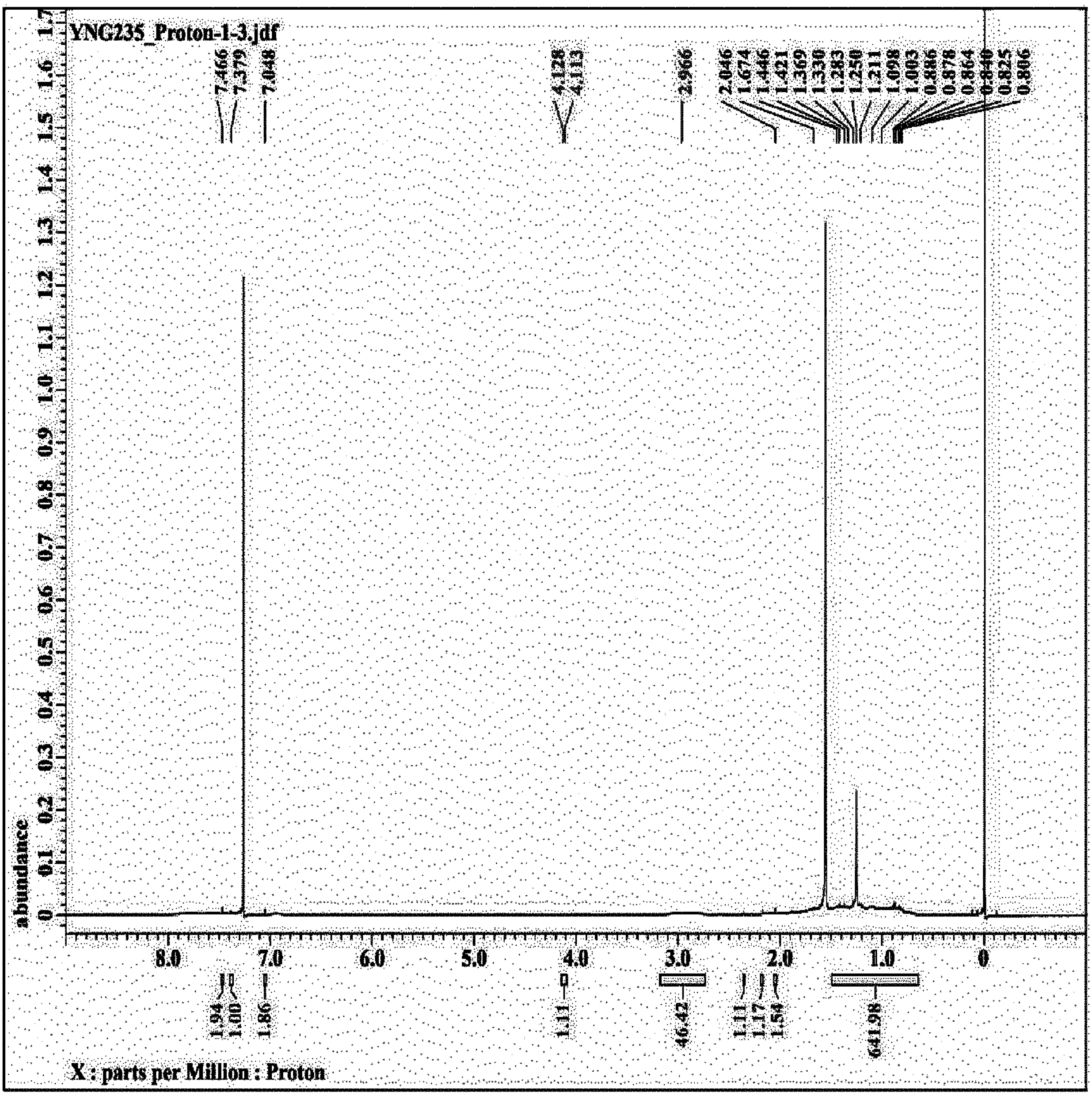
YNG235_Proton-1-3.jdf
7.466
7.379
7.048
4.128
4.113
2.966
2.046
1.674
1.446
1.421
1.369
1.330
1.283
1.250
1.211
1.098
1.003
0.886
0.878
0.864
0.840
0.825
0.806
abundance
1.94
1.00
1.86
1.11
46.42
1.11
1.17
1.54
641.98
X : parts per Million : Proton

# INTERNATIONAL SEARCH REPORT

International application No.
**PCT/KR2023/010924**

## A. CLASSIFICATION OF SUBJECT MATTER

**C07D 333/28**(2006.01)i; **C07D 277/32**(2006.01)i; **C07D 307/56**(2006.01)i; **C07D 345/00**(2006.01)i; **H10K 85/60**(2023.01)i; **H10K 30/20**(2023.01)i

According to International Patent Classification (IPC) or to both national classification and IPC

## B. FIELDS SEARCHED

Minimum documentation searched (classification system followed by classification symbols)

C07D 333/28(2006.01); C08G 61/12(2006.01); C08G 73/06(2006.01); C08K 3/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 유기 반도체 화합물(organic semiconductor compound), 유기태양전지(organic solar cell), 유기전자소자(organic electronic device)

## C. DOCUMENTS CONSIDERED TO BE RELEVANT

| Category* | Citation of document, with indication, where appropriate, of the relevant passages | Relevant to claim No. |
|---|---|---|
| X | CN 110628002 A (HUZHOU TEACHERS COLLEGE) 31 December 2019 (2019-12-31)<br>See abstract; claims 1 and 2; paragraphs [0002], [0029], [0035], [0038] and [0047]; and figure 2. | 1-4,7,9,12,13 |
| A | | 5,6,8,10,11 |
| X | CHEN, L. et al. Side-chain engineering of benzodithiophene–thiophene copolymers with conjugated side chains containing the electron-withdrawing ethylrhodanine group. J. Mater. Chem. A. 2015, vol. 3, pp. 12005-12015.<br>See figure 1 (copolymer PEHBDT-T-R); formula 1 (compound M); and table 1. | 1-4,7 |
| X | WAGNER, K. et al. Indanedione-substituted poly(terthiophene)s:processable conducting polymers with intramolecular charge transfer interactions. Macromolecules. 2010, vol. 43, pp. 3817–3827.<br>See formula 1 (compound 5). | 1 |
| A | KR 10-2015-0032215 A (LG CHEM, LTD.) 25 March 2015 (2015-03-25)<br>See entire document. | 1-13 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **13 December 2023** | **14 December 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT

International application No.
**PCT/KR2023/010924**

## C. DOCUMENTS CONSIDERED TO BE RELEVANT

| Category* | Citation of document, with indication, where appropriate, of the relevant passages | Relevant to claim No. |
|---|---|---|
| A | KR 10-2019-0103064 A (KONKUK UNIVERSITY INDUSTRIAL COOPERATION CORP) 04 September 2019 (2019-09-04)<br>See entire document. | 1-13 |

Form PCT/ISA/210 (second sheet) (July 2022)

**INTERNATIONAL SEARCH REPORT**
**Information on patent family members**

International application No.
**PCT/KR2023/010924**

| Patent document cited in search report | Publication date (day/month/year) | Patent family member(s) | Publication date (day/month/year) |
|---|---|---|---|
| CN 110628002 A | 31 December 2019 | CN 110628002 B | 09 September 2022 |
| KR 10-2015-0032215 A | 25 March 2015 | CN 105637009 A | 01 June 2016 |
| | | CN 105637009 B | 12 June 2018 |
| | | EP 3048123 A1 | 27 July 2016 |
| | | EP 3048123 A4 | 03 May 2017 |
| | | JP 2016-536441 A | 24 November 2016 |
| | | JP 6200096 B2 | 20 September 2017 |
| | | KR 10-1638693 B1 | 11 July 2016 |
| | | US 2016-0237204 A1 | 18 August 2016 |
| | | US 9777108 B2 | 03 October 2017 |
| | | WO 2015-037966 A1 | 19 March 2015 |
| KR 10-2019-0103064 A | 04 September 2019 | KR 10-2167007 B1 | 16 October 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Nature Communication*, 2016, vol. 7, 11585
- *Nature Materials*, 2017, vol. 16, 363-369
- *Macromolecule*, 2015, vol. 48 (3), 453-461
- *Adv. Mater.*, 2020, 1908205